# EUROPEAN PATENT APPLICATION

(11) **EP 1 593 687 A2**
(43) Date of publication of application: **09.11.2005**
(21) Application number: 04027154.6
(22) Date of filing: 09.06.1999
(51) Int. Cl.: C07K 14/47, A61K 38/17

(54) **Human genes differentially expressed in colon cancer**

(30) Priority: 10.06.1998 US 88801
(62) Divisional of application: 99923781.1
(71) Applicant: BAYER CORPORATION, Pittsburgh, PA 15205 (US)
(72) Inventor: The designation of the inventor has not yet been filed
(74) Representative: Burkert, Frank

(57) **Abstract**

This invention relates to novel human genes, to proteins expressed by the genes, and to variants of the proteins. The invention also relates to diagnostic assays and therapeutic agents related to the genes and proteins, including probes, antisense constructs, and antibodies. The subject nucleic acids have been found to be differentially regulated in tumor cells, particularly colon cancer cell lines and/or tissue.

SEQUENCE LISTING

<110> BAYER CORPORATION

<120> NOVEL HUMAN GENES AND GENE EXPRESSION
PRODUCTS

<130> CCD-257 (PCT)

<150> US 60/088,801
<151> 1988-06-10

<160> 850

<170> FastSEQ for Windows Version 3.0

<210> 1
<211> 359
<212> DNA
<213> Homo sapiens

<400> 1
tacaaaacta acgatgaagt tattcatggc atcttcaaag cttacattca gaggctgctt 60
cacgccttgg ctcgacactg ccagctggaa ccagaccatg agggggttcc tgaggagact 120
gatgactttg gggagtttcg catgagggta tcagacctgg taaaggactt gattttcttg 180
atagggtcta tggagtgttt tgctcagtta tattctactc tgaaagaagg caacccaccc 240
tgggaggtga cagaagcggt tctctttatc atgactgcta tagcaaagag tgttgatccg 300
gaaaacaatc caacacttgt ggaagtccta gaaggagttg tccgcctccc ggagaccgt 359

<210> 2
<211> 901
<212> DNA
<213> Homo sapiens

<220>
<221> misc_feature
<222> (1)...(901)
<223> n = A,T,C or G

<400> 2
tacactacct tttaaaaaaa attggtatat attactttta ctgtaaagaa atgctttaaa 60
tcaggggtcc ccaaccccca ngtcacanac ctgaangggt ccatgntatg nnatgaacca 120
ngccacacag nnggangtaa gcanctgaga gcgagggaag cctagnntgn atttacagaa 180
aagggaagct ncatctgtat ttacagccac tccccactgc tcacattatg gcctgagctc 240
tgcctcccgt nagatcagga gacattatat tctcatagga gcatgaacac tattgngaac 300
tgcacatnca anggatctgg gttgtctggg ttgtgcgctc cttataaaaa tctaatggtg 360
gatgatttgt cactgtctgc catcatccct agatggaaaa caagctcacc caaagtctcn 420
cttntgccna ggngtncctg atgccaagat tcncattttt gacctggggc ggaaaaaggc 480
naaagnggat gagttccgct ttgnggccac atgntgtnag atgaatntga gcagctgcct 540
ctgaagccct ggaggctgcc cgaatttgng ccaatannta ccccgaagcg ctggtacgat 600
tcccaagggg agcgcctttt acactgngcc ctganacttc nnttccagat cggtcnggcc 660
ttttaacttt tggtttcccg tttgtcaaan gacattgctt cctttanttt tncagctggt 720

gngncttgga aaggattggg ccctggcttc tcnaggatgg ctaaggatga anngatatca 780
aggnctggca tgaaanaant cnccggtccn nctttnggct nggttncctt gggacctggc 840
cgggccggtc cgtttcgaaa gggcnaaatt ctggcagaat ttccttgana cctgggcggg 900
g 901

<210> 3
<211> 553
<212> DNA
<213> Homo sapiens

<220>
<221> misc_feature
<222> (1)...(553)
<223> n = A,T,C or G

<400> 3
actgctttct gctgccgctc aggatagcac tggctttcac agggattanc cttctggtgg 60
tgggcacaac tgtggtggga tacttgccaa atgggaggtt taaggagttc atgagtaaac 120
atgttcactt aatgtgttac cggatctgcg tgcgagcgct gacagccatc atcacctacc 180
atgacaggga aaacagacca agaaatggtg gcatctgtgt ggccaatcat acctcaccga 240
tcgatgngat catcttggcc agcgatggct attatgccat ggtgggtcaa gtgcacgggg 300
gactcatggn tgtgattcac agagccatgg tgaaggcctg cccacacgtc tggtttgagc 360
gctcggaagt gaaggatcgc cacctggtgg ctaagagact gactgaacat gtgcaagatn 420
aaagcaagct gcctatcctc atcttcccag aaggaacctg catcaataat acatcggnga 480
tgatgttcaa aaagggaagn tttgaaattg nagccacagt ttaccctggn gctatnaagt 540
atgaccctca att 553

<210> 4
<211> 565
<212> DNA
<213> Homo sapiens

<220>
<221> misc_feature
<222> (1)...(565)
<223> n = A,T,C or G

<400> 4
actgctttct gctgccgctc aggatagcac tggctttcac agggattagc cttctggtgg 60
tgggcacaac tgtggtggga tacttgccaa atgggaggtt taaggagttc atgagtaaac 120
atgttcactt aatgtgttac cggatctgcg tgcgagcgct gacagccatc atcacctacc 180
atgacaggga aaacagacca agaaatggtg gcatctgtgt ggccaatcat acctcaccga 240
tcgatgtgat catcttggcc agcgatggct attatgccat ggtgggtcaa gtgcacgggg 300
gactcatggg tgtgattcag agagccatgg tgaaggcctg cccacacgtc tggtttgagc 360
gctcggaagt gaaggatcgc cacctggtgg ctaagagact gactgaacat gtgcaagata 420
aaagcaagct gcctatctca tctttccaga aggaacctgc atcataatac attggtgata 480
tgtcaaaaan gggaagtttt gaaatgganc cccagtttaa cctgnngntt tnagtttnac 540
ccttaatttg gcaagccttt tggan 565

<210> 5
<211> 500
<212> DNA
<213> Homo sapiens

<400> 5
caggtacaca ttcaggggtc actgactctt cagataatgc cctaaacaac tggagtgtgg 60
gcttgtttgc tccaagagca gctgccctgt cagtggaact ccggcgcact tccactcaat 120
actggactgg gggggatgaa agagggattt ttaaatggca gaaaagtgtt cttctgggct 180
gtctggcccg ggcagggcgg gttgtgactt ggaaaagaag gggaaggtag ggaggccttg 240
aacttaggga cagccagcaa atgatccttg cagcttttgg aacacaaggc agggctaagg 300
ttacctttca gcttccttgc ttaagtagca gtggctaagt gggttaaact ttgctcggcc 360
tgcaggctcc ccctgttggt cagatacttg cattgacatc ctcagtgttc aatgctcctg 420
gaagagccca ggagagggcg gcactggccc agggattgca ggtcagggaa ctctagcaaa 480
ttcccacacc ctagggtacc 500

<210> 6
<211> 622
<212> DNA
<213> Homo sapiens

<400> 6
acaaggaaat gtcagtcagg ggtgttgcat attacataca tgtggttacc gaacttggtt 60
tacattattg attaaattca ttttctcttt ctctttttta gacctttgga tatctcctcc 120
tccttcccct tatctataaa tatgtaagaa agaaaacatg tttaaaatac aatattttat 180
ttcttttgat cacagattag acttaaagaa cagagatgcc ctataatgtg atctttaaga 240
gatattacaa agcttccaat ctcactgtga ggatcgttaa agtataataa taaaaaaaaa 300
tgtatattat aaaagaatgt aagaatgtgc atatttattt ccttgcatat taatggcata 360
agaaactgtt aacagggact tggggtaagg cttgtgggaa ggaaggtagt tttcactgta 420
ttccttttgt attgttttaa gtttttactt gttttttaag caagcatgta tcactttata 480
tgatatttaa aagttgctct tctcaagaca gaaaatcatt ttgattcatt tctaattcaa 540
ataagcacta attgaggata ttttaatata tcctcacatt gtgaaaggat taaggcacaa 600
tttctagctt caaaactgta cc 622

<210> 7
<211> 621
<212> DNA
<213> Homo sapiens

<220>
<221> misc_feature
<222> (1)...(621)
<223> n = A,T,C or G

<400> 7
ggtacccttg tctttaaaag gattccccct tataaggact cttcaagtaa atccacacat 60
atatagtcaa ctaatttttg acaaagacac caagaataca caatggggaa aggatagtgt 120
cttcaataaa cagtattgga aatactggat atccacatgc aaaagaatga aattggatga 180
aatatggtga aattatttta caccgtaccg gctccccaac gtgcacggca ggagctacgg 240
cccagcgccg ggcgctggcc acgtgcagaa atggagtttc atcatgttgt cctctcgaac 300
tcctgacctc aagtgatcca cccgnctcgc ccttccaaag tgctgagatt acaggaagag 360
tctaacctgc tctgcaagct cttgagtccc gccaagatga tatttaaaac gtctgtatga 420
agttgaaagc tgcagntgat ggcctnttca agatgattca aaccncngat gcnnacttgg 480
atgtaancca ccntaattca agccggtnan nccncnnant taacccnaag ggcctggatt 540
tgaattcagg cnttggnaag gttnccgggc ccttaaaana nattggggtt aacgcaaacc 600
ggcttccntt ccttttcttg n 621

<210> 8
<211> 649
<212> DNA
<213> Homo sapiens

<400> 8
actgatctcc tgttggcctg cttcatttgt cctgcagttg tcaatccaga acaatatgga 60
ataatttccg atgctcctat taatgaagta gcacgattta atctgatgca ggtaggccgc 120
cttttgcagc agttagcaat gactggctct gaagagggag atccccgaac aaagagcagc 180
cttggaaagt ttgacaaaag ctgtgttgcc gctttccttg atgttgtgat tgggggccgt 240
gcagtggaga cccctccatt gtcttccgtc aatcttctgg aaggattgag cagaactgtg 300
gtttatataa cctacagtca ggcttattac tctggtgaat tttatgaaag agtgtgatgt 360
ctggagatca actgagagaa gatagaatgg ctcttgacaa tttattggca aacctacccc 420
cggccaagcc aggaaaaagt agcagtttag aaatgactcc ctacaataca cctcagctat 480
ctccagcaac cactccagca aataaaaaga atcgattacc tatagcaact cggagcagaa 540
gccgcaccaa tatgctaatg gacctacata tggaccatga aggatcatct caagaaacca 600
tccaggaggt gcaaccagaa gaggtgttgg tcatttcctt aggtacctc 649

<210> 9
<211> 645
<212> DNA
<213> Homo sapiens

<400> 9
acttagtgca acatattgaa cttaaattcc agttttcctg gaattacttg tgtcttgagc 60
taaaggctgt atttgatata acagggaagg aaagaaatta tttttcctat aaaattagtt 120
tagtttaaaa acacatataa ttaaacaaaa taaaaatatt attccatctt ttaaagaaca 180
tttactaatt cacagatatt acccgaagtt tagaaagtca cctaagaaca attgtttaaa 240
aattatttag ggaaaatgaa gcaaaattgt tttcaatctg agattttaac agccagtgca 300
ctcctgttcc tcagctgaaa gtccccttca ttctgaatgt ctgcagtagt attgaattgg 360
ggagcagtta ggttccaggg acatattcac tcctgttttg ttctcccatc aatctcagcc 420
ctttcggtga ctgtttgggc aaagcctccc ttgtggtaga agatgcctca cttctgggga 480
gaagaggctc ctcatcttgc agacaagaag cagcacccac tgtttcttgc tccaaaagcc 540
attaacatta taaactggcc agttgcagtg gctcaaactt gtaatcccag caccttttgg 600
gaggttgagg cacaaggatt gcttgagccc aggagtttga gtacc 645

<210> 10
<211> 564
<212> DNA
<213> Homo sapiens

<400> 10
cgcggccgag gtacctgggc ttaacagtaa tagagaacct catttatacc atacagacac 60
agcaacttag gaagacagca ctgatagcat ttagctagtt gtaaccaaat ccaaatatgt 120
aaaattgaga attatgatta acatatgcaa ctttagtaat aggaatagat gataattttc 180
ctgtattgtt tcaaataagt gactgttcag ctgggatcca ttggattata atttacaatg 240
tcacataata ttatgctttt caatattgat gagtgatgta aacaatataa agttggcagt 300
ttgtagtagt tcagtatcct agaaatacat tgaacttcat aagtatcagt tcatttttaa 360
gcatacagaa ttgaactgat acttactgaa atcataaact cagaggaaac aagcccatct 420
ttatcactaa ttacttagct tgaatacttt tctattttaa aataatccta attattgcct 480
tttcaattat agtctactgt atttatttat atgggatcaa caggtattta tcaaacatct 540
actgtgtgcc cagcactacc tagt 564

<210> 11
<211> 593
<212> DNA
<213> Homo sapiens

<220>
<221> misc_feature
<222> (1)...(593)
<223> n = A,T,C or G

<400> 11
cgaggtgcct cgcctcgggc attttcttgc agcaagaagg gacgcatgcc tctggcataa 60
atccaaccag agagtcaccc ctctcaagct gattttttaa aaatctagat attatttaga 120
tcatttcagc aaattcttaa tgctttggcc tttcacagta agatgttgct taatcggctg 180
gatctccccc ctccttgcca aggagactca attttgcagt tgcccatatc tgcctagtta 240
aatcgttgct atactaaagg ttctgggagg gtggggacag aatttccccg gtgctaatgc 300
ggcactgaat cgcaggaggc tgccatgcat ttcttcagtc atctacaacc aagaattctc 360
agagcagtcc ctcggcagcc ttttgaagct gtgctagagc agaaagctgc tattgntctc 420
atctctcaac aaggaaagga tcaaactttg cctctttcaa tttgaaagat ttttttttat 480
ggtggtgggg ggaagggatt gcaatcttga tnctcaagtt aactttgagg atttggagtg 540
gtctnccagt ttaaactgca gatcaaatca cagaagccct aacgcctgca tnt 593

<210> 12
<211> 602
<212> DNA
<213> Homo sapiens

<220>
<221> misc_feature
<222> (1)...(602)
<223> n = A,T,C or G

<400> 12
acacacaatt ccactctacc acccaacatc aatgagcatt tattgagcat ctactgaagc 60
tcacagcatt gtgcaggcag gatacatatc atacaaatgc tgtttcctcc tcccaccaaa 120
tgagggagaa ttagatgaga tttttaaaaa ttcctcctag ttctacaacc agtattgtat 180
actgatccaa tttggaagtt taagtttaaa attaattcaa ggattccagt tgaggaaatg 240
gtcccacttc cttggaaagt aaactagctc ggtcaccagg ctaggttacc cacgttgtaa 300
ttgcttgtga ttgactactc caccgtatta atgatgaagt gcccccgact tgagatgcag 360
gcgttagggc atctgtgatt tgatctgcag tttaaactgg gagaccactc caaatcctca 420
aagttaactt tgagtatcag attgcaatcc ttcccccacc accataaaaa aaaatctttc 480
aaattgaaga ggcaaaagtt ggatcctttc cttgttgaga gatgagacca ttgccgcttt 540
ttgntntagc caggtttcaa anggttgcca nggactgntn tganaatctn ggtgganaaa 600
an 602

<210> 13
<211> 487
<212> DNA
<213> Homo sapiens

<400> 13
gcgtggcgcg gccgaggtac tggaggccat ccagcccata ccctggcggg gggcaaacct 60
cagatgcctc cttcttgggt ttcattgggc accaggatcc atcttccatg aattggatct 120

catcacaatc tgaacaggaa ctaagaatct ccataaataa accatcaatg ataagagatt 180
catagggagc cttcttgtca cacacaggac atgtccatgt aggcttcttc tcattcatct 240
gtagataaag ggcagcatcg aagctctgca ggtgggcgca ggtgagggca cgacaaggga 300
cagtcaggcg catcttccct agcgggcaca tgagtgacac ccggagactt gtagtggcca 360
cctcactgtc agggtcagca gtcaatttct ccttgatcag tgcccgcgag tggtctgggt 420
tccggatacc ctttgctctg agtttttgta gaagggttcc tgcagtcaac tgcctcacca 480
ggtacct 487

<210> 14
<211> 300
<212> DNA
<213> Homo sapiens

<400> 14
acagaaattc ttaactgctt atgaaatgct gattgttaaa cagcatccac agctattttg 60
tgttgtttcc ctgaccccac cctgaagaaa agaaaaatta tggcatattg aaaacagcag 120
tatgatgtaa gagaaaagat cacaaattcc ttgagggtgg gtcttttcca tactcataag 180
cctatttata atattcagag taatttattg acacatatta atattccctc ctatcccatt 240
aattgccaaa tcatcaaaca tttattgagc acctactctg tgtagggtgt aagcagtacc 300

<210> 15
<211> 882
<212> DNA
<213> Homo sapiens

<400> 15
acctcataac aaatgcctgc catgtgttcc agattcacct tctttctttc tgccccagcc 60
ctggaatcag ctgcttctcc aagcactcag gactcctctt aacagagaat gataaatact 120
tagaaacccc tgaggcccgg tgtgctcagt gttctaggct gtcctccttc taagcccttc 180
tcgtggccag aaccacacaa agtatcatca cgacagcttt atagtaagtg ctggtgtttg 240
cagggcaaat ggccctcttc ttcacaagtg ttttaattaa tcctggactt gcactcttct 300
cagtgaattc tagtcacctt gtcaggaaag agaagtggct ggatgtcgat gggaacgtca 360
ttgaatgtta agagcaactt tgggagacct gacacctggc atcttccttt ctctgaacat 420
agaggagaat taagcaaatc ttccttaaat gtccttcaat aaagtttata tattttctgc 480
atgcagatct tatctgtctt aaaatttacc ccagatacct ttttgctact gtaagcatta 540
tgttttaaat tacattttgt aaccaattaa attgttggtt taacaaaatg aattgatttt 600
atattttgat cttaaatttg ctcaactctc taatctgttc tgagatccct atttaggaaa 660
ttacatcaca tcacatgcca gtaacagcag ttttatttct gcctttttca ccctctgccc 720
tgctgaaaac agtgttgtga ggctgaggat gatgtgggtt acacaaaact tggctgcact 780
gcagggggga atggaaatct acataaccac cttggaaaaa tcgatatgta tcaatatgca 840
gacgtctgcg ttatcctgca gaactggaca tttgcacgta cc 882

<210> 16
<211> 568
<212> DNA
<213> Homo sapiens

<220>
<221> misc_feature
<222> (1)...(568)
<223> n = A,T,C or G

<400> 16
   ggtactcccg gctttacagt taaaaccagt tttctgggaa catttgtcaa acacagggaa 60
   aggctgtcct tttaagttag tgtttactgc atttcaccta agactaaatg gacaaatgaa 120
   ttataaattc atttttaggg aggcataata aactttggaa atattttttc ttaattagag 180
   ggaagaaatg agcaaaagag aacccgaggc tctagctaga agcccgtgtt tctctgccct 240
   aattgcatca aacaatgcct taataatctg tgtcttcatg tgggaggcat ctactctgtc 300
   ctctactttt tcacttttat gcaaactcag gggaaactca ggggaaaaaa tgattctatg 360
   aaattataat tagagccata tttctagatt ttaattttca acattggcat ttattaattt 420
   cctgcagctg ctgtaacaag ttaccacaaa ctggtaaaaa tggcttaaaa gaacngaaat 480
   ttattttnct acaggtcaag gccggaaatn ccaaatctaa gcatcanggg ggtggggtcc 540
   ctttggangn tcccanggna ntttttcc 568

<210> 17
<211> 584
<212> DNA
<213> Homo sapiens

<220>
<221> misc_feature
<222> (1)...(584)
<223> n = A,T,C or G

<400> 17
acaactgaag accctagaaa taagggtttc aaccctggtt gcccattaga atcatgaaag 60
agcccccgag atttgggttg aattggtctg cagagactcc aggccccttc ttttgaagct 120
ccacagatga ttcttttctg cctgagggga ggtgctgagt tcccatcacc caccagcttc 180
atcctacaca ngtgcaatna gaggcctagt gagagtggca ctggggggtg gccccccagc 240
gagtgccaag tagatcccac caggcccttn ctttaggcca gaggttctag aaactttgat 300
gaatgtngca ataaccaggg ggtgctctga aaaggnccta nggctgggct gcacctgnta 360
aaatnaagcc cagtctttct ggttgggacc agaagattcc naagggcagc ncgctcttta 420
aaaaccaagt gcctttctgn taaacnaatc cttaggnccn ttatgtctgc agttnttaag 480
ntaangggtt ggtaagntan taacntccat taantttnag tntacactta agcttttggg 540
ggtatcngnt tnnagtgnna ttangnagtc tttcacaggt ngtt 584

<210> 18
<211> 560
<212> DNA
<213> Homo sapiens

<220>
<221> misc_feature
<222> (1)...(560)
<223> n = A,T,C or G

<400> 18
ggtactcaaa gcttggactc catccctgaa ggtcttcctg attgatagcc tggccttaat 60
accctacaga aagcctgtcc attggctgtt tcttcctcag tcagttcctg gaagacctta 120
ccccatgacc ccagcttcag atgtggtctt tggaaacaga ggtcgaagga aagtaaggag 180
ctgagagctc acattcatag gtgccgccag ccttcgtgca tcttcttgca tcatctctaa 240
ggagctcctc taattacacc atgcccgtca ccccatgagg gatcagagaa gggatgagtc 300
ttctaaactc tatattcgct gtgagtccag gttgtaaggg ggagcactgt ggatgcatcc 360
tattgcactc cagctgatga caccaaagct taggtgtttg ctgaaagttc ttgatgntgn 420
gacttaccac ccctgcctna caactgcaga cataagggga ctatggattg cttaacagga 480
aaggcactng ntctcaangg cggntgcccn ttgggaaact tntgggccca ccccaaagaa 540

tgtggntttn agtttttcnn 560

<210> 19
<211> 425
<212> DNA
<213> Homo sapiens

<400> 19
ggtacaaaga gaaaaggtca agacattttt caaatgaggg aaaactaaca ggatttatca 60
ctagtaaacc tgctctaaaa gaattcaagg gaagcttttt aaaaagaagg gaagttatag 120
cagaaggaaa cttagaatgg caggaataaa gaaggcataa tgtatagggt aaatataata 180
gacttctctt gaggttttaa aaattacatt tgttatttga aagaaaaaaa ttaacgttgt 240
tgtatgtgat tctctgtaga ggatatacag ttttttttgt tgttcttgtt tctgtttttt 300
taaggtgaag tctctgtcac ccaagctgga gtgcagttct gtgatcatgg ctcactgcag 360
cttcaccctg ggttcaggtg atcctcccac ttcagcctct tcagtaactg ggactacagg 420
catgt 425

<210> 20
<211> 655
<212> DNA
<213> Homo sapiens

<400> 20
tgttacttcc caagcactgt agggcgtaag gaaaatctgg tccttatcaa atcccaggag 60
cttctgctta gttggggaag aaattacatg aagcaaccag aggttataag gccacacttg 120
tatatcgtgc accctgtgtg gacaagatta gggactgttg agagaggagg aaaccagtag 180
agagcaaagc tctacccagg ctccttgtaa gcctctgggc tcccccgaga gggcctcgct 240
actctacgct tccctagcaa cgttgatgtc cccacaaccc cacatcagtg cagctgtggc 300
ttgtgtggag gggctctgag gcctctgagg ccagatgtgt aaacagtgct gaggttcagt 360
aataggatga agtcttcagg tgtggagcag cccaccttgg ctcttcccat gtctctgtgt 420
tacttctcat attctgctgt cctttcaaac ttcaaggaca gtattaattt atactagtat 480
ttcttcctca gttttgtgac ttgaatgcag tgagtgcctt agaggatcca aggatgaagg 540
aatgcgggtt ggtggttctc tctttcagaa tgggaacttc ccaaaaatgg ggctgcgtct 600
cgcctctcag taggttccct acctctgggt cttccaccct tcaaaatctg gtacc 655

<210> 21
<211> 566
<212> DNA
<213> Homo sapiens

<400> 21
ggtacagccc tttctttgaa tggggatctg gggatgcaga ggagcataat gagcctttta 60
taattacaaa catgctcttc tctagctctt aaggttatgc ctaacgctca tttgctcttg 120
gctaaaataa ctgagaaaaa aagtgagtag taaaaaaatg ctggaagtct gaaaatggtt 180
tagacagaac ttcattcctg aagttttagt ctgtagccag attttaattc tggcctgttt 240
tggtttttag atgatagatc ttttagtgtg tcaacaggaa tgtaaagttt gtattaacat 300
ctagggtgat cacctgccat gctattaagt cagcatggta taattaaaag ttacatatgt 360
aggttcagag cctcttagca cagtgttaca ttgtaagctc ttggagggca ggaatgagat 420
tctagtcctt acggaaatgg agtttgggct tctatcccta gcattcattc tagtgccatg 480
cacgtggtag gaattctgta aatatttgtg aaagaaatga atttctgcct gtagggttca 540
gcagtgtata cttaaatgtg atgtgt 566

<210> 22

<211> 269
<212> DNA
<213> Homo sapiens

<400> 22
ggtactaata gcaaggaata atcctaaaca ttttcccaat aaactgacta agcctcaaaa 60
ggacagctta ggaaaatgat taacatgcag tttttctttt ttcctagcca attcagttct 120
acttagataa atctggttgc caatcaatac atatataaat taattttttt ctgctcaatt 180
actaccattt tttctttttc accttttccc caattttctc tagcaacact tttcctttgg 240
tttgatcagt tgaactcaaa aggtttggt 269

<210> 23
<211> 815
<212> DNA
<213> Homo sapiens

<400> 23
gaggtaccct tcatccatca ggactgcacc tcctttccca tgagccttct ggggtcacat 60
tctcctaact gcagctactg ttgctgtttt acttatcgag ggcctattac gtgccaggct 120
ctgcgctgaa cgcttcacgc ccactggatc atttactcat aatagctcag taaggtagtt 180
accccaatta gccccatgtt agagaaaaac accaaggcac agaggtgagt cacttgtccc 240
aggtcacaca tctaggaagt agtagaacca ggactcagct caggtccaaa gtctcaacca 300
tgggccagtc tgctcatctt agtcaaaccc ccaggctgca ttctgtggtc cagctactgg 360
atcctgcaac cttctcagac tctatccatg aagccaagtg cacaggatct aggacatcag 420
gtccagaaaa attggggcca cattcttctg gacctgcaga tgggcaagga ccagactcta 480
gcctgaacag tgagatgcag cccagagaag tgggaatcca cagacagagc ctggcctgag 540
actcctactg agactgccca tgtggccact cggggagttc ccgtcccctg cctgatcagc 600
agtctttttg cttccccctc caagagagct ggggggcatt cctccaggaa gcctgatatg 660
taacaaactc ctttcccatt tcttgctttg cttaaatctc caaagtccct ggagctgaag 720
ccaagcgggc ctcattaggt ccactttaca gaaaagcaaa ctgagtctca aagaggggaa 780
gtcactgagc cgggtacctg ccgcgggccg ctcga 815

<210> 24
<211> 555
<212> DNA
<213> Homo sapiens

<400> 24
ggtacctggg cttaacagta atagagaacc tcatttatac catacagaca cagcaactta 60
ggaagacagc actgatagca tttagctagt tgtaaccaaa tacaaatatg taaaattgag 120
aattatgatt aacatatgca actttagtaa taggaataga tgataatttt cctgtattgt 180
ttcaaataag tgactgttca gctgggatcc attggattat aatttacaat gtcacataat 240
attatgcttt tcaatattga tgagtgatgt aaacaatata aagttggcag tttgtagtag 300
ttcagtatcc tagaaataca ttgaacttca taagtatcag ttcattttta agcatacaga 360
attgaactga tacttactga aatcataaac tcagaggaaa caagcccatc tttatcacta 420
attacttagc ttgaatactt ttctattttt aaataatcct aattattgcc ttttcaatta 480
tagtctactg gatttattta tatggatcaa acaggtattt atcaaacatc tactgtgtgc 540
ccagcactac ctagt 555

<210> 25
<211> 413
<212> DNA
<213> Homo sapiens

<400> 25
ggtacaagct tttttttttt tttttttttt ttttcctttc attgtccagt ccccatgaat 60
tatttatttg ttattaaatt caactgaatg agatttcaaa gcaacgaaaa ttgaagttca 120
aatgaaacca aattaccact ctgagctcca ggtggccctg acagcccagt tttgtgaagg 180
gcccctgagg ctgttcactg aatctgagat gtcaccaggc atggagggtc tctgatcagc 240
atccagagct ccagagtagg gagcaacccc tcaccaccac ttctggcccc caggcaaggc 300
agagaccaaa agaaccctgg taaggttccc caacctccat gttcatttaa aaaaaatgtt 360
taaaactgac aaataataat tgcatatatt catggggtcc atcatgatgt ttt 413

<210> 26
<211> 638
<212> DNA
<213> Homo sapiens

<400> 26
acttagaatc gtgtgtccat ctgaagccag tgcagaggcc aaagtcagtc aatttaatat 60
gaccatcacg atcaatcaaa atattatcag gtttaatatc tctatgaata aaacccattt 120
taaggaacac ctttcaaact gcacaggtaa gttctgctat gtagaatcgt gccagacttt 180
ctggaaagat gcccattcta attaataggc tcatcatatc acccccagga atgtagtcca 240
ttacaaagta taaattgtcc ttatcttgga atgaataata tagacgaact acccattcat 300
tgtcagcttc agccaggata tctctctcag ccttaacatg agcgacttga tttcgaagaa 360
gaacatcttt atttcgaaga gtttttgttg catacaaagc cttagtatct acttttcttg 420
ctagacagac ttcaccaaat gctcctattc ctagtgtctt tatcttcaca aacatagact 480
tgtccatttt agccctttta agacggatgt aattagattc tttttggcaa agcatctttc 540
tcatttgatc ctgggcatct tgagataatc caacccgcat catttcattc tctaattgtt 600
ttttacgatg tagacgctgc tgatgagatt tgagtacc 638

<210> 27
<211> 236
<212> DNA
<213> Homo sapiens

<400> 27
ggtacacgtc gttctcttca agatctcata gacaatcgtg ctccgggttt tgctgtcgaa 60
aaaggaatcc ttatcagaca agtcaaatag atgctgcttc tcccgggaga agggatagga 120
gagtctcttc atggtctggg gcctgtgctc agccactttg ggctggatgg gatctgtgat 180
tttctggagc acagagttga tttttttcag gaggccacgg gtctcattaa tgtggt 236

<210> 28
<211> 607
<212> DNA
<213> Homo sapiens

<400> 28
ggtaccacgg gaaagatcag gactttggct gcaccctttt ccagctcctc catgttacag 60
atcatatggg cacaagtggt aaaaatctcc acggctcggg aacgggttcg aataccatac 120
acctcagcca tggtgaagat cttatacatc tctgggagaa tgacaggagc aacaaagtgg 180
catctgtgtg tctgttactt tcacgagtga attctgtcag cacacgcatg gctccatgga 240
cggcatttaa gtctccgctc accaacatct ccatgagcag gttgaagagt tggggccaag 300
cttcaggcca gtcccagtgg gcaatggctg acactgcata ggccacactg gagcgcactt 360
tgcttatcga ttctctcaac ccattaggca atagctcccg gataacaatt tttgcccttt 420
ctgtagtttc aggaggccta aatttctctg attgggcaca ccagtgagtc tccacatatt 480

gtttcaagat gactgatgcc agctgacgga ttgccagtgc cccctgggga tctacagtca 540
gttctgccaa gtgaacacca aattcctccg tcacctccag caccttaatc tgttcttcag 600
cagccgc 607

<210> 29
<211> 612
<212> DNA
<213> Homo sapiens

<220>
<221> misc_feature
<222> (1)...(612)
<223> n = A,T,C or G

<400> 29
ggtactaact cgctttacct ttctgatatt cgtcctaaga ttttacttcc tattatatag 60
tgtttgcagt ataccagggt gaaggacctg tcacttctta atgaatggcc ttggtcaagg 120
gtttttaaag tttcaggtca gaaatgtgga tgtgaaaaaa tgttttttaa gaccttcaca 180
ggcttactag tatcacagca ataaatgatt ctaccaggat attcttcgta gacttagttg 240
gcctggaggt agacttttaa ggatatatct gtgcttctga ataaaattag ctaagaattc 300
aacattatgg aattcaataa attccagggg gaaatcagtg aattaggata cactgcctct 360
taaattctaa accctatata tcccacctgt tgcatgtang gggcatgtgt gcatgtggca 420
tcaaaactag ctgnggaccc ttttttttcc ataaaatttg gncntactca tccttgggng 480
aaaaancctt gaaggnaaaa tctggggtna aaaaaaagct ttgggctgtg gaccaacctt 540
ccangttccc ngggaaggga ttnggaccta gnaaaaannc cntggaantg gcttgggcct 600
tggattactg cn 612

<210> 30
<211> 286
<212> DNA
<213> Homo sapiens

<400> 30
ggtactgtta tcatagcagc actatccaac atgaaagtaa tcttataatt tgcatttgtg 60
cccactccca gctctttcat tttagcttca atccacttca tatttgttgc agaccaaata 120
acaatgtcat aatcttcata ggcagatgtt agaaattcat gaagatatgg ccgcattaat 180
tctaccccag tctctgcaca agacctgtgg tcaaataatg tataatcaac atctagcacc 240
aaaagctttt tcccttccct gggaggattc aaaatttcca ctttgc 286

<210> 31
<211> 606
<212> DNA
<213> Homo sapiens

<220>
<221> misc_feature
<222> (1)...(606)
<223> n = A,T,C or G

<400> 31
accttatttt gctgagctta ttatataata ccagagcaga atagaaggta gacccacggg 60
aattcaaatc ttggctgtgc cacccacttc ctgggcaagt cacttcctct ctctgtgtcc 120
atttccaaat ctttgaaatt cagttagaaa catcacttta aaaacagggt tgttgtgaag 180

attttatgag ataatgtata aaataagttc ttaccaagta tcagctatga tatttatgat 240
attttagagt tattaattat actgtgagga ttaaggaact tggcagagga atacagtagg 300
tgcttaaatg gtatcctaaa atattattta aaaataaatg acagtaatgg gaataccgca 360
attacttttg caccaacgta ataatagtag gatatttaaa gttgagatca caggaatcag 420
tgcagatatg tctcatttta cccacaggtg gcgctcatgg ccgggttaaa ttctgaaaaa 480
ccttaaaaag tcccttgggc gngaaccnnc ttanggcgaa ttcccgnnca ctngngggcc 540
gtctaangga nnccnatttg ggccaacntt ggggaaccng ggcanaccgn tcccggggna 600
aatggn 606

<210> 32
<211> 615
<212> DNA
<213> Homo sapiens

<220>
<221> misc_feature
<222> (1)...(615)
<223> n = A,T,C or G

<400> 32
ggtactcatg catcttcatg agcagctctc ttatcttctc agtaacatag tcacctcctc 60
actggaaagg tctgtatttt atactctttt gggttaagtc actggcagac agaaacatca 120
atatcctaat tcaggatgga tgccacagtc tgcccagtta gctcattaat tagataattc 180
tttaaaaata ttgacaaacc attaattaag agctgattat tcacacatca aacaattctt 240
cacttaaact agaggatttc tttaaatagc agctccccct ggctgcattt atctctttgt 300
gtaagtttat tagctatttg gcagagaaat ttcagaatgc cagctacaag tcagtgcagt 360
tgaagaacag aatgtaatgg agggaaagta tttctggaag catggcattt attccaagaa 420
attatctaag aatgnaattc ctttggaaag tgcttaatat aattatatat gnaatcncaa 480
ttaatttctt aaataantct ngggaatggn ccagattttc tggtttggaa aagcccgggt 540
ntttngaatc caaataantt gnccaggctt tttnnntnng nccnnggtng accngggttn 600
gattcaangt ttcnn 615

<210> 33
<211> 297
<212> DNA
<213> Homo sapiens

<400> 33
acagacttcc atctccccaa catcttgaag atgtatcaat ttttttaaat taagaattac 60
tttaaacagc actcatttca gaagataggc agaggttatc aaacttctgc tccaatcttc 120
tcattattcc aaggttcata aaaaccactt aggaagacct tggttactgt gacacatcac 180
agctataagt gtaggtggcc tagactctcc ctatctctta gctgccctga gtcatgtgaa 240
ataagatagt gaccttctcc atcatcccta gaggctctct ccccgagaga gagtacc 297

<210> 34
<211> 468
<212> DNA
<213> Homo sapiens

<400> 34
actgtttagt gggatccatt ttatacaggt gacggtcagt gacaaaaatt gctctgtctt 60
ccaccttact aaatcgattt accttacgga cgtgacagga aaagaggaca ttcatgtatt 120
tgtccttccg tttcaattca ttagcaacag ggacaaaagt gcctgaggtc tgaggtgtat 180

ctggctttga agcaagatag ttgccctccc aggccctctg gagcccgagg tcagcccttt 240
gacccttcaa catttccacg gctgcaacct ttgccctgac ctggggcagg tctgaggccg 300
gaatgctctt gatgagctgg gatgctctcc atctattgaa aatcgtctgc agggcctcct 360
caaaacggcg aagaacttta ggagggcttg gccacttcac gtgcttcccg tagtctcgca 420
tggtcttgac gccatggaaa cgtctggcca cctcgtggat gtacctcg 468

<210> 35
<211> 314
<212> DNA
<213> Homo sapiens

<400> 35
ggtacttatg gctccagata aaatctctgg tggccacatt attcaagact ttttaaagtg 60
ctttatctga aatatcttca tagacatgaa tatgaaagtt ctgaaaattg tgttcaatgg 120
cccgtgtgtc ccagaagatc ctaatgtaaa gatgcatatt tataaagtaa tttatagaat 180
aggattaaac atatgtagaa ctttattaag aaaatataat gactttggga ccaattacag 240
gcccttgaac agccacaata ggctcaggag ggctgtgctt ctgtgtaaag tcccctccca 300
gacaccacca gggt 314

<210> 36
<211> 600
<212> DNA
<213> Homo sapiens

<220>
<221> misc_feature
<222> (1)...(600)
<223> n = A,T,C or G

<400> 36
acccaatgtc atgggaatga tgtgcctgtc acccccattg gacaagctgg ggaacagcca 60
tagggggacc agcttctgcc agaagttggt gtctctcttc aatttccaca actatgacaa 120
cctgaggcac tgtgctcgga agttagaccc acggcgtgaa ggggcagaaa ttcggaacaa 180
gactgtggtc aacctgttat ttgctgccta tagtggcgat gtctcagctc ttcgaaggtt 240
tgccttgtca gccatggata tggaacagaa agactatgac tcgcgcacag ctctgcatgt 300
tgctgcagct gaaggacaca tcgaagttgt taaattcctg atcgaggctt gcaaagtgaa 360
tccttttgcc aaggacaggt ggggcaacat tcccctggat gatgctgtgc agttcaacca 420
tctggaggtg gtcaaactgc tttcaggatt accaggaatt tctacacaac cttttgaaac 480
tcaggcttga gggcacaann tgaaggccct nttcnaaang aaacttttaa aaagccttng 540
gttttaaccc ncgggtcant gnnnaatccc tggtttaana aaaaancctn gacttggccg 600

<210> 37
<211> 516
<212> DNA
<213> Homo sapiens

<400> 37
ggtactgctg taggaaagaa attaaggaca gttagtatgg gcctgtgaat tctggcatac 60
atgtttaaat caattacaat tatgcaagta aaaaaaggat atcccctact aattcatgca 120
ggctgaaaag tctagtatgt aaacctgcag cagaatctaa ttttaagaaa caggcaccta 180
attttgattg tgaaactcac tcacctgagg aaagcttcca tcaggctcac tatgcccctt 240
gtgctgactt gcacactaaa attagcaaaa cagactccaa ctattaaaaa tatcaaactc 300
ttcgtataca tacttttgtt ttaactttaa gtatgcttag agcaaagtag gtgcctttac 360

taagctatat ttagagcact atggggggag ctctagtgtg agaaacagtt tctcaagggt 420
aacaatccta aaaatctagg atttggaatg aaaactttca ataatttgaa agtattttga 480
gcagaaaaat acatttgatc caagtataga aagcgt 516

<210> 38
<211> 319
<212> DNA
<213> Homo sapiens

<400> 38
actgaaagga tgaaaaggtg gtgtcatgtt ttggggagaa tcttacttct caaatggaaa 60
ttgcactttt tgctgaatcc tttgcatttt tttggtagta agcagttcat tgagtatcag 120
gtcctcaaag gaatgagttg gcccggctag ggtgggccct cttgacctaa cttcagaggg 180
ggccttggct cagtaggtgt gaatcaggga agccacattg tcctcagggt gctgtatgaa 240
gctgggtgtg ggcggattcc tcccacacct tcacactggc ctgcctccaa ctcatacaga 300
tctcggagcg gtcggtacc 319

<210> 39
<211> 592
<212> DNA
<213> Homo sapiens

<220>
<221> misc_feature
<222> (1)...(592)
<223> n = A,T,C or G

<400> 39
acctacactt ggaataagac actgttctga atttgtgtca tagttttttt ttcatattga 60
cattaataga ggcttctatt ggggttaggc taaaaatctt ttgtaaaaaa ttttaaatga 120
cactgctgat ttttctccgt taattatcag tttataagct aataaaaact ttggcttgat 180
attacattct agtggttaaa tttgtcatag aaggaatatg tgctgagtta cttatgtatt 240
gtaatcttga gattacgatt ttttatttga aaattagaca aagtttgttt ttaattttta 300
tttcatttta ataattgagt tcagattaaa tgggaaggct aaatttgaat tccgtttttc 360
tctcaaaata ctgnttttct attattttaa ggcattcctt ggaggtctaa aattgggcat 420
ttataggtgt tgatgaaagc acacccgatt taaagaatgg atgacccccc ttctgnatna 480
aacctttaat ngaattttaa annccaaact ttgggtcctt taaacctngg acctcctttc 540
ccnnaatccc cttaaaaaaa ncntnggcnt tngcanaatt cnntttgccc aa 592

<210> 40
<211> 577
<212> DNA
<213> Homo sapiens

<220>
<221> misc_feature
<222> (1)...(577)
<223> n = A,T,C or G

<400> 40
ggtacagaac ctaaaggttt cactgaatgc gaaatgacga aatctagccc tttgaaaata 60
acattgtttt tagaagagga caaatcctta aaagtaacat cagacccaaa ggttgagcag 120
aaaattgaag tgatacgtga aattgagatg agtgtggatg atgatatcaa tagttcgaaa 180

gtaattaatg acctcttcag tgatgtccta gaggaaggtg aactagatat ggagaagagc 240
caagaggaga tggatcaagc attagcagaa agcagcgaag aacaggaaga tgcactgaat 300
atctcctcaa tgtctttact tgcaccattg gcacaaacag ttggtgtggt aagtccagag 360
agtttagtgn ccacacctag actggaattg aaagacccag cagaagtgat gaaagtccaa 420
accnggaaaa ttccaagaac tcgngtcctn gactggatct tgggganaac ccttggttnt 480
taaaannggg acntttttnc cggcttgggg cccntttaga tttcaaagtt tcangaaccc 540
aaacggtcct tnattaaanc cggngattgt tcgaagg 577

<210> 41
<211> 490
<212> DNA
<213> Homo sapiens

<400> 41
ggtacacaag agtataggta tataaaacta aatgaagtca atcatattga ttatcccccc 60
aaaaaaaata taatctaaag aataatcagt tcctaaataa ttgaaagctg cccttacaaa 120
ataaaacaaa agaacacaca tttcgttgtg ttgcccaggc tggtctcgaa ctcctgggct 180
caagcagtcc tcccacctcg acctcccaag atgctgggat ttcgggacat gagccaccac 240
gcccgggcca aagctgcctt tttttaacat ggattttttt tcccccattc gttgtgctca 300
gaagtcattt cctcttattt ttctctgcta atgtgtgctt taacaaacct gtttaaaacg 360
acaagccttt aatcaactgg ggtgttttgt tttgtttttt tcttattttc ttaggagtca 420
gtggatcggt ggggaaaatg ctgcttaccc tgggccctgg gctgtagaaa gaagacacca 480
aaggcaaagt 490

<210> 42
<211> 571
<212> DNA
<213> Homo sapiens

<220>
<221> misc_feature
<222> (1)...(571)
<223> n = A,T,C or G

<400> 42
ggtacttgcc ttttaacttt cccccacatt actgttgagt catggaataa tgtttaagtt 60
gttatttgca tggaaattaa gtaggctgtt tatttatcta aaggaatcaa gtccactctt 120
ctgcctgcaa catttgttca aaaactaacc aaggtaaaat atttatttga aagcccaact 180
ttgatgttaa atattcttga ataaatctgt tattttaaga atatcacatt attcaatgca 240
tataaaacta tcagaagtta gtaaatcata ccagcactaa aaataagaca attggaatat 300
attttagcat cagtttacaa acaactttat tatcaacaga aattttagct cttttctttg 360
caagatatat cacagctgct ttgggcagta gctgaagccg aagtatgaac agtccatttt 420
gtttcttaaa atttgaagtc gtgtctgtcg tagcattttt actaccagca gtatgttact 480
taaaaaacta catggctttc cttgaattta tttgaccgna ttatgtaata gacttgaaac 540
aattgccatc tttgtagnta tgcctgggtt c 571

<210> 43
<211> 708
<212> DNA
<213> Homo sapiens

<220>
<221> misc_feature

<222> (1)...(708)
<223> n = A,T,C or G

<400> 43
aggtactgca aaaatgaagt attattctct aagtattcat tttatccctt tcatttcagc 60
aaaatcacac atttgaataa acaggatcga aatacgacac ttgtctttcc tcttaattta 120
aggaatatat tgtttagatt attgttcata ttagacaact gcctcaaaaa tgttttaatg 180
ccatccaata aataaacttt tgatagatta tgactttttt taattttaag ttgttaagaa 240
tattaacttt gagtctccta ttaatattct aaaagctagg attcaattca gcagtttcct 300
ataacatttt agaacccaag gcataactac aaagatggca attgtttcaa gtctattaca 360
taatacccgt caaataaatt caaggaaaag cccatgtagt ttttaagtaa ccatacctgc 420
tggtaagtaa aaaatgctta cgaccggacc acgactttca aaatttttaa ggaaaaccaa 480
aaatnggacc tnggtnccat taccttttgg gnntttcaag cntaccttgg gccccaaaag 540
ccaagcttgg nggaatataa tccttggcca aaggnaaaaa ggaagcctta aaaantttcc 600
ngggngggaa naantnaaaa gttnggtttg gnaaaaaccn ggangcctaa aaaattttta 660
tttncccaaa ttggggccct naaatttttn aaagggcnng ggganang 708

<210> 44
<211> 632
<212> DNA
<213> Homo sapiens

<220>
<221> misc_feature
<222> (1)...(632)
<223> n = A,T,C or G

<400> 44
ggtactaggt ctattaaatc tacctgctta aaaaggtttt gaactgaaga ttccaggagc 60
tgagcagctg cctcttcaaa ggttttgaga gtaacaaatt ggacctggta gtttttgcta 120
acagggtgga ggccgttgat catgccctca gtggtgatga tggccaggta tgcaccgcag 180
gggctcactg ctatcccgtg agtccttact gagccaaaca catctgagag tttaatcaac 240
tggtgttcaa acttcaatgc aacatctgtg aaaatgggaa tcagctgcct cacctttccg 300
tcactggagc aagtatagac tgttccattc tgtttgtctg cagtcatgga gacaattggc 360
agtgagttga aggcctgtga catgggaatt gtgaaccatt nagccctgct ttggagatca 420
gaagangaca ccaaaattca taagancctc ttgcagccca cttactaaag ctgcnactac 480
actttttggt aagggatgaa taaangtggc ccacatttng atactgngca cnagntaact 540
tgggnccatt tcttttccnc aagannacca gggttgnctt aaagnggaaa tannctttna 600
cngntttnaa aattncccng gaaaattttt tt 632

<210> 45
<211> 664
<212> DNA
<213> Homo sapiens

<400> 45
ggtacccggt ctacagtaga gaggttttat gaaaataaaa tacaagacca aattcaaaga 60
gctttaaaaa ccacagagcc agacaaatgt gagaggttat tatgagcaaa caatgacatt 120
acagaagtga aagtgctcaa gtgccatcaa gaacaagggc tctatttcac tcccatgtgt 180
caccataata aagacagagt ccctgatctt aaaggcatca atttgccccc actggaagcc 240
ttaattgtaa ttcattaata cagcagcatc ctaaaagtta ctgccgtttc taggaatcca 300
aacaactggt tttaggtcct aaagaatttg aatcattaag aaatttaaag tacccactct 360
gggccagttg atggctgcga agagagcaga aggggtgctg ctgtaggaaa tcaatggctc 420

ggaagaccac actgaggaag gtgtgagttg atactggaag atctccaggt ttgaggcatc 480
ttcagaggta tatggtggtt ttgtgtgtgt tgagggtgtg gtagcgcagc agctccctag 540
ggaattagaa ggttttattg aacatttacc ctgtgacagg cactgcaggc attcagcgcg 600
cagtgtcatc ttcattttac aggtgaggaa aagactcagg ttcaagtaga tggtcaaggc 660
cagt 664

<210> 46
<211> 633
<212> DNA
<213> Homo sapiens

<220>
<221> misc_feature
<222> (1)...(633)
<223> n = A,T,C or G

<400> 46
ggtacgtgtt tatgggatgg gcacactaga tgagatggaa gaagatgtgc cagtgatgtg 60
gagacaggga gtgtgggaga ggagcaggta gagctcagag acggtgcact taggcctgtg 120
gtcattgggg gtgacccaag tagccagcag ctgcccagcg ttttgtgttt ctctcctggg 180
tccctaggag tggaatttgt gtaagaacaa tgtgtgaggt tgtggcctgc ggggcagtta 240
gcagttgtca gaccggtgcc tggaagtgtt tcttggatca ggaaatcagg actgaaaggg 300
gcattaagtt tgtctggacc accctgtcat tgtgcaatgg ggagatcgag gccttttggg 360
aggaaaggcc ctgcttaagg gccgtataat tgaagtcagt ggctgtgttg gggcctttga 420
acctgccaaa agctggtgcc tttctccact cctcagtgct tatgccccaa gtgagggtct 480
agncca[?][?]ct ctcccacttt cctcccactt tcactaagca cctgctctgg taggcccagt 540
gctgtatgct gtgaactcag gctggttagg tgctaattta ttcacccagc cagacattct 600
agtgtctcct gcatggcagg cactgttcga agt 633

<210> 47
<211> 433
<212> DNA
<213> Homo sapiens

<400> 47
accagttgct cctccatgat ggtctgggat cacagaggct ccaagtgggg acttcactac 60
ctagaccagt cccccacatg gtccctccct gggctgcatc tttgcctgtc ttagtctcct 120
gtgttccttg agaaagtgga gtcaataaca cctttctctt caggttgtgg gagaacggct 180
cccagccacc ttctgttttc ccttctcttt gagctctaga ttcagggagg ggttaaggca 240
agaccaggtc ccagaagctt ggctgagacc agaagccagt gcttactgtg ctactgccac 300
cttcagcagc aagggcccca ccaatcaggt ccctagattc aggccccagg tggagctgcc 360
ctcccgattc tagggagcct ctctacctga aaggtgcaca gaaaaacact gcagaaaact 420
cacccagcaa ggg 433

<210> 48
<211> 633
<212> DNA
<213> Homo sapiens

<220>
<221> misc_feature
<222> (1)...(633)
<223> n = A,T,C or G

<400> 48
acttcttcag gtaacactgt aaggatctcc agcaaaaaag gcaaagaagt cacatcattg 60
ctgtattttt ccaccagtgt ttgcacacat cccttccagg aaggcatctg tagggcaaga 120
tctgctattg ctaaagccag ctgcgttaca ataacaggtg acaagtcttt caagttctgg 180
atatgggtta gcaatgagtc ccgtaaagag gcatgagagt ctgtggggag ctcataaaat 240
gaggtctgaa tcttcatttt catggtctgt gcagcaaaat agcatgactc cacatcctgc 300
cggatctgta acaactggtc tgagatctcc catgcatgaa ccgaacgctg cagcttccca 360
agcnaaaaag aggngccgct cctttcccgc tgggatctgg ggtccgtggt aaanccgcct 420
gcactggctt ggtaccacca ataaaggnca atttncgaaa aaaaaanaaa aaaaaaaacc 480
ttggccggga ccacncttan ggcgaaatca acacactgcg gccgtctang gatccactng 540
naccaacttg gcgtancatg gcnnactggt tcctggggna attgtanccg ttcaaattcc 600
ccaattacaa cccganncta aannaaactn ggg 633

<210> 49
<211> 624
<212> DNA
<213> Homo sapiens

<220>
<221> misc_feature
<222> (1)...(624)
<223> n = A,T,C or G

<400> 49
ggtacccctc tctcacacat gtcaaatatg aagaggcaga aggagccaat ggcaatgggt 60
ccgacttgct tccaataccc tgcgatgtgg ttccgctcgt gctgatccat catgtgctcg 120
ccacagaaga tgatccagaa ggacagaagc atcgcataga agatgccctg tcggatgtca 180
ccaaacagca gcatccaggt ccagtcaaac ccgatggaaa accattccac tgggatattg 240
ataaaggtca tggaaatccc aagggcaaag atgacttttt tcagaagcac cgggggtcgg 300
gacatcatgg tgatcctcct ccaataccac accataatga tgaagatgct gggccgtaag 360
gaaggtcttc atggcaaacc acaccttggt gaagcctcca ttttggtgga tccccaccaa 420
cccggatatc ctttatctcc caattcccac attgatttct tcttcttatt cacaggcagn 480
cggatgttna aangnaaaac ttatggccac agacccattt natgaaagga agacttacat 540
catagtacgg ccttatgctt ggatcttgga anntgagggc attgagntcc nggactgccg 600
gcgggcntta aagngaatcc acnn 624

<210> 50
<211> 733
<212> DNA
<213> Homo sapiens

<220>
<221> misc_feature
<222> (1)...(733)
<223> n = A,T,C or G

<400> 50
ggtaccacaa agacagaagc ttcacaggaa gagcggtcta attcaagcgg cctcacatct 60
ctcaagaaat caccaaaggt ctcatccaag gacactcggg aaatcaaaac tgatttctca 120
ctttctatta gtaattcgtc agatgtgagt gctaaagata agcatgctga agacaatgag 180
aagcgtttgg cagccttgga agcgaggcaa aaagcaaaag aagtgcagaa gaagctggtg 240
cataatgctc tggcaaattt ggatggtcat ccagaggata agccaacgca catcatcttc 300

ggttctgaca gtgaatgtga aacagaggag acatcgactc aggagcagag ccnntccagg 360
agaggaatgg gtgaaagaag tctatggggt aaaacatcag gggaaagctg gttggatagc 420
agtngatgat gaccnaaatc tggantcttg naagaatgac cggtnattan ggntccaaaa 480
atttaaaccc ttangttttg aaggggccna aacttnggac cnnaaanctt cattgggatt 540
taaccaggtn ggnacntttt gggcacccca ttgacccgna tttcccccat tgggaccttt 600
tcgaatttct tanaaaactt ggnccnngga aaaaagggaa cccgggaaaa agggtaaaat 660
ggaaaaggaa aaacctggnt tngggaaaaa aaaaacnttt gcccaaanaa aaaaaangaa 720
aagccccttt ttt 733

<210> 51
<211> 565
<212> DNA
<213> Homo sapiens

<220>
<221> misc_feature
<222> (1)...(565)
<223> n = A,T,C or G

<400> 51
acattaagtc aagattgagc tttgatttaa aaggaacata aatcctttac attataaagg 60
gaagacataa atctctccaa tctaaatttt ctcatcttgg atgatgtcat taaactgcag 120
ctcaaactga gattagttta gaattttatg taaattacat ctttgaacaa atgagaacaa 180
ataactcatc tgcagaatat ataaagaacc ttcattaatc aaaaggaatt agacaagcac 240
ctagttttaa aaaataaatg gtgaataatt taaacagaaa cctcaaaaaa gaaaatatca 300
gagtggccaa taagcacata gaaagataca caacatcatt agtttttaag agaactacaa 360
attaaagcaa ccataaagat acctccccaa cactacnaga atgactaaat ttttaaagtc 420
cgacagcgtt gtgcccggtg tcccaatacc actcaggtta agtgatttct ggaanggctc 480
cagaactcag aaaagctata cttgctatcc tannggtatg ggttggtacn gtggaaaaat 540
cccggttaaa tcaggtaaag acccn 565

<210> 52
<211> 637
<212> DNA
<213> Homo sapiens

<220>
<221> misc_feature
<222> (1)...(637)
<223> n = A,T,C or G

<400> 52
ggtacgttcc aaagaaccaa ctggttcttg atctgctcct gagagataac cttcaaatcc 60
ttgaaatatc ctgcatgata agagtgagtt tgtaaatgtg gggccttcga tcatgccaaa 120
tagtttatgc taaccatgtg atttatggtg gggaacttga ccatgctgtc agtttgacat 180
ccggaggggc cgagtgttaa gtaactaagg ttggccacat gggcaatcca tgcttctgta 240
actgaagcct aatagaatct ctagacaacg aacagcttgg gtgagcttcc ctgcttgata 300
atattccaca ttgntttctg gaagaattga acattcttta cacagcttca ctaggagcag 360
acaactggaa atttgcctgn ggnctctctt tgggagaact ctgggncttt tacctggatt 420
taaccnggat ctcttnactg naaccaaccn ttaccnttag tatngccaag gataactttt 480
ttgaagtctg ggagtccttc cgaaaatnct taacctgatg gnnttgggan ccccggcaan 540
cttgnggcct ttaaaattan ncntnttgna nggtgggggg gntttaaggg ggtttaattn 600
gagtncttaa aactaagngg gggggntttt ttttggn 637

<210> 53
<211> 632
<212> DNA
<213> Homo sapiens

<220>
<221> misc_feature
<222> (1)...(632)
<223> n = A,T,C or G

<400> 53
ggtacatcca agatttgaag aactgaaata aatcagcttt aaacctgctt tttaaaaata 60
tctgggttgg aatttgcccc tgacaaataa taaaatgatg agtgatgcaa gtgacatgtt 120
ggctgcagcg ttggagcaga tggatggtat catagcaggt tctaaggctc tggaatattc 180
caatgggatt tttgattgcc aatctcccac ctctccattc atgggaagtt tgcgagctct 240
gcaccttgtg gaagacctgc gtggattgtt agagatgatg gaaacagatg agaaagaagg 300
cttgagatgc cagatcccag attcaacagc agaaacgctt gttgaatggc ttcagagtca 360
aatgacaaat gggacaccta ccagggaacc ggagatgtgt atcaagaaag gctggcacgt 420
ttagaaaatg ataaagaatc cctcggtctt canggtaagt gtgntaacag accagtggan 480
gctnanggag agaaaatcna gaattggagt ttggcttgaa aacccngaga gaattgaatg 540
ccccgaagaa tgctgcacag gagctntaat tggacttctt aaactcnaan ttggactgan 600
gctgaaantt acctgagttg actgnnntgg tn 632

<210> 54
<211> 661
<212> DNA
<213> Homo sapiens

<400> 54
acaatagaac tttcagaaaa ttctttactt ccagcttctt ctatgttgac tggcacacaa 60
agtaaggctg ttgctttcaa tgcatgcaat attaactttg agtgtttact aactctgtgt 120
tttgcttacc tggcttttct tccttgaagt tgcttaattt tttttcctcc aagaggaatt 180
atttaaaaag acttttgtct gtgacataac caagatttat tctgtttacc taaggaactt 240
attttctttt ttgcaatttc atttattctg agtcacttta tttgtaataa gtgaagaatt 300
ttaatactta gaaataagtt gtaaagaaaa taatgagaat cttaccatgc tttagaggaa 360
cggtaatttc tagaaatagt taaaagatga aatactaaga tattatttta ccttctttat 420
atagctgtat atactggtag tatgaaagca actagtgtca ttgatgattt tttggggggg 480
tatttttgta ttctaggctt gctgcaacct catttagaga gggttgccat cgatgctcta 540
caggttatgg tggttggtac ttcccccacc aaatcgtaga aagcttcaac ttttaatgcg 600
tatgatttcc cgaatgagtc aaaatgttga tatgcccaaa cttcatgatg caatgggtac 660
c 661

<210> 55
<211> 628
<212> DNA
<213> Homo sapiens

<220>
<221> misc_feature
<222> (1)...(628)
<223> n = A,T,C or G

<400> 55
acaactgcct acattctttc tgtttatcac ttcagttaga agtgttacat tcccaaactc 60
taatgttaat ccgagaacgg tggggagacc ttgtgcaggt ggaaaggtat catgctggaa 120
agtgcctctc cctttcagtt tggaatcaac aggttcttgg gagaaaaact ggaacagcat 180
ctgttcacaa agttacaatt aaaattgatg agaatgatgt ctccaagcct ttacagattt 240
ttcacgatcc tcctttgcca gcttctgatt ccaaattagt agaaagagcc atgaagatcg 300
accacttatc aatagaaaaa ctcctgattg acagtgccat gcaagagctc atcagaagct 360
tcaagaactg aaggccattc ttagaggctt caatgccnat gaaaactctt tcatagagac 420
tggctccagc tcttggtggt nccatcttgg agccctgngg naattcanan tggctgccat 480
tttgnagaat tacattcttg gaaggntcaa tggagcttta tngacttgnc aggccctntg 540
ggtgaatggg aanctnggat gagatttgaa ccaatntacc cggattanca cttaagtttg 600
nttggcaaaa ngttcaggcg nntnaaaa 628

<210> 56
<211> 635
<212> DNA
<213> Homo sapiens

<220>
<221> misc_feature
<222> (1)...(635)
<223> n = A,T,C or G

<400> 56
acctcagctg gggaaccgtc ctagaaagag atggccacta tgctgtagct gccaaatgct 60
atttaggggc cacttgtgct tatgatgcag ccaaagtttt ggccaaaaag ggggatgcgg 120
catcacttag aacggctgca gagttggctg ccatcgtagg agaggatgag ttgtctgctt 180
ccctggctct cagatgtgcc caagagctgc ttctggccaa caactgggtg ggagcccagg 240
aagccctgca gctgcatgaa agtctacagg gtcagagatt ggtgttttgc cttctggagc 300
tactgtccag gcatctggag gaaaagcagc tttcagaggg caaaagctcc tcctcttacc 360
acacttggaa cacgggcacc gaagggtcnt tcgtggaaag ggtgactgca atgtggaaag 420
aacatcttca gcccttgaca cccctgaccg tattanggaa nccttnanaa acttgagaac 480
attnagtacc ttgggccgga acacccttan ggcgaattcc acncactggg ggccgtacta 540
nggggntcca acttgggccc ancttggggg aanatnggcn aacnggttcc ttgggaaatg 600
ttacccttcc aatcccncaa nttnaaccgg aggnn 635

<210> 57
<211> 345
<212> DNA
<213> Homo sapiens

<400> 57
actgcttgga tcctgctctc tccaagctgt gcacacacat aaggcagatg atgaccattt 60
gaaagatgag aaggtccggg aggaaagcat atccactctc atactcctcc tcatcctcac 120
tggccaggct gaggttgggt gaggagggca ggtagaagag gcagaggttg aagtcctcca 180
ggactgactg gcaaagtgag gtcagctctg agtccacgga gctgcttttg ggctgtagga 240
ggctttgcag atacataaag ttcactagca accttttaat gtctttacat cgctttttgc 300
caggagacag tttccgagtc tcacacttct tcagttggtg gtacc 345

<210> 58
<211> 638
<212> DNA
<213> Homo sapiens

<400> 58
ggtacttcct cttcctcctc atcctcacta gaggcttctt ctgcggcatg attagacctt 60
gggggaggag cagtggcagt gccatctgcc ttctggatcg atggcttctg acagatgtat 120
ttggggtccc ttccaagatt acagatttct tcaagtaact tgatgatggc agtcgttgca 180
tctgttttaa gggtgggctg atgtctcatg agctcatcga cagcactccc caggttggat 240
gcagtatccc caaggggatc agaacttctc ctcctccgca tggctgggag gtaatctgga 300
gacagaagaa ctttgaagag gcgttcaaaa ggctgacact gaacaaaaga ctgaagacct 360
cgggcattca aacagagtgc actgaataca tttgggaggg agccaaggac ttcacgggta 420
gcaggaacat ctttgataaa gcagtgcatg cagcatgaca tctggcaatc cattgtcctg 480
gagtgaggag agcagtgatg gttcttgaaa tacaaacaca gtcaccactt cagtagctag 540
gaggaagagt gatgggccac agtattctgc attgctgatg atgtgtttca gggaggtagg 600
cagagaacca tccatcacat gtcgtatgcc atctgaga 638

<210> 59
<211> 728
<212> DNA
<213> Homo sapiens

<220>
<221> misc_feature
<222> (1)...(728)
<223> n = A,T,C or G

<400> 59
gcgtggtcgg cggccgaggt accatgccca gctaattttt ttacttttag tagtgacggg 60
tctcactgta ttgcctaggc ttctcaaact tctggactca agcaatatgc ctgcctccgc 120
ctcccaaagt cctgggatta caggcatgag ctaccgagct cagttttgaa aggtagaagt 180
gtatgctaca agggatgtag gacttgagag tcaaggccta tggtcttgtc ctggctctac 240
cagtaagtgt gaccttcgat gtttttttct caagtaaggc tggtaataat taccacagtt 300
gtgagaattg agaatttgga aatgcagtga aagagactat actcaagtct tgttctggac 360
taacagtgat cttaaaatct ctcatttcaa agaaataaag tattttgatg atctcttgca 420
tgggngtatt aataaacctt ggnataatgg cagaaactgt acctacaaca gggttaccgt 480
taactctttt tggaaggtgg tttggaaaaa naaggaatgg acccttgaat cttggaagaa 540
cgttcaancc tcatgacnta aggaaaaant tggaaaaggg ccattggnga ncccaaggac 600
ccaatgcccn tgctcttnaa aagggaaaag ggggaccang ggntcaaaat tggaaaaacc 660
gtttttccng gaaatccttt gggccccntt nnaaaggtcc ccaccttngg ggaattttga 720
aaaaaaaa 728

<210> 60
<211> 581
<212> DNA
<213> Homo sapiens

<220>
<221> misc_feature
<222> (1)...(581)
<223> n = A,T,C or G

<400> 60
ggtactggcc caaggcaaag atggagaata tgaagagctg ctcaattcca gttccatctc 60
ctctttgctg gatgcacagg gtttcagtga tctggagaaa agtccatcac ccactccagt 120
aatgggatct cccagttgtg acccatttaa cacaagtgtt cccgaagagt tccatactac 180

catcttgcaa gtttccatcc cttcattatt gccagcaact gtaaacatgg aaacttctga 240
aaaatcaaag ttgactccta agccagagac ttcatttgaa gaaaatgatg gaaacataat 300
ccttggtgcc actgttgata cccaactgtg tgataaactt ttaacttcaa gtctgcagaa 360
gtccagcagc ctgggcaatc tgaagaaaga gacgtctgat ggggaaaagg aaactattca 420
gaagacttca gaggacagag ctccggcaga aagcaggcca tttggggacc cttccttcca 480
ggcccccaag gcaggacacc tcatggatga caacccccttc gnactcgaaa agtcagactt 540
tcttttggcc cgggcttttt taaaatccaa agttacnaga g 581

<210> 61
<211> 681
<212> DNA
<213> Homo sapiens

<220>
<221> misc_feature
<222> (1)...(681)
<223> n = A,T,C or G

<400> 61
acgagcccaa gccctgttcc atcagccaat tgcaaacctg ctccttggtc cacttggcaa 60
atggcatatc caagtcactg ttagactgtc ccaagtctcg agaccaacct aatcggggcc 120
ccgcggttgc ccttgtccct cctcttttga attcaggctc agacatgtca tctgggttga 180
atgtagttga ttgacttctc ctaagttttc caaagagttt catgatacct ctggatttct 240
ttttggaatc tggagatgga ggcggtatct ggaagggact gttcctctgt gaatcttttg 300
gccgagaaag aagcaccagc cagatctagg tgctctgctg nctctttttc tgnttcaact 360
aaatttggtg cacttgctgg tctcttggta cttttgattt taaaaaagcc ccngccaaag 420
ggaanactga cttttcgagt gccnaaaggg ttgcatccat ngangtgtcc tgcccttggg 480
gcctgggaag naaggtccaa atgggctggt ttctggccga nctttggcc tttggannc 540
ttctggaaaa gttnccnttt tcccattaaa cgntntttct tnaaaatggc ccagctggtt 600
ggacntttgg naacttgaag ttnaaagntt ttcccccant tgggnnttaa caggggggncc 660
cagggatatg ttnccttant t 681

<210> 62
<211> 569
<212> DNA
<213> Homo sapiens

<220>
<221> misc_feature
<222> (1)...(569)
<223> n = A,T,C or G

<400> 62
actgggatta caggcgtgac ccaccacacc cggcccctaa ccactcttga aagtcccttc 60
acatctgtta gttctttaag gatgaaggct gagaattaac cttgttccct attccccgaa 120
gtgtctgacc cagtgctgaa tgtgtggtcg gagcttggtg aattctttcc aaataaagga 180
attcccacaa cagccccacg aaggacttga ggcaaggatt aggatcccca cttacagaag 240
aggaggacaa ggcccagaga agatccccca gactcagcca gggcacgagg ggtcgggtga 300
gttttgagat cgatagagcc ttctttcact ctcctgtgac gacatgacag tagataaaaa 360
gcatatacct tcatgcactc tcatgggctc tggcaccatg tttagagtcg ggctagggtt 420
ctttgcaatc tggtaaccta tggcttaaac ttatacccaa acctctcttc ctgcttcttg 480
nctgtgcaca tctctttcca tcagaccatc catagctcaa gctcaacagc tttnccagct 540
agtgntcctn ctccttttnc atggagtgc 569

<210> 63
<211> 650
<212> DNA
<213> Homo sapiens

<220>
<221> misc_feature
<222> (1)...(650)
<223> n = A,T,C or G

<400> 63
gaggtacaat ggaggtatct gtgggaagga aaatgcaggt aaagatgaag aggaaaatct 60
gccttgttaa agcccagctc cccaaagtat tagacacatg aatttgcttc tgtgctgagg 120
ccatctgtgg ccgtcaggct agctgttttc tggctgatac tttttgggaa tgttattgtt 180
gctgagaaag atagttccat gtcagagcta tcaacagaat gtggccatct ggacaaccat 240
gtataaacca acttattgct tcttgaatgc cacctacaaa catgactacc tgtcctttct 300
tgtttgaagg ggcactaaca atacttggga agatggaaag tgaactggac attaaggcag 360
agatgaagaa ttctgccttg cttcctgcac tccatggaaa aaggaggagg acactanctg 420
ggaaaagctg ttgaaccttg aactatggat ggnctgatgg aaaaaggatg tcncngacca 480
naacnngaaa aaaaggtttg gtttaagtta ancctnaggt acccgaatgc aagaacctac 540
cccactttaa catgggccca anccttaaaa gcctnaagnt atgnctttat tcnggattnt 600
ncccgaaang naaaagnttt ttgantnaaa attncccncc ccnggccggg 650

<210> 64
<211> 676
<212> DNA
<213> Homo

<220>
<221> misc_feature
<222> (1)...(676)
<223> n = A,T,C or G

<400> 64
cgaggtgcca attgggagga accttctttg gatgagggtg ctcggtttag caatatcaag 60
gtgtggctcc agataattca atcatctaat taagattcca gttatgctaa tctgttttaa 120
aattccgttt gtgtaaattc ttttacaaag cctcaacccc aatttccagg gagggttcag 180
agcctcaggt tgagttgatg accaacagcc tatagtttaa cccatcatgc ctctagagtg 240
aggtctccaa aaaaatccaa aaggaatagc tgtagagagc ttctggataa cactaactgg 300
aaggtagagc gccactccaa acaagacggg accaaaaatt tttctgaatt tttcgcaata 360
tctgcaacaa taaaatggga aatgtaatgg ccctcctacg tgttgggagc tctttcagcc 420
aatggatgcn actattacna ggantggtgg aaacctggat tataaccagc tgctgaaaaa 480
gccagtaaac aacgtaaggc tttcattggt aatantattg gaaggacagt cntgtgggac 540
ttcggccctt tgnaactaat ggtatgcccc gnanataacc gtncccttgg atttcaagac 600
cccctttggt tggnanaatt tttgggcatt tgcttgctgg cttaattacc attggaatca 660
aatcttttcc ggccnn 676

<210> 65
<211> 660
<212> DNA
<213> Homo sapiens

<220>
<221> misc_feature
<222> (1)...(660)
<223> n = A,T,C or G

<400> 65
acgtggcctg aagagatgtt attctttaaa atggtctcgg ctgtgggcga ggtgccccca 60
tacaacaact ctcgggctat catggcagtt accgtggcct tggcaggatt cggagctgcc 120
ctggtaaaat ctttggtgtg atgtccttga ctaactccta cagcctgggc gacctcgggc 180
accatgggaa gaattccagc aggcagctgc tgatgactta gataaggcat cctgaactca 240
tcctctttat tactagtccc attttcatcc ccagagccag gttcaaaaaa ggttactttt 300
cttccatccc ctggtttctt tatgggtgtc ttctcctctg acttgagtgc cggtttggtg 360
gctgcgcctg cgggactttg aaacccagga tcttcaacat gntctcgctg cattgccttg 420
gccaccttct tgtggtgccc gtccttntgc aatgggggtt ctaaccttna cctgnatnac 480
aaacttcctt ncgcnccgga aggctngctt cntgaagaac gtgtaccttg ggcgngaaca 540
cgcttanggc gaantccacn cactgggngg ccgtactann ggaatccaac ttcggaccaa 600
cntggggnaa catggcaaac tggttcctng ggnaaatgta tccgttacaa ttcccncana 660

<210> 66
<211> 678
<212> DNA
<213> Homo sapiens

<220>
<221> misc_feature
<222> (1)...(678)
<223> n = A,T,C or G

<400> 66
actcaaatct catcagcagc gtctacatcg taaaaaacaa ttagagaatg aaatgatgcg 60
ggttggatta tctcaagatg cccaggatca aatgagaaag atgctttgcc aaaaagaatc 120
taattacatc cgtcttaaaa gggctaaaat ggacaagtct atgtttgtga agataaagac 180
actaggaata ggagcatttg gtgaagtctg tctagcaaga aaagtagata ctaaggcttt 240
gtatgcaaca aaaactcttc gaaagaaaga tgttcttctt cgaaatcaag tcgctcatgt 300
taaggctgag agagatatcc tggctgaagc tgacaatgaa tgggtagttc gtctatatta 360
ttcattccaa gataagggcc atttatcctt gtaatggcta cattcctngg ggtgatatga 420
agagcccatt aattanaatg ggcatctttt ccagaaaggc tngcaccaat ctaccttagc 480
cagaacttac ctgngccngt tgaaagtggt ccttaaaatg gggtttaatt cttagagatt 540
tttaacctgg ataatatttg antggaccgn gaagggcctt attaaaatgg cttgctttgg 600
ccttngactg cttnanatgg ccccccaatc taagtncctg ggccggaacc ccttangggc 660
naattcagcn cactgggg 678

<210> 67
<211> 695
<212> DNA
<213> Homo sapiens

<400> 67
ggtactatgt gtgaagaaat ggagaaaagg aaaaatcagt gtagaaaaat aaaaaaagca 60
agagtgaggt tggtgcctac agttcacagc atgtgataag gactgagcat ttattctatt 120
atttggtcat aaaaatgcag gctgtaaggg cctacacaca ccagcttatc gcagacttgg 180
ctctgagctt tcctgcagcc aatacaaaca gggagacaca acagagaatt gccaatgctg 240
gaagctagat gtctaatgct gatcctgctt gtgactaaag tctgaatctg ggctaagtca 300

cacatgtcct gacactctgg aagctctgtc tggtgggtct gggaacgggg gagaagtgaa 360
agaggaagta gcaaggaaag atgcagaggc ggagcctggg agctagggca gtgccaggtg 420
ggactgacat ggcaccagga gtccctcctg cagggatctg tcctgattca ggtcagctgc 480
atcctgcatc tctagggaat gagaccacat ctgcaactca ccaggactgt tcactgtttt 540
ttccaccccc caatctcact cccactcaat cccttggatg tgggaaggag aaatacttaa 600
gctgaatgtt gctgtggccc atttgatgac aggttaccag tgtgggggat gacccccaat 660
gactgcaaga agtggtccag atgtcagaag tgggt 695

<210> 68
<211> 579
<212> DNA
<213> Homo sapiens

<220>
<221> misc_feature
<222> (1)...(579)
<223> n = A,T,C or G

<400> 68
ggtaccaagg aagacattca gagtgtgatg actgagatcc gcaggtccct tggagaggta 60
tgttttactt tagtaaatgt tagtttatat ggtaattttt cctttaggaa aatctgactt 120
tttatagtga tttgcttaca ttatttacac ttctgagtta gattttgttt gaacaaaatg 180
ttctgtgttt attaaaaaaa aaaaaaaaaa aagaagcagt agcttgtaaa attctgcttt 240
agcctgtatt ctgaaggaag aatgccttag agtaagtctg acttcagaat atttatgcag 300
taaaactgac agtattcttc atcctaacaa ccttatggta gaatagaaag aacagtggac 360
taattatcag gagacctgac aattagttct agtcattgtt gtgtcgacag ttagctggag 420
gaccttgaat ataagttcct caacctaact tgacatcagt gnttttcacc tataaaataa 480
attaaaatag gtaatgatta aatactctta aggctcttat attangnaat ggactgggat 540
tgagtaataa atacctaata gcccttcagt taattnaaa 579

<210> 69
<211> 661
<212> DNA
<213> Homo sapiens

<220>
<221> misc_feature
<222> (1)...(661)
<223> n = A,T,C or G

<400> 69
cgaggtacaa gctttttttt tttttttttt tttttttcag aatgctaaat tctatttttg 60
tagagcagag actccattaa aaactcccaa atgacaaact agaaaaaaaa tttacaacac 120
tgtgtgaaaa tcanagtgtg atttccttta atatacaaag agctcttgca aaccaacaag 180
aaaaacacaa atacccaaat ggaaaaatca acaaaggaca ggaatagtta gttttcagaa 240
aaagaaatat gaattaccaa taagtgtgaa aatggtgctc aatgccatca tgattaaaga 300
aatgtaacca aaacagtggt gagcccattt ttcatgtggc agattactca attttagtaa 360
tttattctga aaacaatctc ccacaagtgt atacttccac ttgnatgcnc aaggaagtac 420
aagctttttt ttttttttnt tttttttttt ccttggctgn agtcatgagc cttttgaaaa 480
aggcctccaa agtaaatntt tcagggggaa tagggaaagt ntttttttaa anaaggcngt 540
gattntaant tccccgggac tatggtgaaa tactntggaa aaattnaant ggtccatggt 600
[?]gccnaaatg gngctnttta aaanggnggg gaaaaaantt tttgngggaa aatncccaag 660
661

<210> 70
<211> 697
<212> DNA
<213> Homo sapiens

<220>
<221> misc_feature
<222> (1)...(697)
<223> n = A,T,C or G

<400> 70
actgagtttc cagaaagcgc agtgcacttt tagtgcgcca aactggtaat ttgccattta 60
gagaattctt cctaaagtag attatttctg ttaaagcaaa tcactattcc taactgattt 120
ataattttgg taaatctaaa ttttcatgaa ataggcttat aaagcgtgcc acatttctgt 180
tttctcctat ggacaggaag aaaaagttgg atggggacag aaggacagaa cagggtgcgg 240
aaaccatagg ataaaagctg tgggttttcc cccaaaagtt gctcaaaaga ataatatgac 300
ttctgctttt cttctcctct gggtggcaat tggggaatcc agcagcctgt tgagaggaca 360
gaattggtta agttgtggag aggtgcagtc taattggtaa atctttaaaa gtcttggttg 420
tctaacctgc tggttttctt gctcacagcc cctgcagata tcttctcacc taccttaacg 480
ctggcatgca aggntttctt ctttgctgag tggcatttng gttaatttcc atgttnaatt 540
ctaaccttgg ccatgattac naagccccta ctatgggctt gctttgagtt angccctggg 600
gctttaagna atncctanaa ttcncccntt cttnattctt aagggcttgg anatnccaaa 660
atgatnganc ttgacnttgg tttgggaggg naactna 697

<210> 71
<211> 705
<212> DNA
<213> Homo sapiens

<220>
<221> misc_feature
<222> (1)...(705)
<223> n = A,T,C or G

<400> 71
accacacagt caatgatgtc agccactccg agctttaggg tcctgggagt ggcagtaggt 60
gatagctctg tctctccaaa aagcaaaagg atcctgcttg gggacacccc aaggtgggtg 120
gccatgtggt ccaccacact ctgcaggggc tccgacatcc tgaggggcaa tctgaccagg 180
tcagcccggc aacggatttt gagtgggaag aggcttccta gatgacgggt gatgaagccc 240
aatcttccag gtggagagga cagcatgacc aaaggaagga cgtggaggtg acatggcatg 300
tgcagggaac tacactgaac actgcagaga gccactggca ggacccaggc cagggagcac 360
ctacttggtc atactgggga gcttggcctt tctcttggtg gtctggagat cccaaaagaa 420
tttatgccaa aaagttagag gtggatagat tttaaatact ggggttttta aatacccgan 480
ggattttaaa tactcttgat gggttaatct aaatttangg ggaaccaaaa ctggaggcnn 540
ntnaaaaggn cccttataag tggaaaaant gaaaagagnt tgnattangg cnncnnaaat 600
ttntggtggc nttttaagtn ccnttngatt tcccannaaa attnaatcng ggggatttta 660
atcccggaat tgggggaana aannnnggaa gggttnccaa ttttg 705

<210> 72
<211> 683
<212> DNA
<213> Homo sapiens

<220>
<221> misc_feature
<222> (1)...(683)
<223> n = A,T,C or G

<400> 72
actgaatgaa gtaaccgaag acaacttaat agacctgggg ccagggtctc cagcccgtgg 60
tgagcccaat ggtggggaac acagcgcccc catcttccct ctcctcccag cttgcaggct 120
tagacttggg gacagagagc gtcagtggca ccctcagttc actccagcaa tgtaatcccc 180
gtgacggctt tgacatgttt gcccagacga gaggaaactc cttggctgag cagcgcaaga 240
cggtaaccta tgaggatcct caggctgtcg gaggacttgc ttctgcacta gacaatcgaa 300
aacagagttc agaaggggta ggtctttaac cctgtttttc tgcctggagt cttctggagg 360
gaaagtcagg tggtttggca aaactggctg ggtaattcag cagaaactgg cttgcacagg 420
gggcanggac accctggggg gaaaaaccna cgggggacac cccgtggaac ccaagtantg 480
ccttatttga gtcttnacct naccccgtga gataaggccc ccatgagctt tccaatccac 540
ccaagagaaa cnagtncagc nggtgggana cagcttgnac ncccanaagc nnacngaagc 600
cgggttccaa tctnggataa gggcntttcc aaancctggt ggtcttacca aagggcccaa 660
ttttcaggcc aanttttntg gnn 683

<210> 73
<211> 566
<212> DNA
<213> Homo sapiens

<220>
<221> misc_feature
<222> (1)...(566)
<223> n = A,T,C or G

<400> 73
acagtgtgga aatttcaaca tgtatataca tccgtgaaac cattatccca atcaacatca 60
tgaatttaac catcacccca aaaagtcttc tcatgatctt ttgtaatacc ttcctctttc 120
ctgtcccgtc ccccacaacc gtctgttttt tgttctatta gtttgcattt tctagagttt 180
tatataaatg aaatcaatac attatacctt ttttgtctag cttctttcac tcagcataat 240
taatgtgaga gctgtccatg ttgtctaatg tattagtagt ccatttctat ttttgtgggg 300
ttgggcaggg gctgggtagt attccattaa gaggatacac tacagtttgt ttattcattt 360
tcctattcat ggatgttttg gttgtttctg gtttgaggcc tataatgtca cttgaagata 420
gattgtgatg ttaaaggtgc atactgtaaa ccctaaaata gtcactaaaa taacnaaaac 480
gaaaaggtat tggtaataag ccaacaaagg aaataaatca aatcataaaa tacnaaagaa 540
agcngaaaaa gaccaagggc acctgg 566

<210> 74
<211> 690
<212> DNA
<213> Homo sapiens

<220>
<221> misc_feature
<222> (1)...(690)
<223> n = A,T,C or G

<400> 74

cgaggtgtac aagctttttt tttttttttt tttttttttt ggctccctgt agcctcgact 60
tcccagcaat cctcctgctt cgcctcacag caggcacacg ccaccatgcc cagctaattt 120
ttgtattttt tgtagagaca gggttttgcc atgttgccta ggctggtctc aaactcctgg 180
gctcaagcaa cccatctgcc ttggccaacc aaagtgctgg gattctaggt gtgaaccact 240
gtgcccagcc aatctctgtc ttttaaatga gggtgtctgc atcgtttgtt tcacatggnt 300
atttaggact aactctatca ttctgctgct cagtaatttt gtttgccagg ctgcctttgg 360
tctttttctg ctttcttttg nattttatga tttgatttta tttcctttgn tggcttatta 420
acaataactt ttcgttttgg taatttaagn gactatttta ggggttacag tatgcaccnt 480
taacatcaca atctatcttc aagtgacatt atangnctna aaccngaaac cacccaaaca 540
tcntgaatng gaaaatgaat aaccaactnn annggaancn cttaaaggaa actaccaacc 600
ctggccaanc cccaaaatng aaaggcctct aatccnttna cacntgggcc ggtttncata 660
atntcntggn gaaaaacttt cccaaaaggn 690

<210> 75
<211> 447
<212> DNA
<213> Homo sapiens

<400> 75
ggtacaaact gtgttattca catctggccc ccaaggtatg taagggaaaa ctttaaataa 60
atctttaagc tcatcaggtg acaaagcaca gtctctatcc aaatcatgct tgtcaaaggt 120
gctttggaga aataaatatg catgatgatt taattcagta gtgcaatcag gaggtatttt 180
cagcaggggg aacaaatatt caggtgtcaa atccaggtca tcatcataac caaatcgtcg 240
aagcacagtc caagtagttt cgtgtctccc tctctggata aaaagtgtgt gtaaaaagag 300
aaaacctttc agggtcaacc cactgtcagc cacaccatca cttatatgtt ttctgactac 360
attcttgaca tcctccagag cttgaggagc taatggagtg ttgaaacaaa tcctctgaaa 420
gaagttgagt tcagcatcat tgagagt 447

<210> 76
<211> 674
<212> DNA
<213> Homo sapiens

<220>
<221> misc_feature
<222> (1)...(674)
<223> n = A,T,C or G

<400> 76
actgttaggt aattttgata ttttacttag ttggtttctt ttgtttttgg agacagggtc 60
ttgctctgta gcccaggctg gactgcactg gaactcctgg gctcaagcaa tcctcctgcc 120
tcggcctcca agtagctggg actactacag gcactcacca ccattcctgg ctaattttta 180
gtttagtttt gtagaaagta agactaaata cactggatca ttcagaatgt cagaaagtaa 240
tgttttcctc agtttatttt ttcttaatag cacacaccat gttattggtt tgtgttttgt 300
tagtgcttgt aactagagtg caacttaatt aacaatttgc tcctcctcat gaggttcatg 360
gcagtataga cttaaattct agtcccatgt ttgncattta ttagctgtgt gctaagactt 420
ggttttccta tcagcagaat tgctatgtat atctaagggt atgttaaggg ttcaaaccag 480
gaaccctctt tgtaagtgaa aggtgggggg gagctattgg taaatttttt ggtcagaaat 540
tggcatacct aatttaatta ctaccttact aaangnatca attaccctca tctatttcan 600
nggtttaatg ggnccaagtg gaatattcct ttacttaaaa gccagtttta ctgggaaatc 660
ncttancaag gntt 674

<210> 77

<211> 441
<212> DNA
<213> Homo sapiens

<400> 77
acatggtctt ttgttcccta aaagactgca tcacacctct gattgggagg ccaactgtca 60
tttaactgag tgtttgagtg tctaaaacca agttcagcat ttgtctatct agcaagcttc 120
cctttccaac ttgcttactc ctctcaattt catctgcaga tctcctggtt caataaggct 180
caaaaactgg ctgttccctt gcattcctct ctcttctccc aggcactctt catccttttt 240
tctctcaggc tcacccttac aatccaacac cttccaatgg cctctcctag tccagtccat 300
cctgacacca agtaactggc ccgctttgga agtcctgaca ctttcagtcc ctctttcctg 360
ttctttccac tttcctcggc ccccaggagg atcctggatg gtcgtcacag ctgacaaatg 420
atgagcagaa tgccctgtac c 441

<210> 78
<211> 623
<212> DNA
<213> Homo sapiens

<220>
<221> misc_feature
<222> (1)...(623)
<223> n = A,T,C or G

<400> 78
ggtacacgat taacttaaca caaaaacccg aacttcaaaa tgaaggtgtg tggaggaaag 60
gtgctgctgg gtctccctac aactgttcat ttctttgtgg ggcagggggt agttcctgaa 120
tggctgtggt ccaatgacta atgtaaaaca aaaacagaaa caaaaaaaac aaggaactgt 180
catttccacg aaagcacagc ggcagtgatt ctagcaggcc tcagggccct gggcctggag 240
aggctacatg agggggagcc tcagtcacag gatcaacctg gggcccgaag gagcagggtt 300
ccctgcctct ccctctgcaa cagatcatcc catccaacac aacccccaaa atgttgatga 360
tgacgcacat ggtcaaccct caagaccttt aagacaaaac agagcacata ggaaaaaaaa 420
aacnaaacgc ccaatttctg ctgtgtcaat ggtagggcac cattttaaaa agtctgctaa 480
acagtctgct ttacttggan ggacgtatgc aaacataatn cttgttagtg aagaaccatg 540
acgcctctac ttactctaag ttagtngaca ntaaacttct gctcccttca agttaaagnc 600
nttcnaactg ggtggggaat act 623

<210> 79
<211> 462
<212> DNA
<213> Homo sapiens

<400> 79
accagttaaa aatgtattta ccaataagtg ataacagcaa caatagctaa ctgacaattg 60
attaaagaca gtatacaggg atccttttgt ggttcataag catgatgatt agattttcat 120
gctattgggt gagatatgcc ttcctcagac tttgttacag cataggcaca ttacaacctg 180
tctgatagga gaaagaaagt aaagatggta tacaggccag gtgcggtggc tcacgcctgt 240
aatcccagca ctgtgggagg ctgaggtggg tggattgctt taggcctgga gttcaagacc 300
agcctggccc acatggcaaa accccatctc tactaaaata caaaaaaatg gttgtggtgg 360
cacacacctg tatttcccgt tgcttgggag gctaaggcac aagaatctct tgaaccagga 420
ggtggaggtt gcagtgagcc aatatcgcac cactgtacct cg 462

<210> 80

<211> 640
<212> DNA
<213> Homo sapiens

<220>
<221> misc_feature
<222> (1)...(640)
<223> n = A,T,C or G

<400> 80
acccgttgct gctgccatgt gtgtgcttaa aacagggttc ctttttgtag catcagaatt 60
tggaaaccat tacttatatc aaattgcaca tcttggagat gatgatgaag aacctgagtt 120
ttcatcagcc atgcctctgg aagaaggaga cacattcttt tttcagccaa gaccacttaa 180
aaaccttgtg ctggttgatg agttggacag cctctctccc attctgtttt gccagatagc 240
tgatctggcc aatgaagata ctccacagtt gtatgtggcc tgtggtaggg gaccccgatc 300
atctctgaga gtcctaagac atggacttga ggtgtcagaa aatggctggt tctgagctac 360
ctggtaaccc caacgctgtc tggacagtgc gtnacacatt gaaaaatgaa tttgatgcct 420
acatcattgn gtctttcgtg aatgccacct aatggtggnc cattggagaa actgtnaaaa 480
aagtgactga ctctggggtn ctnggganca cccngaactt ngcctgntnc ttattaggag 540
atgatncntg gngcaaggct ttccaanngn attnggacaa tccaacctac caganaagtc 600
atggntggaa naaccctgga aagaaacaat ggtgaagggg 640

<210> 81
<211> 643
<212> DNA
<213> Homo sapiens

<220>
<221> misc_feature
<222> (1)...(643)
<223> n = A,T,C or G

<400> 81
actgccattc cttaaattca tttagattac agtgtgtaat cataactttt gatccatcag 60
ctccctttgt caaacactgg tcatactgca tgagttgatt tgcttcattg attctgaaaa 120
gctgattccc tcccatcctg tggcagggtc ctagttcaac aaagcctcca tttgtttttc 180
ccatgctatc aatgcagtaa gcagtttcga agcctctgat ttctccccag tcaacatttt 240
tgggtggcaa agggtagtgt gaggtgatat cataagctat ttcttccatg aaccacttaa 300
aacttttgca gttgtgatct tctcgaaatt ttttcaagct ccgatatatc cccatatggt 360
aatgcctgcg attcaggacg actagcatag aagtagtctt tatattcatc caccaaacct 420
tcacaactct aacataattc ttcagagttg gagaagaccc aacataaatg ggcngaggat 480
tncttggcag ccctcaagac ggtagatatg tccacacgag aaccanggac caaataataa 540
tttgncacca cacttggcat atcttggatg agatctcaaa gtttcaccac cccaaatttg 600
gaaacctgga tcttgagacc caattcaaag aaaacttttg ttn 643

<210> 82
<211> 642
<212> DNA
<213> Homo sapiens

<400> 82
accaagtcat tatttctgac agcattgtgt attagaagga acactggatt tagtcaaaag 60
ataggagttt gaatcccgat gccacctctt accaactggg taaccttgga taggaattgc 120

ataacttctc tgagcctgtt ctcaaattgc ctacctcata aggttgctgt gaagaataaa 180
tgcatgatgg tttctgaagc acttatcccc tgccgttaga tctcctgagc tgcatttctg 240
tttaacacgg gcccccagtt tgtcagccaa gcagctcaaa tatatgaagt ctaaaatgaa 300
agtaatgacc ctttatgatc tctttctatt gttctcaatc agttcctttt tttttagtta 360
cctaattctg ctcacggtgt gtccctgttg ttcagattcc agatgtcagt gattgtggac 420
tcctcctttt tcttaacaga ttacataata cctgcagctg ccaagtcttt gtctgtgttt 480
tcattatttc atcatttaca tcagatcttt cttttctctt cccgttgaca caccctagtt 540
caggcctcat tcaagtcata cccagagtat tgtatcagcc tcctaattga tctttactcc 600
ttcactttgc aacctattct gtatgccttg tgaagtacct cg 642

<210> 83
<211> 584
<212> DNA
<213> Homo sapiens

<220>
<221> misc_feature
<222> (1)...(584)
<223> n = A,T,C or G

<400> 83
ggtacagtag agtctgagaa ctgggtcaac actgaagcat tcacaccttc aggatatgaa 60
gcagagcttc ctgtcacatc tgcagatgtt gtgctgttgg tcaagagcca gtgtgcagtg 120
atctctccac ctctcatggg tgcgactgac ctagacacag tctcagtctg agacatggga 180
cttccatttt gcacctcaga gctgctggca agctgatgtt ctccaaaggt tggggaatca 240
ttttgccaac gcaaagacgt aagtccaaat tcattttctg tggatggttc aatgaattcc 300
tcatcccctg gattcccagt tactctactg nttcttctcg attccactgc agagggtgaa 360
agaaggactg aggatgaagt ccgtagcaat tctggagtcc ttggggaagc cttctgtctt 420
gctcacaggt tccagactga cccgtcaaag atccgcagcg ttctcgggcc accttcagtg 480
aacacggggg caacatgcat tggctttgtt gactgactna ggagctttgg aggcccagtn 540
gganttgtta agcttctctg nacctgcccc gggcggccnc ccgg 584

<210> 84
<211> 558
<212> DNA
<213> Homo sapiens

<400> 84
ggtaaagaaa gaaaaaaaaa aaaggcctgg atactgcttt tgctgtctct gttatgagat 60
ggaagactta catggtttgt gataaaaggg gaccatgaga atgaattggc ttggcttact 120
ttccccctga aatcctctct cctgcagact gtcttgaaga cctggtgact ggtaaataaa 180
gccctgcatg gaggctgcac agcaggggca agaggcccat cccccagcat ctcactgagg 240
acagcttcag gctgccttcc tctgaacgtg gtccacacct tcctctcctc cacagagagg 300
gtgccgccag aatcccctgt cgctttctgt gtctgcaatg gggggcagca cagggatcaa 360
agccatctaa agagtttcca gagaaagtat taattcagaa caagccaaag accctgagcc 420
tcaccacaaa caggcctttt ggagtgtgaa tttgagttga agatacaaga tcggagaatg 480
attttctggt cttaactaat cctcgtcttc atgtttgatc tttaagaagt catcacccat 540
tgatttcagt tttgctgt 558

<210> 85
<211> 499
<212> DNA
<213> Homo sapiens

<400> 85
acaaaaccat cgccatcaaa aaaacgctgt tctgacaaca ctgaagtaga agtttctaac 60
ttggaaaata aacaaccagt tgagtcgaca tctgcaaaat cttgttctcc aagtcctgtg 120
tctcctcagg tgcagccaca agcagcagat accaccagtg attctgttgc tgtcccggca 180
tcactgctgg gcatgaggag agggctgaac tcaagattgg aagcaactgc agcctcctca 240
gttaaaacac gtatgcaaaa acttgcagag caacggcgcc gttgggataa tgatgatatg 300
acagatgaca ttcctgaaag ctcactcttc tcaccaatgc catcagagga aaaggctgct 360
tcccctccca aacctctgct ttcaaatgcc ttggcaactt cagttggcag aaggggccgt 420
ctggcccaat cttggctgca actatttgct cctgggaaaa tgatgtaaat cactcatttg 480
caaaacaaaa cagtgtacc 499

<210> 86
<211> 146
<212> DNA
<213> Homo sapiens

<400> 86
acaggatact taaaatggaa taactttttg gttgcaaaac agagacatgg ttctataatg 60
cttcatgtcc ctccaagatt tgagatcaat ttagggattg tgaaattttt tttttcaaat 120
ttcatacaat catatttccc agtacc 146

<210> 87
<211> 572
<212> DNA
<213> Homo sapiens

<400> 87
atccctagca ttttaaaatt cagttgttac agggatccca cataatattt tgtcatttat 60
atgagggtgg atgagggctg aaatttcatc ttgggtcttg gaacagattc atgggcacac 120
attttaaagc tattggtcct cagttctgca gattaagaaa ctccaattta ttgattcccc 180
agggtaatga gaaaatgcat tgagtgatat ataacatcca ctacattcac aggaaatgct 240
gtcctggatc aaaaactgac ctggtcattg aattatgttg gagaactcat aaaaattcca 300
tggagaaagt gatattcaag ttggctcatg aattctgagt aaaagtttaa aagcaaagga 360
gaggatagcc ttacagagat aacaatagga acaaagtcac agacttgtgg aaatggaaga 420
ccgggctaga aattaggaca gttcatattc aagcaagcag ggttgggttt gtgaacaaat 480
accttgaagc tttggatgcc ttggagccct tgacagtttt tgagaatgta tcaaaacaat 540
taaatagtct atttggaagt gagagccctg gt 572

<210> 88
<211> 512
<212> DNA
<213> Homo sapiens

<220>
<221> misc_feature
<222> (1)...(512)
<223> n = A,T,C or G

<400> 88
ggtaccttat ctccagaagc agactgtttg gggacaggcg cagtgcctgt ggagcggcac 60
ttgacatcag cgtctcttcc cacatggagt gaggagcctg gccttgacaa ccctgccttt 120
gaggagagcg ctggagctga caccacacaa cagccactta gtttaccaga aggagaaatc 180

accacgattg aaattcatcg gtccaatcct tacattcagt taggaatcag cattgtgggt 240
ggcaacgaaa cacctttgat taacattgtc atccaggagg tctatcggga tggggtcatt 300
gccagagacg ggagacttct tgctggagac cagattcttc aggtcaacaa ctacaatatc 360
agcaatgtgt cccataacta tgcccgagct gncctttccc agccctgcaa cacactgnat 420
cttactgggc tttcgagaga agcgcctttt ggcaacccga ngcacacaan cattctgaaa 480
ggnaactctc cccnagaaaa aaattttncn ng 512

<210> 89
<211> 573
<212> DNA
<213> Homo sapiens

<220>
<221> misc_feature
<222> (1)...(573)
<223> n = A,T,C or G

<400> 89
actcggctgc tcctccgcgt tctgagtcgc ctcctcaaca atctggacct caagtgcttt 60
aagggcaaca gcaggggacg cggcactggc tttcagcatt gcaactgcct cactgtgact 120
taaattggtc aaatcaatgc cgttgatatt tagcaacaca tcacctctct ttattctgcc 180
atctcgtgca aggcagccat ggggtggcac actggtcaca aagatgggca gctcaccact 240
cttacttccc ctgcccccag caacggtcat gccaagggat tcatgtggtt ccttctttac 300
agtaatgtgt ttttcttggc atgtaacaca ctgagtaaga tccttatgtg agcttggtct 360
gctataatac ggtggtggtg tgtggtgctg gctgctgctg ctatgatttc ctgcttctct 420
aatggtgtta ccaggctggg gtttccctgg tctagcaatt ggtaaattca ctctntctcc 480
actggcctga ataatctggg cagcaagctc cggaagttcc atacttcagg tcgtgcccat 540
tgatggccac actcggcatt gctgcttanc ctg 573

<210> 90
<211> 658
<212> DNA
<213> Homo sapiens

<220>
<221> misc_feature
<222> (1)...(658)
<223> n = A,T,C or G

<400> 90
ggtacctttt aacccaccct cctccaatca tgggaggagt tgttcgggat ctcagcatgt 60
ctgaagagga ccagatgatg agagcaattg ctatgtctct gggacaggat attccaatgg 120
atcaaagggc agagtcacct gaggaagttg cttgccggaa ggaggaagag gaacggaaag 180
ctcgggaaaa gcaggaggag gaagaggcta aatgtctaga gaagttccag gatgctgacc 240
cgttggaaca agatgagctc cacactttca cagatactat gttgccaggc tgcttccacc 300
ttcttgatga gctgccagac acagtatacc cgtgtgtgtg acctgatcat gacagcaatc 360
aaacgtaatg gagcagatta tcgtgacatg attctgaagc cagtagtcaa tcaggtgtgg 420
gaagcttgct tgatgtattg gatcaaaagc ttnttctttc cctggataac cangtggaca 480
caaaaaaccg tggtcanaaa tgggttaaag tcanatnggg ccccacttgg ccccaaggcc 540
ttccaatttn ggctanctta aaaatccttg gcttttaacc nctacttttt tgnagggaat 600
ttgaagctta cctttgggcc ttgggtgggg ttgnaatcna agngggattc ctttnngg 658

<210> 91

<211> 570
<212> DNA
<213> Homo sapiens

<220>
<221> misc_feature
<222> (1)...(570)
<223> n = A,T,C or G

<400> 91
acctctgact acaccttcat gttgggccct gaccaacaga ccctcaggtt gtgagttttg 60
gcttcgggga gaaaattctt cctgcttgat gtagggcaaa gtagctgatt tggcagattc 120
ctgttgccgt ggcagtccaa gagagataga tcccactgac ggcttgggtg tttcttgagt 180
gtaggaagcc tgattatgag aagtcaaata agtgcctggt gttccctgtg agatggagcc 240
tcccattata aaagatggtt tttctgaagc cactgtggtt ttggatgacg ggatgagagg 300
gggccggtgg cctggttggt cgagttgtcg gaagcccgaa cgccttcagg gagattagtt 360
atcacttgat gtggagcagg ctgaaggact tcccactctc tgtttggact cttggatgtg 420
ccacatggac ttgtagaact tctacattcc aaatctatct ggncttggct ctggccnttg 480
ttcctncagg agtgctgact catgcnttgn tttaatgngt cgctggtaga naacatancc 540
gttactgggg tccaatggga tgtacatngg 570

<210> 92
<211> 603
<212> DNA
<213> Homo sapiens

<400> 92
ggtacacatg tttttattag attcagtcct cacaacgaat ccattcaaag atacaactca 60
cagtggtgaa atgactggcc agaggttagc caggtagcac gtggcagagg cagggatacc 120
aagagtcctt tccatcatat cacactgact aagttttcct gggttctgtc gaaaatatta 180
atggttcatt gggcataatg gtttctagtt cttttctatt atttcatcca aatgaatttt 240
ccttctcatt tactatgaaa gattttgtta gccttcacat cttgccctac tgcttataaa 300
ctaaggaaag gcaggttcct ccacacagaa cagctctctc ctctatcact ttctatatga 360
aactttcaat aagacatatc gtgtttatct caagcccacc atagctgagg aggaatcgct 420
tgctttcccc tataattccc agtgcccagc attctcacaa ctaggaggtt cttgagaatc 480
tcctcattta tacaatatga agtaaaagcc aatttaaact tttaaatggt aacttaattc 540
aatgctgaat atcaaaataa tcaactgtta aaaatttaaa tgattgtttt gatatattct 600
tgt 603

<210> 93
<211> 627
<212> DNA
<213> Homo sapiens

<400> 93
   ggtacacatg tgtgcccagc attaaaaaaa gatgacacag atgctgctca caaatgtcgt 60
   tttgaaagga agaaaatata tataatcata aaacaaacaa caaaataaga taaaatatgg 120
   ggaaatgccc aaaccaactc catgccaagg aaagagcaat tggctaattc ctaaattcac 180
   caataggttc ctagaagctg gtctttgata aaatttttat tggttttcag taaaggtgga 240
   aaaacaagga gaatttattg agcttcttta aaaaaaaact aaattttttt caactcaaaa 300
   agattatccc ttttttaaga ttagcctttc ttatttgaga agccatcaac aaaccctttc 360
   tctgactgat agtgacatac ataactggtt tgtttatgca attttaatgt cattttttgg 420
   atgtggatag aggcagaaga aaagagaaga catcctgggc ccagattgca acacaaacac 480
   agaactgacg tgacagctgt gggggatatg ggacagagat acaggaagga ggagcctggc 540
   cagggttgca gagtgcagta aaatcagact ggggagctga gagagccctc ttggagaggc 600
   tttgaaatgc aggccgggga gtctgga 627

<210> 94
<211> 331
<212> DNA
<213> Homo sapiens

<400> 94
ggtacctatg ataatcagat ggagatctgg ggaggggaga acgtggaaat gtccttccgg 60
gtgtggcagt gtgggggcca gctggagatc atcccctgct ctgtcgtagg ccatgtgttc 120
cggaccaaga gcccccacac cttccccaag ggcactagtg tcattgctcg caatcaagtg 180
cgcctggcag aggtctggat ggacagctac aagaagattt tctataggag aaatctgcag 240
gcagcaaaga tggcccaaga gaaatccttc ggtgacattt cggaacgact gcagctgagg 300
gaacaactgc actgtcacaa cttttcctgg t 331

<210> 95
<211> 752
<212> DNA
<213> Homo sapiens

<220>
<221> misc_feature
<222> (1)...(752)
<223> n = A,T,C or G

<400> 95
ggtcctgtcc cgcccctctc cccaagcgcg ggcccggcca gcggaagccc ctgcgcccgc 60
gccatgtcaa agaaaaaagg actgagtgca gaagaaaaga gaactcgcat gatggaaata 120
ttttctgaaa caaaagatgt atttcaatta aaagacttgg agaagattgc tcccaaagag 180
aaaggcatta ctgctatgtc agtaaaagaa gtccttcaaa gcttagttga tgatggtatg 240
gttgactgtg agaggatcgg aacttctaat tattattggg cttttccaag taaagctctt 300
catgcaagga aacataagtt ggaggttctg gaatctcagt tgtctgaggg aagtcaaaag 360
catgcaagcc tacagaaaaa gcatttgaga aagctnaaaa ttggcccgat gtgaaaccgg 420
aaagaacnga acncaggctt accaaaaaga agctttcttc acnttcgaag aaccaaaggg 480
gaaccagctt taanggccna aagttgnaaa aatttccaaa ggactggnga atccncnaag 540
tttgtgggaa aaaaattccc ttanccttan ttccccaatt aaaaatnttt ggggncccaa 600
aagnaaaaat ttnggggttt tgaaanaaaa tttaaaantg ggntngaaac ntttttggga 660
aattccccaa aanaactttt gccttccctt tgnccttaaa aantttncca tgggggggna 720
aaanggattt nnccttgncc cnggggnggg nc 752

<210> 96
<211> 405
<212> DNA
<213> Homo sapiens

<400> 96
tacaacaaac accgaaaaca aagtaaaaaa tgaaacacaa ctagagaaaa tgtttaggac 60
acatgtcagg aggttaatat ccctaatact gaaaaatttc ttgctagtaa gccaaacaac 120
ccaataaaac tctaaatgat acttcgtgag ttgataaaat gatttccaac ttgagttgtc 180
agacaaaaca tttgagatag actaacaaaa ttattgttta tctaaaactc taattgggca 240
tgttgtattt ttatttgtgg aaggtggcaa cactatttca gacacttgtt ctcatttggc 300

cctgcagtaa ctcaatgaga tggggaaaga ggttaattaa cctctccaac agcagtttcc 360
tcatctgtca aatacagtgt gagaattaaa ttggataata taggt 405

<210> 97
<211> 499
<212> DNA
<213> Homo sapiens

<220>
<221> misc_feature
<222> (1)...(499)
<223> n = A,T,C or G

<400> 97
   acagaaactt ggtgggaaaa ggggactgtg gccagagttg ggaccctgga gcagcatcct 60
   ctgcagagaa ggattttgtc tggccagagc ctggagaaac ctgaaaaaga accagtcagc 120
   tagccagggt ctcagagaaa agcagattac acactcaaat tgggtaattt gagcagagct 180
   taataaaggc agtatttaca aagtgtgggc taagcctccc atgagagtgc agaaccctgg 240
   ggctagcagt gtggggcgct attcccagcc ccctcaatcc attggctgag gccgctggaa 300
   gccaccgggc caagggagct tgttgatgtg ggtcacacgg gcatgttccc aggtcaagag 360
   aggagagtgg agagtgaatc tanggagact caagagggaa gaagtgactt ccactacctt 420
   tcctttctgg ccgttttgct tccanctggc ttctcttttt ccgannccnt agttttgggt 480
   ttaanggnan ntangtnaa 499

<210> 98
<211> 688
<212> DNA
<213> Homo sapiens

<220>
<221> misc_feature
<222> (1)...(688)
<223> n = A,T,C or G

<400> 98
naggtacaag ttatcaatcc gagggacaag agggagggac aagaaccagg tctcagctgc 60
attcacatcc tggaccctgt catctcaaag ccagttccct ccctgccttc caacttggtt 120
tcattcactt tggattgagt tgcgttctca ctgaacagaa acccacaacc caaaacaagg 180
gcagcccatg gccgtgatta agctctgcac cagtggcgaa gggatcgagt gggagaccag 240
aattcagctc cgcctctgtg cggcctcaag ggagttatga acttctgagc cttagacatg 300
cttctgagct gccaccaagc tgcctnatgg ggctgcctaa ggattaatgn attaatccaa 360
tcccaggcac atnagtcatt aataaaatta agaatacngn gaccactaaa cccactactt 420
tngaagtact tcctactaac tacnttaaac cccaacttga aggttttgga aaaganaatg 480
nccacttgga aaccaaaccg gcnnaaangg aaaggtacct tggaggcact ttttcccttt 540
tggggcttnc ctanaatccn tttccatttt ctttttgacc tnggnaaatt ncccngggga 600
ccccatttac aaagtttcct tgggcccggg ggntttnaag ggctttancc aagggnttan 660
ggggcttggg aaaaagnccc ccacttgn 688

<210> 99
<211> 657
<212> DNA
<213> Homo sapiens

<220>
<221> misc_feature
<222> (1)...(657)
<223> n = A,T,C or G

<400> 99
ggtacttttc ttagtatctt aacatcacat gcattttgta gtttatggtc tccagtctcc 60
agctgttttt ggagcacctt ctaactttga gagggtgagc tctagcctgt aaaatggact 120
gtgggtggct cgtggagaag gtgccctggt gtgcttttct gtgtcctctc tggattctcc 180
ctgagctgtc cacctctgaa gcctgcttca ccttcagact gccagggcaa gacatgcagc 240
ttctgcagaa ctcatggcag ccgttttcca cttggccgag ctgggtctgt gaagcagaga 300
ggaatcagta ataggaaaga aatgtaagtt gnttttttcc cccttagaat acctaccata 360
ctggatttca gcttggagtg cgcagcatga agcatttgtg gtcaaaaaag aggncttcct 420
ttttccttct nctggtttct tttcttnctt cttcccaact tccccaangc ttactggctt 480
tcttntnaag ncacgtgtgt aaaatancct tgagggaaaa aanggttccg gcttgggana 540
tttggatnta cctaaagggn cagaataacc cttctttgcc tggttcnttt ttggcctaat 600
cnagggaatt tttcgactgg ggncattaat ggncctccgg cggccgttaa anggcaa 657

<210> 100
<211> 504
<212> DNA
<213> Homo sapiens

<220>
<221> misc_feature
<222> (1)...(504)
<223> n = A,T,C or G

<400> 100
atttcttctt tgcatgcagg aagaaaattc actcgccgtt tgataatttg ttatggtctt 60
atttgacctg ttatccctgc ctcccatgtt ctctttaccc tacaacccat cagctgttag 120
agtttccttt tccaagactc tccatgtcca tcccctctgc attcccccct ttcactccat 180
cttctgtaac ccagcccctc gggagctgag gaggtggagg cggatataga cacggagagt 240
gctggatgca aaggtgttac ttgtggcaaa ggcgccgtgt gtgctgagga tagatggcag 300
gtatgagaga gggcaggatg aagcacaggg gtggagggga gcagagagac ctacaacaaa 360
acccactcaa ggggtatgtg agatagactt ttttttctgg nctttttgtg tgtctgtaat 420
gggggttgga aagtggggtg gtctcancag ntaattctct ggagntctct ggacttgagc 480
ctngtcnnaa nagcccagaa nttt 504

<210> 101
<211> 685
<212> DNA
<213> Homo sapiens

<220>
<221> misc_feature
<222> (1)...(685)
<223> n = A,T,C or G

<400> 101
ggtgcctgtt ttgccactta ggaagctgga aagaattttc gagtcaagtt aacccaaccc 60
cctcttcttt tcacatgtaa gcacactggc tcagccagaa ctcaggtctt tcaacctcac 120
agttggtgaa gactcttaca tgttggttcc aagttgctca actctcaggg ctcagcctac 180

aaaagactcg gcatttcgac cagctcagtc cagaggactc cagagaatga ctgctgagac 240
caccccactt tccaaccccc actacagaca cacaaaaaga acagaaaaaa aagtctatct 300
cacatacccc ttgagtgggt tttggtgnag gtctctctgn tccccttcac ccctgngctt 360
catcctgcct ctctcatacc tgccatctat cctnagcaca cacngngcct ttggcacaag 420
tacacctttg cattcaagca ctnttcgggn ctatatncgg cttcaacttc ttagcttccg 480
aaggggcttg ggtacngaaa aaggatgaaa ggggggaatg ncaangggat nggcctggga 540
aagttttgga aaaggaacct ttaccnctga agggttgtag gggnaaaaaa aacctgggag 600
ggccgggtta ccnggtcaaa taggaccttn ccaantttta acnggggagg gaatttnttc 660
cngctgccaa naaaaannnc ttccn 685

<210> 102
<211> 498
<212> DNA
<213> Homo sapiens

<220>
<221> misc_feature
<222> (1)...(498)
<223> n = A,T,C or G

<400> 102
ggtaccatat acttaaggct atagtttatt tcataacttt ttttctagcc ttcatatctt 60
gtgttttcag gttgtcacaa tattctttta aaaattaagc attcttacgg cttcactcat 120
gtgcaacatt tataattatt tgcatttgcc ccctcaatga tctcaataga ataaatcagg 180
ctccactata ctcatttcac aaagacacat tcattacaaa ggataaagga ctgaaatatt 240
tgttttgcaa tctgttgacc taagtaggaa taggaagcac agtttcagtg cttccaagtt 300
tttaacccct gactgagacg ttttggttga gtattactat tcttattcta ccaatgataa 360
agggaaactg aatgcccaac catgtgctgg ctgtttacac atatgcaaca ttgactggtt 420
ctcacaacca ccttgaggaa taggcattgn cttcaattta caaatgagga aaacaaccat 480
tttcaangng cattttnc 498

<210> 103
<211> 697
<212> DNA
<213> Homo sapiens

<220>
<221> misc_feature
<222> (1)...(697)
<223> n = A,T,C or G

<400> 103
gnnatctgaa attcgccttt cnagcggcgc cgggcaggac taaaaatgta agtttatttt 60
gccatacccc taacaacatt ttatttaaat tatattgtga cttgattaca aatcttttaa 120
atgacattat tggcatattt ttcttaaact ttgtaagaaa aagataacat ttcacatttt 180
agtagcaaaa tcattgttaa gagatagtca attttgtgaa aatatttgag tgctaatcaa 240
tttttccagg atgatcttct atcctttaat atttagatct tccttttgaa gcacttacat 300
catcatcaaa tttttggtca tttgntgngn catctaattt ctggttcatt ttctaatggc 360
ttcgtatgtg aatgaatttt agttattcct aacgtcattg gtagccactc ttttgaaatt 420
tttttttaaa ccaggctttc aattttaatt tatanggaat ttgcattggg atatagatga 480
ccgctcaaaa ttcccatgng agactgntga aatgncctaa acnattcgcc tggacnctgg 540
attaanccgn ggcctcttaa ggtaatctng anggggtggc ttattgggaa aatttggatt 600
nnggcccggt tactntgcca ggttngactt nnaagggccc anaaggacct nggaaatnaa 660

gatnccctna acccttcctt ggnaaanaaa naagttn 697

<210> 104
<211> 504
<212> DNA
<213> Homo sapiens

<220>
<221> misc_feature
<222> (1)...(504)
<223> n = A,T,C or G

<400> 104
accatcattc agaataactc ttccaatttc tgctttcaga catgctgcag gtcctcatct 60
gaactgttgg gttcgttttt tgtttttttt cctgctccaa gaaagtgact tcaaaaataa 120
ctgatcagga tagattattt tattttactt tttaacactc cttctcccct tttcccactg 180
aaccaaaaag aaatcccatc cctaaaacct gccttctcct tttatgcaaa actgaaaatg 240
gcaatacatt attatagcca taatggtata gatagtgatt gcgtttggct atgtgttgtt 300
ttcttttttt ttaaattatg aatatgtgta aaatctgagg taacttgcta accgtgaatg 360
gtcatataac tttaaagata tatttataat tatttaatga catttggacc cttgaaacat 420
ttcttagtgn attgatatgt tgactttcgg tctctaaaag tgctctttat taaaataaca 480
aatttcttta aagggnctaa aanc 504

<210> 105
<211> 746
<212> DNA
<213> Homo sapiens

<220>
<221> misc_feature
<222> (1)...(746)
<223> n = A,T,C or G

<400> 105
ggtactaggt gtctcataat tgaaccctct atccacatgt gcggctttta gctgactatg 60
tctttgctat gaagcctggc gatttagagt tttgcttaac tatgaaacca cagaacattt 120
ttctgtagtt caatgattta cttgtgcttg tctttttaat atgacaagag tcataattac 180
cccaaagaaa ttagaaaacc acatcactcc agcatttcat gctgataaag ggctaaaggt 240
tgttttttaa atccctaatt accgctttag aaggcaaagc tgtgttagag gcattcaaag 300
atctgaaaga actaaacata acatttcctt catacatcac aaaaacaatc tatatctaaa 360
atatttggag aagggaagta ttttttaaaa tcacattgng ccctggatga acctggaaat 420
ggcttancca tatttcaaga atatggntct aggacccact ggaaggaaaa tttgggtaat 480
ttaaataaaa ganccccttt ttaggaggan ccgaaagtcc aaccttattc aattcccctt 540
angaaaatng tttcaagggg gtcccnaaag ggccatttaa antaattttt taaaatatta 600
tcctttaaag ggtttttttg ganccenttn nccggttgnc caaggtttnc ccttcgnaat 660
ttttncccct ttttccctaa antttaaaaa aaannggnaa acccccccct ttgnccaaag 720
cccatncctn ttttttacc ccttng 746

<210> 106
<211> 645
<212> DNA
<213> Homo sapiens

<220>
<221> misc_feature
<222> (1)...(645)
<223> n = A,T,C or G

<400> 106
acaagctttt tttttttttt ttttttttga gatggagtct cacattgttg cctgggctgg 60
agtgcagtgg cacgatctcg gctcccgggt tcacgtggtt ctcctgcctc agcctcccag 120
gtagccggga ttacaggtgc ccaccaccat gcccagataa ttttttatat ttttagtaga 180
gacggggttt taccatgttg gccagactgg tctcaaactc ctgacctcat gatccgcctg 240
cctcaacctn ccaaactgct gggattacag gcgtgagcca ccacacccgg ctgagttgtt 300
gattttttag tttgntcagc tttttacttg gtagaatgaa gtgatgactg ncgacctcct 360
taagggccag actagaaact gggagtctcc tatttangnc gccttaaaaa ttgnaagctn 420
gacattggtg gtgaagcatt ggaacaattc ttaattctgg tacctganan gggtgaattt 480
tggtttcact ngcngcttat cagtantcaa ttccttgaac ttttaaaacn ttagttaccc 540
ttngtaggga cagnnttcaa attttccttg acttagggaa cccttantct ngggacaagt 600
tttattctaa ctgactgttg caaacttang gcttcntacc tggcc 645

<210> 107
<211> 684
<212> DNA
<213> Homo sapiens

<400> 107
acagccagat cttaagatga gtctgtgtca aaatgacctg aacgcaagtc tgtattcttg 60
cagagtaaca gagtgttcgt ctgtttctgt ctaaaagtca taactataca gatatctggg 120
aatgcttgca tgaagctttt actcccgaga gcatactact acttacggtt ataacttgtt 180
gatgtctata ttggcttaat tcaaatgaaa agttcactcc aggagcagct ctttgtaatc 240
cacaccaccc cccagactgt tctgaataaa cccagaacaa ctcatacacc agcctaagca 300
tggtctattt ttctgggatg ggacagaaca taattgtatt aaaatataaa atcagtttta 360
aaaggtctgg aaggacatat cttaaggcca tgatagtaag tacagctggg gtgctgggga 420
ggggacctca actagggttg gtggcaaaaa tgggactttt aactttggct ttaacatcct 480
ggtcctaaaa agaagactag atttacctat tatatatgca atctaaaatt aattcaaaaa 540
gtcatcagcg aggacccccc taagattctg ggtggtaagt ccaccaaagg ccaagagcta 600
aaacaaaagc cttttccaca tgttctgaga agttggccca aaactgctga atctataggt 660
cttagcatgc tctatctatg tacc 684

<210> 108
<211> 236
<212> DNA
<213> Homo sapiens

<400> 108
ggtacacgtc gttctcttca agatctcata gacaatcgtg ctccgggttt tgctgtcgaa 60
aaaggaatcc ttatcagaca agtcaaatag atgctgcttc tcccgggaga agggatagga 120
gagtctcttc atggtctggg gcctgtgctc agccactttg ggctggatgg gatctgtgat 180
tttctggagc acagagttga tttttttcag gaggccacgg gtctcattaa tgtggt 236

<210> 109
<211> 497
<212> DNA
<213> Homo sapiens

<220>
<221> misc_feature
<222> (1)...(497)
<223> n = A,T,C or G

<400> 109
acgagaagtg tggtgctgga atatctttcc ggtgaggcct caagaagttt acagtcacgg 60
tggaaggcaa tgaggagcca gcatatcaca tggtgacagc aacagccaga gcaaaagagg 120
gagggagagg tgccactcac acttaaacaa ccagatctgg tgtgaactga ctcatcacca 180
aggggatggc actaacccat tcatgaggga tctgccccca tcatccagac acctcccacc 240
aggcctcatc tccaacactg gggattacat ttcatcatga gatttggagc ggacaaacat 300
ccaaaccata tcagtaggat gtctgacatt catcatacga tgtctgagtg aagggaggtt 360
taagggctta ttttgtctcc ctggatagta atggaaaatg tatatctgaa agagatgtct 420
gaaaaagaaa gtttaagtgg gtggcttgca cacttttggt ttgctagngg gctttttgag 480
ctcanattct catttgn 497

<210> 110
<211> 722
<212> DNA
<213> Homo sapiens

<220>
<221> misc_feature
<222> (1)...(722)
<223> n = A,T,C or G

<400> 110
ggtacagccg gtcctcttct tccaggaatt ggctactgtc cctctgcaat cccattcatg 60
ataaaagcat tcttatacaa cacaaaagat gctgcatcaa tgattctcaa acctccaaga 120
catccaaatc aactagcatg cttaagatgc agattcctgt gctcgactca ccaacttcca 180
gaattttcca ttccctaggt ctgaggtgaa cctgggaatc tgccttgcta acaaatgatg 240
ctgacactgt tgatttgggg accccacttg gagaacctgg gctctagatc tctaccctct 300
tactgaagtc ttcttccact tcctgcttta actggaatcc aacccgccac ccctgnagcc 360
cttgcaaagt gaattgccct tttcccttac tctggttttt tctcctctgg ttctagccta 420
gattccangg aacatnaact ttgggcntgg cattttcccc tngatntggg atccttttgg 480
nccagntttt ccccaaagna agccntnaat tcaaaatctt tccccnttng gttcctattn 540
acccggacct tcngggggna aaaaatnccc aaaagccccc ttacnaaatc cctttttccc 600
aaacttcaat tgggaaactn gggctttaaa aaagnccccn tttnccaaan ccnaaaantg 660
ggcctaaccc ccccccnttn aaactttntt ttttnnanaa attntttttn anaaattncc 720
tt 722

<210> 111
<211> 614
<212> DNA
<213> Homo sapiens

<400> 111
accagggctc tcacttccaa atagactatt taattgtttt gatacattct caaaaactgt 60
caagggctcc aaggcatcca aagcttcaag gtatttgttc acaaacccaa ccctgtttgc 120
ttgaatatga actgtcctaa tttctagccc ggtcttccat ttccacaagt ctgtgacttt 180
gttcctattg ttatctctgt aaggctatcc tctcctttgc ttttaaactt ttactcagaa 240
ttcatgagcc aacttgaata tcactttctc catggaattt ttatgagttc tccaacataa 300
ttcaatgacc aggtcagttt ttgatccagg acagcatttc ctgtgaatgt ggtggatgtt 360

atatatcact caatgcattt tctcattacc ctggggaatc aataaattgg agtttcttaa 420
tctgcagaac tgaggaccaa tagctttaaa atgtgtgccc atgaatctgt tccaagaccc 480
aagatgaaat ttcagccctc atccaccctc atataaatga caaaatatta tgtgggatcc 540
ctgtaacaac tgaattttaa aatgctagga ttatcccttc cctagcacta tgtcattttt 600
aaaggtgtac ctcg 614

<210> 112
<211> 499
<212> DNA
<213> Homo sapiens

<220>
<221> misc_feature
<222> (1)...(499)
<223> n = A,T,C or G

<400> 112
acttttctgg aaattggctt taagagctca tcctgcattt ttaaaatctc tccaactgga 60
tcaaattttt tatatactcg tttgataggt ttttttaaaa cacatgactc ttcaggacta 120
caagcagtat tagtctggtt tcctacagaa gcctgtcctg aggaagaatt tggactagct 180
ggtctggaac ttaagttaga acccacaaca gctgtctttc catcactatt atttttacat 240
tctgtatcaa tgattaaaca ctcctcatct gtatcactgc tgcagagaac tgtaccttca 300
gtttttgctg cttctgatcc aacagtcttt tcctttgagt tgtctaggtt ttctagaaca 360
ttaggtcttt caccatcagc atgtaatata tctatagtca tatcattttt attagaagtt 420
tcaatttcct gagaatttct aactggaagg catcagatgt tttcaaggca ctatcttgga 480
tcaaangctt ggcaaaaaa 499

<210> 113
<211> 697
<212> DNA
<213> Homo sapiens

<220>
<221> misc_feature
<222> (1)...(697)
<223> n = A,T,C or G

<400> 113
   gcgtggcgcg gcccgaggta cctaacatga cagatgctcc tacagccccc aaagcaggaa 60
   ctacaactgt ggcaccaagt gcaccagaca tttctgctaa ttctagaagt ttatctcaga 120
   ttctgatgga acaattgcaa aaggagaaac agctggtcac tggtatggat ggtggccctg 180
   aggaatgcaa aaataaagat gatcagggat ttgaatcatg tgaaaaggta tcaaattctg 240
   acaagccttt gatacaagat agtgacttga aaacatctga tgccttacag ttagaaaatt 300
   ctcaggaaat tgaaacttct aataaaaatg atatgactat agatatatta catgctgatg 360
   gtgaaagacc taatgttcta gaaaacctag acaactcaaa gggaaaagac tgttggatna 420
   gaagcagcaa aaacctggaa ggtccagttc tctgcacant ggatncccan tgaanggaag 480
   tggtttaaat caattggttc ccggaatggt aaaaaattaa ttagtggatg ggaaaagacc 540
   agcttgttgg nggggttctn aacttaaagt ttcnanacca nnntangtcc naattttttc 600
   cttnagggaa agggcttttn tnggnaaacc gncttaaaac gggttngnan cccctaanaa 660
   ntcttggngt ttaaaaaaaa cctttttanc cgngttt 697

<210> 114
<211> 497

<212> DNA
<213> Homo sapiens

<220>
<221> misc_feature
<222> (1)...(497)
<223> n = A,T,C or G

<400> 114
acccacttct gacatctgga ccacttcttg cagtcattgg gggtcatccc ccacactggt 60
aacctgtcat caaatgggcc acagcaacat tcagcttaag tatttctcct tcccacatcc 120
aagggattga gtgggagtga gattgggggg tggaaaaaac agtgaacagt cctggtgagt 180
tgcagatgtg gtctcattcc ctagagatgc aggatgcagc tgacctgaat caggacagat 240
ccctgcagga gggactcctg gtgccatgtc agtcccacct ggcactgccc tagctcccag 300
gctccgcctc tgcatctttc cttgctactt cctctttcac ttctcccccg ttcccagacc 360
caccagacag agcttccaga gtgtcaggac atgtgtgact tagcccagat tcagacttta 420
gtcacaagca ggatcaagca tanacatcta acttccagca tgggcaattc tctggtgggg 480
ctccctgnnt ggantgg 497

<210> 115
<211> 687
<212> DNA
<213> Homo sapiens

<220>
<221> misc_feature
<222> (1)...(687)
<223> n = A,T,C or G

<400> 115
ggtactatgt gtgaagaaat ggagaaaagg aaaaatcang tgtagaaaaa taagaaaaag 60
caagagtgag gttggtgcct acagttcaca gcatgtgata aggactgagc atttattcta 120
ttatttggtc ataaaaatgc aggctgtaag ggcctacaca caccagctta tcgnagactt 180
ggctctgagc tttcctgcag ccaatacaaa cagggagaca cancagagaa ttgccatgct 240
gggagctaga tgtctatgct gatcctgctt gtgactaaag tctgaatctg ggctaagtca 300
cacatgtnct gacactctgg aangctctng ctggtgggtc tgggaacggg ggagaagtga 360
aagatgaagt agctagggaa nagatgcaga ggctgnncct tgggaactta ggcaagtgcc 420
aggtggggac tgaccatggt anccaggaat tccnttcctg gtangggatt ctggtcctng 480
aattcagggt taagcttgcc attcctgcat ttcttntagg ggganttgan aacccccttt 540
ttggaaactt cancaaggan ttggtctccc nggntttttc ccccccccta aattnaattc 600
cccnttaatn cctttgaatt cnggnaaggg nnaattcttt ancctaantg ttcttggggc 660
nctatttggt ngacagggtt ncnangg 687

<210> 116
<211> 508
<212> DNA
<213> Homo sapiens

<220>
<221> misc_feature
<222> (1)...(508)
<223> n = A,T,C or G

<400> 116
ggtacccatt ttctatttca agtagattaa ccccttatat tctgctaaaa tcatacttgt 60
tgcctaacac ccagttaaca aagcaaaaaa aaatcagtta atttataaaa acaaaatgct 120
aattcttatt ctatgtgaat gtatttcata gattttaagg ggttaatcac caattagaag 180
acatgctgtg tccacactat tttaagatta aacgttaatg ggaatatatt aattcaaatt 240
aacatggtca tgtaaaatat ataacccact caaccattta aaaactagtg tgaacactgc 300
tcaattctag aagagacaaa gacaaaacaa acaaaacagc cacacaaagg acaataaatg 360
ccaggctctg catccaaaat ccctccttta tcaaatggca gatgtgacac tgagcttttg 420
aaaaccttgg ncaaaaatcc ttccgatgtc ttggcagcaa cccctggcag gatcaatccc 480
ctctgntata aagntttggg cccngccc 508

<210> 117
<211> 644
<212> DNA
<213> Homo sapiens

<220>
<221> misc_feature
<222> (1)...(644)
<223> n = A,T,C or G

<400> 117
acaggggtta aggaaggctt tgccggaaga acaattgtaa atcatgagag ttactacttg 60
cgcattgtgt ggtagtctct ttaatgcata atggtccttt ttaataccaa aaattaatta 120
ataaaggaaa tgattacatt gtccaaataa ctgttaaaca catgacagat ctgttttatg 180
atactgtgtt tgacagttaa acattaagta aacatttaat tgactttaag cttgaaatgt 240
tcagaatgct ctaacccttg ctacagaatc ttttctgcag caagttaagt attttgtgtg 300
ttttttccca cctgtagctt atcaggcccg gtccaaagcc ttctagcaga ggggattgat 360
cctgtcaggg gttgctgcca agacatcgga aggatttttg accaaggntt tcaaaagctc 420
aatgncacat ctggcatttt gataaaagga gggattttgg atccaaagcn tggcnttatt 480
ggccttttgg gtggctggtt agggtggntt tggctttngc cttttcttaa aaattaacca 540
nggttnccac ttantttttt aaaagggtga atggggtaaa atttttccnt ggaccnngta 600
aattgnaata aaaattcccc tttaccgtta aacttaaaan angg 644

<210> 118
<211> 500
<212> DNA
<213> Homo sapiens

<220>
<221> misc_feature
<222> (1)...(500)
<223> n = A,T,C or G

<400> 118
ggtacaaacc catgcagcct ggccctcacg tggtcaagat cttctttgct ggggacacta 60
ttcctaagag tcccttcgtt gtgcaggttg gggaagcctg caatccaaat gcctgccggg 120
ccagtggccg aggcctacaa cccaaaggcg tccgtatccg ggagaccaca gatttcaagg 180
ttgacaccaa agctgcagga agtggggagc tcggtgtaac catgaagggt cctaagggtc 240
tggaggagct ggtgaagcag aaagactttc tggatggggt ctacgcattc gagtattacc 300
ccagcacccc ggggagatac agcattgcca tcacatgggg gggacaccac attccaaaga 360
gcccctttga agttcaagtt ggccctgaag cgggtatgca gaaagtccgt gcttggggcc 420
ctgggctcca tggtgggatt gtcnggcggt caacngactt cgtggnanaa tccattggct 480

ctgaaatnng gnctctgggg 500

<210> 119
<211> 624
<212> DNA
<213> Homo sapiens

<220>
<221> misc_feature
<222> (1)...(624)
<223> n = A,T,C or G

<400> 119
actcaatctt tgcctgagag gggccttcaa tggcaaaccc cagagacccc acttcagagc 60
caatggattc taccacgaag tctgctgacc gcccgacaat cccaccatgg agcccagggc 120
cccaagcacg gactttctgc atacccgctt cagggccaac ttgaacttca aaggggctct 180
ttggaatgtg gtgtcccccc catgtgatgg caatgctgta tctccccggg gtgctggggt 240
aatactcgaa tgcgtagacc ccatccagaa agtctttctg cttcaccagc tcctccagac 300
ccttaggacc cttcatggtt acaccgagct ccccacttcc tgcagctttg gtgtcaacct 360
tgaaatctgt ggtctcccgg ataccgaccg cctttgggtt gtaggcctcg gccactggcc 420
cggcaggcat ttggatgcan gctttcccaa cctgcacaac gaanggactt ttangaatag 480
tggncccagc aaagaaaatc ttgaccacnt tganggccca gctngatggg tttggacctt 540
tggccggaac acccttangg ccaantccng canttggggg ccgtacttag ggaccaactt 600
ggnnccaact ttggngaata tggn 624

<210> 120
<211> 504
<212> DNA
<213> Homo sapiens

<220>
<221> misc_feature
<222> (1)...(504)
<223> n = A,T,C or G

<400> 120
acaggcatgg caccgacatc tgcttggctt ctgctgtagc ctcaggaagc ttatagtcgt 60
ggcagaaggc aaagagggac ggcaagagag gaagcaagag agagagcgag gaggtctcag 120
actctcttta ataatcagat ctcctgataa ctcatttcca tggggagggc accattcatg 180
agggatccgc tcccatgacc caaacagccc ccaccgggcc ccactgtcaa cactgaggat 240
cacatttcaa catgaaatgt ggaggggaca gacatccaaa ctatatcacc tccatactgt 300
tttccacagc attcccacca acagtgcaca ggggtttcag tgtctccaca tcctcatcac 360
acttgttatc ttctgttttt gtttgtttgt ttgtttgttt tttatagtag ccattctcat 420
gantgtgaag tattaacagt gtcttttgaa gatcagaaat ttctaatttg atgaaagtcc 480
ngnttancan ntttttcnt tttn 504

<210> 121
<211> 630
<212> DNA
<213> Homo sapiens

<220>
<221> misc_feature

<222> (1)...(630)
<223> n = A,T,C or G

<400> 121
ggtactatcc taagtttaac actgcttcac agtaaggaaa gccgatcaaa atttaaggag 60
agattagaat ccagaaatag gcccacacat atatatagtc attgattttt aataaaggtt 120
caaaggcaaa acaatgaaga aaggatggtc ttttcaataa atgatgcaga aacaactgga 180
catccacgta tgcaaataaa ctttaatcca tgccttttac tttatccaaa agctaatcca 240
aaatagaaac ctccctttcc tccctcaaaa aagcttctag agaaaacaca ggagaaaatc 300
tttgtaacct tgggttcaca aagatttctc aggtatgaca ccataagtat gatccagaaa 360
agaaaaaaaa tgataaactg gacttcatca aattagaaat ttctggatct tcaaaagaca 420
ctgntaatac ctcacactca tgagaatggc tactataaaa acnaannanc caaccaacca 480
ataacngaag attncaggtt gatgangntt ggagacnctg aanccctgng cactgttggt 540
gggaatnntt ntggaaaaca gttggangng aattagntng gngnntngcc cttccanttc 600
atgggnaagg gacctnagnn tgancgnggg 630

<210> 122
<211> 431
<212> DNA
<213> Homo sapiens

<220>
<221> misc_feature
<222> (1)...(431)
<223> n = A,T,C or G

<400> 122
actgaaaagc ttggtcataa tcttcctgaa catggaatga tctagctagc tgatagcagc 60
tctctgcttg catagcttcc acttctgtat tatggaatgc atggagggcc agatgctgga 120
ctttactata atcctttttg aagaaaaagt gatttgccaa atggttcaat accatagggt 180
tgctaggatc aatagtatag gctctggaaa gaagctggac accattttta atggaatcag 240
cctctttatt gttgagttct agaacagcca gtccaaccaa tgctcccacg catttggaat 300
tgagttccag ggctctgctg aatgccagac gagctttttc cagtttgtta agtttcacaa 360
agcaatgacc cattcctaaa cnaacttccg ctggacattc ctgggttaag tacctnnggc 420
cgngaccacg c 431

<210> 123
<211> 504
<212> DNA
<213> Homo sapiens

<220>
<221> misc_feature
<222> (1)...(504)
<223> n = A,T,C or G

<400> 123
actggctgtc ctctgaggca ccttggtgtc ttttccacaa tggtttattt tcctccagta 60
ggctagactg gcttccttat ttggcagttt cagggcagca tttcaaaagc aggaaggtgg 120
aagtggcaag gccccttgag gccctttctt cagagctcac acagtgtcac ctttaccaca 180
ttctattggt caaagcaact tccaggccag ccaaaattca aagggtgagg tagtagactc 240
tacctctttt ttcttttgag acagaattgc gctctattgc ccactctgga gtgcagtagc 300
agcctcatgg ctcactgcag cctcaacctc ctgggctcaa gcgatccttc catctcagcc 360

tcccgagtag ctaggaccac aggcacatac caccacagtc agctaattaa aacatttttt 420
ttggtagaag atgggttctc acttttttgc ccaagctgat catgaactcc tggccacntt 480
ngggcntttc aaggggnaac cccc 504

<210> 124
<211> 632
<212> DNA
<213> Homo sapiens

<220>
<221> misc_feature
<222> (1)...(632)
<223> n = A,T,C or G

<400> 124
ggtacaaaca cagtaaagaa caacacagat accagtcctg cctttatcag gaaagacaaa 60
acaaaaacaa aaagtaaaca ttccagtaaa ggaatgatta gtgctattat gacaaggaaa 120
gcatagggaa ctattcgatc aaagaagaga ggttacagtt ccccaaatct agggtgtttg 180
gaaaggaaga atatccttag taaatgacat tgaagctaaa acctaaacta tgtatagcag 240
tcagctagaa aaaacaggca agaaagaata tttcaggtgg agagaaacac atgttttcag 300
gccaaaagct ggagaacaag gtgagtttaa agaactgana gaggtttagt gattacaatn 360
gttgaacaaa aggggggcat tgtggaatga atannaaaga ntggttttgt anattggaat 420
ctctgcagca aaactccatt cagaaggtat aagttcangc cttggtgggt tactttggna 480
aggccgtagt gggccaggag nttcatgntn cancttgggc caaaaagnng agaacccatt 540
ttttccaaaa anaatgnttt naatttacct ncntgggggg ggaatgnncn tngggtcctt 600
anttctttgg aanggtttaa attgnaaggt nc 632

<210> 125
<211> 496
<212> DNA
<213> Homo sapiens

<220>
<221> misc_feature
<222> (1)...(496)
<223> n = A,T,C or G

<400> 125
acaagattag gaggggggaa aaacctgaac aaatcctgga acacacctat gtatttacgt 60
catgggaaaa ggggagagaa cacttcaaat atcaacaagt tctgcgccat taactcatta 120
atagctaaat ggccacacca aattgcatgt gaatgttaga acctctcaga tagccacaat 180
aagtccatat ttttttttaa aaaaaggaaa acacagaaat aactaccaac agtgtctgag 240
aagagagact aagttaacat acattgcatg tattgcaggc aaggcagagg cattttttta 300
aagcttttgc acagacttca tataatctta aaaaaaatat gcaggccttt gcaagatttg 360
acttgctgaa atccaaacaa ttttgactca tgaaaagtca taagacttca gctgaaaaaa 420
aagaaaaaag ttccagcctt agaccaaaaa aaaaaacctg gaanagtntg atagatttaa 480
cnanggtngg cacgct 496

<210> 126
<211> 631
<212> DNA
<213> Homo sapiens

<220>
<221> misc_feature
<222> (1)...(631)
<223> n = A,T,C or G

<400> 126
ggtacacctt gttaccaaat aggttgttct cttccccacc cacctttgag cttttgctct 60
aaaatacatt caggttccaa gcctgaccat ccttgtttaa tctatcatac tcttccaggt 120
tttttttttt ggtctaaggc tggaactttt ttcttttttt tcagctgaag tcttatgact 180
tttcatgagt caaaattgtt tggatttcag caagtcaaat cttgcaaagg cctgcatatt 240
ttttttaaga ttatatgaag tctgtgcaaa agctttaaaa aaatgcctct gccttgcctg 300
caatacatgc aatgtatgtt aacttaagtc tctcttctca gacactgttg gtagttattt 360
ctgtgttttc cttttttaaa aaaaaatatg gacttattgt ggctatctga gagggtctaa 420
cattcacatg ccaatttggg ggtggncatt taactattaa tggagttaat gggcccaaaa 480
cttggtgata ttttnaaggg gtctcttccc ntttttccaa tgccgtaant cntttngggg 540
tggttccagg aatttgntcc aggntttttc ccccncctaa aatnttgaac cttgnccngg 600
cnggnccttt caaagggcna attnnanccn t 631

<210> 127
<211> 518
<212> DNA
<213> Homo sapiens

<400> 127
caggtactcg gtgcttccca acacctcctt attggaaaac agccaaggag atggtggcta 60
actggaggca tcacccagca gtggtggagc agtggagcaa ggtcatttgt gcactcactt 120
ccagattgct acgctttaca tatggtcctt catttcctgc atttaaagtt cccgatgaag 180
atgccagtct gatccctcca gaaatggata atgagtgtgt tgcacagaca tggtttcgct 240
ttttacacat gttaagtaat cctgtggatt tgagtaaccc agctattata agctctactc 300
ccaaatttca ggaacagttc ttgaatgtga gcggaatgcc gcaagaattg aatcagtatc 360
cctgccttaa acatctgcct caaatatttt ttcgtgccat gcgtggaatc agctgtctgg 420
tggatgcatt cttaggtatt tctagacccc gatcagacag tgctccccca acacccgtga 480
atagattaag tatgcctcaa agtgctgctg tcagtacc 518

<210> 128
<211> 865
<212> DNA
<213> Homo sapiens

<220>
<221> misc_feature
<222> (1)...(865)
<223> n = A,T,C or G

<400> 128
accaaaggat agctgttctg tttaagtagg gacctctcat ggcctacagg ctttgacatc 60
tgagaatcaa actggagaac attccgaagc cgttcttata agtgtctcca tctctacctg 120
ggctgaaatg gaatgtgcaa atgtagccca gcctggtcct tgggtgttgc cagttgattg 180
atgactggga gccaaagtgg catctccttt gacctaaacg ggcgatgatg aaataaaact 240
caacagcctt tctctcatct tgcattgtga gatgcgaaat agagcgtgtc tctctgcctc 300
tcattttagg ctgaggccgt ccaaagcggc catgccccat gtttccacta gatggcgctg 360
acacttcagg catcaaccct catggcctct cagccttgca aaggcagcca cttaaagtcg 420
gtgtcctgtg tggggcacca agctgagctg cagacaccca gtaggcgcga ggcaaatgcg 480

tcccatttta agaggcttgt atttatgagc tctttgcttc ctccctccca ctatctttaa 540
agaattgctc tccatctcct ttggcaaagt tcctttgccc tttgncttat ttttgtgaaa 600
cccttcaagg tatttccagt ccatttgcat ccaatctggc atctttacng aanagcggtc 660
tcatatgcta ttggtggtaa cgtgggacta gtatttatgn ggttgagaac cacttggctg 720
tttgtcaagg aaaagtgtgc ccaaaaacca agaagtacct ttggccgnga accacgctta 780
aggccgaaat tctgnagata tncnntcaca cttggcgggc cggttcgaac cttgcatnta 840
aanggnccca atttggccct tatag 865

<210> 129
<211> 910
<212> DNA
<213> Homo sapiens

<220>
<221> misc_feature
<222> (1)...(910)
<223> n = A,T,C or G

<400> 129
tactctttgt tttggcacac ttttcctgac aaacagccag tgttctcaac acataaatac 60
tagtccacgt taacaacaat agcatatgag accgctctcc gtaaagatgc cagattggat 120
gcaaatggac tggaaatacc ttggagggtt tcacaaaaat aagacaaagg gcaaaggaac 180
tttgccaaag gagatggaga gcaattcttt aaagatagtg ggagggagga agcaaagagc 240
tcataaatac aagcctctta aaatgggacg catttgcctc gcgcctactg ggtgtctgca 300
gctcagcttg gtgccccaca caggacaccg actttaagtg gctgcctttg caaggctgag 360
aggccatgag ggttgatgcc tgaagtgtca gcgccatcta gtggaaacat ggggcatggc 420
cgctttggac ggcctcagcc taaaatgaga ggcagagaga cacgctctat ttcgcatctc 480
acaatgcaag atgagagaaa ggctgttgag ttttatttca tcatcgcccg tttaggtcaa 540
aggagatgcc actttggctc ccagtcatca atcaactggc aacacccaag gaccaggctg 600
ggctacattt gcacattcca tttcagccca ggtagagatg gagaccttat aagaacngct 660
tcngaatggt ctncagtttt gaatctcaga tgtcaaaagc ctgtaagncc atgaaaggtc 720
cctacttaaa ccggaaccag ctatcctttg gnanctggcc gggccgggcc ggttcgaaaa 780
gggcgaaatt ccacaccact tgggcggccc gttacttaan ggaatcccga actttggnan 840
cccaagcntt ggcggtaaat catgggccat anctgggttt cctggggggg aaaatggtat 900
tcccttccca 910

<210> 130
<211> 932
<212> DNA
<213> Homo sapiens

<220>
<221> misc_feature
<222> (1)...(932)
<223> n = A,T,C or G

<400> 130
taccgcttgt ttatccaaat tttcctctgc aagtggagca tctgctagga tcaatagcag 60
cagtgttaag caggaagcta cattctgttc ccaaagggat ggcgatacct ctttgaataa 120
agccctatcc tcaagtgctg atgatgcgtc tttggttaat gcctcaattt ccagctctgt 180
gaaagctact tctccagtga aatctactac atctatcact gatgctaaaa gttgtgaggg 240
acaaaatcct gagctacttc caaaaactcc tattagtcct ctgaaaacgg gggtatcgaa 300
accaattgtg aagtcaactt tatcccagac agttccatcc aagggagaat taagtagaga 360

aatttgtctg caatctcaat ctaaagacaa atctacgaca ccaggaggaa caggaattaa 420
gcctttcctg gaacgctttg gagagcgttg tcaagaacat agcaaagaaa gtccagctcg 480
tagcacaccc cacagaaccc ccattattac tccaaatcaa aggccatcca agaaagatta 540
ttcaagcaag acacatcttc atctactacc catttagcac aacagctcaa gcaggaaccg 600
tcaaaaagaa ctagcatgtc ttcgtggccc gatttgacaa gggcaatatt atggaggtgc 660
agaaaaaggc nggaaactca aaaagcnaac cacctnggaa anccaaacng ggaaaacttc 720
acttgtcaag agcactcccc ttnaaaaaaa ccnccccaag ggggtttnca aaaactcagt 780
cccnttccgg taaccngaaa aagggggacc cgaaaacccc cganacccng gcccaaaaat 840
tntaggacct tgccccggcg ggcccgntnc aaaangggcg aaacttttgg gaaaatccat 900
tnnncctngg cggggcnggt tttgaccatt cn 932

<210> 131
<211> 890
<212> DNA
<213> Homo sapiens

<220>
<221> misc_feature
<222> (1)...(890)
<223> n = A,T,C or G

<400> 131
actagaattt ttggctggta tctggttttc ggtcaccttt tctgttactg gaagtgactg 60
agtttttgaa acaccttggt gttttttgag gggagtgctc tgacagtgag tttcctgttt 120
ggtttctagt tgtttgcttt ttgagtttcc gcctttttct gcactccata tattgccctt 180
gtcaaatcgg ccacgaagac atgctagttc tttttgacgt tcctgcttga gctgttgtgc 240
taaatgggta gtagatgaag atgtgtcttg cttgaataat ctttcttgga tggcctttgt 300
atttggagta ataatggggg ttctgtgggg tgtgctacga gctggacttt ctttgctatg 360
ttcttgacaa cgctctccaa agcgttccag gaaaggctta attcctgttc ctcctggtgt 420
cgtagatttg tctttagatt gagattgcag acaaatttct ctacttaatt ctcccttgga 480
tggaactgtc tgggataaag ttgacttcac aattggtttc gatacccccg ttttcagagg 540
actaatagga gtttttggaa gtagctcagg attttgccct cacaactttt agcatcagtg 600
atagatgtag tagatttcac tggagaagta gctttcacag agctggaaat tgaggcatta 660
accaaagacg catcatcaag cacttgagga tagggcttta ttcaaagagg tatcggcatc 720
cctttgggga accagaatgg aagcttnctg cttaacactg ntgctatgga cctanccana 780
agctccactt tgcanangga aaatttggat aaaccagccg ganccttggc cgggaancac 840
gcttanggcc gaattccnca cacctgggcg gncggttacc taagggaacc 890

<210> 132
<211> 606
<212> DNA
<213> Homo sapiens

<220>
<221> misc_feature
<222> (1)...(606)
<223> n = A,T,C or G

<400> 132
actcaggcac ttcacagttt acttgaaaga ggctttggaa aatagataaa gtgaaagaag 60
aataaataca tatttttaat aatgtaattt taaaaatcct ttataatcag gactaagtct 120
tggtttgcag aagctgtcac ttaccctgaa acacagtatc aaaagggaaa cttaaaacat 180
actgtttgat ttttttattt cctcttacaa tccatgtttt caggtagaat tatgactttc 240

cccccattgt tacacatttc tttacaaagg aggcctgtag aaattggaca cgatcatgct 300
tgagcatgtg agttagtcaa attatgagtc cctgcctatt gtccattaca caccgaatgt 360
taatttaaga accagaggca gaagttctgg cttcctgctt gaaacccaat tcttatatga 420
aaatttttaa aagccagaac ctagcagccc atctgntttt tctcttttgc cggngnattt 480
gganccttgg cgggaacacc cttanggggn aattcngnnc acttgggggc cggtacttan 540
ggganccaac tttgggccca annttgggga aancagggcn anattngtnc ctggggnaaa 600
tggtnn 606

<210> 133
<211> 606
<212> DNA
<213> Homo sapiens

<220>
<221> misc_feature
<222> (1)...(606)
<223> n = A,T,C or G

<400> 133
ggtacttttc cttaatcttc ttcttcttct tcttgtcacc atccttcttt tcttcttcct 60
catcagaacc aacatcttca atttcaggtt tgtcttccga ctctttctct tctttttctt 120
tttcttcttc tttgtcttcc ttttcttcag cctcatcatc gcttacttct ttatcacgtt 180
ccttctccac aaaaagagta atgggatatc caataaactg agaatgtttc ttcacaatct 240
cctttattct tcgttcctcc aagtacttta aatttagtgg ttgctggagc acctaaaagt 300
cagattgtca tgttggaagc ctctgcagag aacattttac agcaggactt ttgccatgct 360
atcaaagtgg gagtgaaata tacccaacaa ataattcagg gcattcagca gttggtaaaa 420
gaaactggtg ttaccaagag gcacctcaga aggtatttac cccttcgcag agaatgngaa 480
atatactcat aaacctgcta tggagagact ctatgcagtt ttacagatac gagcatgaca 540
aggttcngga gatgaagctg taccaaataa gatagatccn gnggaccact aaangaaaat 600
tccgag 606

<210> 134
<211> 598
<212> DNA
<213> Homo sapiens

<220>
<221> misc_feature
<222> (1)...(598)
<223> n = A,T,C or G

<400> 134
tacntcacca tcccgtattt gctgctgtnc canaaggcat ngncaaattg agggtcatac 60
tngatagcan cagggtaaac tgtggctcca atttcaaaac ttncctttat gaacatcatc 120
accgangtat tattgatgca ggntccttct gngaagatga ggataggcag ctngctttta 180
tcttgcacat gttcannnan nctnttagcc accanntggc natccttcac ttccgagcgc 240
tcaaaccaga cgtgtggncn ggccttcacc atggntctct gaatcacacc catgagtccc 300
ccgtgcactt gacccaccat ggcataatan ccatcgctgg ccaagatgat cacatcgatc 360
ggtgaggnat gattggccac acagatgcca ccatttcttg gtctgntttc cctgtcatgg 420
taggtgatga tggctgtcag cgctcgcacg cagatccggt aacacattaa ctgaacatgt 480
ttactcatga actccttaaa cctcccattt ggcangtatc ccaccacagn tgtgcccacc 540
accagaaggc taatccctgt gaaagccagt gctatcctga gcggcancag aaagcagt 598

<210> 135
<211> 617
<212> DNA
<213> Homo sapiens

<220>
<221> misc_feature
<222> (1)...(617)
<223> n = A,T,C or G

<400> 135
actgctttct gctgccgctc angatagcac tggctttcac agggattagc cttctggtgg 60
tgggcacaac tgtggnggga tacttgccaa atgggaggnt taaggagttc atgagtnaac 120
atgtncactt aatgtgttac cggatctgcg tgcgagcgct gacagccatc atcacctacc 180
atgacaggga aaacanacca agaaatggtg gcatctgngt ggccaancat acctcaccga 240
tcgatgtgat catcttggcc ancgatggct attatgccat ggtgngtcan gtgcacngcg 300
gactcatggg tgtgattnag agagccatgg ngaanngcct gcccacacgt ctggtttgag 360
cgctcggaag tgaatgatcg ncacctggtg gntaananac tgactganca tgtgcangat 420
aanngcnagc tggctatnct catcttccca ganggancct gcatcaatna tacatcgntg 480
atgatgttca aaaagggaag ttttgaactt ggagccacag tttaccctga tgctntcaag 540
tatgaccctg aatttgncga tgccttctgg aacagnagca aatncngtat ggngactanc 600
ctcggncgnn ancacgc 617

<210> 136
<211> 610
<212> DNA
<213> Homo sapiens

<220>
<221> misc_feature
<222> (1)...(610)
<223> n = A,T,C or G

<400> 136
cgtgccgtag gccggaatgt taccggctgt tggatctgcg gatgaggagg aggatcctgc 60
ggaggaggat tgtcctgaat tggttcccat tgagacgacg caaagcgagg aggaggaaaa 120
gtctggcctc ggcgccaaga tcccagtcac aattatcacc gggtatttag gtgctgggaa 180
gacaacactt ctgaactata ttttgacaga gcaacatagt aaaagagtag cggtcatttt 240
aaatgaattt ggggaaggaa gtgcgctgga gaaatcctta gctgtcagcc aaggtggaga 300
gctctatgaa gagtggctgg aacttagaaa cggttgcctc tgctgttnag tgaaggacag 360
tggccttaga gctattgaga atttgatcaa aagaaagggg aaatttnatt acatactggt 420
agagacnctg gattanccng accctggtgc cantggcttn tantgttttg ggttgaagct 480
tnaattaggg nnngtnttta acttggaggg ttnttacttt tgggggttca antttgggtt 540
aaacttttnn cnaaaaaaac cttgangcct tnttaatgan nnttttngca agttttttgc 600
canagccttt 610

<210> 137
<211> 645
<212> DNA
<213> Homo sapiens

<220>
<221> misc_feature

<222> (1)...(645)
<223> n = A,T,C or G

<400> 137
acaattccaa gtgcttatag ccaatataag catatttcat attagaaata gttatccata 60
tgttaacaag aaactatggt cctcaaatat gccaatttta gagtctaata actactgata 120
gtaactatgt aaatattttg gaataaacag ttatttacgc aagccacact tcagctgaga 180
tgatcactag acatctgttt ccagagcttc aacaatgtgt gcagcagaag gacgatcttt 240
agggtcttca ttagtgcata cagagaagag ttcaattact ttctggtatg attcatccag 300
ttcttccata ttaataggtg gcctagttcc caaggctgca tagtatgctt catcatcaaa 360
atcactttca tcaaaagttt tatcttcatc atcatcatca tttgaaagat taatgtgtgg 420
aaatccgata aaagtcatca tttcccacaa agtaagggcc aangccaaat atgtctggcc 480
tggccagtaa taacacccat tcttcttcac aggnttcttt tggggttnca atggnttctg 540
ggnccaatgg taaccaggnc ctaangggtc aggtcccggg cataattttc aatncccngg 600
gganaaaaag acctcctaaa nttnccagaa tttnaatngg ttcna 645

<210> 138
<211> 612
<212> DNA
<213> Homo sapiens

<220>
<221> misc_feature
<222> (1)...(612)
<223> n = A,T,C or G

<400> 138
ggtactcctg gtcacttaag atctgatact gaacattcta caaatgaagt tgggacttta 60
tgtcataaaa ctgatttaaa taatcttgaa atggccatta aggaagatca gattgcagat 120
aactttcaag gaatatcagg tcctaaagaa gacagcacaa gtataaaggt aattcagacc 180
aggattcttt tcttcatgag aattcgttac accaagaaga gagtcaaaaa gaaaatatgc 240
cttgtgggga aacagcagaa tttaaacaaa agcaaagtgt taacaaagga aaacaaggaa 300
aggagcaaaa tcaggactca cagacagagg cagaagagct acgcaaactt tggaaaaccc 360
atactatgca acaaactaaa cagcanaggg aaaatattca acaagtgtca caaanagaag 420
ctaagcataa aattacatct gctgatggac acatagaaag gtctgcactt ttaaaagaaa 480
agcanaggca tcgattacat aagttcttgg gtcttagagt tgggaaaacc aatgaggaaa 540
accgtttgga tnttaaggcc aggtgctacc aatgccaccg tntgccngag ggttaagaaa 600
cctnaatntt gg 612

<210> 139
<211> 592
<212> DNA
<213> Homo sapiens

<220>
<221> misc_feature
<222> (1)...(592)
<223> n = A,T,C or G

<400> 139
ggtactccac ttcttcctat tggaagatta acattattta ccaagaagga cttaagggag 60
taaggggcgc agattagcat tgctcaagag tatgtaaaaa aaaaaaaaaa aaaagaacca 120
aaccactgga aataatcaaa tgcaaaaagg taacaaattc ataactggaa agcaaagaga 180

agaacaagta tgatttggat gataaagcat tgttttaatg gtgaaaactt cacagatcac 240
taatgtttct agaggttaac ttcaagtggg caagctgggg tttttaggta gtcagtggcc 300
tagttcctaa agccacagta taggatctgt taaactgaat gtctgttgaa agtttggttt 360
agctgcttgg aggcttcctt ttaagacaaa ctgtatgtga ttaagttgtt tttgagggaa 420
ctgaagacct gatgtacccc tggccagata actgcctgat tctcagatat tattctctgg 480
gaaacatcta catacacagg agcttaaant ggcattatct cttgcctaaa ttcagagatn 540
ttttgnactt gccggnggcc gtcnaanggc gaatccgcac ctggcgccgt ac 592

<210> 140
<211> 618
<212> DNA
<213> Homo sapiens

<220>
<221> misc_feature
<222> (1)...(618)
<223> n = A,T,C or G

<400> 140
ggtncttaca cgtaagattt tagcctatgg tcattttata aagatgactg ttaggattta 60
attcacattt aaagaaaatg agattcgtta tattatggtg tttttatgac ctataaaata 120
cttaccccta caaatttcca taaatgtagt ggttagtaaa gcttttttct tactgaaaaa 180
taatgccagg taaccaagta ttattccttc catcatttat ttaggaaaaa gttttatgta 240
ttagggtaaa gtggtagaag ttaacctaga atctaataat ctccaatcac ccattcctga 300
tctaataagt agccatgaga aaaaatctct agaaagaatc atacctctca aaaaataaaa 360
tatnaaacaa aggctgggtg cagtggctca cacctgtaat ctnagcactt cccngaagtt 420
gaggtgggca gatcgcttga gcctaggcat atcgcttgna gcctgggcaa ctgtggccaa 480
accggtcttn taccaaaaaa atcncnaaag tagcccggcc ttagggccat accacctnga 540
gcccagggan ggtnaagnct accttgganc ngtgattgga ncctgcccng gtggncgttc 600
gaaaagggcn naaatnnt 618

<210> 141
<211> 551
<212> DNA
<213> Homo sapiens

<220>
<221> misc_feature
<222> (1)...(551)
<223> n = A,T,C or G

<400> 141
ggtacttcaa actctcttaa cggtgatgct ctgacattca ctactacatt tactctgcaa 60
gatgtatcca atgactttga aataaatatt gaagtttaca gcttggtgca aaagaaagat 120
ccctcaggcc ttgataagaa gaaaaaaaca tccaagtcca aggctattac tccaaagcga 180
ctcctcacat ctataaccac aaaaagcaac attcattctt cagtcatggc cagtccagga 240
ggtcttagtg ctgtgcgaac cagcaacttc gcccttgttg gatcttacac attatcattg 300
tcttcagtag gaaatactaa gtttgttctg gacaaggtcc cctttttatc ttctttggaa 360
ggtcatattt atttaaaaat aaaatgtcaa gtgaattcca gtgttgaaga aagaggtttt 420
ctaaccatat ttgaagatgt tagtggtttt ggtgcctggc atcgaagatg gtgtgtcttt 480
tctggaaact ggatatctta ttggacttaa cccgatgatg agaancgcaa ggtaatttat 540
atagtacctg c 551

<210> 142
<211> 601
<212> DNA
<213> Homo sapiens

<220>
<221> misc_feature
<222> (1)...(601)
<223> n = A,T,C or G

<400> 142
cgaggtacat ggtctatgcc tcccaggaga cgttcgggat gaaattgtca gtgtaaaacc 60
agaaaaaatg catctcttct agaattgttt aaacccttac caaggaaaaa aaaggggtgt 120
taccaactga gatcgatcag ttcatccaat cacagatcat gaaacagtag tgttcccacc 180
taggagtgtt gggaagttgt gtttgtgttt caagcagaaa aactgagctc caagtgagca 240
cattcagctt tggaaactat attatttaat gtgggctagc ttgttttcaa attttaaaag 300
tttaaaaata aaatactttg cattctaagt tgccaataaa atagaccttc aagttatttt 360
aatgctcttt tctcactaat aggaacttgt aattccagca gtaatttaaa ggctttcaga 420
gagaccctga gtcttctctt caggttcaca gaacccgccg nctttttggg tagaagtttt 480
ctactcagct agagagatct cctaagagga tcttttangc ctgagttgtg aangcaccnc 540
ngcaaacgca ttgccttcca nttggcacaa acnccggtna acggcttgtg ttaaaaaccg 600
c 601

<210> 143
<211> 515
<212> DNA
<213> Homo sapiens

<220>
<221> misc_feature
<222> (1)...(515)
<223> n = A,T,C or G

<400> 143
ggtnncgtaa agaatatatc ttatctggag ctcagcctca atcatgtctt aacaaaatga 60
caggtctnan aaagggggag ctcaatagct caaaagtgac aagtcctttt cacagcaccg 120
ttctcagaac acctctgagt aacgtgtttg ccagtagcta ttctcactga tgcactgatg 180
gccctgaaga agcggatcca gtcacatagg aaaggaggct gtgttagtga aagcacatgg 240
aaggtgttgn tttagaaagg tagtcaggaa aaacattcag gaatagattt atacaccatt 300
attgnattat ttntaaattt tcattcactc ttctgtttgg atacttttgc taattaaccg 360
tcctatgtta atanccacca aagctataag tccatagtca gtaaaacatt ccccttgggc 420
tgtctgagct aaaagcantg gcatctccgn atgtnggaca tccnagaaat agnttggtac 480
ctgcccnggc cgnncgttct taaggctaat ccngg 515

<210> 144
<211> 436
<212> DNA
<213> Homo sapiens

<220>
<221> misc_feature
<222> (1)...(436)
<223> n = A,T,C or G

<400> 144
ggtaccgctc aggattccca tcccaagaca cccggtcctt aaaccgccca ctcatgggtt 60
ggaagggatc tatgtggtag tagaatacaa actgctcagg tcccccgtct agaggacgaa 120
aattccaggt cactgttaga gcatcaccca caggggcaaa gctggagaaa gtgcatttta 180
accgagcatc tgtcccatta acagcctcca gcacccggga ggtataaatt tccacagctg 240
ctataggcca aagagctgtg agctgtatgc caaggagaag aagcaccgca cgagtagagc 300
tcttgccata catgagggaa acccagcctt ggccccagag accggacggg gcagaccgag 360
ggctccaaca ccctgccaag gccactccgg gaggagcaag caccgcgttt tnccagagag 420
aggagtttga gttgag 436

<210> 145
<211> 441
<212> DNA
<213> Homo sapiens

<400> 145
ggtacatccc cactatcatc cgccgggatg acccctccat catccccatc ctctacgacc 60
atgagcacgc aaccttcgag gacatccttg aggagataga gaggaagctg aacgtctacc 120
acaagggagc caagatctgg aaaatgctga ttttctgcca gggaggtcct ggacacctct 180
atctcctcaa gaacaaggtg gccacctttg ccaaagtgga gaaggaagag gacatgattc 240
acttctggaa gcggctgagc cgcctgatga gcaaagtgaa cccagagccg aacgtcatcc 300
acatcatggg ctgctacatt ctggggaacc ccaatggaga gaagctgttc cagaacctca 360
ggaccctcat gactccttat agggtcacct tcgagtcacc cctggagctc tcagcccaag 420
ggaagcagat gatcgagacg t 441

<210> 146
<211> 624
<212> DNA
<213> Homo sapiens

<220>
<221> misc_feature
<222> (1)...(624)
<223> n = A,T,C or G

<400> 146
acgtctcgat catctgcttc ccttgggctg agagctccag gggtgactcg aaggtgaccc 60
tataaggagt catgagggtc ctgaggttct ggaacagctt ctctccattg gggttcccca 120
gaatgtagca gcccatgatg tggatgacgt tcggctctgg gttcactttg ctcatcaggc 180
ggctcagccg cttccagaag tgaatcatgt cctcttcctt ctccactttg gcaaaggtgg 240
ccaccttgtt cttgaggaga tagaggtgtc caggacctcc ctggcagaaa atcagcattt 300
tccagatctt ggctcccttg tggtagacgt tcagcttcct ctctatctcc tcaaggatgt 360
cctcgaaggt tgcgtgctca tggtcgtana ggatggggat gatggaaggg gtcatcccgc 420
ngatgaatag tgggggatgt accttggccg ngaacacgct taagggccaa ttccannaca 480
cttgccggcc gttactaaag ggatnncaac tttngnacca aacttggcnn aaacaatggg 540
ccnaacttgg ttccntggng aaaatggttt cccntcaaat tccccccaan ttacnaccgg 600
aaccttaaag ggaaaacctt gggg 624

<210> 147
<211> 599
<212> DNA
<213> Homo sapiens

<220>
<221> misc_feature
<222> (1)...(599)
<223> n = A,T,C or G

<400> 147
cgaggtacaa gctttttttt tttttttttt tttttttttt cttttttttt tttttttttt 60
tttttttttt tttttttgaa cncanatcan tttattggca tggntttgtt tnaaaaaaag 120
gaaaagngnc aaanccaaaa nacanacttt gntaacaaat ncctgggggn ggctggacnt 180
ttttgcctaa tgctgngcaa anagggggat cctggcccan acatccngct gattccttgg 240
nacaaggttg tntgcctggg cctaantgcn cctttttgaa tacttgnttg caaaccacac 300
nttccanttt aatttccagg ggcagntnat naccctnnat ccactgggtc cagccacgcc 360
cntcntttta acccttttgc anacactgga gcttgntccg tcccagntca ctgnngnatg 420
cncttgcggn catttatgcc tgtcaaacct ctaaaactcn ttcccacctg gaagccatgg 480
angtagttcc taaaaaggct caacgngccg aagaacaana tgggccccgg cctggacaaa 540
actttttggc ngggttaaac aagttggcna ttttcccaag gnccanttgc ctnnnggcc 599

<210> 148
<211> 609
<212> DNA
<213> Homo sapiens

<220>
<221> misc_feature
<222> (1)...(609)
<223> n = A,T,C or G

<400> 148
ggtacttaag taatccaaag ctcgatcctg atctgcatga attagcatca taaatgcatt 60
ccttttgcaa cttgcatcct tctcattcac cagaaaatca tgtatcagtt caggagcatc 120
aggtataaga tgttcaaaat ttctatagat ggtatagatg gccaaaacag catttcttct 180
aacatagctg tgtcgatgct ccaaacatgc acgaatagct ggcattaaag gttctagcaa 240
ttctgcttct ttcaatttgc aaagaaaacg aagagtagat cctcgaataa attcattagg 300
atgttgaaga tcctttctgt atgcatcaca tacaaggatc atctcatgta aaagtctccc 360
atctggagtt gttttaggaa caatttccca aaataccaga agtaatttct tgatagtgtg 420
atcctgaaga aggtagcaca naacgaatgg atggtcatca gaaagtncag gaagtttttc 480
accaattcag aatcataatg gattaccttt cttcaaagct tcagtctttg actttacttc 540
ttcctttttc taaaatcatt ttttaagctt aatttccaaa tgggngggtc ttgaatccat 600
gggcncgtn 609

<210> 149
<211> 589
<212> DNA
<213> Homo sapiens

<220>
<221> misc_feature
<222> (1)...(589)
<223> n = A,T,C or G

<400> 149
   actcaggtag aaccatcatg aaaatgaccc acagtgaact tatggaaaag ttcttaacag 60
   attatttaaa tgacctccag ggtcgcaatg atgatgacgc cagtggcact tgggacttct 120
   atggcagctc tgtttgtgaa ccagatgatg aaagtggcta tgatgtttta gccaaccccc 180
   caggaccaga agaccaggat gatgatgacg atgcctatag cgatgtgttt gaatttgaat 240
   tttcagagac ccccctctta ccgtgttata acatccaagt atctgtggct caggggccac 300
   gaaactggct actgctttcg gatgtcctta agaaattgaa aatgtcctcc gcatatttcg 360
   ctgcaatttt ccaaacgtgg aaattgtcac cattgcagag gcagaatttt atcggcaggt 420
   ttctgcaagt ctcttggtct cttcttcaaa gacctggaac cttcaaccct gaaagtaagg 480
   agctggtaga tctggtggaa ttcacgaacg aaatcaaact ctgctgggct cctctgtana 540
   gtgctccacc cagtgattgg cctagacact ctgggagcaa ctggccccc 589

<210> 150
<211> 353
<212> DNA
<213> Homo sapiens

<400> 150
ggtacaaaga aattttggat agcaaaataa aggaatcttt acccatagat atagatcagc 60
tatcaggaag ggacttctgc cattcaaaga aaatgacagg aagtaacact gaggaaatag 120
actcaagaat ccgagatgca ggtaatgata gtgccagcac tgctcctagg agcactgagg 180
agtctctttc tgaagatgtg ttcacagaat cagaactttc ccctatacga gaggagcttg 240
tatcttcaga tgaactgcga caagataaat cttctggtgc gtcatcagaa tctgtgcaaa 300
ctgtcaatca ggctgaagta gaaagtctga cagtcaaatc agaatctact ggt 353

<210> 151
<211> 492
<212> DNA
<213> Homo sapiens

<220>
<221> misc_feature
<222> (1)...(492)
<223> n = A,T,C or G

<400> 151
ggtacctact ggtgctgaaa aaaggaaaat tccggcttga aggaaaggag tttagaactc 60
tgaaaatttg gtgacattgt ttttccctga aagaaatgtg tgttggattt aacagatgaa 120
attatctgcc ctccaaaagt cctttagaag agccagtgca aggctgaaga ccaaagcgtc 180
aagaacacgc cagactctca gcttcctctg ctttgctcct ttgttgagga aatgcaaatg 240
caaagagctt cccgttaaaa acaaggagtg tctgagagcc acgtgttcaa cacgcttctc 300
ctgctgctga cccctctgca cctgcagagg cagtgagcac ccaacaggtg gcgccaaggc 360
gcccgtcaca cgctcacgtc ctctggccag cagccacgtt tattgaagga gtgtggcact 420
gcccatcatt ggatatgccc tcggccatga aggattccag tggttcacgc tgnccagtat 480
atacaaaaat gt 492

<210> 152
<211> 597
<212> DNA
<213> Homo sapiens

<220>
<221> misc_feature
<222> (1)...(597)
<223> n = A,T,C or G

<400> 152
ggtacataag cctaaacaat ttcacctagg taaaatattg atgtcataac caaactatat 60
ggccccgttt cataaaggtt actatattct atagagagtg aagaggtggc ctttctatcc 120
cagcttaccc tattcttgtt attgttcaaa ttctcctgaa gcttgcataa ctagctgcca 180
tcaggtaaat gctattggct agcagaagac tgcagttctg ttaatattag aaccagcagg 240
gggaacttgg gaacttgaca ttaaaaatct agaaacagaa ttttaggatg ggtctcgtta 300
gaaacctgaa ttgttaatgg acttaagtaa aaaccatccc aaagaatttg agctttaagg 360
tgataaccgt cttttcagag atcatagcac atgaagaacc catggacact acacagacta 420
tgaaccggta gcagaaaaag atctcgtgac taaagtgggg gatgacagca aaaaaaaaaa 480
ttaccaaagg aaaaaagttg agaatncagg aatattacca gatggtaaaa aatattatct 540
tangccaaat gaggcccttc ggattcccaa accttgcttc ttctcctttc gtcttgn 597

<210> 153
<211> 596
<212> DNA
<213> Homo sapiens

<220>
<221> misc_feature
<222> (1)...(596)
<223> n = A,T,C or G

<400> 153
actggttgct acccattttt tcaagtctag gtgatggctg ctcctttcca acttgccttg 60
ttaaccagga tcctgaacaa gcatctactc ctgcagggct gaattccaca gctaaaaatc 120
tcgaaaacca tcagtttcct gcaaagccat tgagagagtc ccagagccac cttcttactg 180
attctcagtc ttggacggag agcagcataa acccaggaaa atgcaaagct ggtatgagca 240
atcctgcatt aaccatggaa aatgagactt aactcttcaa gcaagataaa ttcatacttt 300
ataaaagtat caatgctgta gatggatgga agaggcttcc cacaggaagg tgccaccagt 360
cagtttgtgc ctatgtccct ttggctggaa atgcagaata tgaattgatt aagttctctt 420
ccaagccatt gcttaaaata taacatgttt tgggatccaa tacacacatt ggtacaacta 480
acacaaattc ctattaaata ttaaaagtag ttctgggtta ttaatcaacg gggaaaacat 540
tttttccaaa aaaacttgga ataaatccan ggaccagttt tancccaata tttggg 596

<210> 154
<211> 297
<212> DNA
<213> Homo sapiens

<400> 154
ggtacccagt ttcaaagctc tctggttttt tctaagaaat gaagcaagga taggaacccc 60
ttctcccaga acaggcctca aatctatctt caaaggtgac ccagcaatca gtgtcaatgc 120
ctttactgta gttaacctgg taatttcatt ctttagtctc tccaagaaaa tctgaagtgt 180
attaggcaag tcagaaccca aattgtctcc aaggttgcaa ataatttgtc ccatacagga 240
aatagccctt tccttgactt cctgatcaat gtcagctgct tttaatctct taatggt 297

<210> 155
<211> 594
<212> DNA
<213> Homo sapiens

<220>

<221> misc_feature
<222> (1)...(594)
<223> n = A,T,C or G

<400> 155
ggtacttgaa ggagaacagt ttacatcggg cgttagccac cttgcaggag gagactactg 60
tgtctctgaa tactgtggac agcattgaga gttttgtggc tgacattaac agtggccatt 120
gggatactgt gttgcaggct atacagtctc tgaaattgcc agacaaaacc ctcattgacc 180
tctatgaaca ggttgttctg gaattgatag agctccgtga attgggtgct gccaggtcac 240
ttttgagaca gactgatccc atgatcatgt taaaacaaac acagccagag cgatatattc 300
atctggagaa ccttttggcc aggtcttact ttgatcctcg tgaggcatac ccagatggaa 360
gtagcanaga aaagagaaga gcagcaattg cccaggcctt agctggcgaa gtcaagtgtg 420
gtgcctncat ctcgtctcat ggcattgctg ggacaaggcc tgaagtggca gcacattcag 480
ggattgcttc ctcctggtat gaccatagaa tttggttcga ggcaaggcac tgtcaaagat 540
gtggaagaag aaaagtttct acacactgag caggcttata agttnggcag aaan 594

<210> 156
<211> 294
<212> DNA
<213> Homo sapiens

<400> 156
acaggatgca gtttctcagc tggattctga gctgatggac ataactaagc tttatgggga 60
atttgctgac ccatttaaac ttgcagagtg caaacttgca ataattcatt gtgccggtta 120
ttcagaccct atattggtgc agacactttg gcaagatatc atagagaaag aattgagtga 180
cagtgtgaca ttgagctcct cggatagaat gcatgctctt agtctcaaga ttgttctcct 240
tggcaaaatt tatgctggca caccacgctt ctttccttta gattttattg tacc 294

<210> 157
<211> 527
<212> DNA
<213> Homo sapiens

<220>
<221> misc_feature
<222> (1)...(527)
<223> n = A,T,C or G

<400> 157
ggtactgatt gtcatcctga ctttggcatt ggcagctctt atattccgac gaatatatct 60
ggcaaacgaa tacatatttg actttgagtt ataatatggt tttgtgactt atgagctgtg 120
actcaactgc ttcattaaac attctgcatt gggtataatc taagaattgt ttacaaaaag 180
attattttgt atttaccctt cattcctttt tttgatcctt gtaagtttag tataaatata 240
tctagacatt cagactgtgt ctagcagtta cgtcctgctt aaagggacta gaagtcaaag 300
ttccttgtct cactatttga tctgctttgc agggaaataa cttgnttttt ctcatgtttc 360
atcttctttt tatgtaaatt tgtaatactt tcctatattg ccctttgaaa tttttggata 420
aaagatgatg gtttaagttc caatgagtat tactaggtac tcaataccac ttattggagt 480
cctggcccng ggcgggcgnt tcgaaanggc caaatncagc accactg 527

<210> 158
<211> 617
<212> DNA
<213> Homo sapiens

<220>
<221> misc_feature
<222> (1)...(617)
<223> n = A,T,C or G

<400> 158
ggtactgaaa aagaggcgtg aggtgctccc tgtggatata accaccgcta aagatgcatg 60
tgtcaacaac agtgctctcg ggggagaagt ttatcgatta ccgcctcaga aagaggagac 120
acagtcctgc cctaacagtt tagaagataa caacttgcaa ttagaaaaat cagtttctat 180
acacacacca gtagtcagtc tctctcctca caaaaatctg cccgtggata tgcagctgaa 240
gaaggaaaag aaatgtgtga aactcatagg agttcccgct gacgctgagg ccttaagtga 300
aagaagtgga aacaccccta actctcccag gtcagtgtcc tcttttcctc caggcagcca 360
gcagacctct ccatctctcc tctctcgctg catgaactgt gctgnctgnt tctttatcta 420
ctttcttaca attgcatgca gtataattcc tcagtttcat ctacctacct tcaacttttn 480
cagaacttta agaaagactt aaactgattg caangggaaa ggactcttgg aataaggcaa 540
tcncattaaa aagttacncg tttctgggtt catgaaaggg atntcncagt ttaccccatn 600
tttgaaaggt ttatnng 617

<210> 159
<211> 1002
<212> DNA
<213> Homo sapiens

<220>
<221> misc_feature
<222> (1)...(1002)
<223> n = A,T,C or G

<400> 159
ggtaccagct tacctatttg attcagttgc tgttttctca ctctctatat ccatttgaaa 60
ttgatttatt ttagatgttg tatacttacg ttaggctttc tgttaatagt ggtttttctc 120
ctgttgacag agccaccgga ttatgacaca ggatgaggaa gattaaggat aatcaattga 180
ctaatttcat ttagaatatt atcaaacatt tcaactaggt atcagaaaaa ggctttcttt 240
cataagacta ttttaaatag aaattatttc aacaattaaa gtaatgttga ccatccccct 300
ctcagctgaa taaagaaaaa tttagttcaa tttattgcaa tttaattaca atactacctt 360
cacaacattt tcatgtgttt taaataaata ttttttaatt ggctaaagga cattcaagca 420
aagaaatgct ttctttactt aaaatgtcta tctcatttgc tgctttttca ctaagccttt 480
actttgttaa taaaagtgtc cattgtgtga tgtttttgat tttacagttt gctaaatctt 540
attttcttgg agttgctttt tggtaacagc tccattgcta ctccccattt tattggttta 600
catcaatgca tgcttcgttg tgatccctca agatgtaaca cttggtatgc tcggntgagg 660
atatgaaaaa atactttccg aaaccaggga attcagtgga tgnttggttt atctggttgg 720
ataagaaaag tagggnccag ccttaagcag nacagaagcc nctggtanaa gcatagtcag 780
ggaacttttt ttaattcntt tangnctaag ggncaggagt ggattnnaaa gggaggagag 840
cccttattat ggcctatncc ccgntttgga gaagancctt actgggaacc tggcccggcg 900
ggccgttcaa aagggcgaaa ttccgncacc tgggnggccg gttcttaagg ancccnactt 960
gggcccaaan nttggggaaa nnnggggcna aannggntcc cg 1002

<210> 160
<211> 434
<212> DNA
<213> Homo sapiens

<220>
<221> misc_feature
<222> (1)...(434)
<223> n = A,T,C or G

<400> 160
ggtacaagtc atcanggtca gcattctccc actttcaagt gcactaacaa ggctgctggg 60
atttccactg gagtgtcaac agcagtattc ttgttgcagg aactctcaga atttgggggt 120
ccataacagg tttagcctat gacccaggtc caaaagttcc agccttctct gccacctcca 180
gagctagctt caggttctgg tcaaagagct cacacctgat aggcatttct aaggaataga 240
atggattctt gagggcaaag tctgagtaaa tctcataaat ctttcggaga agagaatcta 300
ttccagcttg cctaggatct gctagaacca caaacttgat ccctgtcagt gtctggtagc 360
agtgcaattt gaatgtgtct gtctncagca tctcaatgcc tgagcttncc tgttcangag 420
acagntggna gcca 434

<210> 161
<211> 652
<212> DNA
<213> Homo sapiens

<220>
<221> misc_feature
<222> (1)...(652)
<223> n = A,T,C or G

<400> 161
acagactcca agggaagact gggctccaaa gccacatgcc tttgttggca gcgtcaagag 60
tgagaagact tttgtggggg gtcctcttaa ggcaaatgcc gagaacagga aagctactgg 120
gcatagtccc ctggaactgg tgggtcactt ggaagggatg ccctttgtca tggacttgcc 180
cttctggaaa ttaccccgag agccagggaa ggggctcagt gagcctctgg agccttcttc 240
tctcccctcc caactcagca tcaagcaggc atttatgggg aagctttcta aactccaact 300
gagttccacc agctttaatt attcctctag ctctcccacc tttcccaaag gccttgctgg 360
aagtgtggtg cagctgagcc acaaagcaaa ctttggtgcg agccacagtg catcactttc 420
cttgcaaatg ttcactgaca gcagcacggt ggaaagcatc tcgctccagt gtgcgtgcag 480
cctgaaagcc atgatcatgt gccaaggctg cggtgcgttc tgtcacgatg actgtattgg 540
accctcaaag ctctgtgtat tgtgccttgt ggtgagataa taaattatgg ccatgggaaa 600
caaannanan nnnnnnnnaa aaaaaaagct tgnaccttgg ccgngaccac gc 652

<210> 162
<211> 638
<212> DNA
<213> Homo sapiens

<400> 162
ggtacttgaa gatttgcata aagccaacat tcgcaccgtc atggtcacag gtgacagtat 60
gttgactgct gtctctgtgg ccagagattg tggaatgatt ctacctcagg ataaagtgat 120
tattgctgaa gcattacctc caaaggatgg gaaagttgcc aaaataaatt ggcattatgc 180
agactccctc acgcagtgca gtcatccatc agcaattgac ccagaggcta ttccggttaa 240
attggtccat gatagcttag aggatcttca aatgactcgt tatcattttg caatgaatgg 300
aaaatcattc tcagtgatac tggagcattt tcaagacctt gttcctaagt tgatgttgca 360
tggcaccgtg tttgcccgta tggcacctga tcagaagaca cagttgatag aagcattgca 420
aaatgttgat tattttgttg ggatgtgtgg tgatggcgca aatgattgtg gtgctttgaa 480
gagggcacac ggaggcattt ccttatcgga gctcgaagct tcagtggcat ctccctttac 540

ctctaagact cctagtattt cctgtgtgcc aaaccttatc agggaaggcc gtgctgcttt 600
aataacttcc ttctgtgtgt ttaaattcat ggcattgt 638

<210> 163
<211> 1002
<212> DNA
<213> Homo sapiens

<220>
<221> misc_feature
<222> (1)...(1002)
<223> n = A,T,C or G

<400> 163
acatataaat atatatataa aatgaacata gttcatgctt tcagataaaa tgagtagatg 60
tatatttaga ttaatttttt tagtcagaac ttcatgaaat ccacaccaaa ggaaaggtaa 120
actgaaattt cccttggaca tatgtgaaat ctttttgtct ttatagtgaa acaaagccag 180
agcatctttg tatattgcaa tatacttgaa aaaaatgaat gtattttttt ctccaaagaa 240
cagcatgttt cactcaatgg tgaaaaggtg gaaacattta tgtaacttta tgtgtatctg 300
tcttgatatc tactgacatt gtctatatga ggaaaatgat tactggtcat gctcctgtga 360
gttttttggg aaggtagggt catttctccc tgcctgcttt gtgccaacta gcatgttgca 420
tctacatgca ttatgagtct ggttaggcat tactttaaac atacataaag agacagtagg 480
acattgtggc tgagtctacc cagctcaagg taaaggagaa tattgctaat tttttagcaa 540
actagaccag cattattact caaactaaaa atatcacacc tgaaaaattt aatttaggac 600
ctaaaatgtc tagattagct ttctgctttt tttatttgaa taactcattc agttgtgaat 660
gaattcctct ttaattggtg ccacagtcac caaatgacaa ggatttgcca ctttcccccc 720
aaatnggagt gcttgtaatt taggctctct accntnaaat cagtntaagg gaaccgtaat 780
tatgatggat tttttccaag atgaccagct ggggtgaaaa ccatttttct ttggccaatg 840
gcaaaactaa taagctttaa aaacttcccc tttatgggga aagttttaaa actgggaaag 900
gttangaacc naccngtgga aanccntgga agggaaaaaa anaaaggggn ccttggnccg 960
gaacaccctt aaggggaatt cancccattg ggggccnttc nt 1002

<210> 164
<211> 572
<212> DNA
<213> Homo sapiens

<220>
<221> misc_feature
<222> (1)...(572)
<223> n = A,T,C or G

<400> 164
acagcatgca tttacaacca gcgctgatct agtctatttt gtcatataaa cttgaataca 60
aaaatccaat ttaaataaga ctagacttac tataatagta aacaaacaaa aacaaaaaac 120
aaaaaaaaaa aacacacaca gtagacttag tttgatactg attaatttta agagtaaact 180
catcctgtcc cctcttaata ctctactgca atttattgat ggctagaata tttactgact 240
taaaaaaggt attaaatact tgtatcatga aattacattc ttattaacaa taagacatac 300
tgtgtaagaa aatagctcat gtgtgaaatg tgtctgaaat gcattttttc cttacaacta 360
tcanaacatc cactcacact aaaatgaaac cactcccaac cccccctgaa aaaatgttna 420
gggaagacng ggtgggctgg gggaggagca agggaaggaa aagatttagc tatactaatt 480
acagcacagt gattaacaat gggtcaggac agaaccaaca gaattnggca aaaaanngcc 540
ctttaaacat ggntaccatt aaaaaccaac nn 572

<210> 165
<211> 594
<212> DNA
<213> Homo sapiens

<220>
<221> misc_feature
<222> (1)...(594)
<223> n = A,T,C or G

<400> 165
ggtactggcc tcctggcact ctgctttttc actgactggc tactgaagag caaggcagag 60
ctgggtggca tctcagaact ggcatctgga cctccctaac tgggccccgc tggtcccatt 120
tgctcattag aatttcctct cacatcagtg ggatacagaa ttcagtttct cccttgccag 180
gtccttggga tggttgaccc ctgcctctgc agtagccttt tgtgagtctg ctaaggtagc 240
tctcacacac ctcggctctg gggttgatac ctgagcctac aatagagccc tgaaatcaag 300
agcatagctt gagtgtgtga atatgatgtg tgcacatgct taatgagcgt gcaagtgtgc 360
acacgtttgt ggagaggagg gtgttctggc ctgagaaggt aaagaagagg catgtccagt 420
atgctttgca gggtgtgttt gctcttttcc atgcccatgc aacccagatt ggggtggagc 480
aggaaggagc tcttttctgt tcccaagcct cagaactctt gagctgtggc ttacttgctg 540
gcttcatcag gttcaagctn cgtgggccac actgctgctg ngccaagaag gtgt 594

<210> 166
<211> 434
<212> DNA
<213> Homo sapiens

<400> 166
gcgtcgcggc cgaggtacta taatggtccc catcttaatt tgaaagcgtt tgagaatctt 60
ttaggacaag cactgacgaa ggcactcgaa gactccagct tcctgaaaag aagtggcagg 120
gacagtggct acggtgacat ctggtgtcct gaacgtggag aatttcttgc tcctccaagg 180
caccataaga gagaagattc ctttgaaagc ttggactctt tgggctcgag gtcattgaca 240
agctgctcct ctgatatcac gttgagaggg gggcgtgaag gttttgaaag tgacacagat 300
tcggaattta catttaagat gcaggattat aataaagatg atatgtcgta tcgaaggatt 360
tcggctgttg agccaaagac tgcgttaccc ttcaatcgtt ttttacccaa caaaagtaga 420
cagccatcct atgt 434

<210> 167
<211> 395
<212> DNA
<213> Homo sapiens

<400> 167
acaaagttaa gtttagccct tttctagaaa gtgatcttta aaattaaaat tgctcctctt 60
ttaaattcac caaatttatg tgtggaaggg caccaaaatg attttgtaag tgccactgca 120
atattccctt tcaagtgtgg cctaaatttc aatcttaagg atggaatgca tgtctgctcc 180
ttgttctgaa aaatataggc atctactaca ttttaaaaca cagtgaaaca tatacataag 240
cctataaaaa aagatttgtg caatttgaaa gcctgttaat tttttatgta gacataccta 300
cacacgaaag ggttaaattc acagccttac tagttccttg cttccagtat ttcaattggt 360
ctcctcccct cattattatt attactacta gtacc 395

<210> 168

<211> 683
<212> DNA
<213> Homo sapiens

<220>
<221> misc_feature
<222> (1)...(683)
<223> n = A,T,C or G

<400> 168
ggtacggtat tctaatcaat gcatttgaaa agtcagcaaa agcccacatt aattcctatt 60
acgcttgttt cttggttcaa tctcagcact ttcagcggct cttgtgcggc gattctgtct 120
tggacttatt tctgtgtctt gaagatcgtt tttatgtgat gcttcccagg cttcctcttc 180
ttctaaaaga tctcttatga tgtctgaact ggaactattg catgaatctg attctgatga 240
agaaagaact tcttgaatat caatacagct agaagaatcc tcttctctgt caggttccaa 300
ttcctctggg gagtccagct ttgattgaga aaagtggttt gttactgagg tcatattatc 360
ttcctgtccc atgcatacag aagatagctt ttctgtagat tcatcttctt ttgttattgt 420
tactgttttt tgtgacattc cagcaatttt cttgtatcct tttctagcct gatccaccag 480
aagctgaaat tcactcttat gttttttacg atatttactg tggatttcat ctatttcctt 540
ttctgnttgg tcctttgtaa aaaccattac actttcattg agtttactag cttcaagacg 600
catcctagtc ttctctatat tttcgatttc tcgaactatt tcagcagctg atttaggatg 660
caaagcatcg cattgggcat tgt 683

<210> 169
<211> 408
<212> DNA
<213> Homo sapiens

<220>
<221> misc_feature
<222> (1)...(408)
<223> n = A,T,C or G

<400> 169
ggtacctttc tgaccacaat gaaataaacc tagaaatcaa taacaagagg aacttttaaa 60
gcagcacaaa taaatggaaa ttaaataaca tgattctgaa tgaccaatgg gtaatgaaga 120
aattaagaaa caaaatttaa atgtcttaaa atgagtgaaa acagaaacac aacatataaa 180
aatgtatggg atgcagcaag agcagtttta agagggaagt atttagtaat aaacacctac 240
atcaaaaaca agaaagatct ggctgggcaa ggtggctcac acctgtaatc ccagtgcttt 300
gggagcccaa ggcaggagga cgacttgatg ctgggtcaag accagcctgg gccatatata 360
tagcaagacc ttatctctaa aaaaaaaaaa nanaaaaaaa aagcttgt 408

<210> 170
<211> 566
<212> DNA
<213> Homo sapiens

<220>
<221> misc_feature
<222> (1)...(566)
<223> n = A,T,C or G

<400> 170

ggtaccaaca cagccaaaga ctgtaagaag gtagctgaag tcctctgcca aataggattg 60
aaaagctaaa atctttctct gtttctttct taagtaacaa ctggtctatt caagctcaac 120
cagagcatat aagagaaaaa actgactaac gagggggtct taaagagctt tgaaggacag 180
tttctagaaa gtagaaagat cactgagtaa attactgcac ctcctctacc ccacaaaaaa 240
aagggtgagg atgaatgtaa aagtgtagag caagctttca gacaacttca agtttgtttt 300
tggcgcttcc gtttgtaagc aatcaagatg gtgagagacg ctatcccaaa gaagaaagtc 360
tgtaggaacc agagtagctg agcccgacca cttgtgatgc ctttatgctt gcacaatact 420
atggcataca aggactctnc cacatgaatc agccaggcaa gccaataccc attgcaaagg 480
anggtgtgat gggngggcac caagtacctg tccgggcggc cctttaaaag gggaaattcc 540
ccacttgggg gcgggnttta gggnac 566

<210> 171
<211> 562
<212> DNA
<213> Homo sapiens

<220>
<221> misc_feature
<222> (1)...(562)
<223> n = A,T,C or G

<400> 171
ggtacctttg caagcaggtg gccagtaaag ctgaggagaa tctgctcatg gtgctgggga 60
cagacatgag tgatcggaga gctgcagtca tctttgcaga tacacttact cttctgtttg 120
aagggattgc ccgcattgtg gagacccacc agccaatagt ggagacctat tatgggccag 180
ggagactcta taccctgatc aaatatctgc aggtggaatg tgacagacag gtggagaagg 240
tggtagacaa gttcatcaag caaagggact accaccagca gttccggcat gttcagaaca 300
acctgatgag aaattctaca acagaaaaaa tcgaaccaag agaactggac cccatcctga 360
ctgaggtcac cctgatgaat gcccgcagtg agctatactt acgcttcctc aagaagagga 420
ttagctctga ttttgaaggt gggagaattc atggccttag angaagtaaa gccangagcc 480
cccaaatgtc ttggacnaac ttctcaataa ctggcttttg agctgtacct gtcccgggng 540
ggcnctttaa aangnnnaat tn 562

<210> 172
<211> 617
<212> DNA
<213> Homo sapiens

<220>
<221> misc_feature
<222> (1)...(617)
<223> n = A,T,C or G

<400> 172
acggtagaac tgctattatt catcctatgt gggtaattga ggagtatgct aagattttgc 60
gtagctgggt ttggtttaat ccacctcaac tgcctgctat gatggataag attgagagag 120
tgaggagaag gcttacgttt agtgagggag agatttggta tatgattgag atgggggcta 180
gtttttgtca tgtgagaaga agcaggccgg atgtcagagg ggtgccttgg gtaacctctg 240
ggactcagaa gtgaaagggg gctattccta gttttattgc tatagccatt atgattatta 300
atgatgagta ttgattggta gtattggtta tggttcattg tccggagagt atattgttga 360
agaggatagc tattagaagg attatggatg ccgttgcttg cgtgaggaaa tcttgatggc 420
agcttctgtt ggaacgangg tttatttttt gggtanaact gggattaaaa gctacatggt 480
taattctaag gccactcagg ntaaaaaanc nngcgagctt aaccctttga aaaangnggc 540

ccccntggcc cgaaacnccc ttaaggggca attccancaa cntggnggcc gttattangg 600
gatccgactt gggcccn 617

<210> 173
<211> 232
<212> DNA
<213> Homo sapiens

<400> 173
ggtaccagat gctagctggg cctggtgggt atccacccag acgagatgat cgtggaggga 60
gacagggata tcccagagaa ggaaggaaat accctttgcc accaccctca ggaagataca 120
attggaatta agcttttgta aagctttccc aaatcctttc atcattctac agttttatgc 180
tatttgtgga aagatttctt tctcaagtag tagtttttaa taaaactaca gt 232

<210> 174
<211> 987
<212> DNA
<213> Homo sapiens

<220>
<221> misc_feature
<222> (1)...(987)
<223> n = A,T,C or G

<400> 174
gcggccgang tacttcacca tcactgactc catggacttg atcagccgcc gctggatgta 60
tccagtctca gcagtnttga cagccgtgtc aatgagcccc tcacgacccc ccatggngtg 120
gaaaaagaac tcagtgggtg tgaggccggc taggtaggag ttctccacaa agccacggct 180
ctcaggcccg tagtcatcct tgatgaagtg aggcactagt ccggtgcttg aagccaaatg 240
gaatccgctt gccctcgacg ttctgctgtc caacgacagc gatgacctgg gagatgttaa 300
tcttggaacc tttagctccg gacacgacca tanacttgaa gttgttgtat tcanacaggg 360
atttntgagc agaggagcca gtcttgtctc gggcatcgtt aagaatgcgg ttcacctgat 420
tctcaaacgt ctgccgcaga gtgttccctg nggngggctc cagctcattg ttgngngcct 480
tctcgatgac ctctattacg tcctgcttgn ncttcttaat agtgttctga atgtcctggt 540
aagncttaga atcagcantg gngtcccaan gcccatactt tgacctatag acagggaaaa 600
acatcagcaa acccctttgg acctctaata nacatggaat ggaattataa ccccagagta 660
taancanggg caccanatnc aaggaggaaa gaaanggatn gtangacagn aagaagttnn 720
agaantcnnn nagacggctt ggaccctgnc cggcnggccg ttcaaanggc caattccann 780
ccactggtgg ccgnnacttn tggaaccgnc ttgganccaa acntggctaa aaanggccnt 840
agcnggttcc cgggcttaaa tggnatncgn tcccaattcc ncccaaatta cggcccgnaa 900
nccttaancn aaaancccgg ggggcctnan gaanggnnta acncccntta aatgggttng 960
ccncaaggcc cnntttcaan tngggan 987

<210> 175
<211> 574
<212> DNA
<213> Homo sapiens

<220>
<221> misc_feature
<222> (1)...(574)
<223> n = A,T,C or G

<400> 175
actccccgcc ccctctgaaa gcatgtcaca tcatgtaaat ttgcttctaa catctgcttc 60
aaactgtctc tggactccaa atttggatgg gtcagcctct gcagaaagtt tgtgttgaga 120
tgctggaaga acagcagagc ctcctgcacc ctcagcaagg gaccagctcc caaaggaaag 180
gtccttgtgt gacatttgga gaatcttcct tcatccagac aactctactc gaagcaagac 240
gaaagcagga tgtggcagtt gcagtgagaa aggaaaggaa agatgggcag actctgcttt 300
ctggaaattt cttcacaaag tagagctcat gaactctgtg ctgtcttctg gtaacatatc 360
atcagtgttt gtattcatgg tgtggcacat ggatccatgg cattgggtaa atctggtggt 420
ttttacacat ggtcagaatg tgttcaaata catctcatga tggagacagt ncccaaggta 480
aatggttggt ttcagcattt taaaaaagac tcccttaaca tttatctcag aatcatgagc 540
ccttcttcta gttgacaatg gcaatggtcc cccn 574

<210> 176
<211> 570
<212> DNA
<213> Homo sapiens

<220>
<221> misc_feature
<222> (1)...(570)
<223> n = A,T,C or G

<400> 176
ggtacagata ttcattcagg agctccagga aactggattt gctctctaga gggcagctca 60
aagggcccat tcactcacaa tccacccaac ggcattcctg gcctccggtc acagcctcag 120
ccacggaagt cctgcagggt ttgtcagtct gtgggggtga gtgccctaac accatgaact 180
gcccactgct cccagaaaga aagaagaact tggaatatga gactccccag gtctcctgac 240
cctcttcctt cttggaatga gacccaggta gtgctcaggg gatttctggt gttggccatg 300
gacaagcaac cagtagtggg ctcactttag ggacgcaaac cacaaagccc acctcaggaa 360
gccaaatttc aactcttgcc ctggggcaaa cttctagcaa ccaggccaga ggcaaatgtc 420
agacaggata agggatgaca tnccatcaat caaagttgna aatgggaagg gacccancca 480
gtttgnaata aaggcnttaa actnggnacc tggcccggcc ggccgtttaa aggcgaattc 540
acacactggn gggccgtcta agggatccca 570

<210> 177
<211> 621
<212> DNA
<213> Homo sapiens

<220>
<221> misc_feature
<222> (1)...(621)
<223> n = A,T,C or G

<400> 177
acagaagagg atgaagaaga ggatgaagag gaagaagaag agtcttttat gacatcaaga 60
gaaatgatcc cagaaagaaa aaatcaagaa aaagaatctg atgatgcctt aactgtgaat 120
gaagagactt ctgaggaaaa taatcaaatg gaggaatctg atgtgtctca agctgagaaa 180
gatttgctac attctgaagg tagtgaaaac gaaggccctg taagtagtag ttcttctgac 240
tgccgtgaaa cagaagaatt agtaggatcc aattccagta aaactggaga gattctttca 300
gaatcatcca tggaaaatga tgacgaagcc acagaagtca ccgatgaacc aatgggaaca 360
agactaacta tttagaaaca tttaagatgc cagtatttta catacaggtt ctggntttta 420
acactggatt aaaacttttt ggngtaaata aaaaatggga ccctttaggn ttttacccag 480

gaagaaagcc aaggtttggt aaaaattaaa aggtanccct tggggccggg gaanccacgg 540
ctttaagggg ccgaaaattt ccaagnacaa ccttggccng ggcccggnta ncttaaaggg 600
ggaatnccca agaccttnng g 621

<210> 178
<211> 403
<212> DNA
<213> Homo sapiens

<220>
<221> misc_feature
<222> (1)...(403)
<223> n = A,T,C or G

<400> 178
actccttcct gagccgctgc aataagcttt ttgctgtgga atatgacgac agctagatac 60
tgtccctgcc acaagagctt ctggttataa atagacaaag actctaattt ctaattgacc 120
tcttttcttt ttcaggttta tacataaatt ttcgtcacct ttataaacag cgcagacggc 180
gctatggaca aaaaangaaa aagatccact aaaaagaaag atttagatgg cttcttgcca 240
gtttgagcct aatctgattc ttacagtttt accttcttga accaatgtaa aagttttttt 300
aatgttaaat gattaaattc tcagtgaggc tatcttcctt ttccccagta acattcctga 360
atttactgnt accttattgt aagtacctcg gtcgtgacca cgc 403

<210> 179
<211> 650
<212> DNA
<213> Homo sapiens

<220>
<221> misc_feature
<222> (1)...(650)
<223> n = A,T,C or G

<400> 179
cgaggtacaa gctttttttt tttttttttt tttttttttg agccaaccag ctaaaggatc 60
actgcagcta aatacagata gagaagcaac aaagccaggc aaatacccat cagagacagt 120
gacaagagca gctgggggca cgggggaggc agaaggaaga gaaagaaggg gaggagcctc 180
cagagtccca gccccaaccc cctctgccat tggctaccct tgctccccac aaatccctgg 240
ggttgaagtg aggaggacta caggctgggg tgaaaataca caaggacagc ccaacaaaat 300
acaacaagga ctagcatcag tctccccctt actccacccc caagaaaaat acccttattg 360
ngactagtat ttatgaaaat ctgtaagaga ctattctatg tagtggctct aatcccatat 420
cacagcaact gcctgngttg ggaacttttc aaatcagtga tttgcgggaa ccaaccggat 480
tttcagcttn ttacggngca tgcagcttta ccaaaacttg ggtaaagncc agncacattt 540
accttctgct tacatntaaa aagggtgang aaagagggaa gggaaaaagg ggttaagggc 600
taggtaaact tactggtnag cagctanatt caccatggtc nttttttggg 650

<210> 180
<211> 639
<212> DNA
<213> Homo sapiens

<220>
<221> misc_feature

<222> (1)...(639)
<223> n = A,T,C or G

<400> 180
acatacggct gtgcgataca ccagcattga attggttgga gagatgagtg aagtcgttga 60
tcgaaatcct cagttccttg accctgtgtt gggctatttg atgaaaggcc tgtgtgaaaa 120
gcccctggct tctgctgcag ccaaagccat tcataacatt tgctctgtct gccgagatca 180
catggctcag cactttaatg gactcctgga gattgcccgc tccctcgatt ccttcctgtt 240
gtctccagaa gctgctgtgg gcttgctaaa agggacagca cttgtcctag cccgattacc 300
tttggataag attaccgaat gtcttagtga actatgttct gttcaggtta tggcattgaa 360
aaagctgttg tctcaagagc ccagcaatgg catatcctca gatccacagt gttcttagat 420
cgccttgcag tgatatttag gcataccaat cccattgtgg aaaatggaca gactcatccg 480
tgtcagaaag tcatacagga aatatggnca gtttatccga gactctaaat aagcaccgag 540
ctgataatcg gattgtagag cgtgttcaag gtgcctgcgc tttgtggtcc tgngaagcna 600
angactgaac actgtgcagc nctagtccac aatgngaat 639

<210> 181
<211> 644
<212> DNA
<213> Homo sapiens

<220>
<221> misc_feature
<222> (1)...(644)
<223> n = A,T,C or G

<400> 181
acaagagagg ttccaggagg gggtgatagg cagaattttg gtccccatca ccttccctgc 60
ccagtgttat gcctatgaat gtgttacatt atgtggtaaa agggactttg cagatgtaac 120
taaaatttct aaaatagaga tattatcctg gattacctgg gggaacccag tgtaattaca 180
tgaaccctta aaaatggaag aggatgcagg agtcagattc aaaggaaggc ccaaggtgct 240
attgctgact tgaagataga ggggccatgt ggaaatcaag agaaggaagt gaatccttcc 300
agtgagcttg gaagagagca ccttgaggca cagatgagaa gcttggcctt acctgatgcc 360
ttgattttag cctggtgaga ccccgagcat ataaatttgc tgtgctatgc cacacttctc 420
acctacagaa acttagttta aagccactaa gtttgtggta atttggtggc tttaggcccc 480
ttgagggtag agatttatgg cttgtgttac aagtagaaga gcagtggaaa agttgggctt 540
tggtaattct ttcaagggtg aattgtagtt ctgggagtcc tatctanctt gggntcagaa 600
cnttgttggg cangncctgc tggggacttc ctggtttaac cttg 644

<210> 182
<211> 609
<212> DNA
<213> Homo sapiens

<220>
<221> misc_feature
<222> (1)...(609)
<223> n = A,T,C or G

<400> 182
ggtacagaaa agtcagatca aattggatat gtagacattg ctaaggattt tgaactctaa 60
gggcattgat aagctactca agggttttta gtaggggagt gacttgatta gacttattta 120
tttgttgaaa agtctgtgtg gctggtgtgt ggaaaataga atggattgaa aaggaactca 180

agtggagcat caagactcag ttaaggagtt aatctaggtt ggaaataatt gtagcttagg 240
cctggatgct ggcaataggg aaggggatgg attcatgaaa gaatgggata cttgagaaga 300
aatatttctg tgctggagaa gtagattggg gaagttcatg gcataaacat tataatggat 360
gctatgggca tagataacat aaacatgtag agaaagtaaa ggtgacctag ggcagaagcc 420
ttaggaaccc aaaatttaag agtagactga agagaaccgc tgtagaagtg ggaggaaanc 480
tgctcgtgtg ggtagacaag gagaccnttc aaaaggatca tcattacagt naaaagctgg 540
caactcggcg tcttggtgaa agtncctgcc cgcggccgtc naggcnatca gccatgcgcc 600
gtcttaggn 609

<210> 183
<211> 401
<212> DNA
<213> Homo sapiens

<400> 183
ggtactcatc ctttgccagc aaagatgcac aactataact atggtggtaa cttacaggaa 60
aatccgagtg gccccagcct catgcatgga cagacctgga cttctcctgc ccaaggacct 120
ggatattcac aaggatacag gggacatatt agcacatcaa ctggcagagg cagaggcaga 180
gggttaccat actgagtatc tgtttttcct caggcacatc atttttatct ggaaagactt 240
ttctagctgc aatttaaggc agcaatccaa gagacttgaa taataataat tcaacaacag 300
ctttattttt atgtggagaa gggtcttgca tacaatagtt taaaaaagac aaaaaaaacc 360
tttgcttaaa ttcatgctgt tctaaaaact agatcgattg t 401

<210> 184
<211> 423
<212> DNA
<213> Homo sapiens

<400> 184
ggcggcggat ggaggtcagc ggtggtgctc gctgcggttt ggaatcactt gctaggagtc 60
ttgtctctct gccacccagg acatcatggc agctcacctg gtaaagcgat gcacgtgcct 120
cctgagagaa gctgctcgtc aggcccctgc catggctcca gttggccgac tgagacttgc 180
ctgggtagcc cataagactc tgacttcctc agccacctca cccatttccc acctcccagg 240
ttccttgatg gagccggtgg agaaggaacg agcatctact ccctacatag agaagcaggt 300
ggaccacctc atcaagaagg ccacaaggcc agaggagctc ctggagctac ttggtggcag 360
tcacgacttg gacagcaatc aagcagcaat ggtactaccg gcgctacaaa gtgaagtcgt 420
acc 423

<210> 185
<211> 669
<212> DNA
<213> Homo sapiens

<220>
<221> misc_feature
<222> (1)...(669)
<223> n = A,T,C or G

<400> 185
acccgcagct tgtccccatc ctcatattca tccaggcaaa tggcacagac atcatactgg 60
tctcccttct gatagtcatg tgtaggaatc tgtttcagtt gctctttggt aagtcgattc 120
cgctggagcc gtttccggtg ctggatacaa cgagctatca ttactgctcc catggccaaa 180
accagcagtc ccacaatccc tgtgaaaggg atgaggtaat agcccaaggg gaaggtattg 240

tctggaacca gaagcacccg agccccttc tcgtagacaa agagggcacg caggtacaaa 300
gagagaaatt ttaaagctgg gtgtcagggg agacatcata tgtcggcagg ttctgtgatg 360
ccccctaagc ccgtaaaacc agcaagtttt tattagtgat ttccaaaagg gggaagggag 420
tgtatgaaat agggtggtgg gtcacaagag atcacatgct tnacaaggta ataaaaatat 480
cacaaggcaa aatggaggca gggttgagaa cacnggacca cattgaccaa gggcgaaatt 540
aaaaattgtg aagtgaagtt cnggccacgc antgncantg atacatctta tcaggagaca 600
ggntttgaga gcngaccanc agtctggncc aaaattaata agtgggaaat ttcttggcct 660
aataagccg 669

<210> 186
<211> 638
<212> DNA
<213> Homo sapiens

<220>
<221> misc_feature
<222> (1)...(638)
<223> n = A,T,C or G

<400> 186
ggtacatgtg cgttggcatt atggatcgat ttttacaggt tcagccagtt tcccggaaga 60
agcttcaatt agttgggatt actgctctgc tcttggcttc caagtatgag gagatgtttt 120
ctccaaatat tgaagacttt gtttacatca cagacaatgc ttataccagt tcccaaatcc 180
gagaaatgga aactctaatt ttgaaagaat tgaaatttga gttgggtcga cccttgccac 240
tacacttctt aaggcgagca tcaaaagccc ggggaggttg atgttgaaca gcacgcttta 300
gccaagtatt tgatggagct gactctcatc gactatgata tgggtgcatt atcatccttc 360
taaggtagca gcagctgctt cctgctgnct canaaggtct aggacaagga aaatggaact 420
taaagcagca gtattacaca ggatncncag agaatgaagt attggaagca tgcagcacat 480
ggccaaaaat gtggtgaaag aaatgaaaac ttacctaaat catcgccntc aagaataagt 540
ntgcagcngc aactcctgaa natcacttga cccttagntg accttaaagc ccgnaaanac 600
cttgcctccc ccggaaggaa ggcctaggtt cccgggcc 638

<210> 187
<211> 628
<212> DNA
<213> Homo sapiens

<220>
<221> misc_feature
<222> (1)...(628)
<223> n = A,T,C or G

<400> 187
ggtacataga aattcattga ggtatataga tactcatctg tctaggcagt tcccaatttt 60
ctgaagaatg ttttacagca aaattttcta ttttctttta ttaaatagtg acacgtcaaa 120
caatgtcaca tccaaaacac tagtttcatc aatttctagc agtaataata gacttgctgt 180
aagtattgtt ttctgatgcc atacccttgt catacatatt attaaatgac caatattatg 240
tatgaagtag acaaaaaaat ttactcaaac ttcattcaaa tcctaattgt gataattttt 300
gttttatatt taattataaa ccaaaataca tttgcatttt taagctaatt tgtctcaaaa 360
ttttgcttta tatttttgga tcaggttaaa gtcctgggga tcccctgaat gttattgccc 420
tcttggattg gtttttactt ctgagctata ccgtcaaaag acacataagc ttcaaaagtc 480
aagacaaacc tcatttgcca taaaaatcaa gatatagatg tctggtccga aactncttga 540
aaaacatttt aagcatcaat atgactggtt ccatgaactt aagtacttct taatgagtat 600

tctttctgaa gctgaaagaa gattgttt 628

<210> 188
<211> 654
<212> DNA
<213> Homo sapiens

<220>
<221> misc_feature
<222> (1)...(654)
<223> n = A,T,C or G

<400> 188
cgaggtacaa ggtggactgt gcatgcctca aagaaaaccc agagtgccct gttctaaaac 60
gtagttctga atccatggaa aatatcaata gtggttatga gaccagacgg aaaaaagaat 120
aaaaagacaa agatatttca aaagaaaaag atacacaaaa tcagaatatt actttggatt 180
gtgaaggaac gaccaacaaa atgaagagcc cagaaactaa acaaagaaag ctttctccac 240
tgagactatc agtatcaaat aatcaggaac cagattttat tgatgatata gaagaaaaaa 300
ctcctattag taatgaagta gaaatggaat cagaggagca gattgcagaa aggaaaagga 360
agatgacaag agaagaaaga aaaatggaag caattttgca aggcttttgc cagacttgaa 420
aagagagaga anagaagaga acaagctttg gaaaggatca gcacagccna aactgaagtt 480
aaaactgaat gtaaagatcc cagattgcag tgatgctgag ttatttanga acnagccata 540
gaagaaaatg ctagcagcca acccctgcca agtaatagac taancgggga aaagttttct 600
cgagtaggac tacttggcag caccgtcgga gaccngactg tcacatggtt anan 654

<210> 189
<211> 650
<212> DNA
<213> Homo sapiens

<220>
<221> misc_feature
<222> (1)...(650)
<223> n = A,T,C or G

<400> 189
ggtactttaa gataattgta ttgatctttt ttcagattcc ttgtattttt aataaagtaa 60
tcttaaataa aactcagata ggttaagtgt tagaaatttt aaacagctta cattgttagc 120
gtaaagttat cttttctttt ttcctaatca gagttcttga ccctttggtt attgagttta 180
aaacttcaat tgaaattcaa tagtatttat tttttaaaaa aatcactaaa ctgtgcctaa 240
agaacataac tgccatatta atgttttggt ttatatcctc tatagtaata gaaaaacatt 300
taatacttgt aatgctgatg tgttaatttg ataccagttg agtagaatgt gatcaatcca 360
gtttacaatc tatcatgagt attattaact aaaatctatg tgcttttcaa taggaatcat 420
tcttctcttg ctgnaacact tgccttaact tttangaaag nggtcatttt taaactgcac 480
tggnaagggt gaaagttang actcttggat ttggngaccg naatctgaag ccgaatantt 540
aaagggagaa aaagaaacca ggtctttttg ccaaaggctg ggaaccntat tcanctttgg 600
gnaagtaatt ggatatncca agggtgggan gacaagtctg aaaatcacng 650

<210> 190
<211> 699
<212> DNA
<213> Homo sapiens

<220>
<221> misc_feature
<222> (1)...(699)
<223> n = A,T,C or G

<400> 190
accagctcta atctgtggcg tccagttttc tttctttttt tttttttctt ttttaatgtc 60
aaagtgaatg tctgaagttt tgtctttttt tctttgtcct tttccatctg cttcattctg 120
tggggataaa atacttgtgt ttaatcagaa caactggaac gcattgagga agggatggac 180
caaatcaata aggacatgaa agaagcagaa aagaatttga cggacctagg aaaattctgt 240
gggctttgtg tgtgtccctg taacaagtag gtgctgcctg cctgcctgaa gctttgattt 300
cccaaggccc atctccaagc cttgacaaag ctcattcctg ccaagctcat aggcaggatg 360
aagcatgtgg catgcagaaa cagatcaata cccgcttcaa tgcattcatc tcatagcata 420
gaagatatta accaggaagt tactgggtga tgcanttaaa aaatcaaggc catacctaca 480
ggtggaaagc nttcacntgt cagcnaacnt ttaattggat gaaccggttt caaccatttt 540
nccaaaaaag gtgtacctgg ggnnaagggg gtgggcccag tggcccccaa gtgggacctn 600
ttgaaaatga aaagggtggt tcntttccac tgggcccttt gggccttggt aaccaagncc 660
tcttccgcgg gggcaaggca antanccttg gcccggnan 699

<210> 191
<211> 378
<212> DNA
<213> Homo sapiens

<220>
<221> misc_feature
<222> (1)...(378)
<223> n = A,T,C or G

<400> 191
acaaagattc cagacagact ttgttttttg gcttataaca atgtgtagat actacacaaa 60
gaatgaggat gtaattttca tttacaagca aaatgtgacc aaaatccctt ttcttcttaa 120
aattgaaaaa tgaaattctt gagaatacta attagtgacg gccaaatctt agactatttt 180
aaattagcca tggttaaaca taggtgagtt aaacattgtg cctttccaaa attaaggttt 240
gcagttagaa acataaacat ttgataaaac ttctcaaaat taattatgag tggcttattc 300
atgtcctttg gattccagac acacactana aaaagtaaac gttaaagagg tgatattttg 360
gaaagcatcc ctagtacc 378

<210> 192
<211> 624
<212> DNA
<213> Homo sapiens

<220>
<221> misc_feature
<222> (1)...(624)
<223> n = A,T,C or G

<400> 192
acagtaaaaa gtaaacttcc ctccatccca ggcctgccag catccctgat gccgactttc 60
tgggtgtggc ctagggcccc tcagtgtaat gtaggggttg tgagcacaga ctttggtgcc 120
agtttgctag gttcgaatcc tgactccctc tttgtagctc tgtgcttcaa ttgaaatact 180
gtgcctcagt ttctccttta taaaggcagg gatcatgaga gtgcctgtcc cttgtgagca 240

ctatgaaagt gttagctgtt ctttaccaga ataaatgcat ttctatatct tcccatatgc 300
attttgttaa tttttaaagt atttcaaaca caaagtttga aacagaaaat tgtgtaacat 360
taactatgaa cttaccaccc agaatttaca aatgctgaca ttttgcaata tttatttcgg 420
atctattttt aaggggggga accctgcagt tactgcttaa tcctctttcc accccaacct 480
tttattttta cacaaggagc catagtggtc atacttaagc tatttttttc agtaactnaa 540
tatattttgg aaganctccc tcctaggnca tanaagcttt gncccttttt tttacagtgg 600
taaacctttn ggactaaagg gcng 624

<210> 193
<211> 348
<212> DNA
<213> Homo sapiens

<400> 193
actgctactt ctataaacgg acagccgtaa gactaggcga tcctcacttc taccaggact 60
ctttgtggct gcgcaaggag ttcatgcaag ttcgaaggtg acctcttgtc acactgatgg 120
atacttttcc ttcctgatag aagccacatt tgctgctttg cagggagagt tggccctatg 180
catgggcaaa cagctggact ttccaaggaa ggttcagact agctgtgttc agcattcaag 240
aaggaagatc ctccctcttg cacaattaga gtgtccccat cggtctccag tgcggcatcc 300
cttccttgcc ttctacctct gttccacccc ctttccttcc tttccacc 348

<210> 194
<211> 627
<212> DNA
<213> Homo sapiens

<220>
<221> misc_feature
<222> (1)...(627)
<223> n = A,T,C or G

<400> 194
ggtaccttct cagccagctg cagcaaagcc aaatggcaga gaagcagtta gaggaatcag 60
tcagtgaaaa ggaacagcag ctgctgagca cactgaagtg tcaggatgaa gaacttgaga 120
aaatgcgaga agtgtgtgag caaaatcagc agcttctccg agagaatgaa atcatcaagc 180
agaaactgac cctcctccag gtagccagca gacagaaaca tcttcctaag gatacccttc 240
tatctccaga ctcttctttt gaatatgtcc cacctaagcc aaaaccttct cgtgttaaag 300
aaaagttcct ggagcaaagc atggacatcg aggatctaaa atattgttca gagcattctg 360
tgaatgagca tgaggatggt gatggtgatg atgatgaggg ggatgacgag gaatggaagc 420
caacaaaatt agttaaggtg tccaggaaga acatccaagg gtgttcctgc aagggctggt 480
gtggaaacaa gcatgtgggt gcaggaagcc aaaagtcaga ctgtggtgtt ggctggtgct 540
tgtgancccc ccaagtgtng gacccgccgc caaggcaagg aaaccttggg ccctttttaa 600
cgggcccngg aattcccaag gttcntt 627

<210> 195
<211> 405
<212> DNA
<213> Homo sapiens

<400> 195
ggtacaattc cacttatcca tactattcct ttataaaagg cagatttcag gtaagcttct 60
aaatgcatgc gtaatgtaga ggctaatatt ttctggcagt ccttggttcc tgaaatttga 120
acttcatatg tgttttaaac ttttgtcaaa atagtcatga aagatatgtt atttttgcat 180

aatgaggtaa tatatcaggg gcgggcactc ataagacagt ataaatccac ttgtctaaac 240
ttgcatgagg ctgtgtgcat tgtaaaatgc cataaagagt tttgggtcag tgaatatttt 300
gctgaaggaa taacacttac atttaactga gcacttttct gtaataaata ccaaagtagg 360
tttttgtagc tgtaaactgt gtacctgccc gggccggccg ctcga 405

<210> 196
<211> 658
<212> DNA
<213> Homo sapiens

<220>
<221> misc_feature
<222> (1)...(658)
<223> n = A,T,C or G

<400> 196
ggtgaaagga gttaaaacgc ccagtggtca ttaagtgaaa catcttttat caacctgcaa 60
aagctgcagc gttctctgcc aggtcaaatg ggcatgttta gaaaataaga gaagatggct 120
gagtatagct aatgaataaa tggttgtttc tttagaaaat taaacacaca cagagtgtaa 180
gaggagagga tacggccctc cctgaaggat aaagtccacc tggacggtgc cctgccctcg 240
cttctcacat taactgccca ggaatgtcat gctgattggt tcccggaagg gtgtttggca 300
aggggcagtg tatggagcta cgtgtagaag gagagaaatt tgtgtgtggc ttttgtaaat 360
tttgaccgat tgcagcaatt aaataagttg attactgngt tgatttaaat acttatgaaa 420
gctttcaaga cnaaaaataa acctttcacg ttacccccaa annaaaanan tnnnnnttta 480
nataaaaaaa acttggancg gnatgnggtt tcttggaaaa agtttggatg ccatttgcna 540
aattcttcnt tttnggtttn aaaattgaac ncaggnattn ggggggancc nttttggaaa 600
aancccataa gcttggtttn cttgnnnaaa ctttgnaant tngccccngg nttaattn 658

<210> 197
<211> 615
<212> DNA
<213> Homo sapiens

<220>
<221> misc_feature
<222> (1)...(615)
<223> n = A,T,C or G

<400> 197
ggtacagaga aagaaataaa agatactgag aaagaggtgg atgacctaac agcagagctg 60
aaaagtcttg aggacaaagc agcagaggtc gtaaagaata caaatgctgc agaggaatcc 120
ttaccagaga tccagaaaga acatcgcaat ctgcttcaag aattaaaagt tattcaagaa 180
aatgaacatg ctcttcaaaa agatgcactt agtattaagt tgaaacttga acaaatagat 240
ggtcacattg ctgaacataa ttctaaaata aaatattggc acaaagagat ttcaaaaata 300
tcactgcatc ctatagaaga taatcctatt gaagagattt cggttctaag cccagaggat 360
cttgaagcga tcaagaatcc agattctata caaatcaaat gcacttttgg aagccnggtg 420
tcatgaaatg aaacccaacc ttcgggccat cgcagagtnt aaaaaggaag gaagaattgn 480
atttgcaccg gtagcagaat tggccaaaat acttntgaag ggaccggttt agaccaaaaa 540
anaannntan aaaaaaaann nttnacttgc ccggnggccc ttnaangggg attcncccat 600
gggggccttt tangg 615

<210> 198
<211> 557

<212> DNA
<213> Homo sapiens

<220>
<221> misc_feature
<222> (1)...(557)
<223> n = A,T,C or G

<400> 198
gggacctgca gttggtattg atcttggcac cacctactct tgtgtgggtg ttttccagca 60
cggaaaagtc gagataattg ccaatgatca gggaaaccga accactccaa gctatgtcgc 120
ctttacggac actgaacggt tgatcggtga tgccgcaaag aatcaagttg caatgaaccc 180
caccaacaca gtttttgatg ccaaacgtct gattggacgc agatttgatg atgctgttgt 240
ccagtctgat atgaaacatt ggccctttat ggtggtgaat gatgctggca ggcccaaggt 300
ccaagtagaa tacaagggag agaccaaaag cttctatcca gaggaggtgt cttctatggt 360
tctgacaaag atgaaggaaa ttgcagaagc ctaccttggg aagactgtta ccaatgctgt 420
ggtcacagtg ccagcttact ttaatgactc taacgtcagg ctaccaaaga tgctggaact 480
attgctggct caatgtacct nggccgcgaa cacgctaagg gcgaattnca cacacttggn 540
ggncgtctan tggatnc 557

<210> 199
<211> 498
<212> DNA
<213> Homo sapiens

<400> 199
acaatgatgc ttctcacagc ttcaaagaca tgtctgaggc atcctaactg cgaatcagcc 60
cataaaaaca aagaaggagt atttgaccgt atgaaagtgg cattggataa ggtcattgaa 120
attgtgactg actgtaaacc gaatggagag actgacattt catctatcag tatttttact 180
ggaattaagg aattcaagat gaatattgaa gctcttcggg agaatcctta ttttcagtcc 240
aaagagaacc tttctgtgac attggaagtc atcttggagc gtatggagga ctttactgat 300
tctgcctaca ccagccatga gcacagagaa cgcatcttgg aactgtcaac tcaggcgaga 360
atggaactgc agcagttaat ttctgtgtgg attcaagctc aaagcaagaa aacaaaaagc 420
atcgctgaag aactggaact cagtattttg aaaatcagtc acagtcttaa tgaacttaag 480
aaagaacttc atagtacc 498

<210> 200
<211> 615
<212> DNA
<213> Homo sapiens

<220>
<221> misc_feature
<222> (1)...(615)
<223> n = A,T,C or G

<400> 200
ggtaccctct cttccagcac ccaggccagt attgagatcg attctctcta tgaaggaatc 60
gacttctata cctccattac ccgtgcccga tttgaagaac tgaatgctga cctgttccgt 120
ggcaccctgg acccagtaga gaaagccctt cgagatgcca aactagacaa gtcacagatt 180
catgatattg tcctggttgg tggttctact cgtatcccca agattcagaa gcttctccaa 240
gacttcttca atggaaaaga actgaataag agcatcaacc ctgatgaagc tgttgcttat 300
ggtgcagctg tccaggcagc catcttgtct ggagacaagt ctgagaatgt tcaagaattt 360

gctgctcttt gggatgtcac tcctcttccc ttggtattga aactgctggt ggagtcatga 420
ctgncctcat caagccgtaa taccaccatt cctaccaagc agaccacaga ccttcactac 480
ctatcttgac aaccagtctg gtggncttat tcanggttat gaagcgaccn gccttgccaa 540
ggataccacc tgnttggcaa gttttaactn caggcttcct tctggacccc aggngttccc 600
aaattgaagt ccttt 615

<210> 201
<211> 256
<212> DNA
<213> Homo sapiens

<400> 201
actgcacttt ataaaagcat ggataatatt aaaggatcac aaaaggcagc attagcattc 60
tctatccagg tattattaaa tctttttatc ccatgccccc ctcaaatata ggagaattat 120
tatctgataa gcctgaaacg acttttttta ataccataac ctaaaaagac acttcttaca 180
ggtgtatgca actttggtca gcagaaacac aatacgagcc tctggcctag ctaaggcact 240
ctattctgaa agtacc 256

<210> 202
<211> 584
<212> DNA
<213> Homo sapiens

<220>
<221> misc_feature
<222> (1)...(584)
<223> n = A,T,C or G

<400> 202
acttttcaat ctgatccatt atcttctcga ctctttctgg aggcactttc ccacgagttt 60
gcatcctttc ggccacattg tggtagaaat cctgagcaca ctctgactgt tcttcaatgc 120
ttagatccct tttgtaatgc attccttcca aaaacagctt ggtctgttta tagatttctt 180
ggcctgtctt gtggaaggtc ttgagaaatt ctatgaactc cttagacact ctatccgttt 240
caatgctggt ttgccggttt atggaaggac tgggagcttt tgcttcctga atttccttct 300
ttgatccgac cctggaagaa tgcactgaag aaattcttca ctgggggaac cctgccggtc 360
ttcttgntgg gtttcttttc ttcaaacttg gaaaatgtna aggattgggc ccctgggtgg 420
gttnactggt ngcaaaggct ttttttcttc cctgaggcnt tccgcagtcc annctctgaa 480
ttgntttgcc tggcttgngg acctggccga cacctanggg aaatccacca ctgggggccg 540
tctaagganc cncntgggcc aacttggggn anntnggtan nntt 584

<210> 203
<211> 608
<212> DNA
<213> Homo sapiens

<220>
<221> misc_feature
<222> (1)...(608)
<223> n = A,T,C or G

<400> 203
ggtactctta tacacacctg ttttctccaa tgttctcctt tagtatggct ggtaattgtt 60
ttggtgattg ccaccccctc gagatgcctt gccataagtg ctctgttggc ctattttgaa 120

aacacagaat tctcatttag ttttctacaa aactttcttt acaaacacaa actattaaat 180
ctacaaatct ttgcatgcta aataaaaagt attaagatat tttagcaccc attagatgct 240
actcataaat catacatcct agttcattta taaccaccag tctatgttag tataatcatc 300
ctatgattgt aacatgcctn aaacacttaa ctccgaacac tttaatggaa agcccataca 360
cacaatttca gaacaggatt gtatgttaac aatgaatttt aataccactg ctttataaaa 420
ttaagttaaa tattcttacc actgnaatct gcatatcctg nccatatcat aggtcccata 480
ggtataccca ggataaacat attcggcata gcactatggt ttgaacacct ggcccggccg 540
gccggtncaa aaggcgaatt cancnactgg nggccggtnc natggatcca ncntcgnacc 600
aactttgg 608

<210> 204
<211> 621
<212> DNA
<213> Homo sapiens

<220>
<221> misc_feature
<222> (1)...(621)
<223> n = A,T,C or G

<400> 204
ggtacctgaa gatcttgatt tgctacacga gctttctcta gggcattata gtaagaaact 60
gcttctttct ctcgctcctc tttttcctct ttaagccggt ctacctggcg cattaggtta 120
gtaataagaa gttctagctg ttcttgtctg tattgtagtt cattcacttc ttctttgagg 180
gtggtcttca tactctccat ttctgtcagc tcaatttgaa gagccagcat ctctgaagac 240
atgctttcct gcacacgttc agacattacg cgcagttcct ctgatttaca agagaggagt 300
tccttctgat gatctacttg gtgcttcagc tgcttttcac taagcctggc ttcatctaat 360
tccactttca gtttttctat cttaagtttt taagttcatt cacttcctgc catggcttct 420
gcttagttgt cttccnattt cttcaggtgc attttttggt ggtggttaat agcttcacat 480
tcgcaagctc aaactttcta acattcgact cttgagttca acttctcttt tgaangggat 540
attttcntgg tcataactct tangcatngg gcataattct taccacatta tccaatggat 600
ccggaattca ntttgccctn t 621

<210> 205
<211> 607
<212> DNA
<213> Homo sapiens

<220>
<221> misc_feature
<222> (1)...(607)
<223> n = A,T,C or G

<400> 205
ggtaccacct atcataggta ttaccacaca atttcatgca tggtggcata ttttaactgg 60
ccttggttcc tatcttcaca tccttttcag tttgtataca agaacacttt acctgagata 120
taggccaaaa gtgaagtttc tctttggaat ctggccagtg atcctgtttg agcctctcag 180
gaagcattga tgaatcattc caccaagaaa acaaacaagc acctaccata gacctggcag 240
aataaataag gaaatcctta aagatctaca agttcaaata tgtcatgacc atcacagcag 300
aggagtgact ttctgactaa tgctgccacc cacacagaga ataaggagta gggcctgctg 360
ggtgtttagc tcatggcttt atcttatttg ccccctcctc tttcacgctc cagtttataa 420
aagaaacaga gatgatgtgt gtgtatgcct caaaatgcag aaacaggtgg gcttttctta 480
acanggtnac agtttgtgct gggtataaga aaataaccct ctttcttttn gccaagggtg 540

catgtgaatt atcccttctt aanattggtt aaataagcan tnncttanag cccccaaanc 600
nctntnn 607

<210> 206
<211> 572
<212> DNA
<213> Homo sapiens

<220>
<221> misc_feature
<222> (1)...(572)
<223> n = A,T,C or G

<400> 206
acgcgtgacg tcactcacat agcaggaaga ctcacaacct ccatccagaa gcaccatttc 60
cccatccttg atgagttgat tatttttcac atagtgcaaa gtgtttgacc gattaccacc 120
agccaccaca ggtggatagg ctaaaatgtc tgcgccacga gcccggcatt caaattcaaa 180
cttagcataa agaaaggctt cttccacagg ggctttactg gtgaacatgg tttctatgaa 240
agcctgtgat gtcagcttcc cagcaatctg cattcgttca atttctgcag gagacttgat 300
cagccggagg cgctgtatca gctgctgaac accccgaacc ttgttcttgc tcttggcttt 360
ggcctcagtc aggggctgca tatagtcaga gtgaagctgt gcatgtgagg gccttatcca 420
ggtcatacca aaccatgttc gtctcagctt tcattttttg gtagaagatg ttgaaattct 480
tctagcgtat aggcttcgtc tactccagtt agagctattg gttccatcag tgccagantc 540
gnggaccatt ccaaaaggtt tnnactnggg ag 572

<210> 207
<211> 616
<212> DNA
<213> Homo sapiens

<220>
<221> misc_feature
<222> (1)...(616)
<223> n = A,T,C or G

<400> 207
ggtacctgtc ccattcctaa aaggatttgt gggtaatgct ggcacttggt ggccaggaga 60
atcttctgac cccactctcc ctcctcttca gtcctgaaga ccccaagaac ccagttagga 120
tcccctggcc agaggtctct gtgactgcct ctggactcag cacgtgcagc agcttgggag 180
gatttgagcc agtctcaaaa acttttagcc ccagaatgag accagtgacc ccaagcagga 240
gggctgggat ctggagggaa gagagggggt ccaaggggac cctgtggctg aggccatgga 300
gaaccagtgc cagggcccaa gagacccatt tttccagtta tcagaggtga ctgacatctt 360
ctgccactgc cttgagttca gaaatttaaa aaagcttgca gcaagaaaat gccagtgtgc 420
aactgggtga ctaaagacca aagaaaaaca gttaaaaggg acagcttact tgctctctgt 480
ctcangttta acttctcacc tgaaatctct nataccctaa ttaacacaac caaagtctct 540
ttcatagata ggctactttt aagtttnact gcttctgtgg tgggctttgg gggctttgga 600
agtgggaatt ttttgg 616

<210> 208
<211> 614
<212> DNA
<213> Homo sapiens

<220>
<221> misc_feature
<222> (1)...(614)
<223> n = A,T,C or G

<400> 208
acacaacgtc atgaggttat tcgaaccaca gcgtcttcag aactttcaga gaaaccagct 60
gagtctgtca cttctaaaaa gacaggaccc cttagtgccc agccctctgt tgaaaaagag 120
aacttggcaa tagaaagtca atcgaaaact cagaaaaaag ggaagatgtc tcatgacaaa 180
aggaagaaat caagaagtaa agccataggc tcagatactt ctgacattgt gcacatttgg 240
tgtccagaag gaatgaaaac cagtgacatc aaggagttga atattgtttt gcctgaattt 300
gagaaaaccc acctagagca tcaacaaaga atagaatcta aagtttgtaa ggcagccatc 360
gccacatttt atgttaatgt taaagaacaa ttcatcaaaa tgcttaaaga aagccagatg 420
ttgacaaatc tgaaaaggaa gaatgctaag atgatttcag atatcgaaaa gaaaaggcag 480
cgtatgattg aagtccagga tgaactgctt cggntagagc cacagctgaa acaactncca 540
acaaaatatg atgaacttaa agagagaaag tctttccttt ggaaagcaca tatttcttat 600
ctaatttaaa canc 614

<210> 209
<211> 610
<212> DNA
<213> Homo sapiens

<220>
<221> misc_feature
<222> (1)...(610)
<223> n = A,T,C or G

<400> 209
acactgtttt gatggaagag gacattgtgg acacgaagta actggagatg gccttcagaa 60
tcagctgagc tgctgtctgc tttggaaaac cgttcctgcc gctgccgatg gatggaaatg 120
caatggattt cagcttctta tcatcagcca gggccaagca gtttttcact gtcttttcca 180
gaagttcttc acacttgtct gcaccccaaa ctggactatt acagtggatc acaaacttgg 240
caggcaggcc atggcctgcg ctgacagcag ctccagctac ttccaagggc ccgttctttt 300
tccggagttc caggacagct tccacaaact ccttgccacc tttcttctcc agcgtgtttc 360
ctaggtcatc tttaaggtca atgtcagcat tggtaggatt gattatggcc tncacctcaa 420
aagcccggct aaatactgat ttcactgnga ataanggtca acttttgggc canggaaaag 480
ctctttggtg gaaaaggact gtgaaaaccn tnggcaagng ggccctcggg tgggctttnn 540
gggcttgntg gcnttaaggg antnancngn gttttnggaa ttccggnccc tttttggccc 600
cnggttttta 610

<210> 210
<211> 589
<212> DNA
<213> Homo sapiens

<220>
<221> misc_feature
<222> (1)...(589)
<223> n = A,T,C or G

<400> 210
ggtacccagc tctaattact ggccgtagca gcatattgct taagaatttt gtagaactta 60

tttctcatca gcagctgtcc aaaggactga taaatagaga cagatcccag tcctggatac 120
tttctgtaaa tcctaatcgg agactcactt ctcagcaatg gaggctgaaa gtcttagtga 180
gactcagtaa attccttcag gccttggcag atggatccag taggttgaga gaaagtgaag 240
gacttcagga acagaaagaa aatccccatg ccactagcaa ctccattttt atcaactgga 300
aggaacatgc caacgaccag caacacatcc aggtttatga aaatgggggt tcacagccaa 360
atgtcagttc acagttcagg ctacggtatc tggttggagg actgagtggt gtggatgaag 420
gcctgncatc tactgaaacc tgaaaggatt attgngataa taattccttg ntnaatgaat 480
gctggttgaa ctgtacctgg ccggccggcc cttaaaggnc aattcngcca cttgggggcc 540
gactaaggga nccncttggg ccancntggg gnaacanggc aannttgtn 589

<210> 211
<211> 590
<212> DNA
<213> Homo sapiens

<220>
<221> misc_feature
<222> (1)...(590)
<223> n = A,T,C or G

<400> 211
acgaactgta gcatcagcta caactgccat tgaaattcgt aggcaatcca gtagttatga 60
tgattcctgg aaaataacag atgaacaaag acagtattat gtaaatcagt ttaaaaccat 120
tcagcctgat ctaaacggat ttattccagg atctgcagct aaagagtttt ttacaaaatc 180
aaaacttcct attcttgaac tttctcatat ttgggaactc tcagactttg ataaagatgg 240
tgcattgaca ctggatgagt tttgtgctgc ttttcatctg gtggttgcta ggaagaatgg 300
ctatgattta ccagaaaaac ttcctgaaag cttaatgccc aaactgattg atttggaaga 360
ttcagcagat gttggggatc agccaggtga ggtaggttat tcaggctctt ctgctgaact 420
cctncaagca agtcccatcg atgccattac ttaacccgac ttggnctgac tgaatcaaac 480
cntgaccatg ggaaacatta nngacgcttt ttaagctaca aantttggnc ccattggttt 540
taaatttggc ccnattgnac cggaaccgga ntgggnattc cgnnccattn 590

<210> 212
<211> 614
<212> DNA
<213> Homo sapiens

<220>
<221> misc_feature
<222> (1)...(614)
<223> n = A,T,C or G

<400> 212
   ggtacattcc attactaaat gccacataac tgtttggata acataagaag agtgggtcat 60
   tatatgatac caattagaag atattaggga tggtggaggc agtaatttct gggataagaa 120
   ctataattta cagaataacc agacatcatc tgatctggtg aaacctgtgc attcccacaa 180
   ttaggctttt tcacactttc tctctttaaa tgtgcaacac cttccccatc ccctctttac 240
   ttgtagcaag ttgattttgc ttcttatatc ccgagaaagc aactaccacc aaatctacca 300
   gtcaactcat ctatatttga acttaaagat ctttatgtta gaatggaatc tatccatgtt 360
   ccagcttagg cgaagccctt ctgaagatat ccattccttc cttcctcatc aaattttcct 420
   tcttgactag gattaaaaaa attcaaccag taggcataat ccgaaccttt ggnctcataa 480
   tgaaaaggat agttaataag gctcatcaat tgggccgnaa ttttgntttg ggtcaagngt 540
   tggccaaagc nncnnaaang gccccanttt tgggtaaaan tttttnaggg gttaaaancc 600
   anggggntnc annn 614

<210> 213
<211> 624
<212> DNA
<213> Homo sapiens

<220>
<221> misc_feature
<222> (1)...(624)
<223> n = A,T,C or G

<400> 213
ggtacctctc ttgtcatcaa attttgccca gttatttaat gttggattcc tcaaggctca 60
gtcagcacct tttaagccac tctaaactcc cactaatgga taagctcatt tacttccaag 120
gcttcaatgg tcacaataca acactgctgg ctctccaact tatttttcta taaaataaaa 180
aataataaag gaacaacgta tttttctatt caagactttt tatctgagct tcagatacat 240
atatccaatt gcttacttga catctccact tagaggccag aggcatttaa actcaatacg 300
tcttaattca atctcatgat cttccctctg aaatctaatc tcctactctt ccctatctta 360
atgaaagaca acaccatccg tccctttaca ttaagtgctt cagcttatcc ctacatctat 420
ctcatcacta aagaacaggt attttcaccc ttttgagtat cattcaaatg cnttctactt 480
cttttccatt cntactggta cccccctang ggnaagntat taactttttc ctacctacng 540
ncccttttgn ancccttcca tcaantnttc cnaattgnga nggtnaattt ttnnaacccc 600
aanntggnca tacnnngtgg gnng 624

<210> 214
<211> 612
<212> DNA
<213> Homo sapiens

<220>
<221> misc_feature
<222> (1)...(612)
<223> n = A,T,C or G

<400> 214
ggtacaagtc tgttaatacc ctatgtggtt tcattaggat aactttttac ctatccttga 60
ggtcatccat attcttacag gccttccagt caataatgga agagctcact ctatacaaaa 120
ccaatatgca aggcatgtgt ttgtccaagc aattggatgt gtgcagtagc caatttcatt 180
tactgcatta ctctttggcc tgggaaccct gtggtctgca ctacatgtga atggccttcc 240
acttcagtct taggcagatt tgacctttta ggggcagcaa tgctgaagga cacagcaatt 300
taaattataa tgtgtcaggc tgtgttttca cttcaaacat gtatgagtag tcagctgtaa 360
ttagagaaat gatgacttcc taagagttca gccacgcata attctagatt tcaagagcat 420
ctaagacttg tggattacct catggcatga gagtttcaga ctcagccntn tgagccagtc 480
nagggaaagt ggagtctgca acgcaaatga aaacctggct ttggggccaa nggacttggc 540
tttaaatggg cccccttngg cctgggnttt cctcttttgg cnaaantttt ngtnnccaan 600
gaaagtaatn ag 612

<210> 215
<211> 618
<212> DNA
<213> Homo sapiens

<220>
<221> misc_feature
<222> (1)...(618)
<223> n = A,T,C or G

<400> 215
ggtactcggg aggctgatgc agcagaattg cttgaaccca agaggcggag gttgcagtga 60
gctgagaacg tgccattgca ctccagcctg ggcaagagag cgagactcca tctcaaaaaa 120
aaggtgagaa agataggtgt gaacatgagg tggcaggtgt gaagatagga aaggcaggct 180
cacccctgat gacatgcagt tagagagacg ggggcttccc tttcactttg gagagtaaag 240
agaaggctct gaggtatcaa cagcctgggc tgttgggaaa aggacaaaga atctgtgttt 300
cctgaacgcc aagaggaagt ctctttggtt gctgtgggct aactggtctc ctccagttcc 360
aagaggtcat ccacatattc cacaacttct ccctcatcat catccattat attttcctta 420
nccaaagtca tacaagcttc ntctggagtg gtggncacat ttaagaactg aactgnttta 480
agnctgggct ggaantgctc attcnanagg ccccantggn cctnngggan ctngccngcc 540
ggcccnttaa aggcgaattc cancanntgg gggccggttt tangggancc aacttgggnc 600
caacttggng aaatatgg 618

<210> 216
<211> 595
<212> DNA
<213> Homo sapiens

<220>
<221> misc_feature
<222> (1)...(595)
<223> n = A,T,C or G

<400> 216
ggtactccca ttcagggtga cgaagtgggc agaactggga gccatcttgc ccagcccctt 60
ggtgctatgt ttaccttgaa gcaatccttc ggccttagga ttggcctcta gtagttcatt 120
acactgacct agagctacct ctgataagag cagcagtcct gtattcttta ggcgagaggc 180
aaagcagtaa ttggcactct tggaagacat gtcagcaaag tagattcctt tcccaaacat 240
gtaacctgtg atgggagctt caggtggggc aattcgaagc ccatggctca agattcccac 300
ccagttactc atcctggaac catgccatag aagcatcctg ttatgaaggt cctctctgaa 360
ggcttctttc tcaccatcct tctcacttca aacaaatcca gcaaggtcat ggtataagtc 420
gctgtgtgtg ggaancatgg gtagaatgga aggtacctgg cccggccggc cnttcaaaag 480
ggccaaattc cagcacaatt ggnnggccgt tactaaggga tnccaacctt gggncccaaa 540
cnttggngga atcatgggcc naaactngtt ccctggnggn aaattgnaan cccnn 595

<210> 217
<211> 610
<212> DNA
<213> Homo sapiens

<220>
<221> misc_feature
<222> (1)...(610)
<223> n = A,T,C or G

<400> 217
actgaaaact ttttttaaaa aaggtgatga tgaagtgcat tctgtagcag cagcgcagct 60
atgctttaaa ccacacaaaa ggctgtgtcc aggtgcagcc tccttcaccc ttcctgccca 120

cggtgaggat tgaataacca ggacttgggg atattgtttg ttgtcagggt tattctgtgt 180
ggtaaggaat atttgtttca catttataca ttttcttttt ccactcacgt aagtttctat 240
cttgagagca tagtccaaag tgcaaaactt ggtgtttaca aggaaaattg tcttccagaa 300
ctccactgtc atcactttca ccaaagtgga agtttgcatg aatatgctca gaatctaata 360
ttcaatgttc tgttacattg taagtgaagt ccagctcaaa atagatttaa tatattgaat 420
ttatttgnac cntnggccgg gaacacgcct aagggcgaaa ttncagcacc actggccggg 480
cggttcctaa ngggattccc aaactntggg nnccanactt nggcgnnaan cnatngggcc 540
taaaacttgg tttcccctng nngaaaattg ggttatnccg gttacaaatt tcccncncaa 600
atttccgggg 610

<210> 218
<211> 585
<212> DNA
<213> Homo sapiens

<220>
<221> misc_feature
<222> (1)...(585)
<223> n = A,T,C or G

<400> 218
ggtacaattt gtaaatattt caaaggtcta ggagtcataa ctttttgttt tcatactgaa 60
aatgatgttg atcagagaaa ccaactgttt tgcttttcat tgctctgtga gaaatttgag 120
gattctgttt tgctgttagg taagctaaac tcagaaattg aaaaggaaaa gactggataa 180
acacaggatt ttcagtaaga aaacaacccc agtcttgtct tagaagccac ttgttgagga 240
gtctgttggg ggaaaaaaga ggatatgctt ttaaaggtag aacaaacctt cttctgtgtt 300
aaatcaaaag gatgttcaaa atccaccagg acagatgcta cttgggttta aatggagcca 360
tagatgatac aaagtcctct tggggctgaa aatcacttcc tatttgcatg gctttactaa 420
ctggtttctg ttttccatta tctttttcac agaaagtntt tggtcaagat tttttccagc 480
ctttnaaatt gaaaccggtc agtantttga cccctgnttg gntatttnnt ccagnaattn 540
aaattgnatt cnctggntcc aaaggcntta attccccttc cttng 585

<210> 219
<211> 599
<212> DNA
<213> Homo sapiens

<220>
<221> misc_feature
<222> (1)...(599)
<223> n = A,T,C or G

<400> 219
acaggtcaca gatcctacaa tcctactgtg gcttgtgtct ctttttccga ggcacatcct 60
caaccttgga aaaataaact tttaaattga ttgagacttg cctcagtgat tttctttggt 120
gtatactctg tatcacttga atactttcca agtgaagaca tgctttataa tccagagtat 180
ggactgtttt ggccagatgt tttctatata ctggaaagaa atgtgtattc tgctgttgtt 240
gaatggcatg ttctataaat ctcaattaca tcaagttggt tgatagtctt gatgtcttct 300
atatctctgt ggatttccca tttgttctag tgattattga gagaaaggta ttgatatatc 360
tgcctataat tctggattta tctacttctc tttggagatt tctccatttt tgcttcatgt 420
attttggaag cccctacttc acccagcatn ggnctttctt gagccccttc caagaagtaa 480
ttttaaccac ccangnccca tccaacccct aaccccaang gnnaaccaac cgnnggcang 540
tnanttgggc ctaaccnggg gaacccattg ggggnccttn ggnattaggg ganaccnng 599

<210> 220
<211> 602
<212> DNA
<213> Homo sapiens

<220>
<221> misc_feature
<222> (1)...(602)
<223> n = A,T,C or G

<400> 220
ggtacccatt taatataact atgatgcact taaattgaag ctatgccaca ggatagaaaa 60
tgaattacaa cttaaataca tgttggaagt gtaacactgt ttttcaaggt ttaaaaaaat 120
tcctaatgtc ttttagcctt ctttaatatt tttaggtaag gaaagtatgt ttggattttt 180
tcctctttgt aggtatatga gattgaaatg tgaagtattt ggacaacaaa cgtcaagcaa 240
tgggaagcca ttttgatttc ttgagtaatc ttgtaagcat taagtgaatg acaaagtagt 300
agtgtaactt atttcttatg gtataacttc agtcaattaa tataaggata gtttttgttg 360
tatgtacact aagtggtaat ataatngcca ttgaantata ctaatctttc tcttaanaga 420
ctattcnnct nttaattgnt tcctaatggg aacanttntg gcctaacccn gaaaaagggg 480
ganaaaggat tnccctgccc nggccgggcn tttccaaagg ggcanatttn cgnncacctt 540
ggnngcccgt tntctanngg aatccnannn tggtcccaan anttgggggg aatcttnggc 600
nn 602

<210> 221
<211> 573
<212> DNA
<213> Homo sapiens

<220>
<221> misc_feature
<222> (1)...(573)
<223> n = A,T,C or G

<400> 221
acctaatgaa aagatctcca agaggtttgt ctcattctcc ttgggctgta aaaaagatta 60
atcctatatg taatgatcat tatcgaagtg tgtatcaaaa gagactaatg gatgaagcta 120
agattttgaa aagccttcat catccaaaca ttgttggtta tcgtactttt actgaagcca 180
atgatggcag tctgtgtctt gctatggaat atggaggtga aaagtctcta aatgacttaa 240
tagaagaacg atataaagcc agccaagatc cttttccagc agccataatt ttaaaagttg 300
ctttgaatat ggcaagaggg ttaaagtatc tgcaccaaga aaagaaactg cttcatggag 360
acataaagtc ttcaaatgtt gtaattaaag gcgattttga aacaattaaa atctgtgatg 420
tanggagtct ctctaccact ggatgaaaat atgactggga ctgcccttga ggcttggtac 480
cnttggcncc aancccttgg gaaccccaaa aactntggaa gagaannggg gttttcctgn 540
caggcaacat attgcctttg gcctnctttg ggg 573

<210> 222
<211> 168
<212> DNA
<213> Homo sapiens

<400> 222
ccaccatctt ggaacgggag gcggagcaga gtcgactggg agcgaccgag cgggccgccg 60

ccgccgccat gaaccccgaa tatgactacc tgtttaagct gcttttgatt ggcgactcag 120
gcgtgggcaa gtcatgcctg ctcctgcggt ttgctgatga cacgtacc 168

<210> 223
<211> 564
<212> DNA
<213> Homo sapiens

<220>
<221> misc_feature
<222> (1)...(564)
<223> n = A,T,C or G

<400> 223
actgcagaca aaatctgctt ttagaggcaa gcggatttct gacaaagtaa ctgatccttt 60
ggatggcata aattcacttt ggggactagc cttattcttc ctctgaggtc cttcgttctt 120
caatttattc aattcatcaa tcaaaagtgt tctcttccca gttgcaatta gaagaagtct 180
ttctgcttca gcttcttcta ggggcccttt tccatgttct tcatcaacac agcagttaag 240
agcctggcta gcttgataga tcactgtctg ttgcatattt atttcgttat tgagttcctg 300
cattttctgt ttgatattaa cttgacaagg aaaggcatta tttttttcat ccagttttga 360
agtaacatct tccttccgaa caatcacctg ctttattgat ggacgttctg tttctttgaa 420
tctttgagat ctatatgcat caatgctgta aagaagatca cgatcttcag aaccaaggct 480
atcacnagat tcaggtcgag ggacacgaag ttctttngaa tttcctgggt ttggactttc 540
atcacttctg ctggngcttt caan 564

<210> 224
<211> 277
<212> DNA
<213> Homo sapiens

<400> 224
acaaggctgg cggttgttgg gggacggttg agccttggga gggagggtca gggtctggac 60
aggagccgcg gccgccagat gggaaagaac acgtgggagc agtaatgtca agtgacactt 120
aaacccttag acgccgattc gttataacgc gaggaaatct aatcccacgt ccctaacggt 180
cttcggaagc gaagcagtgt caacagtccc tggtaaacac aagtagtatt acaagtcggg 240
agctcttcaa gtcttggatg agactgtaga gcggacc 277

<210> 225
<211> 589
<212> DNA
<213> Homo sapiens

<220>
<221> misc_feature
<222> (1)...(589)
<223> n = A,T,C or G

<400> 225
ggtacctgga ggctcaacgg cagaagcttc accacaaaag cgaaatgggc acaccacagg 60
gagaaaactg gttgtcctgg atgtttgaaa agttggtcgt tgtcatggtg tgttacttca 120
tcctatctat cattaactcc atggcacaaa gttatgccaa acgaatccag cagcggttga 180
actcagagga gaaaactaaa taagtagaga aagttttaaa ctgcagaaat tggagtggat 240
gggttctgcc ttaaattggg aggactccaa gccgggaagg aaaattccct tttccaacct 300

gtatcaattt ttacaacttt tttcctgaaa gcagtttagt ccatactttg tactgacata 360
ctttttcctt ctgtgctaag gtaaggtatc caccctcgat gcaatccacc ttgggttttc 420
ttanggtgga atgtgatggt cagcaacaaa cttgcaacaa gactgggcct ttggttggta 480
ctttnnaaaa ggccncnttg atcccatttg agnaattncn cccggcccaa aaaaaggtcc 540
taangttggt aaaatttgca agctttttaa ggtttgccca aagnatgnt 589

<210> 226
<211> 636
<212> DNA
<213> Homo sapiens

<220>
<221> misc_feature
<222> (1)...(636)
<223> n = A,T,C or G

<400> 226
ggtcaagaag catgccacct ccacaactcc tacctggacc tccagcgcag gtatgggaga 60
ccctcgatgt gcagagcctt cccctgggag aaggagctga aagacaaaca ccccagcttg 120
ttccaggcat tgctggagat ggatctgctg accgtgccaa ggaaccaaaa tgaatctgta 180
tcagaaatcg gtgggaagat atttgagaag gctgtaaaga gactctctag cattgatggt 240
cttcaccaaa ttagctctat cgtccccttt ctgacggatt ccagctgctg tggataccat 300
aaagcatcct actaccttgc agtcttttat gagactggat taaatgttcc tcgggatcag 360
ctgcaggggc atgttgnata agtttggttg gaggccnngg ggagtgagaa gctgcttcaa 420
tgaatcttgg gtataaacac taccaaggta ttgacaacta ccccctggac ttgggaactg 480
ncgtatgcct actacagcaa ccntggccnc caagaaaccc cttggaccag cacacacttg 540
gaaggngaat caggcctttt gttgaaacca tttgacttaa aggattgttg gaaatcttca 600
nggnaccttg cccggcgggc cctttnaaaa ggggna 636

<210> 227
<211> 451
<212> DNA
<213> Homo sapiens

<220>
<221> misc_feature
<222> (1)...(451)
<223> n = A,T,C or G

<400> 227
acccaaaaac cacccccaac gccccccaac cctcaggcgt gcctgtgagt gtgtctgtgt 60
gtctcactct gactcaccca gacaactgac ttcagcagcc aaccttggtc attcccagaa 120
ccaccactgg ggggcatacg tgtggctaga ctgggggcgc ccgaatatct gtctctacaa 180
aaagtaaaaa aaaaattaat ggggtgtggt ggtggtgcgt gcctgtggta tcagctgctt 240
gggacgctgg ggcangagga tcacttgagc ccgagaattc aaggctacag tgagttaaga 300
ttacgccact gcactccatc ctgggtgaca gagcaagacc ttgtctcaag aaaaaatttt 360
taaatgagta aaattcaaaa aaaanaanaa aaanaaaagc ttgacacctg aaacatgggt 420
tactgcatat ggnacctngg cngagacacg c 451

<210> 228
<211> 408
<212> DNA
<213> Homo sapiens

<400> 228
ggtcccttat atggcagaat cttgcaggca gcatgtcgag tttgatatgc tggtgaagaa 60
tagaacccaa ggaatcattc ctttggcccc catatctaaa tcattgtgga cttgctcagt 120
agaatcttcc atggaatatt gtagaataat gtatgatata tttcctttca aaaagctggt 180
gaattttatt gtgagtgact ctggagcaca tgttttaaat tcttggactc aagaagacca 240
aaatttacag gggctaatgg cagcattagc cgctgttggg cctcctaatc ctcgggcaga 300
tccagagtgc tgcagtattc tgcatggcct tgttgcacag tggaaactct ctgcaaaatt 360
actgaatacc aacatgaggc tcgtacctgc cccgggccgg ccgctcga 408

<210> 229
<211> 270
<212> DNA
<213> Homo sapiens

<220>
<221> misc_feature
<222> (1)...(270)
<223> n = A,T,C or G

<400> 229
ggtacacagc agcatcaaaa aggctattta caagagattt tcttcaacag aatccacttg 60
aaagcactga gaatttgcat cttagctaag agcagtttac caaggaacag ggccatctaa 120
gtgcctaact agcatttaaa gttgtcaagg ggtggggatg tgcaaattaa gcagcaaaag 180
attattatct tgttntgctt taagggaaag taatantggt cagaggggcc agttccaagg 240
gctggtccaa gggggccgc tggtcttggt 270

<210> 230
<211> 425
<212> DNA
<213> Homo sapiens

<220>
<221> misc_feature
<222> (1)...(425)
<223> n = A,T,C or G

<400> 230
ggtacattat ccaatttcag ggaaaaaaaa tacagttttc ttaccaaatt atccagtgta 60
tatgactggt tagaatttta agttttgatt tttactgaaa ttcagagtat gaaatgcaaa 120
cattcaggat aaaatgaatt cataattaca cacagttata tcaacttgca acaaagcagc 180
aaatatgagg gcctaacaca catctcgact ctccccttcc cttctgatcc ctcaaaaaaa 240
agtgcaaaat caaagagtca ctgcttggtc caaaaaataa aatacattgt gtataaacat 300
ttgaaatctg atggaatcca gcttctattc cacaggttgt cttcagtaag aatcaacgtc 360
cgaagatgga actcagttcc agaagaatta attctacaat ctgattctgg tcctgccggg 420
cggnc 425

<210> 231
<211> 639
<212> DNA
<213> Homo sapiens

<220>

<221> misc_feature
<222> (1)...(639)
<223> n = A,T,C or G

<400> 231
gcgtggttcg cggccgaggt actccaagaa gtctgtctgc cattgatagg gctggagcag 60
aggtgaagag tagaacaacg cttttcagaa agattggaga ctttagaagc ttggagaaga 120
tttcacggga agtcaaatca attacgatta tcggtggggg cttccttggt agcgaactgg 180
cctgtgctct tggcagaaag gctcgagcct tgggcacaga agtgattcaa ctcttccccg 240
agaaaggaaa tatgggaaag atcctccccg aatacctcag caactggacc atggaaaaag 300
tcagacgaga gggggttaag gtgatgccca atgctattgt gcaatccgtt ggagtcagca 360
gtggcaagtt acttatcaag ctgaaagacg gcaggaaggt ngaaactgac cacatagtgg 420
cagctgtggg cctggaaccc aatgttgagt tggccaagac tggtggcctg gaaatagact 480
cagattttng tggctttccg ggtaaatgca tnacttccag cacgctttta ccatcttggg 540
tggcangaaa atgctgcatt gcnttctacg atntaaaagt tgggnaagga ggccggttan 600
aacncccntg aacncccttt tgtgantggg aaaattgcn 639

<210> 232
<211> 369
<212> DNA
<213> Homo sapiens

<400> 232
ggtactaaaa ggcctcaaaa taattagtga cagaaatagt gttattaatt tgctaagctc 60
aacaataagc aattccttaa ttaaaatctt cgagatataa atttgatgac tattctcttc 120
agaaatgaca tacctggatt atgttaatca tcacaagcct tattagtcac acatataaac 180
atggcctcat gcaatcattt gtctgtatat gttactctaa gttgcatgag cacaaggttt 240
aatatctata tctttaagaa aatacttgat attataaaca gagtaaaaga catgatatag 300
tagtgattac taaaaaaaaa aaattagcag cttaaatcta tctatatttg aaaaaacgta 360
gtcacaagt 369

<210> 233
<211> 618
<212> DNA
<213> Homo sapiens

<220>
<221> misc_feature
<222> (1)...(618)
<223> n = A,T,C or G

<400> 233
accctctctt ccagcaccca ggccagtatt gagatcgatt ctctctatga aggaatcgac 60
ttctatacct ccattacccg tgcccgattt gaagaactga atgctgacct gttccgtggc 120
accctggacc cagtagagaa agcccttcga gatgccaaac tagacaagtc acagattcat 180
gatattgtcc tggttggtgg ttctactcgt atccccaaga ttcagaagct tctccaagac 240
ttcttcaatg gaaaagaact gaataagagc atcaaccctg atgaagctgt tgcttatggt 300
gcagctgtcc aggcagccat cttgtctgga gacaagtctg agaatgttca agatttgctg 360
ctcttggatg tcactcctct ttcccttggt attgaaactg ctggtggagt catgactggc 420
ctcatcaagc gtaatacccc attcctacca agcagacaca gaccttacta cctattctga 480
caaccagnct ggtgngctta ttcanggttt attaaaggca accttccctg acaaaggata 540
ccacctgctt ggcaaggttt gaactcccag gcctgccngg aaggaatgcn cggggggatt 600
nctggggggg ggnccncn 618

<210> 234
<211> 603
<212> DNA
<213> Homo sapiens

<220>
<221> misc_feature
<222> (1)...(603)
<223> n = A,T,C or G

<400> 234
accagatgga aaatgttttt ggtgatctgg ctgctgctta aagccagttt tccctaagaa 60
ctccaaaggc taaactctac taggggcaga gtgtgaggat agatttctaa tcagagaaaa 120
gtggcctcca ggagctttca tttatgtctt ctccagacca ggttttcctg ttatcttcct 180
ttaatcccct ttcaaccaac aggtgaagtt cttccagccc acagaggtag taatatcatc 240
ttttctatct cctcctctcc tttggccatg taatgaagca aaatattatt tatttagccc 300
aggcttgaga gccactgttt gtggacagtc ttcatctaga ttccataccc tggcctaggc 360
gaggtaaggc tctctggtta ttgccaggat ggagcccctc taccccangt ctgctgtang 420
gaatacccta attagttgan gcatgctttt ggaatcctgc atgttggcat atggctggnc 480
tatccttttt aaaanctctg ggtgggggna tctggatatn gattaagang ggacaaggag 540
ccttttcttg gctaanggtt ncaatacctt tttgaatggg gccagccctc aggcttccca 600
ccc 603

<210> 235
<211> 328
<212> DNA
<213> Homo sapiens

<220>
<221> misc_feature
<222> (1)...(328)
<223> n = A,T,C or G

<400> 235
gcgtgtcgcg gccgangnac atggacnaca ggtgangaac aggtgaacat ggaggttgta 60
gancccangg gagggggagt cacttggttt ggggcaaact tgctaaatgc aggaccacag 120
gaaccanctn ttcanctncc gtgaganttt ggctgcccan gccanttagg ggtgtgggcc 180
tgcacggnag acagttatcc ctttctantc tggctcgtgg gactntnnan ggantcantc 240
tgcaacagta agtggtgant tcttctgncc ancgtcagta ttttgatggt ggctttagac 300
ttgccagatn acactacntn acatcagt 328

<210> 236
<211> 352
<212> DNA
<213> Homo sapiens

<400> 236
ggtacacctg ttaggagctc tatcactctg aaagccaaaa gatagaatgc tcatttgagc 60
atttgcaaaa tgttctctat ttatattttt aaaaatctga tacatgtaag tttttctggc 120
agattctttt tgtatgttac aaaacaaaac atcaaaagct cagagtaaga taagaatccc 180
tttttcttag aaaggtcaag cagatacttc ttgacatcat gtcctttata caatggcata 240
ttgttcatat aaaaggtctc ttatcctata aaaatcttga caaaggcagc cttctaatcc 300

aatgcgtcca gtttccgttc tgcggactgc tacttgattg ttgcaaacaa gt 352

<210> 237
<211> 607
<212> DNA
<213> Homo sapiens

<220>
<221> misc_feature
<222> (1)...(607)
<223> n = A,T,C or G

<400> 237
   ggtacaaatg cgcttccagc aggaggtcat ggacagccct atggaagagg tcctgctggt 60
   caatctttgt gaaggaacct tcttaatgtc ggttggtgat gaaaaagaca tcctgccacc 120
   gaagcttcag gatgacatct tagactctct tggtcagggg atcaatgagt taaagactgc 180
   agaacaaatc aacgagcatg tttcaggccc ctttgtgcag ttctttgtca agattgtggg 240
   ccattatgct tcctatatca agcgggaggc aaatgggcaa ggccacttcc aagaaagatc 300
   cttctgtaag gctctgacct ccaagaccaa ccgccgattt gtgaagaagt ttgtgaagac 360
   acagctcttc tcacttttca tccaggaagc ccgagaagag caagaatcct cctgcaggct 420
   atttccaaca gaaaatcttg aatatgagga acagaagaaa ccngaagaaa ccaagggaaa 480
   aaactgtgaa ataagactgt ggtgaattag aatggctaga gctaccccca ttntnggctt 540
   tagccctgcc aagtggcagg ntcancaact gtcagnttcc naatcctaat cntactttgg 600
   gnnntgg 607

<210> 238
<211> 391
<212> DNA
<213> Homo sapiens

<220>
<221> misc_feature
<222> (1)...(391)
<223> n = A,T,C or G

<400> 238
acaaacttag aagaaaattg gaagatagaa acaagataga aaatgaaaat attgtcaaga 60
gtttcagata gaaaatgaaa aacaagctaa gacaagtatt ggagaagtat agaagataga 120
aaaatataaa gccaaaaatt ggataaaata gcactgaaaa aatgaggaaa ttattggtaa 180
ccaatttatt ttaaaagccc atcaatttaa tttctggtgg tgcagaagtt agaaggtaaa 240
gcttgagaag atgagggtgt ttacgtagac cagaaccaat ttagaagaat acttgaagct 300
agaaggggaa gttggttaaa aatcacatca aaaagctact aaaaggactg gtgtaaaana 360
aaaantgtna nnaaaaaaaa agcttgtcct n 391

<210> 239
<211> 466
<212> DNA
<213> Homo sapiens

<220>
<221> misc_feature
<222> (1)...(466)
<223> n = A,T,C or G

<400> 239
gggagggaga cgggggagag agagaaaaaa aaaaaaaaaa aaaaaaaaag cttgtgttgg 60
tcccagcggt tcagctgagg tagggacgtg ccgtaggccg gaatgttacc ggctgttgga 120
tctgtggatg aggaagagga tcctgcggag gaggattgtc ctgaattggt tcccattgag 180
acgacgcaaa gcgaggagga ggaaaagtct ggcctcggcg ccaagatccc agtcacaatt 240
atcaccgggt atttaggtgc tgggaagaca acacttctga actatatttt gacagagcaa 300
catagtaaaa gagtagcggt cattttaaat gaatctgggg aaggaagtgc gctggagaaa 360
tccttagctg tcagccaagg cggagagctc tatgaaagag tggctggaac ttagaaacgg 420
tttgcctctt gcttgttcan tgaagtgagg aatgtgttta ctgggt 466

<210> 240
<211> 616
<212> DNA
<213> Homo sapiens

<220>
<221> misc_feature
<222> (1)...(616)
<223> n = A,T,C or G

<400> 240
ggtacaactc ttgctaatgg aatgctataa tgcacaaggt caaggattta ataaattcta 60
aaagtgtcta catatatcag tgataactgt attattagaa atataaatgt atagaaatat 120
aaagtatatg gtattaaaaa cagaccttgc taatataaac atatataaag tatgtcactt 180
ctcctgtaat aacagcataa agatcgatct acagtttgcc cttcgcctgg cactcttaaa 240
ccactcctcc aatggtcaat gttgaccttg aatcaacagc cgctgaaccc aggagacccc 300
acagatgtgt agattcagca cctanagggc ccccctaccc tctgtgctgt gtgttcccat 360
gactccagaa ataattaatc gcaacttgca ttattaagtc cacaggcaag ttttgaaatc 420
taactagaaa aagtagcagc aaaggccaaa ataccgcggg aatttgttaa gaaaagcaac 480
cagaatttct taaaatgctt tcanttcaag gtctgaatta aggtgacntt aggtcccacc 540
agcnttaacg nagttggggn atgttttgct gntggttttt naaaaaagaa gaatctgcna 600
taaacatgtc ctttgg 616

<210> 241
<211> 598
<212> DNA
<213> Homo sapiens

<220>
<221> misc_feature
<222> (1)...(598)
<223> n = A,T,C or G

<400> 241
ggtactctat gaatgtgtta cccaggagac cccagagatg ttgcctgcat acatagcaat 60
ggatcaggct ataagaagac ttgggagaag agaaatgtct gagacttctg aactttggca 120
gataaagttg gtgttagagt ttttcagctc ccgaagccat caggagcggc tgcagaacca 180
ccctaagcgg gggctcttta tgaactcgga attcctccct gttgtgaagt gcaccattga 240
taataccctg gaccagtggt tacaagtcgg gggtgatatg tgtgtgcacg cctacctcag 300
cgggcagccc ttggaggaat cacagctgag catgctggcc tgcttcctcg tctaccactc 360
tgtgccagct ccacaagcac ctgccaccta taggactaga agggagcaca agctttgctg 420
aactgntctt caaatttaac agcttaaaat gccagtgcga gctttgttga natggctcct 480

ttgcttcttg gaaatccaca gccatggtga tgtgaccgtg ttggccggga acctacctga 540
acgtgacttn tggcacaacg tgaccaacct naaacttaag catgttttaa gtttangg 598

<210> 242
<211> 565
<212> DNA
<213> Homo sapiens

<220>
<221> misc_feature
<222> (1)...(565)
<223> n = A,T,C or G

<400> 242
acagagcttc gggtagcaga agaggaatgg cctatggaca tattgactct tatggggcag 60
atgatagtga ggaggagggg gctgggcctg ttgagcgacc gccagtgaga gggaaaactg 120
gcaagtttaa agatgataag ctgtatgacc cagagaaagg ggcaaggtct ttggctgggc 180
cacctccaca tttctctagt tttagccgtg atgtgagaga ggagcgagac aagttagacc 240
cagtccctgc agcaagatgc tcagctagca gagctgactt cctgccacaa agtagtgtgg 300
ccacacagtc gtcttctgaa ggcaagctgg ctacaaaagg tgacagctcg gagagggaga 360
gaagggagca aaatttacct gcacgttcca ncagggctcc tgtgagtatt tgtggtggtg 420
gggaaaacac ctnaaagaag tgcagaggaa cctgtggtca ggccccaaat cagaaacctg 480
gcaggtccaa ctgcgtgaaa cccaaaattt ttttttgatc ctgatgatga ntgaccatnt 540
ccncaccgta cctttggcgn gaaca 565

<210> 243
<211> 647
<212> DNA
<213> Homo sapiens

<220>
<221> misc_feature
<222> (1)...(647)
<223> n = A,T,C or G

<400> 243
ggtacttgga atgggggctg ttttttggct ggtctgagtg caggactttg ctgctaggat 60
gcttaccaaa tagaaatttg actcagagcc tgtggctggg gaattgtcct caggaagtaa 120
aatggctcgc cagctttcct acctgcttgt ggatgcctca gatagcaatg gtcggacagg 180
acacttcagt gtgggaagca gcatccggtg aggctgtgct ctggcacagg gggatcctga 240
atctccccat ctcttctaag ctgacctgtc cacacattct gagggattaa gcttagagca 300
cctaagaaca gcagcctccc caggagaggc cagggaccaa agtggcagga atcctagaca 360
actctacgct ttttctgcac taaccagctg ggtgactcta aacatgtcac ctccctntgg 420
cctnaacttt ctcatcgacc aaacgaanga gagtagactg ngctttcagc ttaagaccga 480
aaaccgtatc ttaacccttt tctggnacct tgcccggccg gccgttcnaa angggcaaat 540
tccnnacact gggcggccgt actaagggat cccacttngg gcccaaactt ggggtaaaca 600
tggcanaact ggtncctgng gnaaatggta anccgttcca aatcccc 647

<210> 244
<211> 603
<212> DNA
<213> Homo sapiens

<220>
<221> misc_feature
<222> (1)...(603)
<223> n = A,T,C or G

<400> 244
acaacattca gggctttctt tttttcttcg gcaagctctt cttcctcagc agttttcttt 60
tcatttacct cttcctgttc ctcttcactg tcagtttcta gaaatcgaga gtccatgcgg 120
aatctgtcat cggtgccaaa gtgcgactgt aaatccatga gcttctgtcc agctctgccc 180
tcaaactgag gtttaatttt gaacctatta ctgtcatctt cagaatcaga ttcgtcatca 240
tcactgctat caaacagctt ccctgatgtt ttacccatag actctttcac ccattcctct 300
cctggatggc tctgctcctg agtcgatgtc tcctctgttt cacattcact gtcagaaccg 360
aagatgatgt gcgttggctt atcctctgga tgaccatcca aattgccaga gcattatgca 420
ccagcttctt ctgcactctt tgctttttgc ctcgcttcca aggctgncaa acgcttcttn 480
attggcttca acatgcttat ctttagcact cacatttgac gaattactaa tngaaagggg 540
agaaaanagt tttggattcc ccgagngccc ttggatgana cctttgggga ttcttganaa 600
aag 603

<210> 245
<211> 640
<212> DNA
<213> Homo sapiens

<220>
<221> misc_feature
<222> (1)...(640)
<223> n = A,T,C or G

<400> 245
actgggcacc attaatgagg atgcaggaga tcaggtggcc caggccttcg aagatatact 60
ggaacttgtg ctgctgaagg ctggcgctca tggcctcttc aatggcgctg atatctttgt 120
tgagcttgac caccaggggg tcataatcca tactttccac attagccaca atggcatagt 180
tcccctcctt tgcaagaggg ataagatagt ggaaacagtg aaccctcact tccagatgta 240
agacaagcaa gcagcggtca gccatatcct ggaacgattt ggcaagttca ctgagagtct 300
gcatgatctg ctctgacact ggggggagat ccgtgttcgt gtggctgctt gagcaggaga 360
aagcatctgg gatgtagaaa gattggaaga aagctgactt ttgttcgact tgccaaccat 420
tccaagcttt catgcntgtt ngccaaggct ttganggcac ttgaccgtca cgaaggatnc 480
ttgtggaagg antaatttat caccaaggtt ccaatagaac tttagactcc ttgncaaaac 540
tggccttatg aaaacttntt cntcnctctt ttggcctanc tgnttngggt tgngcctntt 600
cattccantt gggnaaaaat tcaaanattg ctggttcttn 640

<210> 246
<211> 608
<212> DNA
<213> Homo sapiens

<220>
<221> misc_feature
<222> (1)...(608)
<223> n = A,T,C or G

<400> 246
cgaggtactg tcattgaagt ggaaccagcg gccttcgtga gttgcgtatg ctgtgtaatg 60

tccagaacca accccggaac catggtgcac caccacagcg gcgaggtcat acaggcagct 120
ctccgggcca ctgttctcag gctctagtaa gtagcatttc atgtctaggc ctctcagtgg 180
aaattctacg tatgtatcaa ctttatttct taaatatgct gtccaatgaa atcttttcaa 240
atgtaagcat agcaccttgg gtagtttttg aatccaaaac ttttttgtgg acttttgttt 300
ctttttgcat ttatggcaca tatataactc tgtctcatca agttcttcta agtcggtaaa 360
actgcgaaga caatctcgta acgaacaaac tggtccattt tcttgattct tagagcgctt 420
acttctgaac tgacttggaa tatctaatga aaggtctang gaatggatca aacttttaga 480
atctgcccca tatgaggcag ttacctcatt ttggagaagc ctccgaatat agccggacaa 540
cagtnaagct ccattatgna ccttggtacc ttgcagacag ngtaaaatnt cctgcaaaat 600
gntgaccg 608

<210> 247
<211> 632
<212> DNA
<213> Homo sapiens

<220>
<221> misc_feature
<222> (1)...(632)
<223> n = A,T,C or G

<400> 247
acagaaagtc agagaacact tacagaactt ggaaaactca gctttcacag ctgacaggca 60
taagaaaaga aaacttttgg aaaactcaac actaaacagc aagttattaa aagtaaatgg 120
aagcaccact gccatttgtg ccacaggcct tcggaatttg gggaacacat gtttcatgaa 180
tgccatcctt cagtcactca gtaacattga gcagttttgc tgttatttca aagaactgcc 240
cgccgtggag ttaaggaatg ggaaaacagc aggaaggcgg acataccaca ccaggagcca 300
aggggataac aatgtgtctt tggtagaaga gtttagaaag acactctgtg ctttatggca 360
aggcagccag actgnattta gcccagagtc cttaatttat gttgtttgga agaatatgcc 420
caactttagg ggctatcaac agcaggacgc catgaatcat gcgctccttt tggaccccta 480
ccttggaact tcaggcggnt caacggggtt tccgctnaac attttgcagg gaaatctact 540
ttgctgcagt accaagtggt gctaaatgga catttntggt gcacggtntt ttcgagggnt 600
ntccaaatnn ggttactgcn tanttgggga aa 632

<210> 248
<211> 624
<212> DNA
<213> Homo sapiens

<220>
<221> misc_feature
<222> (1)...(624)
<223> n = A,T,C or G

<400> 248
actccgaggg gcctggcgag gacatgtaga aagactgcgt tttccttttc aatcgggccc 60
ttttgttggc caacaccaga ctgcgccggc ttgaactgat gatttccgaa atgaacttct 120
tgcagtccac acacacctcc atggtgctcc agtcctccat caactctttg ggaaactgga 180
gttcttcatc tgatttgtcc atagacttag attttgagga gaacctggca atgctccgaa 240
gtggccgatg atgggcagtg gagggttttt ctgacctcat actactttcc cctctttgca 300
gagcagaagg tcccaatgaa aagataggaa gagtggagta tggtttggag ggcagcccgc 360
atctttttgc aacactgtga gcacaccggc ctnttacaga actgacaggt ataagaccaa 420
gtgaagaagg aaaaccttct ggttcggcaa ccaaagcaga gctttncttt tttcaagncg 480

tgtnaagnct ttatctggtg atattttcca ntntgcntta ccaggaccgg cgaatatgnt 540
ncttnttccc agtagacnag nattcnctgg gaccaaattc taaanaccgg acttnctgaa 600
gnggaggact gcttcgttta ggct 624

<210> 249
<211> 636
<212> DNA
<213> Homo sapiens

<220>
<221> misc_feature
<222> (1)...(636)
<223> n = A,T,C or G

<400> 249
acagtaaaaa gtaaacttcc ctccatccca ggcctgccag catccctgat gccgactttc 60
tgggtgtggc ctagggcccc tcagtgtaat gtaggggttg tgagcacaga ctttggtgcc 120
agtttgctag gttcgaatcc tgactccctc tttgtagctc tgtgcttcaa ttgaaatact 180
gtgcctcagt ttctccttta taaaggcagg gatcatgaga gtgcctgtcc cttgtgagca 240
ctatgaaagt gttagctgtt ctttaccaga ataaatgcat ttctatatct tcccatatgc 300
attttgntaa tttttaaagt atttcaaaca caaagtttga aacagaaaat tgtgtaacat 360
taactatgaa cttaccaccc agaatttaca aatgctgaca ttttgcaata tttatttcng 420
atctattttt aangggggga accctgcagt tactgnttaa tcctttccac ccacctttta 480
attttacacc angagcatag tggtcatacc tangctaatt ttttcagtac ctgatatatt 540
tggagaactc cttcctaggc ataaactttg nccctttttt taanagtggt taacctttgg 600
gacnaaaggg cttgaacaat tggcccatcc ctttgg 636

<210> 250
<211> 669
<212> DNA
<213> Homo sapiens

<220>
<221> misc_feature
<222> (1)...(669)
<223> n = A,T,C or G

<400> 250
ggtacataat ccggcagctc catggcatct cgcttctggt gctgtgcctc agccccaatc 60
agaaggttga aatgagtggc caaatgtctt cgcagcaaag tcttattggg tgggatgttc 120
aataactgag ccattgtttc tacgttaaaa cgaggctcta gaaccatgag cccaccatgg 180
acaccactgc ctctgagatt gggcgcatat tctgccaagt ccacggagcg cagccactcc 240
atcactcgat ggttagtcca cttctgaact tctgatgggg cgatggtatt ctcatcagat 300
ggccgcctcc gtagacagtt tggttcaaaa gttattgatc ctcaggacct ggatggccct 360
tttgatactg agatggtgta ncacacttac cacctttcag agacagtaag tcatcaacag 420
tcatgtaatg taacattcga ccatnaaccc ggccttnatt aaactgggtc ttatatttga 480
gggaaggncc atggcattcc aaccctntaa nggacccnnn ttggaaatcc actttcccat 540
gaatgggttc ntttttnaaa atcccanggc nttngaaagg ctaacttggg nggttcnttt 600
tcatgaaang aaagcctgga ttccaaggtc ccttttttaa aactttgtgg naaaccctgc 660
aaaaacntn 669

<210> 251
<211> 670

<212> DNA
<213> Homo sapiens

<220>
<221> misc_feature
<222> (1)...(670)
<223> n = A,T,C or G

<400> 251
actattcaag aggtgaagag aaatgtgtat gaccttacaa gtatccccgt tcgccaccaa 60
ttatgggagg gctggccaac ttctgctaca gacgactcaa tgtgtcttgc tgaatcaggg 120
ctctcttatc cctgccatcg acttacagtg ggaagaagat cttcacctgc acagacccgg 180
gaacagtcgg aagaacaaat caccgatgtt catatggtta gtgatagcga tggagatgac 240
tttgaagatg ctacagaatt tggggtggat gatggagaag tatttggcat ggcgtcatct 300
gccttgagaa aatctccaat gatgccagaa aacgcagaaa atgaaggaga tgccttatta 360
caatttacag cagagttttc ttcaagatat ggtgattgcc atcctgnatt ttttattggc 420
tcattagaag ctgcttttca agangccttc tatgtgaaag ccccgagata gaaagcttct 480
tgctatctan ctnccccntg atgnaaagtg tggtnaccca cgggttctgn gttaccaaat 540
gctttgggcc tgnaanccat tgggttcctt attctgggtc aaaaattttt taacccgggc 600
nttgggaact tgccaanggn ntccaccnga gccangaatt ttcactttgg gccaaaaaac 660
cttttgnggg 670

<210> 252
<211> 498
<212> DNA
<213> Homo sapiens

<220>
<221> misc_feature
<222> (1)...(498)
<223> n = A,T,C or G

<400> 252
acacagcaca ttctcttaag agaaaacagg aatgaacatt ctcagaaaca ttcacattgc 60
tcatcaaatg tagctttacc caaagtatat aggaaatggc aaaaacctaa cctagctgga 120
cattttatac aagtaagtca aagttcaaag gaatcatcct atctttattc tcagaaatcc 180
aatgttgaat atcacagttc ttctttaatg gaagcagaag attcagagtc cttgtctccc 240
aaaatgcctc agccagggtc agcacagaga gtggaatata aaaagcttaa ttgtgttaat 300
acatggaaga caacagttct cagtcaacct agccacaatt ttctgtcttg gccatctgta 360
agaaatgact accgtttgaa attcaacttt cacattcaaa aaaaagaaaa tcaattcagc 420
tttnagacac aaagcaaaac caaaacaaaa aaacnaatgg catagtctac atatttnacc 480
ccttgacaat tgggggaa 498

<210> 253
<211> 433
<212> DNA
<213> Homo sapiens

<400> 253
acgtttcagt tcaagtgcaa aaaataacta tttgctgaat tctatttctt tcagttattt 60
tatttttaag ctgtgtttta ttgtgaagcg agacatccaa gtgtagaatt tcttatccca 120
aatgcagtat tgctccttgg ttacgcttcc tggggagaca ggggttgctg tgcttgagtt 180
caaagtcaag tccatcatac ggttagtaat ttcacctgtc tggggctgca gagtgggttc 240

actgttcatg tttggagctg ttggcaaagt aacggtgtct gagacattga gccctgtttc 300
caaaaggttt cttttctcac gcatttttgg tgatatggtg aggaaagagg taaaggaaga 360
atttgttggc aggataagtt aactggtgac ttgcattggt ggggtgaagt tggttgggcc 420
aatctttggt acc 433

<210> 254
<211> 652
<212> DNA
<213> Homo sapiens

<220>
<221> misc_feature
<222> (1)...(652)
<223> n = A,T,C or G

<400> 254
ggtacaaacc caggcctggg cctaggaaag ggcagaagaa aggcaaaggg tcccttggag 60
caggaaccca tccctctctg cttataccca gcacccctca tcccaggttc ctttcttcaa 120
cctccgcctg cctctgggaa cacagagcac caagaactga caaaccggga ccctccaggg 180
ccacagcgtg ggcagagtcc caggcttctg tctccccgca gtgggagatc tggggagctc 240
agtgaacctc ctcaccctcc tgccagtatg aagttgggaa gcgccttctc tgtcccccag 300
aacagaacaa actcttgttc tctgtggttg gggaaaaggt gtggggggct tggacctagg 360
aagaagctga gctgaattcc tccagggccc aggtgaaacc cccaagggga gtttctgaga 420
cttctagact tggccattct ccactttttc cttccaatga ctccggtgaa gcagttaaaa 480
gtctnggctt agggcaactg gtaggacagt ngggaatttg ncccaagaca tttgngggtt 540
tcaaatnaag gtttcccaac accngaatca ttatatggan cctgccnggc nggccgttca 600
aagggcnaat tcngnccctt ggngggcgta ctaagggaac ccactttggg cc 652

<210> 255
<211> 605
<212> DNA
<213> Homo sapiens

<220>
<221> misc_feature
<222> (1)...(605)
<223> n = A,T,C or G

<400> 255
ggtacgacag ttgtgtgggt ttattgggaa cctccaacat ctccacaaca atgtagtatt 60
gtggaaggcg ggtaagttta atgaacagtt tattcttaga aaggtttcca ataggatgag 120
ttgagtaatt ggaaagctgc aatgtttcac tgcttatcgt aggcagatgt tttatagact 180
gcttgcaacg ctgttgtcca agccaaaact taagttgctg aatccagggt atgattcgtt 240
tcatatcatc attcacagac ttctccatgt catccagagt ggcctggtca agtccataaa 300
gcatcaattg aaacattcca gaatgtaaat ctacaaaaat gtgcaggcac tctgaattac 360
cacagggctc caagatggga acaacaagag ctgggagtgc agtctctatg gaagagtttc 420
attggcattg aagcctctaa gaatggcctt cagttcttgg agcttctgat gagctcttgc 480
atggacactg gnaatcangg agttttctat tgataagtgg gccgatcttc atggctcttt 540
ctactaattt ggaatcanaa nttgcaaagg aggatcgtga aaaatttnna aggtttggaa 600
acatn 605

<210> 256
<211> 654

<212> DNA
<213> Homo sapiens

<220>
<221> misc_feature
<222> (1)...(654)
<223> n = A,T,C or G

<400> 256
acagttcaca agcttcaggc aaggggcagc ctgagactat ccgagtgatg ttgaggcaat 60
ccaggcacag caagtcattc agccacttct ccactgcatc cccaggggcc gtatcggatt 120
gactcctgga gggaaacctc atgcagtgtc cgcgctgatg ccaatctggc tgtcgtcgtg 180
gtcttattct cagcagtggt gctgacctgg ctctgggcgc tctgttgacg gagctgctga 240
attagcttga gggacagtga ccggccagtg ccctcatagc cattgatggt ggatgccatg 300
aaaacaaggt aggggccaag taggctcttc accaagggga gggggatggc ggcagcttca 360
tcaatcacaa ctagttcagc ctggcccagc ttcacagcat ctgcaggatg tatatactga 420
atagtctggc tgngtctcga aatacattca ctctgatcac tgntttggta aattcangaa 480
ttanagactg gataatctca taatccaaag gttcctgaaa nttgcanaac attnaaatcc 540
nttnaatncc aattcaaccc aattttgang ttttaanggc tttgggangg aaccaanaan 600
ttggggtacc ttggccggaa cccccttaag gggnaattca gncacntggg gggn 654

<210> 257
<211> 594
<212> DNA
<213> Homo sapiens

<220>
<221> misc_feature
<222> (1)...(594)
<223> n = A,T,C or G

<400> 257
actgctcttt tattacggta atacttgcta gtgggatttc tctcttcacc aaggctgcct 60
ttactgtgtg aaggacctgt cagtctggct gcagccaagt tggatggagt cctcattcga 120
agacttgact tagccatttc atgatgttca atttcagcct ttttcatata aaatattttt 180
ttaattgaat ttgcatcctt gaatacttga gagccaggct cattataagt tttggcattt 240
tttgcgagga gatctatatc tttggccatt gcatgaatac ttttgtagct tccattctgt 300
atcctctggg caatggtctt gagatctata ggctccttaa ttattgcata ataatctgga 360
tattgcactt tagaaggcaa gtttctgaaa aaagtcgcta atgagacgtn ctgatggatt 420
gnagctacca ctatggcttc aagaaactgc ttcaggaact ncttcaagta agctggagaa 480
aaatcttnag cactgggncc tggatgggct tggccatctt catcaataac ttcgncaatt 540
ggttctcntt ttgaaccaac ctcattnttg gtccaaggna ccttggncgg gaac 594

<210> 258
<211> 648
<212> DNA
<213> Homo sapiens

<220>
<221> misc_feature
<222> (1)...(648)
<223> n = A,T,C or G

<400> 258
cgaggtacct tgctgtttat tccttagtct agcagcatcc ttagtttgta gtatatctta 60
cttagttgca actaaaaaaa attgctagcc taggctttaa ctgggagttt ctattatcta 120
gaaggttact gtgaaccttt cagaaaagtg gaaagcaacc aaaagagctg tctcaaagac 180
tgtgtccccc cagagtttgt ccagctctta ctgtagacac tctgaacagg cacggttatc 240
tcatgtccaa agctcataac agcacattag aagaaagtgg ggagcctgtt agaagcaggc 300
atattgatag tgtgggagaa gacatagcaa attacttagc agatatttta aaaattttaa 360
aatccaacag cagtctgagg caaatgattc tgnatacctc agggctgana gaatcacttt 420
atacatattt ggtatagccc tttcatttta tgaaagtgtt tacataccnn agactngatc 480
ctataataat accttatgaa tatactttac ttttcatcat ggaaaatgtg aatatactng 540
cntgatggtt aagaagaagg ccggagggtt cctaccntnc ntgaancctn ccttaaaaat 600
aatccnngtt taaanngtgg ncttggnaaa ttccttantt tcccaaaa 648

<210> 259
<211> 224
<212> DNA
<213> Homo sapiens

<400> 259
ggtacttcaa aaagaacatc aggattaatg ttcctcagag tatgttctgc tgcttgaact 60
ttacttaatc ctgcttgatg aggttggaag aaaagtctat tcatattggc tagttccacc 120
ttgtcataat caaagagtag caacttacca atgccacatc ttgtcagcat ttcagcagtc 180
acactaccta ctccaccaac acctactatt gctacggcaa aggt 224

<210> 260
<211> 584
<212> DNA
<213> Homo sapiens

<220>
<221> misc_feature
<222> (1)...(584)
<223> n = A,T,C or G

<400> 260
ggtacttcaa actctcttaa cggtgatgct ctgacattca ctactacatt tactctgcaa 60
gatgtatcca atgactttga aataaatatt gaagtttaca gcttggtgca aaagaaagat 120
ccctcaggcc ttgataagaa gaaaaaaaca tccaagtcca aggctattac tccaaagcga 180
ctcctcacat ctataaccac aaaaagcaac attcattctt cagtcatggc cagtccagga 240
ggtcttagtg ctgtgcgaac cagcaacttc gcccttgttg gatcttacac attatcattg 300
tcttcagtag gaaatactaa gtttgttctg gacaaggtcc cctttttatc ttctttggaa 360
ggtcatattt atttaaaaat aaaatgtcaa gtgaattcca gtgttgaaga aagaggtttt 420
ctaaccatat tgaagaatgt tagtgggttt tggggccctg ggcatcgaag aatggtgtgg 480
ttcttttctg ggaaactgna taatcttaat tggacttaat ccagnatgat gaagaaaccg 540
caggaattcc cattnggaan gggataaatc tngcttaatt ggan 584

<210> 261
<211> 526
<212> DNA
<213> Homo sapiens

<220>
<221> misc_feature

<222> (1)...(526)
<223> n = A,T,C or G

<400> 261
ggtacttgat gttctgcagc ttctgaaagg cttcctgata ctgctcaggg gtgtcaaggc 60
tgaagatgct cttccacact gcagtcaccc tctccacgaa agacccttcg gtgcccgtgt 120
tccaagtgtg gtaagaggag gagcttttgc cctctgaaag ctgcttttcc tccagatgcc 180
tggacagtag ctccagaagg caaaacacca atctctgacc ctgtagactt tcatgcagct 240
gcagggcttc ctgggctccc acccagttgt tggccagaag cagctcttgg gcacatctga 300
gagccaggga agcagacaac tcatcctctc ctacgatggc agccaactct gcagccgttc 360
taagtgatgc cgcatccccc tttttggcca aaactttggc tgcatcataa gcacaagtgg 420
cccctaaata gcatttggca gctacagcat agtggccatc tctttctagg acnggtcccc 480
agctgangna cctgcccggc gggcgcttct aaanggcgaa atcttg 526

<210> 262
<211> 703
<212> DNA
<213> Homo sapiens

<220>
<221> misc_feature
<222> (1)...(703)
<223> n = A,T,C or G

<400> 262
cgaggtacag aggctgcaag aaggtggcat agagggctga aggtctgggt ggcagggcca 60
ctcctttaat aaaccaatgt catgctcaca ctcctattgc ctaccttggc atgctggatc 120
agctcacaga tgcaggatca agtcttgaaa gccaatcaga aaatccttca taggcttaca 180
aaggaccacc catggaacat tgtttcccgt aagactgaaa agacaaacta caccaaccac 240
caccactctt ctttttcctt tttggcccca tcaaaggaca tggagaaggt agacaagttt 300
tcttatccct acttttctaa ctcgaggatt ctccaaattt acatcagcag ctctaaggat 360
attcctcaca ggtcacaaac tgaaccaaaa atgaaaatcc tttctataaa actacacatt 420
ctttattcat acntatgact aaaggctact gaatggnacc tgccccggcc ggccgttcga 480
aagggccaan ttcaacacac ttggccggnc cgtactanat ggaatccnaa ctttgggacc 540
caagctttgg cggtaatcca tgggccataa gcttggttnc ccggggggga aaattggtat 600
tnccgnttac caatttcccc accaaccntt cccaancccg gaaaccntta aaggggtaaa 660
anccttgggg gggccccaaa nggggtgggc cttaacttcc ann 703

<210> 263
<211> 475
<212> DNA
<213> Homo sapiens

<220>
<221> misc_feature
<222> (1)...(475)
<223> n = A,T,C or G

<400> 263
ggtacttgtt agcttacccc aaaataatac ctggtatacc ggacccaata tctgctgatt 60
gatctaacct aaatgaatac aaaccatttc agaaaaagat atacaataga ccacatatcc 120
aggtcatgaa aattaaagct ttcaggtcac ctagcttagt gactattgct tttctgaccc 180
tagactcttg aaagcctatt taaactggcc tctttctcca caccaaaact gataaaaagg 240

agactgatta tgagccagga tttacacaga gattctctat ataaggcata aaggtgaggg 300
gtgagagaga gagagagaga gagagagaga gagagagaga gagacgtgag ggagggagag 360
aaaagagaac agacngaaga tnagagaaag agaaaggtat acagtctggn gcctcaattc 420
cagtatgntg atttggcttc aacacccgng tacctggccc ggcnggccgn tngaa 475

<210> 264
<211> 601
<212> DNA
<213> Homo sapiens

<220>
<221> misc_feature
<222> (1)...(601)
<223> n = A,T,C or G

<400> 264
ggtactacaa aaaccaagtg ctcgattacc acttaacatg ttcagcttga aatgactgct 60
acctttgcct tcaattcctt cccacacacc caggtataca aatatctttt ataccaagag 120
tccttgtgaa agtaaataga gggaactccc agggataagg gagggcaaaa aacaggaagc 180
acttgaagcc aaaatctgga gcaactttta agaaggaaga gacgtccgtc ctattttcat 240
atctctgcat ggatctccca tggagaactt gagttaaatg taatgattac acgtggcaga 300
aagacaactc tctagcacag tgtttctttc acataggctg ctacattcat tccataagct 360
caacaatttt aataaaaaat atttctgcta aatactttat attcatcatc ataaaaaatg 420
cacagccatt tgaaaaaaan ggcaattacc ctaaatgaat attgcccaaa gcacagatca 480
actttatata nggattcttt ccttggtctg aaaaatcgca ancggaactg gcagacttta 540
tttaccaacc atggattttg nccagcatgg agttaaattt antgctgtct ggagcaggaa 600
a 601

<210> 265
<211> 643
<212> DNA
<213> Homo sapiens

<220>
<221> misc_feature
<222> (1)...(643)
<223> n = A,T,C or G

<400> 265
actatgaaag gcaggtttcc ttgtctggag gaaaaggtcc ttgagacacc acaggaaatt 60
cacaccgtaa gcagcgaggc tgtcagcttg ttggaagagg tcatcactcc ccggaaggac 120
ctgcctcctt tactcctcaa attgaatgag aggcctgccg aacgcctgga ttacctgggt 180
gtttcctatg gcttgacccc caggctcctc aagttctgga aacgagctgg atttgttcct 240
gtttatctga gacagacccc gaatgacctg accggagagc actcgtgcat catgctgaag 300
acgctcactg atgaggatga ggctgaccag ggaggctggc ttgcagcctt ctggaaagat 360
ttccgacggc ggtcctacct tgctctctac cagttcaata cctnggccgc gaccacctta 420
gggccaaatt cacacactgg cnggcgtact aatggatcca cttngttccc aacttggcgt 480
aatcatggca taactggttc gggngaaatg gtatccgtta caattcccac acatacaanc 540
cggaanntta agtgtaannc tgggtgctaa tgatgactac ttncttaatg ngttggctac 600
tgccgtttca tcgggaactt ntgccattgn tataatgcnc ccc 643

<210> 266
<211> 582

<212> DNA
<213> Homo sapiens

<220>
<221> misc_feature
<222> (1)...(582)
<223> n = A,T,C or G

<400> 266
actgtttacc agatctttgc agatgaggtg cttggttcag gccagtttgg catcgtttat 60
ggaggaaaac atagaaagac tgggagggat gtggctatta aagtaattga taagatgaga 120
ttccccacaa aacaagaaag tcaactccgt aatgaagtgg ctattttaca gaatttgcac 180
catcctggga ttgtaaacct ggaatgtatg tttgaaaccc cagaacgagt ctttgtagta 240
atggaaaagc tgcatggaga tatgttggaa atgattctat ccagtgagaa aagtcggctt 300
ccagaacgaa ttactaaatt catggtcaca cagatacttg ttgctttgag gaatctgcat 360
tttaagaata ttgtgcactg tgatttaaag ccagaaaatg tgctgctttg catcaacaga 420
accatttcct caggtgaagc tgtgtgactt ttggattgca cgcatcattg gtgaaaagta 480
ttcaggagac tgtggaggac tccactacta nccctgaagt cttcgagcaa ngtacaccgt 540
cctanaatgt ggcatgggag tatattatgg anctatgcca tt 582

<210> 267
<211> 565
<212> DNA
<213> Homo sapiens

<220>
<221> misc_feature
<222> (1)...(565)
<223> n = A,T,C or G

<400> 267
actttgggag gctgaggcgg gcagatcaca aggtcaggag ttcgagtccc agcctggcca 60
atatggtgaa accctgtctc tactaaaaat gcaaaaatta gccaggcatg gtggtgcatg 120
cctggagtcc cacctacttg gggctgaagc agaatggctt gacccaggag gtggaggttg 180
cagtgagcca agatcatgcc atggcactcc aacctgggtg acagagcaag actccatctt 240
aaaaaaaagt atactaatgt ccctcaagtt cttccatatg aggtaaaggg atccaagatt 300
aaggttgaaa ttcttaaact gttcaacaat tttgtggtgt catcaaaaaa ggaatatttc 360
atatatatta atttaacctc aatgatcaac attgttaaaa gtcagtatgg agaaagatca 420
ttctgacctc ttcagaaacc acctggtata tgaacattct gatcccanat tattttggga 480
nctaaggacn atggtgaaaa gaatcncnan attaaaagtt ctattttcna tggaccttng 540
gcccgngaac acncttaagg gccna 565

<210> 268
<211> 661
<212> DNA
<213> Homo sapiens

<220>
<221> misc_feature
<222> (1)...(661)
<223> n = A,T,C or G

<400> 268

cgaggtacta caaaaaccaa gtgctcgatt accacttaac atgttcagct tgaaatgact 60
gctacctttg ccttcaattc cttcccacac acccaggtat acaaatatct tttataccaa 120
gagtccttgt gaaagtaaat agagggaact cccagggata agggagggca aaaaacagga 180
agcacttgaa gccaaaatct ggagcaactt ttaagaagga agagacgtcc gtcctatttt 240
catatctctg catggatctc ccatggagaa cttgagttaa atgtaatgat tacaccgtgg 300
cagaaagaca actctctagc acagtgtttc tttcacatag gctgctacat tcattccata 360
agctcaacaa ttttaataaa aaatatttct gctaaatact ttatatcatc atcataaaaa 420
atgcacagcc ttttgaaaaa angggcanta cccctaaatg aatattgcca agcacagatc 480
aacttatata ggattctttc cttggttctg aaaaatcgca accgaactgg cagactttaa 540
ttaacaacat tgatttggcc agcctggagt tnaatttant gcatgtcctg gaggcnggan 600
aaatgatcca gaagtaagca ccaccgnctg cngggnccan gttcaagaac ttaagccngg 660
g 661

<210> 269
<211> 643
<212> DNA
<213> Homo sapiens

<220>
<221> misc_feature
<222> (1)...(643)
<223> n = A,T,C or G

<400> 269
actgatggga aggccaatat ttgatgcaat caccacagtg agggcagatg ccagttcaat 60
actgaagcca ctagagggtg tgatcggtgt cagatccttc cccatggtct ggataactct 120
tcttccccaa acccacagac caacacagat accaacacca ccatagagta gaagccatat 180
tggtgttgcc acttttgaag aaacatctcc tgtgccataa accaaatata aagcaaccag 240
aggcccaatg gcattgctta cgtcattgcc accatgggcg aatgacccaa agcaggctgt 300
aaggatctgc aggaactgga aganggagag agacttcagg gcttatcctg ggcataccat 360
tctttctaga agaaccctta ctttcttttc tgncacctaa acccatcttt gnctttgcac 420
ttatggctat cttaaaangc tnaatgaaag ncagacacng cattgcagta actggggnac 480
tgncatttna antcccttct tggagctgna ntaggcctgt cacttctcat ttcttngccn 540
ttggtaactt ttttgnncgg atgaatcnga gnatgcncat atgcntggat tganntactn 600
tatggcctaa gggtgnncgn ggtcctcant tcncttggan aga 643

<210> 270
<211> 650
<212> DNA
<213> Homo sapiens

<220>
<221> misc_feature
<222> (1)...(650)
<223> n = A,T,C or G

<400> 270
gggccacatc tgccagagcc tggagtctgc gaaggccggg acccggttcc ccggcccaca 60
gtgggggtgt gcaaacccga gagaactggg ttgcaaattc gtgaagaatc agcatcatgt 120
ttggcagctg agtattggag ccaggagcct gccatgaggt tttgagaaca gagtgctgtt 180
ttagagctgg cagcagcatc tcagcccaag agaaggttat attcccagag gatgtcagtc 240
ccaaggacca gtagctgcca tcagtttgga ttctgaaaac taactggcat caacactggg 300
tgtagaaaca tgcttgcctt atgtatcaga ggacatgctc agcaagatcc aagagatata 360

tttggcaact ttttctagaa aaggcacatt gggtatcatt cattacattc ttgagttttt 420
ttgggttttt tttttttttt tgaacagtct tgctgnattg ccangctgga atgtggtggc 480
caatcacanc ttattgcatc ctaatcaccc aggcctaagc aatcctcccc ttganctggg 540
actanggtta cagncacctg gtaaaatttt ttttgtgaac ggntcttatg tgccagctgg 600
nttaggttct nggntnaang gcctctgcta nnttcaaggc nagccatttg 650

<210> 271
<211> 620
<212> DNA
<213> Homo sapiens

<220>
<221> misc_feature
<222> (1)...(620)
<223> n = A,T,C or G

<400> 271
ggtacacagg tcccaagctc tttaaggagc ccagtagtaa atcaaacaag ccgattattc 60
acaatgccat atcccattgc tgcctggctg gaaaagtgaa cgaaccccac aagaattcca 120
tattggagga gctggagaag tgtgatgcca atcactacat catactgttt cgtgatgctg 180
gctgccagtt cagggcgctt tactgctact atcctgatac tgaggaaatc tacaaactca 240
ctggcacggg gccaaagaac atcaccaaga aaatgatcga caaactgtat aaatacagct 300
cagaccgaaa acagtttaac ttgatcccag ccaaaaccat gtctgtcagt gtggacgcac 360
tcacaatcca caaccacctg tggnanccaa cggnctgcat gccaagaagg ccaaactcgt 420
aatgacccgg tgcactggcg tccaagggtg accagactcg taaatgatgc cttgtggtgg 480
atcaaaggtg cacgggggcc tanttantgg ttanctattt ggtcctgccg gcnggcgttn 540
aaagggaatt caccactggn ggcgtctaag gaccacttgn ccacttgnga anatggntan 600
gttctnggga aanttccccn 620

<210> 272
<211> 670
<212> DNA
<213> Homo sapiens

<220>
<221> misc_feature
<222> (1)...(670)
<223> n = A,T,C or G

<400> 272
cgaggtactt tatattacta aatgtctgaa gacaaaagag caattggaaa tctctgtttc 60
ttgtttcgtc atacatagga aggcgacgtg atgcaaattt taacacaaga ttttattaaa 120
gacgggcaaa ttggtgaggc atacctgaat ttctggagat atacaaatgc gtgaggctgg 180
catcatatgc aaatgtggct ttacaaattg gttttatttt ctagctgtat ttaaagaggt 240
gttcaaaatt ccctactaat caagaagcac ccctgaaaaa actatgagat aagatagtgt 300
tattaatggt ttgcatctaa agaccaggaa acacattagc caatacagtc cacaatcggt 360
gaaatgctgc cgtgcnaaat gcacgtgcat atgcnttttt actatattcc ctnagagacc 420
gtaaaacaac naccaccacc aaaaaaaaac ngtgctcnta aatngnggac naacctttcc 480
aaaccaccgn cttactctta ctggggttta agggaattca ggaagcttcn tttanccana 540
aagctnaacc ccttcagttc ataanctttt nccttggaat aaggcctgnt ntggctacct 600
aaaaccaagt ctgggggaaa aggactcatt ccattattaa cnnttacncc taaggganga 660
ataagggnnt 670

<210> 273
<211> 688
<212> DNA
<213> Homo sapiens

<220>
<221> misc_feature
<222> (1)...(688)
<223> n = A,T,C or G

<400> 273
acacaggtaa ccttatgcag cacattgtgc taaaagtatg gaacagttaa cactttcagc 60
cattactgaa aataaacatg tagaaactaa gcaacaagtt aaaatacagt aatgcacaac 120
ttaacaattt taagttttcc acatggagca ataaagcagg taactgaata atttaaggag 180
atgcaaatgg ccctcttcat tcttaattct cggcaattta ctcaggaaaa taaatttctg 240
gtcgcagccc gaacagttcc agtccgatct caccttgatg gaaagtcttc attatctgtg 300
cttgcccgag gacttatgaa tgnttcttct ctttcttttc ttctgaactg gccccgttct 360
ctttcttttc tatcctttct ttatcatgcc tggactcctt ttggcacccg aaggagaatt 420
taaccatctt ctcagaatta aatggaatca ctggcttttt cnttggcctg aagaatttga 480
cttanttttt tncttggctt tctcaattng attaagggga ttcnccaagg acttttactt 540
ttaaggtttt gnaaacccca atnggtncat tcttcccctt taccgctctt gggttaaanc 600
ccggggggac tttaccgggc cttggttgaa ngaacccntt ttcggtcttt tcngggcctt 660
ttaacttttt ctcnctttnn ctgggagn 688

<210> 274
<211> 674
<212> DNA
<213> Homo sapiens

<220>
<221> misc_feature
<222> (1)...(674)
<223> n = A,T,C or G

<400> 274
atttaaacct ggtttggata tgcgcctgta tgaggaagat gatttggacc ggttagagca 60
gatggaagat tcagaaggga cagtgagaca gataggtgca ttctctgaag gcatcaacaa 120
tctgacgcac atgttaaaag aagatgacat gtttaaagat tttgctgccc gttcccccag 180
tgccagcatt acagatgaag actcaaacgt ttgaccgtag cacctggatg aacattagga 240
gtgcttagtc ttttttctac ttgcttttcc aaacactcac agtatataca acaggcagcg 300
gattgnctat tgnttgttgn tccaacttct gctgccagaa gtttaaacag aaagcaggaa 360
taatgtgccc attctgaagt tgccacaaaa aataagaccc tggtgaatga aaatataatt 420
ggttttcttc taattaatgg aaaaatctgg gatatattat atttaaaggt ggtgcattta 480
aagaatgagt attttacccc gaagtggttc ccttcatatt ccccggattg aaggatttga 540
nggaccgtac cnggatgggn atgaatttgg tacttcatgg tcacttgaac ccnctaagtn 600
ggccnttttt ggattcanaa tcatatgggg aacttcttta agccttcagg ggccncttaa 660
tgccnnncca cctn 674

<210> 275
<211> 638
<212> DNA
<213> Homo sapiens

<220>
<221> misc_feature
<222> (1)...(638)
<223> n = A,T,C or G

<400> 275
ggtactggca tggcaccaac atttgctcag cttctggtga gggcctcagg aagcttacag 60
taaaggcgga aggtgaaggg ggagcaggca tatcacatgg cgagaaagag gggagaggtc 120
tcagactctt ttaaacaacc atatctatgt gaattgagtg agaactcact catcaccaag 180
gagatggtgc tgagccattc atgaaggatc ccctctcatg atccaaatac ttcccaccag 240
gctccacttc caacactggg aattacattt caacatgaga tttggagggg acgagcatcc 300
aaaccatatc agatggtgag acaggagaac tttgtgtgtc cagctgcact ggtctgaaga 360
tataactaag tccctggact ttttctcctt aattggagaa ttcctaatgt tcatgatcag 420
cctgantgac cagtggctga ctggcctgaa aggggagata aaacngacca cagctttctt 480
catagaccaa tttaaccttt attcatctgn gcagcagaag ggactggncc anatanccat 540
caggtaggng cttgaatatg ggtactttcc nanatacttg ccggccggcc ntttaaggca 600
attccaccaa tggggccgtc tannggatcc actcggnc 638

<210> 276
<211> 638
<212> DNA
<213> Homo sapiens

<220>
<221> misc_feature
<222> (1)...(638)
<223> n = A,T,C or G

<400> 276
ggtacgtcag atctacagcg aacacaacta ctgccgcctt atcctctaaa tggggagcat 60
acccaggccg gaactgccat gtccagagct aggagagagg acctgccttc tctgagaaag 120
gaggaaagct gcctactaca gagggctaca gttggactca cagatgggct aggagatgcc 180
tcccaactcc ccgttgctcc cactggggac cagccatgcc aggccttgcc cctactgtcc 240
tcccaaacct cagtagctga gagattagtg gagcagcctc agttgcatcc ggatgttaga 300
actgaatgtg agtctggcac cacttcctgg gaaaagtgat gatgaggagc aaggacccac 360
cgttcctgca gacaatggtc ccattcccgc tctagtggga gatgatnntt agagaaagga 420
ctggcccagc tcttgcagtc atccactatg aaggatcctg taatgtgacc ccagttccac 480
actgatctca ccgctgatgc tgcagaacag anatttgatg acgaataggc ttggngntta 540
tgcctctatg aggaaagtat ctngacnaga aacttgaaac cangnttntg tttacagtct 600
ttgatggtcc atcatcatga nnngatgaac gccaaccg 638

<210> 277
<211> 734
<212> DNA
<213> Homo sapiens

<220>
<221> misc_feature
<222> (1)...(734)
<223> n = A,T,C or G

<400> 277
   ggtacagaga tagatgaatg gaaatgggta agggaggtgt tcattcacat ccatctaact 60
   gcaaaataca aaagtaagaa gtcattgaca tgaagcaacg atgaccaaga cgttctcaga 120
   tctaaaggtg aatgatctca gtcagcctgg aaatgcacaa ggtggaaaaa taacataaaa 180
   aagccataag accttgaaga acatcaatgt caaagataaa ttctaaagtc ccagagaaaa 240
   aagaatggga atcaaattga cctcagacta tacgtgagaa acacggagag ccagaaaact 300
   gtgatgttcc atcctcagag tttgaaggaa atatttgaag gctgaatttt acatccagct 360
   taactatcaa ggcatgccaa gtcatgttat tcttaggcct tcaaggnctt ngcccttttt 420
   ctcngaaaag cccgaatttn aaatgctctt aaagaccgtt cttcaacccn gaagagaaaa 480
   gaaanccngg gangggtgct cttgagatat ttcagtcncc cacaggttnc ccaaatnggg 540
   cctaaggaaa ttccgaagag gtcncgaaat nttnacccat taccttcccc caatngggga 600
   accccccgac agggntttan ccatnggggt taaaggtttt ttgacccggg ggggccttgg 660
   caaggtancc tggccccggg cgggcccntt cnaaangggc caaanttccn gncccccttg 720
   ggggggccgg tanc 734

<210> 278
<211> 586
<212> DNA
<213> Homo sapiens

<220>
<221> misc_feature
<222> (1)...(586)
<223> n = A,T,C or G

<400> 278
acatggtgaa tggaccacca cattttacag aaagcacagt gtttccaagg gaatctggga 60
agaattgcaa agtctgtatc tttagtaagg atgggacctt gtttgcctgg ggcaatggag 120
aaaaagtaaa tattatcagt gtcactaaca agggactact gcactccttc gacctcctga 180
aggcagtttg ccttgaattc tcacccaaaa atactgtcct ggcaacgtgg cagccttaca 240
ctacttctaa agatggcaca gctgggatac ccaacctaca actttatgat gtgaaaactg 300
ggacatgttt gaaatctttc atccagaaaa aaatgcaaaa ttggtgtcca tcctggtcag 360
aagatgaaac tctttgtgcc cgcaatgtta acaatgaagt tcacttcttt gaaaaccacc 420
aattttaaca caattgccaa ataaantgca tttgccaaaa attaatgact ttggattatc 480
accctggacc ccaaccatac caaggtggct ggctatgttn ccaggaagtn aangngcccc 540
cttatttggt agaatatatc agtancttgg gcgggaacac ccttan 586

<210> 279
<211> 664
<212> DNA
<213> Homo sapiens

<220>
<221> misc_feature
<222> (1)...(664)
<223> n = A,T,C or G

<400> 279
accaccgagg ctagcacagt caagcctcca gctaagctgg atccctgaag cctgctatca 60
tgcagacagg ctatgcggct gcctcggacc atgctaggcc acttgctggg gtgtcaacct 120
accaccaaag ggtcttttta gcaaacctca tggggaacag gaacattcct gttcatccct 180
ggccacaggc tgcagaccca gcactggccc ttgcgtgagt cagagcctgg ggctggccct 240
agccccttct actgacttcc tcatttaagc caattatata agctcacatt gatcagggag 300
ggagggaaag agctaaagag ggtcacacaa gtggctattt tccctgcagt gtttctgtgt 360
ggtgaaaata acccagtcca ctaaggggcg ggagtgaatg gatggctgga ttttccccaa 420

[?]ctcctata gcctaatgct gctaggatgt gagtatgagg aatttagcct tttatagtga 480
aatgagtcca actctgggct ttgcttanan gaaagctncc gtcaggcttn ctataatatg 540
aaaagaagtc accattgggg aactagagac cccagacctt ttcatatgga tatttgagaa 600
tgtaatgcat ntangcctng tgctggaact ttaggcctnt aggcnggtta aaacacttga 660
tttt 664

<210> 280
<211> 448
<212> DNA
<213> Homo sapiens

<220>
<221> misc_feature
<222> (1)...(448)
<223> n = A,T,C or G

<400> 280
actaccacag actgttgact tttagtttct taaagagaaa aattgccttt ttactagaaa 60
gcctttgtat attgcaattt ttctgtttgg gaaaatctaa ggatttactg tggttagtct 120
tacagaagaa atgtggattt gataaactag tgcctatgat tttaacttat gtttgatata 180
tagtagtaag ggttttatga atgttgatta ttttgtgcca acagcccaga attgtcactt 240
atatgtaagc agaaaacaat gagctctgct tccaaagtta tttaatttc tcagtgtttg 300
aatgttattt tttgtaagtg tgttaataaa agtgtaaaga attggaaaaa atataaatat 360
tcttaactca agcatttgct ggatcatttt tctacaaaac ttggttgtac tgngaacctg 420
tgtatcancg ttgtgtaaac ctagtacc 448

<210> 281
<211> 677
<212> DNA
<213> Homo sapiens

<220>
<221> misc_feature
<222> (1)...(677)
<223> n = A,T,C or G

<400> 281
gcgtggcgcg gcccgaggta caccttcaca gggaatccgc aggcggggat cttcagtctc 60
ctttaacacc ggaaagtatc aacgggacag atgatgaaag aacacctgat gtgacacaga 120
actcagagcc aagggctgaa ccaactcaga atgcattgcc attttcacat agttcagcaa 180
tcagcaaaca ttgggaggct gaactggcta ccctcaaagg aaataatgcc aaactcactg 240
cagccctgct ggagtccact gccaatgtga aacaatggaa acagcaactt gctgcctatc 300
aagaggaagc agaacgtctg cacaagcggg taatttcagg gctgatgtct atagggattt 360
agggctaaca ggttttcttg atcagaagaa attttgcatg tagattcagc acagggatat 420
cttctagttc taggatgtca gaacatagat atgggttgna tgatatgcat ttggttgatt 480
aagaaaaata ttttccatag tttaatgaga atgaagaata tacccctttg aagcaacaaa 540
ncatgtgatt cccatattat catggggcta gngtatgcnc agtcctgccc ggcggcgtaa 600
ggcaatcagn cctggngccg tctnnggacc acttggccac tggngacagg caactgtctg 660
ggaatgncct ccatccc 677

<210> 282
<211> 691
<212> DNA

<213> Homo sapiens

<220>
<221> misc_feature
<222> (1)...(691)
<223> n = A,T,C or G

<400> 282
cgaggtacct tgctgtttat tccttagtct agcagcatcc ttagtttgta gtatatctta 60
cttagttgca actaaaaaaa attgctagcc taggctttaa ctgggagttt ctattatcta 120
gaaggttact gtgaaccttt cagaaaagtg gaaagcaacc aaaagagctg tctcaaagac 180
tgtgtccccc cagagtttgt ccagctctta ctgtagacac tctgaacagg cacggttatc 240
tcatgtccaa agctcataac agcacattag aagaaagtgg ggagcctgtt agaagcaggc 300
atattgatag tgtgggagaa gacatagcaa attacttagc agatatttta aaaattttaa 360
aatccaacag cagtctgagg caaatgattc tgtatacctc agggctgaga gaatcacttt 420
ataacatatt tgntatagcc ctttacattt tatgaagtgn tttacataca tcagagctgg 480
atcttataat aatacattat gaatataact ttaacttttc atcatgaaaa tgtgaattat 540
actgacctga tgttaagaan aangccggaa ggtttctaac atacctgaaa tctcccttaa 600
aataattcca ggtttaaang tggncttgga aanttcctta ctttccaaaa tntatgacct 660
gccgggggcn ntnnaaggng aatccnncct n 691

<210> 283
<211> 668
<212> DNA
<213> Homo sapiens

<220>
<221> misc_feature
<222> (1)...(668)
<223> n = A,T,C or G

<400> 283
acatggttct gtgacatggc tggaggtggg cgttctggac aagtaaacaa tttactgggg 60
aggtgtctgt gtttcacact taggtcgcta agtttttagc caaggcttta gttgtcctcc 120
atgagcaatt gtagaaattg gaaatttgta atgatttttt atgagaaagg ccacgaatgt 180
gtgttactat tagagtatat ccacatattg tccagtcatg gaaaatggcc taaaagataa 240
tttacctgca aaacagaata ttatgcagct attaaaataa tgcatatgaa gatttgccat 300
agagtggaaa aatgcttgtt aggtaaaaat caaaaaaaca tgtaggaaac aaaattttac 360
atatttgatc tccactgtat aaataaataa aatggagaaa catttgagaa aaatcatcca 420
ataatggttg tctgtgggtg gtaaaagcaa ttgaaatgtc ttccttacac ttttaataat 480
ttttaaaaag tatgtaaaat gccaattatg acaatgctaa gctagatgaa catcccattc 540
aaattggaag cccatttaaa atttagaaag cncggttgga ttcccttctc tatccttttt 600
taaagcaaat ggcccannnc tggngnnttt ttgacccaac ctttcaaaat tnggctaact 660
ttntgaat 668

<210> 284
<211> 777
<212> DNA
<213> Homo sapiens

<220>
<221> misc_feature
<222> (1)...(777)

<223> n = A,T,C or G

<400> 284
acagtattta agggattttc cttttagctt ttcatctcca gtggcattaa acataaaaag 60
accctggcat tttttcacat acttgaatcc ctaaatgcac ctgtctttca ctttttgaga 120
cagactgaat atatctaaaa tttccagcaa taaaaaaaaa gcatttaact tgcaccaagc 180
aagaaaatat aaatacagtt aactgcatta agataatcac gttaaaattg ttactatgca 240
gcacagaact tcattcttat agtattcttg ggttcaacct ttgaatcaat tttaccactg 300
attaaataaa tgactcaaag acatctgtaa gtcatgctgc tgtgttttga aagtctttaa 360
ctaaattaag aatgcagaat ggatagtgat tattcaatta gaatttaagt aaggggatgg 420
tgatantana aggctggaaa atnccttaat ttttaaaaaa atcagaatag gcntttaaat 480
aggtaaaatc actttcaatt nttccccaaa acctgnangt ttcccggaaa aaaggtttta 540
aggctttnaa ggtggggaat gncccaaggt ttttaactta tnccatggaa gccanngcct 600
tgcatgggnn ccttagggna acccccngaa tcccnttccc aaaagggggg tttacccntt 660
tggaattnaa tttggggnaa ccttattngg nccttngggg nttaccttng gaaanaaaat 720
ttnnttttaa atnntttcan ggggnnggaa atttaaaggc cttttttttt gggaaaa 777

<210> 285
<211> 692
<212> DNA
<213> Homo sapiens

<220>
<221> misc_feature
<222> (1)...(692)
<223> n = A,T,C or G

<400> 285
ggtacaagct tttttttttt tttttttttt tttttttttt aaggatttac ttttcttaac 60
aagtgaacaa tttgcttcta agcgtcaatg aaaggcaaca cctccctnta atggccaaag 120
gaagagagtg gcagtaagct ggcttttcca atgngtcaca caatccttca tgccattaag 180
ttctccttgt tggaaaagaa attaggttgt tttgataact tagaaaagtt agttttagac 240
aacagtgact ttcagctaca aatacaaaat caaatccatg tatataaggc ttctgtaatc 300
gatgtcttag aggaacatct gctcattttc tccaagcccc agtcctataa atcaaggcaa 360
gtcaagtaat taagcttcaa ctattttggc agctttgcaa ttaaaatgag cnaagcacta 420
tatctatcct tcatatcngg atatattaaa ggtccaactt ggtacnccca atnttacatg 480
ccgagaggcc taaaatttnc nntttgggtt ccnggtttaa ttaaagncca taanggnctt 540
gcnacnaatc tttttcccct ncccaaggga aatttccctc nnattaccaa acccctgnct 600
caatttnttt ccccggnaat ttgaaaggcc gggtttntcc tttcaaaana aattttcccc 660
ggggattaan atttgggccc caatttctta nn 692

<210> 286
<211> 709
<212> DNA
<213> Homo sapiens

<220>
<221> misc_feature
<222> (1)...(709)
<223> n = A,T,C or G

<400> 286
   actgtgccag ggatattgag atgctctggg ggtgtattgt atacctgcca gttttcttca 60
   tttttgaatt gagttttttt tttttgatgt tggttttttt tatattattt taaggtttag 120
   atttgtgaag gaataagcat gatggaaata atagtcttga aaggagatat gttgtatata 180
   atcaggagga agaggaagga aggacttacc cattttgata ttttgctgta ggtggccagt 240
   tttgtttctc atagggaaat ctgacccacc tgtcatgttg gctcctaagg aactgctgtt 300
   gtaagcggct catcaagagt tgaacttcac gtagccttgt tgggaatatg gaaaaggaag 360
   aaagccacag gactgcccat tcagtcttgg gaagattggg atgattctgc acaagcaaaa 420
   atgactgaag tttatgtata gacacacctc taccaatcca tcttcagctg actgaatgtt 480
   gnatgatacc cttcttcaaa gcagangtag aatggtcang gttcacccat ggaattttct 540
   acttaatttc gtttttngga atcaacttta ccnnaatncc aggtcccctt tnggaaaaaa 600
   tccttaaatc ttttgctttt ttnaaaaaat aanttnggtt catanttaaa ggcccttggn 660
   ttaanccang gttncnggtn ccnatttatt tgaacccttt gcccttana 709

<210> 287
<211> 231
<212> DNA
<213> Homo sapiens

<220>
<221> misc_feature
<222> (1)...(231)
<223> n = A,T,C or G

<400> 287
acaagctttt tttttttttt tttttttttt ttttgtanag atgcgggtct cactatgttg 60
cccaggctgg tctcaaactc ctgggctcag gttctcctcc tgcctgggcc tcccaaagtg 120
ctgacatcac aggcgtgagc caccacaccc agcccctttg ggtgttttta aatataactt 180
tggcatttat aacaaatgca accacatgtt anatcttatt agaagtacct n 231

<210> 288
<211> 681
<212> DNA
<213> Homo sapiens

<220>
<221> misc_feature
<222> (1)...(681)
<223> n = A,T,C or G

<400> 288
   accctctctt ccagcaccca ggccagtatt gagatcgatt ctctctatga aggaatcgac 60
   ttctatacct ccattacccg tgcccgattt gaagaactga atgctgacct gttccgtggc 120
   accctggacc cagtagagaa agcccttcga gatgccaaac tagacaagtc acagattcat 180
   gatattgtcc tggttggtgg ttctactcgt atccccaaga ttcagaagct tctccaagac 240
   ttcttcaatg gaaaagaact gaataagagc atcaaccctg atgaagctgt tgcttatggt 300
   gcagctgtcc aggcagccat cttgtctgga gacaagtctg agaatgttca agatttgctg 360
   ctcttggatg tcactcctct ttcccttggt attgaaactg ntggtggagt catgactgcc 420
   tcatcaagcg taataccacc attcctacca agcagaccag accttnacta cctatctgac 480
   accagcctgg ngngcttaat canggttatg aaaggcaaac gtgccatgac caangataca 540
   acctggtttg gcaaggttga aactacaggc ttacctntgg accccgaggg gtcctnaaaa 600
   tgaagtcctt ttgacattga gcccaggggt actcaaggnt ttgttnggca aaaancttgg 660
   ccggaaccct angggaattn n 681

<210> 289

<211> 565
<212> DNA
<213> Homo sapiens

<220>
<221> misc_feature
<222> (1)...(565)
<223> n = A,T,C or G

<400> 289
actcaaccta acttatagtt agcagctgga attctcaact cttccctgcc agcactatac 60
cacagtgtgg aagaaattag tcaaatgctt gttttcctgc ttctcttttc agctgttact 120
gtgctttgtt tgaaagtagt tttctctctc aaagccgttg cttatatcgt taagaatgaa 180
ggtttgtgtt taaaatttat tgcattgcaa agggtagttt cactgaagtc atgcaccatt 240
aaataagatg aaatatttgt atttattgtc ctacttccta agccgtaact tcttttcctc 300
tgtgaatttg cattgagtca ctcatgctac actacatcgc tttagtattt gagatggcat 360
ttatgtttcc tctcgtttat catgaaatgg ggtcagattc catcagattc cacctctgtc 420
aggtggactc ttgtctgcct tccatgatga gatttttttt tctccttccc tttctttaag 480
agaggctgcn gaactangng gcaatcaatt tggnaaccag tctctggntt tttttcatta 540
gtaatttcta tcatagttca ctggg 565

<210> 290
<211> 699
<212> DNA
<213> Homo sapiens

<220>
<221> misc_feature
<222> (1)...(699)
<223> n = A,T,C or G

<400> 290
   ggtacacaat tctgcatttc tctcttggta atgggatccc agttttattg caggaggcag 60
   tgtgccagtc tcagtagatg gaacacgatt ggtctattca gccatgacaa ttctgttccc 120
   tgctgtctta gctttgtttg cagctagagg tgcaatggta gctggctcgg gccaagggca 180
   tctaagtgaa gatatgcaga gggagagagc aggaaacaga cttctgacga ggttttactt 240
   tctgatagaa ggtgacaggt ccagctagtt tggcccttcc tcttcctcca cccctccttc 300
   cttgaacgca gacatgattc ttggggatac agcagccatc ttgggaccat gaagtaacga 360
   gcactgagat taaggcaaaa ggatcaagac gtgaccccta ccttcgtgga gttggtgaac 420
   caataccatt aacccaccca tctccagaat ccatgctatg tggnaaaaca atcttctggt 480
   tggttaaacc actgnaattc aaggtttncn ttncttgcaa ctgaatggaa gnccttttta 540
   naaggtacct tgaccaaaat gccnaaggaa ncttggcctt tggaaattgg ancccgnaan 600
   acctgggttt ttaagcccat tttggcnncn tttnggnaag ctttaagggt aaggcctgaa 660
   cctttggccn aaagggggna actngggttc cccctttcc 699

<210> 291
<211> 699
<212> DNA
<213> Homo sapiens

<220>
<221> misc_feature
<222> (1)...(699)

<223> n = A,T,C or G

<400> 291
   ggtacttggg gacttcaggc atacagcctg tccagaatat ggctatccta ctctcctact 60
   cagaaagaga tcctgtccct ggaggctgta atttggagtt cgatttagat attgatccca 120
   acatttactt ggagtataat ttctttgaaa cgactatcaa gtttgcccca gcaaacctag 180
   gctatgcgag aggcgtagat cccccaccat gtgacgctgg gacagaccag gactccaggt 240
   ggaggttgca gtatgatgtc tatcagtatt ttctgcctga gaatgacctc actgaggaga 300
   tgttgctgaa gcatctgcag aggatggtca gtgtgcccca ggtgaaggcc agtgctctca 360
   aggtggttac cctaacagct aatgataaga ccagtgtttc cttctctcct tccnggacaa 420
   ggtgtcatat accatgtcat tggttgggac ccggttctaa atcatctgct ggctacattc 480
   ctgntnacac atacccttgc aactttgang cnngaaaagg taagtggggc cttcctaagg 540
   aaaaggnctt tccaaggggt cntcaatctt tttgncccgg ntnggntnct tnaattgggt 600
   ntttggaccc cnaatttggg aaaccgaaat attnttnana ggctttannn nnggggaann 660
   tntttnaaaa ccggntccnn nantggccct ttnaggtnn 699

<210> 292
<211> 688
<212> DNA
<213> Homo sapiens

<220>
<221> misc_feature
<222> (1)...(688)
<223> n = A,T,C or G

<400> 292
   acagtcatcc cactacctgg ctatttcatt acttggtgct ctagacaagc tcccaagaac 60
   tgactggatc ttggcttgtt ctgtttctgt cattgctaat ataatatgga aaacattgct 120
   gaaaagaaca gagatggcca tggatatggc taggttaggt attcatatcc aaatatctga 180
   actctaacct aatgtggata tgattctgta gcattatatt aaaagctatg atgatgcaat 240
   gcaggaaata acctttcatt ctccccccta gaggatcacg acaggtgctt caatgcctgc 300
   cttatctatg ggacagtagt gtgattctca gtgagaagtg aaggcctttg gggatttgag 360
   tcaggaaagg gaacatggct aagtgcctgg aaactctggc aacagtctgc gggtagaatc 420
   tacttggcct ctggataaga aaatctgtgc ttcantgaac ttaagnggtt tgggaaaatt 480
   taacccagaa ttttnnanga agcataagtn cctggttcaa ganaaccagc ttacggaaca 540
   tgcacattct taacatangc aacctttggc caatnaatcc catnggatgg cccccttaag 600
   ggaaagccat tttgggttct tggatcccaa cnttttaagt tcaaactttt tttttaagnt 660
   tttagntcct nggccccttt agnaaggn 688

<210> 293
<211> 572
<212> DNA
<213> Homo sapiens

<220>
<221> misc_feature
<222> (1)...(572)
<223> n = A,T,C or G

<400> 293
ggtactgctc tgctaggcca gtgacaaatg gccatcagag atgtggctcg ggtcagcatt 60
gtccttcctg gtgcaggcca tggttttatc agagcactga ccaccctgtg gcactgtaac 120

aggtgaccat aggagacttg tgcctggaga acttggggcc actgtggtag ggacagcagg 180
ggttctggaa atggacacta atcctaggat tggaaccccg gcttgctgtc tgctctctgg 240
gtgtctcagc ctgtctccca cctgcctggg actgttttct cttgggtgga ttgggaagct 300
catgtgtggc ctcatctcac ggggtgaggt gaagactcaa tgaggcacta cctgggttcc 360
acggggtgtc ccccgtgggt ctctccccca gggtgtccct gccccctgtg caagccagtt 420
tctgctgaat tacccagcca gctttgccaa accacctgac tttccttcag aagacttcag 480
gcngaaaaac agggttaaag acctacccct tctgaacttg gttcantgct antgcanaac 540
caagtccttc acaancttag gatcctatag gt 572

<210> 294
<211> 692
<212> DNA
<213> Homo sapiens

<220>
<221> misc_feature
<222> (1)...(692)
<223> n = A,T,C or G

<400> 294
acttcacaag tgtatgaaaa tgatgtgacg ttaacggctg ataaaggcaa aacagaggac 60
actttcttca tgagcaacaa accccaaaga tacaaagaca agctaccaga tagtggtgat 120
tctatgctta ggatcagcac cattgcttca gccattgcag aggcatcagt taatactgat 180
ccttcccaac ttgctgcaat gatcaaggca ctttcaaata aaaccagaga caagactttt 240
caggaagatg agaaacaaaa ggactattct catgtgcgtc atttcttacc taatgattta 300
gaaaaaagta atggatccaa tgcacttgat atggagaaat accttaaaaa aacagaagtt 360
agtagatatg aaagtgcatt ggaaaacttt tcaaggccta gtatgtctga tacttgggat 420
ttatctttgc caaagaacaa actactcaag acattcattc cggtggactt aagtgctcta 480
gtggnaatgt gaaggccccn gaagaaaacn cagcagctat tgttatgttg aaaatggnga 540
gagtgagaat caagaggcnt ttagaancct aaacttctca aatccggttc caattgagag 600
aatacngggc cntanttgat gggaaaactg tccnttgcac caattccaga agtnggaccc 660
atnaaaactn cctaatttcc ctccnttgga gg 692

<210> 295
<211> 459
<212> DNA
<213> Homo sapiens

<220>
<221> misc_feature
<222> (1)...(459)
<223> n = A,T,C or G

<400> 295
cgaggtacaa tgcaacaaaa tacaaaatac atgcttggtg aacattcgtt catatctaca 60
agacggcagc tagagattag gtttcaatac tgaccattta ctatcctaca agcaattagc 120
attacatcat aatatgccat caaggcaact ttttttatac tgaaaaaatc aaaataaaaa 180
ccgttatttg taaactttta tacgaaatgt aactcttcaa gtggaaataa aaaataaaat 240
ttgtctattt actattgaat acacatagga tttcaatttt cattataccg agaaaaaagc 300
tcttttgtgt tgggaaaata atgcttcaaa aaataattag tagaaaaacc cactagtata 360
atgntttgcc tttcaatgcc agcacagatt tgggaacata ctgaggatga aagttataga 420
cattcacagg tgaaatgtcc tgccnggcgg ccgtcgaaa 459

<210> 296
<211> 677
<212> DNA
<213> Homo sapiens

<220>
<221> misc_feature
<222> (1)...(677)
<223> n = A,T,C or G

<400> 296
taaagactac ctacacatag atatatgatt ccaaagtcat actttctcca tccccacatt 60
agccaagtga atacagggcc aaatgggttc ttggaatgat aataacaaag cattacaaag 120
tgggtcccct tggttccagc cttgtccaga gtttttggtt atatatttct atttattaca 180
atttaccttt taaattgtaa aataaacctt tgtgtggaca gagccaatgt ttcaatcttg 240
aatgagtaaa gaaaatactt tggaactgat cctcattttg aaattggttc taaattatta 300
tccatttcca atgtctgaaa ttctcttact tcctgctaaa actctctttc tgccaaagtt 360
gtttcgtaat ctgtctcaat gactataatg taaaattaaa gaagtaacca tgcttctcaa 420
ggggggaatt aaaagtggtt aatggatttt actcaggcta attggttggn cagaaattcc 480
taaggccaca gctttngggg ggtccgtgta natgtccagg anggcagnga cattagttcc 540
ttcttntgnt aatcccaaaa cttagaaacc nataatctta ccctggcatt tcctttntaa 600
aatggccagg ccnttggggg ggaccttggc cggaccccct tanggggaat ccnccactgg 660
gggccgtctt agggann 677

<210> 297
<211> 574
<212> DNA
<213> Homo sapiens

<220>
<221> misc_feature
<222> (1)...(574)
<223> n = A,T,C or G

<400> 297
accgtggtgt tagaatgatt gttatgtact gcagacaaaa tctgctttta gaggcaagcg 60
gatttctgac aaagtaactg atcctttgga tggcataaat tcactttggg gactagcctt 120
attcttcctc tgaggtcctt cgttcttcaa tttattcaat tcatcaatca aaagtgttct 180
cttcccagtt gcaattagaa gaagtctttc tgcttcagct tcttctaggg acccttttcc 240
atgttcttca tcaacacagc agttaagagc ctggctagct tgatagatca ctgtctgttg 300
catatttatt tcgttattga gttcctgcat tttctgtttg atattaactt gacaaggaaa 360
ggcattattt ttttcatcca gttttgaagt aacatcttcc ttccgaacaa tcacctgctt 420
tattgatgga cgttctgntt ctttgaatct ttgagatcta tatgcatcaa tgctgtaaag 480
aagatcacga tcttcagaac ccaggctatc accagattca actcgangga ccnagttctt 540
tggaattttc ctgggtttgg actttcatca cttn 574

<210> 298
<211> 535
<212> DNA
<213> Homo sapiens

<220>
<221> misc_feature

<222> (1)...(535)
<223> n = A,T,C or G

<400> 298
ggtacattta gctttggaat gatggagaga cacagagata tatgtaaacg tcaagagaat 60
cactccactc cacgtctggg tccacaccct tccaggcttt gtctggaaca ttatgtggct 120
ggtgcctgat tccacagtga ggatgcagga gcccaggtgg tgatggataa agcattagga 180
gacaatcaag tgtcaggaat tggtcaataa gaacggctta aataatgatt taacaaggaa 240
gacgagtaaa aaacaatccc atttcatctt tagaaagaat taagtcacta aatgatttct 300
tctaagttgt tgccatttgc ttggatgaga tcttgaaggt tttccattct ttctccaccc 360
agttaagaac acattgacta gaaatttgtg acaagaatct agtaaaggcc ttttccctcc 420
tgctcctcat tatgccaatg caagaacact tatagcttcc tgngccaaag tatttgacat 480
ccatgncttc atcttggcct aacttctgna gtacctggcc gggccggccg ttcna 535

<210> 299
<211> 644
<212> DNA
<213> Homo sapiens

<220>
<221> misc_feature
<222> (1)...(644)
<223> n = A,T,C or G

<400> 299
acatatttcc cgggataaga tcaccaggcc aggagcgaag ctatggaaga aaggggaagg 60
gctccccaac tttgacaaca acaatatcaa gggctctttg ataatcactt ttgatgtgga 120
ttttccaaaa gaacagttaa cagaggaagc gagagaaggt atcaaacagc tactgaaaca 180
agggtcagtg cagaaggtat acaatggact gcaaggatat tgagagtgaa taaaattgga 240
ctttgtttaa aataagtgaa taagcgatat ttattatctg caaggttttt ttgtgtgtgt 300
ttttgttttt attttcaata tgcaagttag gcttaatttt ttttatctaa tgatcatcat 360
gaaatgaata agagggctta agaatttgcc atttgcattc ggaaaagaat gaccagcaaa 420
agggttacta atacctctcc tttggggatt aatgctggtg ctgccgctga gtttcaagaa 480
ttaagctgca gaagactcag gagcaaagaa ccccatntta agggtggagt gtaccattcn 540
tcaaatgcca ctgggaagct gtttaancat ttggngtatt caaaaaaaaa aaaaaaaant 600
ttcttgccga ccctangnaa tcaccctggg cgtnttngan cann 644

<210> 300
<211> 642
<212> DNA
<213> Homo sapiens

<220>
<221> misc_feature
<222> (1)...(642)
<223> n = A,T,C or G

<400> 300
   accttcccaa ccattagagt gagtcaccct agaagcaaat tctccagctc cagtgcatcc 60
   tttagataac tgccactctg gtcactatct tatctacaac ctcatgagaa acctcagcca 120
   gaaccaccca gctaagttgc ctctgaattc ctgagccaca gaaactggga gataatgttt 180
   actgtttaag actttaaatt tggagtaatt tgctattcag ccatagaaag tgacactcat 240
   ttcttcgtgc ccgacactgc tgtctctgtg gtttcacatc cctgtggtta aagctctcca 300
   aygycccacc accaacccca yyacaaaacc caaacccccc aacacaycac ayycccccca 360
   caaactgcct cctgtgtgcc tctcagcccc atccggccca ctctgccttt cctncctgga 420
   tcactccagc tactctgaaa catactgnac cttnctaaat gcngacagat aaaattggca 480
   gacttttcat aggatgccca gtgaaatttg aatttcagat aaccatgaat aatgngtgtg 540
   ggtatacaat atttgggaca tcctatacta aaaatattgc tgacncatat tcttcaaggt 600
   attaatttaa tctgaaatcn catttaatan ggcatnttgg gc 642

<210> 301
<211> 589
<212> DNA
<213> Homo sapiens

<220>
<221> misc_feature
<222> (1)...(589)
<223> n = A,T,C or G

<400> 301
cgaggtaccg tattatgaac taacaaaata tttttgtttt acatcagtct taatagtccc 60
attttgctca attgggaata gtgctagctc tcttgtttga gaactgttac ttcaaaaaaa 120
atccaatgca aggtgctggt aagtcctctt cataacctta attaatactt gttagtgatt 180
tacagtaaaa ctgcttttag tgaagtatat tcacttggcc cataaacact gaaatagatg 240
aggtaatgat acattagtaa tgtagtaata aattagtatg ccaattctga caaaaaatta 300
ccaatagctc cccccacctt cacttacaag agggttcctg gtttgaaccc taacataccc 360
tagatataca tagcaattct gctgatagga aaaccaagtc ttagcacaca gctaataaat 420
gacaaacatg ggactagaat ttaagtctat actgccatga acctcatgag gaggagccaa 480
attgntaatt aagttgcact ctagttacca gcactaacan aacacaaacc aataacatgg 540
gtgtgggcta ttnanaaaaa ataactgggg gaaaacatta cttttntgg 589

<210> 302
<211> 577
<212> DNA
<213> Homo sapiens

<220>
<221> misc_feature
<222> (1)...(577)
<223> n = A,T,C or G

<400> 302
ggtacttgaa atgttgctgg ttaaaagttt ttctgcttta ctcattcctt tgacagcatt 60
aatttgtgaa catttatatt cagttcagct gtatttatgg cacaagatct catttccaaa 120
atggcactaa ttttccttaa gtgtaacagc actctatttt tagcagtaat tatattttta 180
aaggttaatt tgtagaacaa atgttttaac tatacttttt ttctactcta tactccccag 240
ttacagtatt tacaaagggc tgaagtctat ataaaaaaat gatctttggc tgggcatggt 300
ggctcatgcc tgtaatccca gcactttggg aggtcgaggc aggcggatca cgaggttagg 360
agtttgagac cagcctgacc aacatgaaga aaccctgtct ctactaaaaa tacaaaatta 420
gccaggcatg gaggcaggcg cctgtaatcc caactactcg ggaggctgan gcagggagaa 480
tcgcttgaac ccgggaggcc gaaggtgccg tgagttgaga ntggccattg ccttcagcct 540
gggtgacaaa cgagtttcaa aaaaaaaaaa acatttt 577

<210> 303
<211> 673

<212> DNA
<213> Homo sapiens

<220>
<221> misc_feature
<222> (1)...(673)
<223> n = A,T,C or G

<400> 303
ggtacattta gcccatgagc ctggcacaga tccctatcta gacatgaggc cctttagaca 60
tgactttggc attgaccagc ctgttggcaa tgggtcgggg aggcagaggg gatgctcaca 120
ccagtaattc tcatcccctg aatgcttggg atcacctggg gagagttcac aaaatactgg 180
tgcaggggtc ccacctctga tgatgctgag tggtgggtct ggggtgtggc ccaggcatca 240
tgatgtttca ggcccccagg tgacttctta ggcagcccag ctaagcccct agagccttgc 300
aatttccccc aaatgacctc agagggcccg atttgaggga aatgcctaac ttcaggggcc 360
cgtaagaatc ccccagggag catgtgaaat gcagatacca ggcccacccc cagagatgag 420
ctgangtggg tcaaggggtg aaagtgcang gatcaagtgt ttttcacaag ctccatacct 480
tcaggaaatg gtgttgtggt ttgggcccgt anaaaacatt cttgagagtc ctggtgnctt 540
gtgccttggt gcaccttggg gtgggaatnc caatgggncc ttgncnttga ggaaggatgt 600
gccattaacc tggtaagggg aaacccgaaa ccggtttcaa cttgnccttg gcccaaccgg 660
ggacccttcn aaa 673

<210> 304
<211> 426
<212> DNA
<213> Homo sapiens

<400> 304
ggtactgggc tcccatttat ttgaaatgtc caaaataggc aaatttgtag acgaaaagta 60
gatcagtggt ttcctgcagc tgaagtgtag gttgaaagtg gagcatgact gaatgccctt 120
tctaaaacaa gtaaacctat aattcatatt tccttaagaa aataaaaatt ttattaaatc 180
aagatttaat ttaccatgaa gaacacagag ttattattag tgcaagactt tattcatcct 240
ctccccagcc aaatcccaag aggatggcca cctttggaac tttttactgg cagcttactt 300
aacctaagtc agtctcctaa tctagtggtc tttgaaatgg ggatgtataa gacaaccatt 360
tgacacaggt agaaaacttt tactttttta agcccattcc cctggtaaac aatatatgta 420
cctgcc 426

<210> 305
<211> 655
<212> DNA
<213> Homo sapiens

<220>
<221> misc_feature
<222> (1)...(655)
<223> n = A,T,C or G

<400> 305
ggtacgagat tctgtgtgtc agccagttta ccctccagtg tgtcctgaag ggaaacaagc 60
ctgatttcca cctagcaatg cccacggagc aggcagaggg cttctacaac agcttcctgg 120
agcagctgcg taaaacatac aggccggagc ttatcaaaga tggcaagttt ggggcctaca 180
tgcaggtgca cattcagaat gatgggcctg tgaccataga gctggaatcg ccagctcccg 240
gcactgctac ctctgaccca aagcagctgt caaagctcga aaaacagcag cagaggaaag 300

[?]
aagaccgcag tgccagcagc ggggctgagg gcgacgtgtc ctctgaacgg gagcccgtag 420
ctcaggaggc agaattcaat gtgttatcat tgggcagaac tggatcctga aaaattcaag 480
atgctaagca cctacactac tttaagaatt tggaactgaa catgaanaag aagacngaaa 540
ttagaatttg ggaacctgaa tagcttttgc aaaaacaccc aagggccggt taatcgtttc 600
tggtggtgct nnggtggaat gatncatggg ccttgccntg ggncaagggg cngnt 655

<210> 306
<211> 684
<212> DNA
<213> Homo sapiens

<220>
<221> misc_feature
<222> (1)...(684)
<223> n = A,T,C or G

<400> 306
cgaggtacaa cacgcctcca tgtttcagca tctacgtcat gggcttggtt ctggagtgga 60
ttaaaaacaa tggaggtgcc gcggccatgg agaagcttag ctccatcaaa tctcaaacaa 120
tttatgagat tattgataat tctcaaggat tccacgtttg tccagtggag ccccaaaata 180
gaagcaagat gaatattcca ttccgcattg gcaatgccaa aggagatgat gctttagaaa 240
aaaagatttc ttgataaagc tcttgaactc aatatgttgt ccttgaaagg gcataggtct 300
gtgggaggca tccgggcctc tctgtataat gctgtcacaa ttgaagacgt tcagaagctg 360
gccgccttca tgaaaaaatt tttggagatg catcagctat gaacacatcc taacccagga 420
tatactctgt tcttgaacaa catacaaagt ttaaaggtaa cttgggggat ggctaccaaa 480
aggttaacac agtatttttc tcaaatgaac catgccttat tgcagaattc ttcntttttg 540
gaaagaacca ccggccaaaa cattccccaa cttntgtaaa agctggtggg gacctaatgg 600
ccgcccttaa ttctgacttt gaactggaaa nccttttaag naaaacttgg nggcttttnt 660
aacaaaatcc cgcgtanttt gnct 684

<210> 307
<211> 647
<212> DNA
<213> Homo sapiens

<220>
<221> misc_feature
<222> (1)...(647)
<223> n = A,T,C or G

<400> 307
caggtcttgt atacacaagc gtccatgtct cacacaaata ttgatgtgat tattcttaag 60
tgttaaatca ttaacactta aatgacttca ttgggaatat tgagcagagg gactgtgctt 120
ctatgcactg ggcaaggcag tatttgctta ggaaactaat ttagtcatca gagatacttt 180
cctaaaaagg aaaaataaaa aacaaaatgg tgccactttg ggttgaagct actttgttag 240
gcttgaattc atttatatgt cttttgattc ttaaaaaaac aaaaaacatt ccattagaag 300
caccagtttt tttgctcaga ctttgtggat cagactctac actcaacaca ctctaatcta 360
cttaaaggta tacaaaatat gctgatcttt tttaaattat gatttcctga atttttttct 420
taagtcgtct caactgattt actcacttag cttcctttcc tcatcaccta gtataataga 480
atgnatgtta catttttatg aatggcaggt gtcattataa tctgnattga cttaaaaagg 540
ttcttcctca tgatgctaat angtttttgg atanttggga ggatacncat ttgacagttt 600
tgcattttat gnatgagccn gtatccatga cggggcacgg attatag 647

<210> 308
<211> 660
<212> DNA
<213> Homo sapiens

<220>
<221> misc_feature
<222> (1)...(660)
<223> n = A,T,C or G

<400> 308
acctttgttg ctataaacca gatggagact gtggtgctat tttgtatttt tttttaatg 60
gaagggtgtt ggggtggcag tttttatcct tgaagacctc agatatgcta agtcaaccta 120
agcaaagtat actcggtgga accctagctc tgtggggtga tctgcaaaat agagtatcct 180
ggtcatgtaa gttcaggaaa tgctacagac tcaaggatta tttttgggga ttcaccatgc 240
acagcacaca ttgaaggctg aaaagtcctt gcagaaagga aactgactta actttgtttc 300
ttaaggatat ttgaccacaa aacccttagt ctgcatcaca ccaacctgat gcctnctgga 360
acctgtgttc tgtanaatgc gtattagaaa atgttggaca acctgtttca ttatcagaag 420
tcccatttct gangacagtg gtctctgnct ggaaaataan ggtccagaat ctcaanttcc 480
agggaccagn caaggtctgg cacttntanc cagtaaaacc ccattgcata aatcttcatt 540
ccatcaaggg tataanttgc ttgngcccct tnacaaangg ggaaanaact cggaanaaag 600
gtnccttggg ccgggaacac ccttaagggc caaattccan acaattgnng gccgtaatna 660

<210> 309
<211> 401
<212> DNA
<213> Homo sapiens

<220>
<221> misc_feature
<222> (1)...(401)
<223> n = A,T,C or G

<400> 309
ggtacacata tacacataac aagtgtagaa gtatatatta catacataca ctcactctgt 60
ctggtatagg ctaattttga agaactccca taagtttctg ctgcttctcc cataactgct 120
gccaccacca tcagaattca taatcaaacc taaccttttt gtttggggca ccaaatctga 180
agacaaaatt aatttgcacc agtaaacttc aagctgcttt ctttcttgaa aactaaacgt 240
ttaacgtata atgtctgttt ggatactgtt ccaaattgtt gattgcatgt ggttaatgtt 300
gcattagagc actttgcaat tgcataattc attaatgttt tgtgagcttg catttgtgag 360
ttattggatg atcagactga attttgcaag tatcacattg n 401

<210> 310
<211> 502
<212> DNA
<213> Homo sapiens

<220>
<221> misc_feature
<222> (1)...(502)
<223> n = A,T,C or G

<400> 310
   acatgtttat ggggactcct aacacagggc tcccctcttt ttcactagga gtttcactta 60
   cagctgacaa tctatggggg cggggggggg gcgcggcaaa aaagcaatga tggaccttgg 120
   ctaatccccc cgaccccttt cttaacaata taggtagatg tctatcgtca gcttgcctct 180
   ttgccaagac ctaggaggcg gctctgccat gagctgctgt gtgctgccct ccccaccttc 240
   agcacactca tctacacaca cacaggtagc acccacctcg atgagaccgc cttgctctgg 300
   cctgccccaa ccctggaagt tgaaaacata gagccattta tttctgcttc tactctctgn 360
   gcccatgtct tgtccacgaa actttgctga acttccagga ccttacacct gaagccccac 420
   aataacctgg atgttttgaa agccctngga aanccagttn taganaaagg acccccttaa 480
   gccgaaacag ggcctgttaa aa 502

<210> 311
<211> 387
<212> DNA
<213> Homo sapiens

<400> 311
cgaggtacct tactcagagg ggctttgatt tttttcaagc acaaagcaag aagttccctg 60
gattctaaag cacactgtat ccaagttcct ggtggttgaa aatacctttg acattgtttg 120
cagaacgaaa tcgagacttg tttcggaata ccttggctga tgtccacttt acttcgcaaa 180
caggccacac aaatattggc aggatttgga cttatcggaa caccacactc acagcacaag 240
atgtgtccag ggctgcggtc ggtggattct gccatatact ccatcgttct gtatgcctta 300
agttttcgcg cctccagacc agccctggat ttgctgaaaa cccgcaacaa aatagacccc 360
ggctgtcccg tcagctgcca acctggt 387

<210> 312
<211> 654
<212> DNA
<213> Homo sapiens

<220>
<221> misc_feature
<222> (1)...(654)
<223> n = A,T,C or G

<400> 312
ggtacaaaaa aatgcttctg gagatttctt tggcagaaat gcctttcatc tataatttca 60
tggagaactg ctttaattag cctaggtgaa aagtagtcct agcagtgtaa atatgtataa 120
ttagagtttt ctaatttcac tgtgagatct ctaacttttg agtggcaaac agatcaagtc 180
ttttgctcat agacttttct gtggggttat taaaatgcaa aagctttatt ttttttaata 240
atgccatact ccattagtgt cagatgatgg tatggaattt gttcccttgc tttcccccac 300
tgttactgct tcagtttata gattgccagc agagttcaga aatagagcag ggatttaccc 360
gttctttgct tggacatccc atttcttttt gccagaccca tgttggcaat catgtatgaa 420
ctgngttata cttctcagtg ctttcttttt tctttttgat aagatggata tcaaaaatag 480
ttgctgtgcc aaaagtagta agccttcttc aagaagaaaa cccaatcttt ttctaataat 540
aatcctgnga aaatgcttca ttcattcatt taatttttaa gccaaaggtc accaaangct 600
gntgntttta actangaaat ttgaaatgnn agnnttaaag cnttttaaaa aaag 654

<210> 313
<211> 656
<212> DNA
<213> Homo sapiens

<220>
<221> misc_feature
<222> (1)...(656)
<223> n = A,T,C or G

<400> 313
acagttctgt cctggcatca tcattcattg tagtatggtc aataggtgcc atgaaactca 60
gtagcttgct aaggacatga aaccgaagtt tcctgccttt gctggctttc ctatctactt 120
ttttgtggat tttgcttcgt aacttctgga ttgcaagcca ctgccttccc atggccacct 180
gatcgttggg atccaaggag ctggtcttcc gttctatgag ttctcgaagg agctggtggt 240
aaaagtcatc atcatcaaag atttcttcat ccaagtcctt cagatgagca ttagcagggg 300
cttgaggaag gatctccggt tcccctggca aactctctgg gacaggctga gctgctggct 360
caggtttgcc aagaactcga tagacagagc gcttggtctg tgtccttcga agtaatctct 420
ctttgnccat cagaatatgg tcgatctgag tcaaagattg aaccgttcaa angcaccaaa 480
acccttnccc agtttttcag aaacccagtt tggtcttatc gggccatttc tgaantgtgc 540
cggttcctgn aaactggtaa agtcggcaaa acgctttgcc atgaacttgg aatagncctc 600
catntccggt tnctttttgc anggaccctt ntttggtggn tgggtctttt tttttn 656

<210> 314
<211> 649
<212> DNA
<213> Homo sapiens

<220>
<221> misc_feature
<222> (1)...(649)
<223> n = A,T,C or G

<400> 314
ggtacatgga ctggacctgc ctggagccca gcccagagca tctcctcagt gctcatctct 60
atccagtccc tgatgactga gaacccctat cacaatgagc ccggctttga acaggagaga 120
catccaggag acagcaaaaa ctataatgaa tgtatccggc acgagaccat cagagttgca 180
gtctgtgaca tgatggaagg aaagtgtccc tgtcctgaac ccctacgagg ggtgatggag 240
aagtcctttc tggagtatta cgacttctat gaggtggcct gcaaagatcg cctgcacctt 300
caaggccaaa ctatgcagga cccttttgga gagaagcggg gccactttga ctaccagtcc 360
ctcttgatgc gcctgggact gatacgtcaa gaaagtgctg gagaggctcc ataatgagaa 420
tgcagaaatg gactctgata gcagttcatc tgggacagag acagaccttc atgggagcct 480
ganggtttag accctggtcc atctcccttc cccacttaag aagtccagca gaatcctttc 540
cccancccan ggatgganan gcctgggnat ctccttccan aattgaagtc atcttgcaag 600
aaggcaagaa ccaagcagct tcgantccan ggtgtggaat gggggcctn 649

<210> 315
<211> 238
<212> DNA
<213> Homo sapiens

<400> 315
acctgcaggt ggtggcagcg ggtagccggg actcgggcgc cgcgctctac gtcttctccg 60
agttcaaccg gtatctcttc aactgtggag aaggcgttca gagactcatg caggagcaca 120
agttaaaggt tgctcgcctg gacaacatat tcctgacacg aatgcactgg tctaatgttg 180
ggggcttaag tggaatgatt cttactttaa aggaaaccgg gcttccaaag tgtgtacc 238

<210> 316

<211> 637
<212> DNA
<213> Homo sapiens

<220>
<221> misc_feature
<222> (1)...(637)
<223> n = A,T,C or G

<400> 316
ggtactgtgt ttacatggtg agtggtcgtt accatccaac agcacaaggc acaaaaaatg 60
ggcatcaagc aaaccatgca taacgaggcc tggaaaccat caagaacagc cacaaaagag 120
gtcactcaga cctctgattc aaacttctgg tgtttgagtg acaagcatgc acgtttaggc 180
tctgcccaaa tatcagggag gatttccaat ctccacaaga gactggtttc acatatggcc 240
tttctcctgg ctgtcaaacc accagggttc ctccaaaaca aaatgagagc agctgttttg 300
ctgatcaacc aatcacacta gcagttctat ttcagtttaa aacaaccttg caggaataaa 360
ccacataaag actccgtggc taagggctgc tattacttac acctaccaag cgaacacaaa 420
cggctggctc ttctatggta acgcttcact ggcatgcaaa ccccaagggc cactgaatgg 480
aatgaatcca catgaacagc atacctggag caggaacatg ccttcacaag aagtgtcagg 540
agactaacct gtggttgcta acattnttgt gangaaaanc agggtagcag aagggtgggt 600
tgaagtnttg cctaatatnc ttaccatata tataaac 637

<210> 317
<211> 505
<212> DNA
<213> Homo sapiens

<220>
<221> misc_feature
<222> (1)...(505)
<223> n = A,T,C or G

<400> 317
ggtacattgg ccagactcat gcacaccaca tctgctgaca tctccttccg ttctgtgtac 60
tcattcagct gtcctgaagg atccatctcg aaatagacca gctctcctcc tgtcagggca 120
atcaccactt gtcgctggtt cactgcacac ttcacaattg ttttctttcc aggggtcttc 180
cactcattga ctctcttgtc tgctcgtatg tgccgaatgc catctggata gacctgcacc 240
aaggcatcat ctcctaataa ggagcaggac aaggtcgggg tggtccccag gaacccagag 300
tcagtcactt cttctacagt ttctccaatg gacaacacta gggtggcatt cacgaaagac 360
acaatgatgt aggcatcaaa ctcatcttca atgtgtcgac gcactgtcca nacagcgttg 420
gggttaccag gtanctcana aacagccatt tctgacacct naagtccatg gtttaaggac 480
ttttaaanat gatcngggnc ccctn 505

<210> 318
<211> 645
<212> DNA
<213> Homo sapiens

<220>
<221> misc_feature
<222> (1)...(645)
<223> n = A,T,C or G

<400> 318
gcgtgtcgcg gccgaggtac atacaaactg gggttctgtc aatgacaaca aggactatgt 60
gttggttcat atcaaatcca agaatattag acaaccaaac atataacctt cttgtggttt 120
ctcttaatat gcagcattca ttatggtagt taggtccctt cactggtttt ctgcaagtct 180
gaagttgtgt ttcttgtgtc gttgcccgca tctccaccct cagagctgct tttgttttcc 240
tcttctttgc agtctttgtc atcttcatct cctggagatt tccgggactg tttagaggat 300
ttctttgaag tatatgactt tttccgtttt gagcctgctt tttcattctt tcttttgcct 360
tttccatctt cttctactct atcaccttct tcctcactgc ttgcatctgc agtatttcca 420
ccttctcctc agtttctgaa ganctctggt gctgaattgc ctggtaccag taaactttac 480
tnctgggtat tttctatttc cacaatcctt cgttaaatcc tttccgttgg ttgacttttc 540
aaactggcnt tggacctggc ccggccggcc gtcgaaaggc gaattccacc attggcggcc 600
gtactaatgg atcnacttgg ncccacctgg cgtaatatgg catan 645

<210> 319
<211> 424
<212> DNA
<213> Homo sapiens

<400> 319
acttttccat aaagttctag tcacttctgt tggcctgagc caccagatta tgatgttgcc 60
agaattcact caatttgaat aaagatgaac agtatttgtt ttcttgtttc catgaattat 120
atcagtattc taaaacatcg cttcagaaag agaactgttt atttctgcag gcttcctgtc 180
cttttgtggt atggtttttt ggccttattt tcactggctt ttccttctcc aaactttgag 240
gcgtgatttc attcattgaa gaatcaatac atattttgtt tcaaaatgtt tgaaacaaaa 300
gacatagatg gtagactttt attaaaacat atatggatgt ggaaagcaca tatattaatg 360
cagtcatccc ttttcaggtg ggaagagagc aaaccagttg attttttaat tcatccttag 420
tacc 424

<210> 320
<211> 472
<212> DNA
<213> Homo sapiens

<220>
<221> misc_feature
<222> (1)...(472)
<223> n = A,T,C or G

<400> 320
acgaagtcgg gcaacaagaa agcgaggagc agcgtgtatg cccttatcct cagcaagtga 60
gaacaaggca gatcacagca ccgacacaga agatggcctt ctcccatgtg ccagcggaga 120
atccccttcc agccaaatcc tcaggaagca gagcaccaca caagcagcat ttcttggttt 180
ctcatggtca tattcaaaag cgacttttaa atcagaaaat agaaaaagca tttgtggtag 240
gtctttttca aacccagaac acaagttggc taggaaaacg gaaagcttcc tctggcatcc 300
ctgtttggac tcctcctcct cttggaggag tttcctgaac cgcacacaca tcgcttcctc 360
accaagagag atgctcaact aggatctttt ttagtgtgcc agttacaaga cacatttaca 420
ggctatgttt ctaagacctc ttagtggcca acgangaagg agggtacctt cg 472

<210> 321
<211> 588
<212> DNA
<213> Homo sapiens

<220>
<221> misc_feature
<222> (1)...(588)
<223> n = A,T,C or G

<400> 321
acctacctca caggtttgtt gtgaagacta aatgaagata atgcaataaa cggctgagac 60
ccatgccaag cacatggtaa aagtgtgtaa ttgcgtatta gcagcagcag ccagagcaat 120
agccaagggt caattaactc ccagtccagt gttcagttca tgattgtcca tgcattaaga 180
gccaaagcac ccccaaagcc atctcaccct gctgaagcag tctaaagtgc tcaactaagt 240
tggtgcatta atctctagac cagaggtcag cagacgtttt ctgtaaaggg ccagacagca 300
aacattttag gtctctgttg caactactca gctttgccct tgtgaatgaa agcagcaaga 360
caatatgtaa atgaatgggc cgtggcagat ttcatccaca ggggttccct gctttagact 420
gtgccgagag ccatangtct tgagttnaag tccaacctta ccacacttgc aangggtggt 480
ctttgaccaa gtcnnggaag gnntnccaaa agtcaaggcc cttaancctt taaaaaatgg 540
ggaataataa tgccttccnt caagagctgg tnaaacaatg gaagctgg 588

<210> 322
<211> 589
<212> DNA
<213> Homo sapiens

<220>
<221> misc_feature
<222> (1)...(589)
<223> n = A,T,C or G

<400> 322
acagctaatt gaaagtatat aaaaatgtga attagtgtgg ttgcagctaa aagtatgagt 60
gatgtaacaa gaatgacgac gtaatgagtc aagtggtgag actagttcta taagcaccgt 120
aaggagtgcc agtcctaata catgaacttc atccatccct tgtatatcaa ggaggagact 180
gtggtcagag aatgtatttt gtaagctata gtttaaaaat attactcttc agaaatttgg 240
agcccaagca ggaattacag agattcctcc caacagaggc cctgagatct cccctgactg 300
ccacccaaag gatccacact tgcctctgat caaccagatt caggccaagg cttanaagag 360
ggaggaggca gtggccagaa gccagggact ctagaggaga gaaatgatgg cagatgtggg 420
gttcagaaaa aacacaagac gggaaagggg aagaagggga aaaaaaggaa gaaccaccac 480
tggtgangaa attgttnaan aaggccacnt ttgcttgang agtggccctt gnctttttca 540
ccttgcctgt gggcaaangc tggcaagtaa agacaagggc ttaaccctn 589

<210> 323
<211> 582
<212> DNA
<213> Homo sapiens

<220>
<221> misc_feature
<222> (1)...(582)
<223> n = A,T,C or G

<400> 323
actgcttatg taaatcgttt atttttattt catcaaagcc tggcaagtat atgcattcca 60
atttaccatt ggcaaagctt tatttatttt taaggttgga tgttgaatta attttgtggg 120
aaaatgagat ttgtaagtag ttttctttct agataagata acataaacca agctttcaga 180

agttaaggat gatgaataat attgaaatga cttgttatat attgtaaggg ttcccttaag 240
tatcataatt aacaatttgt ggaaattgaa aaagcataaa ctgtgttatt tgattaagta 300
atatgttccc ttaaaattca ttttgaggtg tatgttatac acacagtaaa tttttgttca 360
ggaatgactt gctcattctg tgtttttaaa aataggaaat aaggcatagt gagtcatcat 420
tacatcaatt aaccnaaaaa atatttcatn ccctccgtca ctggaaatta tctacttcag 480
ncacctttct taatcctcgt gttaggaggg ccccgtttat gggccttttt taatttccat 540
gngccatatt gtccactacc cggcagtagc ccaaagctan ct 582

<210> 324
<211> 180
<212> DNA
<213> Homo sapiens

<400> 324
acccgtcggc ggcacccacc aacaaccgcg ggatcttctg aattgtggct agcgagcaga 60
tgtttttgtg gccgcagaat ggcaggcgga ccgtggcgaa ggctctgccc tggttgaaca 120
tttctgtcac ttgggaaggc aggtagctgg tggaggccat gagcactttc ccgaagtacc 180

<210> 325
<211> 575
<212> DNA
<213> Homo sapiens

<220>
<221> misc_feature
<222> (1)...(575)
<223> n = A,T,C or G

<400> 325
ggtacaaata ctgggaaaaa cctgctcttc tgcgttaagt gggagacaat gtcacaagtt 60
aaaagctctt attcctatga tgccccctcg gatttcatca atttttcatc cttggatgat 120
gaaggagata ctcaaaacat agattcatgg tttgaggaga aggccaattt ggagaataag 180
ttactgggga agaatggaac tggagggctt tttcagggca aaactccttt gagaaaggct 240
aatcttcagc aagctattgt cacacctttg aaaccagttg acaacactta ctacaaagag 300
gcagaaaaag aaaatcttgt ggaacaatcc attccatcaa atgcttgttc ttccctggaa 360
gttgaggcag ccatatcaag aaaaactcca gcccagcctc agagaagatc tcttaggctt 420
tctgctcaga aggatttgga acagaaagaa aagcatcatg taaaaatgaa agcccanaga 480
tgtgccactc ctgtaatcat cgatgaaatt ctaccctcta agaaaatgaa agtttctaac 540
acnaaaagaa ccngangaag aagcatgctc atcaa 575

<210> 326
<211> 584
<212> DNA
<213> Homo sapiens

<220>
<221> misc_feature
<222> (1)...(584)
<223> n = A,T,C or G

<400> 326
accagcaatc ttagttacaa aataatactt ttcagtagtc tttcttgatg cacatttaaa 60
aaccagcaca actcctctag tgaaatggtc aatttccctt aaaaaacaac atctgaaatt 120

ataagacctg acaaatcata ttatatttca atattagact gctgtggctc tagaacaaca 180
gaaaagcgta actttcaaac agcttaggga aaaagcactg aaatgtagat gtcgtcaatc 240
agcctcaggc attattgatc ctgtgccatc cacacaccct taaggttttt cacagcactc 300
tgacggtatt atgtgtgttt tgcaaatgac gaatcaacag tatgctgaat aatcagcaat 360
gaaacacagg agataaatta aatgtgtttt tccaaatgtc agaatatcga ggttcccagg 420
agttggcaaa acttctcaag gtgggccatt cagactcang ctgtgcnggg ataaggcttc 480
cttaccgtan gtgaaccggt tgagaatatt ggttccncac acccnagaag ccatttaggc 540
atatactggg caaaaaagaa acctgaatnn aatgggacca atnt 584

<210> 327
<211> 573
<212> DNA
<213> Homo sapiens

<400> 327
ggtacctctc tgaagcacac agaagtagcg ccaggcagag ggtttgaagg atatgtattc 60
atcaagaagt aaacgcaaat ccaagatctc aaccacactt ggctcttaaa gatccaccaa 120
cttaaccctt atggcatgca tatgtgactt ctgcaagaag caacttgaaa acccaagaat 180
gccttgctct accacgtccc gcgactgcaa actcccttcc tctgaaacaa gcagccacag 240
ctttataaga aacatgccgg catgtagtcc atcctgggag gggagaaatc ttcaccactg 300
gctgcctttc agcaagttcc ccttgaaatc tgccggcagt ggaacagatc ccagatccca 360
acgctgtagc ttgggcgtcc tcccaccagg ggttccttgt tctgaaagct gccaccagtg 420
ttgttccgaa agatgcctct gcctttgtgg ggtcatcttc cattatgcct cctaacagga 480
aacaggcttc tatggaagag aagagtccca gccccctgac ctttccgctt tggtcttgga 540
ggatctgagt cacatctgcc atgttgccta aag 573

<210> 328
<211> 422
<212> DNA
<213> Homo sapiens

<220>
<221> misc_feature
<222> (1)...(422)
<223> n = A,T,C or G

<400> 328
ggtactattt tgaagcgctg gaagaagaac tggtttgatc tgtggtcgga tggtcacctg 60
atctattatg atgaccagac tcggcagaat atcaaggata aggtccacat gccaatggac 120
tgcatcaaca tccgcacggg gcaggaatgt cgggatactc agcccccgga tggaaagtca 180
aaagactgca tgctccagat tgtttgtcga gatgggaaaa caattagtct ttgtgcagaa 240
agcacagatg attgcttggc ctggaaattt acactccaag attctaggac aaacacagcg 300
tatgtgggct ctgcagtcat gaccgatgag acatccgtgg tttcctcacc tccaccatac 360
acggnctatg ctgcaccggc ccctgagcag gcttatggct atgggccata cggtggtgcc 420
gt 422

<210> 329
<211> 467
<212> DNA
<213> Homo sapiens

<220>
<221> misc_feature

<222> (1)...(467)
<223> n = A,T,C or G

<400> 329
ggtaccacta tccccacttt acagatgagg aaaaaacagg ctcaagagtg aagtccctcg 60
cttgcttagt atctcaaagc taagctgcaa gcaaagatgg ggctccaagg tctgtgtgac 120
ctgagctctt ggttatccaa tacttcaaaa ctgtcactta ggaaagaaga gaacattttt 180
agaaatagga gaaaacccaa cagccacagt gattgtcaaa gagctgaggg ggcatcagac 240
caggttcggg ggcaccagac caggttcagg gccactgcgt aactgccaat gccctgccca 300
gccccaggag acacgcagac tccactgccc tagacgagtg gccctgctgt taataaataa 360
ataaaggtca ggcacaatcc tacacaaagg ccccagaatt caaaccactg tcttgnttct 420
cagacttttg cttaagagcc nagtacctgc ccgggccggn cgctcga 467

<210> 330
<211> 595
<212> DNA
<213> Homo sapiens

<220>
<221> misc_feature
<222> (1)...(595)
<223> n = A,T,C or G

<400> 330
tcgagcggcc cccgggcagg tacatggccg ccgtcctgga atacctgaca gcggagattc 60
tggagctggc tggcaatgca gcgagagaca acaagaaggg acgggtcaca ccccggcaca 120
tcctgctggc tgtggccaat gatgaagagc tgaatcagct gctaaaagga gtcaccatag 180
ccagtggggg tgtgttaccc aacatccacc ccgagttgct agcgaagaag cggggatcca 240
aaggaaagtt ggaagccatc atcacaccac ccccagccaa aaaggccaag tctccatccc 300
agaagaagcc tgtatctaaa aaagcaggag gcaagaaagg ggcccggaaa tccaagaaga 360
ggcagggtga agtcagtaag gcagccagcg ccgacagcac aaccgagggc acacctgccg 420
acggcttcac agtcctnttc accaagagcc tcttncttgg ccagaagctg aaccttatta 480
cagggaaatc attaattagc cggctttgaa ggtggaggcc taaatcatcc taccaatgct 540
gcattgacct taaagatgac ctaggaacac gctggagaaa aaangtggnn aggat 595

<210> 331
<211> 421
<212> DNA
<213> Homo sapiens

<220>
<221> misc_feature
<222> (1)...(421)
<223> n = A,T,C or G

<400> 331
acccaaaaac cacccccaac gccccccaac cctcaggcgt gcctgtgagt gtgtctgtgt 60
gtctcactct gactcaccca gacaactgac ttcagcagcc aaccttggtc attcccagaa 120
ccaccactgg ggggcatacg tgtggctaga ctgggggcgc ccgaatatct gtctctacaa 180
aaaaaaaaaa aaaaattaat ggggtgtggt ggtggtgcgt gcctgtggtg tcagctgctt 240
ggggcgctgg ggcaggagga tcacttgagc ccgagaattc aaggctacag tgagttaaga 300
ttacgccact gcactccatc ctgggtgaca gagcaagacc ttgtctcaag aaaaaatttt 360
taaatgagaa aaaaaaaann aaaanaaaaa aaaaaagctt gtacctcggc cgngaccacg 420

c                                                                    421

<210> 332
<211> 616
<212> DNA
<213> Homo sapiens

<220>
<221> misc_feature
<222> (1)...(616)
<223> n = A,T,C or G

<400> 332
cgaggtacca ggctacatat ctcggtcagt agctggatcc tttgataatg aaggcattgc 60
tatttttgca cttcagttca catactattt atgggtaaaa tctgtaaaaa ctgggtcagt 120
tttttggaca atgtgctgct gcttatccta tttctatatg gtctctgctt ggggtggtta 180
tgtatttatc atcaatctta ttccactgca tgtatttgtg ttgttactga tgcagagata 240
cagcaaaaga gtctacatag catatagcac tttctacatt gtgggtttaa tattatcaat 300
gcagatacct tttgtgggat tccagccaat cagaacaagt gaacacatgg cagcttgcag 360
gtgctttgca ttgctgcaag cttaancttt cttgcagtat ctgagaaccg attaccaaac 420
caagagttcc agaccctttc nttttggggg atactacttc agngctgggt cctanggcat 480
tattgntatc nggtacattg cccctggatg gcngttantc ntgggaaccg ggatncaaaa 540
cccntccata tgctanggnt gncctaacct acaatngggg cttttttgac aaaaanntgg 600
atncctccgg ggccnn 616

<210> 333
<211> 650
<212> DNA
<213> Homo sapiens

<220>
<221> misc_feature
<222> (1)...(650)
<223> n = A,T,C or G

<400> 333
ggtgggagag ctaagtctgc attatttttt ggaatcatta attaatttgc aatcacagag 60
tcttcaggaa aaaggcaagt tatcagctga agaaaatccc gatgactctg aagttccatc 120
atcatcagga attaactcta ccaaatccca agacaaagat gtcaatgaag gagaaacatc 180
agatggagtg aggaagtcag ttcacaaggt ctttgcttcc atgcttggag agaatgaaga 240
tgatgaggag gaagaggaag aagaggagga ggaggaggag gaggaagaaa cacctgagca 300
acccactgcg ggcgatgtat ttgtattgga gatggttctc aatcgtgaaa ccaagaaaat 360
gatgaaagag aaaaggcctc ggagtaaact tcccagagct ctgagaggtn tnatgggtna 420
ancctcnntt cgttttgnnt gaagagaacg tggngaggcn aatnttgngt gcctgggaat 480
nataaaaaca gctcttttgg cttatggcca tcttacttta ncctgatttt agggccnagg 540
ngcctngaaa atcntgccnt tgagtgatgc tggccttnaa tcccnggccc cnaaaaaggg 600
ttnactggcn aatttttggn nagcctttta ancggttttt ttgnttcaan 650

<210> 334
<211> 734
<212> DNA
<213> Homo sapiens

<220>
<221> misc_feature
<222> (1)...(734)
<223> n = A,T,C or G

<400> 334
tgntatctga gaattcgcct ttcgagcggc gccgggcagg tacagattaa cttaacacaa 60
aaacccgaac ttcaaaatga aggtgtgtgg aggaaaggtg ctgctgggtc tccctacaac 120
tgttcatttc tttgtggggc agggggtagt tcctgaatgg ctgtggtcca atgactaatg 180
taaaacaaaa acagaaacaa aaaaaacaag gaactgtcat ttccacgaaa gcacagcggc 240
agtgattcta gcaggcctca gggccctggg cctggggagg ctacatgagg gggagcctca 300
gtcacaggat caacctgggg cccgaaggag cagggttccc tgcctctccc tctgcaacag 360
atcatcccat ccaacacaac cccaaaaatg ttgatgatga cgcaacatgg tcaaccctna 420
agacctttaa gaccaaacag agcagcatag gaaaaaaaaa accaaacgca ccaatttctg 480
catgtgtcaa tggtagggca ccattttnaa aaagtttggc ttaaacaagc tggctttact 540
tgganggacc taatnccaag cttaattcct ttggtaangg aaaaaaccct tgaaccccnn 600
tctnagctta aantcttaag gttaagtccn aaccanttaa aacnttctgg gttnccccct 660
tccaagnttn aagccccctt ttccctnaac ctggggattg ggggnaattn accnggncnt 720
ttaaatttcc gngg 734

<210> 335
<211> 492
<212> DNA
<213> Homo sapiens

<400> 335
acatccttca ccaccatgga atattttagt ctatgtagtc aaagtcttct ggaattccaa 60
aagttctatc aatttatttt tcttcaaacc caaattttct tttggcccaa gattttattg 120
cgaatatgtt atgtatttct tccacaactt gcggatcaca gtctttgtat ttttctactt 180
ctgcctttag ctgttccctt tggtctcgaa gtgaagaaag ctcttttgct agcctggttc 240
gctcttccgt ttcacatcgg ccaattttag ctttctcaat gcttttctgt aggcttgcat 300
gcttttgact tccctcagac aactgagatt ccagaacctc caacttatgt ttccttgcat 360
gaagagcttt acttggaaaa gcccaataat aattagaagt tccgatcctc tcacagtcaa 420
ccataccatc atcaactaag ctttgaagga cttcttttac tgacatagca gtaatgcctt 480
tctctttggg gg 492

<210> 336
<211> 732
<212> DNA
<213> Homo sapiens

<220>
<221> misc_feature
<222> (1)...(732)
<223> n = A,T,C or G

<400> 336
ggtacatata aatgaatctg gtgttgggga aaccttcatc tgaaacccac agatgtctct 60
ggggcagatc cccactgtcc taccagttgc cctagcccag actctgagct gctcaccgga 120
gtcattggga aggaaaagtg gagaaatggc aagtctagag tctcagaaac tcccctgggg 180
gtttcacctg ggccctggag gaattcagct cagcttcttc ctaggtccaa gccccccaca 240
ccttttcccc aaccacagag aacaagagtt tgttctgttc tgggggacag agaaggcgct 300
tccaacttca tactggcagg agggtgagga ggttcactga gcttcccaga tctccactgc 360

ggggagacag aagcctggac ttttgcccaa cctgtggccc tggagggtcc cgggttgtca 420
attcttggtg ctcttgnggt tccagaagca agccggaagt ttgaaagaaa gggaaccttg 480
ggaatnaagg ggtgcttggg tattaanccn naaaagggat tggggttcct gnttccaang 540
ggancctttt ggcctttctt tttggncctt tncttaaggc cccaggccct nggggtttgg 600
accttngccc cggngggccc aaggggccna aattcccacc ncanttgggg ggcccggtac 660
ttaangggga atcccaactt tgggncccca aactttnggg gnaaancntn gggccaaaac 720
tggtttcctn gg 732

<210> 337
<211> 642
<212> DNA
<213> Homo sapiens

<220>
<221> misc_feature
<222> (1)...(642)
<223> n = A,T,C or G

<400> 337
ggtacaacag tagaagaagc aacaacaata gtaaagccac aggaaattat gttggacaat 60
atagaagacc cttctcagga ggatctttgc agtgttgtcc aatctggaga aagtgaggag 120
gaagaggaac aagataccct tgaactggag ctagttttgg aaaggaaaaa agcagagttg 180
cgagccttgg aggaaggaga tggtagtgtg tcagggtcta gtccacgttc tgatatcagc 240
cagccagcat ctcaagatgg aatgcgtagg cttatgtcta aaagaggaaa atggaagatg 300
tttgttcgag ctaccagtcc agaatctacc agtaggagtt ctagtaaaac tggacgaaga 360
tctccagaaa atggagaaac tgcaattggt gctgaaaaat tcagaaaaaa tagatgagaa 420
ttcagataag agatggaagt agaagaatct tcagagaaat taaagtcctg ccnggccgnc 480
gttcnaangg cnaattncac acctggcggc cgtctagtgg attccacttg gtcccaactt 540
gcgnatctgg gatactggtt cttggngaat tgtntccgtt acaatcncnc acttcaancc 600
ggagcttaan gtaaacttgg ggcntannag tgctnactcc tt 642

<210> 338
<211> 723
<212> DNA
<213> Homo sapiens

<220>
<221> misc_feature
<222> (1)...(723)
<223> n = A,T,C or G

<400> 338
acataaacac acgcatatca caagtctagt caagaaagaa atacatagaa aaacaagata 60
gaattttaaa aataatttgc aagggaagtt ctcaatgctt cagttctaaa atattgtctt 120
cttttagaaa aatttaagac tggaataaca gattgttttt cctgcaatgc tgtaattact 180
gcaaatttat cagcaaagag gtaaacagca atgcaatttt tccttaagct tgaatacata 240
agggaacaat aaagaaacct gattagacct gaactaatta aaagtcacac cagtaatttt 300
caggccagct ctggtctcca ggtagaattc caggacaggt ttgnatcact gggtccattc 360
ccaacaggct ggataggaga gtctggagta attataagga taccaccttc ttctatcctg 420
ggctgccgac tggcattggg cttcacattc ccagaatacc ttctgngnga ataggccctt 480
ttcaggggga ccnggaagga aggaaaaagg gggctntggn aaacatnggg ggattctttg 540
gnaaaatttc tggcctggaa tngtggcnaa cctttggggc ttggggtntn ggaaaatgtc 600
caaggganct ttaangggnc ccttngaact cggagggnaa aatttaaccc ctangggccc 660

ttgggttnaa aaagggcttt atttggggga cccgggttnc ccttgnaaaa aatgccncca 720
ann 723

<210> 339
<211> 356
<212> DNA
<213> Homo sapiens

<400> 339
acaatagtgt aaaggtggtt tttaaaaaca tagccaggtg tggtggcacg tgcctttagt 60
tccagctact caggaggcta aggcaggagg attgcttgag cccaggctgt gtggttcacc 120
ataattgtgt ttgtgactag ctactgcact ccaacctggg caacatagtg ggacttcatc 180
tctaaaacaa aacaaaacaa aattacactt aagcactatt gtttaatttt taattgtcag 240
tttatcatta ttttgggtaa gacattctgg ggtttcttga atcttgtcca aaaaccagtt 300
gttttggaaa attgctttaa attgagcata tttatgtata ttggataaaa atgtcc 356

<210> 340
<211> 502
<212> DNA
<213> Homo sapiens

<220>
<221> misc_feature
<222> (1)...(502)
<223> n = A,T,C or G

<400> 340
caggtacaat taactgtcac acagtcagat ataattcact ctgatgaggc cagagaaaga 60
aaacaaggca aagaaagggc tcatcttgtc cctttaggta atatccaaat atcccagcac 120
ggaaaccatc ttttcctcaa aggttatcta cacacgtggc ctgagaagaa aggcagtaag 180
cctttgggga gttggggaga aggaaggaaa agaaaacagg aggaggaaaa aggaagacct 240
cttttctgaa ccacaaatgc ctcatgctgc gcactccaag ctgaaataca gtatggtagg 300
tattctaagg gggaaaaaaa caactacatt tctttcctat tactgattcc tctctgcttc 360
acagacccag ctcggccaag tggaaaacgg ctgccatgag ttctgcagaa gctgcatgtc 420
ttgccctggc agtctgaagg tgaagcangc ttcanaggtg gacagctcaa ggagaattcc 480
cagaggncnc cnaaaagccc cc 502

<210> 341
<211> 243
<212> DNA
<213> Homo sapiens

<400> 341
acatcatcac cttcttggtc aagttttcca tccaacttaa ttttaggatt ctccggacaa 60
tcaacatttt cactgctttc tgctgcaatt ttctgttttg gattttcagt cacctcgttt 120
tgggcttcca ctgctgactt tctgtcagta gactttacct gctcttcttc cttaatttca 180
cttaaatctg tgttctgata cgttaactct tttttaacat ctttaagggt ttctacgggt 240
acc 243

<210> 342
<211> 669
<212> DNA
<213> Homo sapiens

<220>
<221> misc_feature
<222> (1)...(669)
<223> n = A,T,C or G

<400> 342
tgaggtcaag cttttttttt tttttttttt ttttttttca gctttgttgt agttganatt 60
ctgatgttca cctaacaaag tccctgacaa aacagacttc cttcaatcca ggtcataatt 120
tgaaacgtta tacaataatg agatttaagt gatgaatgga aagaaaagaa ggagactgaa 180
aagatatcag aaatttctat tngtttttag attcagaaaa atataattac aggccaacat 240
gggtntgaca gagaggaagg acgtcagcag ttacttgaat gtaacccctt cccagcattt 300
ccaaagacct gcaatgngct cattgngatc caagggcctt gntacctagt ttctaggnga 360
tctacagant tgaaacaacc cagcacaact ttatttcttg gagaagatga acccttaact 420
ntgaaggtgc ntaaaggaaa tnttnaactg gtcacttcca tgggtccggt ttcaaagcca 480
caatcnttcc gattaaanta aaacctggga naaaagccaa cggngggcaa ncaaacgggn 540
gggattctac ntttggtaac ccattgaacc gggggcttcn ttttaaanan gtgntcattg 600
gtttggtttt anaacctaaa nccccttttt tnaaaaaant ggtgnaaatt ttccncntnt 660
aacccggtt 669

<210> 343
<211> 500
<212> DNA
<213> Homo sapiens

<220>
<221> misc_feature
<222> (1)...(500)
<223> n = A,T,C or G

<400> 343
ggtacagggc agtgacatga gctttgacaa acagttcatg ctaggagtag agactgtgtc 60
ccaggactga gggatctgcc taagatcaag ggaaaaatct gaaagactcg tcctaacaaa 120
gtgtaaaact aaggttttat aagttcaagg gaactgacta ctgattagct gccagtgaaa 180
acaaaaatca acactctcag gtaacagaaa tcagaattgc tacaatgcat caccaacaat 240
gtccagctta caatttttaa ggacgactaa ataggagact cccagtttct agtctggcac 300
ataaggaggt cggcagtcat cacttcattc taacaagtaa aaagctgaac aaactaaaaa 360
atcaacaact cagccgggtg tggtggctca cgcctgtaat cccagcagtt tgggaggttg 420
aggcaggcgg atcatgaggt caggantttg agaccagtct ggcccacatg gnaaaacccc 480
ggtctactta aaanataaaa 500

<210> 344
<211> 483
<212> DNA
<213> Homo sapiens

<220>
<221> misc_feature
<222> (1)...(483)
<223> n = A,T,C or G

<400> 344
ggtacttcgg ccaaaaacag gagcccattg tgacaggcat ctggcatcac tacaaaggac 60

ccctggggct ccatggcaac caggcaggca ctaaggatag aaggagagtc tgcggcagag 120
attccacaca tccggcacac atccttgagc tttttgctga ttgtctgtag tgaacattct 180
ccaaggagga tactccaatc tttaagctcc ccatggccaa gacgcccaag tcgcccgatt 240
acaactctcc agggtagaga tgtcatttgg acaatcccta tgcaccactc ccataacttc 300
tgtagtccaa ttttacgtgc agatacttta ctcctccgtg acctaacaaa taaagaaatg 360
gggaagggga aggggtccct agataaatca gagttattta tcacttataa gaccaacact 420
agaaatttcc aagaacctat ccatgctgna cctgccnggc ngccgtnnaa aggcgaantc 480
agc 483

<210> 345
<211> 667
<212> DNA
<213> Homo sapiens

<220>
<221> misc_feature
<222> (1)...(667)
<223> n = A,T,C or G

<400> 345
ggtacaggag agaaggctct tatgaccgat acctacgaat ggatgactat tgcaggagaa 60
aggatgactc ttattttgac cgttacagag atagctttga tggacggggc cctccaggcc 120
cagaaagtca gtctcgtgca aaagagcgtt tgaaacgtaa ggaacggcgt agagaagagc 180
tttatcgtca atattttgag gaaatccaga gacgctttga tgccgaaagg cccgttgatt 240
gttctgtgat tgtggtcaac aaacagacaa aagactatgc tgagtctgtg gggcggaagg 300
tgcgagacct gggcatggta gtggacttga tcttccttaa cacagaagtg tcactgtcac 360
aagccttgga ggatgttagc aggggaggtt ctccttttgc tattgncatc acccacaaca 420
ccagatcacc gntcctgcac aggtcaacat catgtttgga accccgnaag aaccttgnaa 480
catgccccaa gncnatgcca tggtgctggt ggccanaaat ttttagccgt tccaggaatt 540
aattcccgga anaaggaacc tnagggnaat gccnaaccgg ccntcaaann gcccatgaaa 600
ccttcttgcg gaaaaaaaaa gggggcctna ggagggatcc ttggggcccc tttaancntt 660
caancnn 667

<210> 346
<211> 754
<212> DNA
<213> Homo sapiens

<220>
<221> misc_feature
<222> (1)...(754)
<223> n = A,T,C or G

<400> 346
actgaactac ttcattacca actcggccca gatattgaca tgcctgatga taacaaaaga 60
attagaaggg tgcgtctcct ggtggaagag ggctgtgaag atcgaattct ggtagcacat 120
gacatacata cgaaaacccg gctgatgaaa tatggaggtc acggctattc tcatatactc 180
accaatgttg ttcctaaaat gttgctgaga ggcataactg agaatgtgct tgataagatt 240
ctaatagaga accctaagca atggctaact ttcaaatagg atggttgctt atgaattcac 300
accttgagta taaaacttgc agagaacatt cagcgatttc cagtccactg tgagatatta 360
atcagttacc taggactaat gacagatcat ttccttctga tgagaactag gaggggtttg 420
ccttctctga gacccagcta ttacaactgg gccctntaag ggaggtactt aagcctaaat 480
tgagccccta ataatttnaa cttaacccaa anttaattnc cggaanttcc cttngggccg 540

ggaaaccacn ccttaagggg ccnaaatttc cagcnccaac ttgggcgggg ccggttactt 600
aanggggaat ncccaaactt tggggncccc aaanctttgg gcggaaaacc atngggccct 660
aaacctnggn tnccccnggg nggaaaaatn ggnaattccc ggtttnanaa atttccccnn 720
ccaanntttt tcnnaacccc ggnaagccnt taaa 754

<210> 347
<211> 444
<212> DNA
<213> Homo sapiens

<400> 347
accgtctcga tcatctgctt cccttgggct gagagctcca ggggtgactc gaaggtgacc 60
ctataaggag tcatgagggt cctgaggttc tggaacagct tctctccatt ggggttcccc 120
agaatgtagc agcccatgat gtggatgacg ttcggctctg ggttcacttt gctcatcagg 180
cggctcagcc gcttccagaa gtgaatcatg tcctcttcct tctccacttt ggcaaaggtg 240
gccaccttgt tcttgaggag atagaggtgt ccaggacctc cctggcagaa aatcagcatt 300
ttccagatct tggctccctt gtggtagacg ttcagcttcc tctctatctc ctcaaggatg 360
tcctcgaagg ttgcgtgctc atggtccgta gaggatgggg atgatggagg ggtcatcccc 420
ggcggatgat agtggggatg tacc 444

<210> 348
<211> 693
<212> DNA
<213> Homo sapiens

<220>
<221> misc_feature
<222> (1)...(693)
<223> n = A,T,C or G

<400> 348
ggtactttaa gaccctttgc cttaaagtac tataccaaca cagactttat agtatgttta 60
aaaatcccaa ctgcaagata cacaggatgc tgtaggcctg atttcctgtt gtagaacctc 120
cagccctgtg ttgaatgagg aggtgcaaat atatagaccc ttaagatcag accacagcag 180
gcattcaggt ggaggggatg aactccattc attccagctg tgcagtggga catctgcgcc 240
ctccgcatct cggctcattc ctcatctgag ccactcaaga gggcggtctg gtaagtgtca 300
tctgaattca gcttctgaat tccaatgatt tctccccttc cgtgtctctt catccgagtc 360
aaaaggcagt aaacaagaga atagttgacg gccacaatgc tgaaggcagc aggtagtgcc 420
agcagaaaca catggtgatg aacatgaagg tggcatcatc cttctggncc attcnggtgg 480
tncaaaaggt gggaacngga caaaccncaa ttttgccnaa ccangttccn tgnaaaatga 540
ttaaactggg tccggaaaaa gttccagcnc aatggnggtc ccggaaanat cnccntttng 600
ggggantctt acnccncctt ttgaaaaggg ctttccncng gaatgaanng aatnncttgg 660
nccaacggaa ggcccgtttg nggcntngta atn 693

<210> 349
<211> 299
<212> DNA
<213> Homo sapiens

<400> 349
cgaggtacat tctctaaaaa ttgttactga ctggtaagaa atagacctga gtttttattt 60
ctaacaccca atcactaaac cacggcagca agcactggcc accgatttaa tggattacga 120
cacaggaaac cccatcaggg ttctatgtaa tttagtgata ctcatgtcac taatattgag 180

cattatactt gatctgcatt atattgttga tatgcagagg ctaaactagt catcatttgc 240
tctttcatct atcagtagag tccaaagttg tttgcttgaa tggactacat gttaaaggt 299

<210> 350
<211> 622
<212> DNA
<213> Homo sapiens

<220>
<221> misc_feature
<222> (1)...(622)
<223> n = A,T,C or G

<400> 350
actgtttacc agatctttgc agatgaggtg cttggttcan gccagttngg catcgtttat 60
ggaggaaaac atannaagac tgggagggat gtggctatta aagtaattga taagatgaga 120
ttccccacaa aacangaaag tcaactccnt aatgaagtgg ctatnttaca gaatntgcac 180
catcctggga ttgtaaacct ggaatgtatg tttgaaaccc canaacgagt ctttgtagta 240
atggaaaagc tgcatggaga tatgttggaa atgattctat ccnnngagaa aantctggct 300
tccagaacga attactnaat ncatgntcac acagatactt tgangccttt gaggaatctg 360
cattttaaga aatattggtg cnctggnatt taatancnna aaaagggctg cttgcatcaa 420
tagaanccat tncttaggtn aagctngtat nactntgnat tgcacccctc atttgcngaa 480
atgtcnttcn ngnnaactnt ggtacggaac tcctccatnc ttatcccngn aagttntccn 540
gagccanagg gtncnaccnt atcctatana nnagntcnnt cnggacntna tcnnctttng 600
ggnnccntag tggccctttn cc 622

<210> 351
<211> 574
<212> DNA
<213> Homo sapiens

<220>
<221> misc_feature
<222> (1)...(574)
<223> n = A,T,C or G

<400> 351
gctttaacaa tagcagcaga caaaggtcac tacaaatttt gtgaactcct gattcatagg 60
ggagcccaca ttgatgttcg taacaaaaag ggaaatacgc cactttggct ggcatccaat 120
ggaggtcatt ttgatgttgt gcagttgcta gtgcaagcag gtgctgatgt ggatgcagca 180
gataaccgga aaatcacacc tcttatgtca gcatttcgca agggtcatgt aaaagttgtt 240
caatatttgg taaaggaagt aaatcagttc ccttctgata tagaatgcat gagatacata 300
gcaacaatta cagataagga actgntgaaa aaatgtcatc aatgtgtcga aaccattgtg 360
aangctaaaa gaccacaagc tgcaaaagca aataaaatgc cagtntcttt taaggaactt 420
gatctggaaa agtcaganaa agacngaaac agctttgtgt aaagagaaaa gaangaaaga 480
gnaagaatag agaccgaagg actgagaata naacactagg atcgactcca gtaataagga 540
ttaattgnaa ntctaacttt nccctcatga ttgn 574

<210> 352
<211> 399
<212> DNA
<213> Homo sapiens

<400> 352
ggtacataat attccagtag gaaactgctt ccaagtttaa gcatgagctc cccaaactgg 60
agaaaacata ttttgctatt ctgagacaac aatcagaata cagactttgg attccaggtc 120
acagtttgct ttttagacaa ggtaaagcaa agaaagccac attgtgccat cttcagctcc 180
agtggcttta gcagtgactg tttgacataa aacatgtaag aattgcttgt tgggaagagt 240
gctttaggga cccactgttt tcatttcttc ttggagttta ccttgtttca gatgcagcca 300
tgggtaggtc agagatggac tgttggtgca ataaacccaa gaatcaatgt agcctcttaa 360
tcccatcaag atgtagtttg tagcagcaaa agtgtacct 399

<210> 353
<211> 727
<212> DNA
<213> Homo sapiens

<220>
<221> misc_feature
<222> (1)...(727)
<223> n = A,T,C or G

<400> 353
ggtactttta cccatttcca gttccacctt tactttatca agtggaactt tctgtgggag 60
gacagcaatt taatggcaaa ggaaagacaa gacaggctgc gaaacacgat gctgctgcca 120
aagcgttgag gatcctgcag aatgagcccc tgccagagag gctggaggtg aatggaagag 180
aatccgaaga agaaaatctc aataaatctg aaataagtca agtgtttgag attgcactta 240
aacggaactt gcctgtgaat ttcgaggtgg cccgggagag tggcccaccc cacatgaaga 300
actttgtgac caaggtttcg gttggggagt ttgtggggga aggtgaaggg aaaagcaaga 360
agatttcaaa gaaaaatgcc cgccatagct gntcttgagg agctgaagaa agtaccgncc 420
ctggcttgna ttggaccgaa gttaaggcct anaatccaaa tgaaanaccn aaancccctt 480
ggtncaangc cnccagaccc anggccccat aattttttgg ccncnggggg attcaaatnn 540
ccnttttaan ccncgacttg ggnccncnaa attcncgccn ggggccnaaa naaaggggta 600
naaaggggan ccccaanagt tacccttgnc ccngggcnng ggnccgtttt tnaaaanggg 660
gtcnaaantt cccatntcnc attggggggg gcccgttttc ttagggggaa tcccgagctt 720
tggggnc 727

<210> 354
<211> 411
<212> DNA
<213> Homo sapiens

<220>
<221> misc_feature
<222> (1)...(411)
<223> n = A,T,C or G

<400> 354
ggtaccatag gtcatttctg gccgatagtc tgaatttaca gcccattgct ggtgaaagtt 60
tagtaatttt aaattgtttc tgtgagccca tgtaacactg acaaaattct ccatttcctt 120
ttccttcatc ccattctaat acaaagtttt ggattttaga accattgtca ctaggtgcct 180
tccattgcaa agtgagtgaa tttttggtcc gattggctat ccttggtgga ttaggtatat 240
caggttcaca gctcaaggtg gtaaagattt cagcctctga aggagttccc tttatagaat 300
tatattctgc ctggactttt gcatggtaat ccatggctgg cttgagatca tttaaagtga 360
tatttgnttc ttctctacat atacactttt ggatttccca tcttttccag t 411

<210> 355
<211> 331
<212> DNA
<213> Homo sapiens

<400> 355
ggtacttttc tctatctgat tcagccattt ctgccagagg gaaaaggtcg gcagaaaaga 60
tgtattgagt gaatagttaa ggataggatc tttgtccaaa aatttcagaa agattgagca 120
aatctgacgt attcattgag tgagtttctg tgttttcaaa ggtggaggag aaatttgtgc 180
tggaagtttt taagcctccg ttttcttgga aatcagtctg taacactggc aagtcttaag 240
atagtcccgt ttagactttg cagatgctga acctggctct gtaacgctgg gaagtcttaa 300
gatagtcctg tttagacttt gcaaaccctg t 331

<210> 356
<211> 678
<212> DNA
<213> Homo sapiens

<220>
<221> misc_feature
<222> (1)...(678)
<223> n = A,T,C or G

<400> 356
ggtacttttt aattcagcac cttttcaaaa tatgtgctgg gatggattct tcttagggaa 60
agccccatat agaattctca ttttggagca tcatttttat atgctatctc cccagtgtat 120
cttctcaata tttataacac tttatgaaat aaatattggg ttgcctgtaa gaagagaaaa 180
atatagctct ttctgagaaa gagcatttgg cttgcagttt acagcaagag ctgaaattag 240
agaccatagg gatttccaag accaatttga ccagaaatac aaaaattctg atgtcaaaaa 300
ccctctcaca aaatttaaca ggtagaaatt atttagcag tatagcctga aatccagtgc 360
aacaaaaatg natcccaatt ctatgatatg ncataagtat gntctcttan ctggcttncc 420
ttacttggtc ctactcccta cttggacctt tngggaagaa aatggtcggc ccaancccat 480
ctttcaaatt ttcnaattcc ttaatatgga accettagcc atggaataac caggggcntt 540
aaagttcccc ccatttaaat aatgnccctt aatntggnaa anggcttgaa ancctggncc 600
aaagggctgg ggtcttttaa gccctttgaa ggttaacctt caaaaggggg aaaaaaccnt 660
ttttttttta agttgggg 678

<210> 357
<211> 414
<212> DNA
<213> Homo sapiens

<220>
<221> misc_feature
<222> (1)...(414)
<223> n = A,T,C or G

<400> 357
acaccgagaa ccataatgaa aaaaccttcc gtgtgttttg tcatgttttg ttccagggaa 60
gcagttgatg agtgctgtta ctaatgcttt ctcccagatc cattcagtgg tggagaggag 120
gaaaatgggc tggttggatg tggtcttggt gccttgcagt tactctgcac tggttatgca 180
tttaattctc ctcttttcta gttaaccttt tgccagtggg ttttccatag tctgggtatt 240
tgtccttata tcagttatac cacctaaggc aactgggtgc aaaatgcatt ctgttcactc 300

actgtctggg ccttccccac cctagtcttg gcacattcct tcaagaatgt agttaccgtc 360
tgcttgggaa gatgtcagtg caaatgtgaa gataatgggc atcggnaaac ccct 414

<210> 358
<211> 633
<212> DNA
<213> Homo sapiens

<220>
<221> misc_feature
<222> (1)...(633)
<223> n = A,T,C or G

<400> 358
cgagggtact tcaaagaaag tcaaatccta agcctgccca ggcccaaaga caaagccagc 60
caggacctga ccacctgtat cctcttggtg gcaatctgct gaagccagat gagttctgct 120
ttttaattcc aatcctattc tgccactgaa actaggcctg ggcaaccact cttaatcatt 180
aacatatcaa aaggagtatc tcctctgaga aaagagcttt tctcaggttc tagaagctag 240
cttttacaaa agacgtcttc aaataggggc cgggtgcagt ggctcacgcc tataattttg 300
gcactttagg aggctgaggt gggaggattg cttgaggcca ggagtccaag accagcctgg 360
acaacgtagt gaaacatcta tttctaccaa aaaatttaaa aaaggaaaaa attatgtcct 420
aaaatattaa anggncatta aaanggccca ctngaacttg gaactttggg gaatctagtg 480
caacaacccc ttgccggana gaagaanctt naaccagctn ttgaattgcc nggtcaaant 540
ggtttatatt aaaaccgata ccactttttn ataatccttt ggnaaatnaa ctgtaagccn 600
tttttccctg aacggaccnt gcctgcccaa ttt 633

<210> 359
<211> 635
<212> DNA
<213> Homo sapiens

<220>
<221> misc_feature
<222> (1)...(635)
<223> n = A,T,C or G

<400> 359
acagattctt ttagaagctg gggcagatcc taatgcaact actttagaag aaacgacacc 60
attgttttta gctgttgaaa atggacagat agatgtgtta aggctgttgc ttcaacacgg 120
agcaaatgtt aatggatccc attctatgtg tggatggaac tccttgcacc aggcttcttt 180
tcaggaaaat gctgagatca taaaattgct tcttanaaaa ggagcanaca agaaatgcca 240
ggatgacttt ggaatcacac ctttatttgt ggctgctcag tatggcaagc tagaaagctt 300
gagcatactt atttcatcgg gtgcaaatgt caattgtcaa gccttggaca aagctacacc 360
cttgtcattg ctgctcaaga gggacacacc aaatgtgtgg agcttttgct ctccagtggg 420
gcagatcctg atctttactg naatgangac agttggcagt ttcccnatca tgccagnttg 480
cccaaatngg gccntncaaa aatcttggac ttggtaatnc cccttaactn accgggncct 540
gggacccttg gcttaaccaa agtnagncct tgttaattaa naaaggtttg ggggncttga 600
aaantgcttn naantnttct ccggaatggg ttcng 635

<210> 360
<211> 403
<212> DNA
<213> Homo sapiens

<220>
<221> misc_feature
<222> (1)...(403)
<223> n = A,T,C or G

<400> 360
aggtgaaagt tcaccgagtg gtgctatggg cctgtccggg tgtcgctgta tgacctggct 60
tctgtggaca gctgtgagga gaactcagtg ctggagatca ttgcctttca ttgcaagagc 120
ccgcaccgac accgaatggt cgttttggag cccctgaaca aactgctgca ggcgaaatgg 180
gatctgctca tccccaagtt cttcttaaac ttcctgtgta atctgatcta catgttcatc 240
ttcaccgctg ttgcctacca tcagcctacc ctgaagaagc aggccgccct cacctgaaag 300
cggaggttgg aaactccatg ctgctgacgg gccacatcct tatcctgcta gggggatct 360
acctcctcgt gggccaactg tggtacctng ggccggacca cgc 403

<210> 361
<211> 631
<212> DNA
<213> Homo sapiens

<220>
<221> misc_feature
<222> (1)...(631)
<223> n = A,T,C or G

<400> 361
ggtacaagct tttttttttt tttttttttt tttttttttt cgtttttaaa aactcgggtt 60
ttatncaata gaatgttttn tagcanatgc ctnttgtttt aatatattaa aattttgcaa 120
agccntttga gctactgcct tagtctaccc actgtccttt ngttatgagg tanaggatnt 180
catgacacca tacacacaaa cccatcattg cctgtgaatg cacgtagggc canaattcct 240
cagttcccgc tcctctgagg gttgatactg ctgggaatgc caaccantnc acaagcanag 300
ggaagccccn tcaggcctnc aggaggagcc gcagcagggg gtccaattna aaccagcngc 360
aaaagagcct gacattttcc catccatnta tgaggaaagc cattttacag aacntggaca 420
tagggcactt gntttcccca cacnaanggg atgggaattt tctacctata gncattcctt 480
gnacttctgg anttactcan gaccanggnc caactaaang gcaaaaccct tttggntctt 540
taaccagaaa agcantnctn nggactgggg acctncccgg gnggccnttt aaaggngaat 600
ttccnnnntt ggggcggtnt aggggaccan g 631

<210> 362
<211> 660
<212> DNA
<213> Homo sapiens

<220>
<221> misc_feature
<222> (1)...(660)
<223> n = A,T,C or G

<400> 362
ncnggtacct canttgnctg cttacgctnn anccagcatg tgtgagctag gtcatttnct 60
gcaagccagg caaccacacc agngtataan cctcaagcaa atgtnactcc naagcccnan 120
atgggactaa ggcctttgct gggctaggcg tggtgtaaan cccangcctg naagctnnta 180
cccaaccnta attagtntca ncttactntc aatatgtgca tantttcata aagcacacat 240

tnncatgagg aaaagangat ggtggtgaaa gggnaggggt gangggacat nttcaagtca 300
canaggctgn anaactcagc atgacttgtg gacggaccac aggncatnca gggnnacaac 360
acngacataa ctcaaccagt ggtnaacngn tctaaaccag ggtnaacagg agangggacc 420
aaangnaact tcctggattt ngctgcaagt ttaaaagata agttctacct tagctttaag 480
cttagnccct tatgggggca aaaaaanggn aaagtcaatt cttgccncaa atccaagctt 540
gggccngcca aaaaagggaa atnggggttn ttaggcccca aaacctnaat tgagntccca 600
aggnttcaag gcccaggcaa attgnaaagt tcctgccttn aaagcttggn ccaataaaaa 660

<210> 363
<211> 486
<212> DNA
<213> Homo sapiens

<220>
<221> misc_feature
<222> (1)...(486)
<223> n = A,T,C or G

<400> 363
ggtaccttca accttctcta ttttaatctg aggggaaatt aagagaatct caaaagttac 60
tacagagttt gggtaggcta gatacattta ttaatagtaa aagcaaccat ggcaaaagca 120
accatactca ttcttgataa tgaaaggatc ttctatatac aaacctagca aattaaaaaa 180
aaatactaaa acaaagtgtc tgaagataat gaaaggcagt tcaattcatg taatgtcaag 240
taactttcaa ttgtaataga atcatttata ttcttatagt gccttacagc atattttatc 300
gttaatgaga aaatgaacca aaactatagt gctaaccctg aaaccttaaa ccgaacctta 360
caaagttaaa gactaagtgt tggtcagaag gaaaaggatg caccatgcat cttcacaggg 420
aaaaatgaaa atagcnaaga tggcagaaat gcctgaactc atgggtacct gcccggcggc 480
cgttng 486

<210> 364
<211> 686
<212> DNA
<213> Homo sapiens

<220>
<221> misc_feature
<222> (1)...(686)
<223> n = A,T,C or G

<400> 364
   ggtgctcgga ataacttcct gcagcgacca acaggctaaa gagggggaag gtctggaggg 60
   atccagcacc ggctcctcct ccggcaacca cggtgggagc ggcggaggaa atggacataa 120
   acccgggtgt gaaaagccag ggaatgaagc ccgcgggagc ggggaatctg ggattcagaa 180
   ctctgagacg tctcctggga tgtttaactt tgacactttc tggaagaatt ttaaatccaa 240
   gctgggtttc atcaactggg atgccataaa caagaaccag gtcccgcccc ccagcacccg 300
   agccctcctc tacttcagcc gactctggga ggatttcaaa cagaacactc ctttcctcaa 360
   ctggaaagca attattgagg gtgccgaccg cgtcatcact gcagaaaccg tgcaaggcag 420
   aacccgatca gaactaccaa ttccaccagc atgccgtatt cccacttggc ttattggtgg 480
   ggaaatacct tgccngggcn ggnccgttca aangggcgna anttccagct cacttggccg 540
   gccggtactt aatggggatc cnaaactttg gnaccccana cnttggggcg nnaatncatn 600
   gggcaaaaat tggntnncnc tgggggnaaa atggtaatnc cggttcacaa nttcccccca 660
   attttctann cccggaagct taaagg 686

<210> 365
<211> 639
<212> DNA
<213> Homo sapiens

<220>
<221> misc_feature
<222> (1)...(639)
<223> n = A,T,C or G

<400> 365
ggtacatcct aaagcattct ggtacaaatg aaatggaact gcctcttgtg ggtctatttc 60
agaagtctgt tgtcagagtt cagttcacag gcatcaacca gaagcctagt gaggccgttt 120
gaaattctgg cccagattaa ttttttaaag ctgcatttgg agctttttaa agtcgagctg 180
tttccaaagg cttaactgaa gagtaactga tttcactgga aataaaagtc cacatgtgat 240
cccagctgga gtgtggtcat atttttcttg caaacctaga atgtcttggg gaacaaacgg 300
ctgtcacgtg tccccttcca aaaatgtctt aaacaccgga aaggagggca ggctaaggtg 360
tagcccttcc caccctgggt gccagggttg ggggtgctat aagtgaaata tcaaagcttg 420
aggcactaat attctgaatt tcagcctcaa agganggann gtntcnngaa tcnangaagg 480
aggggaagga cccaganacg gggaatggcc tggatgggat naatccanna cntggggnaa 540
agctggtttc ctgaataatg nggtcntggg gaccttgccc ggccggncgt tcnaaaggca 600
attccacccc atggnnggcc gttactaagg ggntccgcn 639

<210> 366
<211> 586
<212> DNA
<213> Homo sapiens

<220>
<221> misc_feature
<222> (1)...(586)
<223> n = A,T,C or G

<400> 366
cgaggtacaa aattgcagat agtggcttac tgagtttaag atcaagatca gacttaaact 60
caacaagatc accaaaggta tttctactga gttttcctat gtcccacagt aagctgggtt 120
agagagaact caaattcctg atggaaaaca aaaccgaaca aaaaaactag aaaaaaaagg 180
tgttaaaaat gctgtgtaag ttgctgcaaa aggggaaaaa gaatagacac taactccatg 240
taattttaga catgcagctt ttgtgttttt ttttgttttt gttttttttt ttttgaaaaa 300
aaccagttta ttttgagatc agtgaaaaga gtctangcca cagaaaagaa cagctcttta 360
atgcaagtta aaatgtgtaa atgaatgacc cgggacactt gacaccttta gatgcagact 420
tcattcggca ctggttggct cagacttgcc ggcngccgtt naaaggcnat tcaccnctgc 480
ggccgtctan tnggtccaac ttgtccaact gnnaanaggn tanntgtctt gggaaannnt 540
nntncattcn cnntnaccga gctaagntag cgggngnntg nggnnn 586

<210> 367
<211> 628
<212> DNA
<213> Homo sapiens

<220>
<221> misc_feature
<222> (1)...(628)

<223> n = A,T,C or G

<400> 367
gcttcctgag gagcaggcca gaacggaagt cttggtttta tttatagttg ataacttaca 60
tccggcctgc tcctcaggaa gcacagcagg gaggagacag agcccaaagg agacggcgac 120
aaaaatgccc aaacccctga gctaatgtgg tgactgagag caagcctaaa gctcccttct 180
gagctcccca gcagccaaag caaagagaga aacagggtcc tgcagcatga tgtcacagaa 240
aaccagggac cctggagcct gggttccaat aagaacctta cattctgacg ccttagattt 300
ctccctggaa aatggggaga aaaatactga attggttggg agggccatgc aacacaccca 360
gcacagtgtc tggatgcatt tcagaggccc caccagtcta gggtctacag aaagacagta 420
ccttnggccg ngaccacgct angggcgaat tccactcact ggcgggcggt tctaatggat 480
ccnacttcgg accaactttg gcgttatcat nggcataact tgnttcctgn gggaaaattg 540
gtatcccgnt tcaaattncc ccccanttct aancgaannc ttaangttta aacctggggg 600
ncaaataagn gcttacctcc tattgggn 628

<210> 368
<211> 618
<212> DNA
<213> Homo sapiens

<220>
<221> misc_feature
<222> (1)...(618)
<223> n = A,T,C or G

<400> 368
acaattcata gggacgacca atgaggacag ggaatgaacc cggctctccc ccagccctga 60
tttttgctac atatggggtc tcttttcatt ctttgcaaaa acactgggct ttctgagaac 120
acggacggtt cttagcacaa tttgtgaaat ctgtgtagaa ccgggctttg caggggagat 180
aattttcctc ctctggagga aaggtggtga ttgacaggca gggagacagt gacaaggcta 240
gagaaagcca cgctcggcct tctctgaacc aggatggaac ggcagacccc tgaaacgaag 300
cttgcccctt ccaatcagcc acttctgaga acccccatct aacttcctac tggaaaagag 360
ggccttctca ggagcagtcc aagagtttca aaagatacgt gacaactacc atctagagga 420
aaggtgcccc ttagcagaga agcccagagc ttactctggt cgtttncaga nacaactgnt 480
ggcttgcttg ggatgccccc agcctttgan aggcccttac ccattgacct tttgccatcc 540
cttgggcatt aacttnnggc cttgggnttt aancttgntt gccttnaang gncaggtttt 600
gcttaanccg gntgnggc 618

<210> 369
<211> 443
<212> DNA
<213> Homo sapiens

<220>
<221> misc_feature
<222> (1)...(443)
<223> n = A,T,C or G

<400> 369
gcagggcggg cngcggggtc ttggcgaacg gtcttcggaa gcggcggcgg cgcgatgacc 60
acgctacggg cctttacctg cgacgacctg ttccgcttca acaacattaa cttggatcca 120
cttacagaaa cttatgggat tcctttctac ctacaatacc tcgcccactg gccagagtat 180
ttcattgttg cagaggcacc tggtggagaa ttaatgggtt atattatggg taaagcagaa 240

ggctcagtag ctagggaaga atggcacggg caccgtcacg gctctgtctg ttgccccaga 300
atttcgacgc cttggtttgg ctgctaaact tatggaagtt actagaggag atttcagaaa 360
gaaagggtgg attttttgtg gatctctttg taagagtatc taaccaagtt gcaagtaaca 420
tgtaccttng gtcgcganna cgc 443

<210> 370
<211> 636
<212> DNA
<213> Homo sapiens

<220>
<221> misc_feature
<222> (1)...(636)
<223> n = A,T,C or G

<400> 370
acatttgttt atttaaagca caggaaatga ataaaatgcc acctaaaaag tatctgcaat 60
gaataaatta tttccagtga agcactgcag atccacacac accagtctgc taacctttac 120
caaggccatg tccggtgggc ttgtgcttgt tccagttgac tcttccttga gacctttccc 180
ttctgtgcaa tgaccacagc attagagacc agtcctgcat gcgctggcct tcctcgtagg 240
catggcagac cacgtggatg agcagtgggc tggcatgcag taggcttnaa caaatggcac 300
ttcactgttt ccagtgaccc tgaaatgttt tacgtaagtg gggcctgggc tttaaagaaa 360
agagccaggg ttcctcaagc tgggcccctt tacttgaggc cagcttcagg aaatactggn 420
cttaaggagc cagcaacttg tccaggagtt ttgagccctt antttgaagg aaaatggccc 480
cttggngtcc ntgcaagcac cagnnatttc cgtgatngtg ancaagtnac cnnccttaag 540
ggaaggccaa tcccnctttg ggnggantcn agggcnctan tcctgtttgg aagggcttga 600
aggttgggaa tntttaaaat ggaggnntng gcttcc 636

<210> 371
<211> 615
<212> DNA
<213> Homo sapiens

<220>
<221> misc_feature
<222> (1)...(615)
<223> n = A,T,C or G

<400> 371
ggtacaagct tttttttttt tttttttttt tttttttttc tgttaaagaa tgctttatta 60
atacaaatac acacaaactc tgaagcacta anaaatttaa atatctatgt cacagcaaac 120
aggtggcaat tcaacatcca gggtcgacag aatgcttgaa gganactgca acagattgga 180
ttcccatggt gganagggca tnttcacagg tgaagggggg cccagctgaa acagcttttc 240
aagctctctc tcctcgtcaa ggatcatgag aggcactcca ctcaagggga ggtgcgcaat 300
ctggtgctct tcaggcaggt caaaactctc aaagtctaga ggattgaagg gaaagaattt 360
ttctatttct ggataggcat catctgaggc aggaacagag ctttttgctt taacagtctt 420
ctcagtcatc ttttttggca aaaaagcttg gctggttttg tttgangggg tccttgggct 480
ttacagactt ttctgnaact ctgttgacca gnttcccaaa gcctttttta gtaactttta 540
ggtaaggctt ntgggggcat taaacctttt tccaaacctg gggttgaaac ttggaaccnc 600
ctttaagggt ttgnt 615

<210> 372
<211> 612

<212> DNA
<213> Homo sapiens

<220>
<221> misc_feature
<222> (1)...(612)
<223> n = A,T,C or G

<400> 372
actttttttt tgttctagga atgagggtag gataaatctc agaggtctgt gtgatttact 60
caagttgaag acaacctcca ggccattcct ggtcaacgtt ttaagtagca tttccagcat 120
tcacacttga tactgcacat cangagttgt gtcacctttc ctgggtgatt tgggttttct 180
ccattcaagg agcttgtagc tctgagctat gatgctttta ttgggaggaa aggaggcagc 240
tgcagaattg atgtgagcta tgtggggccg aangtctcag cccgcagcta agtctctacc 300
taagaaaatg cctctgggca ttcttttgaa agtatagtgt ctgagctnat gctanaaaga 360
atcaaaaagc nagtgtggat ttttagactg naattaaatg aggcnaaang atttctattc 420
ccagtgggaa agaanacctt tctactgaag ttgtgggggg antatgttng aatgttagag 480
agaaccctta aggnntnctt tgattggccc ttggagaccg nttggannac atnncccgga 540
attnnantan aaattntttc nggnttnaag tttccccntg tngtngnann ccaacctngt 600
ttttgccccc cc 612

<210> 373
<211> 638
<212> DNA
<213> Homo sapiens

<220>
<221> misc_feature
<222> (1)...(638)
<223> n = A,T,C or G

<400> 373
ggtactcagt atttcaaatc atgaacacaa gattggaact tttggaaaaa tgggttcaag 60
ctttcctatt agccatggaa atgcaaagtt tagcagaagc aagcaattag gcagagaaca 120
aaaatgttaa gcatggtgtt gtctatctta ttgaagtggt tggaaatgaa agcttttaat 180
ttgatagatt tatcagtata aaattaggga aaccacgtgt ggggaatgaa tcaatttaga 240
gcttcgggaa ttgtgaggtg acttttgtaa cttttgttct gtgtgtgacc tgtgaaccac 300
tagatgtgat ctgcccttgt gggcaggtcc agcatagtta ggagttaggc tttancataa 360
aattctagct gcatctgagt ctcctgggat gggtgctctt tggctngttt tggcctgccn 420
gattggtgag atccagancc agctttttcc tgctgcttgg cccctnncaa ttaatttgtt 480
gggattgcca gtgcnagaan accttagttg taaagaattt taatcctacc ncgaccnagt 540
tccaaaangc ngggttttga atgtgggaan tttnnnaatt ttcccttana aagtctaaat 600
tttgtccngt tanactnttg gttttaaagg gaagggaa 638

<210> 374
<211> 503
<212> DNA
<213> Homo sapiens

<220>
<221> misc_feature
<222> (1)...(503)
<223> n = A,T,C or G

<400> 374
ggtacagatt aacttaacac aaaaacccga acttcaaaat gaaggtgtgt ggaggaaagg 60
tgctgctggg tctccctaca actgttcatt tctttgtgag gcagggggta gttcctgaat 120
ggctgtggtc caatgactaa tgtaaaacaa aaacagaaac aaaaaaaaca aggaactgtc 180
atttccacga aagcacagcg gcagtgattc tagcaggcct cagggccctg ggcctgggga 240
ggctacatga gggggagcct cagtcacagg atcaacctgg ggcccgaagg agcagggttc 300
cctgcctctc cctctgcaac agatcatccc atccaacaca acccccaaaa tgttgatgat 360
gacgcaacat ggtcaaccct caagaccttt aagacaaaac agagcagcat aggaaaaaaa 420
aaacaaaacg caccaatttc tgcatgtgtc aatggtaggg caccntttta aaaaagtctg 480
tctaaaacan nctntgttta ctt 503

<210> 375
<211> 611
<212> DNA
<213> Homo sapiens

<220>
<221> misc_feature
<222> (1)...(611)
<223> n = A,T,C or G

<400> 375
ggtacaaaag ctgttgaact taatcccaaa tatgtgaaag ctctctttag acgtgcaaaa 60
gcccatgaga agctagacaa taagaaggaa tgtttagaag atgtcactgc tgtgtgtata 120
ttagaagggt tccaaaatca acaaagcatg ctgttagccg ataaagttct taaactcctt 180
ggaaaagaga aagccaaaga aaaatataag aatcgtgaac ctctgatgcc atctccacag 240
tttatcaaat cttacttcag ttctttcacg gatgatatca tttcccagcc catgcttaaa 300
ggagagaaat ctgatgaaga taaagacaag gaaggggagg ctttagaagt gaaagaaaat 360
tctggatact taaaggccaa acagttatgg aagaagaaaa ctacgatana atcataagtg 420
aatgcccana aaaaaaaatn atttaaaaaa aagcttgtcc ctgccggccg gccgttcnaa 480
agggcgaatt canctccctg gngggcggta ctannnggat ccaacnttgg gccaaccttg 540
gngnaaacan nggntatant gtttcctggg naaatggtnt ccngttncaa tccccnaatn 600
ntngngccgg g 611

<210> 376
<211> 601
<212> DNA
<213> Homo sapiens

<220>
<221> misc_feature
<222> (1)...(601)
<223> n = A,T,C or G

<400> 376
cgaggtcttt tctctctttc tgtcttcatc ccagatcaaa gaatcccgag ttaggatctg 60
gatgaaggat aagcccctga attgtcgatg ggctcacccc cacactgacc cagcatctga 120
acttgcttaa cagggagccg gggctaaact gcttcaccct gcctgagaac cagggagcac 180
tgcatttctc cacagggtgg aggagaagag gcagaataaa ccaagcctgg gacacctccc 240
tcctgtctag gtgtacagca cacaggttaa tactcttcac cctcatcctc tccgtcagca 300
ctatctgctc caacctcctc ataatccttc tcaagggcag ccatgtcctc acgggcctct 360
gaaaactcgc ctggaccaca aagtttgacc tgatgtatgc caagccgtgc ctttggtcac 420

tggnacctgg ccnggccggc cgttcaangg cgaattccac acactggcng gccgtactan 480
tggatccnaa ctnggaccag cttgngtaat catggcatnc tggttcctgg ggnaaatggt 540
atccgttaca attccnccan ntcnanccgg aacctaaagg gtaaacctgg ggngctaatn 600
a 601

<210> 377
<211> 621
<212> DNA
<213> Homo sapiens

<220>
<221> misc_feature
<222> (1)...(621)
<223> n = A,T,C or G

<400> 377
   ggtacaagct tttttttttt tttttttttt tttttttttg tctgttcaag aaccagtctg 60
   ggatcttgta cccagctcta attactggcc gtagcagcat attgcttaan aattttgtag 120
   aacttatttc tcatcagcag ctgtccaaag gactgataaa tagagacaga tcccagtcct 180
   ggatactttc tgtaaatcct aatcggagac tcacttntna gcaatggagg ctgaaagtct 240
   tagtgagact cagtaaattc cttnaggcct tggcagatgg atccagtagg ttgagagaaa 300
   gtgaaggact tcaggaacag aaagaaaatc cccatgccac tagcaactcc atttttatna 360
   actggaagga acatgccaac gaccagcaac acatccaggg tttatgaaaa tgggggttca 420
   cagncnaaat gtcngntcca agttcaggct ncnggatttt ggtttggagg actgaatggt 480
   gtggattaaa ggcttncatt ttcttgnaac cttgaaaggg tttttnggan aanaattcnt 540
   tgntaatgna agctnggttt aaacttgacc tngcccgggn gggccnttca aaagggcgna 600
   ttnccgcncn ttggggggcc g 621

<210> 378
<211> 327
<212> DNA
<213> Homo sapiens

<220>
<221> misc_feature
<222> (1)...(327)
<223> n = A,T,C or G

<400> 378
acatctccga cagtatctgt ttcagcatct ttgcncttct gaagtctttn atacttgtgg 60
caaaagttcc tgaaactggc ctccangtgt ccctccacct gtgctggcac ttgggcgttt 120
ccacnaaact tcccaaacag ctcacaatcc tggctgactg ggacaataat tcagcaaact 180
ggctactcag acctggcacc aaatgtcctg tccaaaatgc tgttcactga accagtgctg 240
ggcgcccctg ggcagggtgg ctcgatcacc cgccacatnc acttggccgc cagaagccng 300
nggggaagga cctnggcgcg acnacgc 327

<210> 379
<211> 517
<212> DNA
<213> Homo sapiens

<220>
<221> misc_feature

<222> (1)...(517)
<223> n = A,T,C or G

<400> 379
actcacaagt aagaaacttt ctctactgaa ggatactgtc acagagtttg ttgcagagca 60
tctatatata tatttattna tttattttaa aaaantaaac aacantgatg aacganccca 120
ggttcctaga accaattctc ttgattctct acttccacaa aataaagtgt atcatttggc 180
caagactaca gatgtgtttt tnttttttca canatgcaag tgccatgcaa aaataaatta 240
aagaacagat accaaaacat acatgtgata aaactacana tggtagattt ttaaaggcat 300
ttatataaac ntaatttata aatacttctc tttntgcctt tatatacagt cncaaanctg 360
gntgttatac atntaggatt tcctntgcnt gaccttnggc cgtnacnacg nntaagggcc 420
gaattctgga agattccatc tacaattggc ggctcgtttn tancatncct ttntanggcc 480
caatttngnc cnntannnga gtcngattac aanntcn 517

<210> 380
<211> 351
<212> DNA
<213> Homo sapiens

<400> 380
acgctgtgga gggctgcagt gctcgtggat tcaaaatcac agagggctgg taaatggcag 60
cttctgtagg aataactgca gcaggagctg gaaatgtgta ggagggagga gacaggcatg 120
gtaacttaca tggcggtggg gataagccat ttcgatttaa agtgcccccc attaacacaa 180
agttcatctc ctcagctgaa cactgaaaga cttcaacata tctgtccttc atgttttttt 240
atgacacttc tgtgcagcca taaatgctct gtccgcagac ttcatctgga taaaggcatc 300
tcctgatggg cggccctggt gattcaaaac catgtgaacc ccatgagtac c 351

<210> 381
<211> 622
<212> DNA
<213> Homo sapiens

<220>
<221> misc_feature
<222> (1)...(622)
<223> n = A,T,C or G

<400> 381
acacttccaa ttgtccatat aattaagctt tccacaatct tacacaccca tcatctcctg 60
aagatgctag caccgttcct gttatattcc aactcactcg ccagacctga gaattatgat 120
tatcgaactg agccactata tggatttcaa actttgttgg cccaccagag gaagtcagtt 180
ctttcctcac aggctttaat gtaaaaattc tcacatcttt ggtcgctatt gctagaatat 240
ggaaagatct tcccaaattt ggagcgaatg caatatcatg aacaggatca gtgactgtca 300
taagagtttc agcttttgca tatttcctgg tgttttcatt atattcaaaa atctgaacct 360
tggccattgc gttggggcta ctgncatcac tttctacggc gatcatgggg gaatgagcac 420
gagagctttg naggggtncc aagaaatnca cttccagctt agcttacttg aganctctgg 480
ctggnaaaga cccctnggct gagaattcnt aaccatctgg ggccctcaaa nantcttacc 540
tttccattng nggacaaggt ggttacttag aaccccnggn cttgggacca acttnccntt 600
cggtnncana gttttggtnt cc 622

<210> 382
<211> 509
<212> DNA

<213> Homo sapiens

<220>
<221> misc_feature
<222> (1)...(509)
<223> n = A,T,C or G

<400> 382
ggtactctca tcccgccccc attcaggctg atagtaacag cctaggtaga gtcaacacat 60
aaaaaagtgt aattccaggg gaggaggatt agaataagga cacaaaggaa gggaggaaaa 120
tgttctttga ggctgaaatt ccattaattt ttcatagtat tgagtttata tttgccattg 180
catccttcaa tctttctaaa aaggaaatcc ccggaacata ataaaatctc ttctgtatag 240
aaaagctaca gctccacact aagaggaatg ccgtctgcct taaagaatgg aatcatcagt 300
gaccaagaat tacttccaag gagaaattca ttgatattaa aaccaaagcc agatccagct 360
cagcaaaccg acagccagaa cagtgatacg gagcagtatt ttagagaatg gtttccaaac 420
ccgccaacct gcacggtgtt atttctgcca cgtgtctctg gaacacacat taaactgtgg 480
aaactnnctn ctttccgctg ggggtcccc 509

<210> 383
<211> 380
<212> DNA
<213> Homo sapiens

<400> 383
acaattccac ttatccatac tattccttta taaaaggcag atttcaggta agcttctaaa 60
tgcatgcgta atgtagaggc taatattttc tggcagtcct tggttcctga aatttgaact 120
tcatatgtgt tttaaacttt tgtcaaaata gtcatgaaag atatgttatt tttgcataat 180
gaggtaatat atcaggggcg ggcactcata agacagtata aatccacttg tctaaacttg 240
catgaggctg tgtgcattgt aaaatgccat aaagagtttt gggtcaagtg aatattttgc 300
tgaaggaata acacttacat ttaactgagc acttttctgt aataaatacc aaagtaggtt 360
tttgtagctg taaactgtgt 380

<210> 384
<211> 317
<212> DNA
<213> Homo sapiens

<400> 384
ggtcccagac ccaagaccaa ccgatggagg aggaggaggt tgagacgttc gcctttcagg 60
cagaaattgc ccagttgatg tcattgatca tcaatacttt ctactcgaac aaagagatct 120
ttctgagaga gctcatttca aattcatcag atgcattgga caaaatccgg tatgaaagct 180
tgacagatcc cagtaaatta gactctggga aagagctgta tattaacctt ataccgaaca 240
aacaagatcg aactctcact attgtggata ctggaattgg aaatgaccaa ggctgacttg 300
gatcaataac ccttggt 317

<210> 385
<211> 275
<212> DNA
<213> Homo sapiens

<400> 385
acttttagtc cctgttttac aggggttaga atagactgtt aaggggcaac tgagaaagaa 60
cagagaagtg acagctaggg gttgagaggg gccagaaaaa catgaatgca ggcagatttc 120

gtgaaatctg ccaccacttt ataaccagat ggttcctttc acaaccctgg gtcaaaaaga 180
gaataatttg gcctataatg ttaaaagaaa gcaggaaggt gggtaaataa aaatcttggt 240
gcctggaaaa aaaaaaaaaa aaaaaaaaag ctgta 275

<210> 386
<211> 606
<212> DNA
<213> Homo sapiens

<220>
<221> misc_feature
<222> (1)...(606)
<223> n = A,T,C or G

<400> 386
ggtacatgga tattcccaaa ccattccatt agaaaactgc cctccctgca cacacaacaa 60
aaacagcgct atttcctaca cctattggac tgaaagtgct tggaaatgga atggttttag 120
aatatgaaga agaacacaaa ccaagtagct gtgggttgaa cctggacgtg agctggctgc 180
agggccgttg ggtagaaaac cagcatctca taaacaggtc actacaaaaa taggaagagt 240
ataaaaatag aatatattat gtcactattt cgtcttctct ttatagtagc gtatcgtagg 300
agtgggacag gtggcctttc ccgaccctgc tacgctggct ggtgcccgac aaacctccac 360
tggatggttt gtcactggat ggtttgttgg ggtggtggtc acaggcgcaa aggacatgca 420
cacgggcacg ctcgctactg naacccagan gtgacttcag cntgaataaa ggngaaaagg 480
tccccatnta nctcnggaat tattncctnc ccaggnccta ttaaggggct ttntggcttt 540
tnaccancca agncccnccc cttgaaangc caaacttttt tgaaaaaaag gganccttgn 600
atngnc 606

<210> 387
<211> 339
<212> DNA
<213> Homo sapiens

<400> 387
accacttgca gtcaaatgaa ttccttcgaa atgtatttga acttggaccc ccagtgatgc 60
ttgatgctgc aacgcttaaa acgatgaaga tttctcgttt cgaaaggcat ttatataact 120
ctgcagcctt caaagctcga accaaagcta gaagcaaatg tcgagataag agagcagatg 180
ttggagaatt cttctagatt ttcagaactt gaagactatt ttctaatttc tatttttttt 240
tctatttcaa tgtatttaaa ctctagacac agtttttatc ctggattaac ttagataact 300
tttgtagcag tggttatatt gcttataatt taatgtacc 339

<210> 388
<211> 667
<212> DNA
<213> Homo sapiens

<220>
<221> misc_feature
<222> (1)...(667)
<223> n = A,T,C or G

<400> 388
taccagttgt catcatagcc ggagatggac acttcaggag ggtagcgtac attcccatga 60
caccaatact acagttttcg gagtcacagt aagatacaca gaattacatc cgtaattaat 120

atgaatgcca acatgtcaag cagtaatttg ttacatggca aacaaaatca agaaagcaac 180
catcaaacaa aagagaccca tagcttcaga caaggcaaat cccaggatag catatgagaa 240
cagctgctgc ttcagcgaag ggtttctggc ataaccaatg ataaggctgc caaagactgt 300
tccaatacca gcaccagaac cagccactcc tactgttgca gcacctgcac caataaattt 360
ggcagcagta tcaatgtctc tgctgattgc actggtctga aactcccttt ggattagctg 420
agacacacca ttctgggccc cattaaatac cgtagagccc tctccagtcc tactagcctc 480
tggtcgagat aacactgatg cagaaattgg tctgtatgca actctggatc cagctcggat 540
cagagagggg gtgcaggcga gcttggcgca ggcgaacatc ttacactctt cgggactgcg 600
cggctggaga tattgggtga caggcgacgt gggctcctct cccgcttnct ctctttccag 660
gaagcgg 667

<210> 389
<211> 613
<212> DNA
<213> Homo sapiens

<220>
<221> misc_feature
<222> (1)...(613)
<223> n = A,T,C or G

<400> 389
ggtaccagtt gtcatcatag ccggagatgg acacttcagg agggtagcgt acattcccat 60
gacaccaata ctacagtttt cggagtcaca gtaagataca cagaattaca tccgtaatta 120
atatgaatgc caacatgtca agcagtaatt tgttacatgg caaacaaaat caagaaagca 180
accatcaaac aaaagagacc catagcttca gacaaggcaa atcccaggat agcatatgag 240
aacagctgct gcttcagcga agggtttctg gcataaccaa tgataaggct gccaaagact 300
gttccaatac cagcaccaga accagccact cctactgttg cagcacctgc accaataaat 360
ttggcagcag tatcaatgtc tctgctgatt gcactggtct gaaactccct ttggattagc 420
tgagacacac cattctgggc cccattaaaa taccgnagag ccttttcagt cctactagcc 480
tctggncgag ataacactga tgcanaaatg gnctgtatgc caactctgga tccacttcgg 540
ttcaaaaagg ggtgcaggca acttggccca ngcgaacatn tacacttttc gggactgccc 600
gnttggnnaa tgg 613

<210> 390
<211> 278
<212> DNA
<213> Homo sapiens

<220>
<221> misc_feature
<222> (1)...(278)
<223> n = A,T,C or G

<400> 390
actagtcctc tagaaatagg ttaaactgaa gcaacttgat ggaaggatct ctccacaggg 60
cttgttttcc aaagaaaagt attgnttgga ggagcaaagt taaaagccta cctaagcata 120
tcgtaaagct gttcaaaaat aactcagacc cagtcttgng gatggaaatg tagtgctcga 180
gtcacattct gcttaaagtt gtaacaaata cngatgagtt aaaaanannt cttttnttga 240
actctnanga aaancttgga ccttngccgn gaccacgc 278

<210> 391
<211> 604

<212> DNA
<213> Homo sapiens

<220>
<221> misc_feature
<222> (1)...(604)
<223> n = A,T,C or G

<400> 391
ggtctttttt tttttttttt tttttttgaa cacagatcac tttattggca tggctttgtt 60
ttaagaaaag gaaaagtgac aaagccaaga gacagactnt gctaacagat gcctgggggt 120
ggctggacat ttttgcctca tgctgtgcaa agagggggat cctggcccac acatcctgct 180
gattccttgg gacaaggttg tctgcctggg cctcactgca ccttcttgaa tacttgcttg 240
canaccacac cttccactct natctncagg tgcagctcat caccctngat ccactgggtc 300
cagccacgcc ccttcttctc acccttctga cacactggag cttgctccgt cccagtcact 360
gtgtcatgca cttgcggnca tctatgcctg nagatcctcc taaactcctt tccaacctgg 420
aagtccatga tgnantncct aaaagngctc accgtggcgg angatcatat ggtcancggc 480
ntgaacgaan tnttttggcg ggnttcanna agttgcccat ttttgcgcaa gggcccattg 540
gncgtnnagg gcccangtnc tttgcngnnc ccctnagggn gaatccccac nttggggccg 600
tntn 604

<210> 392
<211> 610
<212> DNA
<213> Homo sapiens

<220>
<221> misc_feature
<222> (1)...(610)
<223> n = A,T,C or G

<400> 392
acgaggggag cgagacgaaa ggagaacggt gattattcat gacaggcctg atatcactca 60
tcctagacat cctcgagagg cagggcccaa tccttccaga cccaccagct ggaaaagtga 120
aggaagcatg tccactgaca aacgggaaac aagagttgaa aggccagaac gatctgggag 180
agaagtatca gggcacagtg tgagaggcgc tccccctggg aatcgtagca gcgcttcggg 240
gtacttattg gcacaaattc gggcagcctc cagggcttca gaggacagct gctcatattc 300
atctgacacc atgtggccac aaagcggaaa ctcatccact tttgcctttt tccgccccag 360
gtcaaaaatg cgaatcttgg catcagggac acctcggcag aagcgagact ttgggtgagc 420
ttgttttcca tctagggatg atgggagaca gtgacaaatc atccaccatt agatttttat 480
aaggagcgca caacccagac aacccaaatc cctttggatg tgccagttca caatagtggt 540
catgcctcca ttgagaatat aatggctctn gacttgccgg aaggcaaact taaggccata 600
atgggaccng 610

<210> 393
<211> 314
<212> DNA
<213> Homo sapiens

<400> 393
ggtcccagac ccaagaccaa ccgatggagg aggaggaggt tgagacgttc gcctttcagg 60
cagaaattgc ccagttgatg tcattgatca tcaatacttt ctactcgaac aaagagatct 120
ttctgagaga gctcatttca aattcatcag atgcattgga caaaatccgg tatgaaagct 180

tgacagatcc cagtaaatta gactctggga aagagctgta tattaacctt ataccgaaca 240
aacaagatcg aactctcact attgtggata ctggaattgg aatgaccaag gctgacttga 300
tcaataacct tggt 314

<210> 394
<211> 498
<212> DNA
<213> Homo sapiens

<220>
<221> misc_feature
<222> (1)...(498)
<223> n = A,T,C or G

<400> 394
accagacctg tcaacgtcna tttctcggna aatttnttgg tatttttgaa tctncgtcca 60
gagaatgtaa aactccttca gncccagctt gccactcccg tccgaatcta gcatgtcaac 120
cataatttng aatcttcgtc cagagaatgt agaactcctt cagccccagc ttgccactcc 180
cgtccgaatc tagcatgtca accataattt tgcatgnctc gatgctgaag ccatctgact 240
ggatatcttg gcgctttgct agaacccttc tcaggatggt ctgcngctca aaggcanaga 300
tctccgnatc ctctcctgcc aactgggcaa acagnctcct gaatccatca tcaatgtcat 360
cctcgctgat gtcgaactct tcaagattgg cctcgatttc atcatcgaca gcttggtagt 420
cagctttctt ttcagaaaag acccggatgc agaaatcccc atccttgntg ggttcgaagg 480
tggaaggcac ganaatgt 498

<210> 395
<211> 629
<212> DNA
<213> Homo sapiens

<220>
<221> misc_feature
<222> (1)...(629)
<223> n = A,T,C or G

<400> 395
gccgcccgtc aagctgtcca catccctggc ctcagcccgc cacatcaccc tgacctgctt 60
acgcccagat tttcttcaat cacatctgaa taaatcactt gaagaaagct tatagcttca 120
ttgcaccatg tgtggcattt gggcgctgtt tggcagtgat gattgccttt ctgctcagtg 180
tctgagtgct atgaagattg cacacagagg tccagatgca ttccgttttg agaatgtcaa 240
tggatacacc aactgctgct ttggatttca ccggttggcg gtagttgacc cgctgtttgg 300
aatgcagcca attcgagtga agaaatatcc gtatttgtgg ctctgttaca atggtgaaat 360
ctacaaccat aagaagatgc aacagcattt tgaatttgaa taccagacca aagtggatgg 420
tgagataatc cttcatcttt atgaccaang gaggaattga gccaaccatt tgnatggttg 480
gatgggtgtg gttgcaattn ggtttactgg ggaaactggc cattangaaa agggntcctg 540
ggtaaaagaa tccctatggg ggccnnaacc tttgnttnaa agccntngcc ccaaaaangg 600
gnttttggg cggnatgttt cnaaaaacn 629

<210> 396
<211> 614
<212> DNA
<213> Homo sapiens

<220>
<221> misc_feature
<222> (1)...(614)
<223> n = A,T,C or G

<400> 396
ggtacttggg cttctttcag ctgcttcaac agagtggcag caaccaagct ggagtccaag 60
ccccctgata aaaggcagcc aatccttctg tctgtcatca aacgtttctt tacagcatta 120
ttaaaaagga tcctgaggtt gttcttcaca gtttctatct caaaacctgg aaagagtttc 180
tccacattgt catagagggc gtgcaggggt tcatcccgac agtgatgata tttaaccatt 240
tccacggatg caactttgcc atttggcttt aaatccaaaa cttcatagtg tccaggaaga 300
aaaggctcca cttttaaaaa gggagtcgcg gagtgcttca atgtaacaag acctttaact 360
tctgaacata cagccaaaaa tcatctttct gncattgctt taaaccaang tctgactcca 420
tatggtatct cttacccagg aacccntttc ttaatgggca ggtantccag ttaaaaccaa 480
atggcaaacc ccanccantc caaccnttcc naaatggntt gggttnaaat nccttccttt 540
gggcataaaa gaattnaang ggnttnnttt tancctttcc ccttttgggc ccggggattt 600
cnaaaattcn aaaa 614

<210> 397
<211> 588
<212> DNA
<213> Homo sapiens

<220>
<221> misc_feature
<222> (1)...(588)
<223> n = A,T,C or G

<400> 397
acctgggcat aggaaggaac caggacaggg ctggggacag aaggtggtca cagtcatggt 60
ttcactctca gaaatatcct gggcctatgg cttaaggctt cgtggagcag ggagtggacc 120
ttgtggttat ttacaaggct gggccatata aaagcattgc aaacatggag tggagaggat 180
ccttggagat gagctggttc aatcactcct ctgaccaaca aggaaacaaa ggcccagaga 240
ggagaaggca gtgcctggcc agacgtggga cctgaaccca gccagggctc tgactcccag 300
tcccccagtc ccctctctac ctccttgctt ggctgagtct ttttttgata aaggccccag 360
acagcctctc cgacagtctc aggtcaggct ggggttataa atggagcagt ggactcagag 420
tcagaggccc agactctgnt cttgggcctt nacattacca agncttgcta ataaccacga 480
ggccctggtg tggaggggct gctctctttt aagctcagct cntatctgga acaggccaca 540
aagttncatg ggataanggn tgaggccnna gcccacagng tggaggnc 588

<210> 398
<211> 348
<212> DNA
<213> Homo sapiens

<400> 398
ggtactagcc ggacttggat tttctggaaa gatttcagtt gaggaacggg aacaaagatt 60
atgatagctt tccgaccacc accaacttca atttccttag ctgccgtaat attcagctcc 120
ctgagctgag ccttgaggtc cgagttcatc tccagctcca gaagagcttg ggagatgccg 180
gactcgaact cgtccggctt ctcgccattg ggcttcacga tcttggcgct cgaactgaac 240
atggctttct cctgggagaa cttgccgagc gccggcttag gaagagaccc aaatctcgcg 300
agagcacgtc aaaatccggc gtccgaaggc aagaggcgga aacagcgc 348

<210> 399
<211> 630
<212> DNA
<213> Homo sapiens

<220>
<221> misc_feature
<222> (1)...(630)
<223> n = A,T,C or G

<400> 399
   acatccaagt ttaaaattat cagcgaaatg gtccatgttt ttccaattac ctgctgacac 60
   ggttctaagc taagtgaagg ggaagatctg agagcgtgct gtttgtggct gttgatgcat 120
   attcgtgatg taacaggtcc tggggcctca ctttacccca tttgtaaaat ggggctaatg 180
   tcacctgcct cttacctacc tcagagggat ttggtgaagc aaactgttaa tcttcgaaaa 240
   cgaccatttc acttcttgga tatcaagtgc taacccagta tgttcttctt ttttatgtaa 300
   gggacagctt tctccacaga gtcctttctg ctggtgagga cagcatttct gagcagggct 360
   ttgttctcta tgtgcattag gacttttatc atgcccttgg tctatgtgta gttacttgac 420
   agcatcaaat gccggctctt cctaatgncc ttcaaggttt catgaactaa caaccccacc 480
   tttcancatg ggtctggccc ctgaatttgc tgngacttcc agaccacact ggttctacca 540
   cctgaacagg ccnttaaagt tcccaanggt cancttcctt aattccttgg ttcccggtgt 600
   atggggaact tggcctanaa aagggccncc 630

<210> 400
<211> 619
<212> DNA
<213> Homo sapiens

<220>
<221> misc_feature
<222> (1)...(619)
<223> n = A,T,C or G

<400> 400
actgaacagg taagtcatcc ctcagccaga gattagtcta cttcttccat gcgtgatgtg 60
tcgtcatctc cttcaagggg tggcatttct tcagttacag cagcactggt atcatcagca 120
gtagggtcat cttcatcaat acccagacca agtttgatca tcctgtagat cctgttagca 180
tgtgtctggg gatcttccag actgaagcca gaagacagga gcgcagtttc ataaagcaag 240
atgaccagat ccttcacaga cttgtcgttc ttatcagcct ctgccttttg ccttaaggtc 300
tcaataatgg aatggtcagg gtttatctcc aggtgtttct ttgctgccat gtaacccatt 360
gntgagttgc tcttagggct tgagctttca tgattcgctc catgnttgct gccagccata 420
tgtgcttgtg acaatacagn atggagatgc accaatcggt tggacaaacc acctttcact 480
ttttcttcca tangctttca gatttgcaaa gttctaaact ttgggttttc ccttctgntc 540
ttttcctttt atctttggaa gtccaggctt nttggggacg ncctaagctt ccctnaatct 600
ttagtgtgga nnagncntn 619

<210> 401
<211> 663
<212> DNA
<213> Homo sapiens

<220>
<221> misc_feature

<222> (1)...(663)
<223> n = A,T,C or G

<400> 401
cgaggtactt gggcttcttt cagctgcttc aacagagtgg cagcaaccaa gctggagtcc 60
aagccccctg ataaaaggca gccaatcctt ctgtctgtca tcaaacgttt ctttacagca 120
ttattaaaaa ggatcctgag gttgttcttc acagtttcta tctcaaaacc tggaaagagt 180
ttctccacat tgtcatagag ggcgtgcagg ggttcatccc gacagtgatg atatttaacc 240
atttccacgg atgcaacttt gccatttggc tttaaatcca aaacttcata gtgtccagga 300
agaaaaggct ccacttttaa aaagggagtc gcggagtgct tcaatgtaac aagaccttta 360
gcttctgaac atacagccaa aaatccatct tctgcattgc tttaaacaaa ggtctgactc 420
catatgtatc tctacccagg aacactttct taatggcagt attcagtaaa accaatgcca 480
acccaccatt ccacatacca aatgggttgc tcaaatcctc cttggcataa agatgaaagg 540
ttatttnacc atncactttg gccgggattc aaattccaaa agccggtgca tttttntaan 600
ggtgganaat tnncccttgn accnaanccc caaatccggg attttnttnc ctcnaatngn 660
tgg 663

<210> 402
<211> 673
<212> DNA
<213> Homo sapiens

<220>
<221> misc_feature
<222> (1)...(673)
<223> n = A,T,C or G

<400> 402
ggtacgtgtc cagctctgaa gggcaaagtg cagaagatcc taatctggaa gtggggtcag 60
ccaccatctc ccacaccagt gcctcggcct ccagatgctg atcccaacac gccctcccca 120
aagcccttgg aggggcggcc agagcggcag ttctttgtga aatggcaagg catgtcttac 180
tggcactgct cctgggtttc tgaactgcag ctggagctgc actgtcaggt gatgttccga 240
aactatcagc ggaagaatga tatggatgag ccaccttctg gggactttgg tggtgatgaa 300
gagaaaagcc gaaagcgaaa gaacaaggac cctaaatttg cagagatgga ggaacgcttc 360
tatcgctatg gggataaacc cgagtggatg atgatcaccg aatcctnaac cacagtgtgg 420
accagaaggg ccacgttcca ctacttggat ccaagtggcn ggacttaccc ttacgaatca 480
nggcnttttt ggaanaatga aggttttnga aaatccagga ataccnacct ggtcaagcng 540
anctttttgg naatcccnng ggagttnatt gaaggggtaa aggaaggcnn nacccagcca 600
agaaagcttt aagaaagggg naactttcgg aaattggaaa aggccttcan aacnccaacg 660
gttgttccac ngg 673

<210> 403
<211> 616
<212> DNA
<213> Homo sapiens

<220>
<221> misc_feature
<222> (1)...(616)
<223> n = A,T,C or G

<400> 403
ggtaccgatt atatcatctc agtcttgaat ttactcacgc tgattgttga acagataaat 60

acgaaactgc catcatcatt tgtagaaaaa ctgtttatac catcatctaa actactattc 120
ttgcgttatc ataaagaaaa agaggttgtt gctgtagccc atgctgttta tcaagcaatg 180
ctcagcttga agaatattcc tgttttggag actgcctata agttaatatt gggagaaatg 240
acttgtgccc taaacaacct cctgcacagt ctgcaacttc ctgaggcctg ttctgaaata 300
aaacatgagg cttttaagaa tcatgtgttc aatgtagaca atgcaaaatt tgtagttaaa 360
tttgacctca gtgccctgac tacaattgga aatgccaaaa actcgagtct ttaattgtaa 420
tggctttggt ttatccacag ttaggccctt tctcaataca tatttatgna tttcactggg 480
catggcaaca tggctggaaa aatcactgga tgtaaccaaa caggcctttt ttaanaaatg 540
ncncggntta accaaanaaa aaaaaaaaaa anaaagnttt gaccttcccg ggngggcctt 600
taaaaggnna attccn 616

<210> 404
<211> 613
<212> DNA
<213> Homo sapiens

<220>
<221> misc_feature
<222> (1)...(613)
<223> n = A,T,C or G

<400> 404
cagtgctggg cctaaaggag ataacattta tgaatggaga tcaactatac ttggtccacc 60
gggttctgta tatgaaggtg gtgtgttttt tctggatatc acattttcat cagattatcc 120
atttaagcca ccaaaggtta ctttccgcac cagaatctat cactgcaaca tcaacagtca 180
gggagtcatc tgtctggaca tccttaaaga caactggagt cccgctttga ctatttcaaa 240
ggttttgctg tctatttgtt cccttttgac agactgcaac cctgcggatc ctctggttgg 300
aagcatagcc actcagtatt tgaccaacag agcagaacac gacaggatag ccagacagtg 360
gaccaagaga tacgcaacat aattcacata atttgtatgc agtgtgaang agcagaaggc 420
atcttctcac tgggctgcaa atcnttatag cctttacaat ccggactttg gggaaatggt 480
atacctggat ctactctgnn tttanacctt tgggacntng gaaanntccc caaaanggga 540
aaggctttca aangtaaact ttgaacctga aaataagttt gttnaaacnc ctattgcaag 600
tttgtttttn gga 613

<210> 405
<211> 605
<212> DNA
<213> Homo sapiens

<220>
<221> misc_feature
<222> (1)...(605)
<223> n = A,T,C or G

<400> 405
ggtactgagg tgtaaaggga tttatatggg gacgtaggcc gatttccggg tgttgtaggt 60
ttctcttttt caggcttata ctcatgaatc ttgtctgaag cttttgaggg cagactgcca 120
agtcctggag aaatagtaga tggcaagttt gtgggttttt tttttttaca cgaatttgag 180
gaaaaccaaa tgaatttgat agccaaattg agacaatttc agcaaatctg taagcagttt 240
gtatgtttag ttggggtaat gaagtatttc agttttgtga atagatgacc tgtttttact 300
tcctcaccct gaattcgttt tgtaaatgta gagtttggat gtgtaactga ggcggggggg 360
agttttcagt attttttttt gtgggggtgg gggcaaaata tgttttcagt tctttttccc 420
ttaaggtctg ctagaatcct aaaggcaaat gactcaaggt gtaaccagaa aaccagaaaa 480

tcccattttc nggatatnng acccccccag gttancggtt attnaacttt naccnnttta 540
cctttaggct ttgggaaaaa atttnccttg gaaaaagggt tgggannacc ttttttnccc 600
ccccc 605

<210> 406
<211> 255
<212> DNA
<213> Homo sapiens

<400> 406
ggtactacct gcggcctgtc tcccagcagg agtttgacaa gaacaccttg gatctcaggc 60
aacagaacgg aactgcctca tcacggaaga ccctctggaa tcaagaactc tacatccagc 120
aggacaactc agagaggaag cggaaacacc ttccagaccg acaggatggg cctgcagcca 180
agagtgagaa agcagccccc agaagtcagc actggttgca cagggacctg cgtgtgcggt 240
ttgtggacaa catgt 255

<210> 407
<211> 601
<212> DNA
<213> Homo sapiens

<220>
<221> misc_feature
<222> (1)...(601)
<223> n = A,T,C or G

<400> 407
ggtttttttt ttaagaggaa aacccggtaa tgatgtcggg gttgagggat aggaggagaa 60
tgggggatag gtgtatgaac atgagggtgt tttctcgtgt gaatgagggt tttatgttgt 120
taatgtggtg ggtgagtgag cccnattgtg ttgtggtaaa tatgtagagg gagtataggg 180
ctgtgactag tatgttgagt cctgtaagta ngagagtgat atttgatcag gagaacgtgg 240
ttactagcac agagagttct nccagtaggt taatagtggg gggtaaggcg aggttagcga 300
ggcttgctag aagtcntcat aaagctatta gtggnaagta gagtttgaag ccttgaaaag 360
aggatatgat nccactntga gtgcgttcgg tgtttgagtt ngctaggcag aatattantn 420
atgatgtaag cccgtggcca ttatgagant gactgccntg ttaagnttna nggggtttgg 480
atgangaatg gctngtaact actaaggcct atgntggctg gttnaanagn ttcnatntnc 540
nnantttann tcttgcttgt ctatgcagaa tnganctgnt attnatatgc ctcacnangg 600
g 601

<210> 408
<211> 630
<212> DNA
<213> Homo sapiens

<220>
<221> misc_feature
<222> (1)...(630)
<223> n = A,T,C or G

<400> 408
ggtacaaaag gagtctcagg cttgaagagg ttatgtaact tgcctaaggt cacacagtta 60
agtggcagaa atgagataca aaccaaagtc tgtctaactc cagagttcac accatcatgt 120
tatagtgcca tcttcgtaca ttgagctcca tagagacagc gccggggcaa gtgagagccg 180

gacgggcact gggcgactct gtgcctcgct gaggaaaaat aactaaacat gggcaaagga 240
gatcctaaga agccgagagg caaaaatgtc atcatatgca ttttttgtgc aaacttgtcg 300
ggaggagcat aagaagaagc acccagatgc ttnagtcaac ttctnagagt ttctaagaaa 360
gtgctcanta gaggtggaaa gaccatgttt gcttaaagag anaggaaaat ttnaagatat 420
tggcaaagcg gacaaaggnc cgttttgaaa gangaaatga naacctatat cccttccaaa 480
gggggagacc caaanagaag tttcaaggat nccaatggca ccccaagaag gcntncttng 540
gccttcttnc tcttctgctc ntgagtattc ggcccaaaat tcaaagggag aacatcttng 600
gcctggccat tggtgatgtt ggcaaaaaag 630

<210> 409
<211> 614
<212> DNA
<213> Homo sapiens

<220>
<221> misc_feature
<222> (1)...(614)
<223> n = A,T,C or G

<400> 409
cgaggtaccg ggatgcagca gtgatggctt ttggttgtat cttggaagga ccagagccca 60
gtcagctcaa accactagtt atacaggcta tgcccaccct aatagaatta atgaaagacc 120
ccagtgtagt tgttcgagat acagctgcat ggactgtagg cagaatttgt gagctgcttc 180
ctgaagctgc catcaatgat gtctacttgg ctcccctgct acagtgtctg attgagggtc 240
tcagtgctga acccagagtg gcttcaaatg tgtgctgggc tttctccagt ctggctgaag 300
ctgcttatga agctgcagac gttgctgatg atcaggaaga accagctact tactgcttat 360
cttcttcatt tgaactcata agttcagaag ctcctagaga ctacagacag acctgatgga 420
caccagaaca acctgaggag ttctgcatat gaatctctga tggaaattgt gaaaaacagt 480
gnccaaggat tggtaatcct gctgnnccag aaaaacgact tttggncatc atgggaacga 540
ctggcacang gtcttcaana tggagtcnca tatccgagcc cattccattg gaatnccgtt 600
caangacttn ntct 614

<210> 410
<211> 611
<212> DNA
<213> Homo sapiens

<220>
<221> misc_feature
<222> (1)...(611)
<223> n = A,T,C or G

<400> 410
cgaggtaccc atgttatgct ttcacctctc accccaatgg agtcacacag gcctgagttt 60
gaacagttaa cacagcttgg aagggacaca tgcctgattc ccatccttgg agaacaatat 120
catgctatga ggagtaggaa gggcaagaga tatgaaaaga acagaggaaa tgtggttcct 180
agaagtcaga aggcatcaag ggtccatcag tgtagaagtg gctggggcgg gagacgtaaa 240
cctcatccac ggtgttctgg ccagccaaca gtgggtcacc attcggcatg atttcttcaa 300
tctttacaca gtttctgaag atttccattg gctcagtgtt caaatgtctc agatcacagg 360
gcaaatctgg ctctggcact ggctgtgata caggtccttg gtctggctct ggcactgntt 420
gtgataccca tgcatagtgt gggctctatc acangctcca gagtggactt cagcacagac 480
tctagctttt ggccccagaa tccagccttg nctttaacca gtggctntta atncaggctg 540
acctctggct ntggcaccag ncctagttca gcttntaang ctccantttt gctntggttt 600

aagctccacn g                                                          611

<210> 411
<211> 590
<212> DNA
<213> Homo sapiens

<220>
<221> misc_feature
<222> (1)...(590)
<223> n = A,T,C or G

<400> 411
ggtacccttg tctttaaaag gattccccct tataaggact cttcaagtaa atccacacat      60
atatagtcaa ctaatttttg acaaagacac caagaataca caatggggaa aggatagtgt     120
cttcaataaa cagtattgga aatactggat atccacatgc aaaagaatga aattggatga     180
aatatggtga aattatttta caccgtaccg gctccccaac gtgcacggca ggagctacgg     240
cccagcgccg ggcgctggcc acgtgcagaa atggagtttc atcatgttgt cctctcgaac     300
tcctgacctc aagtgatcca cccgcctcgc ccttccaaag tgctgagatt acaggaagag     360
tctaacctgt ctctgcaagc tcttgagtcc cgccaagatg atattttaaa acgtctgtat     420
gagttgaaag ctgcagttga tggcctctcc aagatgattc aaacccagat gcagacttgg     480
atgtaaccaa cataatccaa gcggatgagc ccacgacttt aaccaccaat gcgctggact     540
ttgaattcag tgcttgggaa ggatacgggc gctnaaagac atcggaacan                590

<210> 412
<211> 609
<212> DNA
<213> Homo sapiens

<220>
<221> misc_feature
<222> (1)...(609)
<223> n = A,T,C or G

<400> 412
ggtacagaag atgctgtgga ctattcagac atcaatgagg tggcagaaga tgaaagccga      60
agataccagc agacgatggg gagcttgcag cccctttgcc actcagatta tgatgaagat     120
gactatgatg ctgattgtga agacattgat tgcaagttga tgcctcctcc acctccaccc     180
ccgggaccaa tgaagaagga taaggaccag gattctatta ctggtgtgtc tgaaaatgga     240
gaaggcatca tcttgccctc catcattgcc ccttcctctt tggcctcaga gaaagtggac     300
ttcagtagtt cctctgactc agaatctgag atgggacctc aggaagcaac acaggcagaa     360
tctgaagatg gaaagctgac ccttccattg gctgggatta tgcagcatga tgccaccaag     420
ctgttgccaa gtgtcacaga actttttnca gaattttcga cctggaaagg tgttaccgtt     480
tttctacgtc tttttggacc agggaagaat gtnccatctg gtttggcgga ntgctcgaan     540
aaagaggaag aagaagcncc gggagctgat ccaggaagaa cnnatcccgg aagtggagtn     600
gctcantna                                                             609

<210> 413
<211> 420
<212> DNA
<213> Homo sapiens

<400> 413

ggtaccgcca catcgctgac ttggctggca actctgaagt catcctgcca gtcccggcgt 60
tcaatgtcat caatggcggt tctcatgctg gcaacaagct ggccatgcag gagttcatga 120
tcctcccagt cggtgcagca aacttcaggg aagccatgcg cattggagca gaggtttacc 180
acaacctgaa gaatgtcatc aaggagaaat atgggaaaga tgccaccaat gtgggggatg 240
aaggcgggtt tgctcccaac atcctggaga ataaagaagg cctggagctg ctgaagactg 300
ctattgggaa agctggctac actgataagg tggtcatcgg catggacgta gcggcctccg 360
agttcttcag gtctgggaag tatgacctgg acttcaagtc tcccgatgac cccagcaggt 420

<210> 414
<211> 621
<212> DNA
<213> Homo sapiens

<220>
<221> misc_feature
<222> (1)...(621)
<223> n = A,T,C or G

<400> 414
   acatagtttt atagtagcca cagtaacttc cagtgactgg caaatttctt tgcatcagct 60
   ggcatgtgtg gtgaatggaa ttcccatgaa cagctcttac atccttccgc tttccttcta 120
   caggcctcgg tcttgtttcc aaaggtgact gcagtgagga tgtaaggtcc atgacctcta 180
   gggataatgc catccactca ggaagaaaga tgctgagaaa ctctagggat atctaagttt 240
   acatcacagg gggagaatca attgtggagg ttttaagaag acatttgaat ttttgcccct 300
   aatcaagaag tgttttgcca tctggtttac attcaataac tagttggctc atcatttgca 360
   gaaataaact ttcctctaga ttaggaaact tcatcatgag atctgagata tactggtttg 420
   gaaaggttnc tcagttctct tggctttcna agtccccggc cttggaatgg ggtnaaggcc 480
   cattggangc ncattnaatt ggccttgggg taaaggaaac tttggantgg cgnccaaatt 540
   nnaacccggg tgggccattn nttttnacnc ggtaaattaa ggntgggccc cggaaaattt 600
   ggttttccgg aananntttn g 621

<210> 415
<211> 619
<212> DNA
<213> Homo sapiens

<220>
<221> misc_feature
<222> (1)...(619)
<223> n = A,T,C or G

<400> 415
acaagctttt tttttntttt tttttttttt tttttttaaa gatcaacaaa cattttatta 60
attctgattc cttttatcat gtgctttttt atacaaagca ctttnaaatn cattacatta 120
tcttaaatat ataataggag tttctttcgg attcagttta aaaatgacaa atagcattcg 180
ttgcgcccaa gttagaatta caccaaaatt accatgngct ggcacatacc atcatcccac 240
tggtggctgg aaaactgggt tgcaggagtg tctgcactga gatgggccac caccccagtg 300
gccatatagg tatagatgag ggaaggatgg actanaanca agctgggctt tcngggtcgt 360
ctatantcct ttttcacttc attccgtttt ccccattgng cnttgaaccc agggaatctn 420
nttgacccat ccttggagct nttaaaaagg acctgngttn aaggtgccnc cntttgaaaa 480
ggggccccct ttgnatnaan tgggccgttg aaaaaggccc tttngatttg gancccaang 540
acngggaaat ttcacttngg cattaacnan tgtcnccgaa atnttcnctn ngntatgaac 600
tttantaana tngnttngn 619

<210> 416
<211> 611
<212> DNA
<213> Homo sapiens

<220>
<221> misc_feature
<222> (1)...(611)
<223> n = A,T,C or G

<400> 416
ggtacactaa ggtatgagct gaagctttag gttctccgtg cttccctcaa gacctccttc 60
ttgctaacag aagcagtagg caattgctgc agtgcgtttc tcaccctgcc aataggtctg 120
tctgtatctc tgttaaggaa aatagcctgg tccctcctgg cagtgcttgg aagcttgatg 180
ctaattttta tatagcgtgg caagctgacc agcagtgcca ggccttgatc tgtattctgc 240
actatccctt tacttggttc ctggcactga atggtctcca gccctgaaga atcacgtgtg 300
atcacagcag ctgacctggg ctttctcccc gagaggaagg ggcatgtcat ttttatttga 360
cagagggaaa atgggaactg ccttgactgc ctttgntgng ctttcccgcg taagaaagca 420
ctgngtttaa actgtgcaat acactngctt tgccatngat gtaaatgtaa gaaaatccct 480
anctttaaaa cctantggtt tgaacnttat tatatnaaan actttttaac ctattnngna 540
atttngggnc cttgccggta agntttnggg ggggnaaacn ngttncaaaa ggaaaggtcc 600
tttaactttn g 611

<210> 417
<211> 609
<212> DNA
<213> Homo sapiens

<220>
<221> misc_feature
<222> (1)...(609)
<223> n = A,T,C or G

<400> 417
caggtactga gacatcacat tactggccag tgttggcaaa gaaactgcca caaacaccat 60
gagaaggcag gcaattttat actcttcttc tggactaatg ttttccgatt tttgtgaaga 120
aagagctacg accaatgcag gatcaatctc acaaggtaat ccggcagctg atgataactc 180
atacacattc attgcaacct tcatatcagt ttcccttgga atgtgatcct taaaatcttc 240
aattgaactt acaagaaaag gaatgtggta ggataacaca tctctaagtg cttcttgtgc 300
caatgatcgg aaggataaaa ttacaccaat tattgtcatc ctcttcaaga cactgtcaac 360
agatgataat cttttaaaca gtgcagccat ctggtctggt ttgtcaaagc tggtcctcat 420
ttgtgttaac acatcaacat tctccaccac aagtttctta agttcaagca accttgtgat 480
gaaatatgcc acataaggct ttcacttaga aacntcatac catatgggcc taataagtct 540
ggataatgac ctcattctga natggtcaga atattcntnt gcattggaan gtaaatcaat 600
ttctggagg 609

<210> 418
<211> 643
<212> DNA
<213> Homo sapiens

<220>

<221> misc_feature
<222> (1)...(643)
<223> n = A,T,C or G

<400> 418
ggtactcccg attgaagccc ccattcgtat aataattaca tcacaagacg tcttgcactc 60
atgagctgtc cccacattag gcttaaaaac agatgcaatt cccggacgtc taaaccaaac 120
cactttcacc gctacacgac cgggggtata ctacggtcaa tgctctgaaa tctgnggagc 180
aaaccacagt ttcatgccca tcgtcctaga attaattccc ctaaaaatct ttgaaatagg 240
gcccgtattt accctatagc acccnctcta ccccctctag agcccactgt aaagctaact 300
taggcattaa ccttttaagt taaagattaa gagaaccaac acctctttac agngaaatgc 360
cncaactata tactacccgt atggcccacc atanttacct ccnatactnc ctacactatt 420
tncttatnaa cncancttna naatattaat ctcataatta ccagctanct ttncttaacc 480
aatgnccnat tanaaattaa anntattatn taccatactc cntgtnntcn nnataatgta 540
nngnananat tggnntcggc ttcaatttat nnggtcccaa aaatgcctan gcttaactcn 600
gnactngtnc gggcggcncg ttngnaaagg ggctgaaatt cng 643

<210> 419
<211> 607
<212> DNA
<213> Homo sapiens

<220>
<221> misc_feature
<222> (1)...(607)
<223> n = A,T,C or G

<400> 419
accagaatat ggacacattc caagctttct tgtcgatgct tgcacatctt tagaagacca 60
tattcatacc gaagggcttt ttcggaaatc aggatctgtg attcgcctaa aagcactaaa 120
gaataaagtg gatcatggtg aaggttgcct atcttctgca cctccttgtg atattgcggg 180
acttcttaag cagtttttta gggaactgcc agagcccatt ctcccagctg atttgcatga 240
agcacttttg aaagctcaac agttaggcac agaggaaaag aataaagcta cactgttgct 300
ctcctgtctt ctggctgacc acacagttca tgtattaaga tcttctttaa ctttctcagg 360
aatgtttctc ttagatccag tgagaataag atggacagca gcaatcttgc agtaatattt 420
gcaccgaatc ttctttagaa caagtgaagg ccntgaaaag atgcttntac ccccggaaaa 480
gaagcttcca atacnggntt gaanaagnac cttgggcggg aacacnctta nggnggaaat 540
tcngnccact tggnggccgt actaangggn nccaacttng gnccaacttt ggggaaacan 600
ggcanaa 607

<210> 420
<211> 494
<212> DNA
<213> Homo sapiens

<400> 420
ggtacatgag aacatatatt tattgcatga ttttctagat acacagtcta tgcattattc 60
atatacattt attttagcct aaagtggttt tcaaatccag ttcttcaagc cataaatgac 120
caagatccaa gcaatctgaa tttgtttttg tgattatttg actggaatgc ttcttaagtg 180
gaataactat actccgttat ccacccgatt tcctaatgta attgaaagat tttctatttt 240
gccacacact tggagacaat aagggttttt agttttatct actcttctat tgaagttaaa 300
gaaagaaaaa aagatttttt tatttgtatt aatgaaaagc tttagtttaa aataaggaga 360
tccagaataa aaagaagaga ctgatctctt caattattgt catctgtagc caccagcaca 420

tcactcttat gtaatcccca aaggcttggc atgccgtaag tgtgtggtgg ggtagactgc 480
tgccggggaa tcgt 494

<210> 421
<211> 366
<212> DNA
<213> Homo sapiens

<400> 421
ggtaccaagg ttattgatca agtcagcctt ggtcattcca attccagtat ccacaatagt 60
gagagttcga tcttgtttgt tcggtataag gttaatatgc agctctttcc cagagtctaa 120
tttactggga tctgtcaagc tttcataccg gattttgtcc aatgcatctg atgaatttga 180
aatgagctct ctcagaaaga tctctttgtt cgagtagaaa gtattgatga tcaatgacat 240
caactgggca atttctgcct gaaaggcgaa cgtctcaacc tcctcctcct ccatcggttg 300
gtcttgggtc tgggtttcct caggcatctt ggctaagtga ccgcacagga ccaacggcac 360
agccac 366

<210> 422
<211> 418
<212> DNA
<213> Homo sapiens

<220>
<221> misc_feature
<222> (1)...(418)
<223> n = A,T,C or G

<400> 422
ggtacaagag tgtttcatga aatccgtttt taaaatgaac atctctgtgt gccacagttc 60
ctaggactgg ggcaaggaca cagtgtcaag tcttgttttg aggatgagtc tctgaagaga 120
cagaattcct gccagaatgc gcacagaaca taagtcagcc aagtgtgtcg tgccagggat 180
actttgactt tggtttgctg ctgctgctag ggatattggg agggttatcc tttccaggtt 240
gtaggagagg gttgtgggta aaggtctgtc gtaaaggacc cctggctgct agctccaact 300
gattccgcat gcgttgttca cgctctcnca gctgacgccg tcatttcagc atttttccag 360
cctttttgta aagctctcta ggaagccttt ccgtggaggt aatttgtcca ggtcatgt 418

<210> 423
<211> 374
<212> DNA
<213> Homo sapiens

<400> 423
ggtctattct gcatatagag aactgagggc tttccctgag aaacagttga gttgtgttgc 60
caaccagaat ggctcgcaag ctgactgtga gctcggaaat ccttttaaaa gaaattcaaa 120
tgtcactttt tatttggttt taagtacacc tgattttcat gacaaatacg gtaatgctgt 180
attagctagt ggagccactt tctgtattgt tacatggaca tatgtagcaa cacaagtcgg 240
aatagaatgg aacctgtccc ctgttggcag agttacccca aaggaatgga ggaatcaagt 300
aatcatccca actggtgtaa taatgaattg tttaaaaaac agctcataat tgatgccaaa 360
ttaaagcact gtgt 374

<210> 424
<211> 610
<212> DNA

<213> Homo sapiens

<220>
<221> misc_feature
<222> (1)...(610)
<223> n = A,T,C or G

<400> 424
ggcggagctt gaggaaaccg cagataagtt tttttctctt tgaaagatag agattaatac 60
aactacttaa aaaatatagt caataggtta ctaagatatt gcttagcgtt aagtttttaa 120
cgtaatttta atagcttaag attttaagag aaaatatgaa gacttagaag agtagcatga 180
ggaaggaaaa gataaaaggt ttctaaaaca tgacggaggt tgagatgaag cttcttcatg 240
gagtaaaaaa tgtatttaaa agaaaattga gagaaaggac tacagagccc cgaattaata 300
ccaatagaag ggcaatgctt ttagattaaa atgaaggtga cttaaacagc ttaaagttta 360
gtttaaaagt tgtaggtgat taaaataatt tgaaggcgat cttttaaaaa gagattaaac 420
ccgaagggtg attaaaagac cttgaaatcc atgaccgcag ggagaattgc gtcatttaaa 480
gcctagttaa cgcatttcct aaaccccaga ccaaaaatgg ggaaggatta attgggagtg 540
gtaggatgaa ccaanttggg ngaagatgaa gttggaagtg gaaactggaa aaccgaaagt 600
ncctcggccc 610

<210> 425
<211> 368
<212> DNA
<213> Homo sapiens

<400> 425
ggtataagtt cagagagaaa gattccttcc caaggtcatg cagctagtaa atgatagaat 60
caggattcat agcatcacta taggggtca atatttacac aaaaaaggaa agtcacaagc 120
ctgtttaaaa tgaagtgacc accttttctt gcatagacta aataactcga actggcattt 180
ttaggttgga aagacagctg aattagtagt taagtctgat agccaagtaa gttttaaaaa 240
ccaaagcatc caggatgcac acccctgcac catttgctgt gcgaattaat agttctgtct 300
ctctctctct ttcttttttc tttttattct ttgagatgga ttttcgctct tgtcgcccag 360
gctggagt 368

<210> 426
<211> 630
<212> DNA
<213> Homo sapiens

<220>
<221> misc_feature
<222> (1)...(630)
<223> n = A,T,C or G

<400> 426
actaccacag cctttaagtg acattgattt ataacttggt cacaattcac tgcatttagg 60
aaaaccagca ttcttatctg gtcagtgctc gcttcttagc aacccctaat taaatttaat 120
tcatctctaa atcttagctt caactttatt caattacatt tggctgacgg ctgttttcta 180
aaacccttaa gtgttgacca taaatgcaaa acttccagta tctgttgggt tttattagca 240
gatgctgctt ttatttaaaa aaaaccgaca gtataactgt cataattatg gaaggcactg 300
cttccgataa ttatattcta ttaaaaaaac accatttata gtgaactctg tcactgataa 360
ataaacaata aatatctcag tgccaaaagg acagaaagct ctcccctaag attaacactt 420
tggccaaaat ttggtagcat attattcttt aaagtctgac aaactgagtc tgcaactaaa 480

cacctgaaac tggtctcttt caatgggctt tggaagaacc aaaataccaa gaactaaatg 540
gaggcttatg ggggaagggn cgaggaaata aatatctaag cnttggcttc tggccctctt 600
tcataaannc ctgaggtaca tattangctn 630

<210> 427
<211> 224
<212> DNA
<213> Homo sapiens

<400> 427
ggtgggaggg tggtgtccac tgcccagttc cgtgtcccga tgcccagcgc cagcgccagc 60
cgcaagagtc aggagaagcc gcgggagatc atggacgcgg cggaagatta tgctaaagag 120
agatatggaa tatcttcaat gatacaatca caagaaaaac cagatcgagt tttggttcgg 180
gttagagact tgacaataca aaaagctgat gaagttgttt gggt 224

<210> 428
<211> 543
<212> DNA
<213> Homo sapiens

<220>
<221> misc_feature
<222> (1)...(543)
<223> n = A,T,C or G

<400> 428
ggacgctctc agctctcggc gcacggccca gcttccttca aaatgtctac tgttcacgaa 60
atcctgtgca agctcagctt ggagggtgat cactctacac ccccaagtgc atatgggtct 120
gtcaaagcct atactaactt tgatgctgag cgggatgctt tgaacattga aacagccatc 180
aagaccaaag gtgtggatga ggtcaccatt gtcaacattt tgaccaaccg cagcaatgca 240
cagagacagg atattgcctt cgcctaccag agaaggacca aaaaggaact tgcatcagca 300
ctgaagtcag ccttatctgg ccacctggag acggtgattt tgggcctatt gaagacacct 360
gctcaagtat gacgcttctg agctaaaagc ttccatgaag gggctgggga accgacgagg 420
actctctcat tgagancatc tgnttcagaa cccaacccag gaagctgcan ggaaantaac 480
cagagtctac caagggaaat gtaccctnng gnccgngaac cacgcttaan gggcgaaatt 540
cca 543

<210> 429
<211> 346
<212> DNA
<213> Homo sapiens

<400> 429
actatctttt cattcagtcc cttaagcagc ttactcttca atgccaacaa aactttattt 60
tttaaatagt cttaaaagtg cttaagggag ttctggttcc tctttttagc ctgcacagtt 120
taagatcaat ggtaaaggta ggaaataatc ataagggcac tggaagaagg aatgagtcta 180
aataatgtat aatgactgtt ccgccatacc aattttgtca tggtgattat tcactaattt 240
tataggagag tgtattgaga tctgctacag cttcttggat ctttgaagca ctgctgaatt 300
acatacacaa agcagagcag atgtcagcac ctgattaatc agtacc 346

<210> 430
<211> 605
<212> DNA

<213> Homo sapiens

<220>
<221> misc_feature
<222> (1)...(605)
<223> n = A,T,C or G

<400> 430
ggtggcgcgg ccgaggtaca gctggtgctt ctgccttacc ccatcctctc ctctcagatt 60
caccgaggac tgttcaggtg gtaacattct cttagggtag ggaactctgc agagggagag 120
ctgaggaggt tccggccata gttgtttgta atcttagggc tctgggcttg gctgaaacat 180
gacggtattg cttggtttca ggcttgacac tgccaggcgc ctattgcttg acctctgttt 240
aaatgaggga cttcaagact agacagcatg gctcttttca gtttattgca tgaaggagtt 300
acactagtcc aagttaaaag cggaccccaa atggttacat tatacaagct gtgaggtttt 360
taaacctgtg acaagggaga gaagggaaat tctactcatt gcaaggaaat cctcacttaa 420
gcttcagtga gccacaagca cttaaaaccc atgaaccttc agctgatcgt ccttagccag 480
tccaatctct acgaggaact ggcatatgtc ttgcgttggc accctgtagc tgaattactt 540
ctcatattcn gatgctaatt ncagacctgn ccggcggccg tcaaaggcna atccacnact 600
gnggn 605

<210> 431
<211> 430
<212> DNA
<213> Homo sapiens

<220>
<221> misc_feature
<222> (1)...(430)
<223> n = A,T,C or G

<400> 431
acactaccaa cagatcaaag aaacccctcc ggccagtgag aaagacaaaa ctgctaaggc 60
caaggtccaa cagactcctg atggatccca gcagagtcca gatggcacac agcttccgtc 120
tggacacccc ttgcctgcca caagccaggg cactgcaagc aaatgccctt tcctggcagc 180
acagatgaat cagagaggca gcagtgtctt ctgcaaagcc agtcttgagc ttcaggagga 240
tgtgcaggaa atgaatgccg tgaggaaaga ggttgctgaa acctcagcag gccccagtgt 300
ggttagtgtg aaaaccgatg gaggggatcc cagtggactg ctgaagaact tccaggacat 360
tatgcaaaag caaagaccaa aaaanaaann nnaaaaaaaa aagcttgtac ctnggccgng 420
accacgctaa 430

<210> 432
<211> 479
<212> DNA
<213> Homo sapiens

<220>
<221> misc_feature
<222> (1)...(479)
<223> n = A,T,C or G

<400> 432
acaagctttt tttttttttt tttttttttt ttggaacgta ggctttctct tgtctttatt 60
ctggggagga ggaatcctcc tcatcatctt cctcatcttc atcattgaac gaacaggggg 120

tctcgcctcg ggactcggag cagtgagagg ccgcactgct ggactggtga ctgtttgggg 180
ccaggaactg cccagttgct aaggccactt ctgcatccaa gcataaccct tggtttacac 240
ttgactgggg taaggtggca ccagtggtca ggtctaaatt tgaaactgat tgggtagaag 300
ttcagaagta gtccctgatt taaccaagaa ggtcctgtgg agatatctgn gatataacct 360
tctaaagcct ttggcaccag ggatttcgca agttttcaan atcctccaga gagcatttgc 420
ctgacttcag gcnaaacgac attcccatnc gctttangac cttgggcgng accacgcta 479

<210> 433
<211> 600
<212> DNA
<213> Homo sapiens

<220>
<221> misc_feature
<222> (1)...(600)
<223> n = A,T,C or G

<400> 433
ggtacccaac aataccaccg accaggagct gcaacacatt cgcaacagcc tcccagacac 60
agtgcggatt aggcgggtgg aggagcggtt ctcagccttg ggcaatgtca ccacctgcaa 120
tgactacgtg gccttggtcc acccagactt ggacagggag acagaagaaa ttctggcaga 180
tgtgctcaag gtggaagtct tcagacagac agtggccgac caggtgctag taggaagcta 240
ctgtgtcttc agcaatcagg gagggctggt gcatcccaag acttcaattg aagaccagga 300
tgagctgtcc tctcttcttc aagtccccct tgtggcgggg actgtgaacc gaggcagtga 360
ggtgattgct gctgggatgg tggtgaatga ctggtgtgcc ttctgtggcc tggacacaac 420
cagcacagag ctgtcagtgg tggagagtgt cttcaagctg aatgaagccc agcctagcac 480
cattgccacc agcatgcggg attccctcat tgacagcctc acctgagtca ccttccaagt 540
tgttccatgg gctcctggct ctggactgtg gccaaccttc tncacattcc gccaatctgt 600

<210> 434
<211> 417
<212> DNA
<213> Homo sapiens

<400> 434
ggtaccaacg cgctaagaaa tcagctccaa ttcgaagtgc acctgttccc cccaaagatt 60
gcacacctcc tacccgcttc tccttgagtg ctgggctgtc atccccaagg gcaagacgag 120
aagcacagct ccggaactca gccaggccca ggattggcag atactcgtga tttaggctat 180
tgtcattagc aatcttctgc tccactttct tcactactgg caaaacccag ggatggcagt 240
catccgtgcg atatgctccc actcccaggt tgaccttgcg ggggtccgga tcctccctga 300
agtcggcagt gagcttgaag accaggacag gctgggcctg cggaacctcg gcaaagactg 360
acggaggtgc catatcgaga gactaggaat caagagattt caccccacgc ccggagc 417

<210> 435
<211> 672
<212> DNA
<213> Homo sapiens

<220>
<221> misc_feature
<222> (1)...(672)
<223> n = A,T,C or G

<400> 435
ggcagagaac gatgtggaca atgagctctt ggactatgaa gatgatgagg tggagacagc 60
agctggggga gatggggctg aggcccctgc caagaaggat gtcaagggct cctatgtctc 120
catccacagc tctggctttc gtgacttcct gctcaagcca gagttgctcc gggccattgt 180
cgactgtggc tttgagcatc cgtcagaagt ccggcatgag tgcatccctc aggccattct 240
gggaatggat gtcctgtgcc aggccaagtc gggcatggga aagacagcag tgtttgtctt 300
ggccacactg caacagctgg agccagttac tgggcaggtg tctgtgctgg tgatgtgtca 360
cactcgggag ttggcttttc aagatcagna aggaatatga gcgcttcttt taatacatgc 420
ccaatgtcaa aggttgctgg tttttttggt gggctggcta tcaagaaagg atgaagaagg 480
tgctgaanaa anaactgccc natattcgtc ctgggggact tcaagcccgt atnctaancc 540
tggcttcgaa ataagancct taancttaaa cncataaaca ctttatttgg atgaatgngn 600
taanancttg aacagtngac atncttcgga tgtcnggaaa ttttncnatg accccccana 660
anngncntgn tt 672

<210> 436
<211> 469
<212> DNA
<213> Homo sapiens

<400> 436
ggtacaagct tttttttttt tttttttttt ttttttataa aagcatttta ttgaacacat 60
tctggaggta agttagaacc aaaacaaaat ttgggattgg ggtggggatt ctgttttgat 120
gatttagatt tgggaaaact ttggattctc gtgtcagcag gggccatgct gtgggaaacc 180
tgaaggctga tttgaagcag aatatagaac tgcggcacgg gagaccaggg gctgggaatg 240
gggctctcct gggaaccaaa gaatgtggtt ctgcaattgg cttggtctag actactctcc 300
agaaaaggat aaaacatggc ttgagcaact gcctagaaga ggcaatctcc atgggctggg 360
ttgctgcact tggaaggcag tgacttgcag caggttctta gctcttgaag ctcttccggg 420
aggaggaggt ggtggagaca aatttgacgc tggggctgct acccccgcc 469

<210> 437
<211> 457
<212> DNA
<213> Homo sapiens

<400> 437
actgaggcat cttcttcagc atctgggaca ggtcccgcat ggtgggtctt ctctccagta 60
ttcattctct tgctagaaga aaaatctttc agagaccggg gtgacttctg ggacacctct 120
gcgatgtgct tgtggcgcag tgctatccac aggtcgtcgt cctcgtccag gagcacctcc 180
ttcacccgtg cctccccgat gccgctggtc tcatacttgt atacatcatt ttcgataggc 240
agcagatcat aactcatagc ctgaaaagtc aattcatgga gcacagggga gctggggtca 300
aagcctcgat ccaggatcag gagctgggag cgtgccttgt ctgggccctc ccccattgtt 360
ggatcatcag ctttataggc atcgagcttg tcctggatta gctgagccag cagggcattg 420
tccttgtatt ccccccgata ccgcatagcc gggtacc 457

<210> 438
<211> 731
<212> DNA
<213> Homo sapiens

<220>
<221> misc_feature
<222> (1)...(731)
<223> n = A,T,C or G

<400> 438
accaattatt cagaatcaaa tggatgcact tcttgatttt aatgttaata gcaatgaact 60
tacaaatggg gtaataaatg ctgccttcat gctcctgttc aaagatgcca ttagactgtt 120
tgcagcatac aatgaaggaa ttattaattt gttggaaaaa tattttgata tgaaaaagaa 180
ccaatgcaaa gaaggtcttg acatctataa gaagttccta actaggatga caagaatctc 240
agagttcctc aaagttgcag agcaagttgg aattgacaga ggtgatatac cagacctttc 300
acaggcccct agcagtcttc ttgatgcttt ggaacaacat ttagcttcct tggaaggaaa 360
gaaaatcaaa gattctacag ctgcaagcag ggcaactaca ctttccaatg cagtgtcttc 420
cctggcaagc actggtctat ctctgaccaa agtggatgaa agggaaaagc aggcagcatt 480
agaggaagaa caggcacgtt tgaaagcttt aaaggaacag cgcctaaaag aacttgcaaa 540
gaaacctcat acctctttaa caactgcagc ctctcctgta tccacctcag caggagggat 600
aatgactgca ccagccattg acatattttc tacccctagt tcttctaaca gcacatcaaa 660
gctgnccaat gatctgcttg anttgcagca gccaactttt cacccatctg tacctttggg 720
ccgngaacac g 731

<210> 439
<211> 470
<212> DNA
<213> Homo sapiens

<400> 439
ctgcgagcca ggattcccga tccagagaca atggccccga tgggatggag cccgaaggcg 60
tcatcgagag taactggaat gagattgttg acagctttga tgacatgaac ctctcggagt 120
cccttctccg tggcatctac gcctatggtt ttgagaagcc ctctgccatc cagcagcgag 180
ccattctacc ttgtatcaag ggttatgatg tgattgctca agcccaatct gggactggga 240
aaacggccac atttgccata tcgattctgc agcagattga attagatcta aaagccaccc 300
aggccttggt cctagcaccc actcgagaat tggctcagca gatacagaag gtggtcatgg 360
cactaggaga ctacatgggc gcctcctgtc acgcctgtat cgggggcacc aacgtgcgtg 420
ctgaggtgca gaaactgcag atggaagctc cccacatcat cgtgggtacc 470

<210> 440
<211> 353
<212> DNA
<213> Homo sapiens

<220>
<221> misc_feature
<222> (1)...(353)
<223> n = A,T,C or G

<400> 440
ggtacattga agagaacaag tatagcagag ccaaatctcc tcagccacct gttgaagaag 60
aagatgaaca cttcgatgac acagtggttt gtcttgatac ttataattgt ggatctacat 120
tttaaaatat caagagatcg tctcagtgct tcttccctta caatggagaa gttttgcttt 180
tctttgggct ggaggaagag catcctatgg tgtgtcaaaa ggcaaagtgt gttttgagat 240
gaaggttaca gagaagatcc cagtnaggca tttatatcnn nngatattga catacatgaa 300
gttcgnattg gctggncact actcnnntgg aatgntcttg gngaanaana att 353

<210> 441
<211> 647
<212> DNA
<213> Homo sapiens

<220>
<221> misc_feature
<222> (1)...(647)
<223> n = A,T,C or G

<400> 441
acattattga tgaacgcagt gactctgaag aataatcaga ggatgacatg ggagagccca 60
atggcttcat tgattgccca tccctgtgag gacagggaaa tgggagcttg tgggattctg 120
gggatgacag aggtgagtga ggtgaagccc taggggatgg tgaatggtag ctccggatcc 180
ctggtgagga gcttcctctt aagtctgagt tactgagagg gaagagggag aagctgggtg 240
aggctagcat cgtcgacctt ggggaatccg ggctggggga ctgttcacaa gaagagccag 300
acaagaccct actgttctta ggtgcagaca ggattatgaa acctgaagct cccagggacc 360
ccaacaaatt ttcaaaccct gagaatgaag gagtgtgtgt gactgtgaga gtgtgtgtgt 420
gtgtgtgtgg tgtgaggtat gcgctcctta agaaaatgga aataaaccaa ccaatgagac 480
agacagacag acagagactc acttatccaa gtgttctgtc cagtcctctg aatccggttc 540
caagtcgcaa gaccctttga gctccaagtc catacagagc ccggcaaaat gctccggccc 600
gctgctcggc tcttgtgacg atctgagtac ctcgggccgn gaccacg 647

<210> 442
<211> 1002
<212> DNA
<213> Homo sapiens

<220>
<221> misc_feature
<222> (1)...(1002)
<223> n = A,T,C or G

<400> 442
acagaagttg aagtgaaatc tactgaggag gcttttgaag ttttctggag aggccagaaa 60
aagagacgta ttgctaatac ccatttgaat cgtgagtcca gccgttccca tagcgtgttc 120
aacattaaat tagttcaggc tcccttggat gcagatggag acaatgtctt acaggaaaaa 180
gaacaaatca ctataagtca gttgtccttg gtagatcttg ctggaagtga aagaactaac 240
cggaccagag cagaagggaa cagattacgt gaagctggta atattaatca gtcactaatg 300
acgctaagaa catgtatgga tgtcctaaga gagaaccaaa tgtatggaac taacaagatg 360
gttccatatc gagattcaaa gttaacccat ctgttcaaga actactttga tggggaagga 420
aaagtgcgga tgatcgtgtg tgtgaacccc aaggctgaag attatgaaga aaacttgcaa 480
gtcatgagat ttgcggaagt gactcaagaa gttgaagtag caagacctgt agacaaggca 540
atatgtggtt taacgcctgg gaggagatac agaaaccagc ctcgaggtcc agttggaaat 600
gaaccattgg ttacctgacg tgggtttgca gagttttcac cnttgncgtc atgcgaaatt 660
ttggatatca acgatgagca gacactttcc angctgattg gaagccctta gagaaacgac 720
ttacttacga caaatggatg attggtgagt ttaacaaacc atntaaagct tttaaagctt 780
ttgtaccaga aattggcaat gctggtttaa gtnaaggaaa ancccctgcc anggggaact 840
taatggaaan ggggaaaaag atttngnccc aaattggaat tnaaccnccc gaaaaaaaaa 900
annnnnnaaa aaagancttg gncgggaacc ccccttaggg gaattcnncn ccttgggggc 960
cnntnntaan ggacccantt ggnccaaaat ttggggaaan tg 1002

<210> 443
<211> 486
<212> DNA
<213> Homo sapiens

<400> 443
acattagtct taattgactt attacataat cgattcgtgt ctagttttga gagctttaag 60
ttctcaatta tagttctttg aaaactgaat agcaaataac aatatgatta acttcatatt 120
tattatttca acgatctttt ttataaccga gtttaatttt taaattaaat ttctaaaata 180
gattaccaat attaaaatac cttaagatat ttatctttag caataatagg caatattaaa 240
gttgtattaa cttttaaatt aagtaagagt atttggtgga tgccttgggt ctgaaagtcg 300
atgaaggacg cgattacctg cgataagctt cgtggagttg gaaataaact atgatacgga 360
gatttccgaa tggggtaacc taactgagca aacctcagtt gcattttgat gaatccatag 420
tcaaattagc gagacacgtt gcgaattgaa acatcttagt agcaacagga aaagaaaata 480
aatacc 486

<210> 444
<211> 625
<212> DNA
<213> Homo sapiens

<220>
<221> misc_feature
<222> (1)...(625)
<223> n = A,T,C or G

<400> 444
gagggatgca cgttgcctta gccgagcttc ggagagaagc ctgatatgta acccaggcag 60
gtgggagcct cagtctgtcg ggctgaggtc tggcatctac aaagcctctt ggccgtgttc 120
tgaacttgaa gcctggagga gttctctgct cagcacagcc aaggaacaga attagaagaa 180
aaggaaccct ggcctgaggc aggtgacaaa cattaccacc ccagctgtgc acgatgcagc 240
agatgcaacc agatgttcac agaaggagag gaaatgtatc ttcaaggctc caccgtttgg 300
catcccgact gtaagcaatc tacgaagacc gaggaaaagc tgcggcctac caggacatcc 360
tcggaaagta tttattctag gccaggctcc agtattcctg gctcaccagg tcatactatc 420
tatgcaaaag tagacaatga gatcctggat tacaaggatt tagcagccat tccgaaggtc 480
aaggcaattt atgacattga acgtccagat cttattacct atgagccttt ctacacttcg 540
ggctatgatg acaaacagga gagacagagc cttggagagt ctccgaggac tttgnctnct 600
acttcatcag cagaagggta cctcg 625

<210> 445
<211> 1002
<212> DNA
<213> Homo sapiens

<220>
<221> misc_feature
<222> (1)...(1002)
<223> n = A,T,C or G

<400> 445
accacaactc ccaggatttt cctggatcaa accttgtatc tcttctgcaa gtattgtgta 60
tattggtctg agagacgtgg accctcctga acattttatt ttaaagaact atgatatcca 120
gtatttttcc atgagagata ttgatcgact tggtatccag aaggtcatgg aacgaacatt 180
tgatctgctg attggcaaga gacaaagacc aatccatttg agttttgata ttgatgcatt 240
tgaccctaca ctggctccag ccacaggaac tcctgttgtc gggggactaa cctatcgaga 300
aggcatgtat attgctgagg aaatacacaa tacagggttg ctatcagcac tggatcttgt 360
tgaagtcaat cctcagttgg ccacctcaga ggaagaggcg aagactacag ctaacctggc 420
agtagatgtg attgcttcaa gctttggtca gacaagagaa ggagggcata ttgnctatga 480

ccaacttcct actcccagtt caccagatga atcagaaaat caagcacgtg tgagaattta 540
ggggacactg tgcactgaca tgtttcacaa caggcattcc agaattatga ggcattgagg 600
ggatagatga atactaaatg gttggctggg tcaatactgn cttaatgaga acatttacac 660
attctcacaa ttggtaaagg ttcccctcta ttttggtgac caatactact ggaaatggaa 720
tttggntttt tgcagttcac agggtantaa tatggctcag taccttnggc cgcgaacacg 780
cttaagggcn aattccacac acttgggcgg ccgttcttaa nggatccgaa ctnggancca 840
agcnttggcg taaacatggg cnataantgg tttctggggg gaaatggtat ccggttacaa 900
tttcccccca nattccnaac ccggaagncn tnaagggtaa aacccggggg gccctaangg 960
ggngctaact ccaaatnaaa tgggttgngc ttaatggccc nt 1002

<210> 446
<211> 367
<212> DNA
<213> Homo sapiens

<400> 446
ggtacaaaag agtatgggct cacaagaaga tgattcagga aacaaaccat ccagttattc 60
ttgaaactaa catccatcct gagctaaaca agagaaacta ccatcttggc cagtgacaag 120
tgttcggagg gcagcagaga ggaccaagcc tgtgtcacct ggagactaag aaattaagtt 180
ttgttttgac atcttcagtc ctgtgtgctt tcagaaaacc attttctctg caaagaaagg 240
aaacagattt gcaaacttta aagtctgtcg tggatttatt tatcctcaga ttattgttac 300
tgcattaaat ctaccttttt gttttaagtt gcttgaaaaa aaaaaaaaaa aaaaaaaaaa 360
aaaaagc 367

<210> 447
<211> 754
<212> DNA
<213> Homo sapiens

<220>
<221> misc_feature
<222> (1)...(754)
<223> n = A,T,C or G

<400> 447
actcttgggg tggaaaagat ctacacataa caagttcaga aaccacagtg ataaactaac 60
ctaagaaaat cgtttaactt ttatctacct gaaacacaaa attaaaaggc aacctataaa 120
ctggaaaaaa atatttgcat caaatataac aaaagattat caatatcctt aagatgtaaa 180
tggcttttgc aaaacaatca atagaaaaat gactaggaat tagaaaatca tacacacaca 240
cacacacaca cacacgcaca cacacacaca ccacaaatgg ccaattgaca catggtagag 300
atgttcagtc accagcagac aaagcaatgt tcacatccac agggaaagca gactcgatcc 360
gtcggaggag caaaggtttt caatgtnata aagcccggtt ctgaggaaan angggaaggc 420
atcagggttt ncctcaccca gtgaagaaca cctaattnga aaaaaatccc ttcccttgct 480
tggggccagt tttaaccaat tatggaaccc ttgaaagtct ttaaagaagt ttnaaccagt 540
caatttncct ttcttcngaa atggtatggt atttcaggca tttcccaaag gaggtttanc 600
canccggacc gttgaaaaaa ggtcntggaa ccttccnagg gnaaagttca tttgccaagg 660
gtnttaattt ttcttaagga agggaaaaaa aaaaancttg naaaaatncc ctnngattgn 720
ccccattggn aancccggnn atnggtttaa aatt 754

<210> 448
<211> 551
<212> DNA
<213> Homo sapiens

<400> 448
accagaaccg agttcgggat actcacaggc tcatcactca gatgcagctg agcctggcag 60
aaagtgaagc ttccttggga aacactaaca ttcctgcctc agaccactac gtggggccaa 120
atggctttaa aagtctggct caggaggcca caagattagc agaaagccac gttgagtcag 180
ccagtaacat ggagcaactg acaagggaaa ctgaggacta ttccaaacaa gccctctcac 240
tggtgcgcaa ggccctgcat gaaggagtcg gaagcggaag cggtagcccg gacggtgctg 300
tggtgcaagg gcttgtggaa aaattggaga aaaccaagtc cctggcccag cagttgacaa 360
gggaggccac tcaagcggaa attgaagcag ataggtctta tcagcacagt ctccgcctcc 420
tggattcagt gtctcggctt cagggagtca gtgatcagtc ctttcaggtg gaagaagcaa 480
agaggatcaa acaaaaagcg gattcactct caagcctggt aaccaggcat atggatgagt 540
tcaagcgtac c 551

<210> 449
<211> 398
<212> DNA
<213> Homo sapiens

<400> 449
accttcaaca ggcatctcaa cagccccatc accaacacct gtgtgcaagg catagccatc 60
acgcggaaaa gtctcaggac tcagaactac accataaatg caggatcttt ttatttcata 120
taaaaatgat caatgtgaaa aaagccaaac tgtatgctgg ttttacagac tccgaccctt 180
cctgacagtc gtcttgtctg gccaggctgg gggcccagca ttcctggaag ggagagacag 240
cccggcatct cagtatttca ttgggacaac aagctggatg tggcagggaa agctgagagc 300
gccaaggtcc ccttgcttta tcccaagctc ggagggacgc agcctggcat ggctctggcc 360
tagcagccag gtgacatggc caggcacctt cctgtacc 398

<210> 450
<211> 672
<212> DNA
<213> Homo sapiens

<400> 450
accttattag aaagcgacgg caaactatgt gccagcagcc gcggtaatac ataggtcgca 60
agcgttatcc ggaattattg ggcgtaaagc gtccgtaggt tttttgctaa gtctggagtt 120
aaatgctgaa gctcaacttc agtccgcttt ggatactggc aaaatagaat tataaagagg 180
ttagcggaat tcctagtgaa gcggtggaat gcgtagatat taggaagaac accaataggc 240
gaaggcagct aactggttat atattgacac taagggacga aagcgtgggg agcaaacagg 300
attagatacc ctggtagtcc acgccgtaaa cgatgatcat tagttggtgg aataatttca 360
ctaacgcagc taacgcgtta aatgatccgc ctgagtagta tgctcgcaag agtgaaattt 420
aaaggaattg acgggaaccc gcacaagcgg tggagcatgt ggtttaattt gattctacgc 480
gtagaacctt acccactctt gacatcttct gcaaagctat agagatatag tggaggttaa 540
cagaatgaca gatggtgcat ggttgtccgt cagctcgtgt cgtgagatgt taggttaagt 600
cctgcaacga gcgcaaccct tttctttagt tactaatatt aagttaagga ctctagagat 660
actggctgga cc 672

<210> 451
<211> 554
<212> DNA
<213> Homo sapiens

<220>
<221> misc_feature

<222> (1)...(554)
<223> n = A,T,C or G

<400> 451
acacgctgcc aaagtaattc ctgctcatcc atgccctgtc tctgtctctt ttagagtcat 60
accttatttg agtataggtt gcttaatttt gctagacttc ctgaaaacac taaggtggag 120
tatcagaagt gattttagtc acagttctgc gggagagctt agaataacat cctcctttgg 180
gaggtggtct tgggtgcgtg gatgttggta tacagtcttt attgtaagtc tgatacaaaa 240
tgctaataaa tttaatgttt ttcttcctta atttattggc atagttcttc aggtagcacc 300
tcatttttat taatgatatt gggattaact atgaacaagc tatatgtaga catttgcatt 360
taaggacatt gcagtggttc aaagatccca tcattgcagc ttgnatcctt tagatccaat 420
cggaaacttc tggagcttac attaaatgct catttgagct aaatagnaat ctggtnaacc 480
aganttgggc aatactttta aaganactgg ggacnattan ggntaganng ggctatttcc 540
cctttnaggg nggg 554

<210> 452
<211> 566
<212> DNA
<213> Homo sapiens

<220>
<221> misc_feature
<222> (1)...(566)
<223> n = A,T,C or G

<400> 452
acaaataaat tgtatgcttt ccggataagt gacatgttta tatggtgata aagggaatta 60
taatgctctt aactcttatg tagtatgttc tcatcaaaat caccaagcat gagaacactg 120
tttagtctca ttcatcactc agcacagcct ctttctgtcc acttcagggc caagtctttg 180
ccatggcccc acataacgtg taaattagct tcaggatcaa aaatctttgg aaaacccagt 240
ttgctgagcc ttgaaggaag cctttagacc cagcttcaat gaagtcacag ctccctgagg 300
gtcctggtgg actggaggcg gcctcccaag cctgggagct gtgtgcctgg atggtctcac 360
tggggtgatg acccaagctc atggctccct ctcaacctct aacccttctt aacacaagtc 420
acccctggnc ccctgagcac tcctgaagtc cctttgaaag gacatttcta ggctnctaag 480
angcctggtt ccttcagctg gcaccctnan tttaccagcc nggnangcag gntttccaan 540
ttntgctggg tnaanaaanc ccgncc 566

<210> 453
<211> 688
<212> DNA
<213> Homo sapiens

<220>
<221> misc_feature
<222> (1)...(688)
<223> n = A,T,C or G

<400> 453
ggtactccta cttcattttt gaaggcttgt aactgctgag gtgtaggtgc tgtcacattc 60
aacattttca ctgccacatc accatgccac tttcccttgt agactgttcc aaatgatcca 120
gatccaattc tttgtcccac tgtaatctgc ccatcaggaa tctcccaatc atcactcgag 180
tcccgtctac caagtgtttt cattcgattc ctgtcttctg aggatgaaga tgacttcctt 240
tctcgctgag gtcctggaga tttctgtaag gctttcacgt tagttagtga gccaggtaat 300

gaggcagggg gggtagcaga caaacctgtg gttgatcctc catcaccacg aaatccttgg 360
tctctaatca agtcatcaat attgacaggt tctattgtgt ttatatgcac attggggagc 420
tgatgaggat cggnctcgtt gcccaaattg aattccatga tcttcatctg ctgggccgaa 480
nggctgngga aatggaatgg gttttgaaga gaccgactgg tgagaattgg ggcccaatan 540
aatcnaggcg ggtgccgaaa gggatgatcn cantgtaggc agtctttggt aaggaccctn 600
ttctgnggga ttgggggggt taannacttg gggacaaccg caaatcaant ggcctattaa 660
nccttaggga aattntanct gccngggg 688

<210> 454
<211> 565
<212> DNA
<213> Homo sapiens

<220>
<221> misc_feature
<222> (1)...(565)
<223> n = A,T,C or G

<400> 454
actggctgcg aggcgccagt cgatcaatgt atgacaggag ctgagacttg gccacaccag 60
gatcccccat cagacagatg ttgatgttgc cccggatttt catgcctcga ggagactggt 120
ccacaccccc gactagcagg agcagcagtg ccttcttcac atcttcatgc ccgtatattt 180
ctggggcgat tgaagctgcc agcttttcgt agaaaatcct cctctgcaat ttgcctcagc 240
tcctccctgg tgagctctcc agccccagac tcatcatcct cactcttgtt catcttcaca 300
atccgatggg cttccaggta ggtttctgag agtaaaccct gtacttgatg cactttgcac 360
agacagggtg tgttgaatag gcattatttt ataaggaaaa gaagtctgtg gtgactggtt 420
tgaaataaag tggtaatggt gatggagggc agntcttttg gatttgcctg gtantgctga 480
tgggagacng gagaccacct ngggcgcgaa cacgcttaag gggganaatt cngcacactg 540
gggggccgta ctataggngn ccnnc 565

<210> 455
<211> 566
<212> DNA
<213> Homo sapiens

<220>
<221> misc_feature
<222> (1)...(566)
<223> n = A,T,C or G

<400> 455
acagtcctga ttgcatcata attgtggttt ccaacccagt ggacattctt acgtatgtta 60
cctggaaact aagtggatta cccaaacacc gcgtgattgg aagtggatgt aatctggatt 120
ctgctagatt tcgctacctt atggctgaaa aacttggcat tcatcccagc agctgccatg 180
gatggatttt gggggaacat ggcgactcaa gtgtggctgt gtggagtggt gtgaatgtgg 240
caggtgtttc tctccaggaa ttgaatccag aaatgggaac tgacaatgat agtgaaaatt 300
ggaaggaagt gcataagatg gtggttgaaa gtgcctatga agtcctcaag ctaaaaggat 360
ataccaactg ggctattgga ttaagtgtgg ctgatcttat tgaatccatg ntgaaaaatc 420
tatccaggat tcatcccgng tcaacnatgg tnaaagggga atgtatggca ttggagaaat 480
gaanctttcc tngncccttc cntgnatccc ncaanggncc cggggattna acnagcggtt 540
ttnaancccn aanctttaag ggnggg 566

<210> 456

<211> 559
<212> DNA
<213> Homo sapiens

<220>
<221> misc_feature
<222> (1)...(559)
<223> n = A,T,C or G

<400> 456
ggtcctggcc tcagcccgcc acatcaccct gacctgctta cgcccagatt ttcttcaatc 60
acatctgaat aaatcacttg aagaaagctt atagcttcat tgcaccatgt gtggcatttg 120
ggcgctgttt ggcagtgatg attgcctttc tgttcagtgt ctgagtgcta tgaagattgc 180
acacagaggt ccagatgcat tccgttttga gaatgtcaat ggatacacca actgctgctt 240
tggatttcac cggttggcgg tagttgaccc gctgtttgga atgcagccaa ttcgagtgaa 300
gaaatatccg tatttgtggc tctgttacaa tggtgaaatc tacaaccata agaagatgca 360
acagcatttt gaatttgaat accagaccaa agtggatggt gagataatcc ttcatcttta 420
tgacaaagga ggaattgagc caacaattgn atgttggatg gtgggttgca tttggtttac 480
tggatactgg catagaaagt ggtnctggga gaaaaaccta tgggggcaga ncntttttta 540
agcctggcca ananaggnt 559

<210> 457
<211> 552
<212> DNA
<213> Homo sapiens

<220>
<221> misc_feature
<222> (1)...(552)
<223> n = A,T,C or G

<400> 457
gttacgacaa aatttaagag gaataacaaa tacaaatttt ctgttaagaa cggaaaggtg 60
caaactagca gagtcaatac tggtaaccag aaggcactaa tccaaacaca taaatttcaa 120
aagctggtta tattatggaa taccatatat actggccttt gccagtttgg gatttctgca 180
atagcaataa gcctcgtttc tgtttccaat tataacaaca aaaagatgag ttactaatga 240
acattccact acagaagtct aggctatgtt gataaattga aaacttatct agactactct 300
gtctaagagc aataaaaagt aaacactctt ttatccagca gcactaggaa acagggtgaa 360
tttaccaaga taaattaggt tggggatacc tactgccaac ttgtgcggtt gtcgaattca 420
ctgnaatatg tattcctctt attgatagag ctcttgaatg naaaccacct anaagtgagg 480
ggaaaagctt caggatcatg gnccacaatt atgntatagn gcttttngng ggtngagccn 540
aaccccgntn cc 552

<210> 458
<211> 561
<212> DNA
<213> Homo sapiens

<220>
<221> misc_feature
<222> (1)...(561)
<223> n = A,T,C or G

<400> 458
accccaacaa tcttcaagcc acagtccaag agaagtctca ggaaagcaga cgtagaggaa 60
gaatccttag cactcaggaa acgaacacca tcagtaggga aagctatgga cacacccaaa 120
ccagcaggag gtgatgagaa agacatgaaa gcatttatgg gaactccagt gcagaaattg 180
gacctgccag gaaatttacc tggcagcaaa agatggccac aaactcctaa ggaaaaggcc 240
caggctctag aagacctggc tggcttcaaa gagctcttcc agacaccagg cactgacaag 300
cccacgactg atgagaaaac taccaaaata gcctgcaaat ctccacaacc agacccagtg 360
gacaccccag caagcacaaa gcaacggcca agagaaacct caggaaagca gacgtagagg 420
aagaattttt agcactcagg aaacgaacac catnagcagg ccaagccntg gncaccccaa 480
aaccngcngt nagtggttga gnaaaaattt cncccanttt tgggnaactt ccggngcaaa 540
nttnggcccn tntttggnaa a 561

<210> 459
<211> 468
<212> DNA
<213> Homo sapiens

<220>
<221> misc_feature
<222> (1)...(468)
<223> n = A,T,C or G

<400> 459
ggtacctcga catcctgaac actggataaa aaagttgatt aaatccagaa gtgcgatgtc 60
cctgtcttgt ttatatgatt caatccagtc atccaccacg gactgcattg cacttttccc 120
cagtttcacc acctcaaata atgtgacagg ctccccttcc ccattctgtt gagggtgtcc 180
attagctctt ccacggcctg ctcctctaat tccagcttca attctgctct tctcacctgg 240
agattttcga ggtttcttat ttgtagatgg aggccggcca ggacgacccc tttttctttt 300
tcctttgacc tctgtttctt caagctcgct gccagcatcg gaatgggcag tagtttcatt 360
agttgaatcc tgtaacactg gtaattctga agtaatcatt gctggagagg cctttcacaa 420
tgcagcaaaa taatcaagtg ctgnacctgg ccgggccggg cgctcgaa 468

<210> 460
<211> 566
<212> DNA
<213> Homo sapiens

<220>
<221> misc_feature
<222> (1)...(566)
<223> n = A,T,C or G

<400> 460
   acttcttgca tgttgtcaca tgttgctgtg agaatcaggt gctgcctata tggctccact 60
   gggagagggc agatggaagc cgtcgcctca tctgtcgtgg aacgtgtgct gtgcacctcc 120
   tccctttgct gatcttaatc tctgtccttt tactgtaata aactgtaact gtgagcctaa 180
   cagctttcct gagtctagtg agtccttcta gcaaatgaaa ggagggtggt cttggagacc 240
   tatgaacttg cacctgcccc cgtcgttttg aggtctggca cagggaggga ggctggtctc 300
   tttggagggg gtcttcatcc attggggtcg ggtccaactc tggaggccca cgtccttgcc 360
   agctccagtc tctctcccct ctcagtcccg acgctgtcac cttgtgccct ctgtctgtgg 420
   atcctgggaa gagctgntct ctctgctcac agctgaatan gagacatgcc cattagctga 480
   ggcgcttgca tgcttgtact actcgattgn caaangtnca agngntccca nnncnccccg 540
   ggtctatgga naannggggg gnanan 566

<210> 461
<211> 570
<212> DNA
<213> Homo sapiens

<220>
<221> misc_feature
<222> (1)...(570)
<223> n = A,T,C or G

<400> 461
ggtactatag catagcctgc ctttgctggt gtgtggcgat taggcctggt ggaactgcca 60
tcaataaatc aagcgtgatc agggtgagga acagggaaga aggaaatgtg gggaaatggg 120
atgaacatca ggtggatcac agagatgcag tcatgggggt caggtgtggt atccggaata 180
atgtgggagg ctggattgaa gtccgggcca ggaacaatgg taattgtggg acttaacaaa 240
aagtgagaac agctgaagga gtcagggagc agaaagtata tgcgtcaggt gtgaggaaga 300
aaatagattt tggaagttat gagaaatgta gagagtgagt tgagcatagt ttgtgatttt 360
gagggcctct aatagtatta aagcagtggc agcccgctac accgcagaca tganggctag 420
gctaaaacag taagggccaa gttgtttgca cagaaaggct tcagggtgcc ggtcctggct 480
cttgggtaag aattttggac cggacttaac catgcctaag gaaggggaag gagttgtngt 540
tttgtnaggg gacccaggtt tgggaaaann 570

<210> 462
<211> 573
<212> DNA
<213> Homo sapiens

<220>
<221> misc_feature
<222> (1)...(573)
<223> n = A,T,C or G

<400> 462
cgaggtacca ccagtatatg gaatgttagg gaaaaacttt gttccagttc cttttttttt 60
tctttctact ttcaagttta agtgaaccat actgaaatga ccaacaagtc tgcctgtaaa 120
gttacatgtc atgattgtgt tgttaaatga ttatggggga gaaaatgaag taaatgttgc 180
tgatgatccc catatttatt gatcatatta aggttgttta tatagtttgg aaatgaccag 240
ccccctaagc agtgtttgat taacttatgc taatcagatg attactcata tattctgcta 300
attttctagc tttattcttg ttatttggaa aaattattag ccaaatgcct tcctaggtgg 360
atccagttgg aagatatgtc cagaaacctg aagaaaaatt gacgctgcct ttgtgtgctg 420
gattgctcta cttgattaga tcatgatata tcaaggntga atttttagag ggaaaattaa 480
ttctgatatc ttattggatc ccttgataag ntttttcctg gatttttttt tttccccaaa 540
gaattttca tttgngncct ngcccggcgg gcc 573

<210> 463
<211> 574
<212> DNA
<213> Homo sapiens

<220>
<221> misc_feature
<222> (1)...(574)

<223> n = A,T,C or G

<400> 463
accatatcct gtgtttgaat caaacccgga gttcttctat gtggaaggct tgccagaggg 60
gattcccttc cgaagcccta cctggtttgg aattccacga cttgaaagga tcgtccacgg 120
gagtaataaa atcaagttcg ttgttaaaaa acctgaacta gttatttcct acttgcctcc 180
tgggatggct agtaaaataa acactaaagc tttgcagtcc cccaaaagac cacgaagtcc 240
tgggagtaat tcaaaggttc ctgaaattga ggtcaccgtg gaaggcccta ataacaacaa 300
tcctcaaacc tcagctgttc gaaccccgac ccagactaac ggttctaacg ttcccttcaa 360
gccacgaagg gaagagaggt ttncttttga ggcctggaaa tgcccaaaat cacnggcctt 420
aaaacaggaa ggttggaaaa tctctttcaa tgagaaaatg tggggnaact cttgggcctt 480
aaacaagctg tgaaaggtgc ccggtcccgg taatttgggg ccttttcccg gaagacnttt 540
ttgtggaaag gnttacctga ngggggggcc cttt 574

<210> 464
<211> 458
<212> DNA
<213> Homo sapiens

<400> 464
ggtactgccg ctcggagatc tttacttgtt tttactttga acatgagcag agaaaagaca 60
aagaaaaaga tggccatggc aaagctgatc cgatacacag ctttataacc aaccagcaca 120
tcacaatctt tatctgcatt tatatcagcc tcatggattt taaatccccc ttcacaaaat 180
ccaggaatct tcttcaagta agtttccatc tcttttctct gcatgatata ggatacgaca 240
gtgctcagga ggagaatgaa agcataaatg aggcgagtca ccgtggaatt cttactgtta 300
ggacagcaac tacacagcaa acatgaggca ccgctgcaga ggcatggaac ccagctggcg 360
agggagaaga cacccagcac agcccccatg gtgacgccag tgatggaggt ggccggtcct 420
gaggctgctt tctaacacgg tggtaactgc cagctgag 458

<210> 465
<211> 580
<212> DNA
<213> Homo sapiens

<220>
<221> misc_feature
<222> (1)...(580)
<223> n = A,T,C or G

<400> 465
gcggccgang tacttcacca tcactgactc catggacttg atcagccgnc gctggatgta 60
tncagtctca gnagtnttga cagccgtgtn aatgagcccc tcacgacccc ccatggngtg 120
gaaaaagaac tcagtgggtg tgaggccggc taggtaggag ttctncacaa agccacggct 180
ctnaggcccg tagtcatcct tgatgaagtg aggcagagtc cggtgcttga agccaaatgg 240
aatccgcttg ccctcgacgt tctgctgtnc aacgacagcg atnacctggg agatgttaat 300
cttggaacct ttagctccgg acacgaccat anacttgaag ttgttgtatt canacaggga 360
tttctgagca gaggagccag tcttgtctcg ggcatcgtta agaatgcggg tcacctgatt 420
ctcaaacgtc tgncgcagan tggtccctgg ggngggctcc agctcattgt tgngngnctt 480
cttnatgacc tctantacgt cctgnttggg gcttttaana gggcctgaat gncccgggaa 540
ggnnttanaa ttncnatggg gttcccaagg ccanacttnn 580

<210> 466
<211> 566

<212> DNA
<213> Homo sapiens

<220>
<221> misc_feature
<222> (1)...(566)
<223> n = A,T,C or G

<400> 466
caagcctttt tttttttttt tttttttttt gggcatgcct gtgttgggtt gacagtgagg 60
gtaataatga cttgttggtt gattgtagat attgggctgt taattgtcag ttcagtgttt 120
taatctgacg caggcttatg cggaggagaa tgttttcatg ttacttatac taacattagt 180
tcttctatag ggtgatagat tggtccaatt gggtgtgagg agttcagtta tatgtttggg 240
attttttagg tagtgggtgt tgagcttgaa cgctttctta attggtggct gcttttaggc 300
ctactatggg tgttaaattt tttactctct ctacaaggtt ttttcctagt gtccaaagag 360
ctgntcctct ttggactaac agtaaattta cnagggggat ttaaagggtt ctgggggcca 420
aatttaaagg ttgaactaag aattctatct tggaccaacc agnttttcac cangcctcgg 480
gaaggtttgg ccgcctntac ctattaaact tncccctatt ttgggaccta naccgggngg 540
ggctcctttt aacngggcnt aagggg 566

<210> 467
<211> 597
<212> DNA
<213> Homo sapiens

<220>
<221> misc_feature
<222> (1)...(597)
<223> n = A,T,C or G

<400> 467
gcgtggtccg gccgaggtac gtgatgccct tacagctgaa aaatccaaga ttgagacaga 60
aatcaagaac aagatgcaac agaaatcaca gaagaaagca gaacttcttg ataatgaaaa 120
accagctgct gtggttgctc ccattacaac gggctatacg gtgaaaatca gtaattatgg 180
atgggatcag tcagataagt ttgtgaaaat ctacattacc ttaactggag ttcatcaagt 240
tcccactgag aatgtgcagg tgcatttcac agagaggtca tttgatcttt tggtaaagaa 300
tctaaatggg aagagttact ccatgattgt gaacaatctc ttgaaaccca tctctgtgga 360
aggcagttca aaaaaagtca agactgatac agttcttata ttgtgtagaa agaaagtgga 420
aaacacaagg tgggattacc tgacccaggt ttgaaaangg agtgcaaaga aaaaggagaa 480
gcccttncta tgacactgga accagaatcc tngtnagggg attgatgaaa ggtcttaaga 540
aaaatttttg aagaangnga cattgatttt gaagcgnacc ctttattnan gcttggg 597

<210> 468
<211> 562
<212> DNA
<213> Homo sapiens

<220>
<221> misc_feature
<222> (1)...(562)
<223> n = A,T,C or G

<400> 468

ggtactggat aaagggctga catcaagagc aaacagaagt cttttcctag tgcatatgca 60
aactggccaa ttccttccaa ctgaatgcat atttgccaga tgttactgtt catggagcaa 120
atagtgggac ttggctttga gaaggctaga aaagatgtaa cttggtaggt gtgttcacca 180
gacgtgatgg cttggaggcc tgggtgctcc atcatcagct cctctcccat ttcctcagtt 240
tcaagacagg taaccaaata ccaattttct tgacttgtgt attcttcaag tatagatgtc 300
acaatctctc tcagttcttc tgggtttgtt ttaatatgtt tttcgtgaag atcctcaacc 360
tccagcccag cagcccctgt aaccagttca ttaaggatca tggcagcttg cttccggtaa 420
accacagatt gatggtaaag ttccataaag tgatccacaa gcnaataaaa gattnccata 480
ataaccaagt agcttgacaa acctggctna agagcntgaa gaatctctta tccgtgaaga 540
aaccggaata tcttctntng gg 562

<210> 469
<211> 533
<212> DNA
<213> Homo sapiens

<220>
<221> misc_feature
<222> (1)...(533)
<223> n = A,T,C or G

<400> 469
cgaggtacca ataccaccaa ttttgtagac atcctggaga ggcaggcgca agggcttgtc 60
agttggacga gttggtggta ggatgcagtc cagagcctca agcagcgtgg ttccactggc 120
attgccatcc ttacgggtga ctttccatcc cttgaaccaa ggcatgttag cacttggctc 180
cagcatgttg tcaccattcc aaccagaaat tggcacaaat gctactgtgt cggggttgta 240
gccaattttc ttaatgtaag tgctgacttc cttaacaatt tcctcatatc tcttctggct 300
gtagggtggg ctcagtggaa tccattttgt taacaccgac aattagttgt ttcacaccca 360
gtgtgtaagc cagaagggca tgctctcggg tctgccattc ttggagatac cagcttcaaa 420
ttcaccaaca ccagcagcaa caatcaggac agcacaagtc aggctgagat gtcctgnaat 480
catgnttttg ataaagctct gggtcctggg ccatcaatga tagccatagt acc 533

<210> 470
<211> 672
<212> DNA
<213> Homo sapiens

<220>
<221> misc_feature
<222> (1)...(672)
<223> n = A,T,C or G

<400> 470
ggtacaccat ataaacagca gatgaagtcg gagagatagt ctaatacact tagatcatgt 60
tccaccacaa tgatatatct atctggattt attagagatc gtatagtaat agcagccttt 120
aaacgctgct tgacatctag gtaactagaa ggctcatcaa acatgaaaat atcagctttc 180
tgtatgcaaa cgacagcaca agcaaatctc tgcaactctc ctcctgaaag atcttcaaca 240
tttcgttctt ttaggtgggt taaatcaagc tgctgacata caattgcctg tgtctttgtt 300
tcatcttttc ggtccaaaat agatcccact gtcccctttg cagccttagg aatctggtct 360
acatattgag gtttgatgat ggcttttagg tcatcttcta gaatctttgg aaagnaattt 420
tgnaattcag atccacngaa ataagtcaaa atcttctggc agtcaaggan gatcatcgga 480
cctgncccgg ccggccgntt cgaaaggcca aattccagca cacttggccg gccggtactt 540
agnggaatcc nagcttcggg ancccangcn ttggcgnnaa tcatngggca taactgggtt 600

ccctgggggg aaaaatggta atcccggtta ccaanttcnc cccnacatac cnaacccgga 660
agccttanan gg 672

<210> 471
<211> 387
<212> DNA
<213> Homo sapiens

<400> 471
cgaggtgagc tttgaaacaa ctgatgagag cctgaggagc cattttgagc aatggggaac 60
gctcacggac tgtgtggtaa tgagagatcc aaacaccaag cgctccaggg gctttgggtt 120
tgtcacatat gccactgtgg aggaggtgga tgcagctatg aatgcaaggc cacacaaggt 180
ggatggaaga gttgtggaac caaagagagc tgtctccaga gaagattctc aaagaccagg 240
tgcccactta actgtgaaaa agatatttgt tggtggcatt aaagaagaca ctgaagaaca 300
tcacctaaga gattattttg aacagtatgg aaaaattgaa gtgattgaaa tcatgactga 360
ctgagacctg cccgggccgg ccgtcga 387

<210> 472
<211> 241
<212> DNA
<213> Homo sapiens

<400> 472
ggtacgaatc gtctcctggc actgtgcagg cccacagctg acggcgatga cctccttcac 60
cagcttcttc tccttgagcc gcacagcctc ctccaccgcg atctcacaga aggggttcat 120
ggagtgcttc acaccatccg tgaccacacc ggtcctgtca ggcttcactc ggatcttcac 180
ggcgtagtcg atgaccctct tgacagctac gagcacgcgc agctccgcca tcttcccgcc 240
g 241

<210> 473
<211> 470
<212> DNA
<213> Homo sapiens

<400> 473
ggtactagtt cactatcggt gtctgattag tatttagcct taccgggtgg tcccggcaga 60
ttcagacagg gtttcacgtg ccccgcccta ctcaggatac atctatgaga ttttatgatt 120
tcgtatacag gaatatcacc ttctatgttg aagctttcca acttcttcta ctatcataaa 180
attttgtaac tcaatgtaag atgtcctaca accccttttt acaggtttgg gctctttcgc 240
tttcgctcgc cactactgac gaaatcatta tttattttct tttcctgttg ctactaagat 300
gtttcaattc gcaacgtgtc tcgctaattt gactatggat tcatcaaaat gcaactgagg 360
tttgctcagt taggttaccc cattcggaaa tctccgtatc atagtttatt tccaactcca 420
cgaagcttat cgcaggtaat cgcgtccttc atcgactttc agacccaagg 470

<210> 474
<211> 637
<212> DNA
<213> Homo sapiens

<220>
<221> misc_feature
<222> (1)...(637)
<223> n = A,T,C or G

<400> 474
acctcttcct gataagattg aagtaaaaac tggtgaggaa gatgaagaag aattcttttg 60
caaccgcgcg aaattgtttc gtttcgatgt agaatccaaa gaatggaaag aacgtgggat 120
tggcaatgta aaaatactga ggcataaaac atctggtaaa attcgccttc taatgagacg 180
agagcaagta ttgaaaatct gtgcaaatca ttacatcagt ccagatatga aattgacacc 240
aaatgctgga tcagacagat cttttgtatg gcatgccctt gattatgcag atgagttgcc 300
aaaaccagaa caacttgcta ttaggttcaa aactcctgag gaagcagcac tttttaaatg 360
caagtttgaa gaagcccaga gcattttaaa agccccagga acaaatgtag ccatggcgtc 420
aaatcaggct gcagaattgt aaagaaccca caagtcatga taacnaggat atttgcaaat 480
ctgatgctgg aaacctgatt ttgaatttca ggntgcaaga aagaaagggc ttggtggcat 540
tgaaccactg ntcattaaga atgcttcact gctaaaaatg ngattatgcc aaattaancc 600
agcaataaga ctcgtggccc ccttaactga actgttt 637

<210> 475
<211> 647
<212> DNA
<213> Homo sapiens

<220>
<221> misc_feature
<222> (1)...(647)
<223> n = A,T,C or G

<400> 475
ggtacaagcc atagtggaaa gaatgaatct ctccctaaaa tagcagttgc aaaagcagaa 60
agggggagac agagaatatg gaaccccaca gatgcaactg aacctagcat tattaacagt 120
aaattttttg agcctgccca aaggccacat gttatcagca gctgaagagc atctacagaa 180
accagctgca aggacaaaaa cagaacaact gatttggtgg agagatccga taacacgaag 240
ttgggaaata ggtaaaataa taacttgggg gagaggttat gcttgtgttt ctccaggcca 300
atatcaatag cctatttgga taccatcaag acacctgaaa ccttatcgtg agccagatgc 360
tgaggaatag actccgggag ggatcctgag aaccccccag ttgcagccat gtttgagact 420
gatgctgagg aggactccaa ctgtcacgag cacagccccc atctggggac agatcaagaa 480
gctgtcacag atggaagaag aaaaccttga ggaaagcagg acaatcggtc ccatgagtaa 540
aatctgatgg tagctataaa ccggttttan cacnccatgn tattctttng ttaaggctga 600
cncngagaac aattatacct antggggata tttatcatct tggtngg 647

<210> 476
<211> 665
<212> DNA
<213> Homo sapiens

<220>
<221> misc_feature
<222> (1)...(665)
<223> n = A,T,C or G

<400> 476
accttattag aaagcgacgg caaactatgt gccagcagcc gcggtaatac ataggtcgca 60
agcgttatcc ggaattattg ggcgtaaagc gtccgtaggt tttttgctaa gtctggagtt 120
aaatgctgaa gctcaacttc agtccgcttt ggatactggc aaaatagaat tataaagagg 180
ttagcggaat tcctagtgaa gcggtggaat gcgtagatat taggaagaac accaataggc 240
gaaggcagct aactggttat atattgacac taagggacga aagcgtgggg agcaaacagg 300

attagatacc ctggtagtcc acgccgtaaa cgatgatcat tagttggtgg aataatttca 360
ctaacgcagc taacgccgtt aaatgatccc gcctgagtag tatgctcgca agagtgaaat 420
ttaaaggaat tgacgggaac ccgcacaagc cggtggaaca tgtgggttaa tttgattcta 480
cgccgtagaa ccttacccac ttcttggaca tcttctgcaa agctatngga gatatagtgg 540
anggttaaca gaatggcccg aaggtgcatg ggtggccgca gctcgtgtcg tgagaaggta 600
nggtnaagtc ctgnaacgag cgccaaccnt ttctttagta ctaatattaa gttaaggact 660
ntagn 665

<210> 477
<211> 319
<212> DNA
<213> Homo sapiens

<400> 477
cgaggtactt ttcaattatg ttaacgtaaa atactcgtaa cgaatgtagt atgagtttaa 60
agtgagcttt tcagatccta taagtgcatc ctaagtaatg acaggcttta agataaggaa 120
tatatgcatt ttgttaaggc agaaatctca taaaatttca tgaaaaacca tggtcaatcc 180
aatgatgcac tttttaagac aagtttgtct ggaaactgga agggtcaaaa gacaacaaaa 240
aagcacacac caaaaaacct cactttaagc aaatctataa cttgaaaaaa aaaaagccta 300
agaatattct gagagtggt 319

<210> 478
<211> 419
<212> DNA
<213> Homo sapiens

<400> 478
acccacgatg atgtggggag cttccatctg cagtttctgc acctcagcac gcacgttggt 60
gcccccgata caggcgtgac aggaggcgcc catgtagtct cctagtgcca tgaccacctt 120
ctgtatctgc tgagccaatt ctcgagtggg tgctaggact aaggcctggg tggcttttag 180
atctaattca atctgctgca gaatcgatat ggcaaatgtg gccgttttcc cagtcccaga 240
ttgggcttga gcaatcacat cataaccctt gatacaaggt agaatgggct cgctgctgga 300
tggcagaggg cttctcaaaa ccataggcgt agatgccacg gagaagggac tccgagaggt 360
tcatgtcatc aaagctgtca acaatctcat tccagttact ctcgatgacg ccttcgacc 419

<210> 479
<211> 312
<212> DNA
<213> Homo sapiens

<400> 479
acatcctgga gacctgaaga attctgttga agtcgcactg aacaagttgc tggatccaat 60
ccgggaaaag tttaataccc ctgccctgaa aaaactggcc agcgctgcct acccagatcc 120
ctcaaagcag aagccaatgg ccaaaggccc tgccaagaat tcagaaccag aggaggtcat 180
cccatcccgg ctggatatcc gtgtggggaa aatcatcact gtggagaagc acccagatgc 240
agacagcctg tatgtagaga agattgacgt gggggaagct gaaccacgga ctgtggtgag 300
cggcctggta cc 312

<210> 480
<211> 640
<212> DNA
<213> Homo sapiens

<220>
<221> misc_feature
<222> (1)...(640)
<223> n = A,T,C or G

<400> 480
ggtaccaaca attcctccta ccagtggctg agcatactct gcagagtcag cctgcagcac 60
tgtggtgact tctcttggac tcaggtgatt aacttcgctg ctgctatagc gaactggggt 120
ttcctcatgg tccactgctt ttgcaggaag aaactgcttc attcctttcc accaacctgc 180
ccggccccag taaggtaagt cataggtgcc ttcagttttt ttctttctgt ttctccagtg 240
ccaagcacac actaatatga gaatgagagt agtgaggacc atgaccagca cagggacaag 300
aactgcagcc agcgctacat ctttggttac atttggagtt acggtagtat ttctgatatc 360
aggactggca gttgtttgtt ctgtctgtgc aggaaattca ttgctactgc gaagttgtag 420
tggttgcgta aattttgggg cacgaccttt ggctattttg gaggggctgt agtggttttg 480
aggncattgc tgttncnaag aggtggaggt tgagtaagtt ttggangacn actttangaa 540
taaactgaca tccgagcagt tcattttcat ggcaatttct gctgccatgg gtaaggatta 600
ctctaataaa cgtgccataa ttggtggcaa aagtattccc 640

<210> 481
<211> 501
<212> DNA
<213> Homo sapiens

<400> 481
ggtacatttc cttgtagact ctgttaattt cctgcagctc ctggttggtt ctggagcaga 60
tgatctcaat gagagagtcc tcgtcggttc ccagcccctt catggaagct tttagctcag 120
aagcgtcata ctgagcaggt gtcttcaata ggcccaaaat caccgtctcc aggtggccag 180
ataaggctga cttcagtgct gatgcaagtt cctttttggt ccttctctgg taggcgaagg 240
caatatcctg tctctgtgca ttgctgcggt tggtcaaaat gttgacaatg gtgacctcat 300
ccacaccttt ggtcttgatg gctgtttcaa tgttcaaagc atcccgctca gcatcaaaag 360
ttagtatagg ctttgacaga cccatatgca cttgggggtg tagagtgatc accctccaag 420
ctgagcttgc acaggatttc gtgaacagta agacattttg aaaggaagct gggcccgtgc 480
gcccgagagc tgaaagcgtc c 501

<210> 482
<211> 306
<212> DNA
<213> Homo sapiens

<400> 482
ggtacctata cagggatggc tcccacgcat ccctcagtga ccccaaaccc atctccactt 60
acactcaggc actcccagga cctgacagct actccccgtt atcgtccttc agttcgaagc 120
cctggccaat ctaccagccc acatgacgca gttacctggc catttctcca cggttcccgt 180
gagggcccca cacccagccg cacaagagcc cctcctgcat tccgtcctca cacacaggcc 240
tgtgtatgca cttgctactg tcacactctt gctagcagaa gaggcccctg taatggccga 300
tatccc 306

<210> 483
<211> 663
<212> DNA
<213> Homo sapiens

<220>

<221> misc_feature
<222> (1)...(663)
<223> n = A,T,C or G

<400> 483
acagaatttc ttatttcttg aagactctgt ggttgaccac ttcttcatta gttacctgca 60
gcaagacacc ttccatttta ctaccaacac cactgaagga accaagaaaa gctttattaa 120
tgatcacttg gcttgcctca gctgttgaaa tgaagcactt tacagtcttt gtggcagcag 180
aatatacttg tccatggttc atatcaatgc catggcaaat aggaagaagc tcagtatcgg 240
ctcctcccac cataaccccc acttcctcca ctgcctcctg gaccatagtt tcctccacca 300
tatggtcccc ccatgttcct gctaccacca aagtttccac tcttcacacg ggccaagtca 360
gaaagaccat gacataaaga gagatggcga aactgaaacg gattatttct tttgncttca 420
aaacatctca tcaatttatc actcatccat tctacctggg acttagaaaa ctccaccaca 480
ttgtaactga cattatttag gagtgccaat gagtaaacac ccaatcctgn atctttagtc 540
cctccaaatc tggatccaag aagtttagcc aggttccaaa cttntggctg ntgggggcca 600
ctgntattaa cacattttca ttancttgaa nnggttccag gacanttggc anaacttgtt 660
ant 663

<210> 484
<211> 228
<212> DNA
<213> Homo sapiens

<400> 484
cttgggtctg aaagtcgatg aaggacgcga ttacctgcga taagcttcgt ggagttggaa 60
ataaactatg atacggagat ttccgaatgg ggtaacctaa ctgagcaaac ctcagttgca 120
ttttgatgaa tccatagtca aattagcgag acacgttgcg aattgaaaca tcttagtagc 180
aacaggaaaa gaaaaaaaaa aaaaaaaaaa aaaaaaaaag cttgtacc 228

<210> 485
<211> 672
<212> DNA
<213> Homo sapiens

<220>
<221> misc_feature
<222> (1)...(672)
<223> n = A,T,C or G

<400> 485
acggagccct ctgaaaaatg acaaagatgg tatgatgtat ggcccaccag tggggactta 60
ccatgacccc agtgcccagg aggctgggcg ctgcctaatg tctagtgatg gtctgcctaa 120
caagggcatg gaattaaagc atggctccca gaagttacaa gaatcctgtt gggatctttc 180
tcggcaaact tctccagcca aaagcagcgg tcctccagga atgtccagtc aaaaaaggta 240
tgggccgccc catgagactg atggacatgg actagctgag gctacacagt catccaaacc 300
tggtagtgtt atgctgagac ttccaggcca ggaggatcat tcttctcaaa accccttaat 360
catgaggagg cgtgttcgtt cttttatctc tcccattccc agtaagagac agtcacaaga 420
tgtaaagaac agtagcactg aagataaagg tcgccttcct tcactcatca aaaagaaagg 480
cgcttgatta aagcatttca atttcctatg gccccatctt ttnttcacag gtccngggat 540
antcaaggtc tattncctta agaagagaat tnccttccan gggncctttc cnaggtcccc 600
aatagtttna aaaactggnc ctggtnggta ancctttann aaagcccttg gttaaaancc 660
cnaaananng ng 672

<210> 486
<211> 637
<212> DNA
<213> Homo sapiens

<220>
<221> misc_feature
<222> (1)...(637)
<223> n = A,T,C or G

<400> 486
ggtacaatag agctttggat ctgatacaag aatttagaaa tataaaacaa aataactata 60
aaagttagga ggcatttgaa tggcatttcc ttagaagaac ctgctaactc tgtatcattc 120
tgatgtggat tcctagtcat gtggggtgaa atgcatattt ttcccccttt gctggatcac 180
tggcctttct tcaaaagcta taatgccatg aacacacatc ctaggagtct ctataatgtt 240
aacagaagct ccaaatacca agccaatcaa agatgggaga gggcagggga accataaagg 300
cgaagggtcc aaaggtggct gttactgaga acttgccctt tccaaaatgt gaaagtcata 360
gtgcttcttg cttgttctca gcttaaactt gttaactgag ttaatttgtt tcttcagtgc 420
attctgtgca gctgaaatgg aggggaatgt ggctaagacg gtgtangtgg angccaagtc 480
actgggttta gaaccgttca agggttggca gtggtggncc ccactggcca cagcagaagg 540
ggttgaccac cctgggttgg gactgggggg tncccggann cccccggatn ttggngccca 600
attttaaaga agttncccca aaaacttttt aacttng 637

<210> 487
<211> 618
<212> DNA
<213> Homo sapiens

<220>
<221> misc_feature
<222> (1)...(618)
<223> n = A,T,C or G

<400> 487
ggtacctctt cccatgactg cacccagctc caggggccct tgggacagcc agagctgggt 60
ggggacagtg ataggcccaa ggtcccctcc acatcccagc agcccaagct taatagccct 120
ccccctcaac ctcaccattg tgaagcacct actatgtgct gggtgcctcc cacacttgct 180
ggggctcacg gggcctccaa cccatttaat caccatggga aactgttgtg ggcgctgctt 240
ccaggataag gagactgagg cttagagaga ggaggcagcc ccctccacac cagtggcctc 300
gtggttatta gcaaggctgg gtaatgtgaa ggcccaagag cagagtctgg gcctctgact 360
ctgagtccac tgctccattt ataaccccag cctgacctga gactgtcgga gaggctgtct 420
ggggccttta tcaaaaaaag actcagccaa gacaaggagg tanagagggg actgggggac 480
tgggagtcaa aacccctggc tggggttaag tccacgtntg gcnagcactg gctttttctt 540
ttgggccttg gttccttgtg ggcaaagaat gatgaccnct attttcagga cttttccttc 600
ngttncaagg tttttntg 618

<210> 488
<211> 618
<212> DNA
<213> Homo sapiens

<220>
<221> misc_feature

<222> (1)...(618)
<223> n = A,T,C or G

<400> 488
ggtacagtcg tctgaagaag ctctgagggc ggcaggacca gccagcagca gcccaagctt 60
ccctccatcc ccctttaccc tctttgctgc agagaaactt aagcaaaggg gacagctgtg 120
tgacatttgg agagggggcc tgggacttcc atgccttaaa cctacctccc acactcccaa 180
ggttggagcc cagggcatct tgctggctac gcctcttctg tccctgttag acgtcctccg 240
tccatatcag aactgtgcca caatgcagtt ctgagcaccg tgtcaagctg ccctgagcca 300
cagtgggatg aaccagccgg ggccttatcg ggctccagcc atctcatgag gggagaggag 360
acggagggga gtagagaagt tacacagaaa tgctgctggc caaatagcaa agacaacctg 420
ggaaaggaaa ggtctttgtg ggataatcca tatgttaatt attcaacttc atcaatcact 480
ttatttattt tttttctaac ttcttggaga cttaatttac tgntttatta gggtgaaaac 540
tggcnttcta ngtagggttt tnttatccca ggactacctt gggttttaan ttaaaaaaaa 600
aaagaaatgg ntnaaaaa 618

<210> 489
<211> 624
<212> DNA
<213> Homo sapiens

<220>
<221> misc_feature
<222> (1)...(624)
<223> n = A,T,C or G

<400> 489
   naggtnctga tgattctcca natccangta tagaatatga ncncgnnctn cgaaantggn 60
   gtganttgat tcctggggct gagtatcgat gtttatgnca tggaaaacna gcttattggt 120
   atttctcaga gagactacac acaatactat gatcatattt ctaaacagna ggaagaaatt 180
   cgcanatgca tacaagactt tttcaagaaa cacatacagt acaagctttt ntnctattta 240
   attgntgtnt ttttttgtgg taacnngaaa gtttattnnt gtctgaaagc ttttataagt 300
   atttaaatnn acnnagtaat gaactattca attgctgnaa tcggtcaaaa tttncnaaag 360
   ncgcacacaa antnntatcc ttgnncacgn anctncatac actgnccctn gccaaacacc 420
   cttgccggga accaatcngc atgacatttc tgggccggtt aaatnttata aagccaaggg 480
   cccnggcact ggttaaggng ggccttanac cttttagggg agggcccnaa taccctnccn 540
   cttaaacntc tggggggngg tananatttc ttataggnac cgncccttta aatcnattgn 600
   canttttnng nccctttggt tttt 624

<210> 490
<211> 620
<212> DNA
<213> Homo sapiens

<220>
<221> misc_feature
<222> (1)...(620)
<223> n = A,T,C or G

<400> 490
ggtacctctt cccatgactg cacccagctc caggggccct tgggacagcc agagctgggt 60
ggggacagtg ataggcccaa ggtcccctcc acatcccagc agcccaagct taatagcccc 120
ccccctcaac ctcaccattg tgaagcacct actatgtgct gggtgcctcc cacacttgct 180

ggggctcacg gggcctccaa cccatttaat caccatggga aactgttgtg ggcgctgctt 240
ccaggataag gagactgagg cttagagaga ggaggcagcc ccctncacac cagtggcctc 300
gtggttatta gcaaggctgg gtaatgtgaa ggcccaagag cagagtctgg gcctctgact 360
ctgagtccac tgctccattt ataaccccag cctgacctga gactgtcgga aggctgtctg 420
gggcctttat caaaaaaaag actnagccaa acaaggaggt agagagggga ctgggggact 480
gggagtcana gccctggctg ggttcangtc cacgttgggc aggcacttgc ttttcttttt 540
nggnctttgg ttccttgttg gcaaaagagt gattgaaccc cttattttca agggcttttc 600
nctnatgttn cangntttnn 620

<210> 491
<211> 630
<212> DNA
<213> Homo sapiens

<220>
<221> misc_feature
<222> (1)...(630)
<223> n = A,T,C or G

<400> 491
acatttcctt gtagactctg ttaatttcct gcagctcctg gttggttctg gagcagatga 60
tctcaatgag agagtcctcg tcggttccca gccccttcgt ggaagctttt agctcagaag 120
cgtcatactg agcaggtgtc ttcaataggc ccaaaatcac cgtctccagg tggccagata 180
aggctgactt cagtgctgat gcaagttcct ttttggtcct tctctggtag gcgaaggcaa 240
tatcctgtct ctgtgcattg ctgcggttgg tcaaaatgtt gacaatggtg acctcatcca 300
cacctttggt cttgatggct gtttcaatgt tcaaagcatc ccgctcagca tcaaagttag 360
tataggcttt gacagaccca tatgcacttg ggggtgtaga gtgatcaccc tccaagctga 420
gcttgcacag gaattccgtg aacagtagac attttgaagg aagcttnctt gaggcccaat 480
gtgttcaacc caaccgggaa aactnttncg ggtagaagtg aaatccgaag ttgctattgc 540
ttccagaata acctgggncn tnccccnaaa actttaaaac gttcccacct tgggcgggaa 600
cccncttaan gggggaattc ccgnccncng 630

<210> 492
<211> 412
<212> DNA
<213> Homo sapiens

<220>
<221> misc_feature
<222> (1)...(412)
<223> n = A,T,C or G

<400> 492
acactaccaa cagatcaaag aaacccctcc ggccagtgag aaagacaaaa ctgctaaggc 60
caaggtccaa cagactcctg atggatccca gcagagtcca gatggcacac agcttccgtc 120
tggacacccc ttgcctgcca caagccaggg cactgcaagc aaatgccctt tcctggcagc 180
acagatgaat cagagaggca gcagtgtctt ctgcaaagcc agtcttgagc ttcaggagga 240
tgtgcaggaa atgaatgccg tgaggaaaga ggttgctgaa acctcagcag gccccagtgt 300
ggttagtgtg aaaaccgatg gaggggatcc cagtggactg ctgaagaact tccaggacat 360
tatgcaaaag caaagaccan aaaaaaaaan nnaaaaaaaa aaagcttgta cc 412

<210> 493
<211> 633

<212> DNA
<213> Homo sapiens

<220>
<221> misc_feature
<222> (1)...(633)
<223> n = A,T,C or G

<400> 493
acactggcca gtgtgttttt ggcgattaaa cataatcctg tgaatcagat taattcactt 60
gctgagtgtt catttgcggc atccctctgt tgggtcttgg gggccctcca cgacctcgtg 120
gggctccccg tggtccactc tgcccagagc ctcgcttgaa attctgctga tatccatccc 180
gttgatagcc agagtaatcc cggggagcac tgaactgaga ctgtgtataa ccactgtttg 240
gagtgttaga gaatgaaggg cggtaaccat natatcctcc tctgaatcca ttggcagggc 300
cccggtatcc attcatcaag cctctagcac cacgggagcc ttcacgagac gcaccacgac 360
tattgtaata ggggctgatt gctacgtgga aatncagtgt tctgctgaag aagctgctgg 420
tgggtaccag tcacttgatg ggactggtct gggggaaccc atggtaaagt gcccaaccac 480
tggttgnaac ttgtcttgct tgaanctctg gttggtctac cttggggaag cttgactaaa 540
aaaacttttg gtataaattg ggctgggacc ccctangggn gcaaccctgg gccanntttt 600
tcctnannct taaaaagggg ggggnatgaa ggn 633

<210> 494
<211> 609
<212> DNA
<213> Homo sapiens

<220>
<221> misc_feature
<222> (1)...(609)
<223> n = A,T,C or G

<400> 494
acttaaaagg taaagtagta accaaagaga aaatccagga agccaaagat gtctacaaag 60
aacatttcca agatgatgtc tttaatgaaa agggatggaa ctacattctt gagaagtatg 120
atgggcatct tccaatagaa ataaaagctg ttcctgaggg ctttgtcatt cccagaggaa 180
atgttctctt cacggtggaa aacacagatc cagagtgtta ctggcttaca aattggattg 240
agactattct tgttcagtcc tggtatccaa tcacagtggc cacaaattct agagagcaga 300
agaaaatatt ggccaaatat ttgttagaaa cttctggtaa cttagatggt ctggaataca 360
agttacatga ttttggctac agaggagtct cttcccaaga gactgctggc ataggagcat 420
ctgctcactt ggttaacttc aaaggaacag atacagtagc aggacttgct ctaattaaaa 480
aatattatgg aacgaaagat nctgttccag ctattctggt ccacagcaga acacagtacc 540
ttggccgnga cnacnctaag gcgaaatccg ccactggggg gccgttataa nggatcccnc 600
ttnggaccn 609

<210> 495
<211> 606
<212> DNA
<213> Homo sapiens

<220>
<221> misc_feature
<222> (1)...(606)
<223> n = A,T,C or G

<400> 495
ggtaccaagc tatctttgat aataccacta gtctgacgga taaacacctg gacccaatca 60
gggaaaatct gggaaagcac tggaaaaact gtgcccgtaa actgggcttc acacagtctc 120
agattgatga aattgaccat gactatgagc gagatggact gaaagaaaag gtttaccaga 180
tgctccaaaa gtgggtgatg agggaaggca taaagggagc cacggtgggg aagctggccc 240
aggcgctcca ccagtgttcc tggatcgacc ttctgagcag cttgatttac gtcagccaga 300
actaaccctg gatgggctac ggcagctgaa gtggacgcct cacttagtgg ataaccccag 360
aaagttggct gcctcagagc attcagaatt ctgtcctcac tgataggggt tctgtgtctg 420
cagaaatttt gtttcctgta cctgccnggc ggncgctcaa agggcgaatt cacacactgc 480
ggccgtacta gtggatccaa ctcggaccaa cttggcgtaa tatggcatac tgtttctgng 540
ggaaatgtat ccgtccaatt cncccacata cganccganc ntaaaggtaa gcttggggcc 600
tataat 606

<210> 496
<211> 279
<212> DNA
<213> Homo sapiens

<400> 496
ggtactcaat gatgctggtc agcgacttcc acgggagaaa atcttgctga atgtccgtga 60
aatccttccc atatttttcc agggcttcct cgaaaaggtt ggcctctgat gcagaccact 120
cctccatctc gtccctgcag agcacgggcc cgccctgcgg caccagcgcc gagatggcct 180
tggagatgtc gtagatgttc ttgtggagag tatccatggc gtggaacagg gtgatgtctc 240
gggaggcagc tgcggcgctc atgtgcaggc tgggctgtc 279

<210> 497
<211> 633
<212> DNA
<213> Homo sapiens

<220>
<221> misc_feature
<222> (1)...(633)
<223> n = A,T,C or G

<400> 497
ggtacacaac agggcaaaag ctttttcgca agtcataaaa ttgagttgaa aataacttgt 60
tgattcagct acaggaagac aactaacaat taacaggctc atgaatattt atgaataaag 120
tgccactaat tttattgtaa taagatataa atagaataaa tcctgacatg gatagtagct 180
tctgtgttct ctccatcctg agaacagaag ggccataaaa aaacaaagaa gcattaccaa 240
aggggagttc tagacccaca cggggaactc ctaatacaaa agcaacaaga aagacangta 300
agactttaaa agttgcagaa gtcctaagaa tagcgccaat gtagtaggcc ctttttaaca 360
acaacaaana ataaaaataa gagagagaga gaaattagaa atttangaag ttcattaaat 420
aactggtact tatattcaag ggaatttatt agtggccagc ctantggggg acccagcntn 480
taggaaaaga cccttgaaaa ggaccttccc ncacctggga canaaggata gnaccgaccc 540
cccagggaag nccgccntgg aaangggatc cnaacttgan gctttttagg gtttcaaaan 600
tccttgctng gccccaangg gcaggntttn ntn 633

<210> 498
<211> 601
<212> DNA
<213> Homo sapiens

<220>
<221> misc_feature
<222> (1)...(601)
<223> n = A,T,C or G

<400> 498
acattcttca gaacagtttt ggtcgtttaa aaaaaatcac acatttataa gcagtgattt 60
caatcatgtt taaaaacaaa aatattaaac aaattcattt cctaatccag atgatacaga 120
atccaagaaa tttctgtagg cacttcactt tccatagaac ttcttgttca gcaggtatat 180
gagaaggttt acattcactt taaccttatc aaacattttc attacagcta ctccttcata 240
ttgcatctga agtaaatcct gaatattgag ttgcaccttt tccatctcaa caccaaggaa 300
ttttgatctt acatcgaaaa tgcctacatc ttcagtagct atgatatcaa atgtaacatt 360
cttaaactgg tttgtttgaa gatcatctat atctagcagg acacctttct catgcagctt 420
tgctgcagtg tacaaactgc aggctccatc ctcgtgggct cgcactatgt gcgcttttaa 480
aaaatattat ttctaataaa tctttgaagt taaaataccg ttctttcagt tggnccaaaa 540
aaaaannnnn nnnanganag aanngnaang aaagtggggt gnnnttgggg nggaaaaacn 600
n 601

<210> 499
<211> 293
<212> DNA
<213> Homo sapiens

<400> 499
ggtactcaag cttttgacct catgccttgt gtagtaaaaa aggatttggg ggttttgttt 60
ggttcctgag agggttgtgt tttgtttttg tttccttttg tttatgtttt ggcctttcct 120
ctttgtcttt ccatgtagac cagatatttg aaagggcaga cgatggctag aggtgtaatg 180
tgcagcttgt ttatacggta ttttgggaaa cttaccttgg atgggaaatc gaatcgtgga 240
ttcaccaggc cggtgctggc acactcaccc tcgccctttc cctccggttc agt 293

<210> 500
<211> 630
<212> DNA
<213> Homo sapiens

<220>
<221> misc_feature
<222> (1)...(630)
<223> n = A,T,C or G

<400> 500
gggtactcat gaattcaagc cacagagtgg agcagagatc aaagaagggt gtgaaacaca 60
taaggttgcc aacacaagtt cttttcacac aactccaaac acatcactgg gaatggttca 120
ggcaacgcca tccaaagtgc agccatcacc caccgtgcac acaaaagaag cattaggttt 180
catcatgaat atgtttcagg ctcctacact tcctgatatt tctgatgaca aagatgaatg 240
gcaatctcta gatcaaaatg aagatgcatt tgaagcccag tttcaaaaaa atgtaaggtc 300
atctggggct tggggagtca ataagatcat ctcttctttg ncatctgctt ttcatgtgtt 360
tgaagatgga aacaaagaaa attatggatt accacagcct aaaaataaac ccacaggagc 420
caggaccttt ggagaacgct ctgtcacaga cttncttcaa acccaaggag gaagtgcctn 480
atgctgaaaa gttttggatg actcaactgg atggggtatt ccctgnaacc aaaacctggn 540
acccaagtcc ttaaaanccn nggagactta cattntgntg nacaatttgg gttaaaccnn 600
ttcncaaagc tttccatggg ggcanggccc 630

<210> 501
<211> 240
<212> DNA
<213> Homo sapiens

<400> 501
acatctgaaa taccccccaa acccagaaag cttttcaaca gctaggttgt ccaagaactt 60
ggaaaattca ccttctgatg tcctccaaga cagattccat tttttataca ccttatttgc 120
tcagacctgt aacttcagcc tggagtgaac acagacacct agttttcctc aaactcctct 180
tgggctttag agagaaggtg ctggcccttt gagccaagca ggttattggt tagtagtacc 240

<210> 502
<211> 481
<212> DNA
<213> Homo sapiens

<220>
<221> misc_feature
<222> (1)...(481)
<223> n = A,T,C or G

<400> 502
ggtacctgtt cttctatcca aacctttcaa ttcatgctac ctgattcatt tatttgacat 60
agatcttagg cccacttgaa ctcttttctt gtttatctag catagcacaa acgtttttcc 120
agtcttcttt atcaacacta atgcctctta attgcatcag tatttcctat tggaaaatac 180
atctgttcca gaaaaacatt tggcattcct gaataatttc caaatgtttt taatccaaag 240
aaaaaggttt aaagcttatt tccctttctt atacacacct gaataaaatt gatgtgcatg 300
ttttagggat caattaccta actgttcctt ggtctattta tgtataagaa tgctttttaa 360
agcacatgtc tcattttaaa tgacgcacaa actgaagatg ttaataaaat ttaagagtaa 420
tacaatgaaa aatattantn ttnnanatan aaaagcttgg acctgccngg gcggccgntc 480
g 481

<210> 503
<211> 643
<212> DNA
<213> Homo sapiens

<220>
<221> misc_feature
<222> (1)...(643)
<223> n = A,T,C or G

<400> 503
ggtactgcat tatttgagaa gctgctcaac ttgcaaaatc agttttcctc tcaataaaat 60
tatagctcta atgtttgcat ataagggaag tagttatcat gttagtaata cctctaatag 120
tataaacccc accccaaaat tagccagtaa tcctgtagga aggtacaagt ctcagactaa 180
gtttttagcc acttgtcaaa ttcagtttta aatgcttaga aaacactgag gacacctatt 240
gaggagggag gggggaaggt cacctgtaaa ggagtccaaa gtatgtgctg gagcagatga 300
tgacaaagac agaacatcta agaagataga catggaggaa agggagtagt atttccacac 360
actatgacat tgaaaattca atcatttatg ataggatttt gatccactgc cattactacc 420
ttgtgggaaa aatctnccaa tgaaaaggtt gaaaaattca ttctccaaaa attggcccng 480
ttttaangag aaaattttag agcagcaccn ttaaaccatg ccgggaactt tggtttaaca 540

aaatatngtg gggccccaaa aagctcctgt tgcttttagg cctcnagaga tttacccaga 600
acttaaaggn ttncnctggc cttgttcctt aangttgaaa acc 643

<210> 504
<211> 624
<212> DNA
<213> Homo sapiens

<220>
<221> misc_feature
<222> (1)...(624)
<223> n = A,T,C or G

<400> 504
ggtactgcat tatttgagaa gctgctcaac ttgcaaaatc agttttcctc tcaataaaat 60
tatagctcta atgtttgcat ataagggaag tagttatcat gttagtaata cctctaatag 120
tataaacccc accccaaaat tagccagtaa tcctgtagga aggtacaagt ctcagactaa 180
gtttttagcc acttgtcaaa ttcagtttta aatgcttaga aaacactgag gacacctatt 240
gaggagggag gggggaaggt cacctgtaaa ggagtccaaa gtatgtgctg gagcagatga 300
tgacaaagac agaacatcta agaagataga catggaggaa agggagtagt atttccacac 360
actatgacat tgaaaattca atcatttatg ataggatttt gatccactgn ccattactac 420
cttgtgggaa aaatccttca caatgaaaag ggttgaaaaa ttcattcttc caaaattggc 480
ccnngtttta aggagaaaat nttagagccg ccccttaanc ctgcccggaa cttggnttta 540
ccaaatntca gggngncccc aaaancttct gntgccttta ngncntncan agacttnacc 600
cnngaacttc naggntttnc ctng 624

<210> 505
<211> 652
<212> DNA
<213> Homo sapiens

<220>
<221> misc_feature
<222> (1)...(652)
<223> n = A,T,C or G

<400> 505
acaagctaca aatgcttgtt cagcagctga ggggcactct tgagtagcgt gtctgaagag 60
tgaataaaaa tccatataaa acaaatattc aaatagtttc cataggaaca cagataagtg 120
tgacccatat cctagtcttc catatggctg catcatggcg accctactct tacaaagaca 180
tttcaaaact agcagtaatt aagttacatg gtccccccaa atcccttaat tcaagctaaa 240
cttgcagtta acagctacca gagtgctatc tacacattaa tactagcccg aagcacaggc 300
tgctctgtgg cgtttcatcc cactctccca ggcacaagac acaggcaggg tgctggcatc 360
ctgttcctct acttcgggtg gggaaagtcg gggttctgga attgctgcat gagttgccac 420
gcaggccctg acatcacata gtaanatcgt ccggcctttt gggaaaccca ttgnacctan 480
aaggcancna gcaaccagtg gtaagccgcc ccaaggtttt cnaaagagcc tttccaatna 540
ccccccatgc cnttttaang gcnnggttac caagggcttn aaaaaatccg atttnanggg 600
ccnttacaag gttggggccc ccanaatgcn cggatngnaa aaaanacctt tt 652

<210> 506
<211> 545
<212> DNA
<213> Homo sapiens

<220>
<221> misc_feature
<222> (1)...(545)
<223> n = A,T,C or G

<400> 506
acaagctttt tttttttttt tttttttttt tttttttatc taaaagtgcc caggtgggct 60
taaggctgcc anactgcacg cacatctaca gcaacaaggg cttctattcc atctacaact 120
tggatcgggg gaaaagggag atgtaggaga ggaaggaaaa aagaggggaa aaatatacca 180
ccaaccctcc cccacaaaaa aagggaaaaa aaaaaatccc accacaggga gatctatgtg 240
ccaagcataa tggaagagtg tgctccccaa acagatggtt ttgcacaggc taatgttctg 300
ctggttttcc ttagagacct attttgaaaa agtttaaaaa gacaggagat ttcaaaataa 360
ttcaatcctg gcagaaattc aaactccaaa actaggagca aaatcatcct tcactgaatt 420
aattcctttt ctctttctct tttcttaaac attttattca ttttatagaa agatttcttt 480
ttttggntgc ntttggtcca atcntttgga nantggttga aggagtacct tggncgngan 540
ccccc 545

<210> 507
<211> 625
<212> DNA
<213> Homo sapiens

<220>
<221> misc_feature
<222> (1)...(625)
<223> n = A,T,C or G

<400> 507
   acctgtctct ctgccttctg gaggctctct aggattggaa aagttcaaga aacccgaggg 60
   aagctgggac tgtgaattgt gcctagtgca gaataaggca gactctacca aatgtttggc 120
   atgtgaaagt gcaaagccag gcacaaaatc tgggtttaaa ggctttgaca catcttcctc 180
   atcttcgaac tcagcagcct cctcatcctt caaatttggt gtctcatcat cctcttctgg 240
   gccttctcag actttaacaa gcactggaaa ttttaaattt ggagatcagg gaggattcaa 300
   aataggtgtg tcatctgatt ctgggtctat aaaccccatg agtgaaggct ttaaattttc 360
   taaaccaata ggagatttta aatttggagt ttcatctgaa tctaagcccg aagaagttaa 420
   aaaagatagt aagaatgata atttttaagt ttggacttct ttggtttaac cacccagttt 480
   ctttaacttc atttcaattg gggtatctaa tcttggacag gaagaaaaag aaagangaac 540
   ctggcccaaa tctttcctnt gcaggnttta nccttnggac ccttggccgc naaccaccct 600
   aaggggggaa ttccnnacac tgggg 625

<210> 508
<211> 612
<212> DNA
<213> Homo sapiens

<220>
<221> misc_feature
<222> (1)...(612)
<223> n = A,T,C or G

<400> 508
ggtcgaagac agaggttcag gtcgttccag gggtagagga ggcatgaagg atgaccgtcg 60

ggacagatac tctgcgggca aaaggggtgg atttaatacc tttagagaca gggaaaatta 120
tgacagaggt tactctagcc tgcttaaaag agattttggg gcaaaaactc agaatggtgt 180
ttacagtgct gcaaattaca ccaatgggag ctttggaagt aattttgtgt ctgctggtat 240
acagaccagt tttaggactg gtaatccaac agggacttac cagaatggtt atgatagcac 300
tcagcaatac ggaagtaatg ttccaaatat gcacaatggt atgaaccaac aggcatatgc 360
atatcctgct actgcagctg cacctatgat tggttatcca atgccaacag gatattccca 420
ataagacttt agaagtatat gtaaatgnct ggttttcata attgctcttt atattgggng 480
gtatctgacc agatagtatt ttaagaaaca tgggaattgc anaaatgact gnagtgcaan 540
agtaattntn gggcactttt cgtttttaag ntggaaattc nctacanttc ctgaaccant 600
ttanggtttt tt 612

<210> 509
<211> 473
<212> DNA
<213> Homo sapiens

<220>
<221> misc_feature
<222> (1)...(473)
<223> n = A,T,C or G

<400> 509
cttgggtctg aaagtcgatg aaggacgcga ttacctgcga taagcttcgt ggagttggaa 60
ataaactatg atacggagat ttccgaatgg ggtaacctaa ctgagcaaac ctcagttgca 120
ttttgatgaa tccatagtca aattagcgag acacgttgcg aattgaaaca tcttagtagc 180
aacaggaaaa gaaaataaat aatgatttcg tcagtagtgg cgagcgaaag cgaaagagcc 240
caaacctgta aaaaggggtt gtaggacatc ttacattgag ttacaaaatt ttatgatagt 300
agaagaagtt ggaaagcttc aacatagaag gtgatattcc tgtatacgaa atcataaaat 360
ctnatagatg tatcctgagt agggcggggc accgtgaaac cctgtctgaa tctgccggga 420
ccaccccggt aaggctaata ctaatcanac accgatagtg aactagtacc tng 473

<210> 510
<211> 632
<212> DNA
<213> Homo sapiens

<220>
<221> misc_feature
<222> (1)...(632)
<223> n = A,T,C or G

<400> 510
ggtacctatg tggattccaa gagcctgata gcattcttgt ccttcagagc ctccctggca 60
aacaattacc atcacacaaa gccatacttt ttgtgcctcg gcgagatccc agtcgagaac 120
tttgggatgg tccgcgatct ggcactgatg gagcaatagc tctaactgga gtagacgaag 180
cctatacgct agaagaattt caacatcttc taccaaaaat gaaagctgag acgaacatgg 240
tttggtatga ctggatgagg ccctcacatg cacagcttca ctctgactat atgcagcccc 300
tgactgaggc caaagccaag agcaagaaca aggttcgggg tgttcagcag ctgatacagc 360
gcctccggct gatcaagtct cctgcagaaa ttgaacgaat gcagattgct gggaagctga 420
catcacaggc tttcatagaa accatgttna ccagtaaaag cccctgtgga agaaccnttc 480
tttatgctaa gtttgaattt gaatgcccgg ctcgtggcgc agacatttta acctattcan 540
cttgtggtgg cttggnggta attcggncca aacactttgc ncttttgtga aaaaaaatcn 600
cctcttcang gttggggnaa nggggctttt gg 632

<210> 511
<211> 616
<212> DNA
<213> Homo sapiens

<220>
<221> misc_feature
<222> (1)...(616)
<223> n = A,T,C or G

<400> 511
acagaaccta aaggtttcac tgaatgcgaa atgacgaaat ctagcccttt gaaaataaca 60
ttgtttttag aagaggacaa atccttaaaa gtaacatcag acccaaaggt tgagcagaaa 120
attgaagtga tacgtgaaat tgagatgagt gtggatgatg atgatatcaa tagttcgaaa 180
gtaattaatg acctcttcag tgatgtccta gaggaaggtg aactagatat ggagaagagc 240
caagaggaga tggatcaagc attagcagaa agcagcgaag aacaggaaga tgcactgaat 300
atctcctcaa tgtctttact tgcaccattg gcacaaacag ttggtgtggt aagtccagag 360
agtttagtgt ccacacctag actggaattg aaagacacca gcagaagtga tgaaagtcca 420
aaaccaggaa aattccaaag aactcgtgtc cctcgagctg aatctggtga tagcccttgg 480
ttctgaagat cgtgacttct ttacagcatt gatgcatata gatctcaaag attnanagaa 540
acnggaatgt ccatcaataa acnaggtgat tgttnggaag gaagatgttc tttttaaaaa 600
tnaatgtttn atntng 616

<210> 512
<211> 619
<212> DNA
<213> Homo sapiens

<220>
<221> misc_feature
<222> (1)...(619)
<223> n = A,T,C or G

<400> 512
ggtaccggtc tttctcaaat atcatcagca ccctcaatcc cactgctaaa cgacatttgg 60
tcctcgcctg ccactatgac tccaagtatt tttcccactg gaacaacaga gtgtttgtag 120
gagccactga ttcagccgtg ccatgtgcaa tgatgttgga acttgctcgt gccttagaca 180
agaaactcct ttccttaaag actgtttcag actccaagcc agatttgtca ctccagctga 240
tcttctttga tggtgaagag gcttttcttc actggtctcc tcaagattct ctctatgggt 300
ctcgacactt agctgcaaag atggcatcga ccccgcaccc acctggagcg agaggcacca 360
gccaactgca tggcatggat ttattggtct tattggattt gattggagct ccaaacccaa 420
cgtttcccaa tttttttcca aactcagcca ggtggttcga aagacttcaa gcaattgaac 480
atgaacttca tgaattgggt tgcttcaagg atcactcttt tggaagggcg ggatttnccg 540
aaatacnggt tttggaggng tgaatcaggg atgaccntat tcccttttta anaaaaaggg 600
gttcccntnt gcntntgnn 619

<210> 513
<211> 175
<212> DNA
<213> Homo sapiens

<400> 513

ggtacatcct cggccgggag tccccactgt ctctctacaa tgaggagctg gtgagcatga 60
acgtgcaggg tgattatgag ccaactgatg ccaccgggtt catcaacatc aattccctca 120
ggctgaagga atatcatcgt ctccagagca aggtcactgc caaatagacc cgtgt 175

<210> 514
<211> 597
<212> DNA
<213> Homo sapiens

<220>
<221> misc_feature
<222> (1)...(597)
<223> n = A,T,C or G

<400> 514
actagttact gcatctgatt ttacagacag agaagagtca aggcccagag agcagacagc 60
tcaccccaac atcacacagc agtcagctgc gaggggcttg gtgctactca gatttctcct 120
aagaatgttt ggaaacaacc tgagggagag ttaagtaata aaggaaaatc acaaacagag 180
acagagaccc agaaagggac tcacgggaat aaaagcagaa agtgacagag atacatagag 240
atgatgagac agagacagag agatcagaga tagggttcag aaaaaaagaa gagagaggct 300
gggcacagtt gctcacgcca gtaatcccag cactttgaga ggcggagatg ggaggatctc 360
ttgagcccag gagtttgaga ccagcctgga cagcatagta agaccccatc tttatttaaa 420
aaaaagtttt attaatttaa aaaaaatgcc nagagagata accccccnta gaaggttgga 480
aagccaaaag ctttttgggg gcttaaaagn accccaaccc ggnccnggga ganaggtttt 540
tttttgaggg aanaatccgg ttcttggcca ngcttaanng gcctatttcc aaaaaac 597

<210> 515
<211> 574
<212> DNA
<213> Homo sapiens

<220>
<221> misc_feature
<222> (1)...(574)
<223> n = A,T,C or G

<400> 515
ggtacactgg ttgatatgaa gattgaattt ggtgttgatg taaccaccaa agaaattgtt 60
cttgctgatg ttattgacaa tgattcctgg agactctggc catcaggaga tcgaagccaa 120
cagaaagaca aacagtctta tcgggacctc aaagaagtaa ctcctgaagg gctccaaatg 180
gtaaagaaaa actttgagtg ggttgcagag agagtagagt tgcttttgaa atcagaaagt 240
cagtgcaggg ttgtagtgtt gatgggctct acttctgatc ttggtcactg tgaaaaaatc 300
aagaaggcct gtggaaattt tggcattcca tgtgaacttc gagtaacatc tgcgcataaa 360
ggaccagatg aaactctgag gattaaagct gagtatgaag gggatggcat tcctactgta 420
tttgtggcag tggcaggcag aagtaatggt tngggaccag tgatgtctgg gaacactgca 480
tatnccgtta tnagctggcn tcncttanac caactgggga agttcaggat gtgtgggctt 540
ctctttgact nccaatggnc ttggctntca accn 574

<210> 516
<211> 450
<212> DNA
<213> Homo sapiens

<220>
<221> misc_feature
<222> (1)...(450)
<223> n = A,T,C or G

<400> 516
aaaaaggcgt aaagcggaaa gcagatacta ccacccctac acctacagcc atcttggctc 60
ctggttctcc agctagccct cctgggagtc ttgagcctaa ggcagcacgg cttcccccta 120
tgcgtagaga gagtggtcgc cccatcaagc ccccacgcaa agacttgcct gactctcagc 180
aacaacacca gagctctaag aaaggaaagc tttcagaaca gttaaaacat tgcaatggca 240
ttttgaagga gttactctct aagaagcatg ctgcctatgc ttggcctttc tataaaccag 300
tggatgcttc tgcacttggc ctgcatgact accatgacat cattaagcac cccatggacc 360
tcagcactgt caagcggaag atggagaacc gtgattaccg ggatgcacag gagtttgctg 420
ctgatgtacc tcgggcgcga acacgcttan 450

<210> 517
<211> 611
<212> DNA
<213> Homo sapiens

<220>
<221> misc_feature
<222> (1)...(611)
<223> n = A,T,C or G

<400> 517
actcctctga ggactacatt aagtcaggag ctcttcttgc ctgtggcata gtgaactctg 60
gggtccggaa tgagtgtgac cctgctctgg cactgctctc agactatgtt ctccacaaca 120
gcaacaccat gagacttggt tccatctttg ggctaggctt ggcttatgct ggctcaaatc 180
gtgaagatgt cctaacactg ctgctgcctg tgatgggaga ttcaaagtcc agcatggagg 240
tggcaggtgt cacagcttta gcctgtggaa tgatagcagt agggtcctgc aatggagatg 300
taacttccac tatccttcag accatcatgg agaagtcaga gactgagctc aaggatactt 360
atgctcgttg gcttcctctt ggactgggtc tcaaccacct ggggaagggt gaggccatcg 420
angcaatcct ggctgcactg gaaggtgngc anaaccnttt cgcanttttg nccacacacc 480
tggnggatgt gtgngcctat tcncgctttt ggnanatgcc tnaagggcna caaattggtc 540
caatttgnnn nnaacctttg cctccaaaga aagggggaaa naaaagtttc ccccnanngg 600
gggcgggccc c 611

<210> 518
<211> 395
<212> DNA
<213> Homo sapiens

<400> 518
ggtgatttat ctaatcagaa ctcttcagat caggcaaatg aagaatggga aacagcttct 60
gaaagcagtg atttcaatga gaggcgagag agggatgaaa aaaaaaatgc tgacttgaat 120
gcacaaacag ttgtaaaggt tggagagaat gttctacctc caaagaggga aattgcaaag 180
agaagttttt ctagtcagag accagtagat cgtcagaatc gacgtggcaa caatggtcca 240
cccaaatcag gaaggaattt ctcaggtcct agaaatgaaa ggagaagtgg cccaccatca 300
aaaagtggga agagagggcc atttgatgac cagcctgcag gcacaactgg ggttgacctc 360
atcaatggca gctctgcaca ccatcaggaa ggagt 395

<210> 519

<211> 626
<212> DNA
<213> Homo sapiens

<220>
<221> misc_feature
<222> (1)...(626)
<223> n = A,T,C or G

<400> 519
ggtaccgaaa gcacagtaat cactggtgtc gatattgtca tgaaccatca cctgcaggaa 60
acaagtttca caaaagaagc ctacaagaag tactgatttt aaaaactaat aacttaaaac 120
tgccacacgc aaaaaagaaa accaaagtgg tccacaaaac attctccttt ccttctgaag 180
gttttacgat gcattgttat cattaaccag tcttttacta ctaaacttaa atggccaatt 240
gaaacaaaca gttctgagac cgttcttcca ccactgatta agagtggggt ggcaggtatt 300
agggataata ttcatttagc cttctgagct ttctgggcag acttggtgac cttgccagct 360
ccagcagcct tcttgccact gctttgatga cacccaccgc aactgtctgn ctcatatcac 420
gaacagcaaa gcgacccaaa ngtggatagt ctgagaagct nttcaacaca catnggcttt 480
gccaggaanc ntttntacca tgggagcntt cccngacttt tagnaaatta agggcntttt 540
tcacttttta acccaaacgg ggaaaaattt ttnctttaag ttaanaaact tgcnntgcaa 600
tggaanccgn ngggaatcca atacgg 626

<210> 520
<211> 322
<212> DNA
<213> Homo sapiens

<400> 520
ggtacccaag catctagtct ggaactgaca gagataaata gagaaaatgt tccaaagtct 60
ggcacgcccc agcttaggct gccattcgct gcaaggttga acacccccat gggccctgga 120
cgaactgtcg tcgttaaagg agaagtgaat gcaaatgcca aaagctttaa tgttgaccta 180
ctagcaggaa aatcaaagga tattgctcta cacttgaacc cacgcctgaa tattaaagca 240
tttgtaagaa attcttttct tcaggagtcc tggggagaag aagagagaaa tattacctct 300
ttcccattta gtcctgggat gt 322

<210> 521
<211> 613
<212> DNA
<213> Homo sapiens

<220>
<221> misc_feature
<222> (1)...(613)
<223> n = A,T,C or G

<400> 521
ggtaccatcc tcatctcggt gggatgtgca gttttctgtg cccttatcgt ctggttcttt 60
gtatgtccca ggatgaagag aaaaattgaa cgagaaataa agtgtagtcc ttctgaaagc 120
cccttaatgg aaaaaaagaa tagcttgaaa gaagaccatg aagaaacaaa gttgtctgtt 180
ggtgatattg aaaacaagca tcctgtttct gaggtagggc ctgccactgt gcccctccag 240
gctgtggtgg aggagagaac agtctcattc aaacttggag atttggagga agctccagag 300
agagagaggc ttcccagcgt ggacttgaaa gaggaaacca gcatagatag caccgtgaat 360
ggtgcagtgc agttgcctaa tgggaacctt gtccagttca gtcaaagccg tcagcaacca 420

aataaactnc agtggccact accagtatca caccgtgcat aaaggattcc gggctgtanc 480
ttgcccggcc ggccgtntaa aggcgaattc cagncacttg ggggccgntc taaagggatn 540
ccactttggn ccaacnttgg gggaatctng ggcaaantng tccctgngna aatggtatcc 600
gtcaaatncc cnn 613

<210> 522
<211> 319
<212> DNA
<213> Homo sapiens

<400> 522
accagggagg catgacattg cttttgttga atttgaaaat gatgggcagg ctggagctgc 60
cagggatgct ttacagggat ttaagatcac accgtcccat gctatgaaga tcacctatgc 120
caagaaataa catttgggat agtcgtcttt aaaagacttg gtgttattta cagtgtttgt 180
tttgataaca tttggctggg tcattttaat agttagagat gaggaggagt aaaagtgaaa 240
tttttgtgaa ggacttaaat tatccagtgt ttctttagcc ttggtgaact atgaaatacg 300
aaggccttaa ttttgtacc 319

<210> 523
<211> 589
<212> DNA
<213> Homo sapiens

<220>
<221> misc_feature
<222> (1)...(589)
<223> n = A,T,C or G

<400> 523
acagcgcgcg gctctacacg cttgggtagc gggataagtc actgttttct ttatttcttt 60
aaaaaaaaaa aagttctgtt gcaaacgact gctgttggat tctgagggtg gggagggaga 120
gagagggagg gagagggagt gaagagcctg ccctcctata tggattcttc agggccctcc 180
acatctgagg tggctcattc ccatcacaca cagattgtcc tggtgttcat ttcaaggcca 240
gtgttcagca gcagcgtttg gaaagcaggt tctgtgggac cccccgcccc gcccccacac 300
tccttcatag cagcagtagt ggcttctcca tcctgntttc tgcaacattc tatacaaaac 360
tgtgctgtga ccttgcggta agcctggatc tggcaaagag aatcaaatga aaccccttct 420
ttctcttttc gtccacaact ctgtanaact ntntgmaccc ttaccccttt ccaccttttg 480
gattnaattt taaggccgtg nanctttggc cggaacaccc ttagggcnaa ttcnnnccat 540
tgggggccgt ctaagggann ccaattggnc caanttgggn aacanggnn 589

<210> 524
<211> 621
<212> DNA
<213> Homo sapiens

<220>
<221> misc_feature
<222> (1)...(621)
<223> n = A,T,C or G

<400> 524
ggtacattgg agagatctcg cctactgccc tgcggggtgc ctttggcact ctcaaccagc 60
tgggcatcgt tgttggaatt ctggtggccc agatctttgg tctggaattc atccttgggt 120

ctgaagagct atggccgctg ctactgggtt ttaccatcct tcctgctatc ctacaaagtg 180
cagcccttcc attttgccct gaaagtccca gatttttgct cattaacaga aaagaagagg 240
agaatgctaa gcagatcctc cagcggttgt ggggcaccca ggatgtatcc caagacatcc 300
aggagatgaa agatgagagt gcaaggatgt cacaagaaaa gcaagtcacc gtgctagagc 360
tctttagagt gtcagctacc cgacagtcca tcatcatttc cattgtgctc cagctctntc 420
gcagcttctt gggatcaatg ctgngttcta atactcacca ggaatcttca aggatgcagg 480
tggttaaaaa ncccatttat gccncctttg ggcccggtgn gggtnaaacc anacttnccn 540
nggaggnncc tnttttnnng ggggaanggc cngaaaaaag gncttcgcct ttaaanngcc 600
cttggaggga agnttttttt n 621

<210> 525
<211> 384
<212> DNA
<213> Homo sapiens

<400> 525
acagcacttt gagaggacat cactagacaa gtaatacaca catggcctgc aggaggtcaa 60
gggcggcgag ggggctgggc aggggacatt tttgtgactt ccactgttat tatatttcac 120
gacaacagca gcagcacaaa tggtgtgctc accactggag aatgagagct gctgagtctt 180
gaggatggcg agacagcctt cctgcatttg ctgctttagt ttctgcttta gagctaagtt 240
ttatacagag aataaaatga ccatcttctc ttacaaacac gatgatgtat gaccccacac 300
aacacaaggt attatgaagt atctgaaact gaggataatc tgactgaaga tgcttgccga 360
gagggtacct cggccgcgcc acgc 384

<210> 526
<211> 621
<212> DNA
<213> Homo sapiens

<220>
<221> misc_feature
<222> (1)...(621)
<223> n = A,T,C or G

<400> 526
actgtagctc cccatgagat gtgatgagta tgccttcacc cttggtgtca tactggggtc 60
ttccggcacg tcccagcatc tgcagaatgt ccagtgctcc cagttctgtc caacgcccct 120
tctctggact gtacaatgtc actgacggat cctgccagct gtttgtgtat gggggctgtg 180
acggaaacag caataattac ctgaccaagg aggagtgcct caagaaatgt gccactgtca 240
cagagaatgn cangggtgac ctggccacna gcangaatgc agcggattcc tctgcccaag 300
tgcttnagaa ggcagnattc tgaagactac tncagcgata tgttcaacta tgangaatac 360
tgcacngtna accgcattna ctgggntttg ncngtgcatc cttcnacgct ggtaccttcg 420
gcccgggacc acgcttaagg gcgaatncan gnactactgg ccgggtcgtt actantngaa 480
tccgagnttc gnnaccaagc tttgcgtaaa atattgggca taagttggnt ttctgngnga 540
aaaatggtan atcngttnan aattcccnaa tatatncanc cngtnccttt aattntaaat 600
ccgggggtnn taantnantn n 621

<210> 527
<211> 611
<212> DNA
<213> Homo sapiens

<220>

<221> misc_feature
<222> (1)...(611)
<223> n = A,T,C or G

<400> 527
acagctcatc cacttcctca tctgtaaacc gatcccccat ggttgtcagc agctctctta 60
ggtaatcttc ctgaatggtg cctgttgctt cttcatcaaa gcaagcaaag gcgtttctga 120
tgacatcttc aggatctgtg ccatttaact tctcaccaaa catggtcagg aacatggtga 180
aattgatggg ccctggggcc tcattcatca tggcatcaag gtatgcatca gtgggattct 240
tccctagaga agcaagcata tcatgcaaat cttccttgtc gatgaagcca tctctgttct 300
gatcaatcat gttgaaggcc tctttgaact cctgaatctg tgattggtca aacatggcaa 360
acacattgga tgttgcacgc tgagggcgct tcttggtggt cttggtcttt gcctttttgc 420
ttcgacatgg tggntggtta attncgacgc ccaaacacca gaacccgggg ccancctgcg 480
cganaacgca accaaaacct tnggccggaa cacccttaag gggaaatccc nncactgggg 540
ggccgtataa nggganccna nttnggacca aacttggngg aaaaangggc aaaanngttc 600
ctgnggaaan n 611

<210> 528
<211> 593
<212> DNA
<213> Homo sapiens

<220>
<221> misc_feature
<222> (1)...(593)
<223> n = A,T,C or G

<400> 528
acaagctttt tttttttttt tttttttttt taggtagtgg gtgttgagct tgaacgcttt 60
cttaattggt ggctgctttt aggcctacta tgggtgttaa attttttact ctctctacaa 120
ggttttttcc tagtgtccaa agagctgttc ctctttggac taacagttaa atttacaagg 180
ggatttagag ggttctgtgg gcaaatttaa agttgaacta agattctatc ttggacaacc 240
agctatcacc aggctcggta ggtttgtcgc ctctacctat aaatcttccc actattttgc 300
tacatagacg ggtgtgctct tttagctgnt cttaggtagc tcgtctggtt tcgggggtct 360
tanctttggc tctccttgca aaggtatttc tagntaattc attatgcnna aagnatangg 420
gtaagccctg ctatataagc ctgggtataa attttcancc tttcctttgn ggaccctngg 480
ccggaacacc ctaagggcga aatccancca ctgggggccg tactaaaggg atcccaactt 540
gggnccaact tggnnnaaac cggggcanaa nngtccctgg ggnaaatggn anc 593

<210> 529
<211> 251
<212> DNA
<213> Homo sapiens

<400> 529
accattggtg gccaattgat ttgatggtaa gggagggatc gttgacctcg tctgttatgt 60
aaaggatgcg tagggatggg agggcgatga ggactaggat gatggcgggc aggatagttc 120
agacggtttc tatttcctga gcgtctgaga tgttagtatt agttagtttt gttgtgagtg 180
ttaggaaaag ggcatacagg actaggaagc agataaggaa aatgattatg agggcgtgat 240
catgaaagac c 251

<210> 530
<211> 601

<212> DNA
<213> Homo sapiens

<220>
<221> misc_feature
<222> (1)...(601)
<223> n = A,T,C or G

<400> 530
acagtataaa atgtttccat aggaacacaa aagaaactgt cactagtggc ctgctgtcag 60
atggcttcta attcatcagt tagccatttt taggacacta gtccagctta ttgctacaat 120
cttcaagttg ttctagtcac ccaaattata atgaattcaa tgtataccag aatttaccaa 180
taaaggctca aagagttata taatatacac caatatacac aaaacagcta ttctgagtaa 240
aatgaatatt ccatacttaa ataagaacca agaatagtaa ttttaggcta ctctattatc 300
cttgtgattg gtatttttaa aattttgagc aaagtgcaca gtgaatgaaa cagtcagcag 360
acacgatcct tctgtgaact ctcaaattcc tgccttagaa tcacgtcacc tgagaaatga 420
gaacctttga gacctggtgc atatcaaata gcttcacatg tcaaaccaca ggggccgctt 480
ggangccatt ctngggcaca ggangncaac tggttcnttn aaaatggnnc ccttncctgt 540
gcangggccc tgtgttaaag gccccaaaac cggcctcngg ggaaacaagg ttgntaatta 600
a 601

<210> 531
<211> 607
<212> DNA
<213> Homo sapiens

<220>
<221> misc_feature
<222> (1)...(607)
<223> n = A,T,C or G

<400> 531
ggtacaagct tttttttttt tttttttttt ttttttttct cagccttgga tttcttctta 60
gcttccttct gctttaagct cttggtctct tgtttccgct natttctggc ctgcccttgg 120
atagtagtct gacactctcc ccgttgaacc ttctgcctca tcttcttctt gcttttagca 180
atctttgctt tatcctcctc attcaatgtt tcttgggcct ccagtttctt tagggggcgg 240
ttgtctgtct tgttcaatag ctcagtgatt ttgaccttag gtggccgacc tcgaccccgt 300
ttcaccttgg ggacttcctt agtcttagcc ttctcagtgt ttcaaggtcg accccgtttg 360
ccagtaattg cctgaatcct cgacgggatc tcctctgctg aaagctgcac ccactgcaag 420
ccctttggcg ngnctctttt cttcaaagaa atctccaaca nggcatacgg ggactgaanc 480
ttaanngctt nttggnggaa actgggnacc tggccgggca ngggcctntg ttttacctnc 540
tggnaatnaa aagggaaaat ncaaaanttt accctnttna ccnngtttnt ggggtngggg 600
gaaaang 607

<210> 532
<211> 608
<212> DNA
<213> Homo sapiens

<220>
<221> misc_feature
<222> (1)...(608)
<223> n = A,T,C or G

<400> 532
   ggtactgaac aggtaagtca tccctcagcc agagattagt ctacttcttc catgcgtgat 60
   gtgtcgtcat ctccttcaag gggtggcatt tcttcagtta cagcagcact ggtatcatca 120
   gcagtagggt catcttcatc aatacccaga ccaagtttga tcatcctgta gatcctgtta 180
   gcatgtgtct ggggatcttc cagactgaag ccagaagaca ggagcgcagt ttcataaagc 240
   aagatgacca gatccttcac agacttgtcg ttcttatcag cctctgcctt ttgccttaag 300
   gtctcaataa tggaatggtc agggtttatc tccaggtgtt tctttgctgc catgtaaccc 360
   attgttgagt ngctcttagg gcttgagctt tcatgattcg ctccatgttt gctgtccagc 420
   catatgtgct tgngacaatc agcatggaaa ntcaccaatc cggttgacac aaccacnttt 480
   cactttttct ccaaanngcc tttcatgant ttcnnanggt ntcaaacttt gggttttcnc 540
   ntnccgggtc ntttcncntt ttaaaccctt nggaattccn gccttttttg ggacnnacnn 600
   taagnttt 608

<210> 533
<211> 593
<212> DNA
<213> Homo sapiens

<220>
<221> misc_feature
<222> (1)...(593)
<223> n = A,T,C or G

<400> 533
acacatttgc tgatggcttc tcaaaacctg agccgagaat agggtctgat agcccagcca 60
agtttaaaag cagacacaca cgaatgtagt atcgttgtgc ctgaaatgac cattctgggt 120
tgtttagaat ccagaatcat caaaagccat gtggtatgag gaagtaataa atatcctctt 180
gaatcttctt accctatttt gcacaaatgg atggctgcat gaacagctct tgtaaattgc 240
tctgagtcca caccaataga aacctgcact cattctatag ctacagaggg tttgttggct 300
taaggggact ttatcatctc agcattaatt tcccttttaa agctattctc aaggttggac 360
tgtctcagag ataaacaaag aggaatcctt ttggcttaga agccaactgg cttactcaga 420
cttcctccct tcctactcca attcccacac taccatanta tcntcttgac tagaaaatca 480
attatttacc tgacataagg gcaagtctat tctttttcca nnccttgccc tnggggcctt 540
ggnaanaaaa atccntgcct ttttggaana agttttggga cnngcttagg ttt 593

<210> 534
<211> 608
<212> DNA
<213> Homo sapiens

<220>
<221> misc_feature
<222> (1)...(608)
<223> n = A,T,C or G

<400> 534
ggtacacttc tgtttatatt taaacaacaa agaaaaaagc atctacacac ttaaaaaatt 60
aattcaatat tcctaaatct attttaactc attttaaaat actacataca gaagccagaa 120
tgcagggtta agaatggaat aaggtgggga gaagaagggg accacgaaga aaaacactta 180
gacaattact tgtctgttgt gggtaaagca acaggaatcc tgggagatac aagaaatcag 240
taacaacttt gctcataact gatattttcc cctcatgttt gtttttaata acgtccatat 300
gggtgctctc tgtatgctcc cttcactggc ctagcaggag gggccttnag cgacggcctg 360

[?]t[?][?][?][?]a[?][?][?][?] [?]g[?][?][?][?]g[?][?][?][?] [?]g[?][?][?]a[?]aag[?] [?][?][?]a[?]aaga[?] [?][?][?][?]gaa[?][?][?] [?][?][?][?]a[?]g[?][?][?] 420
atagtcataa tattctgagt ccccttgact ctggctgnaa ataancttcg tagccttnga 480
actttggtct gcgnatgnat natcatatnc ctaatcntca naagnttntn gngcccgaag 540
ttggnggcaa gggttctttn ggaancccct tnccngcctt tggggnctgg acncnctnan 600
agnggggg 608

<210> 535
<211> 603
<212> DNA
<213> Homo sapiens

<220>
<221> misc_feature
<222> (1)...(603)
<223> n = A,T,C or G

<400> 535
acaaagtgac ccctcgctcc tgccaccggt ttgagcaagc gttctacacc tatgacacgt 60
cttcacctag tatcttgaca ttgacagcca ttcgccacca tgtccttgga actatcacca 120
ccgacaaaat gatggatgtc actgtgacta tcaagtcttc catcgacagt gaacccgcct 180
tggtcttagg ccctctgaag tctgtgcagg agctgcggag ggagcagcag ctggctgaga 240
tcgaggcccg caggcaggag agggagaaaa acggcaatga ggaaggtgaa gaaagaatga 300
ccaagcctcc cgtgcaggag atggtagatg agttacaagg ccccttctcg tatgatttct 360
cttactgggc gcnggnctgg agagaaaatt actgnttcac ngtcatctna agaactgctc 420
ttttatcccc ctttcaatgg aaagcncgtt gntcangtgg gaagaaagct tgcncaaggg 480
aaanttggat tcgagatncn ccgggaaaag gccaggcctg gtttttaaaa agggcccnaa 540
tnccccccgg nanttgnaaa gggaatccna aattggtctt ccntnngaaa aggggncaag 600
ttn 603

<210> 536
<211> 581
<212> DNA
<213> Homo sapiens

<220>
<221> misc_feature
<222> (1)...(581)
<223> n = A,T,C or G

<400> 536
ggtactcctg ggaggctttt gacagccacg ggcaggagag cagcggccag cttcccgagg 60
agctctttct gctgctccag tctttggtca tggctaccca cgaaaaggac acggaagcca 120
tcaagtcgct gcaggtggag atgtggccac tgttgactgc tgagcagaac cacctccttc 180
acctcgttct acaagaaacc atctccccct caggacaggg agtctgatcc atcccattca 240
cccagtgact tctttttgcc caggcctgga ctttttgcat cagtcacgtt aaccagatga 300
ctttgcctgt taccaaacct catgcatcca cgtttgcgtc tggggaggaa taaaaagaca 360
tcgttcccgc ttctgcgttt tgntattcct actgccgcca taggaattat ttcgtggctg 420
aacgttaccc agcancccga gaacactttt ggatagaatt ngagttgagg acattggctg 480
gcttttaaaa ancccnnctt ggaaatngna atncctttcg ntcctttctc cggnggttcc 540
ncctnanggn anttttggtt cgctttgntn caaagngagg g 581

<210> 537
<211> 568

<212> DNA
<213> Homo sapiens

<220>
<221> misc_feature
<222> (1)...(568)
<223> n = A,T,C or G

<400> 537
ggtacggact actcccctca catgcgtcct acctgtgaaa ctctgggaag caggaaggcc 60
caagacctgg tgctggatac tatgtgtctg tccactgacg actgtcaagg cctcatttgc 120
agaggccacc ggagctaggg cactagcctg acttttaagg cagtgtgtct ttctgagcac 180
tgtagaccaa gcccttggag ctgctggttt agccttgcac ctggggaaag gatgtattta 240
tttgtatttt catatatcag ccaaaagctg aatggaaaag ttaagaacat tcctaggtgg 300
ccttattcta ataagtttct tctgtctgtt ttgtttttca attgaaaagt aattaaataa 360
cagatttaga atctagtgag agcctcctct ctggtgggtg gtggcattta agggtcaaac 420
cancnanaaa tgcttggtgc tggttnaaaa agctcangtg gctgctgtgg tggctnatgc 480
ctgnaatcca acattntggg aaggccaagc cggaaaactg ttgngccnng anttaaaata 540
anctgggcac ntacaanntt cgtttnna 568

<210> 538
<211> 598
<212> DNA
<213> Homo sapiens

<220>
<221> misc_feature
<222> (1)...(598)
<223> n = A,T,C or G

<400> 538
ggtttttttt ttttttngtt catgtctttt attaactcat acagttactt gtcttctggt 60
ttgttgaaac agtaagtcag acaacntttg ccacaataat gtctgtcaaa gtgacttgcc 120
ataaanaccc cancaccaca ttcatcataa gggcactctt gacgaaggcg actaattttg 180
ccattctatt tcaggacagc cagctaaacc ttctntctct tgtgcttatt cttcttggga 240
gtggtgtaag acttcttctt ccttttctta gcaccaccac gaagtcttaa cacatgatga 300
agantagact ccttttgaat attgtagtcn gacaagagtn catacatcat accaacttnn 360
tanatacaca gctcagttaa ttagcttgat ggcacagtta tngttnggaa nagagangag 420
tgcancatan gnangagtga ngnggngatt cccacaattt tctnagaacn gaanagtagg 480
nngaattagt aggtactgga aatgaaatnn ggcttagcct gnctggntta gaaanaagaa 540
ttcnaagccc tttgtcaana nttntcaaaa agtnacttta ngcctatntt gcgggnag 598

<210> 539
<211> 607
<212> DNA
<213> Homo sapiens

<220>
<221> misc_feature
<222> (1)...(607)
<223> n = A,T,C or G

<400> 539

ggtacaggct ttaacagaaa ttcaggagtt catcagcttt ataagcaaac aaggcaattt 60
atcatctcaa gttcccctta agagacttct gaacacctgg acaaacagat atccagatgc 120
taaaatggac ccaatgaaca tctgggatga catcatcaca aatcgatgtt tctttctcag 180
caaaatagag gagaagctta cccctcttcc agaagataat agtatgaatg tggatcaaga 240
tggagacccc agtgacagga tggaagtgca agagcaggaa gaagatatca gctccctgat 300
caggagttgc aagttttcca tgaaaatgaa gatgatngac agtgcccgga agcagaacaa 360
tttctcactt gctatgaaaa ctactgaagg agcttgcata aagagtcaaa aaaccagaga 420
cgaattggct ggtgagctgg ggtgccaaac tactggcgnc tggagcccct tacccgggag 480
cccgggnccc anggnttggt cttganncag gggcttcaat tggccttgaa aacnagtctt 540
ttttggttgg attagnaacn cacngtgtca agctncttta agccaaaaat tntccnggnt 600
tttnccg 607

<210> 540
<211> 432
<212> DNA
<213> Homo sapiens

<220>
<221> misc_feature
<222> (1)...(432)
<223> n = A,T,C or G

<400> 540
ggtactgatc attctatttc cccctctatt gatccccacc tccaaatatc tcatcaacaa 60
ccgactaatc accacccaac aatgactaat caaactaacc tcaaaacaaa tgataaccat 120
acacaacact aaaggacgaa cctgatctct catactagta tccttaatca tttttattgc 180
cacaactaac ctcctcggac tcctgcctca ctcatttaca ccaaccaccc aactatctat 240
aaacctagcc atggccatcc ccttatgagc gggcgcagtg attataggct ttcgctctaa 300
gattaaaaat gccctagccc acttcttacc acaaggcaca cctacacccc ttatccccat 360
actagttatt atcgaaacca tcagcctact cattcaacca atagccctgg ccgncctcgg 420
ncgtgaccac gc 432

<210> 541
<211> 597
<212> DNA
<213> Homo sapiens

<220>
<221> misc_feature
<222> (1)...(597)
<223> n = A,T,C or G

<400> 541
gggtaccggc gtgtcaaaaa aatgtcagat gacgaggacg atgacgagga ggaatatggc 60
aaggaggaac atgaaaaaga agctattgcg gaagaaatct tccaggatgg ggaaggggaa 120
gaagggcagg aggccatgga ggcccccatg gctcctccag aggaggagga agaagatgat 180
gaggagtcag atattgacga cttcattgtg gatgatgatg gacagcctct gaaaaaacct 240
aagtggcgga aaaagcttcc tggatacaca gacgcggccc tgcaagaagc ccaggaaatc 300
ttcggtgtgg actttgacta tgatgaattt gagaaataca atgagtatga tgaagaactg 360
gaggaagagt atgagtatga ggatgatgan gctgatggtg aaatccgatg cccccccaga 420
agaccaccca gaaacngtgt tgagcccntn ggagcntttt ttgaaatggt ttganncccn 480
gtngggcttt naaagccnnc nccttacnna ttnggggcct tngantcccn gcccttncct 540
gccttnaaag ggtccanntt ccgttncttc ccagtcangg ggnttaaaaa tnatnan 597

<210> 542
<211> 577
<212> DNA
<213> Homo sapiens

<220>
<221> misc_feature
<222> (1)...(577)
<223> n = A,T,C or G

<400> 542
gcccaaggct cagccagtct ctatttaaga aaatttaaca aatacgagta accctgtccc 60
aatcactgaa tctctagtta ctactcttag aaacacctgt ggcttcttgg ccctcctgtt 120
gcccgctctg aatctctctg cagtctacaa aatcgcccca gtcaactctc cacttggagg 180
gaattgtcca gtgtggcccc tagaattgag tcacccccta gataccaact gtctgacccc 240
gaggagctct gtaagtccct gctcctcctc ttccctttgg ggctggtgct gccactcagc 300
aataatcctc ttttctctgt gctttcttag gtccctgtcc tctgtctttg aggctggtta 360
ggaagcaaga gtcctgatct ttcatgctgc acaatatgag catgcaaaaa gctttttcca 420
gcagaacatg ttccctcgtc tccagttgcc cggaaaagga atttggggga tcaaagaact 480
tagcttggnc taccccatgg ttgagttctg gccttggaaa ancccaagcc aagtnangga 540
ccnagacctt ggccggaaac cnttaagggc aattccn 577

<210> 543
<211> 607
<212> DNA
<213> Homo sapiens

<220>
<221> misc_feature
<222> (1)...(607)
<223> n = A,T,C or G

<400> 543
tcgagcggcc gtccggcagg tacattattg ggcctcattt gcccagcaac ggggcatcca 60
gattgagtgc agtcagggcc atgtcttcac tcgggggact cancaggctt atacctcaag 120
caggcacagt gatgcggcgc cttatctctg attggagtgt tacccanatg gtgagtgacc 180
taagtcaggt gaccgttcac ctgatggcct cacccactga agagaatgct gatcactgtc 240
ttgatccctt ggtaacaaag acccacctgc tgagcttgtc ctccctcacc taccaacggn 300
ntancaattc gcacagctga cgaggagctc tctgntcgtg atggggatcc tacctttcat 360
acanatcagc tgcacttagt nnanttacng atttctggac aaactaccaa tcganacatt 420
gcctttgggt aattgatggg tccctnggcc gngacaanct taggggcgaa tttccatnca 480
actgggcggg ccgntactan cngnatccta nctttgggac ctaatcttgt tgtanccatg 540
gcnttacntg tacctctggg taatcntatc cngtnaanta tccnnanctt tactngccng 600
anntnng 607

<210> 544
<211> 570
<212> DNA
<213> Homo sapiens

<220>
<221> misc_feature

<222> (1)...(570)
<223> n = A,T,C or G

<400> 544
acttgggctt ctttcagctg cttcaacaga gtggcagcaa ccaagctgga gtccaagccc 60
cctgataaaa ggcagccaat ccttctgtct gtcatcaaac gtttctttac agcattatta 120
aaaaggatcc tgaggttgtt cttcacagtt tctatctcaa aacctggaaa gagtttctcc 180
acattgtcat agagggcgtg caggggttca tcccgacagt gatgatattt aaccatttcc 240
acggatgcaa ctttgccatt tggctttaaa tccaaaactt catagtgtcc aggaagaaaa 300
ggctccactt ttaaaaaggg agtcgcggag tgcttcaatg taacaagacc tttagcttct 360
gaacatacag ccaaaaatcc atcttctgtc attgctttaa acaaaggtct gactccatat 420
gtatctctac ccaggaacac tttcttattg gcagtatcca gtaaaacaaa tgcnaacaca 480
ccatccaaca tacaaattgn ttgctcaatt cctcctttgg cataaagatg aaggattatc 540
tcaccaatcc acttttggnc tggnattcaa 570

<210> 545
<211> 330
<212> DNA
<213> Homo sapiens

<400> 545
accgtccagg atctccaggt catagccatc agccagacac cagttgacgc ttgtctcctt 60
agtcttcccg gattgccttt tggaatcata tatgctgact ctgccaacct tggggtggtt 120
gacaataaag ggatgtcgta gtccatcctc aaatgcactc ccatctcttg tcacacgaca 180
gcaaatagca cgggtcagat gcccttggct gaaaaggtaa cccaatgtga cagatttgag 240
ataaatgggc tgcaggaagt gggtcaacag tgccccttgc aggcccagca cgttccagcg 300
taggattttg tcactacagg acatggtacc 330

<210> 546
<211> 589
<212> DNA
<213> Homo sapiens

<220>
<221> misc_feature
<222> (1)...(589)
<223> n = A,T,C or G

<400> 546
ggtaccagag gcactgtgga tgggccacgg aatgaattgt cccgggtctc caaaaagaac 60
atttttcttc tatttaagaa gctctgctcc ttccgttacc gcagggatct actgagactc 120
tcctatggtg aggccaagaa agctgcccgt gactacgaga cggccaagaa ctacttcaaa 180
aaaggcctga aggatatggg ctatgggaac tggattagca aaccccagga ggaaaagaac 240
ttttatctct gcccagtata gtatgctcca gtgacagatg gattagggcg tgtcatacta 300
gggtgtgaga gaggtaggtc gtagcattcc tcatcacatg gtcaggggat tttttttttt 360
cctttttttt ttctttttaa gccataattg gtgatactga aaactttggg gttcccattt 420
atcctgcttt ctttgggatt gctaagcaag gncttggcca agccccccct ttttttcccc 480
caaggngaaa agnccnaaan cctaanaagn tatcctttct ttttanccca aggcttccct 540
tagcccttgg nccncctggg ggncccnttc ctttaaaang tttnggttt 589

<210> 547
<211> 613
<212> DNA

<213> Homo sapiens

<220>
<221> misc_feature
<222> (1)...(613)
<223> n = A,T,C or G

<400> 547
ggtaccaggt ttaaatgtag tcttctggag aagtattttt gacattgagc tctgggacag 60
gacaccttgg gtttgtggac tgcagcccac tatgatgtta ttacttctct ggccaggcct 120
ccagtggaag tgcacaggca ctcccaatgt tgttaatgct ctgtcttcca tttgttctgg 180
aatcctacgt gttggtctgt ggttccatgc attagctgtt tgtaaataat gcatttgcat 240
actgaaaaag gaatgccacc tgccacagtt gatggtgagg aagctccttt gacgtggtgc 300
aattttgatg agatgtctct ggggacacga ggatgcccta atgatgctga cttgtcatgg 360
ttgcagcatt tgaacttttg gtgttaaaaa naaaaacctg tnagtctgga accctggcaa 420
cattttacaa ccctngnatt tttaaaagaa ggcntttctt attaaaaaaa ttcnnaaacn 480
ccaccagnnc ctattgggtc aaaccaattc ctncncttnt ggggccnctg gttttttaaa 540
ggggcctttg ctngaancaa ttggnantcc cangggtttc ganaaaaant gaaatggttt 600
tnnnccnccc tcc 613

<210> 548
<211> 578
<212> DNA
<213> Homo sapiens

<220>
<221> misc_feature
<222> (1)...(578)
<223> n = A,T,C or G

<400> 548
ggtacatatg tattttacaa tatacttacc atgagtttag aaaaatttga attcccacca 60
ttctatacca accaaccaca accccactgt ctacattccc cagccagaag acttagaatc 120
catgcttgag ccaaagcctc cattaaaacc actgcccgac cctgcattgg atgctgatcc 180
ccaaccaatt gctgcaccag aattagagcc actataagag ttatttccag aaccgaaggc 240
ctggtttggc tccctctgca tgttgccttg gttttggtta ttacccgatg ggcctgactg 300
gttctgctgg ctggctaaca tgcccatcat accccaactg ctctgtantg ctgcctgggc 360
ggcagccatc atggctggat taatgctgaa cgcacccaag ttcatccacc accatattac 420
tacctttgat ggttnccaaa ncaagtcacc cctntggtta ttaccaaatc caccctggat 480
cccaaagccc cctgggatta ccccccaaan tttcncttnt ttntaaatng ccaatgntta 540
tggggcttaa ggtcngcntt ngatttttga accctgnt 578

<210> 549
<211> 620
<212> DNA
<213> Homo sapiens

<220>
<221> misc_feature
<222> (1)...(620)
<223> n = A,T,C or G

<400> 549

ggtacgcatg tcacttccca tcatggaacc actcatggtt gctggtggaa cgccaggatt 60
agcttcataa cctatgccac caccacctcc tagaggtgga aatttctggc ctcctgaacc 120
atagggatct cccatgttca ttgctcctcc gccacccatt cgcatgtctc tttcccgtgg 180
atccatgtag cccattcggc tgtaactttc ctctctttgg cgcctcattt gttcttccat 240
ctcacgttga cgaatcatca tctcttcctc tcttctacgt cgntcctcct cttgcctcaa 300
ttgcatttct ttacgtttct gcatttcttg attgtgaaag ttcttccatg cgtcttaatt 360
cttcctgtcg tctcatcaga tcttggcgca aaagatttgc ctgatgttca tgatanggca 420
ttttccattt cacttttcca atttggnctt ttggcanctt ttcanngntg tntttcaaac 480
ttnggtncct tttggctggg nttttcccat ntcnatncan atgagnnttg nnntgggngg 540
ggagnantgg tngggnccta nnctgtccgg cccntntnaa angggcgnaa tttcnnaagc 600
cncatgggng ggccggtant 620

<210> 550
<211> 577
<212> DNA
<213> Homo sapiens

<220>
<221> misc_feature
<222> (1)...(577)
<223> n = A,T,C or G

<400> 550
acctatgttt cacctcctgg aaatgaagag gaagaatcaa aaatcttcac cactcttgac 60
cctgcttctc tggcttggct gactgaggag gagccagaac cagcagaggt cacaagcacc 120
tcccagagcc ctcactctcc agattccagt cagagctccc tggctcagga ggaagaggag 180
gaagaccaag ggagaaccag gaaacggaaa cagagtggtc attccccagc ccgggctgga 240
aagcagcgca tgaaggagaa agaacaggag aatgaaagga aagtggcaca gctagctgaa 300
gagaatgaac ggctcaagca ggaaatcgag cgcctgacca gggaagtaga ggcgactcgc 360
cgagctctga ttgaccgaat gggtgaatct gcaccaagca tgaaccaatt ggggagcatc 420
aagtccccca cttgggccac acttacccac cttttccaga agtggcttct gnctaccttt 480
nacttanngc catggtgggn accttaattc ccattcccca gggggaagnt ttgaattacc 540
aaagggaagg gtttnacctn gttttagaaa ttngccc 577

<210> 551
<211> 573
<212> DNA
<213> Homo sapiens

<220>
<221> misc_feature
<222> (1)...(573)
<223> n = A,T,C or G

<400> 551
ggtacaaacc atcttctact gtgacttctt ctacttgtat gtgaccaaag tccttaaggg 60
aaagaagtta agtcttccaa tgccaatctg aggaccttca gagacagtct acgccttaac 120
aagcacatga aggaaactat tttgaatgtt ctctttggca acttatccat aatttgggat 180
caaatgttaa aaccagaaaa gtgtttagtg tggatttcag caaaacctga tcatcccacc 240
cagaagacct tctcatcaat agatcgccct taaagaccca ttgtaaggtc ataaaaaacc 300
tcggccaact gcacaaagat ggtgcctcac tgcaacaaga aaccttaagg tgtcttaccg 360
acgaaataaa aaacataaat gattgntctc caaaggcctg agggcaagac tcatgatgag 420
caagtcaacc cccaatctgg aacaatggcc ttctnttaaa atgncccact taagacccgt 480

taaaatatta ggganctggc ccggcggccc tttaaanggc naattcngnc nctggnggcc 540
ntacttangg gaccaacttn ggnccangtt ngg 573

<210> 552
<211> 581
<212> DNA
<213> Homo sapiens

<220>
<221> misc_feature
<222> (1)...(581)
<223> n = A,T,C or G

<400> 552
ggtacattca ggaataatca tatcactggt tacatacaac tctcatgcaa agaaaaccct 60
caaaaaacaa acaaaaaaaa ccctcagtta gttgtttctt taagtctaat taatccaaac 120
taataatagc catttaatta gcaatctgta aatcagagag gtatagaaat tcagcagcta 180
aactgtattt tccacctata gcactgctgc tactcaaact attttcttca cgtattagaa 240
gaattcatag gcattgatgg tcaaaataag aatttcaaca tagcagcaaa tgacagaaga 300
gtgagagaaa gagctcctaa tgtggtgaca gtcttaatga tcctttaaaa ggtagaagat 360
tgngtgcgta tgtgtggaaa ggagtaggaa agaaaagcat gaggttaaga caggtattta 420
aagggaatgg cgagatagct accttagaat atttattttt ttaaaaaact gctctgaaat 480
ctgcccagtg tacctgcccg gcngncnttc naagggcnaa ttttgncnna tntnnttcan 540
cttggcgggc cgtnnacctg gntttttaan ggccccantt c 581

<210> 553
<211> 575
<212> DNA
<213> Homo sapiens

<220>
<221> misc_feature
<222> (1)...(575)
<223> n = A,T,C or G

<400> 553
ggtactgccc ttggaacctt tgctgagggc tttgtaattc ctagttaaaa tccatttgta 60
atattgtttc tgtaaagcac tcatttccat tcttaaaatc tgctcaacct tggcaggaag 120
agatttttcc acatctttct taactcggcg taacagaaat ggctcaagct ccttgtgaag 180
gcttgcataa ccatattctc tccctttgcc atgttcttct tcaaaatctt cccaggaaga 240
aaacttttct ggcataatga aatgtagcaa agaccagagc tctttgaggg aattctgtag 300
aggagttcca gtgataagga gacgatgatt ggatttaaaa tctattaaag ttttatacag 360
aagggagtca tcattcttta atcggtgtgc ttcatcaaca cctataaatg cccaatttaa 420
gaccttccag ggaatgcctt aaaataatag aaaaacagta ttttgagaga aaaaccggaa 480
ttcaaattta gcccttccat ttaatctgac tcaattatta aaatgaaatn naaattaaaa 540
accaactttg gcctaatttt caaataaaaa atcgn 575

<210> 554
<211> 548
<212> DNA
<213> Homo sapiens

<220>

<221> misc_feature
<222> (1)...(548)
<223> n = A,T,C or G

<400> 554
acggaggact ccattaataa catggaaatc tccactctga aagcgattca ccatttctgt 60
cagcaagtca ggccatttct gtggaaaatc ttctctgcca ataatgctaa ttgcatcact 120
taactgcttc tgaatttgct ctgggctgct aagcatcaag tgcactatgt tggctttaat 180
ggccactcga tcggcttcac aaattttgtt tggttcatct tcaacaattc tccagttcct 240
tttaatatag tttttgaatg ttactgaagc acatactttg ataacattat cctgggactt 300
ctccagtaat gtcaaaagca acagtggata attctgattt ccttcaacag attcaagaaa 360
tttctcagct ggacgtcgga tggcaggatc aggatcaagt gtttctttta aatattctgt 420
tagtgtttgc agatttgcat cgctgagttc cattgctata ggatctcgtg gggatacaga 480
aaccgaggaa ggaaccccag ccgcggaccg taactngcac taccccgcta cctngggcgc 540
gaaacacg 548

<210> 555
<211> 576
<212> DNA
<213> Homo sapiens

<220>
<221> misc_feature
<222> (1)...(576)
<223> n = A,T,C or G

<400> 555
actccctgca taacaagaga ttattttgga gacagttgat aaaaaccata catccttttt 60
attgttaagt cataaagagg tatcaaaatt aaaagcaaaa attacagggt aagacttaac 120
aaaactacta ggagcgtcaa aggaagtgaa aatgggacta ggcgcggggc aatatgaatt 180
aatgaacatg ggaaggacaa ggatggggag aacagtgagc atgtgctgaa gatactaggg 240
gagaggatct ggtgaaaaat ttgatcttag acaagcgcct aggtaaagaa ataatgggat 300
aagatttcta aaccccacta tgtgcttaag agtcatcctc gccattggcg ctgnctctgn 360
catcctctcc ttctcacctc tttttcatca tccttgatca actccagctt ggcatncccc 420
cgatcttcat tatcattaat cttccagtan gncccccttc ttagcanaag taatntgnac 480
cccccttana attcattttt ccatttgnct aaattttttt tccnggacnn gtnggnntgg 540
gcccttttng nnntaaaant tttaantctt acnggg 576

<210> 556
<211> 613
<212> DNA
<213> Homo sapiens

<220>
<221> misc_feature
<222> (1)...(613)
<223> n = A,T,C or G

<400> 556
ggtacctctt cccatgactg cacccagctc caggggccct tgggacagcc agagctgggt 60
ggggacagtg ataggcccaa ggtcccctcc acatcccagc agcccaagct taatagccct 120
ccccctcaac ctcaccattg tgaagcacct actatgtgct gggtgcctcc cacacttgct 180
ggggctcacg gggcctccaa cccatttaat caccatggga aactgttgtg ggcgctgctt 240

ccaggataag gagactgagg cttagagaga ggaggcagcc ccctccacac cagtggcctc 300
gtggttatta gcaaggctgg gtaatgtgaa ggcccaagag cagagtctgg gcctctgact 360
ctgagtccac tgctccattt ataaccccag cctgacctga nacttgtcgg aaaagctgtc 420
ttggggcctt ttatnaaata aaaagacttn agncnatgac aangganggt ttaagaangg 480
gacttgnggg gaantnggaa gnnannaanc ccttggttgg ggtttaagnn nccccacgtt 540
tggcccaggc angtggcttt ttccttnttg ggnccttngg tnncnttgng ggacanaagg 600
nnntttgnac ccc 613

<210> 557
<211> 607
<212> DNA
<213> Homo sapiens

<220>
<221> misc_feature
<222> (1)...(607)
<223> n = A,T,C or G

<400> 557
acctggatga aaagcagagg gaccccagaa tcgaagcgag caaagtgctg ctgtgccatg 60
gggagctgcg gagcaagagt ggacataaac tttacatttt cctgtttcaa gacatcttgg 120
ttctgactcg gcccgtcaca cggaacgaac ggcactctta ccaggtttac cggcagccaa 180
tcccagtcca agagctagtc ctagaagacc tgcaggatgg agatgtgaga atgggaggct 240
cctttcgagg agctttcagt aactcagaga aagctaaaaa tatctttaga attcgcttcc 300
atgacccctc tccagcccag tctcacactc tgcaagccaa tgacgtgttc cacaagcagc 360
agtggttcaa ctgtattcga gcggccattg cccccttcca gtcggcaggc aagtccacct 420
gaactgcagg gcctggccgg agctgtacga aaaatgtgaa ggggaaccac cctttgcgag 480
gaactnacag cccaaaggaa ggcattcaca gtttcagtgg tacttcaggt agaaagttga 540
tgaaaaccct taccagantg tggcttttgg cattgcaaat ggcagaggcc agcaagaact 600
taaannt 607

<210> 558
<211> 355
<212> DNA
<213> Homo sapiens

<220>
<221> misc_feature
<222> (1)...(355)
<223> n = A,T,C or G

<400> 558
acaaagacaa agaaacaaac tacattggca tttaagccaa tcaaaaaagg aaagaagaga 60
aatccctggt ctgattcaga atcagatagg agcagtgacg aaagtaattt tgatgtccct 120
ccacgagaaa cagagccacg gagagcagca acaaaaacaa aattcacaat ggatttggat 180
tcagatgaag atttctcaga ttttgatgaa aaaactgatg atgaagattt tgtcccatca 240
gatgctagtc cacctaagac caaaacttcc ccaaaactta gtaacaaaga actgaaacca 300
cagaaaagtg tcgtgtcaga ccttgaagct gatgatgtta agggcagtgt acctn 355

<210> 559
<211> 597
<212> DNA
<213> Homo sapiens

<220>
<221> misc_feature
<222> (1)...(597)
<223> n = A,T,C or G

<400> 559
acccgcaaaa cgggacatag tatgtgacaa tctgcatcga tcatggacta ctaaatgcct 60
ttacatagaa gggctctgat ttgcacaatt tgttgaaaaa tcacaaaccc atagaaaagt 120
aagtaggcta agttggggag gctcaaacca ttaagggtta aaaatacatc ttaaacattg 180
gaaagctctt ctagctgaat ctgaaatatt accccttgtc tagaaaaagg ggggcagtca 240
gaacagctgt tccccactcc gtggttctca aaatcataaa ccatggctac tcttgggaac 300
cacccggcca tgtggtcgcc aagtagagca agcccccttt ctcttcccaa tcacgtggct 360
gagtgtggat gacttttatt ttaggagaag ggcgattaac actttttgac agtattttgn 420
tttgccctga tttgggggat tgntttgttt ttggtgggtt gttttggaaa aacnggttat 480
aaactgggtt tttgnangnt ttgggatttt aaagcccnaa ataaaaaann nnanaaaaaa 540
aaagnctttg gnctttgggc cggaaaccct taangggcna attccagcca ccttggg 597

<210> 560
<211> 559
<212> DNA
<213> Homo sapiens

<220>
<221> misc_feature
<222> (1)...(559)
<223> n = A,T,C or G

<400> 560
gactttgagg caagtgtggg ccactgtggt ggcagtggag gtggggtgtt tgggaggctg 60
cgtgccagtc aagaagaaaa aggtttgcat tctcacattg ccaggatgat aagttccttt 120
ccttttcttt aaagaagttg aagtttagga atcctttggt gccaactggt gtttgaaagt 180
agggacctca gaggtttacc tagagaacag gtggttttta agggttatct tagatgtttc 240
acaccggaag gtttttaaac actaaaatat ataatttata gttaaggcta aaaagtatat 300
ttattgcaga ggatgttcat aaggccagta tgatttataa atgcaatctc ccttgattta 360
aacacacaga tcacacacac acacacacac acacaaaccn tntgcctttg atgttacaga 420
ttttantccg ttnattttta aggatagagc ctttatnggt gnnnanaaaa caatctggan 480
taaaaaaaac ncnccnggcc ttgnatttng ncttnntngg ggtttcccca aanccattnn 540
nnttgncagg ctnggggng 559

<210> 561
<211> 569
<212> DNA
<213> Homo sapiens

<220>
<221> misc_feature
<222> (1)...(569)
<223> n = A,T,C or G

<400> 561
ggtacaagct tttttttttt tttttttttt tttttttact ttttgggana naggctagga 60
ggaggaaggg gtgaaaacag cgtctcactg gagtctcaaa agtgtatgaa tcttctggta 120

[?]t[?]c[?]a[?][?]a[?] [?][?][?]a[?]aa[?]a[?] [?][?]cca[?][?][?]aa [?][?]ca[?]a[?][?][?]a aaa[?]ccccaa gattcttttt 180
gctactgatt tctataatta aaatatgaca tatgtaaggg actagtgcat gatattcaat 240
aaatgtcagt tgtctttcct aactaggttc ctcacaggct aggttatgcc tanatatcat 300
catcctcctt tcagggaatg aagctcacct agaaaactag ggaactaaaa gtgcaatatg 360
gtttgggtaa tgcagttggt tagctgctcc ccatcctccc aactcactat tccagggagg 420
ggctgaaaac agaaatggct cccctgaagc tanntagcat ggcatgcana gtcncatgaa 480
aggtttgggc tggaattttt aagccaagnc ctnttttttg gaaaaaaatn ttgggaaaaa 540
ancccnnccc tnctgnttcn nagctgttt 569

<210> 562
<211> 597
<212> DNA
<213> Homo sapiens

<220>
<221> misc_feature
<222> (1)...(597)
<223> n = A,T,C or G

<400> 562
cgaggtacgg atgctacttg tccaatgatg gtaaaagggt agcttactgg ttgtcctccg 60
attcaggtta gaatgaggag gtctgcggct aggagtcaat aaagtgattg gcttagtggg 120
cgaaatatta tgctttgttg tttggatata tggaggatgg ggattattgc taggatgagg 180
atggatagta atagggcaag gacgcctcct agtttgttag ggacggatcg gagaattgtg 240
taggcgaata ggaaatatca ttcgggcttg atgtggggag gggtgtttaa ggggttggct 300
agggtataat tgtctgggtc gcctaggagg tctggtgaga atagtgttaa tgtcattaag 360
gagagaagga agagaagtaa gcccgagggc cgtctttgat tgtgtagtaa ggggtggaag 420
gtgattttat ccggaatggg aagtgatnct aaggggggtt gtttganncc cttttcntgc 480
cntaaantgg angtngaatt ccnnntnngg cncncatana ttanaggcca aaatnaaatt 540
gaanggnnaa aaaancttnn anggggggga ctgntnnntg agaacccccc taaaatn 597

<210> 563
<211> 574
<212> DNA
<213> Homo sapiens

<220>
<221> misc_feature
<222> (1)...(574)
<223> n = A,T,C or G

<400> 563
   acgccaagaa ccgtattctt tgccacaggg ttttatgtgg gacactttag acttgagtga 60
   tgccgaagtg ctcaaggagt tatacacgtt gttaaatgag aattacgtag aagatgatga 120
   caatatgttc cgatttgact attcacccga gttcctgttg tgggctctgc gtccaccagg 180
   ctggctcctg cagtggcact gtggggtcag agtgtcttca aataaaaaac tggtcgggtt 240
   cataagtgcc atcccagcaa acattcggat ttatgacagt gtgaagaaga tggtagaaat 300
   caactttctt tgtgttcata agaagttgag atcgaaacgg gtagccccag tgctaatccg 360
   agagatcact agaagagtga acctggaagg gatcttccag gctgtgtcaa aagcacactt 420
   ctccanncct cngggccctg cattcctgcg cttntntnna gacactttcc ctttctattt 480
   tactgnggtg actttttcaa acgctgtnac cccaaccctt anantttttn gcccttggcg 540
   gnntatnggt taaanatcac ccttcccngg gttt 574

<210> 564
<211> 600
<212> DNA
<213> Homo sapiens

<220>
<221> misc_feature
<222> (1)...(600)
<223> n = A,T,C or G

<400> 564
ggtacagaat atttctaata aacctaaatt taatcacagt taaaatttct caaaagtatt 60
ttcaagtgct caagaatatt aaagtttggg gggaaatacc taagtcataa ataagcaagt 120
attccctcca agattcacta attgggataa aagtctcagg gtaagcccac aagaatggtc 180
tgcaataaag aaaaatcagg tctgtgtaga gtaatttctg ccatctttag cagaaaagcc 240
aaaaacattc tgagccaaat aaaagcaaag atcttttgat tcagcgcctt ttgttgtgtt 300
agttttaatt tctaacttct caacatgtta tagctcagaa attcccatat gcttactatc 360
tgtaataagg aactataacg ttaaagaaaa aattcagaga ccgtgatcat tttccatcat 420
aggtctggct ctctttggta gaaacagatc aagacttact ttatttttct cttccccncc 480
ngaagaaaan gggggtttta atggcnttta cccttgnnaa anaacccncg ngggtttaac 540
cttnaaattn ggnggggtaa aanancctaa ngntnagccc tttttnanaa ctnggggnaa 600

<210> 565
<211> 600
<212> DNA
<213> Homo sapiens

<220>
<221> misc_feature
<222> (1)...(600)
<223> n = A,T,C or G

<400> 565
accatcggcc atgtggacca cgggaagacc acactgactg cagccatcac gaagattcta 60
gctgagggag gtggggctaa gttcaagaag taccaggctg tttgtgatcg tatcagccgc 120
tatgtgaaac agcctttacc tgatgagttt ggcagctcac ccttggagcc aggggcctgc 180
aatggctcca ggaacagctg tgaaggagaa gatgaggaag aaatggagca tcaggaagaa 240
ggcaaagagc agntttnana aacagaaggc agnggggaag atgagccagg aaatgacccc 300
agtgagacca cccaaaagaa gatcaaaggc cagccctgcc caaaaaggct tntttaccnt 360
cagtcttgtg aactcctatg gaacagctga cataaatttc actttgcagc tnatggaaaa 420
ctacntaaac tcaantnttc ganctacact tggncntgga tttgtgacnt ttgaaaactn 480
tggaganttt tnctatgnnt gtgcncnnaa atttntaggg nttntccnat aaatctctgt 540
tanccttttt gggnaccntt tcnaagnaag atntnangnc cctanggncc nttnaaaaan 600

<210> 566
<211> 576
<212> DNA
<213> Homo sapiens

<220>
<221> misc_feature
<222> (1)...(576)
<223> n = A,T,C or G

<400> 566
ggtactgaac aggtaagtca tccctcagcc agagattagt ctacttcttc catgcgtgat 60
gtgtcgtcat ctccttcaag ggtgtttttc tttatttttg ttaatattaa aaagtctgta 120
tggcatgaca actactttaa ggggaagata agatttctgt ctactaagtg atgctgtgat 180
accttaggca ctaaagcaga gctagtaatg ctttttgagt ttcatgttgg tttattttca 240
cagattgggg taacgtgcac tgtaagacgt atgtaacatg atgttaactt tgtggtctaa 300
agtgtttagc tgtcaagccg gatgcctaag tagaccaaat cttgttattg aagtgttctg 360
agctgtatct tgatgtttag aaaagtattc gttacatctt gtagggatct actttttgaa 420
ctttttcatt ccctgnaggt gacaantctg catggacctg ccccgggcgg cccttnaaan 480
ggcgaanttc annncantgg ngggcnntct tngggnnccn ncctggncca aatntggggg 540
ancngggnca anctnttccn tggggaaatg gntccc 576

<210> 567
<211> 427
<212> DNA
<213> Homo sapiens

<400> 567
ttttggcagt aaatcaattt tatttgtgtt cacagaacat actaggcgat ctcgacagtc 60
gctccgtgac agcccaccaa cccccaaccc tctacctcgc agccacccta aaggcgactt 120
caagaagatg gaaggatctc acggatctca ttcctaatgg tccgccgaag tctcacacag 180
tagacagacg gagttgagat gctggaggat gcagtcacct cctaaactta cgacccacca 240
ccagacttca tcccagccgg gacgtcctcc cccacccgag tcctccccat ttcttctcct 300
actttgccgc agttccaggt gtcctgcttc caccagtccc acaaagctca ataaatacca 360
agagacctgc atttacagca gggggaacat ctcacaccct tgcataagtt aaaataaata 420
ttaccgt 427

<210> 568
<211> 616
<212> DNA
<213> Homo sapiens

<220>
<221> misc_feature
<222> (1)...(616)
<223> n = A,T,C or G

<400> 568
acaagagtga tggcaatgtg actggaacag aaatagtttc taccaggcac acaaaagctc 60
ctgtaagccc cgtagttccg tcctgcaaag ggcctcagtg ggaaccaggt ctgcagaccc 120
gagtgggcag agagacgggt ggaagcaggt gccccagatg gtcccgcagg cgtcaccgtc 180
tggtttggag accttaaggg agttgtgctt caaacttctc tcccagggtc tcaggtggag 240
actagggagt ttgacctaaa ggtcctccaa ggagaggcca aggtcttgga gacagatctg 300
gtttaccatc ttttaacaaa aggcaaatgt cttctcttct tcagaaagag tcattaacac 360
taaaattctt ttcttnngaa gtttcttctt ttccgatgcc atcttccaag tttgnnccca 420
agaatgaaag gcgtcttttn ccnaagggtc aagggtttcc attcacnttg ggccccattg 480
naaaagggac tggttccttt tggggggttg ggncccggac cccccaaana aggnaanggn 540
ttttgtnccc aagcctttnt tcccnggggn gggaagggna anaacctttg ggcccgngna 600
acccacctta angggg 616

<210> 569
<211> 582

<212> DNA
<213> Homo sapiens

<220>
<221> misc_feature
<222> (1)...(582)
<223> n = A,T,C or G

<400> 569
acagaatata acgcagcttg gcaggatgca tacggccctg cgcaggggaa agtatttcaa 60
atcagctggc aggttcaagc ctttctgcac tgtagacttt ccacactctg gaaaagaagc 120
aaacaaacaa accccaaaga acccccgaaa aaaacaaaaa ccatccggga ggtgcatgag 180
tccaatggga atgcaaccgt gatgccgctg tcctatgccc agtgacagca caggtcacgt 240
aagttacagc aggggagggg tagctcaagc tacagaggat tattgtcata ttgctaagac 300
agcataaatc cattcaaaaa aaaaaaaaaa aatccaaacc agggtaagta aagaaaggaa 360
aaccaaatct atacagcatt tacaacaaat aaatctctag ccagctgggg gtaaaatatg 420
catctatgta tagactatgt gtagggtaag aaaagctttt aatatnggtt anaaagaggn 480
cctttgatta aaggccttgg cccgaacncc cttaaggnnn aattcnagnc nattgggggc 540
cggtcnaagg ggatccaacn tgggnccaaa nttggngaat nn 582

<210> 570
<211> 557
<212> DNA
<213> Homo sapiens

<220>
<221> misc_feature
<222> (1)...(557)
<223> n = A,T,C or G

<400> 570
ccgggcaggt acttcttgcc tttaagatag gcaccaggaa atctttcaag gatctcatag 60
tcactctcca atttatagag ggctgacaat ctggcttcca ttaaaatgag taatcgtcct 120
ctggcaacat ctttaatttt cacatattgc atttctggat taacacacac agcaaggtta 180
ctaggtagag tccagggagt ggttgtccaa gcaactaaag atacagtttc atcttcttcc 240
aaagggaaag ttacaaatac tgaaggatct tgaacatcct tataattctg gtgtgactcg 300
aagttggaaa gtggagtgtt acatgccgta gagaagggca tgactttcac acctctataa 360
acaaggcctt tatcatagag ttggttgaag acccaccaga ctgattccat gaattgtgga 420
tacagagttt tatagtcatt ggcaaagtna atncatcggc aagttgctac aggagacttc 480
actnannnaa atctcatcnc aatnnntgga ctnatggata cctnggannc ccntttngcc 540
caatctgggc ctngatn 557

<210> 571
<211> 382
<212> DNA
<213> Homo sapiens

<400> 571
acactgctct cttcctggca attgacagtg gtaaccctcc cgctacgggc actgggactt 60
tgctgataac cctggaggac gtgaatgaca atgccccgtt catttacccc acagtagctg 120
aagtctgtga tgatgccaaa aacctcagtg tagtcatttt gggagcatca gataaggatc 180
ttcacccgaa tacagatcct ttcaaatttg aaatccacaa acaagctgtt cctgataaag 240
tctggaagat ctccaagatc aacaatacac acgccctggt aagccttctt caaaatctga 300

[?] [?] [?] [?] [?] cccatgacga 360
atatcacaga tctcagggta cc 382

<210> 572
<211> 621
<212> DNA
<213> Homo sapiens

<220>
<221> misc_feature
<222> (1)...(621)
<223> n = A,T,C or G

<400> 572
   acaagctttt tttttttttt tttttttttt tttttttgcc atttattgcc atgttttaaa 60
   attcgtgcaa aatatntgaa gccctggaca gagaatacaa agtgatattt tcccaagaaa 120
   cntaaaacta ggaaaagggg tgggggacat tttcccacca nagctncccc cacgccaggc 180
   cccaagcagg gtgaggcctn caacccggcc agctgagcag ggaggactaa gagctacaat 240
   ctggaccang gaaggagggg tggaatttgc aacagngtnt taactaccaa cgagaggaaa 300
   gccagtcaac tgtacaacct cttgcggagc ggggaaggtg actaccngaa caagacatgc 360
   tgcctgccct gtgcttgtgg gctgcaaagt gggnntccaa taagtggttc catgaacgag 420
   gacaggagtt tttgancctt gnggatcaac aaaangttna ctgacatccn tttctgcctt 480
   tccctttcct ggnnctttta anccatgtca acnntgacan acncctntng atggtccctt 540
   tggnagtcct aatnaggctg atttttggan nantnaatnt ttttttggaa cncaaggnga 600
   acntttttgg ngaattttng g 621

<210> 573
<211> 296
<212> DNA
<213> Homo sapiens

<220>
<221> misc_feature
<222> (1)...(296)
<223> n = A,T,C or G

<400> 573
ggtactcatt gtgctctttg gtgcctttcc tttcctacag aaaaggaagt gatctatacc 60
aaggtttgca gggaagtcaa atgttctcaa cctttcatgc cctctggtta ctcatctggc 120
ttgcaaaata atttggatcc ggacagattt ccagtatttt caagtccgct gctttcccgc 180
aaagctcggc ctaacctgga gctagttagg tccgcaggcg ccaccgncgg cgcactccgg 240
agaagaagct ccttcttcag ccgcccagga gagttcctcg agaaagatgc cgccgc 296

<210> 574
<211> 616
<212> DNA
<213> Homo sapiens

<220>
<221> misc_feature
<222> (1)...(616)
<223> n = A,T,C or G

<400> 574
ggtactccaa cgccaccctg tgcagaaatg agagaagaca gtgctagagt ctatgaaaac 60
gtgggcctga tgcaacagca gaaaagtttc agatgagaaa acctgccaaa acttcagcac 120
agaaatagat gtggactttc accctctccc taaaaagatc aagaacagac gcaagaaagt 180
ttatgtgaag acagaatttg gatttggaag gcttgcaatg tggttgacta ccttttgata 240
agcaaaattt gaaaccattt aaagaccact gtattttaac tcaacaatac ctgcttccca 300
attactcatt tcctcagata agaagaaatc atctctacaa tgtagacaac attatatttt 360
ataggaattt gtttgaaatt gaggaagcag ttaaattgtg cgctgtattt tgcagattat 420
ggggattcaa attctagtaa taggcttttt tattttattt ttataccctt aaccaggtta 480
attttttttt ttcctcattg gtnggggatg atgagaagaa atgattnggg aaaattaagt 540
accaacgnac tagaaaagtg agaaccattc tatttcccnt ntggttccng gagnggataa 600
ttcatttgan ggcttn 616

<210> 575
<211> 614
<212> DNA
<213> Homo sapiens

<220>
<221> misc_feature
<222> (1)...(614)
<223> n = A,T,C or G

<400> 575
ggtacaaaca ttttacaaaa aagaacatta ccaatatcag tggcagtaag ggcaagctga 60
agaataaata gactgagttt ccgggcaatg tctgtcctca aagacatcca aactgcgttc 120
aggcagctga aacaggcttc tttcccagtg acaagcatat gtggtcagta atacaaacga 180
tggtaaatga ggctactaca taggcccagt taacaaactc ctcttctcct cgggtaggcc 240
atgatacaag tggaactcat caaataattt aaacccaagg cgataacaac gctatttccc 300
atctaaactc atttaagcct tcacaatgtc gcaatggatt caagttactt gcaaacgatc 360
ccgggttgtc atacagatac ttgnttttta cacataacgc tatgccatcc cttncttcac 420
tgcccagtca ggtttcctgn tgttggaccg aaaggggatc cttttaaaaa tgcttcnttc 480
aagacagaag tgagaaagaa aggagaccct gaggccagan ctattaaaac ttgtgngtcc 540
ccaaaaggaa ggggaaaggn agaattgaaa ggaaacggnt ctttngccca ggatnggaan 600
cgggactacn ttgg 614

<210> 576
<211> 596
<212> DNA
<213> Homo sapiens

<220>
<221> misc_feature
<222> (1)...(596)
<223> n = A,T,C or G

<400> 576
acatcaagac ttttggaaca gcgatcgtaa tcaatcctga gaaagacaaa gacatggtcc 60
aagacctgtt ggacttcaag gacaaggtgg accacgtgat cgaggtctgc ttccagaaga 120
atgagcggtt cgtcaacctg atgaaggagt cctttgagac gttcatcaac aagagaccca 180
acaagcctgc agaactgatc gcaaagcatg tggattcaaa gttaagagca ggcaacaaag 240
aagccacaga cgaggagctg gagcggacgt tggacaagat catgatcctg ttcaggttta 300
tccacggtaa agatgtcttt gaagcatttt ataaaaaaga tttggcaaaa agactccttg 360

[?]gggaaaag tgccccagcc gatgctgaaa agtcctatgt gtcaaagctc aagcatgagt 420
gcggtgcagc cttcaccagc aagctggaag gntgttcaag gacatggagc tttcaangac 480
atcatggtca tttcaagcca gcntatgcag natcngagtg cttcaggcct atagacctac 540
agggacatct nccatggctt ctngccacat aacnccatgg aangccttac cccaaa 596

<210> 577
<211> 617
<212> DNA
<213> Homo sapiens

<220>
<221> misc_feature
<222> (1)...(617)
<223> n = A,T,C or G

<400> 577
ggtaccacaa ctcccaggat tttcctggat caaaccttgt atctcttctg caagtattgt 60
gtatattggt ctgagagacg tggaccctcc tgaacatttt attttaaaga actatgatat 120
ccagtatttt tccatgagag atattgatcg acttggtatc cagaaggtca tggaacgaac 180
atttgatctg ctgattggca agagacaaag accaatccat ttgagttttg atattgatgc 240
atttgaccct acactggctc cagccacagg aactcctgtt gtcgggggac taacctatcg 300
agaaggcatg tatattgctg aggaaataca caatacaggg gttgctatca gcactggatc 360
ttgttgaagt caatcctcag ttggccacct cagaggaaga ggcgaagact acagctaacc 420
tggcagtaga tgtgattgct tcaagctttt ggtcagacca gaagaangaa ggcatattgg 480
ctatgaccaa ctttctactc ccagttcacc agatgaatca gaaaatcaag cncctgtgan 540
aaattaggag acacttngcc ctggcatgtt tacaaaaagg ctttnngaaa tntgangcct 600
ttaggggaaa aaataaa 617

<210> 578
<211> 409
<212> DNA
<213> Homo sapiens

<400> 578
ggtacatgca gaattgtcaa ctacagggaa tgaaaagttc aaaaagtaga tcctacaaga 60
tgtaacgaat acttttctaa acatcaagat acagctcaga acacttcaat aacaagattt 120
ggtctactta ggcatccggc ttgacagcta aacactttag accacaaagt taacatcatg 180
ttacatacgt cttacagtgc acgttacccc aatctgtgaa aataaaccaa catgaaactc 240
aaaaagcatt actagctctg ctttagtgcc taaggtatca cagcatcact tagtagacag 300
aaatcttatc ttccccttaa agtagttgtc atgccataca gactttttaa tattaacaaa 360
aataaagaaa aacatccttg aaaatatatt atcagaggaa ttgtagagt 409

<210> 579
<211> 619
<212> DNA
<213> Homo sapiens

<220>
<221> misc_feature
<222> (1)...(619)
<223> n = A,T,C or G

<400> 579

[?][?][?][?][?][?][?][?][?][?] [?][?][?][?][?][?][?][?][?] [?][?][?][?][?][?][?][?][?] [?][?][?][?][?][?][?][?][?] [?][?][?][?]agagag actagatgct 60
ttcactaggg aatgtcttcc cacccagcca tcacaaatgt ggacaatcac tgcatccaca 120
tctgtaggca tatttctatg gaagtttaat tgacagctat attcattatt tattttacaa 180
tttcattttt ctacaccttt gagatttatg aatgcagttt tttcttaaaa tttattttaa 240
cttgacagta tgtttttagt tcccccaatt taattaatgg accatgtgca tatatatggg 300
agtgtgctta catgttaata atttacttgc atacttatga gaatttcaca ttggaattca 360
taatggtaaa acaacataca tctgccaata tacgtttttt ctgntggttt aagagaagat 420
aactgacagc tttacctact tcctacagat gcatctaaac ccagatttac tgagaagaag 480
tgtattggac tctgagtgga aaaagagtat ggtgtttttt ggttttaagn tctgctctag 540
anccataatt ngnaaaaaat tttaggnctt aanctggtnc cctaaaattg gnnanccaaa 600
ngttnaatga aanggctgc 619

<210> 580
<211> 632
<212> DNA
<213> Homo sapiens

<220>
<221> misc_feature
<222> (1)...(632)
<223> n = A,T,C or G

<400> 580
ggtacaaaca ttttacaaaa aagaacatta ccaatatcag tggtagtaag ggcaagctga 60
agaataaata gactgagttt ccgggcaatg tctgtcctca aagacatcca aactgcgttc 120
aggcagctga aacaggcttc tttcccagtg acaagcatat gtggtcagta atacaaacga 180
tggtaaatga ggctactaca taggcccagt taacaaactc ctcttctcct cgggtaggcc 240
atgatacaag tggaactcat ataacaacgc tatttcccat ctaaactcat ttaagccttc 300
acaatgtcgc aatggattca gttacttgca aacgatcccg ggttgtcata cagatacttg 360
ntttttacac ataacgctgt gccatccctt ccttcactgn cccagtcagg tttcctgttg 420
gtggaccgaa aggggatcat tttaagaaat gcttccttna agacagaaag tgagaaagaa 480
aaggagaccc ttgaggncag gaactaatta aacctggtgt ggtgccccaa aagggaaggg 540
ggaaaggccg gaanttgnaa nggataaccg nttcntttng cccagggant cnggaaccgt 600
ggctcgcttt gggcttggac anncccaaat cc 632

<210> 581
<211> 607
<212> DNA
<213> Homo sapiens

<220>
<221> misc_feature
<222> (1)...(607)
<223> n = A,T,C or G

<400> 581
acataagtga tggagtatca atgctggtgg ttgaggtgga gaaggaattt agttccttga 60
attttctttg ttctcctctg tgttccttct tggccaggta acccctgcta tatcataaga 120
tttcatctgc gagaaaagga ggaattcttc tacagctccc ctgctcaact ttcaggagat 180
tttgacccat gtgctgttaa tcaccgaaat tttttaagga ggcttctcct ggcatgaaag 240
agttggtatt gtgtcccgaa ttggttggtt cttggtctca ctgacttcaa aaatgaagcc 300
gcggaccctc gcggtgagtg ttaacagctc ttaaggtggc acgtctggag tttgttcctt 360
ctgatgttcc ggatgtgttc agagtttctt ccttctggta ggttcctggc ctcgcttggc 420

ttcaggaatg aagctgcaga ccttctcggt nagtgntaca agctcttaan gcaggccgtc 480
tggaagttgt tcgttcctcc tggggctcgt ggtcttgctg gctttaggag tcaagtncaa 540
accttnaggg tgagtgtaca ntcatanaag cagtgtngnc ccaanaatna ncnttnaaaa 600
gccaacn 607

<210> 582
<211> 603
<212> DNA
<213> Homo sapiens

<220>
<221> misc_feature
<222> (1)...(603)
<223> n = A,T,C or G

<400> 582
actgtattct ccatatgtag ctcggatgcg gagggctgtg agattccgca gtaaccttcg 60
atactcaaag taactcagct gggggctcca attattgctt ggatgctcat ttaacctgaa 120
tgtgtaagtc ttggtgagcc cacaaggcag tgtcttgcca agtggcatca agggagctgt 180
gatccgtaga ccagcacctt ccagaatcac atcatgggca gatgggtgtc tgcctcctct 240
gtccacacgg tagtcaaagg acaggctttg accatagctc acctgttgat tcccaagaaa 300
tttggcagga gccacaaaat agacagggtc tagtcgttgg gctgagctaa acacatcttg 360
atgggcgctg tgaccattgg agctttgcag gagacccatt tcgttggaca gccttccagc 420
catcaacatc ttgatgaaag gtanaagtga tcttatggac actgnattct gcanaactgc 480
ggcaacttgg ctgaatgcca tagcagaacc ctgggtacct tnggccggaa cacgcttang 540
gcgaattcag cccacttggg gccgtctann ggnanccact ttgggcccan cttggggaan 600
ant 603

<210> 583
<211> 535
<212> DNA
<213> Homo sapiens

<220>
<221> misc_feature
<222> (1)...(535)
<223> n = A,T,C or G

<400> 583
ggtacacaca ggaccgcctg gggctaaagg aaatggacaa tgcaggacag ctagtgtttc 60
tggctacaga aggggaccat cttcagttgt ctgaagaatg gttttatgcc cacatcatac 120
cattccttgg atgaaacccg tatagttcac aatagagctc agggagcccc taactcttcc 180
aaaccacatg ggagacagtt tccttcatgc ccaagcctga gctcagatcc agcttgcaac 240
taatccttct atcatctaac atgccctact tggaaagatc taagatctga atcttatcct 300
ttgccatctt ctgttaccat atggtgttga atgcaagttt aattaccatg gagattgttt 360
tacaaacttt tgatgtggtc aagttcagtt ttagaaaagg gagtctgttc cagatcaagg 420
gccagaactg tgcccaggcc caaaggagac actaactaaa gtagtgagat agattctaan 480
ggcaaacatt ttccaggctt gccatatttc aagcaanaag ggccnaagcc tgagg 535

<210> 584
<211> 524
<212> DNA
<213> Homo sapiens

<220>
<221> misc_feature
<222> (1)...(524)
<223> n = A,T,C or G

<400> 584
acaactctct taaaagagta tggataacta tattttctgg attctggagg ttgataacca 60
tatgcactta acattatatt ctataaacat taagtagtgc cagttatgag attcccagtt 120
cttactaaat tgtattagca ggagctggta attacttgta ttatcacatg taactaataa 180
tttgaactat acttgaagga ccgtgttgat gtcaggtatt tacagtggtt ggaagatagc 240
agtattatta gcataagctg catacgtaat attcagtaac tgccatatta tataacaaat 300
ttacattcgc aaattcagta tcctgttaaa gtgtcatatt cttgtaatct gcattctcca 360
ggagttttat gtgtttaata gatgaattta ttttatttnt aaaggtattc aaatgntttc 420
agccncntat aggagaaata cccaagtata ttctagttcc ttnatgtccc tgnaccctcg 480
gccgngacca cgctaaaggg cgaaatncaa ncncactggn nggn 524

<210> 585
<211> 618
<212> DNA
<213> Homo sapiens

<220>
<221> misc_feature
<222> (1)...(618)
<223> n = A,T,C or G

<400> 585
actgactata atcaaactcc gaataccatt aaaattaagc tatgcagtcg gaacgtgggt 60
gataacgtcc acgctcgcga ggggaacaac ccagatcgtc agctaaggtc ccaaaattgt 120
gttaagtgag aaaggttgtg agatttcata aacaactagg aagttggctt agaagcagcc 180
accttttaaa gagtgcgtaa ttgctcacta gtcaagagat cttgcgccaa taatgtaacg 240
ggactcaaac acaataccga agctacgggc acattatgtg cgttaggaga gcgttttaat 300
ttcgttgaag tcagaccgtg aggactggtg gagagattaa aagtgagaat gccggcatga 360
gtaacgattc gaagtgagaa tcttcgacgc ctattgggaa aggtttcctg ggcaaggttc 420
gtccacccag gggttagtca gggcctanga tgaggcanaa atgcatagtc gatggacaca 480
ggttaatatt cctgtacctt cggncgngaa cacgctaagg gccgaattnc agcacacttg 540
gcgggnggtc ctagtnggat cccanctntg ganccaactt nggggtaatc ntgggcttan 600
ctggttccct ggtgaaat 618

<210> 586
<211> 337
<212> DNA
<213> Homo sapiens

<400> 586
acaagctttt tttttttttt tttttttttt tgtttcaagt tttaatcaaa gcttgtatat 60
aagattactt tattcctgca tcttctcaat ggtttcttcc ttgtatttgc ccttttcctt 120
tcctacttgg cgagatttgg ctttccgttc gaggatcttt ttgcggtctt tgtccagttt 180
tagcctagtg ataaccacct tgctggggtg aatgcctacg tggacagttg tgccattagc 240
cttttcccgc tgcacccgtt caatgtagat aacatatttc ttcctgtaaa cctggactac 300
tttgccaatt tgctgacctt tatagtgtcc acgtacc 337

<210> 587
<211> 656
<212> DNA
<213> Homo sapiens

<220>
<221> misc_feature
<222> (1)...(656)
<223> n = A,T,C or G

<400> 587
   cgaggtacaa gctttttttt tttttttttt tttttttctt gaggagtggc atggagttct 60
   ttaatttgga aggcaaaagg ttacatttaa tgaaaggcag aggctggatt aataaatgtt 120
   tgttanaaag ttgttctgac acacagtgaa ctctgggctt ttctcctgca taaaaagcag 180
   agctagcagt aagtgcaaat ntgaagaaaa tccatgtgtc caataagctg ccatctccan 240
   aactcttatc caggaaattc aaagagtgaa cattctttta gtctcctact cctcaattaa 300
   gtaaatgaga atgattcagc caacaaagtt catgacaaca aggtgcagga tggtgctggc 360
   aaanagaaaa tnagcaaagg ctcgctctgg ggagatgcct tggaaatccn ntttgntctg 420
   ngggttgatc tgnattcttc agggnaaacc cgctagggat gaaacttccc acccnaagan 480
   aatgaaaccc cgaaagaaaa agangtttaa aggggaaagg ncccccngan ggagaccagt 540
   tacccgaact tggaacnncc ccggcaagca attttttcnc ggcagggtnc cctggcccng 600
   ggcggccntt tnaaaagggg gcaattncca ngncacttgg gggggcgttt tttnng 656

<210> 588
<211> 586
<212> DNA
<213> Homo sapiens

<220>
<221> misc_feature
<222> (1)...(586)
<223> n = A,T,C or G

<400> 588
   actcaaacac aggggggttg tcatttatgt caagaactga tacaatcaca gtgccagtgg 60
   cagtcagcct ccttggcaag ccttgatcca cagctttcaa agagagggtg tatactgcct 120
   ggagttctct gtccaaaggt ttttctaact gaataattcc agataattcg ttaatggaga 180
   actgcccatc agcagagtca atcagtgagt ataaaatctt ccgatttaat cctgcgtcgg 240
   catctgtggc ctgcactctt gtcagcagcg ttcccggctc tgtgttttca aacacggtga 300
   tggcataagg atcggcagag aattcggggg cattatcgtt cacgtcttct agcgtgagca 360
   caatactggc ttggtagaat cttcctcctc catctgtggc cctgacgaga agatgataaa 420
   cagcttgctc ctnacgatca aaggggggtt gacgttttca agtcacctgg nctggattaa 480
   tttgaatttt ctgcacctga cccaatacgg taagtattca gcgtaaccgg atgttgcgtt 540
   gacanaaact gatgacattt tccgaaggac tnttaggaaa aggtga 586

<210> 589
<211> 645
<212> DNA
<213> Homo sapiens

<220>
<221> misc_feature
<222> (1)...(645)

<223> n = A,T,C or G

<400> 589
acaagcagta ttagaaaatc tttttggcaa gggagagaaa taaatacaaa tggaatgcta 60
catttttaaa ttagcaaact gtctcaggaa tgataaaggt atcagtaaag tagcaagggg 120
ataactttaa aacattattt gtctggggct caaaaaacac tcaaaacaat ttatttaaag 180
gttgcacaag agctatgtcc aggcatttac gcttatggga agtaaaatta aaagaggata 240
cttttttccc aaggagaatt tctttaaaac caagcacatt gctaaatagc aacattatac 300
tcggtaaaca ataattggca acaaaataag tttaatattc tgcccaaacc agtcccagat 360
actgtttaat aaccaagata caaactaatt ttgttgnaac aagcctagac caattttatc 420
aaacatgtcc ttggttagat atccaatttc atttaacgtt tttgnaagct canttgacag 480
ccagtcnagt ccttnatacn gacccagttc cntggggttg gcacaaagtg ggnttggacc 540
atacccacca ttcaaaaagg cgcatntngg ttcttggccc aaaaaatccn ggnaaaaaaa 600
aggganggga aattattnaa gggncccttg ggnggnaatg ggcnc 645

<210> 590
<211> 464
<212> DNA
<213> Homo sapiens

<400> 590
ggttcttgac gaggctgcgg tgtctgctgc tattctccga gcttcgcaat gccgcctaag 60
gacgacaaga agaagaagga cgctggaaag tcggccaaga aagacaaaga cccagtgaac 120
aaatccgggg gcaaggccaa aaagaagaag tggtccaaag gcaaagttcg ggacaagctc 180
aataacttag tcttgtttga caaagctacc tatgataaac tctgtaagga agttcccaac 240
tataaactta taaccccagc tgtggtctct gagagactga agattcgagg ctccctggcc 300
agggcagccc ttcaggagct ccttagtaaa ggacttatca aactggtttc aaagcacaga 360
gctcaagtaa tttacaccag aaataccaag ggtggagatg ctccagctgc tggtgaagat 420
gcatgaatag gtccaccagc ttgtacctgc cgggcggccg ttcg 464

<210> 591
<211> 387
<212> DNA
<213> Homo sapiens

<220>
<221> misc_feature
<222> (1)...(387)
<223> n = A,T,C or G

<400> 591
ggaagacgga ggtcctcttt ccttgcctaa cgcagccatg gctcgtggtc ccaagaagca 60
tctgaagcgg gtggcagctc caaagcattg gatgctggat aaattgaccg gtgtgtttgc 120
tcctcgtcca tccaccggtc cccacaagtt gagagagtgt ctccccctca tcatttccct 180
gaggaacaga cttaagtatg ccctgacagg agatgaagta aagaagattt gcatgcagcg 240
gttcattaaa atcgatggca aggtccgaac tgatataacc taccctgctg gattcatgga 300
tgtcatcagc attgacaaga cgggagagaa tttccgtctg atctatgaca ccaagggtcg 360
ctttgctgta cctnggccgc gacacgc 387

<210> 592
<211> 648
<212> DNA
<213> Homo sapiens

<220>
<221> misc_feature
<222> (1)...(648)
<223> n = A,T,C or G

<400> 592
ggtacaaaca ttttacaaaa aagaacatta ccaatatcag tggtagtaag ggcaagctga 60
agaataaata gactgagttt ccgggcaatg tctgtcctca aagacatcca aactgcgttc 120
aggcagctga aacaggcttc tttcccagtg acaagcatat gtggtcagta atacaaacga 180
tggtaaatga ggctactaca taggcccagt taacaaactc ctcttctcct cgggtaggcc 240
atgatacaag tggaactcat caaataattt aaacccaagg cgataacaac gctatttccc 300
atctaaactc atttaagcct tcacaatgtc gcaatggatt cagttacttg caaacgatcc 360
cgggttgtca tacagatact tgntttttac acataacgct gtgccatccc ttccttcact 420
gncccagtca ggtttcctgt tgntggaccg aaaggggata cattttanga aaatgctttc 480
ttcaagacag aaatgagaaa gaaanggaga accctgaggc caggaatcta ttaaaccctg 540
ggggtngnnc nccaaaaggg aagggggnaa aggccnggaa tttgaaaagg ntaaaaccgn 600
ttccttttgn gncccaggga attagggaaa ccttgactna cntttggg 648

<210> 593
<211> 625
<212> DNA
<213> Homo sapiens

<220>
<221> misc_feature
<222> (1)...(625)
<223> n = A,T,C or G

<400> 593
ggtacttaaa atcagagtca aaaaatggtt ttaagtttta atactcttaa ttagctccct 60
gctttatact gtaactccac agaagacata gggccaccta ggattcacag gaaggagcag 120
ctctgattct tacatggctg gctccgatgc ccccacagca ggcctcttcc tccccaagtt 180
tttcctctcc atttcaaaaa agcactattt tatcttcaca tccaagagct ggttggtttg 240
gtttgtttct ttggaaacca ataaaagaag caattttttc ctgttctttt tactcacatc 300
tacctatcag agcggctatt tccttcgaca gttcagtagc acacaggctg acttggccac 360
atggactcat gaatgcatgc attcagaccg catattgcta ccaaatggga atgtgggaat 420
atgctatgca cctcaggttg agaaatgacc aagaaaatca agatctaaag gggtgatata 480
taatatatat atatatcaat gctattattc ataaaaacct tggttagtaa taaaaaaaat 540
tgctttggtt naaatattga atattataag ctggcttctc atgggttgga aaaaataagt 600
ctttntgnaa aagccggggc ctttt 625

<210> 594
<211> 586
<212> DNA
<213> Homo sapiens

<220>
<221> misc_feature
<222> (1)...(586)
<223> n = A,T,C or G

<400> 594

ggtacccaga caaaacccgg ccacgtgtaa gtcagatgct gattttgact ccatttcaag 60
gtcaaggcca tggtgctcaa cttcttgaaa cagttcatag atactacact gaatttccta 120
cagttcttga tattacagcg gaagatccat ccaaaagcta tgtgaaatta cgagactttg 180
tgcttgtgaa gctttgtcaa gatttgccct gtttttcccg ggaaaaatta atgcaaggat 240
tcaatgaaga tatggcgata gaggcacaac agaagttcaa aataaataag caacacgcta 300
gaagggttta tgaaattctt cgactactgg taactgacat gagtgatgcc gaacaataca 360
gaagctacag actggatatt aaaagaagac taattagccc atataagaaa aagcagagag 420
atcttgctaa gatgagaaaa tgtctcagac cagaagaact gacaaaccag atgaaccaaa 480
tagaaataag catgcaacat gaacagcttg gaananaagt tttcanggnc tagtggaaga 540
ataccccggc gtggtattga acnacttgct caagagttaa gaattt 586

<210> 595
<211> 613
<212> DNA
<213> Homo sapiens

<220>
<221> misc_feature
<222> (1)...(613)
<223> n = A,T,C or G

<400> 595
acagaaggtt gacgaaaatt cttactgagc aagaaataac cttgttgtaa ttactaaaat 60
ttgagaaatg tgattcttga ctggaaaaat agatgtgtcg tggaggccga atgtttgcac 120
caaccaaaac ctggcgccgt tggcatcgta gagtgaacac aacccaaaaa cgatacgcca 180
tctgttctgc cctggctgcc tcagccctac cagcactggt catgtctaaa ggtcatcgta 240
ttgaggaagt tcctgaactt cctttggtag ttgaagataa agttgaaggc tacaagaaga 300
ccaaggaagc tgttttgctc cttaagaaac ttaaagcctg gaatgatatc aaaaaggtct 360
atgcctctca gcgaatgaga gctggcaaag gcanaatgag aaaccgtcgc cgtatccagc 420
gcaggggccc gtgctcatct ataatgagga tnaatggtat catcaaggcc tttagaaaca 480
tcctggaaat acctctgctt aatggtaagc caagcttgac cattttgaan ncctgttctg 540
gtgggccttt tgggacgttc tggatttgga cttgaaaggc ttttccggaa ttnnatgaaa 600
tgnccnncgg ccc 613

<210> 596
<211> 616
<212> DNA
<213> Homo sapiens

<220>
<221> misc_feature
<222> (1)...(616)
<223> n = A,T,C or G

<400> 596
   gcgtgggtcg cggccgaggt acaagaacac tccttgggcg tccttgctgt tttgtttgtg 60
   aagttttcta tgcccagtgt tcctgacttc gaaacgctat tctcacaggt tcagctcttc 120
   atcagcactt gtaatgggga gcacattcga tatgcaacag acacttttgc tgggctttgc 180
   catcagctaa caaatgcact tgtggaaaga aaacagcccc tgcgaggaat tggcatcctt 240
   aagcaagcca tagacaagat gcagatgaat acaaaccagc tgacctcaat acatgctgat 300
   ctctgccagc tttgtttgct agcaaaatgc tttaagcctg ccttccatat cttgacgtgg 360
   atatgatgga tatctgtaaa gagaatggag cctatgatgc aaaacacttt ttatgntact 420
   attattatgg agggatgatt atactgggct gaaagaactt tgaaagactc tctactttta 480
   tgaacaggct atactacttc tgcatggcgg cagtcatatc atgtgggaac atttaaaagn 540
   ntatttanng gcttgaatac ctggcaaaga cctgnccggc gccgttcaaa ggggaattca 600
   ccacttggng gcgtnt 616

<210> 597
<211> 631
<212> DNA
<213> Homo sapiens

<220>
<221> misc_feature
<222> (1)...(631)
<223> n = A,T,C or G

<400> 597
accagatggc ttttcagaca gaggttggaa accatcccac ttttgaggat atgcaggttc 60
tcgtgtctag ggaaaaacag agacccaagt tcccagaagc ctggaaagaa aatagcctgg 120
cagtgaggtc actcaaggag acaatcgaag actgttggga ccaggatgca gaggctcggc 180
ttactgcaca gtgtgctgag gaaaggatgg ctgaacttat gatgatttgg gaaagaaaca 240
aatctgtgag cccaacagtc aatccaatgt ctactgctat gcagaatgaa cgcaacctgt 300
cacataatag gcgtgtgcca aaaattggtc cttatccaga ttattcttcc tcctcataca 360
ttgaagactc tatccatcat actgacagca tcgtgaagaa tatttcctct gagcattcta 420
tgtccagcac acctttgact atagggggaa aaaaacccga aattcaatta ctatgaaccg 480
acagcaaggc acaaagctcg aatncccaag cccttgaaac aagtggtaac cagcttttca 540
ccacancacc aaccnncaaa cnccccaggg anttacgccc aaggtacctt nggccgggaa 600
cccncttang gggnaattcn cgncccttgg g 631

<210> 598
<211> 630
<212> DNA
<213> Homo sapiens

<220>
<221> misc_feature
<222> (1)...(630)
<223> n = A,T,C or G

<400> 598
cgaggtgctt cgtcttcggt ttttctcttc cttcgctaac gcctcccggc tctcgtcagc 60
ctcccgccgg ccgtctcctt aacaccgaac accatgcctt caattaagtt gcagagttct 120
gatggagaga tatttgaagt tgatgtggaa attgccaaac aatctgtgac tattaagacc 180
atgttggaag atttgggaat ggatgatgaa ggagatgatg acccagttcc tcctcctcct 240
cctcctgaag atgatgagaa caaagaaaag cgaacagatg atatccctgt ttgggaccaa 300
gaattcctga aagttgacca aggaacactt tttgaactca ttctggctgc aaactactta 360
gacatcaaag gtttgcttga tgttacatgc aagactgttg ccaatatgat caaggggaaa 420
actcctgagg agattcgcaa gaccttcaat atcaaaaatg actttccctc tttttttgta 480
agcaatggct ggctaagtta atgggccagg taacntttag tgacctttta aaaagtttgg 540
ccattggnaa atnaaaccac ttgcaaaaaa gttttntgga atagaatttc cnaatatttt 600
cctttttcat gagtgggaac tgggnaaagg 630

<210> 599
<211> 359
<212> DNA

<213> Homo sapiens

<400> 599
ggtacctacc tcaggagcag agatttgata ttcgagtgct gggcttaggt ctgctgataa 60
atctagtgga gtatagtgct cggaatcggc actgtcttgt caacatggaa acatcgtgct 120
cttttgattc ttccatctgt agtggagaag gggatgatag tttaaggata ggtggacaag 180
ttcatgctgt ccaggcttta gtgcagctat tccttgagcg agagcgggca gcccagctag 240
cagaaagtaa aacagatgag ttgatcaaag atgctcccac cactcagcat gataagagtg 300
gagagtggca agaaacaagt ggagaaatac agtgggtgtc aactgaaaag actgatggt 359

<210> 600
<211> 589
<212> DNA
<213> Homo sapiens

<220>
<221> misc_feature
<222> (1)...(589)
<223> n = A,T,C or G

<400> 600
acccagggac acaaacactg tggaaggctg cagggacctc tgcctaggaa agccaggtat 60
tgtccaaggt ttctccccat gtgacagtct gaaatatggc ctcgtaggaa gggaaagacc 120
tgaccgtccc ccagcccgac acccataaag ggtctttgct gaggaggatt agtaaaagag 180
gaaggcctct ttgcagttga gataagagga aggcatctgt ctcctgctcg tccctgggca 240
atggaatgtc tcggtttaaa acccgattgt atattctatc tactgagata ggagaaaact 300
gccttagggc tggagatgag acatgctggt ggcaatactg ctctttaatg cattgagatg 360
tttatgtatg tgcacaaaaa agcacagcgc ctttttcttt acctcgttta tgatgcagag 420
acatttgttc acatgttttc ctgctgactc tctcccacta ttaccctatt gcctgccaca 480
tctccttttc gaaanggtag agataatgat caataaatac tgagggactn aganactggg 540
ccgcgtaagt cctaatatct gaacgccagt ccctggccca ntttttttnt 589

<210> 601
<211> 240
<212> DNA
<213> Homo sapiens

<400> 601
acatctgaaa taccccccaa acccagaaag cttttcaaca gctaggttgt ccaagaactt 60
ggaaaattca ccttctgatg tcctccaaga cagattccat tttttataca ccttatttgc 120
tcagacctgt aacttcagcc tggagtgaac acagacacct agttttcctc aaactcctct 180
tgggctttag agagaaggtg ctggcccttt gagccaagca ggttattggt tagtagtacc 240

<210> 602
<211> 621
<212> DNA
<213> Homo sapiens

<220>
<221> misc_feature
<222> (1)...(621)
<223> n = A,T,C or G

<400> 602
ggtacctttt acatacaaga aattaaatga gagaaaaaat aactgtagtt acaccatatc 60
acttacaaga atggagaatc tgcttataag tcaaactaga attagaactt atttcttaga 120
ctgcttcata aaaactaaca taccactact ttttaattat ttatttattt gctaaagaac 180
aaaaatttaa gtatgaaaaa caaccaactg attcacccaa ctcagtaagt ttgactcacg 240
ttttctggtt caacaccaat gtcttcacaa aatttctcca tgccttcagg gcctacaaca 300
tcatcagttc ctgcatattc atagaaccat tccaagcacc ttttacttga aaaggcttct 360
tcttcagtct ttattctagt cgaatcatat tttctataca tgctatcatg tctacttttc 420
ttggcagata aatcatctcc agaagcaggt cttctctttt tccttggtgg catcacttta 480
ttaaagcagt ctgaagaact gnaagaaccg agacttcttg gtttggcgac gncttggnca 540
nggctctggt anggtcaanc ttattaangg ngngggaaaa ccttntgaan atttgccccn 600
gttganagat gaaaagtcnn g 621

<210> 603
<211> 655
<212> DNA
<213> Homo sapiens

<220>
<221> misc_feature
<222> (1)...(655)
<223> n = A,T,C or G

<400> 603
acttataatt ggcagtggag gaagggaaca tacgctggcc tggaaacttg cacagtctca 60
tcatgtcaaa caagtgttgg ttgccccagg aaacgcaggc actgcctgct ctgaaaagat 120
ttcaaatacc gccatctcaa tcagtgacca cactgccctt gctcaattct gcaaagagaa 180
gaaaattgaa tttgtagttg ttggaccaga agcacctctg gctgctggga ttgttgggaa 240
cctgaggtct gcaggagtgc aatgctttgg cccaacagca gaagcggctc agttagagtc 300
cagcaaaagg tttgccaaag agtttatgga cagacatgga atcccaaccg cacaatggaa 360
ggctttcacc aaacctgaag aagcctgcag cttcattttg agtgcagact tccctgcttt 420
ggttgtgaaa gggcancggg cttgcaactt ggnaaaaggg tgaatggttg ccaaagaagc 480
caaagaaana aggncctgca aagcntgtan cctttgggcc gggaaccacg cttaangggc 540
cnaaattcca agnacaactt ggccgggccc gttacctaaa ngggatccca actttngggn 600
acccaaaacn ttngggngna aatcatnggg ncnaaaantt tggtttccct gngng 655

<210> 604
<211> 490
<212> DNA
<213> Homo sapiens

<220>
<221> misc_feature
<222> (1)...(490)
<223> n = A,T,C or G

<400> 604
   acaacacacg aattccactc taaacttgaa cgcaaagcta tgttcctctc tgcctcatgg 60
   cagtgggcca cagcatcctt caatctttta gttgagcgat acaactccac tagccggatg 120
   ttcacatgga cgtcatcagg tcttacataa agttctgact gaatcaagtc aaaaagttta 180
   ttccatccat cttcaccttc acaatctaga agctgttcct ttagtttata aattgcagga 240
   cttcctggga aaagttttgc tgctctttcg acccagtatt ttgctcttcc atcaggtaac 300
   atcattttta caaagcaatt ctgcaatctt caacacaaga tcttttgtgt tgggtttaat 360
   cccactgaac gcctgtaaca ttnaacggnt ttctctgtgt tttcttccat tcataaagan 420
   gacccagaaa tctgtgagct ttgggatccc tctctcgcac attaaatgta agtacctngg 480
   gncgcgacca 490

<210> 605
<211> 612
<212> DNA
<213> Homo sapiens

<220>
<221> misc_feature
<222> (1)...(612)
<223> n = A,T,C or G

<400> 605
acagaaggtt gacgaaaatt cttactgagc aagaaataac cttgttgtaa ttactaaaat 60
ttgagaaatg tgattcttga ctggaaaaat agatgtgtcg tggaggccga atgtttgcac 120
caaccaaaac ctggcgccgt tggcatcgta gagtgaacac aacccaaaaa cgatacgcca 180
tctgttctgc cctggctgcc tcagccctac cagcactggt catgtctaaa ggtcatcgta 240
ttgaggaagt tcctgaactt cctttggtag ttgaagataa agttgaaggc tacaagaaga 300
ccaaggaagc tgttttgctc cttaagaaac ttaaagcctg gaatgatatc aaaaaggtct 360
atgcctctca gcgaatgaga gctggcaaag gcaaaatgag aaacccgtcg ccgtatccag 420
ccgcaggggc ccgtgcatca tctataatga ggataatggg tatcatcaag gccttcagaa 480
acatccctgg aattactctg cttaatgnaa gcaagctgac attttgaac cctgcttctg 540
ggnggcctgt nggactttct gcatttggac tgaaantgct tttcggaagt ttantaantg 600
gacctnngcc cc 612

<210> 606
<211> 577
<212> DNA
<213> Homo sapiens

<220>
<221> misc_feature
<222> (1)...(577)
<223> n = A,T,C or G

<400> 606
gactttgagg caagtgtggg ccactgtggt ggcagtggag gtggggtgtt tgggaggctg 60
cgtgccagtc aagaagaaaa aggtttgcat tctcacattg ccaggatgat aagttccttt 120
ccttttcttt aaagaagttg aagtttagga atcctttggt gccaactggt gtttgaaagt 180
agggacctca gaggtttacc tagagaacag gtggttttta agggttatct tagatgtttc 240
acaccggaag gtttttaaac actaaaatat ataatttata gttaaggcta aaaagtatat 300
ttattgcaga ggatgttcat aaggccagta tgatttataa atgcaatctc cccttgattt 360
aaacacacag atacacacac acacacacac acacacacac aaaccttctg cctttgatgt 420
tacagattta atacagttta tttttaaaga tagaatcctt ttataggtga gaaaaaaaca 480
atctgggaag aaaaaaccac acaagacatt gatcagcctg ttngcgtttc canangtctt 540
tgattggcag catggttnca aggaaantag gtacctc 577

<210> 607
<211> 312
<212> DNA
<213> Homo sapiens

<400> 607
ggtaccaggc cgctcaccac agtccgtggt tcagcttccc ccacgtcaat cttctctaca 60
tacaggctgt ctgcatctgg gtgcttctcc acagtgatga ttttccccac acggatatcc 120
agccgggatg ggatgacctc ctctggttct gaattcttgg cagggccttt ggccattggc 180
ttctgctttg agggatctgg gtaggcagcg ctggccagtt ttttcagggc aggggtatta 240
aacttttccc ggattggatc cagcaacttg ttcagtgcga cttcaacaga attcttcagg 300
tctccaggat gt 312

<210> 608
<211> 614
<212> DNA
<213> Homo sapiens

<220>
<221> misc_feature
<222> (1)...(614)
<223> n = A,T,C or G

<400> 608
ggtgcaactt ccttcggtcg tcccgaatcc gggttcatcc gacaccagcc gcctccacca 60
tgccgccgaa gttcgacccc aacgagatca aagtcgtata cctgaggtgc accggaggtg 120
aagtcggtgc cacttctgcc ctggccccca agatcggccc cctgggtctg tctccaaaaa 180
aagttggtga tgacattgcc aaggcaacgg gtgactggag gggcctgagg attacagtga 240
aactgaccat tcagaacaga caggcccaga ttgaggtggt gccttctgcc tctgccctga 300
tcatcaaagc cctcaaggaa ccaccaagag acaaagaaac agaaaaacat taaacacagt 360
gggaatatca cttttgatga gattgtcaac attgctcgac agatgccggc accgatcctt 420
agccagagaa ctctctggaa ccattaaaga gatctgggga ctgcccagtc agtgggctgn 480
aatggtgatg gcccgcatnc ttatgacttc atcgatgaca tcaacagtgg tgctgtggaa 540
tgcnagccgg ttaanccnaa ggaaacttta atnanggtca ttgcactggn aaaaaaaaaa 600
nnaananaaa ggnt 614

<210> 609
<211> 609
<212> DNA
<213> Homo sapiens

<220>
<221> misc_feature
<222> (1)...(609)
<223> n = A,T,C or G

<400> 609
ggtactgagc acccctgttg tcaagaaagt gggagtaaca tctgtaggag gttctttaac 60
tggtgggcca aatatataaa caactctgtt aacgttgtga cacatgcgag gtataagcct 120
agccagaaaa ataagtgatt cccagtcagg ttcatcttta ctggagattc cacacacgta 180
attgtaggaa cgacagtcac cctgcacacc tacagtttta attggcagca agaaggcatt 240
cagtgaatgc agactggtaa tttgcatcag cttctcctga tcctcttctg ttgtgcaggc 300
tttgactctc tgtaataggg tatgtggctt tttaacactt gcagaaaaat cagctactat 360
tttcaaaata ttgttggttt caggaaagtc cttacaaata taaggttctt cagcacatat 420
tactctgatt gccaggccag gacctggaaa tggatgcctg gaaactaact cttctggaag 480
tccaagttct cttggccaaa attctcactt catctttatg aaaatctttc agaggtctat 540
acttttcctc ctttttaact ttctgaatga ctcttgggna tttggaangg tttgatgagt 600

tcactttnc                                                          609

<210> 610
<211> 254
<212> DNA
<213> Homo sapiens

<220>
<221> misc_feature
<222> (1)...(254)
<223> n = A,T,C or G

<400> 610
accattggtg gccaattgat ttgatggtaa gggagggatc gttgacctcg tctgttatgt      60
aaaggatgcg tagggatggg agggccgatg aggactagga tgatggcggg caggatagtt     120
cagacggttt ctatttcctg agcgtctgag atgttagtat tagttagttt tgttgtgagt     180
gttaggaaaa gggcatacag gactaggaag cagataagga aaatgattat gagggcgtga     240
tcatgaaaga cctn                                                    254

<210> 611
<211> 687
<212> DNA
<213> Homo sapiens

<220>
<221> misc_feature
<222> (1)...(687)
<223> n = A,T,C or G

<400> 611
ggtacaagga tgccatccat ttctataaca agtctctggc agagcaccga accccagatg      60
tgctcaagaa atgccagcag gcagagaaaa tcctgaagga gcaagagcgg ctggcctaca     120
taaaccccga cctggctttg gaggagaaga acaaaggcaa cgagtgtttt cagaaagggg     180
actatcccca ggccatgaag cattatacag aagccatcaa aaggaacccg aaagatgcca     240
aattatacag caatcgagct gcctgctaca ccaaactcct ggagttccag ctggcactca     300
aggactgtga ggaatgtatc cagctggagc ccgaccttca tcaaggggtt atacacggaa     360
agccgctgca ctggaagcga tgaaggacta cacccaaaag cccatggatg tgtacctgcc     420
cgggccggcc gctcgaaagg ggcgaaattn agcacactgg ccggccggta cttagtggga     480
tncnancttc ggtaccaaac ntngcggnaa tcatgggcat ancnnggttc ctngggngga     540
aaattggtaa tnccgtttac natttcccca ccaacttccn aacccggaaa ccttnaagng     600
gaaanccntg gggnggccta atgggngggc ttactcncct taattggctt gggcttaatg     660
ggcccctttt caatngggaa acctnnt                                      687

<210> 612
<211> 673
<212> DNA
<213> Homo sapiens

<220>
<221> misc_feature
<222> (1)...(673)
<223> n = A,T,C or G

<400> 612
   gactgatgtt ggtgtcctgc agcgccacgt ttcccgccac aaccaccgga acgaggatga 60
   ggagaacaca ctctccgtgg actgcacacg gatctccttt gagtatgacc tccgcctggt 120
   gctctaccag cactggtccc tccatgacag cctgtgcaac accagctata ccgcagccag 180
   gttcaagctg tggtctgtgc atggacagaa gcggctccag gagttccttg cagacatggg 240
   tcttcccctg aagcaggtga agcagaagtt ccaggccatg gacatctcct tgaaggagaa 300
   tttgcgggaa atgattgaag agtctgcaaa taaatttggg atgaaggaca tgccgcgtgc 360
   agactttcaa cattcatttt gggttcaagc acaagtttct ggccagccga cgtggtcttt 420
   ngcaccatgt ctttgatgga gagccccgan aaaggatggc tnaaggaccg aatcacttta 480
   tncaggcttt tggacangcc tnttcaggag tnaccctgga caaacttgta cctttgggnc 540
   ggngaacacc ncttaagggc naatttcang cacactggcg ggccgtaatt aagggaatcc 600
   aacttnggna nccaancttg gggnaaancn tgggcataan ngttccctgn ggnaaatngt 660
   attccctncc aat 673

<210> 613
<211> 279
<212> DNA
<213> Homo sapiens

<400> 613
ggtacaaaag gagacaatcc atccccgaaa gtcatataag atgaactctt cctgtgcaga 60
tatcctgctc tttgcctcct ataagtggaa tgtctcccgg ccctcattgc tggctgactc 120
caaggatgtg atggacagca ccaccaccca gaaatactgg attgacatcc agttgcgctg 180
gggggactat gattcccacg acattgagcg ctacgcccgg gccaagttcc tggactacac 240
caccgacaac atgagtatct acccttcgcc cacaggtgt 279

<210> 614
<211> 653
<212> DNA
<213> Homo sapiens

<220>
<221> misc_feature
<222> (1)...(653)
<223> n = A,T,C or G

<400> 614
   gtttccacaa acttcgtgga tcaaaacgag gtcttccagt tctgcgggtc agaaggctga 60
   cccggggctc aaatctgggt gtcggcagtc ctgcactcct tctggaggct ctaggggaga 120
   attcatttct ggccttttca tttttagagg ctgaccgtaa ttcttgactt caggctcctc 180
   catcttcaga gccagctgtg ggtagttgaa tctttttccc gtcacctcat tgaggcctcc 240
   cctctcctgc ctccctccac cacttttttt tttttttgag acagggtctt gctgtgttgc 300
   ccaggctgga gtgcagtggc ctggtcatgg catcaaggct cactgcagcc tggacctcct 360
   ggttcaagtg atcctcttgt ctcagtcccc tgagacaatc ccccacgccc agctacatat 420
   tttttgtgga tacagggtct cattctgntg cctagcttgt ctggaactcc tgggctcaag 480
   ggatcttgga gccttaaccc tnctaaagtg cttgggaata taggcatgag tcactggacc 540
   ttgggnccga ccaccttaan ggccgaattt cagcacaatt ggcgggccgg tacttagggg 600
   annccaactt tgggaccaac ntgggngnaa tcatgggccn aactggttnc cng 653

<210> 615
<211> 676
<212> DNA
<213> Homo sapiens

<220>
<221> misc_feature
<222> (1)...(676)
<223> n = A,T,C or G

<400> 615
acatgtgaag attttttggc agcttagcgt ggaaaccatt gatcaccctg ctctcatttc 60
tacctgttct gtgttggcaa gggagagtgc ccaaatgagc aagatatcgc agcaaaacag 120
cactccaggg gtgaacggaa ttagtgttat ccatacccag gcacatgcca gcggcttaca 180
gcaggttcct cagctggtgc ctgctggccc tgggggagga ggcaaagctg tggctcccag 240
caagcagagc aaaaagagtt cgcccatgga tcgaaacagt gacgaagtat cggcaacgcc 300
gagagaggaa caacatggct gtgaaaaaga gcccggttga aaagcaagca gaaagcacaa 360
gacacactgn agagagtcaa tcagctcaaa gaagagaatg aacggttgga aagcaaaaat 420
caaattgctg accnanggat taagtgtacn gaagcatgcc aacgccttag ctnatggccc 480
tggctnctat cagcttggga acccnaaagn accagttttt ccangaatcc ccagaccgaa 540
ngggnccaag gggnccaacg ttcgggactt gaaangggaa aaaaaacttg gancttggca 600
aggacttggg cttncnaaat tgganccgan cccaanggat gaanaacccc ttcaagaaaa 660
ccagcttcct ttctng 676

<210> 616
<211> 694
<212> DNA
<213> Homo sapiens

<220>
<221> misc_feature
<222> (1)...(694)
<223> n = A,T,C or G

<400> 616
ggtaccttct agatcttgga gttgatatga atgaaccaaa tgcctatgga aatacacctc 60
ttcatgtagc ctgctataat ggacaagatg ttgtagtgaa tgaacttata gactgtggtg 120
ctattgtgaa tcaaaagaat gaaaaaggat ttactccttt gcactttgct gctgcatcaa 180
cacatggagc attgtgttta gagcttctag ttggcaatgg ggccgatgtc aatatgaaga 240
gtaaagatgg gaaaacccca ctacacatga ctgctctcca cggtagattc tcccgatcac 300
aaaccattat ccagagtgga gctgtaatcg actgtgagga taagaatgga aatacccctt 360
tgcacatagc aacacggtat ggccatgaan ctgctgatca acacttctta ataccagtgg 420
gtgctgaccc ttgcaaannc gtgggcatac cttggaatgg ttcccccttc cattttggca 480
agcccttaaa ccggnttttt caagaattac tggcnnaaaa accttcnttc ttttanggaa 540
ttnganattn gaaanccccc aanggaattt tngccnggac cttgggntaa catgccantt 600
gnnacttgga agggnaattt gggaanggcc tnaaaccttt tnggngnaaa cctggggccn 660
aacntttatt aaaangggcc caatttnggg gaan 694

<210> 617
<211> 554
<212> DNA
<213> Homo sapiens

<220>
<221> misc_feature
<222> (1)...(554)
<223> n = A,T,C or G

<400> 617
cgaggtaccg caagggaaag atgaaaaatt ataaccaagc ataatatagc aaggactaac 60
ccctatacct tctgcataat gaattaacta gaaataactt tgcaaggaga gccaaagcta 120
agacccccga aaccagacga gctacctaag aacagctaaa agagcacacc cgtctatgta 180
gcaaaatagt gggtagattt ataggtagag gcgacaaacc taccgagcct ggtgatagct 240
ggttgtccaa gatagaatct tagttcaact ttaaatttgc ccacagaacc ctctaaatcc 300
ccttgnaaat ttaactgtta gtccaaagag gaacagctct ttggacacta ggaaaaaacc 360
ttgtagagag agtaaaaaat ttaacaccca tagtaggcct aaaaagcagc caccaattaa 420
gaaagcgttc agactatatc tattgcgcca ggtttcaatt tctatcgcta tactttattt 480
gggtaaaatg ggtttggctt aagggtggct nggaagaaag gtggaatngg aactgcccgg 540
gcnggccgct ngaa 554

<210> 618
<211> 305
<212> DNA
<213> Homo sapiens

<400> 618
acatgtgttc acaagggtta ctcctcaaaa cccccagttc tcactcatgt ccccaactca 60
aggctagaaa acagcaagat ggagaaataa tgttctgctg cgtccccacc gtgacctgcc 120
tggcctcccc tgtctcaggg agcaggtcac aggtcaccat ggggaattct agcccccact 180
ggggggatgt tacaacacca tgctggttat tttggcggct gtagttgtgg ggggatgtgt 240
gtgtgcacgt gtgtgtgtgt gtgtgtgtgt gtgtgtgttc tgtgacctcc tgtccccatg 300
gtacc 305

<210> 619
<211> 604
<212> DNA
<213> Homo sapiens

<220>
<221> misc_feature
<222> (1)...(604)
<223> n = A,T,C or G

<400> 619
acactctcat agtcactgaa agtaatatac actgacctgc aaaagtcaga tgggaagaca 60
taaaggacct catctttggt tattagtggg tgaaaagaat ctccatctgt tccattaatc 120
atattgcact tgtctgttat ccaccagtca agtgacgttt tcccattcca ttccacaatt 180
tttgtaaagt taaggtaact gtcttctcca gttagaaaaa catagtctcc atcattagtc 240
ccatttttct catagaatag gccaaaatag ggagagatat cgggcctgaa aacatggata 300
agggacaaga tttcatcttt gtagccccag agcaattcgt caactgtgtg agtcacaaag 360
agcttctgct gataggcttt caacatggcc tcgatgatct ccctgaggaa gtgcacctgg 420
gaccactcta tgacagtcaa tacaggaata tttaatggtc taattaagtn aaattttaag 480
ggctncaaca gattgggtct cgttcaaaac cataggcctt gttgctaaca gcaganattg 540
gtggttcatt atctncaaat ggaaaattng ctttggttct ggagtncctg naagggtatg 600
gncc 604

<210> 620
<211> 571
<212> DNA
<213> Homo sapiens

<220>
<221> misc_feature
<222> (1)...(571)
<223> n = A,T,C or G

<400> 620
ggtactgtga acatgacttt cagatgctct ttgccccttg ctgtcatcag tgtggtgaat 60
tcatcattgg ccgagttatc aaagccatga ataacagctg gcatccggag tgcttccgct 120
gtgacctctg ccaggaagtt ctggcagata tcgggtttgt caagaatgct gggagacacc 180
tgtgtcgccc ctgtcataat cgtgagaaag ccagaggcct tgggaaatac atctgccaga 240
aatgccatgc tatcatcgat gagcagcctc tgatattcaa gaacgacccc taccatccag 300
accatttcaa ctgcgccaac tgcgggaagg agctgactgc cgatgcacgg gaactgaaag 360
ggggaactat actgncttcc atgccatgat aaaatggggg tcccattgng gtgcttgcca 420
cggccatcaa ggcgctgtga cctatggcaa catgcatgtg gacatttggt gnncagtgta 480
aaccttntga atgcatataa gaagctgcgn ttggactatt accgtntggg ngtgtcctga 540
tcggntnaag ggaggctgtn taaagcggng g 571

<210> 621
<211> 581
<212> DNA
<213> Homo sapiens

<220>
<221> misc_feature
<222> (1)...(581)
<223> n = A,T,C or G

<400> 621
acattcggcc tgagggccag gacagtgctt tctcctggac ggacctgctg ctgaagaata 60
attctgagct gcttaacaac ctgggcaact tcatcaacag agctgggatg tttgtgtcta 120
agttctttgg gggctatgtg cctgagatgg tgctcacccc tgatgatcag cgcctgctgg 180
cccatgtcac cctggagctc cagcactatc accagctact tgagaaggtt cggatccggg 240
atgccttgcg cagtatcctc accatatctc gacatggcaa ccaatatatt caggtgaatg 300
agccctggaa gcggattaaa ggcagtgagg ctgacaggca acgggcagga acagtgactg 360
gcttggcagt gaatatagct gccttgctct ctgcatgctt caccttacat gcccacggta 420
gtgcccaatc agcccactgc actccactca gctgagtatc ngntgacaac ttctgngacc 480
ttggccggac acctaaggca atcaccatgg cgcgtctang gaccactcga ccacttgcga 540
acatggcnat ggtctgngaa tgnccgtaat tccncanntc a 581

<210> 622
<211> 644
<212> DNA
<213> Homo sapiens

<220>
<221> misc_feature
<222> (1)...(644)
<223> n = A,T,C or G

<400> 622
actgtttacc agatctttgc agatgaggtg cttggttcag gccagtttgg catcgtttat 60
ggagaatttg caccatcctg ggattgtaaa cctggaatgt atgtttgaaa ccccagaacg 120

[?]g[?][?][?][?]g[?][?] g[?][?][?][?]gg[?][?][?] [?]g[?][?]g[?][?][?]gg [?]g[?][?][?][?]g[?][?]g g[?][?][?][?]g[?][?][?][?] tatccagtga 180
gaaaagtcgg cttcagaacg aattactaaa ttcatggtca cacagatact tgttgctttg 240
aggaatctgc attttaagaa tattgtgcac tgtgatttaa agccagaaaa tgtgctgctt 300
gcatcagcag agccatttcc tcaggtgaag ctgtgtgact ttggatttgc acgcatcatt 360
ggtgaaaagt cattcaggag atctgtggta ggaacttcag catacttacc cctgaagttc 420
ttcngagcca angtacaacc gntccctana tatgtggnca gtgggagtta tcatctatgt 480
gagcctnaat ggcacatttc ctttaatgng gatgaagatt taatgnccaa tccaaaaggc 540
tgganttatg naccctnggc cgaccccctt anggggaatt ccannnnntt ggggggccgt 600
tctaaggggn nccancttgg gcccaacntg ggggaancat ggcn 644

<210> 623
<211> 662
<212> DNA
<213> Homo sapiens

<220>
<221> misc_feature
<222> (1)...(662)
<223> n = A,T,C or G

<400> 623
acaaagagct actccataaa ttacatcttg ccaaggtggg agattgcatg ggagactccg 60
gtgacaaacc cttaaggcgc aataatagct atacttccta taccatggca atatgtggca 120
tgcctctgga ttcattccgt gccaaagaag gtgaacagaa gggcgaagaa atggagaagc 180
tgacatggcc taatgcggac tccaagaagc gaattcgaat ggacagttac accagttact 240
gcaatgctgt gtctgacctt cactcagcat ctgagataga catgagtgtc aaggcagaga 300
tgggtctagg tgacagaaaa ggaaagtaat gggctctcta gaagaatggt atgaccagga 360
taagcctgaa gtctctctcc tctttcagtt cctgcaganc cttacagcct gctttgggtc 420
attcgcccat ggtggcaatg acgtaagcca tgccatttgg gcctctgggt gctttatatt 480
tgggttatga cccnngagan gttcttcaaa agtggcaaca ccaatattgg nttctactct 540
antggngggg gttgggatct gnggttggtc tgtggggttt ggggaaaaaa aagttttccc 600
naccttgggg aaaggatttg ccnccgttac accctttaag ggtttngtat ttgactngna 660
tn 662

<210> 624
<211> 682
<212> DNA
<213> Homo sapiens

<220>
<221> misc_feature
<222> (1)...(682)
<223> n = A,T,C or G

<400> 624
acaccaagca tgggactttg aaataccaga cagactgtgc ccctaataat ggttacttta 60
tgatcccttt gtatgataag ggggatttca ttctgaagat tgagcctccc ctagggtgga 120
gttttgagcc gacgaccgtg gagctccatg tggatggagt cagtgacatc tgcacaaagg 180
gtggggacat caactttgtc ttcactgggt tctctgtgaa tggcaaggtc ctnagcaaag 240
ggcagcccct gggtcctgcg ggagttcang tgtctctgag aaacactggg acccgaagca 300
aagatccagt ncacagttac acagnctgcg gaaagtttgc attttttaaa gttctgcctg 360
gagaatatna aaatcctngt actcatccaa cctggggcgt tgaaagaagc aagcaccacn 420
gtnccntgtt accaactcca atgccaatgn cggncagtcc ccttcatagt tgctggnnta 480

ccaatngtgg tcttggcntn tgtcccnaaa ttgattnggn gaagcccctt gtaanggccc 540
taaagtttcn tnntcntttt cttctttant ttcctnnang aaggaanncc ttgggttnca 600
ntggntnacc tgngcctggg gttccaancc nnataccnan nntcttgggg tatttngcct 660
acccggtntc nnaaaaanat gg 682

<210> 625
<211> 502
<212> DNA
<213> Homo sapiens

<400> 625
acatttcctt gtagactctg ttaatttcct gcagctcctg gttggttctg gagcagatga 60
tctcaatgag agagtcctcg tcggttccca gccccttcat ggaagctttt agctcagagg 120
cgtcatactg agcaggtgtc ttcaataggc ccaaaatcac cgtctccagg tggccagata 180
aggctgactt cagtgctgat gcaagttcct ttttggtcct tctctggtag gcgaaggcaa 240
tatcctgtct ctgtgcattg ctgcggttgg tcaaaatgtt gacaatggtg acctcatcca 300
cacctttggt cttgatggct gtttcaatgt tcaaagcatc ccgctcagca tcaaagttag 360
tataggcttt gacagaccca tatgcacttg gggggtgtag aagtgatcac cctccaagct 420
gagcttgcac aggaatttcg tgaacagtag acattttgaa ggaactgggc ccgtgcgccg 480
aagagctgaa aaccgtccca cc 502

<210> 626
<211> 935
<212> DNA
<213> Homo sapiens

<220>
<221> misc_feature
<222> (1)...(935)
<223> n = A,T,C or G

<400> 626
acattcatca aagaggaatt tgtcacccaa ggccatgtgc ttttcagtgg aaaggaagga 60
gggaaacctc taaggccgca cggtgggccc acggagctag cacgtgggcg ggactgaagg 120
ctagatgctg ggattgaggt ggggaactag agatgactct aaggcaggaa catctgtacc 180
ttcgggccgc ganccacgcc taagggccga aattcagcac actggccggg cccgttacct 240
aagtgggaat cccgaagctt cgggtaccca aagcctttgg gccgtaaaat caattgggtc 300
caattaagcc ttggnttttc ccttgggggg tggnaaaaat ttgggtttaa ttcccggctt 360
tcaaccaaan ttttcccaac canccaaacc antttanccn aaaacccccn gggaaaaggc 420
cnttttaaaa aggttggtta aaaaaggncc ccttnggggg ggttnggccc cttaaaattg 480
gaaantttgg aaacccttna aaccnttnaa nccattttta aaattttggc ccgttttggc 540
cggcctttta aactttgggc ccccnggttt tttttcccaa agttcccggg ggaaaaaanc 600
cttgggtncc nttggnccca aaccnttggc cantttnaaa ttggnaaatt cnggggcncn 660
aaacggcccc ccgggggnna aaaaaaggcc cngggttttg gccggtaant tnggggcccc 720
cttttttttc ccggcttttc cctttgggtt tnaacttgga acttcnnttt tgggncnttg 780
gggnccnttt cggggttttn cggncaaaac cggggatntc aagntttanc ttcaaaaggg 840
ccgggaaata ncngggtttt cccccngaaa tccgggggnn aaacccccgg gaaaaaacct 900
ttttggacca aaaggcccnc naaanggccc ggaan 935

<210> 627
<211> 680
<212> DNA
<213> Homo sapiens

<220>
<221> misc_feature
<222> (1)...(680)
<223> n = A,T,C or G

<400> 627
ggtaccacaa ctcccaggat tttcctggat caaaccttgt atctcttctg caagtattgt 60
gtatattggt ctgagagacg tggaccctcc tgaacatttt attttaaaga actatgatat 120
ccagtatttt tccatgagag atattgatcg acttggtatc cagaaggtca tggaacgaac 180
atttgatctg ctgattggca agagacaaag accaatccat ttgagttttg atattgatgc 240
atttgaccct acactgactc cagccacagg aactcctgtt gtcgggggac taacctatcg 300
agaaggcatg tatattgctg aggaaataca caatacaggg ttgctatcag cactggatct 360
tggtgaaagt caatcctnag ttggccacct nagaggaaga ngccaagact acagctaacc 420
tggcagtaga tgngantgct tcaagctttt gggcagacca ganaaaggan ggcntattgg 480
ctattgaccc actttctant tccaagttan cccgaaggaa tccgaaaatc nagcccctgt 540
gganaaattt tggggaaact tggcncctgn ctggtttacc aacaggggct ttcccnaaat 600
ttttanggcc tttngggggn ttnanngaaa ccctaaaggg gtnnnctggg gccaaaaccg 660
gccttaanng ggnaaacttt 680

<210> 628
<211> 637
<212> DNA
<213> Homo sapiens

<220>
<221> misc_feature
<222> (1)...(637)
<223> n = A,T,C or G

<400> 628
acttgtaggg tggaggtgtc ggtcaaagac cttctttatg atatcaagaa atagacatgt 60
aacaaccatg aggattatgg caaaccaagc agaaccactt gacaggagct gaataaacac 120
aaaatacata ttctgggagc ccaaaaatgg ccagagaatc cctccataaa acaaggaaaa 180
tacaaaataa aatataatag atccccaggt aacgagatgg ttgatccaag tccaaaaatg 240
agtttccaga gccatcttta ctgtgactgt aataaccatg actgtgaaga ccaaagtgcc 300
aaatgtccag tttccaaaca tctggcattt ccaagcagag atgtatcttt ccctattagt 360
aaataggatc naaaaagaaa ataaaggcat gactgaaccc aggatggtcc aataaagaaa 420
tggtttaata cttaagaagg cggttttact aatggctcga taaaggtggc ttaatttggn 480
acacatgaag gnctacatgc ttgttccaaa agactntttn tcnnaattgg tngggaagta 540
aaccaatttt ggttaaagtc agggnccttg gccggacccn cttanggcga attccnnccn 600
ctgggggccg tcttagggga ncaacttggg cccaact 637

<210> 629
<211> 446
<212> DNA
<213> Homo sapiens

<220>
<221> misc_feature
<222> (1)...(446)
<223> n = A,T,C or G

<400> 629
acttctcatg tccatggtta atgaaaggca gccatttgtt ttgcgctgtg ctgttctcta 60
ttgtttccag tgtttcttgt ataaaaacca aaaaggacaa ggagaaatcg tgtcaacact 120
tttaccttct accattgatg caacaggtaa ttcagtttca gctggccagt tattatgtgg 180
aggtttgttt tctactgatt cactttcaaa ctggtgtgct gctgtggccc ttgcccatgc 240
gttgcaagaa aatgccaccc agaaagaaca gttgctcagg gttcaacttg ctacaagtat 300
tggcaaccct ncagtttctt tacttcaaca gtgcaccaat attctttcac agggtgataa 360
agatcgacag acggggaaac naaatacnaa ccaagaagtg gattattaat ggtgctttgg 420
accttggncg ngancacctt anggcc 446

<210> 630
<211> 635
<212> DNA
<213> Homo sapiens

<220>
<221> misc_feature
<222> (1)...(635)
<223> n = A,T,C or G

<400> 630
actagatatt gtgcctgcaa gtcataaaaa aaaaaaaaaa aaaagaaaaa aatgaaagaa 60
tgcctttccc cttcagacaa aagaattact tttttcattt ttcttaaaaa aagaggaaaa 120
gttataacac gaaacctaaa ttgacttgca aaggaatacc atgtaacaaa tggcttgaag 180
tagtctatca aaaaattggg gagattttta tttaatagtg agtcagcaag gcattttttg 240
ttgtttaaaa aaaatctcat ttccttacag aaacagtttt tagtttttaa tgaacttgta 300
aacnaaaaag ctcccatttc aaaataaaaa cnaaatccca gatcatatta atgnttacng 360
ggggtacctt tatctaagca acatacntac ctgttcagtt gtaaganggt aactaaattt 420
ctgngaccaa natgcntttt ttttaatacc cngaacnttn ttgaggtaat gcnnaatcct 480
aangggaaac tagnngnccc taagntttct taagcnttcc tttaaaagcn gggaattnta 540
gccccattaa ccggccnagn ttttntatgc ctaaancctg gaantttggn gntnccatta 600
atgggttgna acaaaanccc ccntttnaaa ngttn 635

<210> 631
<211> 694
<212> DNA
<213> Homo sapiens

<220>
<221> misc_feature
<222> (1)...(694)
<223> n = A,T,C or G

<400> 631
actcatctta tactgaaaga acgtggtggc tctaaatatg aagctgcaaa gaagtggaat 60
ttacctgccg ttactatagc ttggctgttg gagactgcta gaacgggaaa gagagcagac 120
gaaagccatt ttctgattga aaattcaact aaagaagaac gaagtttgga aacagaaata 180
acaaatggaa tcaatctaaa ttcagatact gcagagcatc ctggcacacg cctgcaaact 240
cacagaaaaa cccgtcgtta cacctttaga tatgaaccgc tttcagagta aagctttccg 300
tgctgnggct nacaacatgc cagacaggtc gcaacctccc agcagtagga caaccacttn 360
agaaggagcc ctcggtacac ctggatacac cattcaaaat tctgntccan ggccaactct 420
ttaagccttt ctttgatgtg aaagatgccc tttcagnctt tggnaacttc cagaacgttc 480
caancccacn gaaaaaggga aacccggtan ccttngccgg gaacccccct taaggggcga 540

aattccannn cacttggggg gnccgttnct aaaggggatc ccaaacttng ggncccaaan 600
nttgggggga aancangggg ccanaaanng gntcccctgg gggnaaaaat ggntatnccg 660
gttcnaaaan ttcccccccn aanatttngg ggcn 694

<210> 632
<211> 252
<212> DNA
<213> Homo sapiens

<400> 632
acggccatct tccagctgct tgcctgcaaa gatgagcctc tgctggtcgg ggggaatgcc 60
ttccttatcc tggatcttgg ccttcacatt ttcgatggtg tcactgggct ccacctcaag 120
ggtgatggtc ttgccggtaa gggttttcac gaagatctgc attttgacct gttagcggat 180
accaggatcc tgccaatcac caaccacgtc cacccacagg gacacaaaca agctcaccca 240
acaaagccaa cc 252

<210> 633
<211> 631
<212> DNA
<213> Homo sapiens

<220>
<221> misc_feature
<222> (1)...(631)
<223> n = A,T,C or G

<400> 633
ggtactgttg attcaacaac aaaccttaat gggtgatgag cttttgcata ccaatatgaa 60
tttgtcagca cttctgaaaa ctggccatca tttttcaaat tcacaatttg ctggatgtca 120
gggaacaata ggaagaagaa tgagcgtcaa ttttcatgtc ttcctttgct tcttcactgg 180
ccttccatag aagtagtcag aaaaaaacaa agcaccatca accacacttc acaaacaatt 240
catgttggcc taagctttgc tcaacattca tatgacagaa gatagaataa tgaaaaggaa 300
ctgctggcat cactttcccc ataatattac ataaaaatgg acagcacatt aaataaacat 360
tctgntatta atcattaaat atattaacac caaaaatcat gtataaaatt aggaaataaa 420
tgtcctgccc ggccggncgc tcaaggccaa atncagncac tggcgggcgg tctagtggat 480
ccnactcgga ccaacttggc gtaacatngn catactggtt cctgggggaa atggtaatcc 540
nttacaantc ncacactnac anccggaanc taaggggtaa acttgggtgc ctaagaggng 600
nctacntnca ttaatgngtg gcncnttgcc c 631

<210> 634
<211> 561
<212> DNA
<213> Homo sapiens

<220>
<221> misc_feature
<222> (1)...(561)
<223> n = A,T,C or G

<400> 634
   gtgaaattgg tgagtttggt ggtgatttcc cggtgcctgc aatgaactcc tggtgaaatg 60
   taggcgaggt tggaaagtag ctgggacaga caggagattt cctgaagttt ggagataaac 120
   acgtggtaga gactggggag taacacagtg aaagtgggga gcttggtggt gatccctggg 180
   atcctggaaa tgactggggc tgaaatgtgg gcgtggttgg agagtagctg ggacagacag 240
   gagggtttgt aagggctggt ggtgaagacg tgagagagac tggcgaggat ctcactgagg 300
   tctctgactt tctaggtgtt tctggggtgt gggagacata caacagctga aaactggaca 360
   tagttggaca gcactgggac agaaaggaga tcgtgatggg tgggggtgac tgtctattgt 420
   gccaacagan taccaaaagt atatcagacc gtttgctttc nttgaatggc ctctggctnt 480
   caaaagcgna tggtangaca ctcagagtat tctnctaagc nttgataata cactgnttat 540
   nctgcntgtg tctanctgcn c 561

<210> 635
<211> 630
<212> DNA
<213> Homo sapiens

<220>
<221> misc_feature
<222> (1)...(630)
<223> n = A,T,C or G

<400> 635
accgaggctg ctaaagctgc cagtcacaac ccagcatgtc aactggttcc tcatgctctg 60
tttggtgtgg aaattcacat gtgccctgac actgaggaag caattgctta aaatcacttt 120
ccaataacag ctgataaaat attttgcagg tttgtcatgc aaggtttatt tattaggtgg 180
ctattcaaag tttgtatagc aaccacttaa gcagaactaa attaatattc actgagcact 240
gtaacgatgg aagagggctt ttcctaaggg ttgggttggg agttgtgctt ctgtgaaatt 300
aacatctctc actcattgcc aagattctct gcttaaaaat attagtttc tgtgctggtg 360
ccaaaatagc aatttaagcn aatgtagtgc cagaatgaca catgaacctn ggactnaggg 420
aacagttncc tgctgnggag taccttgggc gngaacacgc ttanggcgaa ttccacacac 480
tgcgggcgta ctaanggatc caactnggna ccancttggc gaatcatggc atactggttc 540
ctggggaaaa tggtatccgt tacaatcncn cacntaccag ccggaaccta annggnaaac 600
tgggggccta atggngacta cntcattant 630

<210> 636
<211> 640
<212> DNA
<213> Homo sapiens

<220>
<221> misc_feature
<222> (1)...(640)
<223> n = A,T,C or G

<400> 636
actcctattg ccgccagtgg ggcctgtgga atgagtgtgc atggaggccc tcctgtgctg 60
ggggaatgag cccagagaac agcgaagtag cttgctccct gtgtccacct gtgggtgtag 120
ccaggtatgg ctctgcaccc ctctgccctc attactgggc cttagtgggc cagggctgcc 180
ctgagaagct gctccaggcc tgcagcagga gtggtgcaga cagaagtctc ctcaattttt 240
gtctcagaag tgaaaatctt ggaaaccctg caaacagaac agggtcatgt ttgcaggggt 300
gacggccctc atctatgagg aaaggttttg gatcttgaat gtggtctcag gatatcctta 360
tcaganctta nggtgggtgc tcanaataag gcangcattt gangaaaaat cttgggttct 420
ctttacagtg cccacttctt acacaccctt gaggcaagga atgcttgctt acaagtacct 480
tgggcgggaa cacgcttang gccaaattca acacacttgc cggccgtact aaagggatcc 540
ancttnggan ccaacttggn ggaaacatgg cnaaatggtt ccntggggaa atgnaatccg 600
ttcaattccc nnaantntca accggaacct taagggtaan 640

<210> 637
<211> 470
<212> DNA
<213> Homo sapiens

<220>
<221> misc_feature
<222> (1)...(470)
<223> n = A,T,C or G

<400> 637
acctggtgac cttgaatgtg attaggactg ggagctccgt gaggccagag acctatgttc 60
atttagccta cataaaagac actcaataaa tagctggtaa aataacaaat gaataaatac 120
atatcatcaa gggttggggt cagtagacag cagtgcccaa gctggcatcc gtcaggaagt 180
gtgggccttt gtgttttgat gctacacatg tctatggagg gccacttctt ctgtaagtct 240
gtggggcctc agcataccca ataggcagca agtttcagta tttcccagtt gtatgtcctc 300
atggtggggc tatgtctccc ccaccacgtc ccctctcatc aggctagact ttaacatcca 360
tcaatcatgt cttgagtctt gctccttcct cttggcttan tcatgtgact acngatcaan 420
atcntggcct aatggtttaa gtgtnccang taccttnggc cgggcccacg 470

<210> 638
<211> 391
<212> DNA
<213> Homo sapiens

<220>
<221> misc_feature
<222> (1)...(391)
<223> n = A,T,C or G

<400> 638
actggaacat caagttaaat acaaatactc agaactaacc actgtccaac aacagctaat 60
tagggagacg ctcatatcat ggctgcaagc tcagatgctg aatccccaac cagagaagac 120
ctttatacga aataaagccg cccaagtctt cgccttgctt tttgttacag agtatctcac 180
taagtggccc aagttttttt ttgacattct ctcagtagtg gacctaaatc caaggggagt 240
agatctctac ctgcgaatcc tcatggctat tgattcagag ttggtggatc gtgatgtggt 300
gcatacatca gaggaggctc gtaggaatac tctcataaaa gataccatga gggaacagtg 360
cattccaaat ctggtggaat catggnacct n 391

<210> 639
<211> 329
<212> DNA
<213> Homo sapiens

<400> 639
acatgctgac ccaccaggaa ctagcctccg atggggagat tgaaactaaa ctaattaagg 60
gtgatattta taaaacaagg ggtggtggac aatctgttca gtttactgat attgagactt 120
taaagcaaga atcaccaaat ggtagtcgaa aacgaagatc ttccacagta gcacctgccc 180
aaccagatgg tgcagagtct gaatggaccg atgtagaaac aaggtgttct gtggctgtgg 240
agatgagagc aggatcccag ctgggacctg gatatcagca tcacgcacaa cccaagcgca 300
aaaagccatg aactgacagt cccagtacc 329

<210> 640
<211> 764
<212> DNA
<213> Homo sapiens

<220>
<221> misc_feature
<222> (1)...(764)
<223> n = A,T,C or G

<400> 640
gcggccgagg tacttcacca tcactgactc catggacttg atcagccgcc gctggatgta 60
tccagtctca gcagtcttga cagccgtgtc aatgagcccc tcacgacccc ccatggcgtg 120
gaaaaagaac tcagtgggtg tgaggccggc taggtaggag ttctccacaa agccacggct 180
ctcaggcccg tagtcatcct tgatgaagtg aggcagagtc cggtgcttga agccaaatgg 240
aatccgcttg ccctcgacgt tctgctgtcc aacgacagcg atgacctggg agatgttaat 300
cttggaacct ttagctccgg acacgaccat agacttgaag ttgttgnatt cagacaggga 360
tttctgaagc agaaggaacc agtcttggct tgggcattcg gtaanaatgc gggtcacctg 420
aatcttcaaa acgtctggnc cgcaaaatgg ttcccctggg ggttggggct tccancntta 480
attggtgggg gngccctttn ttggaaggaa ccctctaatt aacggtcctt ggctttgggc 540
ctttccttaa ataaggggtn ctngnaaagg gccctngggn aaaggncntt aaaaaaatcc 600
nccaatnggg agnncccccc aanggcccca atnngtnttg gancctttaa aanncccggg 660
ggaaaaaacc ttttngncaa aaacccccnt ttggggnccc ttttaaanaa aacccttggg 720
aatgggggaa tttnttnncc cccaaaanag gtttnaaaac ccgg 764

<210> 641
<211> 540
<212> DNA
<213> Homo sapiens

<220>
<221> misc_feature
<222> (1)...(540)
<223> n = A,T,C or G

<400> 641
ggtacagtag ccatgaacta catacagtga cgcctctaga aacgtggtta gtgcaactga 60
ggaaggaatt tttaatctta tgtgatttta attggcttaa ctttaaacag ccgcatgtgg 120
ttactgtatt ggatagcaca gccctagagc ctgaagaaag caaaccaaag aacaccagct 180
gggtcccaaa cagaaggcag aaagggtaga accatccacc tcaactattc cagccccatc 240
agaaggcacc aggaacaggg caagagaaaa aggcaaaaac ccacccagcc catgaaaatt 300
cactcctcaa ccacccagca catcaaactg gaacaccaca ctatttcctg aaaaaatata 360
ttattatttt ctagaccaag gagatatata tatatagaac cagcacaatt ccacatcctc 420
atatatttgg actgtaaaaa acttgttcgc aantttttaa agacantnaa ggcagctagc 480
gggtaagtaa aaactgggag gtatgaaaca gagaaggaga gctttantta tnaaaaaaaa 540

<210> 642
<211> 608
<212> DNA
<213> Homo sapiens

<220>
<221> misc_feature

<223> n = A,T,C or G

<400> 642
ggtactagtg agaagaggga atatgcattg cagttcagca aagccggaat tctgtgttga 60
acagatgtct gtctccctag tgtgtgactc acaccttgtg gctgccttca gagcgccacc 120
tccagatcag atggggacac acaacccctg gatatgtttc attgtcagat tttgtgcttg 180
attttaagaa tggaattgtg ggtatctttc ctttttttta atgtatctta actgttgcct 240
gtcagtgttt acaaactagt gcgttgacgg caccgtgtcc aagtttttag aacccttgtt 300
agccagaccg aggtgtcctg gtcaccgttt caccatcatg ctttgatgtt cccctgtctt 360
tccctcttct gctctcaaga caaaggttaa ttaaggacna agatgaagtc actgtaaact 420
aatctggcat tggtttttac cttccttttc tttttcagtg cagaaaatta aaagttangt 480
attaaagcac ccgtaaaaaa aaataactnt antacaaana aaagcttgtn caagctttnt 540
ttttttntnn tttttttttt ttatttcccc ggncaaaaaa gttttttnan tcaaantcaa 600
gggttnan 608

<210> 643
<211> 669
<212> DNA
<213> Homo sapiens

<220>
<221> misc_feature
<222> (1)...(669)
<223> n = A,T,C or G

<400> 643
acagagtcat ttacatagat tatgttgtgc tttgtgttta ttctccacac tttcagtcca 60
tattctgtcc tgtatatgtt tcccattttt ccaggcattt tagttccagg ccagactctg 120
ccaatatcac cagttgcaac agctccaggt ctcctgtggg ttttcgtttg accatgcgta 180
gcaggctggc ctttaaatcc ccatcttttc atgacacctt gaaaaccttt accaatagtt 240
ttggctgtga catccacata ctgtcctgga cgaaagtgag cagcataaag aggagtgcct 300
ggtttaattg cagcattatc tgttatatta aagattttaa ctgtctgttt cggcggcaat 360
ccaagttccc ggtaaaattc caatatggat gtagctttac gaaaacgtga tcaggttttc 420
cttctacaga cagggttgcc atttttcatt acaggtttcc ttttgacgta tattttaaga 480
catgacagtc ttgnacacta gaattatggt ttaagtttcc tttggnatta agagatatat 540
aaccctttca aaacaatctg gtccttaaaa aatntcaata atggaatgaa ttttcttaaa 600
aaaggggaga atccaccnnt gcacctgctt tggnnttaan aaaatatggg taaacattta 660
cttccntnn 669

<210> 644
<211> 572
<212> DNA
<213> Homo sapiens

<220>
<221> misc_feature
<222> (1)...(572)
<223> n = A,T,C or G

<400> 644
acaagctttt tttttttttt tttttttttt tttttttttc atattcacta nttgngacat 60
ntaactgctc aangatttct tgaatacgtt tttcaatttg ancctngtca ccttttcctt 120

ttaanagcat ggcatcgtct ttggncacaa ngacctntcc aacttttcct aagtcatgag 180
gctgaacgtc ttcaanattc agggtcaatc cctnttctcc aaacacctac aaaaagagtt 240
aaacgtaaac ctgttgtagg ttacagtttn tgccattata ccaagttnat taatacncca 300
tgcaananaa tcatcaaaat actttatttc tttgaaatga gagattttaa natcactgtt 360
agtccanaac aagacttgag tatagtctnt ttcactgnat ttccaaattc tcaattttca 420
caactggggt aattattacc agcnttactt gnnaaaaaaa cnttcnaagg tcacacttac 480
tgggaanagc caggacaana ncataggccn ttgactntta agtcctanaa tcccttggna 540
catacncttt tacctttnaa actgnngctt gg 572

<210> 645
<211> 690
<212> DNA
<213> Homo sapiens

<220>
<221> misc_feature
<222> (1)...(690)
<223> n = A,T,C or G

<400> 645
ttgtgagacc ctcttcattc tggtgttgtc cttgaaccaa cagcatcccc tggaacgccc 60
caagcaagac caaggcagat actatgaggc aggcagcaca gggcccaaat caagaattgg 120
tgcagtcgaa tcagggctgt gggagaggcc ctatgtattc cggattccca gggcttgctc 180
taattcttgt cgtctctgct gcaccttgga gtagaagtat cggcacacag cctcctgagc 240
ccagggctgg aagtagaact cagctcggcg ctcctcctct gggttaccca ccacatcagt 300
cattgtcttg aggtccctgc actgggactg aagccagtca ttgatgaaac cctgagggtc 360
tctggccaaa cttaacatga actcccgctg agtcttcagc tggttgatgg gtttctattg 420
gctcatggat cttggtggct aaagtaccaa tcttctggtg gcccggcant gggacagcag 480
aaaaagaaat catcttgggg ctttcaaggn ggcattcact ttnaccatca atggcataac 540
aagctggcct ttttctnaac attcgggtca acactgatga cattgaataa nganaatagg 600
ttntggnggc attaaccang natggaaccn cttagggact ttgaaactta tcnntgagac 660
ttaananttn tgnggacctt gccgaacncg 690

<210> 646
<211> 770
<212> DNA
<213> Homo sapiens

<220>
<221> misc_feature
<222> (1)...(770)
<223> n = A,T,C or G

<400> 646
cgaggtacat tccgctcacg gatctcagct tccagatggt ggatgaactg gaggcagtgc 60
ccaacatccc cctggtgccc gatgaggagc tggacgcttt gaagatcaag atctcccaga 120
tcaagagtga catccagaga gagaagaggg cgaacaaggg cagcaaggct acggagaggc 180
tgaagaagaa gctgtcggag caggagtcac tgctgctgct tatgtctccc agcatggcct 240
tcagggtgca cagccgcaac ggcaagagtt acacgttcct gatctcctct gactatgagc 300
gtgcagagtg gaggggagaa catccgggag cagcaagaaa gaagtgtttc anaaagcttt 360
ctcccttgac atcccgtgga gcttgcanaa tgcctgaccc aacttcgtgt tggtggaaac 420
ttccagaact tgtncacaag catttcccgc ttgacccatt caatttaagg gaagaatgaa 480
tgaagtcttc cnggggcttt ttattggggt tttctggaat ggtcattcan tccacttnaa 540

gcccncttgg gaatttnaag cccgaggttt caaaatcttg tanccttggc ccngggccgg 600
gccggttcca aaggggcgaa atttccagcn cacttgggng ggccggtact tannggggat 660
cccaacttcg gnncccaacc ttggnggnaa ancatngggc ctanctnggt tccncgggng 720
gaaaatggta ttnccgttcc aatttccccc canntttnna accggagctt 770

<210> 647
<211> 454
<212> DNA
<213> Homo sapiens

<220>
<221> misc_feature
<222> (1)...(454)
<223> n = A,T,C or G

<400> 647
acttggaatc ctccaggaag ggcttcagga cctggttggg gaagaccttc atcaggatct 60
tgtgtttccg cagctggtgt cgcataagaa gcttgtcctc tgcactcaga gccacattct 120
ggcagacggc tatcattcgg ttgtcctgga aaactgctgc tatctcccgg cggagaagcc 180
tgatgaggcc tatctcctcc tgtggggggc tgggaggaga tggcacgtat cttccaagta 240
tgttctgaaa attaaacagg gtaacctatt tttgatgtta tttcaaactg ctatattcat 300
ctatgtctag ttaaaaacaa tttttggttt attcacttac ataatgttct tatagtgata 360
ttttttccac ttattccana agtgttaggt gattattcta cacttcttgn gcccattcta 420
tggagaataa agatggtcct nggccgcgac cacc 454

<210> 648
<211> 532
<212> DNA
<213> Homo sapiens

<220>
<221> misc_feature
<222> (1)...(532)
<223> n = A,T,C or G

<400> 648
ggtacatgtg ggagaaaaac ttaagtgtga tgagtgtggt aaggaattca gtcagggcgc 60
tcatctacag acccatcaga aagtccacgt gatagagaaa ccatacaaat gtaagcaatg 120
tgggaaaggt ttcagtcgta gatcagcact taatgttcat tgcaaggtcc acacggcaga 180
gaaaccttat aattgtgagg agtgtgggag ggccttcagt caggcctctc atcttcagga 240
ccatcagaga ctccacactg gggagaagcc attcaaatgt gatgcatgtg gtaagagctt 300
cagtcggaat tcacatcttc aatcccatca aagagttcat acaggagaga aaccatacaa 360
atgtgaggag tgtggtaagg gcttcatttg tagctcaaat ctttacattc atcagagagt 420
ccacacagga gaaaaaccct ataaatgtga ggaatgtggt aaaggcttta gtcggncttc 480
aagtcttcag gcccatcagg gagttcacac tggagagaag tcatacatat gt 532

<210> 649
<211> 493
<212> DNA
<213> Homo sapiens

<400> 649
ggtacaaaat tgttggaatt tagctaatag aaaaacatag taaatattta caaaaacgtt 60

gataacatta ctcaagtcac acacatataa caatgtagac aggtcttaac aaagtttaca 120
aattgaaatt atggagattt cccaaaatga atctaatagc tcattgctga gcatggttat 180
caatataaca tttaagatct tggatcaaat gttgtccccg agtcttctgc aatccagtcc 240
tcttagaaat tggtttctct ctttgggaga ttcagactca gaggcagcca gaggggacag 300
gtcaagagct gaaataatca cataactact ctaattttct tcattctatt gactgtgtca 360
agttatagac acagccaaag tgtttttctt ctgcctctga tgatttgaga agatgaagaa 420
catgagcaat ttctcattgc ttaaagaaaa acttggcaca taagaggctg agtgtagtag 480
agtatctgtc ctg 493

<210> 650
<211> 693
<212> DNA
<213> Homo sapiens

<220>
<221> misc_feature
<222> (1)...(693)
<223> n = A,T,C or G

<400> 650
gagactttgg atccttcctg aggacgtgga gaaaacttgc tgctgagaag gacattttga 60
aggttttgtt ggctgaaaaa gctgtttctg gaatcacccc tagatctttc ttgaagactt 120
gaattagatt acagcgatgg ggacacagaa ggtcacccca gctctgatat ttgccatcac 180
agttgctaca atcggctctt tccaatttgg ctacaacact ggggtcatca atgctcctga 240
gaagatcata aaggaattta tcaataaaac tttgacggac aagggaaatg ccccaccctc 300
tgaggtgctg ctcacgtctc tctggncctt ggctgtggcc atattttccc nccggggtat 360
gaacggnttc tttttccgcg gactctttcg caacccnttt ggcaggcccc attcaatgct 420
gaatggcaac ctggtngctg cactggtggc tgctttattg ggactgggtn aaggaactta 480
ntccggttgn aatgcttgat nccgggnccc ttnggtaatt gggcnttttn tgnggactnt 540
tggncaaggt ttgggnccca tgtanccttg ggccggnaac acccttangg gcnaanttcc 600
gcncacttgg ccgggccgta ctanagggaa tcccaacttg gnacccaacn ttggggnaaa 660
catnggcana actggttccc gggggaaaa tgg 693

<210> 651
<211> 678
<212> DNA
<213> Homo sapiens

<220>
<221> misc_feature
<222> (1)...(678)
<223> n = A,T,C or G

<400> 651
ggtacgaagt ttgttaccac agtagagata atttagtaga aaaatgcttt gaggcttcag 60
tatttgtaag attttgcatt agccagatgc taggttgttg aaggcatttc agtgttgata 120
ataacctgag cagacttctt tacaaatggg atctgtttct atatgtgtat atgcccactt 180
accattcaga gagactggtc tttctctttg tcttccttca cattgctgtg tcagttctac 240
acctagtctt ttcagcactt agcaaattca aattttgatt tttttgtcag cttagttcac 300
tttaaggcat attggcatgg tgtgtgaaag tgatgttttg ccccagtatt gaggactttt 360
agatccnaat aatgactcat taaatataat tatgttttaa gtatacctga atttctggta 420
gcttaaaatg ttaattctca ggaatgattt tctcacactt ttggggtggc taataataaa 480
agcactggtt tattctcaaa actccttttt tcaaaattag ggagagagcn naagtggaca 540

ttttatgtga acccctttgn aaanatgggg gntngantgc ngagaaacca atggagtttt 600
ngntgcnaaa aggttttttc ccgnaangta aaattggaat aantggcnat tgaggaccct 660
tgnnctgccc ggcggcnn 678

<210> 652
<211> 676
<212> DNA
<213> Homo sapiens

<220>
<221> misc_feature
<222> (1)...(676)
<223> n = A,T,C or G

<400> 652
ggtacaagct tttttttttt tttttttttt tttttttgtg tttaaatgca ttttattttt 60
agacaaccta catgacatgt ttttcttaaa aacaatgcct ccactccaaa taaatcacag 120
tcaaaataaa tgaagagctc aagatgacat cagtcccatt tgtcttaagt cctggtgttg 180
tgtggatgac aagcagaagc cagttatgat gacaggtgat agatccaaaa taattgccac 240
atttgttaac atttttccat ttctaaacca tccttaaaga aaatcatata tggggtcaca 300
ccatcctcac ggtagtccaa tagagcaacc atgccatctg gattcatgtt ttcaccaata 360
aagaactggt aagttttgga aattagcaag ggatgtgctt gatttgttct gcaacccctg 420
gcataaaaag gtttactctt tctnggctct ggtctttaag gttncctttg aatggattca 480
tgtaaccttt gatgtaccct ggcccggccg gccaagggac ntgtaaaagn gccccaatcc 540
acccganaan aaataagggg tttnttccgc gnttanganc tcctttggac cttttttaan 600
cttgcctgnn ggaaattaat ctggccnttt acctnggana atagaaaata ntttttcccg 660
naaccttgaa cttcnn 676

<210> 653
<211> 468
<212> DNA
<213> Homo sapiens

<400> 653
tcgagcggcc ccgggcaggt actccagcat tggttatagt catgggaaag gaaggtgtcc 60
acggaggcac acttaacaag aaagcatatg aactcgcttt atacctgagg aggtctgatg 120
tgtaagcagc ctctccccat ctacctagca actgtcttca tcaacaaccc taattatggt 180
cacaatgcta ccaaactgta gatggtagct aatttttctt tacctatttt ctaatgtcat 240
gattcctgtt tgcccaatgg atcatttgta tgttaaccac tgtatgtaac caacccttat 300
ctggcaacat aattgcagca caataatgat ttgcatgata ccttgaaatt ggggggaggg 360
ggcatgccaa gttgggcatc actttgtctt agcaattaat gggatattga ttactaaaat 420
aagttaatat taaacaaggt gccggttgta ccttggccgg gaacacgc 468

<210> 654
<211> 612
<212> DNA
<213> Homo sapiens

<220>
<221> misc_feature
<222> (1)...(612)
<223> n = A,T,C or G

<400> 654
   actgaagagc ccatggatac tacttctgca gttatccatt cagaaaattt tcagacattg 60
   cttgatgctg gtttaccaca gaaagttgct gaaaaactag atgaaattta cgttgcaggg 120
   ctagttgcac atagtgattt agatgaaaga gctattgaag ctttaaaaga attcaatgaa 180
   gacggtgcat tggcagttct tcaacagttt aaagacagtg atctctctca tgttcagaac 240
   aaaagtgcct ttttatgtgg agtcatgaag acttacaggc agagagaaaa acaagggacc 300
   aaagtagcag attctagtaa aggaccagat gaggcaaaaa ttaaggcact cttggaaaga 360
   acaggctaca cacttgatgt gaccactgga cagaggaagt atggaggacc accttcagat 420
   tccgtttatt caggtcagca gccttctgtt ggcacctgag atatttgtgg ggaaagatcc 480
   caagagatct atttgaggat gaacctggtn cantaatttg agaaaacctn gacctatatg 540
   gggatcntcg tctaatgatg ggatcccttc actgggcttn aataaanggt ntgccgttgg 600
   caantttttg nc 612

<210> 655
<211> 608
<212> DNA
<213> Homo sapiens

<220>
<221> misc_feature
<222> (1)...(608)
<223> n = A,T,C or G

<400> 655
ggtactttgt cctggaggaa gggcacgact acacttcttc caaggggcag aacatggtgt 60
gcggcggcat gggctgcaac aatgattccc tggtgcagca gatatttaac gcggcgcagc 120
tggacaacta tacccgaata ggcttcgccc cctcgtcctg gatcgacgat tatttcgact 180
gggtgaagcc acagtcgtct tgctgtcgag tggacaatat cactgaccag ttctgcaatg 240
cttcagtggt tgaccctgcc tgcgttcgct gcaggcctct gactccggaa ggcaaacaga 300
ggcctcaggg gggagacttc atgagattcc tgcccatgtt cctttcggat aaccctaacc 360
ccaagtgtgg caaaaggggg acatgctgcc tatagtctgc agttaacatc ctccttggcc 420
atggcaccag ggtcngaacc acgtactaca atgaanccac aggtggcaaa atgttcctcg 480
tgccttctgt ggattaaact gggaccatgg cttgtcctag ncctttgcng ncttaaccaa 540
cacttgattg canttgggag taaatggcaa gcctccagag cncactgtnt tgctgaggac 600
tccgcgcc 608

<210> 656
<211> 659
<212> DNA
<213> Homo sapiens

<220>
<221> misc_feature
<222> (1)...(659)
<223> n = A,T,C or G

<400> 656
accaaactga ccaatgggct gcaagaggtt tagattattg ctacccacaa aattctgagc 60
caaattgata atggtcatca ttagtgacat ctcgccatga tgataagaag acatttcagc 120
cactgatcca gctaattggg caacctttac ttctcgcttg tcattccgtt tgaagcaagt 180
aaacaaaacc tttctctgac ctggtttcaa accatccacc atagaaggga tagatctctc 240
gttatcagaa tttgagaaca agataagttc cttgttgatg aagtcattat atgtcagata 300
tgtggtagtt tgtccataca agtaatcctc aggaagccca agtaactttc gttgtcttct 360

atcctccatg aaattagtta accattcctt tcgatcatct atctgttttt tgctaaaggc 420
caggctgata gcagcatcat cttcaggacc agaatatttg aactggatac gatgtctttt 480
catatctgca aagtatcttt acttcctttg atgtgctggt gcccaaacct ttgnaatatt 540
ggcttttcat ttttatgatt gggagtagaa ctcttncact cttcaaattc aggaangctt 600
naaaatgcct ttcttgcttg gtttagancc tttccatggg agtgataaat cctccgaaa 659

<210> 657
<211> 676
<212> DNA
<213> Homo sapiens

<220>
<221> misc_feature
<222> (1)...(676)
<223> n = A,T,C or G

<400> 657
ggtacagaat tatataattc taacgcttaa atcatgtgaa agggttgctg ctgtcagcct 60
tgcccactgt gacttcaaac ccaaggagga actcttgatc aagatgccca accctgtgat 120
cagaacctcc aaatactgcc atgagaaact agagggcagg tcttcataaa agccctttga 180
acccccttcc tgccctgtgt taggagatag ggatattggc ccctcactgc agctgccagc 240
acttggtcag tcactctcag ccatagcact ttgttcactg tcctgtgtca gaacactgag 300
ctccaccctt ttctgagaag ttattacagc cnagaaagtg tgggctgaaa aatgggtggg 360
ttcatggttt tggattaatg gatctttttg gatgggaaag actatatttt gggacctcat 420
cttttcccag gatgacccag aagctanaac ctgctaaaag gattcttgga acntgaaggg 480
tattaatacn aacccnntca tggnggnatc ctnggaacct gccgggaaga aggccnttgg 540
cccgtttaat gcnccggtgc tnaacaagtc tgnttcttgn ntttcacttc ancttggggc 600
cctggaatca nctggcnctg gtgnncagtt taactatgnc ttgntggaac ccctaaggcc 660
ttangcctta ccaaag 676

<210> 658
<211> 646
<212> DNA
<213> Homo sapiens

<220>
<221> misc_feature
<222> (1)...(646)
<223> n = A,T,C or G

<400> 658
ggtacaatgg aacaaacaac aagaacacac ctgtctatgt gtcctcacca acctgggaga 60
atcacaatgc tgtgttttcc gctgctggtt ttaaagacat tcggtcctat cgctactggg 120
atgcagagaa gagaggattg gacctccagg gcttcctgaa tgatctggag aatgctcctg 180
agttctccat tgttgtcctc cacgcctgtg cacacaaccc aactggaatt gacccaactc 240
cggagcagtg gaagcagatt gcttctgtca tgaagcaccg gtttctgttc cccttctttg 300
actcagccta tcagggcttc gcatctggaa acctggagag agatgcctgg gccattcgct 360
attttgtgtc tgaagcttcg agttcttctg tgcccatcct tctccaagaa cttcggctct 420
acaatgagag agtcnggaat ctgactgntg gttggaaaag aacctgagaa catcctgcaa 480
gtcctttcca gatgagaaaa tcgtgccgat tacttggtcc aatcccccgg ccaaggagcc 540
cnaattgtgg ccagcaccnt tttaacctga cttttgagga tggcnggtat ntgaaacatg 600
gtnaccgatc tggcctgana ctgactnngn ncnntnaanc ctaaan 646

<210> 659
<211> 673
<212> DNA
<213> Homo sapiens

<220>
<221> misc_feature
<222> (1)...(673)
<223> n = A,T,C or G

<400> 659
actgtgtcca acagctgaag gaatttgagg ggaagacttt agtgtcagtc accaaagaag 60
gcctggaact tccagaggat gaagaagaga aaaagaagca ggaagagaaa aaaacaaagt 120
ttgagaacct ctgcaaaatc atgaaagaca tattggagaa aaaagttgaa aaggtggttg 180
tgtcaaaccg attggtgaca tctccatgct gtattgtcac aagcacatat ggctggacag 240
caaacatgga gcgaatcatg aaagctcaag ccctaagaga caactcaaca atgggttaca 300
tggcagcaaa gaaacacctg gagataaacc ctgaccattc cattattgag accttaaggc 360
aaaaggcaga ggctgataag aacgacaagt ctgtgaagga tctggtcatc ttgctttatg 420
aaactgcgct cctgncttct ggcttcagtc tggaagatcc cagacacatg ctaacaggat 480
ctcagggatg atcaaacttg gtctgggtat tgatgaagat gaccctactg ntgatgatcc 540
catgcttgct gnaactgaag aaatgcccnc ccttgaagga gataccaccc ctnacgcctg 600
ggaanaagtn actaactttg gcttanggat nnttaccngt cagaccttgg ncggaccccc 660
ttagggcnaa tcc 673

<210> 660
<211> 580
<212> DNA
<213> Homo sapiens

<220>
<221> misc_feature
<222> (1)...(580)
<223> n = A,T,C or G

<400> 660
acaaaacgcc acattctcac ttgtattggg agctgaaaaa tgggatcaca tggacgcagg 60
acggggaaca acacacactg gggcttttcg ggagacagag cgttaagaaa aacagctgat 120
gcatgctggg cttaatacct aggtgacggg ttgacaggtg cagcaaacca ccatggcact 180
cgtttacctt agtaacaaat atacacatcc tgcccatata ccccagaact tagaaacaga 240
acgaaacaaa agaaaacgag aaagcaatag caaatcgcta gcgggaaaac aaattttcaa 300
actcagaaaa tgacagacca atttttgctt caaatcatgg ttcttaaccc aggtgccata 360
aggtcaggat aaagaatttg attacatatt gtaaataaga catgcagcaa atgaccagaa 420
aaattattcc caacatatgt gtgtcttcga attcaatggt gacgctatct accgggacat 480
aacattagat tccaaagggc cgagtnncac aagactgncc tnccatacta ataacnatga 540
aagccctacg ttgggtttac ctgcttttnt ancagctggg 580

<210> 661
<211> 710
<212> DNA
<213> Homo sapiens

<220>
<221> misc_feature

<222> (1)...(710)
<223> n = A,T,C or G

<400> 661
ggtacatata aatgaatctg gtgttgggga aaccttcatc tgaaacccac agatgtctct 60
ggggcagatc cccactgtcc taccagttgc cctagcccag actctgagct gctcaccgga 120
gtcattggga aggaaaagtg gagaaatggc aagtctagag tctcagaaac tcccctgggg 180
gtttcacctg ggccctggag gaattcagct cagcttcttc ctaggtccaa gccccccaca 240
ccttttcccc aaccacagag aacaagagtt tgttctgttc tgggggacag agaaggcgct 300
tcccaacttc atactggcag gagggtgagg aggttcactg agctccccag atctcccact 360
gcggggagac agaaacctgg actctgcccc acgctgtggc cctggagggt cccggttgnc 420
agttcttggt gctctgtgtt cccagaggca agccggaggt ttgaaagaaa ggaacctggg 480
atgaaggggt gctgggtata aaccagaaaa gggatngggt tcctgnttcc aangggaccc 540
ctttggcctt tcttctggcc tttcctaagg cccaggnctg gggnttggnc ccttgggccg 600
ngaaccacgc ttaagggccg aaattccagc acacttggcc ggccggtacc tagtgggatc 660
ccaactttgg gtccaaactt tggcgtaaat catngggcct aacttngttn 710

<210> 662
<211> 411
<212> DNA
<213> Homo sapiens

<400> 662
ccaaaatctg gaatgttcat agtgtcctca atgtccttca ttccctggta gacaaatcca 60
acatcaaccg acagttggag gtatacacaa gcggaggtga ccctgagagt gtggctgggg 120
agtatgggcg gcactccctc tacaaaatgc ttggttactt cagcctggtc gggcttctcc 180
gcctgcactc cctgttagga gattactacc aggccatcaa ggtgctggag aacatcgaac 240
tgaacaagaa gagtatgtat tcccgtgtgc cagagtgcca ggtcaccaca tactattatg 300
ttgggtttgc atatttgatg atgcgtcgtt accaggatgc catccgggtc ttcgccaaca 360
tcctcctcta catccagagg accaagagca tgttccagag gaccacgtac c 411

<210> 663
<211> 633
<212> DNA
<213> Homo sapiens

<220>
<221> misc_feature
<222> (1)...(633)
<223> n = A,T,C or G

<400> 663
ggtacttggt tttaatgctc gtcagcgaaa agcctttctt aatgcaatta tgcgatatgg 60
tatgccacct caggatgctt ttactaccca gtggcttgta agagacctgc gaggcaaatc 120
agagaaagag ttcaaggcat atgtctctct tttcatgcgg catttatgtg agccgggggc 180
agatggggct gagacctttg ctgatggtgt cccccgagaa ggcctgtctc gccagcatgt 240
ccttactaga attggtgtta tgtctttgat tcgcaagaag gttcaggagt ttgaacatgt 300
taatgggcgc tggagcatgc ctgaactggc tgaggtggag gaaaacaaga agatgtccca 360
gccagggtca ccctccccaa aactcctaca ccctccactc caggggacac gcagcccaac 420
actcctgcac ctgtccacct gctgaagatg gataaaatng aaggaaaata cctcaaagaa 480
ganagagctn gaaggagaaa aggaggttaa actacagccc tgaactgcca tgatgactgc 540
ccggcggccg tcaaaggcna atcaaccatn gcgccgtnta atggntcaac tnggaccant 600
tgcnaacatg cnaacttgtc ctgggaaatg nnc 633

<210> 664
<211> 598
<212> DNA
<213> Homo sapiens

<220>
<221> misc_feature
<222> (1)...(598)
<223> n = A,T,C or G

<400> 664
gcgtggtgcg gcccgaggta ctgggtccaa atgctggaga agttacacaa ggctttgcag 60
ctgcgctcaa atgtggactg accaaaaagc agctggacag cacaattgga atccaccctg 120
tctgtgcaga ggtattcaca acattgtctg tgaccaagcg ctctggggca agcatcctcc 180
aggctggctg ctgaggttaa gccccagtgt ggatgctgtt gccaagactg caaaccactg 240
gctcgtttcc gtgcccaaat ccaaggcgaa gttttctaga gggttcttgg gctcttggca 300
cctgcgtgtc ctgtgcttac caccgccaag gcccccttgg atctctttgg ataggagttg 360
tgaatagaag cagcacatca cacttgggtc actgcagaac ttgaanttga cattggcagg 420
catcnaggat natccatgag tcaccagtct nagccatgtg taggcgtatg acactgcaaa 480
tatttacata ccttcctggg attctatctc tggaagttnn ggtgattttc tttttcatgg 540
naanattaan taaactncat tatttgcaac anntgttaat cntcagggtg tctgaagg 598

<210> 665
<211> 658
<212> DNA
<213> Homo sapiens

<220>
<221> misc_feature
<222> (1)...(658)
<223> n = A,T,C or G

<400> 665
acccaaaagc agtgcaggac ctctgcagct ggagaatctg gagcctggct tgtgggaaga 60
gcagcatcat tgtggcagcc gatgagagca ccatcagctg gggcccatca ccgacctttg 120
gggaactggg ctacagggat cacaagccca agtcttccac tgcagcccag gaggtgaaga 180
ctctgcatgg cattttctca gagccggtcg ccatgggcta ctcacactcc ttggtgatag 240
caagagatga aagtgaaact gagaaagaaa agatcaagaa actgccagaa tacagccccc 300
aaaccctctg atgctccaga gactcctccg actccacacc tctcatggca gctgcatttc 360
catgtgcact gggaccggaa agtcaaacna ggaatttaaa aaagccaaag tggacccaaa 420
ggtgcctttt tatttaaact tcctganggt ncggtttacc agtgatccaa cggtnactac 480
ctttttttct ggttgctttc caaagaccct ttttttctct taatggccaa ataaaaaacc 540
tgnttcgaan tggcntaaca nttctaccaa gaggccnaaa ccttttacca ttaagggggt 600
tttttcttct tctntctgaa acccttncca aaaactcntt tccgtttaat nnntnngg 658

<210> 666
<211> 349
<212> DNA
<213> Homo sapiens

<400> 666
gcggcggcgg gggaagcagc gtgagcagcc ggaggatcgc ggagtcccaa tgaaacgggc 60

agccatggcc ctcccacagcc cgcagtatat ttctggagat tttagccctg atgaattcaa 120
tcaattcttt gtgactcctc gatcttcagt tgagcttcct ccatacagtg gaacagttct 180
gtgtggcaca caggctgtgg ataaactacc tgatggacaa gaatatcaga gaattgagtt 240
tggtgtcgat gaagtcattg aacccagtga cactttgccg agaaccccca gctacagtat 300
ttcaagcaca cttgaaccct cagcccctga atttattctc ggttgtacc 349

<210> 667
<211> 768
<212> DNA
<213> Homo sapiens

<220>
<221> misc_feature
<222> (1)...(768)
<223> n = A,T,C or G

<400> 667
ggtggcgagg tggaggccca ggactctgac cctgcccctg ccttcagcaa ggcccccggc 60
agcgccggcc actacgaact gccgtgggtt gaaaaatata ggccagtaaa gctgaatgaa 120
attgtcggga atgaagacac cgtgagcagg ctagaggtct ttgcaaggga aggaaatgtg 180
cccaacatca tcattgcggg ccctccagga accggcaaga ccacaagcat tctgtgcttg 240
gcccgggccc tgctgggccc agcactcaaa gatgccatgt tggaactcaa tgcttcaaat 300
gacaggggca ttgacgttgt gaggaataaa attaaaatgt ttgctcaaca aaaagtcact 360
cttccaaagg cccgacataa gatcatcatt cttggatgaa acaagaacag cattgacccg 420
acggagccca agcaagccnt tgaaggaaga acccatggga aaatctactt ttaaaaacca 480
cttcgntttc gnccctttgc nttggaaatg gcttttngga ttaagaaaca attngaagcc 540
ccaatttaan tnccccgctt ggggccaatc ccnttccngg taaccttggn cccngggccn 600
ggcccggttt cnaaaanggg ccnaaaattt ccaagcacca ctttgggnng ggncccgntn 660
ncttaanggg gatcccaaac tttgggnacc ccannccttg nggcgnaaaa ncaatgggcc 720
ataaannggg gttcccctgg ggngnaaaaa tgggnattnc ccccncnc 768

<210> 668
<211> 659
<212> DNA
<213> Homo sapiens

<220>
<221> misc_feature
<222> (1)...(659)
<223> n = A,T,C or G

<400> 668
ggtacagtat cctctccaga catttgcaat tggcatggaa gacagccccg atttactggc 60
tgctagaaag gtggcagatc atattggaag tgaacattat gaagtccttt ttaactctga 120
ggaaggcatt caggctctgg atgaagtcat attttccttg gaaacttatg acattacaac 180
agttcgtgct tcagtaggta tgtatttaat ttccaagtat attcggaaga acacagatag 240
cgtggtgatc ttctctggag aaggatcaga tgaacttacg cagggttaca tatattttca 300
caaggctcct tctcctgaaa aagccgagga ggagaagtga gaggcttctg agggaactct 360
atttggttga tgttctccgc gcagatcgaa ctactgctgc ccatggtctt gaactgagaa 420
gtccatttct agaacatcga ntttcttnct aatacttggc tttgccccag aaatgagaaa 480
ttccaagaat gggatngaaa aacattttct gaganaaacc ntttgaggat tccaatctga 540
taccaaagag aatctttggc gaccaaanaa accttnatga tnggaaacct tngntaaaaa 600
tnctggttaa aattnnngga atccttnact tngggtnata atccngangg caaannccc 659

<210> 669
<211> 409
<212> DNA
<213> Homo sapiens

<220>
<221> misc_feature
<222> (1)...(409)
<223> n = A,T,C or G

<400> 669
acgtgccgcg gaaatgctcc gctagcaatc gcatcatcgg tgccaaggac cacgcatcca 60
tccagatgaa cgtggccgag gttgacaagg tcacaggcag gtttaatggc cagtttaaaa 120
cttatgctat ctgcggggcc attcgtagga tgggtgagtc agatgattcc attctccgat 180
tggccaaggc cgatggcatc gtctcaaagt aaggttgggg gctcacattt gggcagagtg 240
agtggactag gactgctcca gaggcgtggt cttaacgttg tccttttccc ctggttctag 300
gaacttttga ctggagagaa tcacagatgt ggaatatttg tcataaataa ataatgaana 360
aaaaannnnn nnnnnnaaaa aaaaaaactt gtcctcggcc ggaccacgc 409

<210> 670
<211> 741
<212> DNA
<213> Homo sapiens

<220>
<221> misc_feature
<222> (1)...(741)
<223> n = A,T,C or G

<400> 670
accgctgtaa gactgccaag aagtcagagg aggagattga ctttcttcgt tccaatccca 60
aaatctggaa tgttcatagt gtcctcaatg tccttcattc cctggtagac aaatccaaca 120
tcaaccgaca gttggaggta tacacaagcg gaggtgaccc tgagagtgtg gctggggagt 180
atgggcggca ctccctctac aaaatgcttg gttacttcag cctggtcggg cttctccgcc 240
tgcactccct gttaggagat tactaccagg ccatcaaggt gctggagaac atcgaactga 300
acaagaagag tatgtattcc cgtgtgccag aatgccaggt caccacatac tattatgttg 360
gggtttgcat atttgatgat gcgtcgttac caggatgcca tcgggtcttc gccaacatcc 420
tnctctacat ccagaggacc nagaagcatg ttncagaagg acccacgtac ctttggccgn 480
gaccacgcct aagggccaaa attncaacac actggccngg ncggttacct aagtggaatc 540
cnaaccttcg gnanccaaag ctttggccgt naatccatng ggccataagc ttggttccct 600
gggggggaaa attggtaatn ccggttcacn aatttcccca ccaacnttcc naaacccggn 660
aagcctttaa agnggtnaaa accntggggg tggccnnaaa ggggggggac ctnaacttnc 720
atttaaatng gggttggccn c 741

<210> 671
<211> 699
<212> DNA
<213> Homo sapiens

<220>
<221> misc_feature
<222> (1)...(699)

<223> n = A,T,C or G

<400> 671
ggtacagcag gaattacaac tactacctca ccgagaactc ctccaccact gactgttcag 60
gatcccttat gtcctgcagt ttgtccctta gaagaattat ctccagatag tattgatgca 120
catacgtttg attttgaaac tattccccat ccaaacatag aacagactat tcaccaagtt 180
tctttagact tggattcatt agcagaaagt cctgaatcag attttatgtc tgctgtgaat 240
gagtttgtaa tagaagaaaa tttgtcgtct cctaatccta taagtgatcc acaaagccca 300
gaaatgatgg gtggaatcac tttattcatc agttatcaat gcgatagaca gtagacgaat 360
gcagggatca aatgtatgtg gtaaggaggg attttggaga tcatacttct ctgaatgtcc 420
agttggaaag atgtagagtt gttgcccaag actctcactt cagtatacca accattaagg 480
aagaccttgg cacttttaga accattgtac ctggcccggc cggccggttc naaanggccg 540
aanttccagc acacttggcn ggccgttact tagtgggatt ccgagcttcg ggacccaagc 600
nttggcggta atcatngggc catagctggt tcccngngtg naaattggta ttccggttac 660
caattcccca ccacnnttcc ancccggnaa ccntaaagt 699

<210> 672
<211> 377
<212> DNA
<213> Homo sapiens

<220>
<221> misc_feature
<222> (1)...(377)
<223> n = A,T,C or G

<400> 672
actgaagctg aaatgcagga agtggtggca aaggtttatt ccagagaagc caggaagccg 60
gtcatcaccc agcctctgag agcagttact ggggtcaccc aacctgactt cctctgccac 120
tccccgctgt gtgactttgg gcaagccaag tgccctctct gaacctcagt ttcctcatct 180
gcaaaatggg aacaatgacg tgcctacctc ttagacatgt tgtgaggaga ctatgatata 240
acatgtgtat gtaaatcttc atgtgattgt catgtaaggc ttaacacagt gggtggtgag 300
ttctgactaa aggttacctg ttgtcgtgat ctgaaaaaaa aaannnnnaa aaaaaaaaac 360
ctnggccgnn accacgc 377

<210> 673
<211> 650
<212> DNA
<213> Homo sapiens

<220>
<221> misc_feature
<222> (1)...(650)
<223> n = A,T,C or G

<400> 673
cgaggtactt gattggacca gatggtgagt ttctagatta ttttggccag aacaagagga 60
agggagaaat agctgcttca attgccacac acatgaggcc atacagaaaa aagagctagc 120
caaagcagtg ttgctggatg cagtattctc ttgctaagag gaaggaaact gtctcgcata 180
ggagcctata taaatataaa catatatacg tgcactctac agaatggcct tcataccatg 240
agaacatttc tgttttggat ggggatgtta cccttgcgtt caaccaaaat tgattcttgg 300
aactgtaaag attacaaccc aaagtctccc aggaagctgt ggggagacca gaggatcaag 360
ctgaagtgaa accagtgaaa aacccacctg tggaaggcat ggcggggcca ggcacaccag 420

[?]g[?]a[?][?][?][?][?]g [?][?][?]g[?]g[?]a[?][?]a gg[?][?][?][?][?][?]a[?]a a[?][?][?][?]g[?][?]g[?] [?][?][?][?][?]a[?][?]g[?] [?][?]ggg[?][?][?][?][?] 480
gctaaatagc tgtgggactg aattcacaga aaagaatnta tttccatagg ctcttgctgg 540
ctcttcttga gtctttntct ttgagtcttg gnggctatac cgncgaatag ggcttggcat 600
tanagtgatg cttgaacttt agttcctata angattnctn tcgattgcta 650

<210> 674
<211> 705
<212> DNA
<213> Homo sapiens

<220>
<221> misc_feature
<222> (1)...(705)
<223> n = A,T,C or G

<400> 674
ggtacaagct tttttttttt tttttttttt ggtgaaaaga tatatatata tatatattca 60
gaattaggca gctggactca gtttagatga tcccaatttt gttggcaaca tccaaagcat 120
cgtaatcagg agccagtcga acatatgcct tcttctctcc atcaggccga atcagggtgt 180
tgaccttggc cacatcaatg tcatacagct tcttcacagc ctgtttaatc tggtgcttgt 240
tggctttaac atccacaatg aacacaagtg tgttgttgtc ttctatcttc ttcatggcag 300
actcagtggt cagcggaaac ttgatgatag catagtggtc aagcttgttt ctcctgggag 360
cgctcttccg aggatatttg ggctgtctcc ggagtcgcag tgtcttcggc cgcccgaagg 420
nggggtgacg tgccggatct tcttcttttt ggggctgtgg accacctttc aacactgcct 480
ttttgggccn ttnaaagccc ttngctttgg ctttagcttt taggaagggg ccaggaacct 540
tnccttnttc gcttttcgga acctgccccg gccgggccgt tcnaaaaggg cnnaatttcc 600
aacncacttg gcngggccgn tactaagggg atnccaanct ttggnancca anctttggcg 660
naaancttgg ggcnataact ggnttcccgg ngngnaaaaa tgntt 705

<210> 675
<211> 622
<212> DNA
<213> Homo sapiens

<220>
<221> misc_feature
<222> (1)...(622)
<223> n = A,T,C or G

<400> 675
ggtaccctaa ttttccttgc acccatgcct gtccaatcag atgactctgg gaaacgccaa 60
acaggctgaa tcaatgtctt tgtgtggttt ttttcttcca gattgttttt ttctcaccta 120
taaaaggatc tatctttaaa aataaactgt attaaatctg taacatcaaa ggcagaaggt 180
ttgtgtgtgt gtgtgtgtgt gtgtgtgtat ctgtgtgttt aaatcaaggg gagattgcat 240
ttataaatca tactggcctt atgaacatcc tctgcaataa atatactttt tagccttaac 300
tataaattat atattttagt gtttaaaaac cttccggtgt gaaacatcta agataaccct 360
taaaaaccac ctgttctcta ggtaaacctc tgaggtccct actttcaaac accagttggc 420
accaaaggat tcctaaactt caacttcttt aaagaaaaga aaggaactta tcatctggca 480
tgtgagaatg caaccttttc tcttnctgca cgcagctnca acacccactc atgcacacag 540
tggccacctt gctaaagtct gttgaacagc ctgcggcgcg tcaagngatc accactgcgc 600
gtctatgacc actcgacact gc 622

<210> 676

<211> 620
<212> DNA
<213> Homo sapiens

<220>
<221> misc_feature
<222> (1)...(620)
<223> n = A,T,C or G

<400> 676
cgaggtgcac aggcaccact aataatcaga cctgattctg gaaaccctct tgacactgtg 60
ttaaaggttt tggagatttt aggtaagaag tttcctgtta ctgagaactc aaagggttac 120
aagttgctgc caccttatct tagagttatt caaggggatg gagtagatat taatacctta 180
caagagattg tagaaggcat gaaacaaaaa atgtggagta ttgaaaatat tgccttcggt 240
tctggtggag gtttgctaca gaagttggca agagatctct tgaattgttc cttcaagtgt 300
agctatgttg taactaatgg ccttgggatt aacgtcttca aggacccagt tgctgatccc 360
aacaaaaggt ccaaaaaggg ccgattatct ttacatagga cgccagcagg gaatttggta 420
cactggaaga aggaaaagga gaccttgagg aatatggtca ggatctcttc atctgcttca 480
gaatggcang tgacaaaagc tatctttgta aaaaaaaaaa aaaaacctgc cgccgncgtc 540
aangccaatt caccctgcgg cgtctatgac cactgnccac tgcnatntgc tactgtnctg 600
ggaatgatcg tncatcncan 620

<210> 677
<211> 691
<212> DNA
<213> Homo sapiens

<220>
<221> misc_feature
<222> (1)...(691)
<223> n = A,T,C or G

<400> 677
cgaggtactg ggtccaaatg ctggagaagt tacacaaggc tttgcagctg cgctcaaatg 60
tggactgacc aaaaagcagc tggacagcac aattggaatc caccctgtct gtgcagaggt 120
attcacaaca ttgtctgtga ccaagcgctc tggggcaagc atcctccagg ctggctgctg 180
aggttaagcc ccagtgtgga tgctgttgcc aagactgcaa accactggct cgtttccgtg 240
cccaaatcca aggcgaagtt ttctagaggg ttcttgggct cttggcacct gcgtgtcctg 300
tgcttaccac ccgccaagcc cccttggatc tcttggatag gagttggtga atagaagcag 360
gcagcatcac actggggtca ctgacagact tgaactgaca ttttggcaag gcatcgaaag 420
gatgtattcc atgaagtcac cagtcttaaa cccatgtggt aagccggtga tggaaccact 480
gtnaaatcaa ttttaacatg aacctttcnt gnggatttct taatctcggt gcaagttttt 540
aagggtgaat tttctttttt ctncatgggg gtaatgattt tnagatgaaa acctttccag 600
ttgattttg tccaaancaa tnatggttaa atatccctcc agggnntttt ncttgaagga 660
aattggtnct ttgaggtttt agcttnccgg a 691

<210> 678
<211> 667
<212> DNA
<213> Homo sapiens

<220>
<221> misc_feature

<222> (1)...(667)
<223> n = A,T,C or G

<400> 678
cgaggtactt gattggacca gatggtgagt ttctagatta ttttggccag aacaagagga 60
angggagaaa tagctgcttc aattgccaca cacatgaggc catacagaaa aaagagctag 120
ccaaagcagt gttgctggat gcagtattct cttgctaaga ggaaggaaac tgtctcgcat 180
aggagcctat ataaatataa acatatatac gtgcactcta cagaatggcc ttcataccat 240
gagaacattt ctgttttgga tggggatgtt acccttgcgt tcaaccaaaa ttgattcttg 300
gaactgtaaa gattacaacc caaagtctcc caggaagctg tggggagacc agaggatcaa 360
gctgaagtga aaccagtgaa gagcccacct gtggaaagga catggcgggg cgaggcacaa 420
ncagtgcatt cctgcctgcg aacagncctn cacactttgc cgctttcatc gcttgggcct 480
tggtaaatac tgtggactga atttccagaa aagaatntat ttcataggnt cttnttgctt 540
tcttgagtct tgtctttgag tcttggggnt aanacagtcn aatanggctt tgcnttcaag 600
tgancttgaa cctaagttcc tntaangana tcctttcnat gctatgaaag gaattttgtt 660
nggggaa 667

<210> 679
<211> 302
<212> DNA
<213> Homo sapiens

<220>
<221> misc_feature
<222> (1)...(302)
<223> n = A,T,C or G

<400> 679
cgaggtactg atggggaagt gccggcgctt cttggatgaa ctagatgcgg ttcagatgga 60
ctgagcttgg atgcttctga ggcaagctga agctttgggt tctgactgac ccaccctaca 120
ggactgctga acagagagcc cagtgtgact agggatcctg agttttctgg gacaattcca 180
gctttaatca atacattttg ttaaatgtgc cataaaatga gactttttac gcctttataa 240
ggccttagat gtaaataaac tcacccaaac aaaaaaaaaa aaaanaaaaa aaaaaagctt 300
gt 302

<210> 680
<211> 649
<212> DNA
<213> Homo sapiens

<220>
<221> misc_feature
<222> (1)...(649)
<223> n = A,T,C or G

<400> 680
ggtacgtgct caggaaatta aaaacaaaaa tcaaggaatt gaacaacaca tgtgaacccg 60
ttgtaacaca accgaaacca aaaattgaat cacccaaact ggaaagaact ccaaatggcc 120
caaatattga taaaaaggaa gaagatttag aagacaaaaa caattttggt gctgaacctc 180
cacatcagaa tggtgaatgt taccctaatg agaaaaattc tgttaatatg gacttggact 240
agataacctt aaattggcct attccttcaa ttaataaaat atttttgcca tagtatgtga 300
ctctacataa catactgaaa ctatttatat tttctttttt aaggatattt agaaattttg 360
tgtattatat ggaaaaagaa aaaaagctta agtctgtagt ctttatgatc ctaaaaggga 420

aaattgcctt ggtaactttc agattcctgt ggaattgtga attcatacta agctttctgg 480
gcagtctcac catttgcata ctgaggatga aactgacttt ggcntttgga gaaaaaaact 540
gtcctgccgg cggccgtcaa aggcaattca ccctgcggcg tntanggacc actnggacca 600
ctgggaantg gctactgtcc tggaatgtnc cgtccatccc aatcaccgg 649

<210> 681
<211> 722
<212> DNA
<213> Homo sapiens

<220>
<221> misc_feature
<222> (1)...(722)
<223> n = A,T,C or G

<400> 681
cgaggtacca ccagagggaa agctggggcg gagggatttg ttcgtgttga cccgagatta 60
tgtgctgaag tctgcagagc tggcaaaagc tggagggtgc aaacatttca acttgctatc 120
ctctaaagga gctgataaat caagcaattt tttatatcta caagttaagg gagaagtaga 180
agccaaggtt gaagaattaa aatttgatcg ttactctgta tttaggcctg gagttctgtt 240
atgtgatagg caagaatctc gcccaggtga atggctggtt agaaagttct ttggctcctt 300
accagactct tgggccagtg ggcattctgt gcctgtggtg acccgtgggt tagagcaatg 360
ctgaacaatg tgggtgagac caagagacaa gcagatggaa ctgctggaga acaaggccat 420
ccatgacctg gggaaaagcg catggctctn tnaagccatg acccccattg gagaaatggg 480
ttttattggc aacccttaca cccattaccc aaatcngnaa tttcanggtc taaaaaaaag 540
tcancctggt ttaactttgg ngggttacta atccttaggc ttcanttcca atcaggaaat 600
gatggggcct ntggattaag gggttcaaaa cccgggtttc cctttggann cttcggggnc 660
ntttggnaaa ataaaaattt gnnnccctnt tttaacttga atnaaaattt nggggggggc 720
cn 722

<210> 682
<211> 530
<212> DNA
<213> Homo sapiens

<400> 682
ggtacttgcc tttagtttat caggggatgt gtaaggagct tcaggagcat aaatcctgaa 60
aatatcagca aggcagcagg ctaccagtaa gcgaacatcc ttatcaggat gcttgaggaa 120
aaaatctgaa gcaagatgta aagctaggtt taaataaagc tccttttctt cttcagagtc 180
ctggtccata tccataaaag ttttcacaac catctataca aaaataaaaa atcaaataat 240
gaaatgctcc atgtaaaact acagtcatgt gaaataaagg tcatgttaat tgctaaggtt 300
aacttcaaat gaatatactt tcatttttct gcagaaagtc tctatttgag agaacacaat 360
tctcctaaaa ctacaaagta aacttctatt taaaagactt actaaaatat tttttcattt 420
acccaaaata tctgctaacc agatttttaa agattaaatt gcccttatgt agtagtcatt 480
attggaagaa ttccaataga atatttgtgg aaacttctgg tctcacttgt 530

<210> 683
<211> 745
<212> DNA
<213> Homo sapiens

<220>
<221> misc_feature

<222> (1)...(745)
<223> n = A,T,C or G

<400> 683
ggtacctgtc tttccttatt ccctcatcct tagtggatca tttgtatctc ctgccttatg 60
agaacctttt gacagaagat gagacaacca tatctgatga tgtggatatc gctcgggatg 120
tcatatgtct tataaaatgc ctccggctga ttgaagagtc agtaactgtg gatatgtcag 180
ttataatgga aatgagttgt tataacctac agtctccgga aaaggctgca gagcagattc 240
tggaagatat gatcactatt gatgtagaaa atgtgatgga ggatatttgt agtaaactgc 300
aagagattag gaacccaatc catgcaattg gactacttat acgggaaatg gattatgaaa 360
cagaagtgga aatggaaaag ggattcaatc cagctcacct ttgaatattc gaatgaatct 420
tacccagctc tatggtagta acacagcagg gtatattgtg tgccagangg gtgcattaaa 480
atccgccagt acctgcccng gccggccgnt cgaaanggcc naatttccac acactgggcg 540
ggccgttact anggggaatc ccaagctttg gganccaagc nttggncgta atcatgggcc 600
ataanctngg tnccctgggn ngaaaatngg taatccggtt aacaattncc ccnccaactt 660
tcccnacccg gnaaccctta aaggggtaaa aaccctgggg gggncccaaa gggagggggc 720
cttaaccttc ccctttaaat tggcn 745

<210> 684
<211> 628
<212> DNA
<213> Homo sapiens

<220>
<221> misc_feature
<222> (1)...(628)
<223> n = A,T,C or G

<400> 684
ggttggagac ccgagaaccg gaggctggag agcaaaatcc gggagcactt ggagaagaag 60
ggaccccagg tcagagactg gagccattac ttcaagatca tcgaggacct gagggctcag 120
accttcgcaa atactgtgga caatgcccgc atcgttctgc agattgacaa tgcccgtctt 180
gctgctgatg actttagagt caagtatgag acagagctgg ccatgcgcca gtctgtggag 240
aacgacatcc atgggctccg caaggtcatt gatgacacca atatcacacg actgcagctg 300
gagacagaga tcgaggctct caaggaggag ctgctcttca tgaagaagaa ccacgaagag 360
gaagtaaaag gcctacaagc ccagattgcc agctctgggt tgaccgtgga ggtagatgcc 420
cccaaatctn aggacctcgc aagatcatgg cagacattcc ggcccaatat gacaactggc 480
tcggaagaac cnagangact ngacaagtcc ttgccggccg ncgtcnaagg caattcacca 540
ctgnggcgtc tatgatccac tgnncactgg gantgctact gtctggaatg ttcgtnatcc 600
cactcacgac tagnactggc tagggata 628

<210> 685
<211> 758
<212> DNA
<213> Homo sapiens

<220>
<221> misc_feature
<222> (1)...(758)
<223> n = A,T,C or G

<400> 685
   gcgtgggtcg cggcccgagg tacggagcaa atgttttatt taataagtta taagatacaa 60
   tttacagtcg gcgtttgatt ccagtttngg cttccgtggt ccaacttaac acaccccgtg 120
   ggcccttcac aataagcttc cggctggtcc actttctgta ngggtgggct tttaccccaa 180
   cactngccca gatctacacc tgccacaaga ntggccactt tctnaggact aagcagcaaa 240
   acctaaaggn ctgcctgcca gaccacacta cacatttggg ctcaggcaac gtccctgaca 300
   ctttaacctc attccaaagc cagctcaggt ctgcaggaag gcaggcaaaa ttccctacac 360
   ctcatttctg gatttctgca ccacacagnt ctnactggtt ctgcccatgg tgaaaagacc 420
   ccaataagct gntggccttn tttccccaac cattcccaac tttnagggcc aagancccca 480
   agaggttcaa tctggcctgc tggacctggc cggcnggccg ntnnaaangg ccaaantcca 540
   ncacaattgg gnggncggta ctaaagggga acccaacttn gggnccaaac tttggggnaa 600
   acatggggnn naanngggnn ccnggggngn aaaatngnna ncccntttcc aaattncccn 660
   ccaanntttn naacccggaa accttaaang ggnaaaancc cgggggggcc caaagggggg 720
   ggccnannnn cccnttaaan ggggnngggc ccccccnn 758

<210> 686
<211> 697
<212> DNA
<213> Homo sapiens

<220>
<221> misc_feature
<222> (1)...(697)
<223> n = A,T,C or G

<400> 686
ggtacagatt gggcggaatg tggagaaggt tggccacagt ccagagccag gagcccatgg 60
aacaacttgg aaggtgactc aggtgaggct gtcaatgagg gaatcccgca tgctggtggc 120
aatggtgcta ggctgggctt cattcagctt gaagacactc tccaccactg acagctctgt 180
gctggttgtg tccaggccac agaaggcaca ccagtcattc accaccatcc cagcagcaat 240
cacctcactg cctcggttca cagtccccgc cacaaggggg acttgaagaa gagaggacag 300
ctcatcctgg tcttcaattg aagtcttggg atgcaccagc cctccctgat tgctgaagac 360
acagtagctt cctactagca cctggtcggc cactgctgtc tgaagacttc caccttgagc 420
acatctgcca gaatttcttc tgnctcctgt ccaagtctgg gtggaccaag gncacgtagt 480
catttcaagt ggtgacattg cccaaggctt aaaaccgttc ttcaaccgnc taatctgcac 540
ttggtctggg aaggttgttg ccaatgtgtg caacttctgg ggccgnggta ttgtngggac 600
cttgcccggc cggccgttca aagggcaatt ccanccaatg ggggccgtac tangggaacc 660
ancttgggnc caacttgggg naanatgggc nnaacgn 697

<210> 687
<211> 668
<212> DNA
<213> Homo sapiens

<220>
<221> misc_feature
<222> (1)...(668)
<223> n = A,T,C or G

<400> 687
acataataac ctcatcaact aacttttaaa ttaactgaat ggctattatg tatttattac 60
tcaataccag tccattacct aatataagag cactaagagt atttaatcat tacctatttt 120
aatttatttt ataggtgaaa aacactgatg tcaagttagg ttgaggaact tatattcaag 180
gtcctccagc taactgtcga cacaacaatg actagaacta attgtcaggt ctcctgataa 240
ctagtccact gttctttcta ttctaccata aggttgttag gatgaagaat actgcagttt 300

tactgcataa atattctgaa gtcagactta ctctaaggca ttcttccttc agaatacagg 360
ctaaagcaga atttacaag ctactgcttc tttttttttt tttttttta ataaacacag 420
aacatttgn tcaaaccaaa tctaactcag aagtgnaaat aatgnaagcc aatcactatt 480
aaaaggcnga atttcctaaa gggaaaanta ccatttaacc aacctttcta aagtaaacat 540
cctttccang ggactgggga tttagnctta cacttgaagg cttcctggga cctgggcggn 600
acccttangg cnattcancc atgggggcgg tctanggnnc cacttgggcc annttggnna 660
attnggcn 668

<210> 688
<211> 375
<212> DNA
<213> Homo sapiens

<400> 688
acatcaattc agtgagaaaa ggtgtgtagg gagccataag tctgcaaaga gaaagcagaa 60
cactaaacaa ggtttctagg gccatgacac aatcctccat cccattttca ccctttaatc 120
ttctgcggtt cattctaaca taccaattgg tcagaatatc tacaaacttg accaggcgag 180
gcaccacagt ataaagccta taagctgcca tttcagtctc aaagaagcca atgagagact 240
gcatgaagga caggatccac cggtctgtaa tgttggggct ttctctaacc gtgttctcat 300
tgtagagaaa ttctatttct tcctccttct ggagcctcag aacgttctgg attaagaagc 360
gataggcatt gtacc 375

<210> 689
<211> 582
<212> DNA
<213> Homo sapiens

<220>
<221> misc_feature
<222> (1)...(582)
<223> n = A,T,C or G

<400> 689
ggtaccaaaa gttaaatgac ttacctgggc tgtttagaaa ctctctacct agaaagattt 60
ccattaccgt cagatgttag gagaggatct aacataggaa aggtcaccag ttgtcacaga 120
aaaagccaaa gaacttaggt ctagtgcccc tttgccactg acaaactaat aacaccctct 180
agacatcctc aagtccttct ccttgctcag gaattttctt ctaccaggtc ttttctacca 240
acttctctgt ataactacat cttactcatc tttcaaagcc cgactcagtt gccccttcca 300
tctagaaaac tttccagacc aaactatccc agcacatggt tatgatctct caaacctctg 360
tgtttcccca tccctgttgc ccgttaaatt ctgccacaag ctcagaccga ctctctattt 420
ggcttatttg tgtctaatcc attgagttct cctccaaagc agagatcatg cttcactcat 480
ttctgcatct ncaggacctt atgaatgaat gaatgtgtga attataagga ttactaaagc 540
cncagggcct gactcaaagc caggacccta gtaggngctt gg 582

<210> 690
<211> 812
<212> DNA
<213> Homo sapiens

<220>
<221> misc_feature
<222> (1)...(812)
<223> n = A,T,C or G

<400> 690
actaaagcgg atgggaatgt cgtttggcct ggagtcaggc aaatgctctc tggaggatct 60
gaaacttgcg aaatccctgg tgccaaaggc tttagaaggt tatatcacag atatctccac 120
aggaccttct tggttaaatc agggactact tctgaactct acccaatcag tttcaaattt 180
agacctgacc actggtgcca ccttacccca gtcaagtgta aaccaagggt tatgcttgga 240
tgcagaagtg gccttaacaa ctgggcagtt cctggcccca aacagtcacc agtccagcag 300
tgcggnctnt nactgnttcg agtcccgaag cgaagacccc ctggtcgttc aatgatgaan 360
atgaaggaan atgatgaagg agggattccc tncttcccaa gaattaaaga ccangaagaa 420
agccctacct tttcaaatat ggtgaatgcc tcaatggtgt ggtttggtaa ntgggtgaag 480
cctcnttggg ttttttgaaa atggaattgg ctttcaagtc cttttggccc tttgggtttg 540
gcacttgggg ngggttcaan nggaaaaanc tttngnggaa aacnccccat ttaggcccaa 600
attcnccatt gaaanggctt tgaaaaatgn atttggnaaa ttgnaaaagg ttnaaccctt 660
aangggggna attgnaaaan tnttgggccc aaccngaacc ccnttnnaan gggnttttnc 720
cccaannaaa agcctggcnt tttttgaggg gaaaaaanng gggggataaa nccccttaaa 780
aaaatttgcc cnnntnnaag ngccaccntt tt 812

<210> 691
<211> 691
<212> DNA
<213> Homo sapiens

<220>
<221> misc_feature
<222> (1)...(691)
<223> n = A,T,C or G

<400> 691
   acctactata atacagtagc taacatgtat tgagcacaga ttttttttgg taaaactgtg 60
   aggagctagg atatatactt ggtgaaacaa accagtatgt tccctgttct cttgagcttc 120
   gactcttctg tgctctattg ctgcgcactg ctttttctac aggcattaca tcaactccta 180
   aggggtcctc tgggattagt taagcagcta ttaaatcacc cgaagacact aatttacaga 240
   agacacaact ccttccccag tgatcactgt cataaccagt gctctaccgt atcccatcac 300
   tgaggactga tgttgactga catcatttta tcgtaataaa catgtggctc tattagctgc 360
   aagctttacc aagtaattgg catgacatct gagcacagaa attaaggnaa aaaaccaaag 420
   caaaacaaat acatgggctg aaantaactt gatgccaagc ccaaggcact gatttctggg 480
   natttgaact tanggcaaat cagagctaca cagacgccta cagaaggttc aggaagangc 540
   agaagccttc aatttgaaag aaatttattg gcaccaaagt aagggccgga tnaaccttta 600
   ggcnttttta nggagggcct tttaaaaagg ntccttggcc ggaacncntt anggngaatt 660
   ccanccntgg gggccgtatt aagggacccg n 691

<210> 692
<211> 271
<212> DNA
<213> Homo sapiens

<400> 692
cgaggtactg ctgctaccac tggaagcgct gcgcctcttt cgggttttgt cccggccgcg 60
atccttctca ctcgactcct tggtggcccc tttatctttt gagcgatcct tggacttctc 120
atctgagcgg tctttgcgtt tggtaggtga aggagcccta gtgctggact ttttattatg 180
agaaacgatc cctaatcgat tgcaatttac gccgaagagc agcatcttcc ctccgccgcc 240
acctcctcct gctttcctca gccgccgagg c 271

<210> 693
<211> 730
<212> DNA
<213> Homo sapiens

<220>
<221> misc_feature
<222> (1)...(730)
<223> n = A,T,C or G

<400> 693
cgaggttttt ttttgccgca catgaaacat tattttaatt ggtttaaagt ccctttataa 60
agagtgctac atggtttaga taaaggaaac atataactat tgagttacag gggattttat 120
taattataaa atgcaatcaa tttaaattac gtaggtttaa gactagtccc ttggataagc 180
cccaagcgaa tttgtcttca gattattaaa attagtgctg taaatcaggg tgggcaattc 240
acagcctttc tgaactgact gaactagagc ttgcagtgaa gtgttctgct gagactgagc 300
accttacaga tatttttctc cagaagatgg tgctgggtaa taaaatcatc acaattaggg 360
gaatggttaa gtggtctcta ctgnggcaaa tgccaactgn tggaattcac tttattgtag 420
aaaaacccaa actgagactc ttaagttttg gttaacaatg nggttctggg atgaaaccaa 480
ctactggggc actgnccagg taggaaacca ttctttcact ggggtttcag cataaatggg 540
aactggatgt tnaaaggcng ggaattaacc ctttttaggc caaaagaaaa agcttaantg 600
gggntttacc aangggntcc ctggggctta aattcaannn tgggncctac anngnccnna 660
anccctggnt aaacccggat taacccttta acctggaacc ccaaccttta aanggggggt 720
tttaaaaggg 730

<210> 694
<211> 700
<212> DNA
<213> Homo sapiens

<220>
<221> misc_feature
<222> (1)...(700)
<223> n = A,T,C or G

<400> 694
cgaggttaca aaccacaaag acattggaac actataccta ttattcggcg catgagctgg 60
agtcctaggc acagctctaa gcctccttat tcgagccgag ctgggccagc caggcaacct 120
tctaggtaac gaccacatct acaacgttat cgtcacagcc catgcatttg taataatctt 180
cttcatagta atacccatca taatcggagg ctttggcaac tgactagttc ccctaataat 240
cggtgccccc gatatggcgt ttccccgcat aaacaacata agcttctgac tcttacctcc 300
ctctctccta ctcctgctcg catctgctat agtggaggcc ggagcaggaa caggttgaac 360
agtctaccct cccttacagg gaactactcc accctggagc cttcgtagac acaccttgga 420
gttttttcga aatatgggtt gggtttttgg gctctttggg tgaattaaaa taaaatttaa 480
atgccttcac gctgngatag gtgccacatg aactaccgag nttcngaaaa agaagggaga 540
actgacactt cttanngntt gcagactntt aangggccct taggactant ngggcttttg 600
ggggtaaaag gtncccttna agaancccng nacctggccn ggggggcgtt naaangggga 660
attcnanccn ctgggggccg tactaagggg acccactnng 700

<210> 695
<211> 690
<212> DNA
<213> Homo sapiens

<220>
<221> misc_feature
<222> (1)...(690)
<223> n = A,T,C or G

<400> 695
ggtacagatg gcactgacaa tcccctttct ggtggggatc agtatcagaa catcacagtg 60
cacagacatc tgatgctacc agattttgat ttgctggagg acattgaaag caaaatccaa 120
ccaggttctc aacaggctga cttcctggat gcactaatcg tgagcatgga tgtgattcaa 180
catgaaacaa taggaaagaa gtttgagaag aggcatattg aaatattcac tgacctcagc 240
agccgattca gcaaaagtca gctggatatt ataattcata gcttgaagaa atgtgacatc 300
tccctgcaat tcttcttgcc tttctcactt ggcaaggaag atggaagtgg ggacagagga 360
gatggcccct ttcgcttagg tggccatggg ccttcctttc cactaaaagg aattacncga 420
acagcaaaaa gaaggtcttg agatagtgaa aatggtgatg atatctttag aaggtgaaga 480
tgggttggat gaaatttatt cattcatgag agtctgagaa aactgngccg tcttcaagaa 540
aattgagagg cttccattca cttggncctg ccgactgacc atggctccaa ttggctataa 600
ggttgcagcc tttaatcgat ttncngggna gggttaaaag cttggnccgt tgggttccaa 660
acctaaaaaa aannnnnnnn aaaaaanant 690

<210> 696
<211> 688
<212> DNA
<213> Homo sapiens

<220>
<221> misc_feature
<222> (1)...(688)
<223> n = A,T,C or G

<400> 696
ggtacagaaa tgaggcgtcg cagaatagag gtcaatgtgg agctgaggga aagctaagaa 60
ggatgaccag atgctgaaga ggagaaatgt aagctcattt cctgatgatg ctacttctcc 120
gctgcaggaa aaccgcaaca accagggcac tgtaaattgg tctgttgatg acattgtcaa 180
aggcataaat agcagcaatg tggaaaatca gctccaagct actcaagctg ccaggaaact 240
actttccaga gaaaaacagc cccccataga caacataatc cgggctggtt tgattccgaa 300
atttgtgtcc ttcttgggca gaactgattg tagtcccatt cagtttgaat ctgcttgggc 360
actcactaac attgcttctg ggacatcaga acaaaccaag gctgtggtag atggaggtgc 420
catcccagca ttcatttctc tggtggcatc tccccatgct cacatnagtg aacaagctgt 480
ctgggctcta ggaaacattg caggtgatgg cttcaatggt nccagacttg ggtanttaag 540
acctggccgg ccggccgttc aaaaggccaa ntccacacct tggcggccgt ctannggatc 600
caactnggac caacttgggg naacatggca aactggttct tggggaaatg gttccgttcc 660
aattccccaa tttcaccgag gctaaagg 688

<210> 697
<211> 732
<212> DNA
<213> Homo sapiens

<220>
<221> misc_feature
<222> (1)...(732)
<223> n = A,T,C or G

<400> 697
gcgggtcgcg gccgaggtac tcccgattga agcccccatt cgtataataa ttacatcaca 60
agacgtcttg cactcatgag ctgtccccac attaggctta aaaacagatg caattcccgg 120
acgtctaaac caaaccactt tcaccgctac acgaccgggg gtatactacg gtcaatgctc 180
tgaaatctgt ggagcaaacc acagtttcat gcccatcgtc ctagaattaa ttcccctaaa 240
aatctttgaa atagggcccg tatttaccct atagcacccc ctctaccccc tctagagcca 300
aaaaaaaaaa aaaaaaaaaa aaaaaaagct tgtaccatct cccagtcctg gaggctggcc 360
atgtgagacc caggtattgc agggctggtt gcttctgagg ctgaggtgtg tcccgtcttg 420
ctccaggccc ttcccagctg gtcttctccc tacatttgca gacngatggc catccgaagn 480
tgacatcatc tcctttgggg ctggctctgg gnccattggg aattaatggt ttanagacng 540
aattcactgg ggtgcttaag cttgggcttc aaaccggtag gnttaaacnn nnttnctttc 600
ttagccttcc aagtaactng atnccnggct taancccctg ggcccanccc aaagttcccc 660
cttttttaan gggcctcttt ttaatngggt taaggnccnc tggaaggatt cntnttaact 720
nggaaancnt na 732

<210> 698
<211> 651
<212> DNA
<213> Homo sapiens

<220>
<221> misc_feature
<222> (1)...(651)
<223> n = A,T,C or G

<400> 698
cgaggtgcca cgtaatgtcc cgtagttcgc tcatcccgtc catgccagat ggattgtggg 60
gaaggtgatt gggacaaaaa tgcaaaagac tgctaaagtg agagtgacca ggcttgttct 120
ggatccctat ttattaaagt attttaataa gcggaaaacc tactttgctc acgatgccct 180
tcagcagtgc acagttgggg atattgtgct tctcagagct ttacctgttc cacgagcaaa 240
gcatgtgaaa catgaactgg ctgagatcgt tttcaaagtt ggaaaagtca tagatccagt 300
gacaggaaag ccctgtgctg gaactaccta cctggagagt cccgttgagt tcggaaacca 360
cccagctaag caaaaatctg gaagaactca atatctcttc agcacagtga agcgggagtg 420
gaagaaggat ctaaagggaa aaactgacat gtttatgtta tggaaaaaga aattttctaa 480
gttcatcaca actgngtcag ttcttgngng ttatgaatac taaaccaatg aataanggct 540
actatggttt tacaaaaaaa nnnaataaaa anaactgnct gccggggcgt naaggnaatn 600
accatgngcg tntntggnnc acttggccac ntggganngg cnantgtctg g 651

<210> 699
<211> 709
<212> DNA
<213> Homo sapiens

<220>
<221> misc_feature
<222> (1)...(709)
<223> n = A,T,C or G

<400> 699
actgtagcat attaataccc tgtgaactgc aaaaaaccaa atacatttac agtagtattg 60
gtcaccaaaa tagaggggaa actttacaat tgtgagaatg tgtaaatgtt ctcattaagg 120
cagtattgac ccagacaacc atttagtatt catctatccc ctcaatgcct cataattctg 180

gaatgcctgt tgtgaaacat gtcagtgcac agtgtctcct aaattctcac acgtgcttga 240
ttttctgatt catctggtga actgggagta ggaagttggt catagacaat atgccctcct 300
tctcttgtct gaccaaagct tgaagcaatc acatctactg ccaggttagc tgtagtcttc 360
gcctcttcct ctgaggtggc caactgagga ttgacttcaa caagatccag tgctgatagc 420
aaccctgnat tgggtattcc tcagcaatat acatgccttc tcgatanggt aagtcccccg 480
acacaggagt tnctgtggct tggagcccgt gtaggggcaa atgcntnaat atcnaaactt 540
caaatggaat gggcttttgg ctcttgccaa tcancngaac caaangttcg ntccctgaac 600
cntttggaaa cccagttnat tcaanttntn tcangggaaa aaacctggga atcnaagnct 660
tttaaaaaaa aaggttcnga ngggncnccg tttttnaacc aaaaaaccc 709

<210> 700
<211> 656
<212> DNA
<213> Homo sapiens

<220>
<221> misc_feature
<222> (1)...(656)
<223> n = A,T,C or G

<400> 700
ggtcagaacc taaaggtttc actgaatgcg aaatgacgaa atctagccct ttgaaaataa 60
cattgttttt agaagaggac aaatccttaa aagtaacatc agacccaaag gttgagcaga 120
aaattgaagt gatacgtgaa attgagatga gtgtggatga tgatgatatc aatagttcga 180
aagtaattaa tgacctcttc agtgatgtcc tagaggaagg tgaactagat atggagaaga 240
gccaagagga gatggatcaa gcattagcag aaagcagcga agaacaggaa gatgcactga 300
atatctcctc aatgtcttta cttgcaccat tggcacaaac agttggtgtg gtaagtccag 360
agagtttagt gtccacacct agactggaat tgaaagacac cagcagaagt gatgaaagtc 420
caaaaccagg aaaattccaa agaactcgtg tcctcgagct gaatctggtg atagccttgg 480
tctgaagatc gtgacttctt tacagcattg atgcatatag atctcaaaga ttnaagaacn 540
gaacgtcntc ataagcagtg atgtccgaag ganatgtctt aaactgntga aaaatancct 600
tcttgcagta ttcaccgaaa gcggactatc caatattcnc nacgggttta ctgcnn 656

<210> 701
<211> 716
<212> DNA
<213> Homo sapiens

<220>
<221> misc_feature
<222> (1)...(716)
<223> n = A,T,C or G

<400> 701
ggtaccttga cagggacgag aggtcgaagg agttgccagc cccatctttg aatgaacatt 60
cagtcagatc gaaaggtggg caggcatact gcgttcgcca ctcaaacaag taggaacaat 120
ctgaagtctc ctttagaaat actggccgct gggtgccgcg gtcacagtag aagaagatgg 180
ctgtggagcg ctgataaacc ttatggcaag tgtccccccc gtgaagttca tttttaacaa 240
gccattttca taagttagct tctgagtcag gagacctgcc actttgtgaa atccctgcgg 300
ttcccgcttt tcctgacatg aggagaccac cttggacttg ncacttgtgg gggcagacgt 360
ctgaggaaaa gctttccaca gaccccgaaa gtaataaagt gtattcgcca gcgctnacga 420
atggtgtcgt tgaagcccaa gggcttnang tcatacaagt tgccatgccc ttgggtcttt 480
caccttacaa gttgnccccn ttcacttttg acaacgggac caggctttca caagttttcc 540

aantaacccg taccttgccc nggccggccg ttnnaaangg gcnaattcca nncacttggn 600
ggccgtacta aggggatccc aactttggac ccaacttggn gnaaanatng ggcntaactg 660
gttccctggg gnaaaatgtt tcccgttcaa aattcccncn aantttgagc cggaag 716

<210> 702
<211> 707
<212> DNA
<213> Homo sapiens

<220>
<221> misc_feature
<222> (1)...(707)
<223> n = A,T,C or G

<400> 702
tgnatntgtc agcggcgcag tgtatggtat ctgnagaatt cgcctttcga gcggcgccgg 60
gcaggtactc atcttatact gaaagaacgt ggtggctcta aatatgaagc tgcaaagaag 120
tggaatttac ctgccgttac tatagcttgg ctgttggaga ctgctagaac gggaaagaga 180
gcagacgaaa gccatttctt gattgaaaat tcaactaaag aagaacgaag tttggaaaca 240
gaaataacaa atggaatcaa tctaaattca gatactgcag agcatcctgg cacacgcctg 300
caaactcaca gaaaaaccgt cgttacacct ttagatatga accgctttca gagtaaagct 360
ttccgtgctg tggtctcaca acatgccaga caggtcgcag cctcccagca gtaggacaac 420
cacttcagaa ggagccctcg ttacacctgg atacaccatc aaaattcctg tccaaggaca 480
aactcttnaa gccttccttt gatgtgaagg atgcacttgc agccttggaa acttcangac 540
gtccagccac agaaaaggaa ccgagtcctn ggccgcgacc ccctaaggca attcacacac 600
tggcggcgtc tagggaccac ttgggccaac ttgngaactg gctactggtc tgggaatgtn 660
ccgtacatcc ncaatnaccg actaagtaac tgggctnngg gctatcn 707

<210> 703
<211> 703
<212> DNA
<213> Homo sapiens

<220>
<221> misc_feature
<222> (1)...(703)
<223> n = A,T,C or G

<400> 703
acctgccaga attagcaaga gctttcttta agaagacatt tgtcaaactc aacaaattga 60
aggttaacac cttaagagtt gtagttactg accagaaata tggacagact tcttagactt 120
ggaggaggta tgcctggact gggccagggg ccacctacag atgctcctgc agtggacaca 180
gcagaacaag tctatatctc ttccctggca ctgttaaaaa tgttaaaaca tggccgtgct 240
ggagttccaa tggaagttat gggtttgatg cttggagaat ttgttgatga ttataccgtc 300
agagtgattg atgtgtttgc tatgccacag tcaggaacag gtgtcagtgt ggaggcagtt 360
gatccagtgt tccaagctaa aatgttggat atgttgaaca gacaggaaag cccgaaatgg 420
ttggttggtt ggtatcacaa gtcaccctgg ctttggttgg tggctttctg gtgtggatan 480
tcaacacttn agcagagctt ttgaagcctt ttccggaaaa nagctttggc antgggttgt 540
ggatcccttt canaatggta aaaggaaagg ttggtaattg atgccttcan aatggancaa 600
ggctaaatna agggcttagg acttgaaccc ggacaanaan tttaaattng gncccttaaa 660
caagcctttt ntcnggcttt attttggctt accnnctttt tnn 703

<210> 704

<211> 683
<212> DNA
<213> Homo sapiens

<220>
<221> misc_feature
<222> (1)...(683)
<223> n = A,T,C or G

<400> 704
cgaggtactg agggatagga gagtatatgg gtttggcacc acagggtggg taggcaaaac 60
aatttggttg ataaggctca gatcctgaac taacctgtaa gggcttgtct ggttcgagga 120
caggtgaaat gggggaattg taagtagagt ttataggctt taaaaggcca tgctgtagca 180
ggcgagtgat aacaggcttt aatcttttta aagcatgctg tgggatggga tattggcatt 240
gagcggggta agggtgatta ggttttaatg agatggtaag gggtccatga tcggtcacca 300
aggagggagt agaggtatct tatacttgtg ggttaaggtg gggggataca agaggaggac 360
gcanaggagg ctttggattg ggaaaaaagg gcaccaatga gatgtaccnt aatccaggaa 420
tagtcaggga aacnnatagt tanttaaaag tgtctcggct aatangggac tgggcagtgg 480
ggatactaaa aaggatgctt aaaaagtatg nctaagttgc accnnattna ngagtttaaa 540
aaggttaaaa acttgctggn aatcctanca ccnttttgga gcnagaaaac aggcccttna 600
aanaaggtat ntgaatggga accccntntt aaaaggggcg gcntaatttc cctgnaaagt 660
cttnaactnt nnaaggccct acn 683

<210> 705
<211> 463
<212> DNA
<213> Homo sapiens

<400> 705
ctgaaagtcg atgaaggacg cgattacctg cgataagctt cgtggagttg gaaataaact 60
atgatacgga gatttccgaa tggggtaacc taactgagca aacctcagtt gcattttgat 120
gaatccatag tcaaattagc gagacacgtt gcgaattgaa acatcttagt agcaacagga 180
aaagaaaata aataatgatt tcgtcagtag tggcgagcga aagcgaaaga gcccaaacct 240
gtaaaaaggg gttgtaggac atcttacatt gagttacaaa attttatgat agtagaagaa 300
gttggaaagc ttcaacatag aaggtgatat tcctgtatac gaaatcataa aatctcatag 360
atgtatcctg agtagggcgg ggcaccgtga aaccctgtct gaatctgccg ggaccacccg 420
gtaaggctaa atactaatca gacaccgata gtgaactagt acc 463

<210> 706
<211> 651
<212> DNA
<213> Homo sapiens

<220>
<221> misc_feature
<222> (1)...(651)
<223> n = A,T,C or G

<400> 706
actatagcat ctgtggaaaa tcttagaaaa aaacattttc tcccccaccc tctctcttcc 60
ctgttaagac catcccaaaa tgcttcaagt aaaaaataac aagtttaagg ggttaagcac 120
ttttaaagtc tgattaaggg ggtgggggga aaaaagagta actaccagcc atttctccaa 180
tggacatctc ttccacagac ctcaacgtga gaactgctct agtttctata aactgtaaac 240

ctgtggtggt ctgattatcc tgatattgga ttttcttgtt ttctgttaca ccttgagtca 300
tttgccttta ggattctaga cagacctaag ggaaaaagaa ctgaaaacat attttgcccc 360
cacccccaca aaaaaaaata ctgaaaactc ccccccgcct cagttacaca tccaaactct 420
acatttacaa aacgaattca gggtgaggaa gtaaaacagg tcatctattc acaaaactga 480
aatacttcat taccccaact aaacatacaa actgnttaca gattgctgaa atggctcaat 540
ttggctatca aattcatttg ggtttcctca aatcgngtaa aaaaaaaaaa aaaaaaagct 600
tggncctngg ccgnaacacn cttangggca aatccanccc ctgggnggcc g 651

<210> 707
<211> 625
<212> DNA
<213> Homo sapiens

<220>
<221> misc_feature
<222> (1)...(625)
<223> n = A,T,C or G

<400> 707
ggtggcggct cgggacggag gacgcgctag tgttcttctg tgtggcagtt cagaatgatg 60
gatcaagcta gatcagcatt ctctaacttg tttggtggag aaccattgtc atatacccgg 120
ttcagcctgg ctcggcaagt agatggcgat aacagtcatg tggagatgaa acttgctgta 180
gatgaagaag aaaatgctga caataacaca aaggccaatg tcacaaaacc aaaaaggtgt 240
agtggaagta tctgctatgg gactattgct gtgatcgtct ttttcttgat tggatttatg 300
attggctact tgggctattg taaaggggta gaaccaaaaa ctgagtgtga gagactggca 360
ggaacccgag tctccagtga gggaggagcc aggagaggac ttcctgcaca cgtcgcttat 420
attgggatga cctgaagaga aagttgtcgg agaaactggc agcacagact tcaccagcac 480
catcaagctg ctgaatgaaa atcatatgtc cctcgtgang ctggatctca aaagatgaaa 540
atctgcttga tgttgaaatc aattcgtgaa ttaactcaca agttgcgtga cacatttgta 600
aatcngcaaa cacntnaaac tgggn 625

<210> 708
<211> 209
<212> DNA
<213> Homo sapiens

<400> 708
actgttccat ctggaagtca agattggtgc cacctaagtg ggttcctgct gcaaggaact 60
taaggacatc ctcctccttc atttgcagga catcaagggc tccggacatt gtgaaagttt 120
ccctttaagt tacgacggga atccagaaca acgccgtatg gacccctctg caggtagcac 180
ggaaaaaaaa aaaaaaaaaa gcttgtacc 209

<210> 709
<211> 643
<212> DNA
<213> Homo sapiens

<220>
<221> misc_feature
<222> (1)...(643)
<223> n = A,T,C or G

<400> 709

ggtactcctt agagccagtt gctgtagaac tcaaatctct gctgggcaag gatgttctgt 60
tcttgaagga ctgtgtaggc ccagaagtgg agaaagcctg tgccaaccca gctgctgggt 120
ctgtcatcct gctggagaac ctccgctttc atgtggagga agaagggaag ggaaaagatg 180
cttctgggaa caaggttaaa gccgagccag ccaaaataga agctttccga gcttcacttt 240
ccaagctagg ggatgtctat gtcaatgatg cttttggcac tgctcacaga gcccacagct 300
ccatggtagg agtcaatctg ccacagaang ctggtgggtt tttgatgaag aaggagctga 360
actactttgc aaaggccttg gagagcccag agcgaccctt cctggccatt ctnggcggac 420
taaagttgca gaccagatcc agctcatcaa taatatgctg gacaaaagtc aatgagatga 480
ttattggtgg tggaatggct tttaccttcc ttaangngct caacaccatg gagattggca 540
cttctctggt tgatgaaaaa gggncccaga ttgcaaagac tnatgtccaa actgagaaaa 600
agggntgaan ataccttgcc tgtgctttgc nctgttncaa ttg 643

<210> 710
<211> 390
<212> DNA
<213> Homo sapiens

<400> 710
ggtactcttc tagcatttag atctacactc tcgagttaaa gatggggaaa ctgagggcag 60
agaggttaac agatttatct aaggtcccca gcagaattga cagttgaaca gagctagagg 120
ccatgtctcc tgcatagctt ttccctgtcc tgacaccagg caagaaaagc gcagagaaat 180
cggtgtctga cgattttgga aatgagaaca atctcaaaaa aaaaaaaaaa gaaaagagaa 240
aaaaaagact agccagccag gaagatgaat cctagcttct tccattggaa aatttaagac 300
aagttcaaca acaaaacatt tgctctgggg ggcagggaaa acacagatgt gttgcaaagg 360
taggttgaag ggacctctct cttaccaagt 390

<210> 711
<211> 683
<212> DNA
<213> Homo sapiens

<220>
<221> misc_feature
<222> (1)...(683)
<223> n = A,T,C or G

<400> 711
cgaggtcaag aaggcagccc gagaagaaac gggaggacaa agctaagaag aagcacgaca 60
ggaaatccaa acgcctggat gaggaggagg aggacaatga aggcggggag tgggaaaggg 120
tccggggcgg agtgccgttg gttaaggaga agccaaaaat gtttgccaag ggaactgaga 180
tcacccatgc tgttgttatc aagaaactga atgagatcct acaggcacga ggcaagaagg 240
gaactgatcg tgctgcccag attgagctgc tgcaactgct ggttcagatt gcagcggaaa 300
acaacctggg agagggcgtc attgtcaaga tcaagttcaa tatcatcgcc tctctctatg 360
actacaaccc caacctggca acctacatga agccagagat gtgggggaag tgcctggact 420
gcatcaatga gctgatggat atcctgtttg caaatcccaa catttttgnt gggggagaat 480
attcttggaa gaaaagtgag aacctgcaca acgctgaccc agcccttgcg tgtccctggc 540
ttgcatnctn acttttggtg ggaaccnaat gggttaaaga aattanccca ataatgccaa 600
atacttgacc cttanttccc aaaaatacct tgcccgggcg ggcccnttca aaagggccaa 660
attccancnc ccttgggggc ccg 683

<210> 712
<211> 605
<212> DNA

<213> Homo sapiens

<220>
<221> misc_feature
<222> (1)...(605)
<223> n = A,T,C or G

<400> 712
ggtacaagct tttttttttt tttttttttt tttctaaaca atagtgcttt attgataaaa 60
ggttagttta aatggataca aaattgctgt gtaaaataag tgttttcaaa atacatttct 120
ataggtagag actatgtctt agtaaaagag cagttatcta ttatcaaaag tatctattta 180
natttgggta gtaaaaccaa aggggatcag aagtgtanca gtgtgggtcc tccctccctg 240
catagctgtt accaggaggc agcgtgcctg aagtacttgg aggaacgaag aataaaggag 300
attgtgaaga aacattctca gcttattgga tatcccatta ctctttttgt ggagaaggaa 360
ccgtgataaa gaagtaagcg atgatgaggc tgaagaaaag gaagaccaag aagaagaata 420
ngaanaagaa gagaaagagt cggaagacaa acctgaaatt gaanatgttg gtctgatgag 480
gaagaaaaaa gaaggtggtg cnagaagaan anaagaagat taggaaagtc ctgccggcgg 540
ccgtcaangc aatccaccct gcggcgtcta ngaccactgn ncactgngat atgctctgtc 600
tggna 605

<210> 713
<211> 376
<212> DNA
<213> Homo sapiens

<400> 713
ggtaccaagg ttattgatca agtcagcctt ggtcattcca attccagtat ccacaatagt 60
gagagttcga tcttgtttgt tcggtataag gttaatatgc agctctttcc cagagtctaa 120
tttactggga tctgtcaagc tttcataccg gattttgtcc aatgcatctg atgaatttga 180
aatgagctct ctcagaaaga tctctttgtt cgagtagaaa gtattgatga tcaatgacat 240
caactgggca atttctgcct gaaaggcgaa cgtctcaacc tcctcctcct ccatcggttg 300
gtcttgggtc tgggtttcct caggcatctt ggctaagtga cccgcacagg accaacggca 360
cagccacacc gacctg 376

<210> 714
<211> 378
<212> DNA
<213> Homo sapiens

<400> 714
cgaggtacca aggttattga tcaagtcagc cttggtcatt ccaattccag tatccacaat 60
agtgagagtt cgatcttgtt tgttcggtat aaggttaata tgcagctctt tcccagagtc 120
taatttactg ggatctgtca agctttcata ccggattttg tccaatgcat ctgatgaatt 180
tgaaatgagc tctctcagaa agatctcttt gttcgagtag aaagtattga tgatcaatga 240
catcaactgg gcaatttctg cctgaaaggc gaacgtctca acctcctcct cctccatcgg 300
ttggtcttgg gtctgggttt cctcaggcat cttggctaag tgaccgcaca ggaccaacgg 360
cacagccaca ccgacctg 378

<210> 715
<211> 310
<212> DNA
<213> Homo sapiens

<220>
<221> misc_feature
<222> (1)...(310)
<223> n = A,T,C or G

<400> 715
acttttgagt gtgtgtgtgc atgtgtgtgt gtgtgtgtgt gtgtgtgtat gtgagagatt 60
ctgtgatctt ttaaagtgtt actttttgta aacgacaaga ataattcaat tttaaagact 120
caaggtggtc agtaaataac aggcatttgt tcactgaagg tgattcacca aaatagtctt 180
ctcaaattag aaagttaacc ccatgtcctc agcatttctt ttctggccaa aagcagtaaa 240
tttgctagca gtaaaagatg aagttttata cacacagcan aaaaaaaaaa aaaaaaaaaa 300
agcttgtacc 310

<210> 716
<211> 624
<212> DNA
<213> Homo sapiens

<220>
<221> misc_feature
<222> (1)...(624)
<223> n = A,T,C or G

<400> 716
ggtaccgatt gccaggctgt ggtctcctcc cagtgtgaca cggctgtagc catctgacac 60
agctctgcta accacctcag ccagttcctg gttggcaaga cccactgagc gtggattcac 120
tatcaggttg ttgtagagat catctttggg gactggagta aaattcaaat ctccaaagtc 180
ttttaggtgg cagcccaaac tggagagcct tttcatcaag ccagcttctc ttatggcagc 240
gggaccatgc tccactccgt ttcttttctg tccttgtgag aacggggctc ctatcacagc 300
cacggagtgg acggatttct tcaggatgga atgcactcgc gtctggagga gacgcgagag 360
gctgccctta gggacatgat cccgcagcac tgagaatctc caaggcagag gctccacatg 420
gccggggtgt tgaaggtctc aaacataatc tgagtcatct tctctctgtt ggccttgggg 480
ttcaaggggg cctcggcaca gcactgggtg ctcttncggg ccacgcgcac ttgtgtaaaa 540
gtgngtgcca nactttcatg cgnccaattg gngaccatcc tctnatggga ctgccggggc 600
cgttnaaggg gaatcaccnt ggng 624

<210> 717
<211> 652
<212> DNA
<213> Homo sapiens

<220>
<221> misc_feature
<222> (1)...(652)
<223> n = A,T,C or G

<400> 717
cgaggtacaa aaattagctg ggtgtcgtga tgggtgcctg taatcacagc tatgtgggag 60
gctgaggcag gagaattgct tgaacctggg aggcgaaggt tgcagtgagc caagatcacg 120
tcactgcact ccagcctctt tgacagagtg cgactctgtc tcagaaaaaa aaaaaaaaga 180
aagaaaagag attacatatt atttagaaaa cagcagctaa acagtctttg ggtctctggc 240
aaagatgaag tgagccagtc ttcttccgac taaatcacca actggacaaa gttctcagct 300
ggaaaacact ccccttctgg gatcctgcgc ccagaagtgg tagcaagaac ttcttggaat 360

agaatggagc agaaccttcc tgagcctgag gaaccaacaa aaagtcaaag aatgaactct 420
ttcgaacaca aaataaaatt tctcaaagcc caggtcatgc tttttctgta aatctttatc 480
cctgcgtcag tatggacatg acatagtcca gagagaaaat tctcagccta ccttatgcnc 540
aagaaaatgc catgatgccg ccagcttgtt gatgcccnag gacantgctn ttganggccg 600
gaaaataggn ctgcagcngg gaaccaaagg ctgttnncct gnttcttaaa ag 652

<210> 718
<211> 544
<212> DNA
<213> Homo sapiens

<220>
<221> misc_feature
<222> (1)...(544)
<223> n = A,T,C or G

<400> 718
cacagaggga gtgaggtgca tttgcagtca gctttcgctc accactaaga tggatgcaga 60
gcatccggaa ctcaggagtt acgctcagag ccaaggttgg tggacgggag agggcgagtt 120
caatttttcc gaagtctttt ctccagttga ggatcatcta gactgcggtg ctggcaaaga 180
cagcttagaa aaacaagaag aaagcatcac agtgcagact atgatgaaca ccttacggga 240
caaagccagc ggagtgtgca tagactctga gtttttcctc accacagcca gtggagtgtc 300
tgtcctgccg cagaatagaa gctctccgtg cattcactac ttcactggaa cccctgatcc 360
ttccaggtcc atattcaagc ttttcatctt tggtgatgac gtaaaacttg tccccaaaac 420
acaagtctcc ctgttttggg ggatgacgac ccttgccaaa aaggagcctc gggttncagg 480
agaaaccnga accggccggc attgaacctg taccttgncc gggccggccg nttcnaangg 540
gcga 544

<210> 719
<211> 626
<212> DNA
<213> Homo sapiens

<220>
<221> misc_feature
<222> (1)...(626)
<223> n = A,T,C or G

<400> 719
accaaagaaa agctgaacag gaaaatgaga agagaagaaa tgtagaaaat gaagtttcta 60
cattaaagga tcagttggaa gacttaaaga aagtcagtca gaattcacag cttgctaatg 120
agaagctgtc ccagttacaa aagcagctag aagaagccaa tgacttactt aggacagaat 180
cggacacagc tgtaagattg aggaagagtc acacagagat gaacaagtca attagtcagt 240
tagagtccct gaacagagag ttgcaagaga gaaatcgaat tttagagaat tctaagtcac 300
aaacagacaa agattattac cagctgcaag ctatattaga agctgaacga agagacagag 360
gtcatgattc tgagatgatt ggagaccttc aagctcgaat tacatcttta nagaggaggt 420
gaacatctca acataatctc gaaaaagtgg aaggagaaag aaaagagctc aagacatgct 480
taatcactca gaaaaggaaa gaatatttag agatagattt aactacaact taaatcnttc 540
acacggtaga ccagangtaa tgaccccagt accaagctcg ttactgcaac atcattnttg 600
agaggcaagc ttggcatggg taaaaa 626

<210> 720
<211> 469

<212> DNA
<213> Homo sapiens

<220>
<221> misc_feature
<222> (1)...(469)
<223> n = A,T,C or G

<400> 720
ggtactcttt agcattaaat tacatcgtgc atatacaact acacccattt agatttgcct 60
tggaatataa tttcaaggcc ttaaatatta aaaataattt tataactatt tcatagttta 120
attggctctt aaatagtttt gctagggagg aaacattttg tgttctttaa gaaattgata 180
tgtgtaaatg tgttcactta aatcttgaga aaacctaagg atgaagtctg ttgttttgtt 240
tttcctaaaa aaggaaaaaa gaaccaaaga aaaatgttga agaacaagaa tatttaccat 300
taaaaagaag aaacattatc caacaaaaag gagacatata gatttgaaaa cacttatttt 360
actgncttca acaacaacaa caaacagata ggcaggggaa gtccagagga ctcagaattg 420
aagcagctct atacaataat gaaggtggac ctgccgggcg ggcgctcga 469

<210> 721
<211> 644
<212> DNA
<213> Homo sapiens

<220>
<221> misc_feature
<222> (1)...(644)
<223> n = A,T,C or G

<400> 721
acaaggtcaa tctcacttcg agtgaccaca atccggacca gggtggagtc atctgtgcca 60
gcacctttca tagcatagta gagcctctca gcaaagaagg cagggcggtt cagggcacac 120
tgcaagatgg tcttcaaacc actttctaca tatccggaaa actcacggct cacactgctt 180
aacaagtctc gattagccat cctagaataa gcctccatgg tagctctcag ctgaggaaag 240
cttcttgtgg caaggatcat gttaaagcaa gattcatcgg tccctagtct cccctcacca 300
gcttgataga gacgctgagc atcttcctga gccatttggt ggtttatact ctggttctca 360
tcacgatttc cctggcacat ggacacaagt aaacgttcaa aatgtcctga tgtatctgac 420
ctaatgncct tttcaaggtc tcgtccaaat tctgactgat aacatctgac aatttctcgg 480
atttcctgat ttggtcttgn gcacaaaatc ttcaatcaat acaccgttcc tgagttcctg 540
ntncctgcat tgntttccga agcttcaggc atcgnaatcc taggangctt gaaaaggccn 600
ggatcagttn ttcctattcn cttactttga ttgaaacntt gata 644

<210> 722
<211> 510
<212> DNA
<213> Homo sapiens

<220>
<221> misc_feature
<222> (1)...(510)
<223> n = A,T,C or G

<400> 722
cgaggtcgga gatctcgccg gctttacgtt cacctcggtg tctgcagcac cctccgcttc 60

ctctcctagg cgacgagacc cagtggctag aagttcacca tgtctattct caagatccat 120
gccagggaga tctttgactc tcgcgggaat cccactgttg aggttgatct cttcacctca 180
aaaggtctct tcagagctgc tgtgcccagt ggtgcttcaa ctggtatcta tgaggcccta 240
gagctccggg acaatgataa gactcgctat atggggaagg gtgtctcaaa ggctgttgag 300
cacatcaata aaactattgc gcctgccctg gttagcaaga aactgaacgt cacagaacaa 360
gagaagattg acaaactgat gatcgagatg gatggaacag aaaataaatc taagtttggt 420
gccaacgcca ttctgggggt gtcccttgcc gctgcaaagc tggtgccgtt gagaangggg 480
tcccctgtac ctgccnggcg gccgtcgaaa 510

<210> 723
<211> 640
<212> DNA
<213> Homo sapiens

<220>
<221> misc_feature
<222> (1)...(640)
<223> n = A,T,C or G

<400> 723
ggtaccaagc gtatcagcat tcacctcctt gcctcacatg ccagtgggct caatcacaac 60
cctgcctgtg aatctgtaat tgactcctca acatttggag aaggcaaagc tccaggtccc 120
ccttttcctc aaactcttgg catagccaac gtggccaccc gcctctcttc catccagctg 180
ggccagtctg agaaggagag acctgaggag gccagggagc tggactcatc tgatagggat 240
attagttcag ctactgacct ccagccagat caggctgaga ctgaagatac agaagaagaa 300
ctagtagatg gtttggaaga ctgntgtagc cgtgatgaga atgaagagga ggagggagac 360
tcagagtgct cctcattaag tgctgctccc ccagcgaatc ggtggccatg atctctagaa 420
ctgtatggaa attctgacca aacccctttc caatcatgag aaaagttgtc cgaccagcct 480
catctacagc tctttccaac gttcccctac catctatttt ggcactcggg atgaaaaant 540
ggagaaactt tcctgggaac cnangaagtt gcttcnatgg aagatgagcn cagggacccc 600
aacattgcaa ccnaccattg gacggncccc tttaaatang 640

<210> 724
<211> 593
<212> DNA
<213> Homo sapiens

<220>
<221> misc_feature
<222> (1)...(593)
<223> n = A,T,C or G

<400> 724
ggtacctgcg cgccctcgac gtcaatgtgg ccttgcgcaa aatcgccaac ttgctgaagc 60
cagacaaaga gatcgtgcag gacggtgacc atatgatcat ccgcacgctg agcactttta 120
ggaactacat catggacttc caggttggga aggagtttga ggaggatctg acaggcatag 180
atgaccgcaa gtgcatgaca acagtgagct gggacggaga caagctccag tgtgtgcaga 240
agggtgagaa ggaggggcgt ggctggaccc agtggatcga gggtgatgag ctgcacctgg 300
agatgagagt ggaaggtgtg gtctgcaagc aagtattcaa gaaggtgcag tgaggcccag 360
gcagacaacc ttgtcccaag gaatcagcag gatgtgtggg ccaggatccc cttttgcaca 420
gcatgaggca aaaatgtcca ccacccccag cattgttagc agatctgctc ttgctttgca 480
cttttctttc ttaaacaaac ctgcataagt gatctgtgtt agaaaaactg ccggcggcca 540
agcaatcacc atgcgcgtct atgaccactn nncactgcna tatgctantg tct 593

<210> 725
<211> 606
<212> DNA
<213> Homo sapiens

<220>
<221> misc_feature
<222> (1)...(606)
<223> n = A,T,C or G

<400> 725
acngcagctg ctccacggcc ccagcacgaa atgtatcaca ggcagcaatg aggacactga 60
agccattctc taacaaccag aaggaaatct tggcaagatt agtagatttc cccactccat 120
taacgccgca gaaggtgacg acataagggc gctggcgacg ctgggcatcc atgatgtccc 180
ggagcatgtc tacacgacgc tgtggctgca gaatctgcac cagggactcc tgtagggctt 240
gctttactgt ggaagtcacc gtgctgaacg tccccatcac cttcccttcc aacttgttgg 300
caacagattc acagagctgg acggcaatgt ctgcagccac gttcttagca atgagatgat 360
cacgcatctt gtccagcaca gattccatgt cttcacgact caagctcttt gaacccacaa 420
ggcccttcag cataccaaac atgccaccca gtgttccttg gtcgcactan gtttggtaga 480
gttttgagca gcccttcgtc atcaanctgt gcatccagat ctgaactgcc ccagaccagc 540
cttgaatagg tgatgcctaa caggagctag ggtcatgngg tggagactgg cgncacctag 600
gcaatc 606

<210> 726
<211> 594
<212> DNA
<213> Homo sapiens

<220>
<221> misc_feature
<222> (1)...(594)
<223> n = A,T,C or G

<400> 726
accacatcat ccatgctgac atctaccgct ggtttaacat ttcgtttgat atttttggtc 60
gcaccaccac tccacagcag accaaaatca cccaggacat tttccagcag ttgctgaaac 120
gaggttttgt gctgcaagat actgtggagc aactgcgatg tgagcactgt gctcgcttcc 180
tggctgaccg cttcgtggag ggcgtgtgtc ccttctgtgg ctatgaggag gctcggggtg 240
accagtgtga caagtgtggc aagctcatca atgctgtcga gcttaagaag cctcagtgta 300
aagtctgccg atcatgccct gtggtgcagt cgagccagca cctgtttctg gacctgccta 360
agctggagaa gcgactggag gagtggttgg ggaggacatt gcctgcagtg actggacacc 420
caatgcccag ttatcacccg ttcttgcttc nggatggcct caaccacgct gataacccga 480
gacctcaatg gggaacctgt cctcggcgga cacctaggca atcacacact gcggccgtct 540
agtgatccac tcgaccactt gcgatatgga tantgtctgg taatgatcgt acat 594

<210> 727
<211> 665
<212> DNA
<213> Homo sapiens

<220>
<221> misc_feature

<223> n = A,T,C or G

<400> 727
gcgtggtcgc gccgaggtgc cgtcaaggag tagaaattgg tatgcttaga agcagattct 60
aaaagcagtt tctcttcaga acatcttttt tcataccact tgataagcat cttgaaacac 120
catggctgta gctgcagtaa aatgggtgat gtcaaagaga actatcttga aacatttatt 180
tccagtccaa aatggagctt tatattgtgt ttgtcataaa tctacgtatt ctcctctacc 240
agatgactat aattgcaacg tagagcttgc tctgacttct gatggcagga caatagtatg 300
ctaccaccct tctgtggaca ttccatatga acacacaaaa cctatccctc ggccagatct 360
gtgcataata atgaagaaac acatgatcaa gtgctgaaaa ccagattgga agaaaaagtt 420
gaacaccttg aggaaagacc tatgatngaa ccacttancc aaatggtcnt tactactaag 480
cacccgtggn attcctcatg gacngnntac agatgtcnta agaatctgaa tcctccaaag 540
accgatgatg ccganggtcc tggggggatc aaaagaaaag ggncccattt gcatttggna 600
aaagccanct gggggttccn tattttttgt aaggaataat gntaaaaatc tttctntttt 660
anaag 665

<210> 728
<211> 624
<212> DNA
<213> Homo sapiens

<220>
<221> misc_feature
<222> (1)...(624)
<223> n = A,T,C or G

<400> 728
ggttacccag gcagtatctc tagagtcctt aacttaatat tagtaactaa agaaaagggt 60
tgcgctcgtt gcaggactta acctaacatc tcacgacacg agctgacgac aaccatgcac 120
catctgtcat tctgttaacc tccactatat ctctatagct ttgcagaaga tgtcaagagt 180
gggtaaggtt ctacgcgtag aatcaaatta aaccacatgc tccaccgctt gtgcgggttc 240
ccgtcaattc ctttaaattt cactcttgcg agcatactac tcaggcggat catttaacgc 300
gttagctgcg ttagtgaaat tattccacca actaatgatc atcgtttacg gcgtggacta 360
ccagggtatc taatcctgtt tgctccccac gctttcgtcc cttagtgcaa tatataacca 420
gttagctgcc ttcgcctatt gggntcttcc taatatctac gcattccacc gcttcactag 480
gaattccgtt acctctttat aatctatttg gcagtatcca agcggctgaa gttgagctta 540
acatttactt cagacttaca aaaactacgc gcttacgccc aatattccga tacgttgcac 600
natgattacc ggggtgtgcc aaaa 624

<210> 729
<211> 449
<212> DNA
<213> Homo sapiens

<400> 729
actgacacac aaagtgcctt cactggacct tacagttctc actgccgttg gactccagtc 60
cagctttggg gctggggaca agtcggcctc gcttgaccct caggccctct ctggggctgt 120
cagtcggact tctctcagga agattattga ctgggacgga tttcgtggtg ggttctcgga 180
ggatggtgcc tgaatctact gggctccgct gagcaacttt gaccttttgt gatctgctgc 240
caccagctgt tggtttggag gactctgcaa gattttcttt gccgagactc agtggggata 300
gcgctaactt ctgtgcaacc aggcgggggc tggtcccagt tgccatggtt gttcttcgca 360
ggatatatgg gctaagtctt tcctgtcggg atgtcagcaa accctttctt tacaacttct 420

ggaagtccct ctggctcaaa ctcagtacc                                    449

<210> 730
<211> 646
<212> DNA
<213> Homo sapiens

<220>
<221> misc_feature
<222> (1)...(646)
<223> n = A,T,C or G

<400> 730
actcattaat cagggagcct caatcttagt aaaagattac attttgaaga ggacacctat      60
tcatgcagca gcaacaaatg gtcattcaga atgcttacgg ctattaatag gaaatgcaga     120
accacagaat gcagtggata ttcaagatgg aaatggacag acgcctctga tgctatctgt     180
tctcaacggg cacacagact gtgtttactc attgctgaac aaaggagcaa atgtagatgc     240
caaagataag tggggaagga cagcgttgca tagaggggca gttacaggcc atgaagaatg     300
tgtagatgca ttacttcaac atggtgctaa gtgcttactt cgggatagca ggggcccgga     360
cgcctataca cctgtctgct gcctgtggac acattggtgt tcttggagcc cttttgcagt     420
cagcagcatc tatggatgca aatccagcca cagcagacaa tcatggatat ccgnacttac     480
tgggcttgta caatggtcac gagacatgtg tagaactgnt tttagaacag gaagttttcc     540
agaaaacgga aggaaatgct tttagtccat tgcattgngc cgtgataaat gccaccaaag     600
ggctgttaaa ngttaattga tcnttanggg ccacattggg aacccc                    646

<210> 731
<211> 639
<212> DNA
<213> Homo sapiens

<220>
<221> misc_feature
<222> (1)...(639)
<223> n = A,T,C or G

<400> 731
acagacttgt ttttgagtgt tgagtagcag ggacaaaata agggaatgtt attttttaag      60
aaaattcatt ttcattgttg tctccttcct tttctgtgaa agtcctcata ctgagaaatt     120
tgtatatttt atattaaatc acttactatt gatttttgtt gtgattttca aaggtggatt     180
cccacagata aaatcttggc tattgcccaa aacatagtaa agggtcacgt gtgacttttt     240
ataataggaa gaaaattctg cctttgtgag tgcacatgtc cacatttcat ccctccttcc     300
ctcaaaaccc tagagagggg cattaaagaa ttgttgatgt atatgcaatg tctgttaaag     360
catgcactat gtatttcatc ctcatttatt gggtctggga ctgaagtttt taacccacat     420
ggacctaacc tactttttgg gataaaattc tctgtttggt acaggcaaaa ttctggtatg     480
gcgtgaatgc catgggtcat tctgaatata ttttttctgg aatttatcat acacgatgtt     540
gcaatacgtg ctttggtttt taatttgaag ccaacttttc tactgttgaa agacattttt     600
gccaactggn ccttctanaa tggagtctaa gttaggncg                            639

<210> 732
<211> 538
<212> DNA
<213> Homo sapiens

<220>
<221> misc_feature
<222> (1)...(538)
<223> n = A,T,C or G

<400> 732
ggtactcgtc ccttcaaaca gtaaacaaga aagtgcagac agtgctgcca gagacaggag 60
gattttcaca tgagactgaa aaagccgaca cacccttaca actaagtcat ggtcgagtcg 120
gacctgccat ccacctccac cagtccctgg aacccggcag gtcagagttt tctctaattc 180
tattccccgg catcaagtga acactagaac tcacacggaa ggccccgagc aaccactggc 240
ctcggggctg ggtgcaccca ctcctcaccc agggagattg tcacaaaaca cgctaggggg 300
cagagacgct gtaaactgga cacacacgga acacaatgcc ctttccactt acacagcgtg 360
gggatgataa aaaggaatct tttgagcaag tctataattt tacagaattt agaggtggga 420
aagatggcca attttccttc tttatgcctg gggcagacca cctgcttctg gggtaaagtg 480
tttgagaagg aaaaagaccc tgnacctgcc nngggcggcg ctcgaaaggc caattcna 538

<210> 733
<211> 351
<212> DNA
<213> Homo sapiens

<400> 733
cgaggtaccc tatggcctat gttgactata agactgtgct gcagattgat gataatgtga 60
cgtcagccgt agaaggcatc aacagaatga ccagagctct catggactcg cttgggcctg 120
agtggcgcct gaagctgccc tcaatcccct tggtgcctgt ttcagttcag aagaggtgga 180
attccttgcc ttcggagaac cacaaagaga tggctaaaag caaatccaaa gaaaccacag 240
ctacaaagaa cagagtgcct tctgctgggg atgtggagaa agccagagtt ctgaaggaag 300
aaggcaatga gcttgtaaag aagggaaacc ataagaaagc tattgagaag t 351

<210> 734
<211> 625
<212> DNA
<213> Homo sapiens

<220>
<221> misc_feature
<222> (1)...(625)
<223> n = A,T,C or G

<400> 734
cgaggtacaa tccttgacct tgtgcattat agcattccat tagcaagagt tgtaccatcc 60
ttcatccaaa tggcaacatc acagagctcc tcctgaagga aggtttcgca cgctgtgtgg 120
actggtcgat tgcagtttac acccggggcg cagaaaagct gagggcggca gagaggtttg 180
ccaaagagcg caggctgaga atatggagag actatgtggc tcccacagct aatttggacc 240
aaaaggacaa gcagtttgtt gccaaggtga tgcaggttct gaatgctgat gccattgttg 300
tgaagctgaa ctcaggcgat tacaagacga ttcacctgtc cagcatccga ccaccgaggc 360
tggaggggga gaacacctag gataagaaca agaaactgcg tcccctgtat gacattcctt 420
acatgtttga ggccccggga atttcttcga aaaaagctta ttgggaaaaa gtcaatgtga 480
cngtggacta cattagacca ccagcccagc cacagagaca gtgctgcctt tcaaacgtcc 540
tgccgggcgg ccgtcaaagg cnattcacca tggcggcgtc tatggaccac tcggaccact 600
gggaactggc tactgtctgg gaatg 625

<210> 735

<211> 677
<212> DNA
<213> Homo sapiens

<220>
<221> misc_feature
<222> (1)...(677)
<223> n = A,T,C or G

<400> 735
   actttctatg agaagcgtat gaccacagaa gttgctgctg acgctctggg tgaagaatgg 60
   aagggttatg tggtccgaat cagtggtggg aacgacaaac aaggtttccc catgaagcag 120
   ggtgtcttga cccatggccg tgtccgcctg ctactgagta aggggcattc ctgttacaga 180
   ccaaggagaa ctggagaaag aaagagaaaa tcagttcgtg gttgcattgt ggatgcaaat 240
   ctgagcgttc tcaacttggt tattgtaaaa aaaggagaga aggatattcc tggactgact 300
   gatactacag tgcctcgccg cctgggcccc aaaagagcta gcagaatccg caaacttttc 360
   aatctctcta aagaagatga tgtccgccag tatgttgtaa gaaagccctt aaatanngaa 420
   ggtaagaaac ctaggaccaa agcaccaaga ttcaanngtc ttggtactcc acgtgtcctg 480
   cagcacaaac cggcggtgta ttgctntnna aaaaccagcg taccttnggc cgngaacacc 540
   cttanggccg aatttccagn ccacttggcn ggccgntnct aatgggaatc cancttcggt 600
   acccannctt ggcggaatca tgggcatanc ttggttccct gggtgaaaat ggtattccgt 660
   tcaaaattcc nccaann 677

<210> 736
<211> 651
<212> DNA
<213> Homo sapiens

<220>
<221> misc_feature
<222> (1)...(651)
<223> n = A,T,C or G

<400> 736
ggtactattg aagaactggc tccaaatcaa tatgtgatta gtggtggagt agctattctt 60
aattctacaa ccattgaaat ctcagagctt cccgtcagaa catggaccca gacatacaaa 120
gaacaagttc tagaacccat gttgaatggc accgagaaga cacctcctct cataacagac 180
tatagggaat accatacaga taccactgtg aaatttgttg tgaagatgac tgaagaaaaa 240
ctggcagagg cagagagagt tggactacac aaagtcttca aactccaaac tagtctcaca 300
tgcaactcta tggtgctttt tgaccacgta ggctgtttaa agaaatatga cacggtgttg 360
gatattctaa gagacttttt tgaactcaga cttaaatatt atggattaag aaaagaatgg 420
ctcctaggaa tgcttggtgc tgaatctgct aaactgaata atcaggctcg ctttatctta 480
gagaaaatag atggcaaaat aatcattgga aataagccta agaaagaatt aattaaaggt 540
ctgattcaga ngggatatga ttcggatcct gtgaaggcnt ggaaagaaac ccannaaang 600
gttcngatta agaaaaaaat naanaagagn gccancaaag gaacttgaaa n 651

<210> 737
<211> 404
<212> DNA
<213> Homo sapiens

<400> 737
cgaggtactg tgtggccacc atgccatgtc tagagccagg ctcccgttgt tggccatgcc 60

[?] [?] [?] [?] [?] [?] 120
ccttatcctc accacttcct tcttaggggt ggaaatgctg gatcaaaggg tcttcacgtt 180
ttctgacttt tccacgcatg gggttagcct gtgctccgga gaccctgtga gcacacatgt 240
ccccagcgca gcttgtgact cctgcctctc tgaccccgcc aggtggatta caaagctgac 300
gagtggctga tgaagaacat ggatcccctg aatgacaaca tcgccacact gctccaccag 360
tcctctgaca agtttgtctc ggagctgtgg aaggatggta cctg 404

<210> 738
<211> 250
<212> DNA
<213> Homo sapiens

<400> 738
acatcaaaga ttacatgaaa tcaatcaaag ggaaacttga agaacagaga ccagaaagag 60
taaaaccttt tatgacaggg gctgcagaac aaatcaagca catccttgct aatttcaaaa 120
actaccagtt ctttattggt gaaaacatga atccagatgg catggttgct ctattggact 180
accgtgagga tggtgtgacc ccatatatga ttttctttaa ggatggttta gaaatggaaa 240
aaaaaaaacc 250

<210> 739
<211> 582
<212> DNA
<213> Homo sapiens

<220>
<221> misc_feature
<222> (1)...(582)
<223> n = A,T,C or G

<400> 739
acagtaagga caaccccaac ctgctgttca acatgtgtgg cttcgagtgc cgcatcctgc 60
ctaagtgccg caccagctat gaggagttca cccacaagga cggggtctgg aacctgcaga 120
atgaggttac taaggagcgc acagctcagt gtttcctgcg tgtggacgat gagtcaatgc 180
agcgcttcca caaccgcgtg cgtcagattc tcatggcctc tgggtccacc accttcacca 240
agattgtgaa taagtggaat acagctctca ttggccttat gacatacttt cgggaggctg 300
tggtgaacac ccaagagctc ttggacttac tggtgaagtg tgagaacaaa atccagacac 360
gtatcaagat tggactcaac tccaagatgc caagtcggtc cccccggttg tgttctacac 420
ccctaaggag ttgggtggac tcggcatgct ctcaatgggc catgtgctca tnccccaatc 480
cgacctcagg tgggtccaaa cagacngatg taggtatcac acactttcgt tcaggaatga 540
gccttgaaga agaccactta ttcccacttg nacctcggcc gg 582

<210> 740
<211> 576
<212> DNA
<213> Homo sapiens

<220>
<221> misc_feature
<222> (1)...(576)
<223> n = A,T,C or G

<400> 740
ggtaggacac cgaacccctg attcagacag caaaaaccac gctgggctcc aaagtggtca 60

[?] 120
tggagcggag agacgttgac tttgagctta tcaaagtaga aggcaaagtg ggcggcaggc 180
tggaggacac taaactgatt aagggcgtga ttgtggacaa ggatttcagt cacccacaga 240
tgccaaaaaa agtggaagat gcgaagattg caattctcac atgtccattt gaaccaccca 300
aaccaaaaac aaagcataag ctggatgtga cctctgtcga agattataaa gcccttcaga 360
aatacgaaaa ggagaaattt gaagagatga ttcaacaaat taaagagact ggtgctaacc 420
tacaatttgt cagtggggct ttgatgatga agcaaatcac ttacttcttc agaacacttg 480
ccttgcggtt ccttggtagg aggacctgaa attgagctga ttgccatcgc aacaggangg 540
cggatcgccc cagttctcaa gctnacagcc gagaan 576

<210> 741
<211> 579
<212> DNA
<213> Homo sapiens

<220>
<221> misc_feature
<222> (1)...(579)
<223> n = A,T,C or G

<400> 741
accttatctg aaactcttgc acttccccaa ccagggcaga aatgaggtgg gagaagtttg 60
actaaaatga gggatggggg aaagtaaaag atgttttttt tttttgaga ctcgctttgt 120
cacccaggct ggagtgcaat ggcacaatct caactcaccg caacctccgc ctcccgggtt 180
caagcgattc tcctgcctca gcctcccgag tagttgggat tacaggcgcc tgcctccatg 240
cctggctaat tttgtatttt tagtagagac agggtttctt catgttggtc aggctggtct 300
caaactccta acctcgtgat ccgcctgcct cgacctccca aagtgctggg attacaggca 360
tgagccacca tgcccagcca aagatcattt ttttatatag acttcaccct ttgtaaatac 420
tgtactgggg gagtatagag tagaaaaaaa gtttagttaa aacatttgtt tacaaattaa 480
cctttaaaaa tntaattact gctaaaaata gaaggctgtt ncccttaagg aaaattagng 540
ccatttgga aatganactt gggccataaa tncaggtgg 579

<210> 742
<211> 578
<212> DNA
<213> Homo sapiens

<220>
<221> misc_feature
<222> (1)...(578)
<223> n = A,T,C or G

<400> 742
ggtactttgg gatgctttac taggtgtttt ccattagaat tagaccttga ttttaaatcc 60
aagcaagctt gaagcccctt ggcttacagc atttgcctgc tgaatactaa acactcacat 120
ggcaagagtt gctctggaga ggtagggcca gaggaatgct gctgcactgc caactcaggc 180
acatgcttag ctgtaaaggg aagcgaggtg aagtcgtcct gcagcgtatt agagtaaaag 240
tctacccctc tgaagcacta ttaagcgctt aaccgtatat ttaaatacta ccatgtgcta 300
tctactgagg aagattcatg ttcaattatt tggaaataat gcaagcatcc actaagggcc 360
tttaagcttt ctttgattat aattaaggtt cattttaagt tntttttttt ctttcaacca 420
gtgtgccatc tccaatattt ctatagtata ccaaccaccc caggaatgca ctttaacaat 480
atcagggatt tatataacca aatagtttca aatccaacaa aattcccttt atgaactttc 540
gctttttaag actactgatg ggtacctgcc gggcggcc 578

<210> 743
<211> 592
<212> DNA
<213> Homo sapiens

<220>
<221> misc_feature
<222> (1)...(592)
<223> n = A,T,C or G

<400> 743
ggtctttaga aagttccatg attctgcata tactgtttga actgaatcat gatgtcttta 60
gaaagtatat gcagaatcag aatgttccgg gaaatattga gttaactgtg aatatcctga 120
caatgggcta ttggccgaca tatgtgccta tggaagttca tttaccacca gagatggtaa 180
aacttcagga gattttcaag acattttacc taggcaaaca tagtggcagg aaacttcagt 240
ggcagtcaac cctaggacac tgtgtgttaa agcagaattt aaagagggta aaaaggaact 300
ccaggtctct ctttttcaaa cactggtgct gctaatgttt aatgagggag aggagttcag 360
tttagaagag atcaagcagg caactggaat agaaggatgg agagttaagg agaacactgc 420
agtcattagc ctggtggcaa aagctagagt tctggcgaaa aaatnccaan ggccaaagac 480
ctttgaanat ggtgacaagt tcanttngta atngatgatt caaaccttaa actttcagga 540
tnaaggatca atcaaatnca aaaaaaaaaa nnnaaaaaaa agcttgttcc ga 592

<210> 744
<211> 578
<212> DNA
<213> Homo sapiens

<220>
<221> misc_feature
<222> (1)...(578)
<223> n = A,T,C or G

<400> 744
ggtaccaaac atagccctta ggcctgggct aggctctcaa aggtctttcc cagaaatgga 60
ggcagcagta gcttcaaaca ggcacaaaaa cagccaggag gaggcagcat ccactccatg 120
aaggcctaag acaatgaaag gaagccagag caacagacca ccttgggatc cggggagaag 180
ggtaaatggg caaaagggtt gtatttcctg atgctctcag aacatcagac cacaccatgt 240
gaatttaagc aggactattt taagtgggga aacaatacta gaagcatttg gtgtattttc 300
ctggcactca cctcctaggt aagcaggaga gcgggacact caggagttgt gactaaactc 360
acacttaagc tgcctgtcca gaccgtcccc ttggctgaac acaacactga aattgtggca 420
gtgtctgttg cnccagtgga cctncactta ctaatgagta tgtaaaacag angagccaca 480
gtgaggcntt tcacaaaacc canggctctt gggggaaaaa cgggtttcca ccttctgnct 540
tttggtgctg gaaagtncct gaggganaag aagtttgn 578

<210> 745
<211> 581
<212> DNA
<213> Homo sapiens

<220>
<221> misc_feature
<222> (1)...(581)

<223> n = A,T,C or G

<400> 745
   acagatcagg caactgtgga aaatctaaac gaactgcgcc aagatctgtc aaaattccga 60
   aatgaaataa gggatttacc tggctttcgg acttctaaat atgctatgtt ttatccaaga 120
   aattaaccat tttctaaatc atggagcgaa taattttcaa taacagatcc aaaagactat 180
   attgcataac ttgcaatgaa attaatgaga tatatattga aataaagaat tatgtaaaag 240
   ccattcttta aaatatttat agcataaata tatgttatgt aaagtgtgta tatagaatta 300
   gtttttttaa ccttctgtta gtggcttttt gcagaagcaa aacagattaa gtagatagat 360
   tttgttagca tgctgcttgg ttttcttact tagtgcttta aaatgttttt ttttatgttt 420
   aagaaggggc agttataaaa tggacacatt gcccaaaaag gttttggaaa antggaagac 480
   ccagcaaatg gtanggcttg acctccttca caaggataca cttggaaata tagaaagtta 540
   tgtttaaata tctctggttt aggagttcac atatagttaa g 581

<210> 746
<211> 506
<212> DNA
<213> Homo sapiens

<220>
<221> misc_feature
<222> (1)...(506)
<223> n = A,T,C or G

<400> 746
ggtacaagct tttttttttt tttttttttt tttttttttt taggtagtgg gtgttgagct 60
tgaacgcttt cttaattggt ggctgntttt aggcctacta tgggtgttaa attttttact 120
ctctctacaa ggntttttcc tantgtccaa agagctgttc ctntttggac taacagttaa 180
atttacaagg ggatttaaag ggttctgtgg gcaaatttaa agttgaacta agattctatc 240
ttggacaacc agctntcacc aggctcggta ggtttgtcgc ctctacctat aaatcttccc 300
actattttgc tacatanacg ggtgtgctct tttanctgtt cttaggtanc tcgtctggtt 360
tcgggggtct tanctttggc tctccttgca aagttatttc tagttaattc attatgcana 420
aggnataggg gttaagtcct tgctatatta tgcttgggta taattttcat ctttnccttg 480
cggnacctgc ccggccggcc gtttna 506

<210> 747
<211> 454
<212> DNA
<213> Homo sapiens

<400> 747
ggtactttgg cttcaatgat tggcaacttc tacaggggcc agtcttttga actggacaac 60
cttacaagta tatgagtatt atttataggt agttgtttac atatgagtcg ggaccaaaga 120
gaactggatc cacgtgaagt cctgtgtgtg gctggtccct acctgggcag tctcatttgc 180
acccatagcc cccatctatg gacaggctgg gacagaggca gatgggttag atcacacata 240
acaatagggt ctatgtcata tcccaagtga acttgagccc tgtttgggct caggagatag 300
aagacaaaat ctgtctccca cgtctgccat ggcatcaagg gggaagagta gatggtgctt 360
gagaatggtg tgaaatggtt gccatctcag gagtagatgg cccggctcac ttctggtatc 420
tgtcaccctg agcccatgag ctgcctttta gggt 454

<210> 748
<211> 569
<212> DNA

<213> Homo sapiens

<220>
<221> misc_feature
<222> (1)...(569)
<223> n = A,T,C or G

<400> 748
ggtaccagct ggcacaggag cagggggcat ggcacctctg ttgtttatgc ccatagcacc 60
tcccatagcc atctgaccca tccgaatctc ctgctctctc gcatcaggga aggttccctt 120
gaatccttcc tgctgtcgcc gcatcatttc ttcttgctgc cgccgcatct cttcttcacg 180
gcgcctgcgc tcttcctcct gcctgagctc cagttgcttt cgtttttgca cctcttggtt 240
gtgcagctct tccatcctcc gaagttcttc ttggcgcctc atcaaatcct gtctcattag 300
catgacctgg tgctcatggc gtgcagcttc catctccatc tccagcttct cacgagcctc 360
cttgatgttg cggtccactt ggtcctgctg ctgcttctcc atctcaatga gtgccttnca 420
gcgcatggca tattcatact caaaggaacc aggctgtgca aatctgggtg gctgctctcg 480
ttccttgtga aatgctggtt ttataaccag cttcnttgga agccctcttc atcaatctaa 540
cctggtccat gggctccaca gtcacaagg 569

<210> 749
<211> 428
<212> DNA
<213> Homo sapiens

<400> 749
acatggatat tcccaaacca ttccattaga aaactgccct ccctgcacac acaacaaaaa 60
cagcgctatt tcctacacct attggactga aagtgcttgg aaatggaatg gttttagaat 120
atgaagaaga acacaaacca agtagctgtg ggttgaacct ggacgtgagc tggctgcagg 180
gccgttgggt agaaaaccag catctcataa acaggtcact ccactggatg gtttgtcact 240
ggatggtttg ttggggtggt ggtcacaggc gcaaaggaca tgcacacggc cacgctacgc 300
tactgtaacc aagaggtgac ttcagccatg aataaggtga agaggttaca catctaccta 360
cggaatataa taacatacaa tgacttataa agtgactaca tgcatatgag caagcaaagt 420
acctcggc 428

<210> 750
<211> 569
<212> DNA
<213> Homo sapiens

<220>
<221> misc_feature
<222> (1)...(569)
<223> n = A,T,C or G

<400> 750
acctgccaga attagcaaga gctttcttta agaagacatt tgtcaaactc aacaaattga 60
aggttaacac cttaagagtt gtagttactg accagaaata tggacagact tcttagactt 120
ggaggaggta tgcctggact gggccagggg ccacctacag atgctcctgc agtggacaca 180
gcagaacaag tctatatctc ttccctggca ctgttaaaaa tgttaaaaca tggccgtgct 240
ggagttccaa tggaagttat gggtttgatg cttggagaat ttgttgatga ttataccgtc 300
agagtgattg atgtgtttgc tatgccacag tcaggaacag gtgtcagtgt ggaggcagtt 360
gatccagtgt tccaagctaa aatgttggat atgttgaagc agacaggaag gccggagatg 420
gttgttggtt gggtatcaca gtcaccctgg ctttggttgn tggctttctg gtgtggatat 480

caacactcag cagagctttg aagccttgtc gganagaact tgtggcaagt ggttgtggat 540
cccattcaga gtgtaaaagg aaaggttgt 569

<210> 751
<211> 568
<212> DNA
<213> Homo sapiens

<220>
<221> misc_feature
<222> (1)...(568)
<223> n = A,T,C or G

<400> 751
acctgaagct caggaggaga tgaaagaagt agccaaacac ccaaagaatc ctgaggttgg 60
cttgaagcct gtgtggtata gtcccaaagt tttcattgaa ggtgctgatg cagagacttt 120
ttcggagggt gagatggtta catttataaa ttggggcaac ctcaacatta caaaaataca 180
caaaaatgca gatggaaaaa tcatatctct tgatgcaaag ttgaatttgg aaaacaaaga 240
ctacaagaaa accactaagg tcacttggct tgcagagact acacatgctc ttcctattcc 300
agtaatctgt gtcacttatg agcacttgat cacaaagcca gtgctaggaa aagacgagga 360
ctttaagcag tatgtcaaca agaacagtna gcatgaagag ctaatgctag gggatccctg 420
ccttaaggat tttgaaaaaa ggagatatta tacaacttca gagaagagga ttttcatatg 480
tgatcaacct tatgaacctg taacccatgt agttgcaagg aancccgtgt gtttgatata 540
cattcctgat ggcacacaan gaaatgcc 568

<210> 752
<211> 312
<212> DNA
<213> Homo sapiens

<400> 752
accgccaggg atgtcccttc cagccctggg atggactaga ggagcacagc caagccctga 60
gtgggaggct gcgggccatt ctccagaatc agggaaactg aaggatgggc ctcagtctct 120
aaggaaggca gagacctggg ttgagcagca gaataaaaga tcttcttcca agaaatgcaa 180
acagaccgtt caccaccatc tccagctgct cacagacacc agcaaagcaa tgtgctcctg 240
atcaagtaga ttttttaaaa atcagagtca attaatttta attgaaaatt tctcttatgt 300
tccaagtgta cc 312

<210> 753
<211> 334
<212> DNA
<213> Homo sapiens

<400> 753
ggtacaagcg tctgcagcag actgtggcgg gcgaaggagc aggattccag ggcgctgttg 60
ggcttggtca cgaacgccag cagcaggggt gcaagggcct tggggaaata gtcctgctgc 120
accatgtggt tcagcgccat cagggggccg tacagttttt tcccacggga caaaaaatgc 180
ctaaggaagg gagaacataa taaaggggtt tctttctctc cctctttctt tcacattaag 240
acctacactt aaatattttc catagaaaac catcttccta attgtctttt gaatgaaatt 300
ctgacttggt gccacaagga ctaatacccg ccga 334

<210> 754
<211> 533

<212> DNA
<213> Homo sapiens

<220>
<221> misc_feature
<222> (1)...(533)
<223> n = A,T,C or G

<400> 754
ggtcgccgcc actgtccggc cacagcctaa cgctcttcgc tgtcgtttgc ggtctcgcgc 60
agggcggccc cggttctggt gtttggcgtc ggaattaaac aaccaccatg tcgagcaaaa 120
aggcaaagac caagaccacc aagaagcgcc ctcagcgtgc aacatccaat gtgtttgcca 180
tgtttgacca gtcacagatt caggagttca aagaggcctt caacatgatt gatcagaaca 240
gggatggctt catcgacaag gaagatttgc atgatatgct tgcttctcta gggaagaatc 300
ccactgatgc ataccttgat gccatgatga atgaggcccc agggcccatc aatttcacca 360
tgttcctgac catgtttggt gagaagttaa atggcacaga tcctgaagat gtatcagaaa 420
cgcctttgct tgctttgatg aagaagnaca ggcaccattc aggaagatac ctaagagact 480
gttgccacca tggggggatc ggtttacana ataagaagtg gatgantgtc ctg 533

<210> 755
<211> 571
<212> DNA
<213> Homo sapiens

<220>
<221> misc_feature
<222> (1)...(571)
<223> n = A,T,C or G

<400> 755
ggtaccttat tagaaagcga cggcaaacta tgtgccagca gccgcggtaa tacataggtc 60
gcaagcgtta tccggaatta ttgggcgtaa agcgtccgta ggttttttgc taagtctgga 120
gttaaatgct gaagctcaac ttcagtccgc tttggatact ggcaaaatag aattataaag 180
aggttagcgg aattcctagt gaagcggtgg aatgcgtaga tattaggaag aacaccaata 240
ggcgaaggca gctaactggt tatatattga cactaaggga cgaaagtgtg gggagcaaac 300
aggattagat accctggtag tccacgccgt aaacgatgat cattagttgg tggaataatt 360
tcactaacgc agctaacgcg ttaaatgatc cgcctgagta gtatgctcgc angagtgaaa 420
tttaaaggaa ttgacgggaa cccgnacaag cggtggagca tgtggtttaa tttngattct 480
acgcgtagaa ccttacccac tcttgacatc ttctgcaagc tatagagata tagtggaggt 540
tacagaatga cagatggtgc atggttgtcc g 571

<210> 756
<211> 570
<212> DNA
<213> Homo sapiens

<220>
<221> misc_feature
<222> (1)...(570)
<223> n = A,T,C or G

<400> 756
ggtccactgg aaaggcaaca tgaccaggct gccccgcctc ctggttctgc ccaagttctc 60

cctggagact gaagtcgacc tcaggaagcc cctagagaac ctgggaatga ccgacatgtt 120
cagacagttt caggctgact tcacgagtct ttcagaccaa gagcctctcc acgtcgcgca 180
ggcgctgcag aaagtgaaga tcgaggtgaa cgagagtggc acggtggcct cctcatccac 240
agctgtcata gtctcagccc gcatggcccc cgaggagatc atcatggaca gacccttcct 300
ctttgtggtc cggcacaacc ccacaggaac agtccttttc atgggccaag tgatggaacc 360
ctgaccctgg ggaaagacgc cttcatctgg gacaaaactg gagatgcatc gggaaagaag 420
aaactccgaa gaaaagaatt ttagtgttaa tgactctttc tgaaggaaga gaaacatttg 480
cctttggtta aaagatggta aaccagatct ggcttccaag acctngcctt ttcttggagg 540
acctttaggt caaactccct agtttcacct 570

<210> 757
<211> 578
<212> DNA
<213> Homo sapiens

<220>
<221> misc_feature
<222> (1)...(578)
<223> n = A,T,C or G

<400> 757
acaagctttt tttttttttt tttttttttt tttttttggg gagtaagaaa aggtggggat 60
taagaanacg tttctggagg cttagggacc aaggctggtc tctttccccc ctcccaaccc 120
ccttgatccc tttctctgat caggggaaag gagctgagtg agggaggtag agttggaaag 180
ggaaggattc cacttgacag antggcacan actcctccag agtanagctt ggagggagat 240
tgaaagtgga gataatactg ctgacacctc ccttgaagct nagatgggaa atggacatac 300
ttagaaattt agtgacttta atagcctgga tttccctntn caaaactttt agaatggaaa 360
atcccatccc cttccttata tagtgacttc tacccactac cttctaccat tttctacttt 420
gggcttatga tgatggccat tatctacatg ngtttttagn accctggttt ggttctaaan 480
ggggatcttg gaacccnagn ttnttgggag atttttaaga aggaagtttt aactgaacaa 540
atggaatggg cnccagaaag aaatccaggg tnncccng 578

<210> 758
<211> 567
<212> DNA
<213> Homo sapiens

<220>
<221> misc_feature
<222> (1)...(567)
<223> n = A,T,C or G

<400> 758
ggtacgagat tgaaaggttg agggttctac tgcaggaaga aggcacccgg aagagagaat 60
atgaaaatga gctggcaaag gtaagaaacc actataatga ggagatgagt aatttaagga 120
acaagtatga aacagagatt aacattacga agaccaccat caaggagata tccatgcaaa 180
aagaggatga ttccaaaaat cttagaaacc agcttgatag actttcaagg gaaaatcgag 240
atctgaagga tgaaattgtc aggctcaatg acagcatctt gcaggccact gagcagcgaa 300
ggcgagctga agaaaacgcc cttcagcaaa aggcctgtgg ctctgagata atgcagaaga 360
agcagcatct ggagatagaa ctgaagcagg tcatgcagna gcgctctgag gacaatgccc 420
ggcacaagca gtccctggag gaggctgcca agaccattca ggacaaaaat aaggagatcg 480
agagactcaa agctgagttc aggaggaggc caaccccgtt gggaatatga aaatgactga 540
taaggtagaa acattatgat gaggagg 567

<210> 759
<211> 266
<212> DNA
<213> Homo sapiens

<400> 759
ggtcaccgac ctctctcccc agctgtattt ccaaaatgtc gctttctaac aagctgacgc 60
tggacaagct ggacgttaaa gggaagcggg tcgttatgag agtcgacttc aatgttccta 120
tgaagaacaa ccagataaca aacaaccaga ggattaaggc tgctgtccca agcatcaaat 180
tctgcttgga caatggagcc aagtcggtag tccttatgag ccacctaggc cggcctgatg 240
gtgtgcccat gcctgacaag tacctg 266

<210> 760
<211> 381
<212> DNA
<213> Homo sapiens

<400> 760
ggtacactag aaagtctttt acaaaataat catcttagat caacagaaga ccaatcttca 60
atgtcgtcct gcaagatggg ttactttaac atctcctcct gttttctcca atgttctcct 120
ttagtatggc tggtaattgt tttggtgatt gccaccccct cgagatgcct tgccataagt 180
gctctgttgg ccactgtagt ctgcatatcc ctgtccatat ccatagttcc catagttata 240
cccagtataa tcatatccgc catagccact atagttttga tcaccaccat aggcactatt 300
gtaatttcca tatccttgat cataatagtt attaaatcct tggttccagt tttggccctg 360
acctcggcca cgacccctcg t 381

<210> 761
<211> 401
<212> DNA
<213> Homo sapiens

<400> 761
actcagctcc aattatctaa tattcttgaa aggatgctga tattgtttgg ttgtgtcccc 60
ccacaaatct caacttgaat tgtatctccc agaattccca cgtgttgtgg gacagaccca 120
gggggaggta attgaatcat gggggccagt ctttcccgtg ctattctcgt gacagtgaat 180
aagtctcatg agatctgatc agtttatcag gggtttctgc ttttgcttct tcctcatttt 240
ttcttgccac aatgtaagaa gtgtcttttg cctcccacca tgattctgag gcctccccag 300
ccatgtggaa ctttaagtcc aattaaacca ctttttcttc ccagtctcgg gtatgtcttt 360
atcagcagcg tgaaaacgga ctaatacagt aaattggtac c 401

<210> 762
<211> 610
<212> DNA
<213> Homo sapiens

<220>
<221> misc_feature
<222> (1)...(610)
<223> n = A,T,C or G

<400> 762
acgcttgttg atttcatcct catacttgtt cttgaagtct tccaccaggt cctgcatgtt 60

tcttagctct gagtccaggc ggccccgttc ccccacgatg ctgtccagct gcctcctgag 120
gttgttgatg tacagtaaaa acacatctaa catctttgaa gaccaaattt cctgctgaac 180
agtattacag atttcatgag cactggaggt ttgtgttgca gcgcttggtc ttcttggcag 240
catttgttgt gtatttggaa acagaaacac tagtgactcg agaagcagtt acagaaattc 300
ttggcattga gccagatcgg gagaaaggat ttcatctgga tgtagaagat tatctctcag 360
gagttctaat tcttgccagt gaactgtcga ggctgtctgt caacagcgtg actgctggag 420
actactcccg acccctccac atctccacct tcatcaatga gctggattcc ggttttcgcc 480
ttctcaacct gaaaaatgac tccctgagga agcgctacga cggattgaaa tatgacgtga 540
agaaagtaga aggaagtggt ctatgatctc tncatccggg ctttaataag gagacggcag 600
cagcttgtgn 610

<210> 763
<211> 578
<212> DNA
<213> Homo sapiens

<220>
<221> misc_feature
<222> (1)...(578)
<223> n = A,T,C or G

<400> 763
cgaggtaccc tgaagaactt ccctaatgcc atcgagcaca ccctgcagtg ggctcgggat 60
gagtttgaag gcctcttcaa gcagccagca gaaaatgtca accagtacgg atgctacttg 120
tccaatgatg gtaaaagggt agcttactgg ttgtcctccg attcaggtta gaatgaggag 180
gtctgcggct aggagtcaat aaagtgattg gcttagtggg cgaaatatta tgctttgttg 240
tttggatata tggaggatgg ggattattgc taggatgagg atggatagta atagggcaag 300
gacgcctcct agtttgttag ggacggatcg gagaattgtg taggcgaata ggaaatatca 360
ttcgggcttg atgtggggag gggtgtttaa ggggttggct agggtataat tgtctgggtc 420
gcctangagg tctggtgaga atagtgttaa tgtcattaag gagagaagga agaagaagta 480
agccnagggc gtctttgatt gtgtantaag ggtggaaggt gattttatcg gaatgggaag 540
tgattcctaa ggggttggtt gatcccgttc tgcaanan 578

<210> 764
<211> 500
<212> DNA
<213> Homo sapiens

<400> 764
actatataac agttggcaca acccacccca caacagaaga gaacacattt ttctcaagca 60
tatgtggaat agtttccagg agaaaccatg tgttaggcca caaaacaaat cttaatgaaa 120
tgtaaaagac tgaaacacaa agtacagcat cactcggatt ctgtgtccaa tggccttagc 180
aggaagattg cttcggaatt tggcacgaac catgccactg tttccatggg cccgagttac 240
ttttccccag atgactctgg ttttgtttgg tttgccgcca ggagtgactg tgttgttctt 300
tgctttatat acataagcgc atctcttgcc caaatagaat tctgtttcat cttcgggccg 360
taaacacctt caattttaag aagagctgtg tgctcccttt ggttccggag accccgctta 420
tagccagcaa aaatggcctt ggaccacaag cctttcagac atagttcctt tagaagtccg 480
acttcggccg gcgaccacgc 500

<210> 765
<211> 578
<212> DNA
<213> Homo sapiens

<220>
<221> misc_feature
<222> (1)...(578)
<223> n = A,T,C or G

<400> 765
ttccagagca tattgatgag agaaggatct gcaatgctgt ttctccagac aaggatgttg 60
atggctttca tgtaattaat gtaggacgaa tgtgtttgga tcagtattcc atgttaccgg 120
ctactccatg gggtgtgtgg gaaataatca agcgaactgg cattccaacc ctagggaaga 180
atgtggttgt ggctggaagg tcaaaaaacg ttggaatgcc cattgcaatg ttactgcaca 240
cagatggggc gcatgaacgt cccggaggtg atgccactgt tacaatatct catcgatata 300
ctcccaaaga gcagttgaag aaacatacaa ttcttgcaga tattgtaata tctgctgcag 360
gtattccaaa tctgatcaca gcagatatga tcaaggaagg agcacagtca ttgatgtggg 420
gaataaatag agttcacgat cctgtaactg tcaaacccaa gttggttgga gatgtgggat 480
tttgaaggag tcagacaaaa agctgggtat atcactccag ttcctgggan gtgtttggcc 540
ccatgacagt ggcaatgcta atgaagaata ccattntt 578

<210> 766
<211> 569
<212> DNA
<213> Homo sapiens

<220>
<221> misc_feature
<222> (1)...(569)
<223> n = A,T,C or G

<400> 766
actgtattta tattgtttat attattttag taatgtaatg ttttgcttcc aaagattgcc 60
ttgcctttac attttgtgca aaaatagcag ctatacatta atgacataat aagtatgtct 120
agtattattt aagtgcctat tcatattttc tcatcaaagc tttttatgaa tgattataat 180
gcattttcta taaaatatta ttgctttcac tgtataccag tgattcaaac tttattgtct 240
tcaacagcaa tgacatgaaa tcactctagt tgcccatcag tggtggattg gataaagaat 300
atgtggtact atgtgactat cattgatgcc ccaggacaca gagactttat caaaaacatg 360
attacagggg acatctcaag ctgactgtgc tgtcctgatt gttgctgctg gtgttggtga 420
atttgaagct ggtatctcca agaatgggca gacccgaaag catgcccttc tggcttacac 480
ctgggtgtga aacaacctaa tggccggggt taccaaaatg ggattccact ggaccaccta 540
cagccagaag agatntgaag gaaattnnt 569

<210> 767
<211> 580
<212> DNA
<213> Homo sapiens

<220>
<221> misc_feature
<222> (1)...(580)
<223> n = A,T,C or G

<400> 767
acgaagctac ccagggagat ctgaatgatg ctaaaaataa acagaaattt gttttaaagg 60
tccaaaagcc tgccaacccc tgggaattct acattgggac ccagttgatg gaaagactaa 120

agccatctat gcagcacatg tttatgaagt tctattctgc ccacttattc cagaatggca 180
gtgtattagt aggagagctc tacagctatg gaacattatt aaatgccatt aacctctata 240
aaaatacccc tgaaaaagtg atgcctcaag gtcttgtcat ctcttttgct atgagaatgc 300
tttacatgat tgagcaagtg catgactgtg aaatcattca tggagacatt aaaccagaca 360
atttcatact tggaaacgga tttttggaac aggatgatga agatgattta tctgctggct 420
tggcactgat tgacctgggt canagtatag atatgaaact ttttccaaaa ggaactatat 480
tcacagcaaa gtgtgaaaca tctgggnttt caatggtgtt gaaaatgctc ancaacaaac 540
catgggaact accagaatcg attactttgg ggttgctgca 580

<210> 768
<211> 355
<212> DNA
<213> Homo sapiens

<400> 768
ggcaggtacc ctatggccta tgttgactat aagactgtgc tgcagattga tgataatgtg 60
acgtcagccg tagaaggcat caacagaatg accagagctc tcatggactc gcttgggcct 120
gagtggcgcc tgaagctgcc ctcaatcccc ttggtgcctg tttcagctca gaagaggtgg 180
aattccttgc cttcggagaa ccacaaagag atggctaaaa gcaaatccaa agaaaccaca 240
gctacaaaga acagagtgcc ttctgctggg gatgtggaga aagccagagt tctgaaggaa 300
gaaggcaatg agcttgtaaa gaagggaaac cataagaaag ctattgagaa gtacc 355

<210> 769
<211> 611
<212> DNA
<213> Homo sapiens

<220>
<221> misc_feature
<222> (1)...(611)
<223> n = A,T,C or G

<400> 769
cgaggtacca cgatcctgat gatgaaccag tggccgatcc ttatgatcag tcctttgaaa 60
gcagggacct ccttatagat gagtggaaaa gcctgaccta tgatgaagtc atcagctttg 120
tgccaccacc ccttgaccaa gaagagatgg agtcctgagc acctggtttc tgttctgttg 180
atcccacttc actgtgaggg gaaggccttt tcacgggaac tctccaaata ttattcaagt 240
gcctcttgtt gcagagattt cctccatggt ggaagggggt gtgccgtgcg tgtgcgtgcc 300
gtgttagtgt gtgtgcatgt gtgtgtctgt ctttgtggga gggtaagaca atatgaacaa 360
actatgatca cagtgacttt acaggaggtt gtggatgctc cagggcancc ttcacccttg 420
ctcttctttc tgagaagttg gcttaaggca gaccaaganc tgctggccct tttaaggaat 480
atgttcaatg ccaaaggtaa aaaaattntg aaattggtcc ccaaatnccc gggcattgcc 540
tttcgccact ttnggcttct tcctggngan ccccaccttt gaccggtggg ggccgtanac 600
nttgacaacn n 611

<210> 770
<211> 508
<212> DNA
<213> Homo sapiens

<220>
<221> misc_feature
<222> (1)...(508)

<223> n = A,T,C or G

<400> 770
ggacaaaacc agctgaagat gaaagtgtgg agacccaggt gaatgacagc atcagtgctg 60
agacagcaga gcagatggat gtagatcagc aggagcacag tgctgaagag ggttctgttt 120
gtgatccccc acccgctacc aaagctgact ctgtggacgt tgaagtgagg gtgccagaaa 180
accatgcatc taaagttgaa ggtgataata ccaaagaaag agacttggat agagccagtg 240
agaaggtgga acctagagat gaagatttgg tggtagctca gcaaataaat gcccaaaggc 300
ccgagcccca gtcagacaat gattccagtg ccacgtgcag cgctgatgag gatgtggatg 360
gagagccaga gaggcagaga atgtttccta tggactcaaa gcctttactg ntaaacccca 420
ctggatctat actcgnctca tcttccggtn aaacccaatt cnctgggatc tggcccaant 480
tnancattna ncttgggnta ttncnncc 508

<210> 771
<211> 587
<212> DNA
<213> Homo sapiens

<220>
<221> misc_feature
<222> (1)...(587)
<223> n = A,T,C or G

<400> 771
acttgttttg ggaatatatg agagaagaaa ctgctgagca ggtcagtaaa gaacagtcca 60
tttcagctgc aggacagttc tctttcccgg gacaagccta catagcctcc aagggagcca 120
aactatccct tccatgcaac aagacacctt gcatggatac tctagccatg acttgctttt 180
ggacaaaaat caactgctaa cgtttttcat ctctaatatc attaacacca tggagaaaaa 240
agaaaaaaat tcaaccctag aaaacttgac aacgagaata agaaaatcca caaggaaagg 300
tcatgctaaa actgatttga cagttgttcc atcaccgcct accacatggg cttgagactg 360
gtgacttcat ggatgcatcc cttcgatgcc ctgccaaatg tcagcttcaa gtctgtcagt 420
gaccccagtg tgatgctgcc tgccttctat tcaccaactn ctattcaaga gatccaaggg 480
ggccttgggc cgtggtaagc acanggacac ncaggtgcca agaagcccca gnaacccttt 540
tagaaaactt tgncctggga tttgggcccc ggnaaccaac cngtggn 587

<210> 772
<211> 577
<212> DNA
<213> Homo sapiens

<220>
<221> misc_feature
<222> (1)...(577)
<223> n = A,T,C or G

<400> 772
ggtacactgc aggagagtgc ctggcaaaaa gatcaaatgg ggctgggact tctcattggc 60
caacctgcct ttccccagaa ggagtgattt ttctatcggc acaaaagcac tatatggact 120
ggtaatggtt acaggttcag agattaccca gtgaggcctt attcctccct tccccccaaa 180
actgacacct ttgttagcca cctccccacc cacatacatt tctgccagtg ttcacaatga 240
cactcagcgg ccatgtctgg acatgagtgc ccagggaata tgcccaagct atgccttgtc 300
ctcttgtcct gtttgcattt cactgggagc ttgcactatg cagctccagt ttcctgcagt 360
gatcagggtc ctgcaagcag tggggaaggg ggccaaggta ttggaggact ccctccagct 420

ttggaagcct catccgcgtg tgtgtgtgtg tatgtgtaga caagctcttn gctctgtcac 480
ccaagctgga attgcantgg tgcaatcatg gttcacttgc agtcttgacc ttttggctca 540
agtgatcctt ccacctnacc tcctgagtac tgggacc 577

<210> 773
<211> 580
<212> DNA
<213> Homo sapiens

<220>
<221> misc_feature
<222> (1)...(580)
<223> n = A,T,C or G

<400> 773
ggtaccacct cctgttccta caaaaccaaa acagattaat ttgccttatt ttggacaaac 60
taatcagcca ccttcagaca ttaagccaga cggaagttct cagcagttgt caacagttgt 120
tccgtccatg ggaactaaac caaaaccagc agggcagcag ccgagagtgc tgctatctcc 180
cagcatacct tcggttggcc aagaccagac cctttctcca ggttctaagc aagaaagtcc 240
acctgctgct gccgtccggc cctttactcc ccagccttcc aaagacacct tacttccacc 300
cttcagaaaa ccccagaccg tggcagcaag ttcaatatat tccatgtata cgcaacagca 360
ggcgccagga aaaaacttca gcaggctgtg cagagcgcgt tgaccaagac tcataccaga 420
gggccacact tttcaagtgt atatggtaag cctgtaattg ctgntgncca aaatcaacag 480
cagcacccag agacatttat tcaatagcca gggcaagcct ggcagtcaga acctgaacag 540
acctgttctt tagttcagga gaaccntgaa acnaaagaat 580

<210> 774
<211> 680
<212> DNA
<213> Homo sapiens

<220>
<221> misc_feature
<222> (1)...(680)
<223> n = A,T,C or G

<400> 774
ggtacctggc catgggcttc cctcccacac ctgccaggac acagcctgca ggtcaggggg 60
ctaaactggg gagttttctc caaagttggg aaaggatggg aagagtaggt gggaatgggg 120
aagttacaca gctacagcag tcaggcctgt ttagtaagaa gaatcacatt taatgagttt 180
ctttcttgca gtttcagatg ctcaagtaca agtaagttat atgacaacga taacacacag 240
gaggaaagcc acggaagcac actgttgtga agttctcatg ctctacgtga agtgttatct 300
tttttttcta agtgacagca agtttattaa gaaagtaaag gaataaaagg aatggctatt 360
tcattggcag agcaccaata aaatcatctg aaggnagatt gtgatgagtt aaangcgtat 420
atgataaacc tgaagaccaa cnagaaanta gcccacngag atntagtgga ttaagttaac 480
caagggaatt aacttgaatc attaaaaatt cttaatctgg gggaaccttt naanaanggg 540
agcttacccc ttggggcaat ttnaaaccna aagccaggtt gattgaattt aagcttacct 600
tttttcaata atccctttta aannaanggt ttnaaccttt cncttaaang gcnnnanttt 660
tcnaattgga ntttaagccg 680

<210> 775
<211> 658
<212> DNA

<213> Homo sapiens

<220>
<221> misc_feature
<222> (1)...(658)
<223> n = A,T,C or G

<400> 775
ggtacctgtg ccagatgaaa ggtttgactt tctttgtcaa taccacaaac cagcaagcaa 60
aattcctgcc tttctaaatg tggtggatat tgctggcctt gtgaaaggag ctcacaatgg 120
gcagggcctg gggaatgctt ttttatctca tattagtgcc tgtgatggca tctttcatct 180
aacacgtgct tttgaagatg atgatatcac gcacgttgaa ggaagtgtag atcctattcg 240
agatatagaa ataatacatg aagagcttca gcttaaagat gaggaaatga ttgggcccat 300
tatagataaa ctagaaaagg tggctgtgag aggaggagat aaaaaactaa aacctgaata 360
tgatataatg tgcaaagtaa aatcctgggt tatagatcaa aaagaaacct ggtcgcttct 420
atcatgattg gaatgaccaa gagattgaag tggtgaataa acccttaatt ttgactcnaa 480
anccatggnc tacttggtna acnttctgaa aaagcttcnt ttgaaggaaa ccaanggtga 540
taaaattaag aaggggtggc cagtttancc agggccttgg catcctttaa gggggcttgg 600
accttaagtt ccanaattga tcttanggna anccaagttt tggaaccacc tgncccaa 658

<210> 776
<211> 659
<212> DNA
<213> Homo sapiens

<220>
<221> misc_feature
<222> (1)...(659)
<223> n = A,T,C or G

<400> 776
ggtactttac ggcctgatct aattgaaagt gcatcccttg ttgcaagtgg caaagctgaa 60
ctcatcaaaa cccatcacaa tgacacagag ctcatcagaa ggttgagaga ggagggaaaa 120
gtaatagaac ctctgaaaga ttttcataaa gatgaagtga gaattttggg cagagaactt 180
ggacttccag aagagttagt ttccaggcat ccatttccag gtcctggcct ggcaatcaga 240
gtaatatgtg ctgaagaacc ttatatttgt aaggactttc ctgaaaccaa caatattttg 300
aaaatagtag ctgatttttc ttgcaagtgt taaaaagcca cataccctat tcagagagtc 360
aaagcctgca caacagaaga ggatcaggag aagctgatgc caaataccag tctgcattcc 420
tgaatgcctt cttgctgcca attaaaactt naggtgtnca nggtgaactg gnngtnctac 480
cgntnccngn ngnggaatnt caggnaaaga tgaaccctgc tgggnaatcn cttattttcn 540
ggntangnnt aaaccttnga tggggccaac cttaccnggt ggttattttt tggncccccn 600
ntaaagaacc tcntnaaang tnccccnttt ttganacggg ggnttaaacc tncccgggg 659

<210> 777
<211> 728
<212> DNA
<213> Homo sapiens

<220>
<221> misc_feature
<222> (1)...(728)
<223> n = A,T,C or G

<400> 777
acttcttgca tgttgtcaca tgttgctgtg agaatcaggt gctgcctata tggctccact 60
gggagagggc agatggaagc cgtcgcctca tctgtcgtgg aacgtgtgct gtgcacctcc 120
tccctttgct gatcttaatc tctgtccttt tactgtaata aactgtaact gtgagcctaa 180
cagctttcct gagtctagtg agtccttcta gcaaatgaaa ggagggtggt cttggagacc 240
tatgaacttg cacctgcccc cgtcgttttg agggtctggc acaggggagg gaagggctgg 300
gcctcttttg gaagggggtc ttcaatccat ttgggggtcg gggtcccaac ttcttggang 360
ggcccaacgt tccttgccca gcttccaagn ctcttcttcc cttcttaagt ccccgancct 420
tgcaaccttt gggcccctnt ggcttgtgga atcctgggaa aaaacttngt ctttttnntt 480
ancacttgaa tnngaanaac tggcccatta actnaagccc ttgcatnnct tngactnctt 540
nnatgggcaa ccttnaaggg attcccaagg gncccctggg tttanggaaa taatgggggg 600
aaaatttttt nggaanttna anaataancc ccccccaaaa ncggggganc cttngggccc 660
gnaacccccc ttaagggccn aaattccngn canatntggg ggggccggtn ctaaggggat 720
cccaaccc 728

<210> 778
<211> 603
<212> DNA
<213> Homo sapiens

<220>
<221> misc_feature
<222> (1)...(603)
<223> n = A,T,C or G

<400> 778
caggtacact gctgccactg ttgtgtcctc gctctgcttg ctgttgcctc acgccaggcc 60
ccgtcctgcc gtgacaccct tcatcctacc cttggaaccc caaggccaag ttggttcaaa 120
ctgttggaga acagagttgg cctgcatctg gaacacactt gtcctcagct taccatctcc 180
tcacacccca gagtggaaag gtgaacacct gcagctgagg cttggaaacg tttcttgtgt 240
tgccctgaaa aatctttgag acctcaggga ggctctgtct ctcttaaaag gtggagaaag 300
atgccattct ctccctaagg tctggtggag tctccccatc ttgcataccc ttctgcaagc 360
catctatctc tgctcactct ccaattgacc cgcctgggaa caaggatgaa aggaggaagt 420
tgggggcttg ggggaatcct gccagttggt gaancctgtg gcangaagga tatgtgacnt 480
agagatcctg atcttttntn ancctgctgt tggttggctt gnatatatgg atggtgactg 540
tttgnaaagn ggagtataag atgccntgct gatnggngta tgctatgctn ttangatgga 600
ctg 603

<210> 779
<211> 654
<212> DNA
<213> Homo sapiens

<220>
<221> misc_feature
<222> (1)...(654)
<223> n = A,T,C or G

<400> 779
cgaggttttt tttttttttt tttccagtta gtgatgtcgt atttcaaaat aggtcgaaac 60
ttcagagaaa tgaaaatcgg gatatcagtg aagttattgc tctcggtgtt cctaatcctc 120
ggacttccaa tgaagttcag tatgaccaaa ggctnttcaa ccaatccaag ggtatggaca 180
gtggatttgc aggtggagaa gatgaaattt ataatgttta tgatcaagcc tggagaggtg 240

gtaaagatat ggcccagagt atttataggc ccagtaaaaa tntggacaag gacatgtatg 300
gtgatgacct agaagccaga ataaagacca acagatttgt tcccgacaag gagttttctg 360
gttcaaaccg taaacngaga ggccgagaag gaccagtgca gtttgaggaa aatccttttg 420
gtttggacaa gtttttggaa aaaacccaac ngcatggngg ctntaaaaga cccttagata 480
ccacccgcnc aaggacnnag cctgaagcca gaaaaggngg aaggattggc caggttttcc 540
aagngaatga ctttanccta acctaangag ccagnttngg ggacccttnt aaagggccgg 600
taaaaccnat ttggggccca nnccnccttn ttttttctgg gaaanggggg gtta 654

<210> 780
<211> 570
<212> DNA
<213> Homo sapiens

<220>
<221> misc_feature
<222> (1)...(570)
<223> n = A,T,C or G

<400> 780
acagtgggca caaaacctgt gcagagtccg cagaagaggc caataaccaa gcgacccagg 60
atcagcattt caaccgactt agctacttta cacagtccca taaagcagcc accagtgaca 120
gccaacaggt tgacaatcag cattgaattg cgcctgccaa agcggttgac gaagagtccg 180
acggaaaagg agccgatcat accccngacg gaaaatatgg ccacagacaa ggaccagaga 240
gacgtgagca gcacctcaga gggtggggca tttcccttgc cgtcaaagtt ttattgataa 300
attcctttat gatcttctca ggagcattga tgaccccagt ggttgtaacc naattggaaa 360
gaaccgattg nagccactgg tgatggccaa tatcaaanct ggggtgacct tctggggccc 420
catcgctgga atctaattca agtctttaag aaagatctan gggtgatttc agaaacnagn 480
ttttnaggcc acaaaccttt aaanggcctt ttaacagcaa ggtttnttcc cgtcttagga 540
aggatncnaa nccnttggcc ggaaccncct 570

<210> 781
<211> 664
<212> DNA
<213> Homo sapiens

<220>
<221> misc_feature
<222> (1)...(664)
<223> n = A,T,C or G

<400> 781
acccaaagtt ctctggggag ggccagggaa gaggctgggt gtcaaaccaa acagattttt 60
atttgcagtc gtcactgggg ccgtttcttg ctgcttattt gtctgctagc ctgctcttcc 120
agctgcatgg ccaggcgcaa ggccttgatg acatctcgca gggctgagaa atgcttggct 180
tgctgggcca gagcagattc cgctttgttc acaaaggtct ccaggtcata gtctggctgc 240
tcggtcatct cagagagctc aagccaagtc tggtccttgc tgtatgatct ccttgagctc 300
ttccatagcc ttctcctcca gcttcctgat ctgaagtcat ggctttcgtt aaaactggac 360
atctgggaaa gacagtcctt ctctttcttg gataaattgg cctggaatca ncgccccggt 420
aaaacaagct ttcatctttc tggttccant ttnattaact ggttttcact nggnccactg 480
ngggggctta ncttcttgac ctggctggna aatttaaggn ggttnaagnt tnttncccgg 540
acctattncn tggnnaaaac cngggaatna tgcnagnctt aaaatttnc ccaangaagg 600
agtccttaan accnggntaa nttggnttta cggaacnggg tggnnacctt gttttnccag 660
gncc 664

<210> 782
<211> 669
<212> DNA
<213> Homo sapiens

<220>
<221> misc_feature
<222> (1)...(669)
<223> n = A,T,C or G

<400> 782
caggtacaag cttttttttt tttttttttt tttttggaat agaatacaac tttattttca 60
gtcatttcta tttccttggt tatgaacaaa ggtagcaaag tgcagttgta tcagcagtgc 120
caatagaaat tacagagttt ttcatatccc tttacagttt gccacaggta tcttaaaata 180
ttgnttacac tcatctctct tcagtttacc attgtttaat aggcctaccc tcgatctttt 240
tattcaatat gttaataaag aaacctatac acatagtatc accgttatca ttttaaaaat 300
attttgacac tgnatataaa tataactagc ttactttgga atcctaccta ttttaatggt 360
gnatgaaaat attattctga aattagccng gcntggnggt gcatgcctan aggcccagct 420
acttgggaag cttaaggggg aaggatccct gaacccaagg ganggccang nttcngggan 480
ctnggatgnn caatggcttc ancctnggna atngaatggg ancccttttt aaaggaaagg 540
aaanggaaat ttggattttg gnaacngann cctggnccaa aaaagggcaa aanccctgct 600
ggaanggccc tntggacctt aaatgccccn nccaaangng gnnattncca tttaannggn 660
cccncaggg 669

<210> 783
<211> 735
<212> DNA
<213> Homo sapiens

<220>
<221> misc_feature
<222> (1)...(735)
<223> n = A,T,C or G

<400> 783
acacagaagc agtgaaggac tgcacagaag ccctcaagct ggatggaaag aacgtgaagg 60
cattctacag acgggctcaa gcccacaaag cactcaagga ctataaatcc agctttgcag 120
acatcagcaa cctcctacag attgagccta ggaatggtcc tgcacagaag ttgcggcagg 180
aagtgaagca gaacctacac taaaaaccca acagggcaac tggaacccct gcctgacctt 240
acccagagaa gccatggccc acctgctctg tgcccgctcc tgaaacccag catgccccaa 300
gtgagctctg aagccccctc ctcaatccct tgatggcctc caccctgtaa gaagctttgc 360
tttggtcaaa ttaaacttaa gtgtaatcaa accccagacc atgggtggtt gcacccagaa 420
agggncccac tnagaaccta aacgttgaag ctgnaacttt ngcccctaat tcccnaagcc 480
caagttagct tgatcccncc accggaatcc ttatttagcc aaagccnttt ngggntttgg 540
ncctggnccc aaanggggct ttgaaaaact ggaaggcttg gcccnttgga agctttnccc 600
caaaancccc aaatttaatt ggggagntna ttttggaacn aaccttgggc tttttngggc 660
cccgggtttg gaaaggaagg ggggataaaa ccttaagggc cctggttcca aaannanccc 720
tttttnaacc ggggn 735

<210> 784
<211> 660
<212> DNA

<213> Homo sapiens

<220>
<221> misc_feature
<222> (1)...(660)
<223> n = A,T,C or G

<400> 784
cgaggtacac attgtattat atacaaacaa gcaacaacaa aaagtttcat catgtaaaca 60
aaagaatata aattatagac ataattggaa gtttcaaaca gtccttaaat cattgtgagc 120
ttctctaaaa ggcacaggtc ttggagtgtg ggcacagagc cattagtcag atgtctgggt 180
ggtctcccat aatagcaatg tatactctaa agtgggcttt ttgtgaactc tgtcagggtg 240
aatgagttag gcctcttaaa ggaatgaaat gctttcacat ttggggcaac aagtgaaaaa 300
tactgaaagg agggatacaa ctagggttag atttattggt gacagtgatt ttagaaatac 360
cactaaaaag gtggtaaaag atttctagat taaattctga ctactgnaaa tnagaaagga 420
tccttttgna nctctaccaa tggttngtga aaaattaaaa gggagaaagt gacccaggag 480
aaaccnaatt gggaagctan ggaggttcca gaaaatnccc agtcttacac gaaaaaacct 540
tganagggcc tttttaaggc caannttggg aaattacctt tgtaacttaa cttgaaaaan 600
acctgccggc ggccgttnaa aggncaattn accnctggng gccgtcttag ggnccncctc 660

<210> 785
<211> 254
<212> DNA
<213> Homo sapiens

<400> 785
actgctgctg gttaaggtca acctggggtg caatgctgct gtcttcatct tcggtcccga 60
agtaatgctc aataagatca aaggcctttt ggtagatctc ctggttttca tgactctgta 120
agaactcaat tttatccaga ccataagctt cttcaatcaa agcacagtaa gggttaatgc 180
cagtgccatt ccttttggct tcctgttctc caagcctcag gatattttcc aagccattta 240
gggcaacctg tacc 254

<210> 786
<211> 688
<212> DNA
<213> Homo sapiens

<220>
<221> misc_feature
<222> (1)...(688)
<223> n = A,T,C or G

<400> 786
   ggtactggct gagctggaag tgccaaaaag cactcctggc tgcttctggt tccatctgat 60
   gatgatgtga cacacactgc tgaaaaggcc caagcagggc aagtgggatg gctgaaggag 120
   ggaaggaggg ggttcagaac ccactggcct ggatgggaga actgggtgga ggcttcccca 180
   agagggaaga cagataaaca aaacaaaaca aaaactgggt aaagaggaat gaatcactca 240
   gccctgatgt ttcaattcta cactgcattc ctggccagtc gcatttgttt aatgcaggca 300
   tggccacagc tctcctagag aattatctca aagacccaga agggacctgg angaggccta 360
   tttcttaagg ttttccagtt ggaccaaggg aangantggg ttcacttagc ttctaaaaaa 420
   ggntttgaac cctaaggtta actgcctccg gaagctgctt gcttttggtt tggcttccca 480
   aaaaggnttc agaatagntt tggacccctt anggaaactt ggatcaagcc cggnaancca 540
   anacttnctt ggtngnaaaa tcaagggggg ctncttgggg nttanccgga agtttgggnc 600
   aggntgtntt aacagggtgg ggantgacca nccnggngcc caggggcctt antaacnttg 660
   ggaancccct gnganggaan ccttnacc 688

<210> 787
<211> 708
<212> DNA
<213> Homo sapiens

<220>
<221> misc_feature
<222> (1)...(708)
<223> n = A,T,C or G

<400> 787
acagtaacac aacatcaaaa gcaacacagg ctgtatacag aaacgtgggt cattcttttc 60
agccctaatg gagatgtaat taacagtatc gagcactctg gaaaatcact ctgcaggttt 120
atatggacta catggagatc atatcctgta gtgtagtgaa agctaagtcc tcaagagcca 180
tatgtataga tacacaatgt tttttaataa tctttaaaac agagatcaaa gttcatttaa 240
gtcctgtttg cattaacaaa aataaaaatg aaataaaaat gggaaccaaa tggatcatct 300
aaaaggttta aaaattccta aattgnccaa tttatccaac tggtgggaga cttaattcag 360
ggttttggaa agtccaggac tggtttcagc tgaacccaga aggcccccaa ttttgcttac 420
tggaactggc cctggggtaa gncatggaat taaaatngct tancnccttc ccctnggttt 480
tgaacttttg gccggttnga attattggtt aaaggcaggc tttaaaccaa gtttnccaac 540
ctgggctatt taacttggat cccattggga aaaattttca aanggaaatt ttttattagg 600
ggccatttca atcnaangga aaattntggg aactttggaa atnccgantc cttgntggaa 660
anaaaaaacc cnggggaaat gggnnggggg nccttnggcc cccaaccc 708

<210> 788
<211> 647
<212> DNA
<213> Homo sapiens

<220>
<221> misc_feature
<222> (1)...(647)
<223> n = A,T,C or G

<400> 788
ggtactctgt ctgctgaggg aatggggtat tttgactccc atagaaagca ctagcctaag 60
tcaccaaatg actgcttggt ccccactgaa gcagtgtagc tctccatagt atttttggtg 120
gttatggatt acatgtgtgg ccagctcatg ctttttcttg agcaggggct gtccatgacc 180
tgtgctcata ccatgctttc taagttctct ttggacaggg cctcagctgc tgcctcagcc 240
tgagtttcag agggtgtgta ggagtcctgg taatcttgaa gcagtttgac cacctccaaa 300
tggttgaact gcacagcatc atccagggga atggtgccca cctgtccttg gcaaaaggat 360
tcactttgca agccttgatc aggaatttaa caacttcgaa tgtgccctta nctgcagcaa 420
catgcnaanc tgggcnccaa gcataagctt tctggtccat atccatggct gacaaggcaa 480
cctttnaana ncttancatt ggcnctntnn gcngcaaata ccaggtggcc nnagcttggt 540
cccaattntg gccttacncc cggggntaan tccaaccaan gccttaggtn caaattngga 600
aattgaanan accccacttt ggcaaactgg cccctnggtt gncccat 647

<210> 789
<211> 650
<212> DNA

<213> Homo sapiens

<220>
<221> misc_feature
<222> (1)...(650)
<223> n = A,T,C or G

<400> 789
acctgcgcgc cctcgacgtc aatgtggcct tgcgcaaaat cgccaacttg ctgaagccag 60
acaaagagat cgtgcaggac ggtgaccata tgatcatccg cacgctgagc acttttagga 120
actacatcat ggacttccag gttgggaagg agtttgagga ggatctgaca ggcatagatg 180
accgcaagtg catgacaaca gtgagctggg acggagacaa gctccagtgt gtgcagaagg 240
gtgagaagga ggggcgtggc tggacccagt ggatcgaggg tgatgagctg cacctggaga 300
tgagagtgga aggtgtggtc tgcaagcaag tattcaagaa ggtgcagtga agcccaggca 360
gacnaccttg tcccaaagga atcagcaagg atgtgtgggc caagatcccc ctntttgccc 420
agcatgaggc aaaaatgtnc agccacccca ggctttnnta acanagctgg ctcttggttt 480
tggcactttt cctttcttta aacaaacctg ccattaagng anttggggtt caaaaaaaaa 540
aattntnnna naataaaaan tttttntctt cgcaccncct tnnggggaaa cncnantgng 600
gcggtntntt gganencinn tccncnttgg gnntangtat aatntttttt 650

<210> 790
<211> 646
<212> DNA
<213> Homo sapiens

<220>
<221> misc_feature
<222> (1)...(646)
<223> n = A,T,C or G

<400> 790
gggtaattcc ggctgttgca ccatggcgtc catggggacc ctcgccttcg atgaatatgg 60
gcgccctttc ctcatcatca aggatcagga ccgcaagtcc cgtcttatgg gacttgaggc 120
cctcaagtct catataatgg cagcaaaggc tgtagcaaat acaatgagaa catcacttgg 180
accaaatggg cttgataaga tgatggtgga taaggatggg gatgtgactg taactaatga 240
tggggccacc atcttaagca tgatggatgt tgatcatcag attgccaagc tgatggtgga 300
actgnccaag tctcaggatg atgaaattgg agatggaacc acaggagtgg ttgtcctggc 360
tggtgccttg gtagaagaag cggagcaatt gctanaccca ggcattcacc caatcagaat 420
annccatngc tattaacaag ctgnttcccg ttgctattga acactggaca agaacaacga 480
taccnccctg gtgacttaan ggcaccgaac cctgattaaa ccgnaaaccc cnctnggttc 540
aagnggnaca gttgcncccc cnatngttaa atctggangc cgcctnttgc ccanttggac 600
ggaaacntta tttgctttca attaaggcaa tggccgcagn tgagan 646

<210> 791
<211> 656
<212> DNA
<213> Homo sapiens

<220>
<221> misc_feature
<222> (1)...(656)
<223> n = A,T,C or G

<400> 791
accatgatat ctggcagatg tataagaagg cagaggcttc cttttggacc gccgaggagg 60
tggacctctc caaggacatt cagcactggg aatccctgaa acccgaggag agatatttta 120
tatcccatgt tctggctttc tttgcagcaa gcgatggcat agtaaatgaa aacttggtgg 180
agcgatttag ccaagaagtt cagattacag aagcccgctg tttctatggc ttccaaattg 240
ccatggaaaa catacattct gaaatgtata gtcttcttat tgacacttac ataaaagatc 300
ccaaagaaag ggaatttctc ctcaatgcca ttgaaacgat gccttgtgtc aagaagaagg 360
cagactgggc ccttgcgctg gattggggac caagaggcta cctatggtga acgtgttgta 420
acctttgctg cntggaaggc atttcttttc cggtcttttg cgcgatattc tggcttaaga 480
aacgaggctg agcctggcct acantttcta angaacttat taccganatt aagggttacn 540
ctgggatttg cttgcctgaa gttnaacccc tgggacctng gccgnacccc ntangggcaa 600
ttccanccac tggngggccg tactaaggga accaacttgg gcccaacntg gggnat 656

<210> 792
<211> 640
<212> DNA
<213> Homo sapiens

<220>
<221> misc_feature
<222> (1)...(640)
<223> n = A,T,C or G

<400> 792
ggtctgacac aatcagaaat tcgagacatc atcctgggta tggagatctc ggcaccgtca 60
cagcagcggc agcagatcgc tgagatcgag aagcagacca aggaacaatc gcagctgacg 120
gcaacacaga ctcgcactgt caacaagcat ggcgatgaga tcatcacctc caccaccagc 180
aactatgaga cccagacttt ctcatccaag actgagtgga gggtcagggc catctctgct 240
gccaacctgc acctaaggac caatcacatc tatgtttcat ctgacgacat caaggagact 300
ggctacacct acatccttcc caaagaatgt gcttaagaaa gttcatctgc atatctgacc 360
ttcgggccca aattgcagga tacctatatg gggtgagccc accagatacc cccaggtgaa 420
agagatcccc tgcattgtga tggtgcccca atggggcctt accanaacgn gcacctgctg 480
gcaantgnct aactgagacc tgcccggcgg ccgttcaang gcaattcngn nactggnggc 540
cgtctaaggg accnacttgg gccaacttgg gnaatatggc nnactggtcc tggggaatgg 600
tntccgtcca ttcccanttc anccggaanc taanggtaac 640

<210> 793
<211> 615
<212> DNA
<213> Homo sapiens

<220>
<221> misc_feature
<222> (1)...(615)
<223> n = A,T,C or G

<400> 793
acctacaact atatctactc cattttccaa aacagagagc tgatcccggg ctgcaacacc 60
tccaattatc agaagctccc ttaatttagg attatcaatg tatttcttaa actgcttgat 120
gttattcaaa gtttgttcag ctaactcccg ggaaggttca acaatgagag ctttcggagc 180
attggggaga aactttgttt gtgtcacctg tgcattacct gagtgctgtg atttgacaat 240
gtaaccatcc ggtgccttgg aaagagcaac aaagccatct tttggtggaa acttaaattc 300
ctcttcaccc gaagttaaat ttcagttcag cattcttcaa aacacaggca ggaaagaggg 360

cttggttttt catatgtggt ggtatttcaa atgccagacc aaganctttt ccatttttgg 420
agaacttgac atgtccttat ctatatcnng tacatccatg ggatcatgcc tagngaatnc 480
tttcataata tcaaatggtg gtatggaatc ttcctgtccc caagccaatc caactggaga 540
ccttggcggc ccntanggca atcancctgn gccgctaggn ccactggcca ctggnacagg 600
cnntgtctgg aatgn 615

<210> 794
<211> 709
<212> DNA
<213> Homo sapiens

<220>
<221> misc_feature
<222> (1)...(709)
<223> n = A,T,C or G

<400> 794
   acttctgaat aagttcagag ccaaccactc tcaagaaagt ggctgaggtt tggtttgcta 60
   ctgctttggc taacaaggtt ttacctgtgc caggtggacc atagagaatg acccccttag 120
   gaggctttat acccatctct tcataatatt caggatgggc gagaggaagc tccacagatt 180
   ccttaatttc ctgaatttgg ttgtccaacc ccccaatatc tgcataggtc tcctgggggg 240
   ccttttctac cttcatcact gtgaccaggg gatccgtgtc atccatcagc acccctatca 300
   cggnatgcac cttgtggttg agcaggaccg agcagccagg ttccagcaga tccttgctac 360
   aaatgaaaga atgctgacgt antgttctga gcccacagat gtagacacga atggcatgat 420
   ggcatcaatg atctctttcc aaggttccta ctgacatcgg ggtccccctc agaatcatcc 480
   acttttggat ctttccttcn tcttgntttt ccttctaaag gggttcaatt tggtncccgg 540
   atttcttaag ngaatctttc cttncnttga aaaaaaaaag gccnttnaaa tnctntttta 600
   acctttangn aanttttaaa cccgggcctt gaattnnnaa gggggcnccc cngggggcaa 660
   ttttncttgg cnnnaatttg gggccccttt gggnttnntt ttttttttt 709

<210> 795
<211> 693
<212> DNA
<213> Homo sapiens

<220>
<221> misc_feature
<222> (1)...(693)
<223> n = A,T,C or G

<400> 795
ggtacggcaa tcaatcttaa taatccagag agccagtcca tgcatttgga aaccagactt 60
gttcagctgg acagtgctat cagcatggaa ttgtggcagg aagcattcaa agctgtggaa 120
gatattcacg ggctattctc cttgtctaaa aaaccaccta aacctcagtt gatggcaaat 180
tactataaca aagtctcaac tgtgttttgg aaatctggaa atgctctttt tcatgcatct 240
acactccatc gtctttacca tctctctaga gaaatgagaa agaatctcac acaagacgag 300
atgcaaagaa tgtctactag agtcctttta gccactcttt ccatccctat tactcctgag 360
ccgtacatgt gcataggaac tgggatatac acaggcacag ggataggcac tggaacatat 420
tctgnctnca agtatcatct gctgaccaag aattggnctg catgtgaagg ttacagtaag 480
tacttttggc attggtaaan ggttgccaaa aaactgnttt ggnccttnan cnctttggta 540
aggggttgga aaaaggggtg gggcttaaac ctggcanttt nggttcnana agtntggaaa 600
ncctggganc ttaagggaag gtttttangg gccnttttga aatggcaatg tgggcncaat 660
ttggtggccc gtnaaaaccc cntanncaag gtn 693

<210> 796
<211> 452
<212> DNA
<213> Homo sapiens

<220>
<221> misc_feature
<222> (1)...(452)
<223> n = A,T,C or G

<400> 796
ggtacattca cgtctcccgg ccgcttcacc tgaaagccat cggtctcctg ggtagtggcg 60
gtcctgtgcc attctaccag atggttgtct ggcccataca ggtctttgtc cagttcaatc 120
accaaggatt taaaaaagga agagaacttc ctcttttgtt tagtggcatc atatttggac 180
aaggctgaat cctccaggag ccgtccttct acccgaagct cccaggaagc caccgtccct 240
tccccatcct cggcatctga cttagccgga ttgaaagtgt tagaaatgaa aattcgcagc 300
ttccgttttt gcttgatggg acgtttcaag gcctcttgga tatctagccg ttcctcatga 360
tagtctggtc cagttccttt caaaagccaa gagatccata taggcctggg attctggtac 420
ctgccnggcc ggcgctcnaa nggccaattc aa 452

<210> 797
<211> 333
<212> DNA
<213> Homo sapiens

<220>
<221> misc_feature
<222> (1)...(333)
<223> n = A,T,C or G

<400> 797
ggtacaagct tttttttttt tttttttttt ttttttatta ngcgcaagtg gtcaaaagtt 60
gtcaaaattg tcctcattcc tcgattgtct cttttttacc agtctcttgc ccttcaaaca 120
gaggatacct ggcctccaca tcagcccatg tgatgttgcc attggctagg tcttggacta 180
tgctgggcag ctcagagatc tctgctctta tctgccgcat tgagtcacgg tccctcagag 240
ttgcagtgtg gggggtcttg ttcactgtgt caaagtcaat ggtgacacca aaagccacgc 300
caatctcatc aagtcctggc atancgcctt ccg 333

<210> 798
<211> 632
<212> DNA
<213> Homo sapiens

<220>
<221> misc_feature
<222> (1)...(632)
<223> n = A,T,C or G

<400> 798
ggtgcttttt tttttttttt tttttttttt tttttggaca cagatcactt tattggcatg 60
gctttgtttt aagaaaagga aaagtgacaa agccaagaga cagactctgc taacagatgc 120
ctgggggtgg ctggacattt ttgcctcatg ctgtgcaaag agggggatcc tggcccacac 180

atcctgctga ttccttggga caaggttgtc tgcctgggcc tcantgcacc ttcttgaata 240
cttgcttgca gaccacacct tccactctca tctccaggtg cagntcatca ccctcgatcc 300
actgggtcca gccacgcccc tccttctcac ccttctgcac acactggagc ttgnctccgc 360
cnagctcact gntgcatgca cttgcggcat ctatgcctgn caaatcctcn ttaaactctt 420
tnccaacctg gaagtncatg gatgtagtcc taaaagtgct ancgngccga tgatcatatg 480
gncaccggnc tgnaccnact tttggctggc ttancaagtt gcaattgcnn aggccattga 540
cttaggcncc agtcttcccg gcgccgtnaa ggcaatcncc attggcggnn tctagggncc 600
nntggncagt tggtnatngg caantntcng ga 632

<210> 799
<211> 462
<212> DNA
<213> Homo sapiens

<220>
<221> misc_feature
<222> (1)...(462)
<223> n = A,T,C or G

<400> 799
ggtactgcgt ctgtttttgt taccccacaa ggaccagcgc cagatgttct ttgtgatcag 60
cctggatccc ccaatcaagc aaggccaaac tcgctaccac ttcctgatcc tcctcttctc 120
caaggacgag gacatttcgt tgactctgaa catgaacgag gaagaagtgg agaagcgctt 180
tgagggtcgg ctcaccaaga acatgtcagg atccctctat gagatggtca gccgggtcat 240
gaaagcactg gtaaaccgca agatcacagt gccaggcaac ttccaagggc actcaggggc 300
ccagtgcatt acctgttcct acaaggcaaa gctcaggact gctctacccg ctggagcggg 360
gcttcatcta cgtccacaaa gccacctgtg cacatncgct tcgatgagac tcctttgcaa 420
cntttgtcgt ggtacctgcc cggccggncg ttcgaaangg cc 462

<210> 800
<211> 702
<212> DNA
<213> Homo sapiens

<220>
<221> misc_feature
<222> (1)...(702)
<223> n = A,T,C or G

<400> 800
gaggtgtcct cccctccaag cagaccacct gtccccttct atcccagctc agagcagctg 60
acccaactca gaatctcttt cctacaggat gaagtgcctt ttgaatgtta ttttaagccg 120
agagttaatt tttctacaca acatatttcc agacatcttt tagtctttta ttgtcttaga 180
tactataaga agatgaacat gacaattttc tagaacctgg tagcgtgtgt gtgtgtggcg 240
gggggtgctg agggagggga gtgagtcaca ggagcctgtc ccccaacagg tgtgattgct 300
ctgacaacct gtggcatgct gcagggtcag gctcctgata ggaggatttc atgactatgt 360
cattgnctcc actcattttt gacccagttt ggaatgtatc tgcaattggt gtggctcaac 420
actttaggaa acaatagaat tattttatat aataattctg atggtgacca agtttngnct 480
tggagggcca caattttctt cctttgaaaa agtggacant ncctggncac ttctggnttt 540
ttaaaactta ctnggccatt ccatttgggg ggtttttttg ggnnggtaaa ttgggtttgg 600
gggttaaaaa cccgtttncc agggaaaanc ccctaaaaaa nccctttggg gaattttaaa 660
anggaaaaat tctgggntaa attngggntt ttttaaaaac cc 702

<210> 801
<211> 719
<212> DNA
<213> Homo sapiens

<220>
<221> misc_feature
<222> (1)...(719)
<223> n = A,T,C or G

<400> 801
aggtactgcc cagagaattt tgtagacatc aagaaaactt tggaacgaga gactcgccag 60
tgccaggctc tggtgatctg gactgactgt gatagagaag gcgaaaacat cgggtttgag 120
attatccacg tgtgtaaggc tgtaaagccc aatctgcagg tgttgcgagc ccgattctct 180
gagatcacac cccatgccgt caggacagct tgtgaaaacc tgaccgagcc tgatcagagg 240
gtgagcgatg ctgtggatgt gaggcaggag ctggacctga ggattggagc tgcctttact 300
aggttccaga ccctgcggct tcagaggatt tttcctgagg tgctggcaga gcagctcatc 360
agttacggca gctgccagtt ccccacactg ggctttgtgg tggaaccggt tcaaagccat 420
tcaggctttt gnacccttgg ggccgnnaac accttaaggg ccgaatttcc agcacaactg 480
ggcgggccgt tactaagngg gantnccgaa cttngggnan cccaagcttt gggcgtnaat 540
cattngggnc ataaacttgg gttnccctgg nggngnaaaa ttgggntaat cccggtttna 600
caaatttccc cccccaactt tttccnaaac cccgggaaag ccttttaaaa ggggtnaaaa 660
acccctnggg ggnggcccct aaatggagtn ggggncttta accttcnccc ttttanant 719

<210> 802
<211> 646
<212> DNA
<213> Homo sapiens

<220>
<221> misc_feature
<222> (1)...(646)
<223> n = A,T,C or G

<400> 802
actcatcgcc attgacctgg cctataactt gcacagtgcc tatggaaact ggttcccagg 60
cagcaagcct ctcatacaac aggccatggc caagatcatg aaggcaaacc ctgccctgta 120
tgtgttacgt gaacggatcc gcaaggggct acagctctat tcatctgaac ccactgagcc 180
ttatttgtct tctcagaact atggtgagct cttctccaac cagattatct ggtttgtgga 240
tgacaccaac gtctacagag tgactattca caagaccttt gaagggaact tgacaaccaa 300
gcccatcaac ggagccatct tcatcttcaa cccacgcaca gggcagctgt tcctcaagat 360
aatccacacg tccgtgtggg ccgggacaga agcgtttggg gcagttggct aagtggaaga 420
cagctganga ggtggccggc ctggatccga cttctggctt gtggaaggaa cagcccaagc 480
cagaatcatt ggcanccagg aanggcatgc tngacccact ngaaggngcc cttactngga 540
cttccccaaa attgggcatt aaagggntcn gggcttcnaa ttcccttttc aggccnggtt 600
tnanggnggg aaaaattcgg ggaatttnat ccttaaagcc nttgnc 646

<210> 803
<211> 544
<212> DNA
<213> Homo sapiens

<220>

<221> misc_feature
<222> (1)...(544)
<223> n = A,T,C or G

<400> 803
acacgtcgtc ctcccggctc aggccctcaa agaaggggat gaggtccagc agctccgtgt 60
ccgtcatgtc atcgaaccag gactgcacag gcactgcatt ctcaggatgg aagatgtatg 120
aggcagggga attgtcaaca atgatcactt tgctcagctc ccgcccaagg cgactcaggt 180
ccttcacgta gttcccacga tgaaaaacac atgattctct gaagagccgg gcccggaaca 240
caccccagcg gtctaggagg tcagccacag ggtctgcata cttggccaag ctggcagtaa 300
agagcacaca ttcaaaaagc tgcccatcct ctggaggaac tcgtccacat gtggccgctt 360
cagcacatac acctgatgta tagttccatc gattcaaccg gaacaataaa atnagcanta 420
ctaaataggc ttaaaacgaa ctgtgcacca atggttcatt ctaaatcaat ggaccaccca 480
ttcttttcca tagtcnagca ccggtacctn tggaanaang tnccttgggc gngnaccccc 540
ttan 544

<210> 804
<211> 642
<212> DNA
<213> Homo sapiens

<220>
<221> misc_feature
<222> (1)...(642)
<223> n = A,T,C or G

<400> 804
cgaggtacat ccttgtggga gagaacctca tcaatttcca catttcttcc aagttctctt 60
gccctgagac ggattctcat cgctttggaa ggcacctgaa agaagcaatg actgacatca 120
tcactttgtt tggtctcagt tctaattcca aaaagtaatt ccactggagc tgctgggaag 180
gaaaacgagc tcttctgatg caaaccaaat gaaaaatagg cattaatcct gaccttagct 240
cgggatgaaa cactgctctt aaaaaaactc agttttcctt ccagaaaatg tgggtgtttt 300
tttttcctag aacagtatct ctcccctgtg aagcataacc ccactacttc cagacttgcc 360
ctcccttggg ggacatctga taaagtctcc cctgatgtct ccgcatcggc ttggattatt 420
aagggatgca aatcttggtg agttaatnaa ngaattanta ngggtgtggn tttacccncc 480
agtggaatgg aaatnggngt gctttntant nggcaanncg aaggcctaag ctttanggcc 540
tttaaccttt ntccangcng ggtaaacttt tggtttgntn aaaanaaaan tnnttnttaa 600
agttggggnc ccanttgagc taaccatttg ganngcctac cc 642

<210> 805
<211> 261
<212> DNA
<213> Homo sapiens

<400> 805
cgaggtacta cagagcccct ggacggtgtg atgttggaaa aggatgtttt ttctcaacct 60
gaaattagta atgaggctgt taatttgaca aatgttttac cagctgataa ttcatcaaca 120
ggatgctcta aatttgtcgt tatagaacct ataagtgaat tgcaggaatt tgaaaacatc 180
aagtcatcca catcattaac tcttacagtt cgaagttcac ctgctccttc agaaaatact 240
catatttctc ctttgaaatg t 261

<210> 806
<211> 311

<212> DNA
<213> Homo sapiens

<400> 806
gcggagagcg gctgatcgca gtccggaggt gaggcggaac tctgagcagg tggtccatta 60
tggctgacat gcaaaatctg gtagaaagat tggagagggc agtgggccgc ctggaggcag 120
tatctcatac ctctgacatg caccgtgggt atgcagacag tccttcaaaa gcaggagcag 180
ctccatatgt gcaggcattt gactcgctgc ttgctggtcc tgtggcagag tactccagtt 240
ctcagccaga accccgcaca ggtctttcct tatgggatac cagcccctca tacattgata 300
aattgggtac c 311

<210> 807
<211> 591
<212> DNA
<213> Homo sapiens

<220>
<221> misc_feature
<222> (1)...(591)
<223> n = A,T,C or G

<400> 807
ggtacctgtt ctttgccagt taagatacat atcttattat ctttgttttt ttcaagtcta 60
tgctcctgtt tgaagctttt cctgtaattt aggttgtctg tgaaatacct ataacatata 120
attcctatag agtatgccac attttttttc taactcattt caaatgaaat tctctcagat 180
tctagttttt gagcttgtcc actagatctg aaaataaagc atcctttcct gagtccactt 240
gaactaattg tgaatttgtt acttaattta ctggcatctt gggaaacaag ttttgctgtg 300
gcaggaaggc tgttttgaga gtgagccgtt gaagtctact ctggtttgtg gatgacattg 360
cattaggggt tatttcctgn attaccagtg cccccttgtg gcaatatact ttatgacttg 420
gaatgcaaca ccacttttaa aagcctggtt tcaagttttg aaagcattgg ttctgtgntg 480
ccataatctg aagnttctgt gaaggattat tnaagcttta aaccttncaa ggtaaaggcc 540
aaattaggcc tggaattacc tggaccttgg ncaaaaattn aaanattncn n 591

<210> 808
<211> 641
<212> DNA
<213> Homo sapiens

<220>
<221> misc_feature
<222> (1)...(641)
<223> n = A,T,C or G

<400> 808
actaaatgga ggcacgtggg agaagggagg ggccattgag gaacaaaaat gtgttttaag 60
gaagagatgg gaaagcagag accaggtaga ggagctaggt aagctgatag gtgttgtcat 120
tggtagaaaa gaagaagata aatggatgta aggattgagg ccttggaaag tagcataggc 180
aggaaaagag gaattagaag aatacgtgaa gaagtgggaa tcatgggctg ggaagggaaa 240
ttttggaaaa ggagcacatt aaggcagaaa actcttttag agcagtggtt ttaaacttca 300
gcaatggtga tccttttata caagtatccc ttactttgga atcccaggaa gtaaaaggca 360
cattcttgtt gaagttgggg aggagcactt ggaaccctgc ttgcttaact tttttctttt 420
tgggcccttg aagtgtagta tattttaaaa tccactggtc tanaagggag tagttaagtt 480
naagggaaan aaaggatgat tgggaaaaga tcngacccga agggactttt tggtnaccca 540

aaagttttng gtncccttgg aaagggaagg ggcccctttt nggaattang ggaaatggaa 600
acttggaact gggnaaantt cctntnagct taaccttgan g 641

<210> 809
<211> 388
<212> DNA
<213> Homo sapiens

<220>
<221> misc_feature
<222> (1)...(388)
<223> n = A,T,C or G

<400> 809
acaagagggt gggctgggcc aggatgcccg agggctggcc acagccaccc ccctcaaagg 60
tgttgatgag aaaagagaca ccttcttcct tgagaacatc tttcagccac aaattagggg 120
atctgttgcc tggcaataaa ggaacgaatt tataaaagag ttcaatggat ttgtgtcgac 180
attctgtctg gggcctccca caatgagcta aaagccactt gaccagatcc aataaacaca 240
atgatgcgga aggtggaaat cctcgcggca aacgtcgttt ctttgcttta tttaaagaaa 300
catgcttctt ttcaatgatg cggcataggt gatcaatggc atcacaacac tgttgaattg 360
tacctcggnc gngaccacgc taaaggcc 388

<210> 810
<211> 175
<212> DNA
<213> Homo sapiens

<400> 810
ggtacatcct cggccgggag tccccactgt ctctctacaa tgaggagctg gtgagcatga 60
acgtgcaggg tgattatgag ccaactgatg ccaccgggtt catcaacatc aattccctca 120
ggctgaagga atatcatcgt ctccagagca aggtcactgc caaatagacc cgtgt 175

<210> 811
<211> 329
<212> DNA
<213> Homo sapiens

<220>
<221> misc_feature
<222> (1)...(329)
<223> n = A,T,C or G

<400> 811
ctgcgcggtt gttctctgga gcagcgttct tttatctccg tccgccttct ctcctaccta 60
agtgcgtgcc gccacccgat ggaagattcg atggacatgg acatgagccc cctgaggccc 120
cagaactatc ttttcggttg tgaactaaag gccgacaaag attatcactt taaggtggat 180
aatgatgaaa atgagcacca gttatcttta agaacggtca gtttaggggc tggtgcaaag 240
gatgagttgc acattgttga agcagangca atgaattacg aaggcagtcc aattaaagta 300
acactggcaa ctttgaaaat gtctgtacc 329

<210> 812
<211> 668
<212> DNA

<213> Homo sapiens

<220>
<221> misc_feature
<222> (1)...(668)
<223> n = A,T,C or G

<400> 812
acggatgcta cttgtccaat gatggtaaaa gggtagctta ctggttgtcc tccgattcag 60
gttagaatga ggaggtctgc ggctaggagt caataaagtg attggcttag tgggcgaaat 120
attatgcttt gttgtttgga tatatggagg atggggatta ttgctaggat gaggatggat 180
agtaataggg caaggacgcc tcctagtttg ttagggacgg atcggagaat tgtgtangcg 240
aataggaaat atcattcggg cttgatgtgg ggaggggtgt ttaaggggtt ggctagggta 300
taattgtctg ggtcgcctag gagggctggt gagaatagtg ttaatgtcat taaggagaga 360
aggaagagaa gtnaccgaag ggcctcttta nttgtgtaat aanggttgga aggtgatttt 420
tatccgnaat tgggangtga tccctaaggg ggttggttga nccccntttc ctgccanaaa 480
tagganggtg ganttctgct tagggcttcc aataattgan gggcctnaaa tnaanttgna 540
aanggtaaat aaaacctttt naagggttgg gaccttgttt cttgngtnna ncccccttan 600
nattccattg gaacttaggc ttggncccat gtnttgggan tggcggataa ttaanttttg 660
aaattncc 668

<210> 813
<211> 312
<212> DNA
<213> Homo sapiens

<400> 813
ggtacaggca gggtagatct aactattgga aggaatccct aacacttttc cagggtagaa 60
ttctggctag tccaaaaagg gtccttcttt taagggtttt gagaaactag acactgcaac 120
ttattagtat cggcgacgtt tgtttggggc aaattcagct ccaggagctg cacggttgaa 180
tgcaggagga gttccaccaa ttgccccaat tccttccatt gtagcagcct gaccaaagcg 240
ttcagttgtt ggtggggtca atcccaaagt tccatccggc atcatagtgg caggtcctgg 300
aggagctggg gt 312

<210> 814
<211> 551
<212> DNA
<213> Homo sapiens

<220>
<221> misc_feature
<222> (1)...(551)
<223> n = A,T,C or G

<400> 814
caggtactct gaagtataca caacaggtct aaacatctcc cttgtcgtaa gtagttgtgt 60
aaaattcaag ataaagattt agtctcatct tttaatgtca gtttttttcc ccatgttaaa 120
gggaatgagg aggagtcctc ttttattccc ccacaagaaa aagggagcca cattaatatg 180
tgtatattcc cataactcta atgtaagtgc ggatctccaa agcctaggga tttttccgta 240
aaagagagtg ggccgttctg gttacccttt tattagaagg gtattccacc acagagagcc 300
ggaggttttc cagatgtgtg taagagagca ggtgcgcaag gcaagcaaat gagcgcaaac 360
agtattatgg aaaacatttg agaagttagc tccatgagga ctgtgggctt cacaagagga 420
ctcgactggg tagccctggc tgacanagga cctgaaaagc ngagtattgc ttcaaacttg 480

gaaccnttca taggagccta acactgttgg aagaagtacc ttggcnggac caccttangg 540
gcaattcnag c 551

<210> 815
<211> 619
<212> DNA
<213> Homo sapiens

<220>
<221> misc_feature
<222> (1)...(619)
<223> n = A,T,C or G

<400> 815
ggtactgata acttcttgct tcagttcatc tacaatgatc tttccctcta aatcccagat 60
cttgatgctg gggcctgtgg cagcacacag ccagtagcgg ttagggctga agcacagggc 120
gttgatgatg tccccaccat ctagcgtgta aaggtgtttg ccttcgttga gatcccataa 180
catggcctgg ccatccttgc ctccagaagc acagagggat ccatctggag agacagtcac 240
cgtgttcaga tagcctgtgt ggccaatgtg gttggtcttc agcttgcagt tagccaggtt 300
ccataccttg accagcttgt cccaaccaca ggagacgatg atagggttgc tgctgttggg 360
cgagaagcgg acacaagaca cccactctga gtggctctca tcctggacag tgtattttgc 420
acacacccag ggtattccat agcttgggtg gtttacctgn ccggcggccg tcnaaanggc 480
gaattcacca tggcggccgt actagngatn caacttggnc caacttggcg gaatctggca 540
tactggttcc tngggaaatt gtttcngtcc aattccncna aattnaaccg gaagnttaaa 600
ggtaaaactt gggggccta 619

<210> 816
<211> 658
<212> DNA
<213> Homo sapiens

<220>
<221> misc_feature
<222> (1)...(658)
<223> n = A,T,C or G

<400> 816
actccagcag ccaggcatcc cagatctcct gtcctggagg gtgctggggc ccctggctcc 60
ccagagtgtg caggcagacc cccagagccc tagctcatcc atttatccat tcctcataat 120
ccagtgtcca aagagtaccc ccagcagggc agggaaggtc cctcccgggg tttacatgac 180
tgattccttc tcagaggcga ccgtggcatc ccctgcgggc ccccgatagt gtttgaggag 240
ggggtttcct tcctcaggct ctgtgcttct cgactccgta caagcttttt tttttttttt 300
tttttttttt tggaaggaga acaattttat tctaaaaata gaacttggta acaatgaaat 360
accaaaagct ggtcattata ataaaaagaa aagaanagtt taactttttt tttgtgaaaa 420
ttcnaaaatt atcactataa tatactgcca actntggtna attnganttt gaattatttc 480
ctttcatngg attatttcaa gggaaatttt taaaattngn ttttggccta aaaccttngg 540
ccgggnaccn cncttanggg gcnaaattcc aatccaantg ggggggnccg taacttaagg 600
gggancccaa ccttgggnnc caancnttgg ggngtaaatc atggggcana ncntgttt 658

<210> 817
<211> 141
<212> DNA
<213> Homo sapiens

<400> 817
actttcttct gccataactt cttcctcagt tcctacaggt gtgacacttt tcaacttctt 60
tggaagaggc atttccactg tatcatcaga gacttggtct gatgcttcta tggtgctatc 120
ctcttcctct tcacgtgtac c 141

<210> 818
<211> 280
<212> DNA
<213> Homo sapiens

<400> 818
ggtacttaag aactcaagta tagaaataaa ctgtgggctg aagtaacatt gtaacctgct 60
cccaacatga ctgcataggt gtctaaggtt aagtgtgaag attactgtga ggtctcaagt 120
tacttgacta atcaatccca tttgaatttc aatccaagca gcatatttta cacacacctg 180
aaggaaatat cttcagtgtg ttcatgtgtg tgtctatgtg catgtatgtg taggggatag 240
gtgtaattag ggaagggctg accgaacaac attgataagt 280

<210> 819
<211> 635
<212> DNA
<213> Homo sapiens

<220>
<221> misc_feature
<222> (1)...(635)
<223> n = A,T,C or G

<400> 819
ggtacttgag tccttctcat gggtggggtg attgcctctt ctcatcagga gccaggagag 60
agggggacag ataggaggtg gcccatagga gcagtcccgc tgcacaatgg taggcatagg 120
ccatggcact ggactgcctc taaggactgc taaaaagaat atttttttgt ggtgtcagaa 180
ctggaaaaag cactttccct tcgggcattt ctggaaatga ttattaatcc acaaagaaga 240
actctgtaag ctttttcttg aattgtancc agtgagaaaa gcagatagac tgaagaatat 300
gaaggatagc tgagctgtnc ctncatagtg gggcatgcct aggcatatgg ctggcttgga 360
gactactgat gcttttccct gagtttgtat tggcactgan gtatggccgg cttgggccac 420
tgacttccca ntaatggaat ctgntnaaaa cttggggatt cctttagctt nntactggaa 480
gaaaantttt gtancnaaaa gatttataac cnnttagnaa taagtttncc agcancccng 540
gatttttttt nngcttgggg gttnttggcg ncctttannn aaggacnggg cnttgnnntt 600
cntctttacn aggccttgnt ntgancntgg agaan 635

<210> 820
<211> 276
<212> DNA
<213> Homo sapiens

<400> 820
acatcttctt cctgagttac gcttacaaaa ttttcaaaca tagcaaccat tgatggggcg 60
gcaatcacat gacaattcac aagatcagat aaaaaacgga ccaaatacac ggcttcatta 120
taattgtttg ctttcaatga ttctttaagt tgacgaatca tggcttctac aaattctcca 180
ccaaaattgt aattcctggc attcagtagt ccaactaatg ttgtataaat tgtcagcttc 240
tcaggtaata ggcgtgcact ggattcataa atcacc 276

<210> 821
<211> 728
<212> DNA
<213> Homo sapiens

<220>
<221> misc_feature
<222> (1)...(728)
<223> n = A,T,C or G

<400> 821
acaatgatgc cagaagcttt ccttcaagaa gctcagataa tgaaaaaatt aagacatgat 60
aaacttgttc cactatatgc tgttgtttct gaagaaccaa tttacattgt cactgaattt 120
atgtcaaaag gaagcttatt agatttcctt aaggaaggag atggaaagta tttgaagctt 180
ccacagctgg ttgatatggc tgctcagatt gctgatggta tggcatatat tgaaagaatg 240
aactatattc accgagatct tcgggctgct aatattcttg taggagaaaa tcttgtgtgc 300
aaaatagcag actttggttt agcaaggnta attgaagaca atgaatacac agcaagacaa 360
ggtgcaaaat ttccaatcaa atggacaagc tcctgaagct gcactgnatg ggccggntta 420
caataaagtc tgaaggcctg gncattttgg aattcttgca aacccgaact tagttaccca 480
aangggnccc aatngccntt attcccaggt antnggggga aacccggnna aagtaacccn 540
ttggggcccg ggaaaccacc nccttaangg ggccnaaatt ttccaggcnn cnacttgggg 600
cggggcccgg ttancttaag gggggaatcc ccnaacnttt ggggacccca anacntttgg 660
gcgggaaaac cnatnggggn ccaaaanacc gnggntnccc ccgnggnggg naaaaaattg 720
gnnttnnc 728

<210> 822
<211> 632
<212> DNA
<213> Homo sapiens

<220>
<221> misc_feature
<222> (1)...(632)
<223> n = A,T,C or G

<400> 822
actttacggc ctgatctaat tgaaagtgca tcccttgttg caagtggcaa agctgaactc 60
atcaaaaccc atcacaatga cacagagctc atcagaaagt tgagagagga gggaaaagta 120
atagaacctc tgaaagattt tcataaagat gaagtgagaa ttttgggcag agaacttgga 180
cttccagaag agttagtttc caggcatcca tttccaggtc ctggcctggc aatcagagta 240
atatgtgctg aagaacctta tatttgtaag gactttcctg aaaccaacaa tattttgaaa 300
atagtagctg atttttctgc aagtgttaaa aagccacata ccctattaca gagagtcaaa 360
gcctgcacaa cagaagagga tcaggagaag ctgatgcaaa ttacccagtc tgcattcact 420
gaatgccttc ttgctggcca tttaaactgt aggtgtgcan ggtgactggc cgttcctcag 480
ntncttgtgg ggaatcttcc gtnaagatga acctgacttg ggancactta ttttttnggc 540
tangnttaaa ccttncatng ngnncaactt tacccangtn gnttantatt tngncccccg 600
ttaanacctt tctncnngnt cctccatttt tg 632

<210> 823
<211> 649
<212> DNA
<213> Homo sapiens

<220>
<221> misc_feature
<222> (1)...(649)
<223> n = A,T,C or G

<400> 823
actgctgcaa cccatgcagc gtcaacttcg tctcatcatc cacgaagatc tccattggat 60
cttgcatgaa cttgcggcag actggacgga tctctttgct caaggtagca ctgaacatca 120
tgacctgctt ctcgtggggg gtcatgcgaa aaatttcctg gacatcccga cgcatgtcga 180
gctgttcaag catcttatca cattcatcca aaataaagtg tttaatgtgt ttgaggttga 240
ggctcttatt tcgagccagg gctaggatac ggcctggagt ccccacgacg atatgcgggc 300
agttcttctt cagcacctct tcatccttct tgatagacag accaccaaaa aaaacagcaa 360
ccttgacatt gggcatgtat ttagagaagc gctcatattc cttgctgatc tgaaaagcca 420
actcccgagt ggtgacacca tcaccagcac agacacctgc ccagtaacct ggcttccaac 480
tggttgcant gnngggccaa gaacaaacac tggtggcttt tccatgcccc natttgggct 540
tggcnccagg aaattcantt cccaaaatgg gcttgaaggg atgccnttnt gcttggactt 600
ttgacgggat gttnaaggcc ccagnttnan aatggncccg gagcaattn 649

<210> 824
<211> 603
<212> DNA
<213> Homo sapiens

<220>
<221> misc_feature
<222> (1)...(603)
<223> n = A,T,C or G

<400> 824
acccccttata aaccagcaat gtcatctgtg aggaagcaaa ttctcaagtg tctgtcattt 60
acttggttct ttttctttgt ggtcttcacc cttataccct ggaaaagtct gtaattacct 120
tagccaggaa gatagatggt catggcaagc gcacagcacc agacttactg gctcaccaag 180
atgatggaaa aaggcagatg attttttaaa aagccgtaat gactccttta gaccagccat 240
ttagcgtggt aattttgaaa ggcctagctc cattgcagac ttccaaaggg tcagctctga 300
gactgccctc caggtgggca gttgattatt tccaccagtg ttttccagag ccttaaactg 360
cctaagtgac aactacctca gttggcagga aaagagacat atagtagaaa gtgaaaaatg 420
agcagtattt gggcagatgc tatggggtac agttgaangg taaaanggac tttccttggg 480
aacccttatn ccctgngaat atgacctngg ccggacacnt taaggcnatt cacnntgngg 540
gccgtctaan ggnnccactt ggncancttg ngnaaaaggc aaactgtnct gngnaatgtn 600
ccc 603

<210> 825
<211> 634
<212> DNA
<213> Homo sapiens

<220>
<221> misc_feature
<222> (1)...(634)
<223> n = A,T,C or G

<400> 825
tgaaaaataa actattntat ttcagtgttt gctccttgcg gttcagaagc acatctactg 60

cctggttgga acccaaggct tttataaaac cgtagagaaa tatgagctct atgtatagag 120
aaaatataca tgttgattaa ttgtgtgact ctttcctgtg caaagcagaa agttctaaat 180
gcaacagcat gattctctcc aagtccttcc ctgggatttg gggggccctg gaggctgtga 240
tctcacctcc aatagagaat ccccaattct tccagcccaa gggaggccca gncatgtaga 300
aagagcagga gataaagtca aagctgacaa ctcatgggtt ccccaagctt ctccggggca 360
ggggctatgt ttgggggcct taccctgcaa agaaggggta gctggggtgc cnaccttggt 420
gggtaagtgc cacactggca ctaaagctgt tgggaagtct agcattgcan ccggccaggt 480
ttatgggtna accagggtgt ccaangggtt tttttcccta aaactngggg ctnaaaggng 540
gggaccctng gcncgaaccc ccttanggcc aaatcccggc aattgggggc cntttttaan 600
gggnnccaac ttgggaccaa acttggngna atnn 634

<210> 826
<211> 507
<212> DNA
<213> Homo sapiens

<220>
<221> misc_feature
<222> (1)...(507)
<223> n = A,T,C or G

<400> 826
ggtacctgaa gaacaaatcc cttcagggtt aagctcgaca ggacactttc cccagtccca 60
ggtttccatt tccctcattc ccaaaagggg cccctccctc tccatgcgca cacagaactt 120
ttcgctcacc caaaagtccc ttctgtctga tcttttccca tcatctttct tccctctact 180
tactactccc tctagaacag tggattttaa atatactaca cctcagggac caaaagaaaa 240
aagttaagca agcagggttc caagtgctcc tccccaactt caacaagaat gtgcctttta 300
cttcctggga ttccaaagta agggatactg tataaaagga tcaccattgc tgaagtttaa 360
aaccactgct ctaaaagagt tttctgcctt aatgtgctcc ttttccaaaa tttcccttcc 420
cagcccatga ttccacttct tcacgtattc ttctaantcc tctttttctg gctatgctac 480
ttttcnangg ctcaaaactt aaattcn 507

<210> 827
<211> 617
<212> DNA
<213> Homo sapiens

<220>
<221> misc_feature
<222> (1)...(617)
<223> n = A,T,C or G

<400> 827
cgccagcgct gcaggagctg acatggaccc aaatcctcgg gccgccctgg agcgccaaca 60
gctccgcctt cgggagcggc aaaaattctt cgaggacatt ttacagccag agacagagtt 120
tgtctttcct ctgtcccatc cgcatctcga gtcgcagaga ccccccatag gtagtatctc 180
atccatggaa gtgaatgtgg acacactgga gcaagtagaa cttattgacc ttggggaccc 240
ggatgcagca gatgtgttct tgccttgcga agatcctcca ccaacccccc agtcgtctgg 300
gatggacaac catttggagg agctgagcct gccggtgcct acatcagaca ggaccacatc 360
taggacctct tctnctnctc ctncgactcc tncaccaacc tgcataagcc aaatccaagt 420
gatgatggag cagatacgcc cttggcacag tcngatnaga ggaggaaaag gggtnttgga 480
ngggcaaaan cttgannctg cagntagcaa tgggccctgc tanaantgnc caccttggtn 540
ttttccaatn nnacncaggc caccnaactt ttgganaaac caanttttnt tgcgnggccc 600

aaggggaagn ngnggat 617

<210> 828
<211> 448
<212> DNA
<213> Homo sapiens

<220>
<221> misc_feature
<222> (1)...(448)
<223> n = A,T,C or G

<400> 828
actgtcacct ttttaagtgg aaagaaatat agtgtggatg atttacactc aatgggagca 60
ggggatctgc taaactctat gtttgaattt agtgagaagc taaatgccct ccaacttagt 120
gatgaagaga tgagtttgtt tacagctgtt gtcctggtat ctgcagatcg atctggaata 180
gaaaacgtca gctctgtgga ggctttgcag gaaactctca ttcgtgcact aaggacctta 240
ataatgaaaa accatccaaa tgaggcctct atttttacaa aactgcttct aaagttgcca 300
gatcttcgat ctttaaacaa catgcactct gaggagctct tggcctttaa agntcaccct 360
taaggccttn gtttatttaa ncatgaactg atggtaactg nacctcngnc gcgaccacnc 420
taaggccaat tccananact gnccggcg 448

<210> 829
<211> 619
<212> DNA
<213> Homo sapiens

<220>
<221> misc_feature
<222> (1)...(619)
<223> n = A,T,C or G

<400> 829
   cgaggtactt ttaaagcagg gagtggggaa aagtattttg aggggacatt ttcatcatca 60
   gttcagcttt ttttttttgg ttgttgctct tttttggggg ggttgggttt gttggtttca 120
   ctgaaacatt taactacctg taaaatctaa acatggctgt tagtgtcaca ccaattcggg 180
   acacaaaatg gctaacactg gaagtatgta gagagttcca gagggggact tgctcacggc 240
   cagacacgga atgtaaattt gcacatcctt cgaaaagctg ccaagttgaa aatggacgag 300
   taatcgcctg ctttgattca ttgaaaggcc gttgctccag ggagaactgc aaatatcttc 360
   atccaccccc acatttaaaa acgcagttgg agataaatgg acgcaataac ttgattcagc 420
   agaagaacat ggccatgttg gnccagcaaa tgccactagn ccatgccatg atgcctggtg 480
   cccattacaa cccgngccat ngttcaattg nccaacttac cnccatgcnt aacagccgct 540
   ttannccttt tggacctttt ttccancttg gcccggcaaa attttccant ggccaattgg 600
   ttccgggant ccgggtcct 619

<210> 830
<211> 618
<212> DNA
<213> Homo sapiens

<220>
<221> misc_feature
<222> (1)...(618)

<223> n = A,T,C or G

<400> 830
ggtacaccct agccaacggg acaaatccta gagggtataa aatcatctct gctcagataa 60
tcatgactta gcaagaataa gggcaaaaaa tcctgttggc ttaacgtcac tgttccacct 120
ggtgtaatat ctctcatgac agtgacacca agggaagttg actaagtcac atgtaaatta 180
ggagtgtttt aaagaatgcc atagatgttg attcttaact gctacagata acctgtaatt 240
gagcagattt aaaattcagg catacttttc catttatcca agtgctttca tttttccaga 300
tggcttcaga agtaggctcg tgggcagggc gcagacctga tctttatagg gttgacatag 360
aaagcagtaa gttgtggggt gaaagggcag gttgtcttca aactctgtga ggtagaatcc 420
ttnnctatac ctccatgaac attgactcgt gtgttcagag cctttggcct ctntggngga 480
gtctngctnt ttgggctcct gggcatcctt ttgaatagtc actctgtaaa actngccann 540
gctttgaaac tgggtncttt acccanggtg naagggnctt tgttggcctt tanaagggtn 600
ggncatncct ccaaaacc 618

<210> 831
<211> 648
<212> DNA
<213> Homo sapiens

<220>
<221> misc_feature
<222> (1)...(648)
<223> n = A,T,C or G

<400> 831
acatgaaaga cacgtccaca tcacagttgc ccccaaactg cctgtgctcc tcgatggtgt 60
ctctccctcc agaaaacgca tgcttattga ccttggtttt gatctgcttg gccgtgtcgg 120
tgaggaagat ggaggagttg gggtcgctgg cactcatttt ggtctgggcg ccctgcaggg 180
ctgggaagaa ggtggagtgc aacagggctg gtttaggata gccgatcctg ggggcgacgt 240
cccttgtcat tctaaagtaa ggatcctggt caatggcaca tgggataagg cactggatat 300
ccgtcctgtc tcggaagatc tgtgggaatg agttgctgaa ggagggagca gcctggatgg 360
caggaaaact gatcttccca atgcagtcgc tgtcagtgaa acncgaaaaa tgcctttcac 420
tttggtttga aggtaacatg cctttttgaa tcttcaccac attttttgta gaaaccttgg 480
nccttnatnc cccatgtagn nccaggttca naanaatntt gaaaagnctt tggtggaagg 540
tcaaaancnc caggccaant aaaggncctt tggnaatttt ttcccnggnt ataactttnt 600
nggcctgggn ccaaggtcaa nggccctttc cnaannaact ttttnggn 648

<210> 832
<211> 689
<212> DNA
<213> Homo sapiens

<220>
<221> misc_feature
<222> (1)...(689)
<223> n = A,T,C or G

<400> 832
gtccccacga actggcctgg ccaagcaccc cacactggag ccatctcttc ctcatatttc 60
agcagtgcag ccggggggca gggaagggca ggcagggtct gttggggtct ctttttatcc 120
ttattcctcc cccgacctaa ttgtctttgt tctgtgatta ttgggggaca cccggctccc 180
tccagacaat gccagcataa atccatccat ccaaaggcag agaaccaaag gggccatgga 240

aggttctctg tgctcctcct acccttccag tgccctaggc ctggcgactg cccctgcctt 300
ttagacccgc ctccctttta tacctgctct tgntctactg agaaaagcct ctcagcaata 360
atgntttcta gtcacttcct ccgncttcgg gacgggcgtg cctggacact tgtaccttng 420
gcccgcgaac cacgcttaag gggcgaaatt ccaagcacnc ttggccggcc ggttaccttn 480
gtngggatnc ccaaccttng gnnncccaaa ccttgggcgg taaaccatng ggnccttaac 540
ctngngttcc ctgggggngn aaaantngta atttccgggt ttacccaatt ttccncccca 600
aacnttntcc caaancccgg gaaaaccctt aaaaggnggg aaaaancccc ttgggggggg 660
gccctnaann nggagggtgg ngcnttanc 689

<210> 833
<211> 726
<212> DNA
<213> Homo sapiens

<220>
<221> misc_feature
<222> (1)...(726)
<223> n = A,T,C or G

<400> 833
ggtactaatg tgaattgttc ctcagaaacg cttcttttcc atcctagtga gaagctggcc 60
ctgcaggtgg tggcagcaat ggtgttgtaa gatttcctcc cgtagttttt tctcctcatg 120
gatttgaatg aaatgccaat aacacgtcca ctttcaacgt gtagtttacg cggagcactt 180
tcgaggcctg gccgggttgg gcctacttct cacctgggcc tatcttctga actcgctagg 240
ttcttatcaa catttggggg ataactttgt atattttttt cattnggctt ttctttacca 300
gtttctgatt tttattctca atatattttt gctaaaacct atttcacaaa tnaccaccng 360
actgaaagtg tgtgnttact gatgcggccc ttgagcttcc atgggcgaaa ggagtgactt 420
ttgcagcngc cgtnaagaac ccgnaaatct ggtttnanag cnccanggaa agtngaccac 480
cnttangggg agcccccncg tangggggcg ctttgtaang cccnccnggg ggaacccccc 540
annnaccggt gggggtcctt aaaagnaana nanaccgggg gtctttaagc ttntttcctt 600
gggccacncc cccaaaannn gggnttttcc caatttntta anacnctntc ttgngggggg 660
tcctnggngg aaatggngga aaaaaangcc cnnntnnttg ttnggggngg gnaccncaan 720
gtggng 726

<210> 834
<211> 628
<212> DNA
<213> Homo sapiens

<220>
<221> misc_feature
<222> (1)...(628)
<223> n = A,T,C or G

<400> 834
ggtacgagag tgtagccaaa gtgagaggct gagagcaaag gagacatttt tttcagtttt 60
gagtcgagta tccagacaga ggcaaatcat tttgtttaac tttttattaa agtgtaacta 120
tagaaacaca tcaatgattt ttcacaagtg gagcactgtg catacaatcg gcaccccaga 180
agccccccgt cagattccct tccagttaac tacctctcca agggaaacca ctatcctgag 240
ttctaagcgc atagattagt ttctgtctgg tttggggaga tatataaatg gaattatgca 300
ttcttcgtat ctggttnctt ttcaccaata ttatgtttgt gagatttttg gtgcatgtat 360
ttgtacagnt ttgctgattt taggtgttgc gcctcattgg gaacagtttg ctataggttg 420
aagagaaaat ttgctcttcc ggtttantgg caccanggag canaatgccc ncagtgtntg 480

gnctcngata atgggtcgaa attgggangt gggctggacn tttttnactt gntctttctg 540
atctngantc ggttncctat tcnatatttg gntntcttcg gaattnnttg ntngaacttg 600
cctgggccng gctgttctan agggnnag 628

<210> 835
<211> 602
<212> DNA
<213> Homo sapiens

<220>
<221> misc_feature
<222> (1)...(602)
<223> n = A,T,C or G

<400> 835
ggtactgaaa tcacaagagc tataactgcc agagaaaaat taaatggggt cttcaagtag 60
tgactgagcc agcaaactaa gtggccaaga gggagacaag agcagctcct aaagaaggtt 120
gaagtcaagc aatctccgga acacagagga tctgaagcat ctgggcagag ccacaggcag 180
gcanggcaag gacacacagc acaccagagc agcaccgtcc ttcactgtgt gagagcaact 240
ctcaggctgc agaaccaatt gccatctcca ctgcctacag ctcaggtctc caactaccag 300
atagggagta aaaaacagtt tgattttatt cacctcaagt ctaaacacgg ngggaaaaaa 360
aactggtcta nagatggaaa ctatatttca tgggggttta ttaaacagag aaagaggaga 420
attttcacat ttcacagggc ttttcntgaa ataaagactt gatctgaaaa ggcaccctta 480
tggcangctt taacttccta agntngggna gnncccaaat tttccannaa tcttgggacc 540
ncttgcccag tngatttttt ttaaataact nagctnaatt gntnggntaa tttnataana 600
ng 602

<210> 836
<211> 355
<212> DNA
<213> Homo sapiens

<220>
<221> misc_feature
<222> (1)...(355)
<223> n = A,T,C or G

<400> 836
acacaatgct tctgccagtc ctattcaggg ccaaggacat gtgcttataa ccatctgcca 60
aattttccaa actgtcacag taacaaccat caaattttag cagatctact ccccagtcag 120
caaaggtctg ggcatcaatg tcgtagtatc caaaactccc agggaagcct gcgcaggttt 180
tatttccaac atctgcataa atccctagct tcagtccttt gctgtgaaca taattagcta 240
gctggcgaat cccatgagga aagcgctgag ggtctgcctg aagtctgcct tctgaatctc 300
tttggggagc catccaacag tcatcaatgc agaggtacct cggncgngac cacgc 355

<210> 837
<211> 611
<212> DNA
<213> Homo sapiens

<220>
<221> misc_feature
<222> (1)...(611)

<223> n = A,T,C or G

<400> 837
ggttttttt ttcgtgattg tattcccata aagctttatt tgtggactct aaaatttgaa 60
ttttatgtga ttttcacata tcacaaacat tcttcttctt ttaatttttc taaccattaa 120
aattataaaa aactttctta tttttgcagg ccatacaaaa ttaggcagtg ggccaaatct 180
ggccgctagt ttagaaggtc cacggtagtc tcgctcgcag gcatggcagt tgcagctggc 240
tggggcaccc tggttctcct ccacaaggcc tttcatcctc cagaagtctg aattggcctt 300
gttcatggca ctttcagggc agcattccaa gaggtggaag ggagagtctg caaagacttc 360
tgaggctggc tccagacctc actcagtatc cccactgctc catttcagtc agagtnaagt 420
cactagtnct gcccagactc aagggatgaa gggaactgnc tntanctcat gatgaagata 480
acntgtgaaa tactgggggc tgagtttttc anttancncc agggagtaat tttcatggnt 540
taaanggcat tcccccttat ttttgaagcc ntaanttcng gcntttanng ggaantaatt 600
aaccnccctt a 611

<210> 838
<211> 650
<212> DNA
<213> Homo sapiens

<220>
<221> misc_feature
<222> (1)...(650)
<223> n = A,T,C or G

<400> 838
ggtacttcca cctcgggcac attttgggaa gttgcattcc tttgtcttca aactgtgaag 60
catttacaga aacgcatcca gcaagaatat tgtccctttg agcagaaatt tatctttcaa 120
agaggtatat ttgaaaaaaa aaaaagtata tgtgaggatt tttattgatt ggggatcttg 180
gagtntttca ttgtcgctat tgatttttac ttcaatgggc tcttccaaca aggaagaagc 240
ttgctggtag cacttgctac cctgagttca tccaggccca actgtgagca aggagcacaa 300
gccacaagtc ttccagagga tgcttgattc cagtggttct gcttcaaggc tttcactgca 360
anacactaaa gatccaagaa ggccttcatg gcccncncca ngcccggatc gggtanctgg 420
ccgggcnggn cngtnnnaaa gggcnaaatt tcngcacact tggccgnccg ttactaagtn 480
ggantccnaa gcttggntan ccaagctttg gngnaattct ngggcatann nctgggtncc 540
ttgnnggnaa aatgntantc ccgtnnnaaa ttcccttcan cnnanctgan cctgaaagct 600
ttaantgggn aaacnttggg ggtccctaat tngggggacn taacntctnt 650

<210> 839
<211> 626
<212> DNA
<213> Homo sapiens

<220>
<221> misc_feature
<222> (1)...(626)
<223> n = A,T,C or G

<400> 839
actaaacgag caggtgaagg aggctgaagg atcgtctgct gaatacaaga aagaaattga 60
ggaactaaag gaactgctac ccgaaattag agagaagata gaagatgcaa aggagtctca 120
gcgtagtggg aatgtagctg aactggctct gaaagctact ctggtggaga gttctacttc 180
aggtttcact cctggtggag gaggctcttc agtctccatg attgccagta gaaagccaac 240

<400> 842
ggtacacttg ctaaatttga atgggcangc agcaaactct gggaagactt ctaatgcttt 60
acgatacaag cgaactgcct cttcaatgtt tccctgttct cgtttgatat tggctaggtt 120
attcagagag tctgcatggg tgggacacag acggagagct gtattataac aatcttctgc 180
ttcagcaacc tgtcaaaaat gcgtgcctct ttcaagacat ttcctaaatt gatataagca 240
tccagaaagt ttgggtcaag ggtgacagcc ttttcaaagt gatgaattgc aagccaaatt 300
tccccttgtg cattgaaaac acagccaaga ttactccaag ctactgcaaa gttcggttgc 360
gtctcaattg ctttcaaata acatgccttg gcttcttcca agcgacccaa ggcttttaca 420
ggtncccagg tcactgcgaa cacagtacct gcccggcggc cgttcaaang gcgaaattca 480
gcacacttgc ggncgtanta gtggantncn agcntcggnc caacttgggn ntataatggg 540
canaactggt ccctggggga aantggtnnc cnntaccatt tcnccacttn cgaccggaag 600
cttaaangg 609

<210> 843
<211> 610
<212> DNA
<213> Homo sapiens

<220>
<221> misc_feature
<222> (1)...(610)
<223> n = A,T,C or G

<400> 843
ggtttttttt cgcaggtatt tcctctgctt taatagacaa ttttagaaag acatgttaac 60
gggggaaaat cacacaatac taaggatctg agggccataa acatcacata tgttgagttt 120
gcttttagtt ttgtttccaa cagttcttaa ccaatgttcc tggctgtaat ctaggtgcta 180
gacgcactgc aaatcctcga aagtgtttaa gatgaaagag caatacactt aagatcttca 240
aaagtttaca ttaacagaat aagcattagc tccttttaac acacacacac aactaaatta 300
acaaatgaaa tgtgtctact tttatatatg cccataaagc agacacttaa cattgaaatt 360
tactatttta gattttcact cctttaagag ctatcaatat agacactnaa gataattcac 420
atttnaaaaa ttatctacct ggaagaatag aacttcttta agaaggaaaa agnaaaagct 480
ggtgaaacca aggattgcct ggggtnggaa ggaccgnttt naacctgggc cttaaatgnc 540
ntgagnacaa ttgattggtc nnncttgggc tntnttggta acaccggcct tcanggtttt 600
cttgacccnc 610

<210> 844
<211> 675
<212> DNA
<213> Homo sapiens

<220>
<221> misc_feature
<222> (1)...(675)
<223> n = A,T,C or G

<400> 844
ggtacacctg aattccaggc caatgaagtt cggaaagtga agaaatatga acagggattc 60
atcacagacc ctgtggtcct cagccccaag gatcgcgtgc gggatgtttt tgaggccaag 120
gcccggcatg gtttctgcgg tatcccaatc acagacacag gccggatggg gagccgcttg 180
gtgggcatca tctcctccag ggacattgat tttctcaaag aggaggaaca tgactgtttc 240
ttggaagaga taatgacaaa gagggaagac ttggtggtag cccctgcagg catcacactg 300

aaggaggcaa atgaaattct gcagcgcagc aagaagggaa agttgcccat tgtaaatgaa 360
gatgatgagc ttgtggccat cattgcccgg acagacctga agaagaatcg ggactaccca 420
ctagccttcc aaagatgccc aagaaaccag cttgcttgtg ttgggcaagc cattgggcac 480
ttcattgaag gattgaccaa ggttttangg ccttggacct ttggtttggc cccaaggctt 540
tggtgttgga attgtaaatg gggtttttgg gacttttttt ncccangggg aaaatttccc 600
tttttttcnc nanttccaat tttgngatcc aaagtnccct tggccccggg gccgggcccg 660
tttcaaaaan gggcc 675

<210> 845
<211> 620
<212> DNA
<213> Homo sapiens

<220>
<221> misc_feature
<222> (1)...(620)
<223> n = A,T,C or G

<400> 845
acagcctaag acacaaggat ctaggcgaag tagccgccaa ataaaaaaac gaagggtcat 60
atcagattct gagagtgaca ttggtggctc tgatgtggaa tttaagccag acactaagga 120
ggaaggaagc agtgatgaaa taagcagtgg agtgggggat agtgagagtg aaggcctgaa 180
cagccctgcc aaagttgctc gaaagcggaa gagaatggtg actggaaatg gctctcttaa 240
aaggaaaagc tctaggaagg aaacgccctc agccaccaaa caagcaacta gcatttcatc 300
agaaaccaag aatactttga gagctttctc tgcccctcaa aattctgaat cccaagccca 360
cgttagtgga ggtggtgatg acagtagtcg cctactgntt ggtatcatga aactttagaa 420
tggcttaagg gaggaaaaga gaanaaatga ncncaggang aaggcctgat caccccgatt 480
ttgatgcctt tnccctntnt gggncctgga ggatttcntc aaatctttgg anccttggcc 540
nnnacccccn ttangggcgn aatccagccc ttggnggncc gttcttaggg gatcncagct 600
tgggnccaac tttggggtan 620

<210> 846
<211> 617
<212> DNA
<213> Homo sapiens

<220>
<221> misc_feature
<222> (1)...(617)
<223> n = A,T,C or G

<400> 846
caggtacata aagcagattc aagggttaaa ataaaaacag aattttggag tgtggtcaaa 60
taaggtgcac agattccaga accctcagag ggcctgctgg ccctctccag acattctgtg 120
tccgtggtgc aggagctggg cccgtcccta acagctccgc actggcttag tgcagtggtg 180
ctcacagttt caggaactac taggtgaagt gtctggctca agtctgccaa gtgtcttcac 240
tccatcgtca gaagtggagc actatcccta ggttcgattc ccatgaaata ttttatgatt 300
tccatcctct ttgcccgctc ttccaaataa ggccctgtga tgccaacnaa gggggcatgg 360
ttgagggtct aaggctctca ttagggccta attctgtgtg gatatnaaca catgacagac 420
acttgctgca ncattnanga catttaaggc agaggggtca tttaangnta cttttncaaa 480
ttaatatttn gnggatnggg cagttcttac ctgnnactgg tnnttattgg ggnaattttt 540
taccangggg ctgtctattt taaatngctt nggnattacn ngtttngnac cctcnaannn 600
ctngggaaac ttnntnc 617

<210> 847
<211> 638
<212> DNA
<213> Homo sapiens

<220>
<221> misc_feature
<222> (1)...(638)
<223> n = A,T,C or G

<400> 847
   ggtacaagct tttttttttt tttttttttt tttttttagc ctttccttat gagcatgcct 60
   gtgttgggtt gacagtgagg gtaataatga cttgttggtt gattgtagat attgggctgt 120
   taattgtcag ttcagtgttt taatctgacg caggcttatg cggaggagaa tgttttcatg 180
   ttacttatac taacattagt tcttctatag ggtgatagat tggtccaatt gggtgtgagg 240
   agttcagtta tatgtttggg attttttagg tagtgggtgt tgagcttgaa cgctttctta 300
   attggtggct gcttttaggc ctactatggg tgttaaattt tttactctct ctacaagggt 360
   ttttcctaan tggccaaaag agctggtcct tctttgggac taaccagtta aattttacca 420
   ngggggaatt taanaggggt tcttgggggc caaattttaa aggtcngaac ttaagantct 480
   tatcttggga caanccagnt nttcaccagg cnttggnaag ggtttngtcn gcctttaccn 540
   taaaaatctt tcccnctant ttnctaccnn aaccgggggg cncttttaaa cgnnntttan 600
   ggganccccc ccnggtttng gggggttnaa ctttgcnn 638

<210> 848
<211> 347
<212> DNA
<213> Homo sapiens

<400> 848
ggtttttttt tttttcaaca gacaaaaaaa gtttattgaa tacaaaactc aaaggcatca 60
acagtcctgg gcccaagaga tccatggcag gaagtcaaga gttctgcttc agggtcggtc 120
tgggcagccc tggaagaagt cattgcacat gacagtgatg agtgccagga aaacagcata 180
ctcctggaag tccacctgct ggtcactgtt ctcatccagg ctgcccatca gcttcttcag 240
cccctcctca tccactttct cccccacaaa gctgggcagc tccttgtgca gaagttcctt 300
catttccccc ttactcagct tgaacttgtc gccctcttgg caggagt 347

<210> 849
<211> 624
<212> DNA
<213> Homo sapiens

<220>
<221> misc_feature
<222> (1)...(624)
<223> n = A,T,C or G

<400> 849
actgctggaa atacaatctt cagcaggtgc tgatgcaggc tggaatttgg ctggagcgga 60
ccctcccatt ggtttagaag ttgctttagt gggtggagca ggcttggctg gcatgctaac 120
tttggctttc tctagcatgg ccaatacctg atctttagaa gttggcttta gtttcccagt 180
agccttggcc atttttttcat atcctaaatg catcatgaag aatggcaagg catcttgggc 240
cttctttcgc acatctccat ttcgatcttc taggcaggag tagagatgag gaacacaaag 300

<223> n = A,T,C or G

<400> 830
ggtacaccct agccaacggg acaaatccta gagggtataa aatcatctct gctcagataa 60
tcatgactta gcaagaataa gggcaaaaaa tcctgttggc ttaacgtcac tgttccacct 120
ggtgtaatat ctctcatgac agtgacacca agggaagttg actaagtcac atgtaaatta 180
ggagtgtttt aaagaatgcc atagatgttg attcttaact gctacagata acctgtaatt 240
gagcagattt aaaattcagg catacttttc catttatcca agtgctttca tttttccaga 300
tggcttcaga agtaggctcg tgggcagggc gcagacctga tctttatagg gttgacatag 360
aaagcagtaa gttgtggggt gaaagggcag gttgtcttca aactctgtga ggtagaatcc 420
ttnnctatac ctccatgaac attgactcgt gtgttcagag cctttggcct ctntggngga 480
gtctngctnt ttgggctcct gggcatcctt ttgaatagtc actctgtaaa actngccann 540
gctttgaaac tgggtncttt acccanggtg naagggnctt tgttggcctt tanaagggtn 600
ggncatncct ccaaaacc 618

<210> 831
<211> 648
<212> DNA
<213> Homo sapiens

<220>
<221> misc_feature
<222> (1)...(648)
<223> n = A,T,C or G

<400> 831
acatgaaaga cacgtccaca tcacagttgc ccccaaactg cctgtgctcc tcgatggtgt 60
ctctccctcc agaaaacgca tgcttattga ccttggtttt gatctgcttg gccgtgtcgg 120
tgaggaagat ggaggagttg gggtcgctgg cactcatttt ggtctgggcg ccctgcaggg 180
ctgggaagaa ggtggagtgc aacagggctg gtttaggata gccgatcctg ggggcgacgt 240
cccttgtcat tctaaagtaa ggatcctggt caatggcaca tgggataagg cactggatat 300
ccgtcctgtc tcggaagatc tgtgggaatg agttgctgaa ggagggagca gcctggatgg 360
caggaaaact gatcttccca atgcagtcgc tgtcagtgaa acncgaaaaa tgcctttcac 420
tttggtttga aggtaacatg ccttttttgaa tcttcaccac atttttgta gaaaccttgg 480
nccttnatnc cccatgtagn nccaggttca naanaatntt gaaaagnctt tggtggaagg 540
tcaaaancnc caggccaant aaaggncctt tggnaatttt ttcccnggnt ataactttnt 600
nggcctgggn ccaaggtcaa nggccctttc cnaannaact ttttnggn 648

<210> 832
<211> 689
<212> DNA
<213> Homo sapiens

<220>
<221> misc_feature
<222> (1)...(689)
<223> n = A,T,C or G

<400> 832
gtccccacga actggcctgg ccaagcaccc cacactggag ccatctcttc ctcatatttc 60
agcagtgcag ccggggggca gggaagggca ggcagggtct gttggggtct ctttttatcc 120
ttattcctcc cccgacctaa ttgtctttgt tctgtgatta ttgggggaca cccggctccc 180
tccagacaat gccagcataa atccatccat ccaaaggcag agaaccaaag gggccatgga 240

gataaggtct gtaggggtgg aacgaagagt aggtagtttc tcaaccagcc agcccagaag 360
ctcttgcctc aagaaaggat tttcttttga gctcttcaga aagaacttct ccttcaacca 420
ttccttnatg cccantctgg ttntggccaa gcatttcaca ggtcgctang ggcaagcact 480
tcgaacattg gtcttgcttg ctccaaggac ttgggaatna anggggangc ctnaaatttt 540
ttancgggtg gcttaaaatt tggggccnan ggttattgcc aaattgtttc cagggatttn 600
aacggtttgg tggncctcgg cccg 624

<210> 850
<211> 636
<212> DNA
<213> Homo sapiens

<220>
<221> misc_feature
<222> (1)...(636)
<223> n = A,T,C or G

<400> 850
acaagttatc aaacttctgt ttggtaacag aatcattgac gttcatggcc ggaacacaga 60
gcttcccagc tttggagagc tgatacagcc tgtgaacacc agtcacgctc tcttccacaa 120
tgcctcggat cttcttaaac acgtttggat acttcttata aacccagtgg gttaagtctc 180
ccccatcatc caggatcatg ttggcctgcc acccatccat gttcacacag cggtcaatac 240
accaccagaa gtcatcttct gactcgccct tccaagcgaa cactgcaact ccagcctcag 300
ccagtgctgc agctacttca ttctgagttg agtagatgtt acaagcagac cagcggcact 360
gagcccccag agcacagagt gtctcaatca acacccgctg tctgggctgt gatgtgtgta 420
tcttnggccg ngaacangct taagggcgaa ttncacacaa cttggcggcc ggtacttagt 480
gggaatccan cttngntacc caagcttggg cgtaantcat ngggcatang cntggttcct 540
nggggaaant ggtatncggt tanaanttcc accaacnttc naancccgga agnnttaaan 600
gntaaaanct tngggggcct aantgagnng anntac 636

## Description

This application is based on Provisional Application No. 60/088,801, filed June 10, 1998, which is hereby incorporated herein by reference.

### Field of the Invention

The present invention provides nucleic acid sequences and proteins encoded thereby, as well as probes derived from the nucleic acid sequences, antibodies directed to the encoded proteins, and diagnostic methods for detecting cancerous cells, especially colon cancer cells.

### Background of the Invention

Colorectal carcinoma is a malignant neoplastic disease. There is a high incidence of colorectal carcinoma in the Western world, particularly in the United States. Tumors of this type often metastasize through lymphatic and vascular channels. Many patients with colorectal carcinoma eventually die from this disease. In fact, it is estimated that 62,000 persons in the United States alone die of colorectal carcinoma annually.

However, if diagnosed early, colon cancer may be treated effectively by surgical removal of the cancerous tissue. Colorectal cancers originate in the colorectal epithelium and typically are not extensively vascularized (and therefore not invasive) during the early stages of development. Colorectal cancer is thought to result from the clonal expansion of a single mutant cell in the epithelial lining of the colon or rectum. The transition to a highly vascularized, invasive and ultimately metastatic cancer which spreads throughout the body commonly takes ten years or longer. If the cancer is detected prior to invasion, surgical removal of the cancerous tissue is an effective cure. However, colorectal cancer is often detected only upon manifestation of clinical symptoms, such as pain and black tarry stool. Generally, such symptoms are present only when the disease is well established, often after metastasis has occurred, and the prognosis for the patient is poor, even after surgical resection of the cancerous tissue. Early detection of colorectal cancer therefore is important in that detection may significantly reduce its morbidity.

Invasive diagnostic methods such as endoscopic examination allow for direct visual identification, removal, and biopsy of potentially cancerous growths such as polyps. Endoscopy is expensive, uncomfortable, inherently risky, and therefore not a practical tool for screening populations to identify those with colorectal cancer. Non-invasive analysis of stool samples for characteristics indicative of the presence of colorectal cancer or precancer is a preferred alternative for early diagnosis, but no known diagnostic method is available which reliably achieves this goal. A reliable, non-invasive, and accurate technique for diagnosing colon cancer at an early stage would help save many lives.

### Summary of the Invention

The present invention provides nucleic acid sequences and proteins encoded thereby, as well as probes derived from the nucleic acid sequences, antibodies directed to the encoded proteins, and diagnostic methods for detecting cancerous cells, especially colon cancer cells.

In one aspect, the invention provides an isolated nucleic acid comprising a nucleotide sequence which hybridizes under stringent conditions to a sequence of SEQ ID Nos. 1-127 or a sequence complementary thereto. In a related embodiment, the nucleic acid is at least about 80% or about 100% identical to a sequence corresponding to at least about 12, at least about 15, at least about 25, or at least about 40 consecutive nucleotides up to the full length of one of SEQ ID Nos. 1-127 or a sequence complementary thereto or up to the full length of the gene of which said sequence is a fragment. In certain embodiments, a nucleic acid of the present invention includes at least about five, at least about ten, or at least about twenty nucleic acids from a region designated as novel in Table 2. In certain other embodiments, a nucleic acid of the present invention includes at least about five, at least about ten, or at least about twenty nucleotides which are not included in corresponding clones whose accession numbers are listed in Table 2.

In one embodiment, the invention provides a nucleic acid comprising a nucleotide sequence which hybridizes under stringent conditions to a sequence of SEQ ID Nos. 1-127 or a sequence complementary thereto, and a transcriptional regulatory sequence operably linked to the nucleotide sequence to render the nucleotide sequence suitable for use as an expression vector. In another embodiment, the nucleic acid may be included in an expression vector capable of replicating in a prokaryotic or eukaryotic cell. In a related embodiment, the invention provides a host cell transfected with the expression vector.

In another embodiment, the invention provides a transgenic animal having a transgene of a nucleic acid comprising a nucleotide sequence which hybridizes under stringent conditions to a sequence of SEQ ID Nos. 1-127 or a sequence complementary thereto incorporated in cells thereof. The transgene modifies the level of expression of the nucleic acid, the stability of an mRNA transcript of the nucleic acid, or the activity of the encoded product of the nucleic acid.

In yet another embodiment, the invention provides substantially pure nucleic acid which hybridizes under stringent conditions to a nucleic acid probe corresponding to at least about 12, at least about 15, at least about 25, or at least about 40 consecutive nucleotides up to the full length of one of SEQ ID Nos. 1-127 or a sequence complementary thereto or up to the full length of the gene of which said sequence is a fragment. The invention also provides an antisense oligonucleotide analog which hybridizes under stringent conditions to at least 12, at least 25, or at least 50 consecutive nucleotides of one of SEQ ID Nos. 1-850 up to the full length of one of SEQ ID Nos. 1-850 or a sequence complementary thereto or up to the full length of the gene of which said sequence is a fragment, and which is resistant to cleavage by a nuclease, preferably an endogenous endonuclease or exonuclease.

In another embodiment, the invention provides a probe/primer comprising a substantially purified oligonucleotide, said oligonucleotide containing a region of nucleotide sequence which hybridizes under stringent conditions to at least about 12, at least about 15, at least about 25, or at least about 40 consecutive nucleotides of sense or antisense sequence selected from SEQ ID Nos. 1-127 up to the full length of one of SEQ ID Nos. 1-127 or a sequence complementary thereto or up to the full length of the gene of which said sequence is a fragment. In preferred embodiments, the probe selectively hybridizes with a target nucleic acid. In another embodiment, the probe may include a label group attached thereto and able to be detected. The label group may be selected from radioisotopes, fluorescent compounds, enzymes, and enzyme co-factors. The invention further provides arrays of at least about 10, at least about 25, at least about 50, or at least about 100 different probes as described above attached to a solid support.

In yet another embodiment, the invention pertains to a method of determining the phenotype of a cell, comprising detecting the differential expression, relative to a normal cell, of at least one nucleic acid which hybridizes under stringent conditions to one of SEQ ID Nos. 1-850, wherein the nucleic acid is differentially expressed by at least a factor of two, at least a factor of five, at least a factor of twenty, or at least a factor of fifty.

In another aspect, the invention provides polypeptides encoded by the subject nucleic acids. In one embodiment, the invention pertains to a polypeptide including an amino acid sequence encoded by a nucleic acid comprising a nucleotide sequence which hybridizes under stringent conditions to a sequence of SEQ ID Nos. 1-127 or a sequence complementary thereto, or a fragment comprising at least about 25, or at least about 40 amino acids thereof. Further provided are antibodies immunoreactive with these polypeptides.

In still another aspect, the invention provides diagnostic methods. In one embodiment, the invention pertains to a method for determining the phenotype of cells from a patient by providing a nucleic acid probe comprising a nucleotide sequence having at least 12, at least about 15, at least about 25, or at least about 40 consecutive nucleotides represented in a sequence of SEQ ID Nos. 1-850 up to the full length of one of SEQ ID Nos. 1-850 or a sequence complementary thereto or up to the full length of the gene of which said sequence is a fragment, obtaining a sample of cells from a patient, providing a second sample of cells substantially all of which are non-cancerous, contacting the nucleic acid probe under stringent conditions with mRNA of each of said first and second cell samples, and comparing (a) the amount of hybridization of the probe with mRNA of the first cell sample, with (b) the amount of hybridization of the probe with mRNA of the second cell sample, wherein a difference of at least a factor of two, at least a factor of five, at least a factor of twenty, or at least a factor of fifty in the amount of hybridization with the mRNA of the first cell sample as compared to the amount of hybridization with the mRNA of the second cell sample is indicative of the phenotype of cells in the first cell sample. Determining the phenotype includes determining the genotype, as the term is used herein.

In another embodiment, the invention provides a test kit for identifying an transformed cells, comprising a probe/primer as described above, for measuring a level of a nucleic acid which hybridizes under stringent conditions to a nucleic acid of SEQ ID Nos. 1-850 in a sample of cells isolated from a patient. In certain embodiments, the kit may further include instructions for using the kit, solutions for suspending or fixing the cells, detectable tags or labels, solutions for rendering a nucleic acid susceptible to hybridization, solutions for lysing cells, or solutions for the purification of nucleic acids.

In another embodiment, the invention provides a method of determining the phenotype of a cell, comprising detecting the differential expression, relative to a normal cell, of at least one protein encoded by a nucleic acid which hybridizes under stringent conditions to one of SEQ ID Nos. 1-850, wherein the protein is differentially expressed by at least a factor of two, at least a factor of five, at least a factor of twenty, or at least a factor of fifty. In one embodiment, the level of the protein is detected in an immunoassay. The invention also pertains to a method for determining the presence or absence of a nucleic acid which hybridizes under stringent conditions to one of SEQ ID Nos. 1-127 in a cell, comprising contacting the cell with a probe as described above. The invention further provides a method for determining the presence of absence of a subject polypeptide encoded by a nucleic acid which hybridizes under stringent conditions to one of SEQ ID Nos. 1-127 in a cell, comprising contacting the cell with an antibody as described above. In yet another embodiment, the invention provides a method for determining the presence of an aberrant mutation (e.g., deletion, insertion, or substitution of nucleic acids) or aberrant methylation in a gene which hybridizes under stringent conditions to a sequence of SEQ ID Nos. 1-383 or a sequence complementary thereto, comprising collecting a sample of cells from a patient, isolating nucleic acid from the cells of the sample, contacting the nucleic acid sample with one or more primers which specifically hybridize to a nucleic acid sequence of SEQ ID Nos. 1-850 under conditions such that hybridization and amplification of the nucleic acid occurs, and comparing the presence, absence, or size of an amplification product to the amplification product of a normal cell.

In one embodiment, the invention provides a test kit for identifying transformed cells, comprising an antibody specific for a protein encoded by a nucleic acid which hybridizes under stringent conditions to any one of SEQ Nos. 1-850. In certain embodiments, the kit further includes instructions for using the kit. In certain embodiments, the kit may further include instructions for using the kit, solutions for suspending or fixing the cells, detectable tags or labels, solutions for rendering a polypeptide susceptible to the binding of an antibody, solutions for lysing cells, or solutions for the purification of polypeptides.

In yet another aspect, the invention provides pharmaceutical compositions including the subject nucleic acids. In one embodiment, an agent which alters the level of expression in a cell of a nucleic acid which hybridizes under stringent conditions to one of SEQ ID Nos. 1-850 or a sequence complementary thereto is identified by providing a cell, treating the cell with a test agent, determining the level of expression in the cell of a nucleic acid which hybridizes under stringent conditions to one of SEQ ID Nos. 1-850 or a sequence complementary thereto, and comparing the level of expression of the nucleic acid in the treated cell with the level of expression of the nucleic acid in an untreated cell, wherein a change in the level of expression of the nucleic acid in the treated cell relative to the level of expression of the nucleic acid in the untreated cell is indicative of an agent which alters the level of expression of the nucleic acid in a cell. The invention further provides a pharmaceutical composition comprising an agent identified by this method. In another embodiment, the invention provides a pharmaceutical composition which includes a polypeptide encoded by a nucleic acid having a nucleotide sequence that hybridizes under stringent conditions to one of SEQ ID Nos. 1-850 or a sequence complementary thereto. In one embodiment, the invention pertains to a pharmaceutical composition comprising a nucleic acid including a sequence which hybridizes under stringent conditions to one of SEQ ID Nos. 1-850 or a sequence complementary thereto.

### Brief Description of the Figure

The figure depicts an exemplary assay result for determining differential expression of gene products in cells.

### Detailed Description of the Invention

The invention relates to nucleic acids having the disclosed nucleotide sequences (SEQ ID Nos. 1-850), as well as full length cDNA, mRNA, and genes corresponding to these sequences, and to polypeptides and proteins encoded by these nucleic acids and genes and portions thereof.

Also included are nucleic acids that encode polypeptides and proteins encoded by the nucleic acids of SEQ ID Nos. 1-850. The various nucleic acids that can encode these polypeptides and proteins differ because of the degeneracy of the genetic code, in that most amino acids are encoded by more than one triplet codon. The identity of such codons is well known in this art, and this information can be used for the construction of the nucleic acids within the scope of the invention.

Nucleic acids encoding polypeptides and proteins that are variants of the polypeptides and proteins encoded by the nucleic acids and related cDNA and genes are also within the scope of the invention. The variants differ from wild-type protein in having one or more amino acid substitutions that either enhance, add, or diminish a biological activity of the wild-type protein. Once the amino acid change is selected, a nucleic acid encoding that variant is constructed according to the invention.

The following detailed description discloses how to obtain or make full-length cDNA and human genes corresponding to the nucleic acids, how to express these nucleic acids and genes, how to identify structural motifs of the genes, how to identify the function of a protein encoded by a gene corresponding to an nucleic acid, how to use nucleic acids as probes in mapping and in tissue profiling, how to use the corresponding polypeptides and proteins to raise antibodies, and how to use the nucleic acids, polypeptides, and proteins for therapeutic and diagnostic purposes.

The sequences investigated herein have been found to be differentially expressed in samples obtained from colon cancer cell lines and/or colon cancer tissue. However, it is also believed that these sequences may also have utility with other types of cancer.

Accordingly, certain aspects of the present invention relate to nucleic acids differentially expressed in tumor tissue, especially colon cancer cell lines, polypeptides encoded by such nucleic acids, and antibodies immunoreactive with these polypeptides, and preparations of such compositions. Moreover, the present invention provides diagnostic and therapeutic assays and reagents for detecting and treating disorders involving, for example, aberrant expression of the subject nucleic acids.

### I. General

This invention relates in part to novel methods for identifying and/or classifying cancerous cells present in a human tumors, particularly in solid tumors, e.g., carcinomas and sarcomas, such as, for example, breast or colon cancers. The method uses genes that are differentially expressed in cancer cell lines and/or cancer tissue compared with related normal cells, such as normal colon cells, and thereby identifies or classifies tumor cells by the upregulation and/or downregulation of expression of particular genes, an event which is implicated in tumorigenesis.

Upregulation or increased expression of certain genes such as oncogenes, act to promote malignant growth. Downregulation or decreased expression of genes such as tumor suppressor genes promotes malignant growth. Thus, alteration in the expression of either type of gene is a potential diagnostic indicator for determining whether a subject is at risk of developing or has cancer, e.g., colon cancer.

Accordingly, in one aspect, the invention also provides biomarkers, such as nucleic acid markers, for human tumor cells, e.g., for colon cancer cells. The invention also provides proteins encoded by these nucleic acid markers.

The invention also features methods for identifying drugs useful for treatment of such cancer cells, and for treatment of a cancerous condition, such as colon cancer. Unlike prior methods, the invention provides a means for identifying cancer cells at an early stage of development, so that premalignant cells can be identified prior to their spreading throughout the human body. This allows early detection of potentially cancerous conditions, and treatment of those cancerous conditions prior to spread of the cancerous cells throughout the body, or prior to development of an irreversible cancerous condition.

### II. Definitions

For convenience, the meaning of certain terms and phrases used in the specification, examples, and appended claims, are provided below.

The term "an aberrant expression", as applied to a nucleic acid of the present invention, refers to level of expression of that nucleic acid which differs from the level of expression of that nucleic acid in healthy tissue, or which differs from the activity of the polypeptide present in a healthy subject. An activity of a polypeptide can be aberrant because it is stronger than the activity of its native counterpart. Alternatively, an activity can be aberrant because it is weaker or absent relative to the activity of its native counterpart. An aberrant activity can also be a change in the activity; for example, an aberrant polypeptide can interact with a different target peptide. A cell can have an aberrant expression level of a gene due to overexpression or underexpression of that gene.

The term "agonist", as used herein, is meant to refer to an agent that mimics or upregulates (e.g., potentiates or supplements) the bioactivity of a protein. An agonist can be a wild-type protein or derivative thereof having at least one bioactivity of the wild-type protein. An agonist can also be a compound that upregulates expression of a gene or which increases at least one bioactivity of a protein. An agonist can also be a compound which increases the interaction of a polypeptide with another molecule, e.g., a target peptide or nucleic acid.

The term "allele", which is used interchangeably herein with "allelic variant", refers to alternative forms of a gene or portions thereof. Alleles occupy the same locus or position on homologous chromosomes. When a subject has two identical alleles of a gene, the subject is said to be homozygous for that gene or allele. When a subject has two different alleles of a gene, the subject is said to be heterozygous for the gene. Alleles of a specific gene can differ from each other in a single nucleotide, or several nucleotides, and can include substitutions, deletions, and/or insertions of nucleotides. An allele of a gene can also be a form of a gene containing mutations.

The term "allelic variant of a polymorphic region of a gene" refers to a region of a gene having one of several nucleotide sequences found in that region of the gene in other individuals.

"Antagonist" as used herein is meant to refer to an agent that downregulates (e.g., suppresses or inhibits) at least one bioactivity of a protein. An antagonist can be a compound which inhibits or decreases the interaction between a protein and another molecule, e.g., a target peptide or enzyme substrate. An antagonist can also be a compound that downregulates expression of a gene or which reduces the amount of expressed protein present.

The term "antibody" as used herein is intended to include whole antibodies, e.g., of any isotype (IgG, IgA, IgM, IgE, etc), and includes fragments thereof which are also specifically reactive with a vertebrate, e.g., mammalian, protein. Antibodies can be fragmented using conventional techniques and the fragments screened for utility in the same manner as described above for whole antibodies. Thus, the term includes segments of proteolytically-cleaved or recombinantly-prepared portions of an antibody molecule that are capable of selectively reacting with a certain protein. Nonlimiting examples of such proteolytic and/or recombinant fragments include Fab, F(ab')2, Fab', Fv, and single chain antibodies (scFv) containing a V[L] and/or V[H] domain joined by a peptide linker. The scFv's may be covalently or non-covalently linked to form antibodies having two or more binding sites. The subject invention includes polyclonal, monoclonal, or other purified preparations of antibodies and recombinant antibodies.

The phenomenon of "apoptosis" is well known, and can be described as a programmed death of cells. As is known, apoptosis is contrasted with "necrosis", a phenomenon when cells die as a result of being killed by a toxic material, or other external effect. Apoptosis involves chromatic condensation, membrane blebbing, and fragmentation of DNA, all of which are generally visible upon microscopic examination.

A disease, disorder, or condition "associated with" or "characterized by" an aberrant expression of a nucleic acid refers to a disease, disorder, or condition in a subject which is caused by, contributed to by, or causative of an aberrant level of expression of a nucleic acid.

As used herein the term "bioactive fragment of a polypeptide" refers to a fragment of a full-length polypeptide, wherein the fragment specifically agonizes (mimics) or antagonizes (inhibits) the activity of a wild-type polypeptide. The bioactive fragment preferably is a fragment capable of interacting with at least one other molecule, e.g., protein, small molecule, or DNA, which a full length protein can bind.

"Biological activity" or "bioactivity" or "activity" or "biological function", which are used interchangeably, herein mean an effector or antigenic function that is directly or indirectly performed by a polypeptide (whether in its native or denatured conformation), or by any subsequence thereof. Biological activities include binding to polypeptides, binding to other proteins or molecules, activity as a DNA binding protein, as a transcription regulator, ability to bind damaged DNA, etc. A bioactivity can be modulated by directly affecting the subject polypeptide. Alternatively, a bioactivity can be altered by modulating the level of the polypeptide, such as by modulating expression of the corresponding gene.

The term "biomarker" refers a biological molecule, e.g., a nucleic acid, peptide, hormone, etc., whose presence or concentration can be detected and correlated with a known condition, such as a disease state.

"Cells," "host cells", or "recombinant host cells" are terms used interchangeably herein. It is understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

A "chimeric polypeptide" or "fusion polypeptide" is a fusion of a first amino acid sequence encoding one of the subject polypeptides with a second amino acid sequence defining a domain (e.g., polypeptide portion) foreign to and not substantially homologous with any domain of the subject polypeptide. A chimeric polypeptide may present a foreign domain which is found (albeit in a different polypeptide) in an organism which also expresses the first polypeptide, or it may be an "interspecies," "intergenic," etc., fusion of polypeptide structures expressed by different kinds of organisms. In general, a fusion polypeptide can be represented by the general formula (X)ₙ-(Y)ₘ-(Z)ₙ, wherein Y represents a portion of the subject polypeptide, and X and Z are each independently absent or represent amino acid sequences which are not related to the native sequence found in an organism, or which are not found as a polypeptide chain contiguous with the subject sequence, where m is an integer greater than or equal to one, and each occurrence of n is, independently, 0 or an integer greater than or equal to 1 (n and m are preferably no greater than 5 or 10).

A "delivery complex" shall mean a targeting means (e.g., a molecule that results in higher affinity binding of a nucleic acid, protein, polypeptide or peptide to a target cell surface and/or increased cellular or nuclear uptake by a target cell). Examples of targeting means include: sterols (e.g., cholesterol), lipids (e.g., a cationic lipid, virosome or liposome), viruses (e.g., adenovirus, adeno-associated virus, and retrovirus), or target cell-specific binding agents (e.g., ligands recognized by target cell specific receptors). Preferred complexes are sufficiently stable *in vivo* to prevent significant uncoupling prior to internalization by the target cell. However, the complex is cleavable under appropriate conditions within the cell so that the nucleic acid, protein, polypeptide or peptide is released in a functional form.

As is well known, genes or a particular polypeptide may exist in single or multiple copies within the genome of an individual. Such duplicate genes may be identical or may have certain modifications, including nucleotide substitutions, additions or deletions, which all still code for polypeptides having substantially the same activity. The term "DNA sequence encoding a polypeptide" may thus refer to one or more genes within a particular individual. Moreover, certain differences in nucleotide sequences may exist between individual organisms, which are called alleles. Such allelic differences may or may not result in differences in amino acid sequence of the encoded polypeptide yet still encode a polypeptide with the same biological activity.

The term "equivalent" is understood to include nucleotide sequences encoding functionally equivalent polypeptides. Equivalent nucleotide sequences will include sequences that differ by one or more nucleotide substitutions, additions or deletions, such as allelic variants; and will, therefore, include sequences that differ from the nucleotide sequence of the nucleic acids shown in SEQ ID NOs: 1-850 due to the degeneracy of the genetic code.

As used herein, the terms "gene", "recombinant gene", and "gene construct" refer to a nucleic acid of the present invention associated with an open reading frame, including both exon and (optionally) intron sequences.

A "recombinant gene" refers to nucleic acid encoding a polypeptide and comprising exon sequences, though it may optionally include intron sequences which are derived from, for example, a related or unrelated chromosomal gene. The term "intron" refers to a DNA sequence present in a given gene which is not translated into protein and is generally found between exons.

The term "growth" or "growth state" of a cell refers to the proliferative state of a cell as well as to its differentiative state. Accordingly, the term refers to the phase of the cell cycle in which the cell is, e.g., G0, G1, G2, prophase, metaphase, or telophase, as well as to its state of differentiation, e.g., undifferentiated, partially differentiated, or fully differentiated. Without wanting to be limited, differentiation of a cell is usually accompanied by a decrease in the proliferative rate of a cell.

"Homology" or "identity" or "similarity" refers to sequence similarity between two peptides or between two nucleic acid molecules, with identity being a more strict comparison. Homology and identity can each be determined by comparing a position in each sequence which may be aligned for purposes of comparison. When a position in the compared sequence is occupied by the same base or amino acid, then the molecules are identical at that position. A degree of homology or similarity or identity between nucleic acid sequences is a function of the number of identical or matching nucleotides at positions shared by the nucleic acid sequences. A degree of identity of amino acid sequences is a function of the number of identical amino acids at positions shared by the amino acid sequences. A degree of homology or similarity of amino acid sequences is a function of the number of amino acids, i.e., structurally related, at positions shared by the amino acid sequences. An "unrelated" or "non-homologous" sequence shares less than 40% identity, though preferably less than 25% identity, with one of the sequences of the present invention.

The term "percent identical" refers to sequence identity between two amino acid sequences or between two nucleotide sequences. Identity can each be determined by comparing a position in each sequence which may be aligned for purposes of comparison. When an equivalent position in the compared sequences is occupied by the same base or amino acid, then the molecules are identical at that position; when the equivalent site occupied by the same or a similar amino acid residue (e.g., similar in steric and/or electronic nature), then the molecules can be referred to as homologous (similar) at that position. Expression as a percentage of homology, similarity, or identity refers to a function of the number of identical or similar amino acids at positions shared by the compared sequences. Various alignment algorithms and/or programs may be used, including FASTA, BLAST, or ENTREZ. FASTA and BLAST are available as a part of the GCG sequence analysis package (University of Wisconsin, Madison, Wis.), and can be used with, e.g., default settings. ENTREZ is available through the National Center for Biotechnology Information, National Library of Medicine, National Institutes of Health, Bethesda, Md. In one embodiment, the percent identity of two sequences can be determined by the GCG program with a gap weight of 1, e.g., each amino acid gap is weighted as if it were a single amino acid or nucleotide mismatch between the two sequences.

Other techniques for alignment are described in Methods in Enzymology, vol. 266: Computer Methods for Macromolecular Sequence Analysis (1996), ed. Doolittle, Academic Press, Inc., a division of Harcourt Brace & Co., San Diego, California, USA. Preferably, an alignment program that permits gaps in the sequence is utilized to align the sequences. The Smith-Waterman is one type of algorithm that permits gaps in sequence alignments. See Meth. Mol. Biol. 70: 173-187 (1997). Also, the GAP program using the Needleman and Wunsch alignment method can be utilized to align sequences. An alternative search strategy uses MPSRCH software, which runs on a MASPAR computer. MPSRCH uses a Smith-Waterman algorithm to score sequences on a massively parallel computer. This approach improves ability to pick up distantly related matches, and is especially tolerant of small gaps and nucleotide sequence errors. Nucleic acid-encoded amino acid sequences can be used to search both protein and DNA databases.

Databases with individual sequences are described in Methods in Enzymology, ed. Doolittle, *supra*. Databases include Genbank, EMBL, and DNA Database of Japan (DDBJ).

Preferred nucleic acids have a sequence at least 70%, and more preferably 80% identical and more preferably 90% and even more preferably at least 95% identical to an nucleic acid sequence of a sequence shown in one of SEQ ID NOS: 1-850. Nucleic acids at least 90%, more preferably 95%, and most preferably at least about 98-99% identical with a nucleic sequence represented in one of SEQ ID NOS: 1-850 are of course also within the scope of the invention. In preferred embodiments, the nucleic acid is mammalian.

The term "interact" as used herein is meant to include detectable interactions (e.g., biochemical interactions) between molecules, such as interaction between protein-protein, protein-nucleic acid, nucleic acid-nucleic acid, and protein-small molecule or nucleic acid-small molecule in nature.

The term "isolated" as used herein with respect to nucleic acids, such as DNA or RNA, refers to molecules separated from other DNAs, or RNAs, respectively, that are present in the natural source of the macromolecule. The term isolated as used herein also refers to a nucleic acid or peptide that is substantially free of cellular material, viral material, or culture medium when produced by recombinant DNA techniques, or chemical precursors or other chemicals when chemically synthesized. Moreover, an "isolated nucleic acid" is meant to include nucleic acid fragments which are not naturally occurring as fragments and would not be found in the natural state. The term "isolated" is also used herein to refer to polypeptides which are isolated from other cellular proteins and is meant to encompass both purified and recombinant polypeptides.

The terms "modulated" and "differentially regulated" as used herein refer to both upregulation (i.e., activation or stimulation (e.g., by agonizing or potentiating)) and downregulation (i.e., inhibition or suppression (e.g., by antagonizing, decreasing or inhibiting)).

The term "mutated gene" refers to an allelic form of a gene, which is capable of altering the phenotype of a subject having the mutated gene relative to a subject which does not have the mutated gene. If a subject must be homozygous for this mutation to have an altered phenotype, the mutation is said to be recessive. If one copy of the mutated gene is sufficient to alter the genotype of the subject, the mutation is said to be dominant. If a subject has one copy of the mutated gene and has a phenotype that is intermediate between that of a homozygous and that of a heterozygous subject (for that gene), the mutation is said to be co-dominant.

The designation "N", where it appears in the accompanying Sequence Listing, indicates that the identity of the corresponding nucleotide is unknown. "N" should therefore not necessarily be interpreted as permitting substitution with any nucleotide, e.g., A, T, C, or G, but rather as holding the place of a nucleotide whose identity has not been conclusively determined.

The "non-human animals" of the invention include mammalians such as rodents, non-human primates, sheep, dog, cow, chickens, amphibians, reptiles, etc. Preferred non-human animals are selected from the rodent family including rat and mouse, most preferably mouse, though transgenic amphibians, such as members of the *Xenopus* genus, and transgenic chickens can also provide important tools for understanding and identifying agents which can affect, for example, embryogenesis and tissue formation. The term "chimeric animal" is used herein to refer to animals in which the recombinant gene is found, or in which the recombinant gene is expressed in some but not all cells of the animal. The term "tissue-specific chimeric animal" indicates that one of the recombinant genes is present and/or expressed or disrupted in some tissues but not others.

As used herein, the term "nucleic acid" refers to polynucleotides such as deoxyribonucleic acid (DNA), and, where appropriate, ribonucleic acid (RNA). The term should also be understood to include, as equivalents, analogs of either RNA or DNA made from nucleotide analogs, and, as applicable to the embodiment being described, single (sense or antisense) and double-stranded polynucleotides. ESTs, chromosomes, cDNAs, mRNAs, and rRNAs are representative examples of molecules that may be referred to as nucleic acids.

The term "nucleotide sequence complementary to the nucleotide sequence of SEQ ID NO. x" refers to the nucleotide sequence of the complementary strand of a nucleic acid strand having SEQ ID NO. x. The term "complementary strand" is used herein interchangeably with the term "complement". The complement of a nucleic acid strand can be the complement of a coding strand or the complement of a noncoding strand.

The term "polymorphism" refers to the coexistence of more than one form of a gene or portion (e.g., allelic variant) thereof. A portion of a gene of which there are at least two different forms, i.e., two different nucleotide sequences, is referred to as a "polymorphic region of a gene". A polymorphic region can be a single nucleotide, the identity of which differs in different alleles. A polymorphic region can also be several nucleotides long.

A "polymorphic gene" refers to a gene having at least one polymorphic region.

As used herein, the term "promoter" means a DNA sequence that regulates expression of a selected DNA sequence operably linked to the promoter, and which effects expression of the selected DNA sequence in cells. The term encompasses "tissue specific" promoters, i.e., promoters which effect expression of the selected DNA sequence only in specific cells (e.g., cells of a specific tissue). The term also covers so-called "leaky" promoters, which regulate expression of a selected DNA primarily in one tissue, but cause expression in other tissues as well. The term also encompasses non-tissue specific promoters and promoters that constitutively express or that are inducible (i.e., expression levels can be controlled).

The terms "protein", "polypeptide", and "peptide" are used interchangeably herein when referring to a gene product.

The term "recombinant protein" refers to a polypeptide of the present invention which is produced by recombinant DNA techniques, wherein generally, DNA encoding a polypeptide is inserted into a suitable expression vector which is in turn used to transform a host cell to produce the heterologous protein. Moreover, the phrase "derived from", with respect to a recombinant gene, is meant to include within the meaning of "recombinant protein" those proteins having an amino acid sequence of a native polypeptide, or an amino acid sequence similar thereto which is generated by mutations including substitutions and deletions (including truncation) of a naturally occurring form of the polypeptide.

"Small molecule" as used herein, is meant to refer to a composition, which has a molecular weight of less than about 5 kD and most preferably less than about 4 kD. Small molecules can be nucleic acids, peptides, polypeptides, peptidomimetics, carbohydrates, lipids or other organic (carbon-containing) or inorganic molecules. Many pharmaceutical companies have extensive libraries of chemical and/or biological mixtures, often fungal, bacterial, or algal extracts, which can be screened with any of the assays of the invention to identify compounds that modulate a bioactivity.

As used herein, the term "specifically hybridizes" or "specifically detects" refers to the ability of a nucleic acid molecule of the invention to hybridize to at least a portion of, for example approximately 6, 12, 15, 20, 30, 50, 100, 150, 200, 300, 350, 400, 500, 750 or 1000 contiguous nucleotides of a nucleic acid designated in any one of SEQ ID Nos: 1-850, or a sequence complementary thereto, or naturally occurring mutants thereof, such that it has less than 15%, preferably less than 10%, and more preferably less than 5% background hybridization to a cellular nucleic acid (e.g., mRNA or genomic DNA) encoding a different protein. In preferred embodiments, the oligonucleotide probe detects only a specific nucleic acid, e.g., it does not substantially hybridize to similar or related nucleic acids, or complements thereof.

"Transcriptional regulatory sequence" is a generic term used throughout the specification to refer to DNA sequences, such as initiation signals, enhancers, and promoters, which induce or control transcription of protein coding sequences with which they are operably linked. In preferred embodiments, transcription of one of the genes is under the control of a promoter sequence (or other transcriptional regulatory sequence) which controls the expression of the recombinant gene in a cell-type in which expression is intended. It will also be understood that the recombinant gene can be under the control of transcriptional regulatory sequences which are the same or which are different from those sequences which control transcription of the naturally-occurring forms of the polypeptide.

As used herein, the term "transfection" means the introduction of a nucleic acid, e.g., via an expression vector, into a recipient cell by nucleic acid-mediated gene transfer. "Transformation", as used herein, refers to a process in which a cell's genotype is changed as a result of the cellular uptake of exogenous DNA or RNA, and, for example, the transformed cell expresses a recombinant form of a polypeptide or, in the case of anti-sense expression from the transferred gene, the expression of the target gene is disrupted.

As used herein, the term "transgene" means a nucleic acid sequence (or an antisense transcript thereto) which has been introduced into a cell. A transgene could be partly or entirely heterologous, i.e., foreign, to the transgenic animal or cell into which it is introduced, or, is homologous to an endogenous gene of the transgenic animal or cell into which it is introduced, but which is designed to be inserted, or is inserted, into the animal's genome in such a way as to alter the genome of the cell into which it is inserted (e.g., it is inserted at a location which differs from that of the natural gene or its insertion results in a knockout). A transgene can also be present in a cell in the form of an episome. A transgene can include one or more transcriptional regulatory sequences and any other nucleic acid, such as introns, that may be necessary for optimal expression of a selected nucleic acid.

A "transgenic animal" refers to any animal, preferably a non-human mammal, bird or an amphibian, in which one or more of the cells of the animal contain heterologous nucleic acid introduced by way of human intervention, such as by transgenic techniques well known in the art. The nucleic acid is introduced into the cell, directly or indirectly by introduction into a precursor of the cell, by way of deliberate genetic manipulation, such as by microinjection or by infection with a recombinant virus. The term genetic manipulation does not include classical cross-breeding, or *in vitro* fertilization, but rather is directed to the introduction of a recombinant DNA molecule. This molecule may be integrated within a chromosome, or it may be extra-chromosomally replicating DNA. In the typical transgenic animals described herein, the transgene causes cells to express a recombinant form of one of the subject polypeptide, e.g. either agonistic or antagonistic forms. However, transgenic animals in which the recombinant gene is silent are also contemplated, as for example, the FLP or CRE recombinase dependent constructs described below. Moreover, "transgenic animal" also includes those recombinant animals in which gene disruption of one or more genes is caused by human intervention, including both recombination and antisense techniques.

The term "treating" as used herein is intended to encompass curing as well as ameliorating at least one symptom of the condition or disease.

The term "vector" refers to a nucleic acid molecule capable of transporting another nucleic acid to which it has been linked. One type of preferred vector is an episome, i.e., a nucleic acid capable of extra-chromosomal replication. Preferred vectors are those capable of autonomous replication and/or expression of nucleic acids to which they are linked. Vectors capable of directing the expression of genes to which they are operatively linked are referred to herein as "expression vectors". In general, expression vectors of utility in recombinant DNA techniques are often in the form of "plasmids" which refer generally to circular double stranded DNA loops which, in their vector form are not bound to the chromosome. In the present specification, "plasmid" and "vector" are used interchangeably as the plasmid is the most commonly used form of vector. However, the invention is intended to include such other forms of expression vectors which serve equivalent functions and which become known in the art subsequently hereto.

The term "wild-type allele" refers to an allele of a gene which, when present in two copies in a subject results in a wild-type phenotype. There can be several different wild-type alleles of a specific gene, since certain nucleotide changes in a gene may not affect the phenotype of a subject having two copies of the gene with the nucleotide changes.

### III. Nucleic Acids of the Present Invention

As described below, one aspect of the invention pertains to isolated nucleic acids, variants, and/or equivalents of such nucleic acids.

Nucleic acids of the present invention have been identified as differentially expressed in tumor cells, e.g., colon cancer-derived cell lines (relative to the expression levels in normal tissue, e.g., normal colon tissue and/or normal non-colon tissue), such as SEQ ID Nos. 1-850, preferably SEQ ID Nos. 1-383, even more preferably SEQ ID Nos. 1-127, or a sequence complementary thereto. In certain embodiments, the subject nucleic acids are differentially expressed by at least a factor of two, preferably at least a factor of five, even more preferably at least a factor of twenty, still more preferably at least a factor of fifty. Preferred nucleic acids include sequences identified as differentially expressed both in colon cancer cell tissue and colon cancer cell lines. In preferred embodiments, nucleic acids of the present invention are upregulated in tumor cells, especially colon cancer tissue and/or colon cancer-derived cell lines. In another embodiment, nucleic acids of the present invention are downregulated in tumor cells, especially colon cancer tissue and/or colon cancer-derived cell lines.

Table 1 indicates those sequences which are over- or underexpressed in a colon cancer-derived cell line relative to normal tissue, and further designates those sequences which are also differentially regulated in colon cancer tissue. The designation O indicates that the corresponding sequence was overexpressed, M indicates possible overexpression, N indicates no differential expression, and U indicates underexpression.

Genes which are upregulated, such as oncogenes, or downregulated, such as tumor suppressors, in aberrantly proliferating cells may be targets for diagnostic or therapeutic techniques. For example, upregulation of the *cdc2* gene induces mitosis. Overexpression of the *myt1* gene, a mitotic deactivator, negatively regulates the activity of *cdc2.* Aberrant proliferation may thus be induced either by upregulating *cdc2* or by downregulating *myt1.* Similarly, downregulation of tumor suppressors such as *p53* and *Rb* have been implicated in tumorigenesis.

Particularly preferred polypeptides are those that are encoded by nucleic acid sequences at least about 70%, 75%, 80%, 90%, 95%, 97%, or 98% similar to a nucleic acid sequence of SEQ ID Nos. 1-850. Preferably, the nucleic acid includes all or a portion (e.g., at least about 12, at least about 15, at least about 25, or at least about 40 nucleotides) of the nucleotide sequence corresponding to the nucleic acid of SEQ ID Nos. 1-383, preferably SEQ ID Nos. 1-127, or a sequence complementary thereto.

Still other preferred nucleic acids of the present invention encode a polypeptide comprising at least a portion of a polypeptide encoded by one of SEQ ID Nos. 1-850. For example, preferred nucleic acid molecules for use as probes/primers or antisense molecules (i.e., noncoding nucleic acid molecules) can comprise at least about 12, 20, 30, 50, 60, 70, 80, 90, or 100 base pairs in length up to the length of the complete gene. Coding nucleic acid molecules can comprise, for example, from about 50, 60, 70, 80, 90, or 100 base pairs up to the length of the complete gene.

Another aspect of the invention provides a nucleic acid which hybridizes under low, medium, or high stringency conditions to a nucleic acid sequence represented by one of SEQ ID Nos. 1-383, preferably SEQ ID Nos. 1-127, or a sequence complementary thereto. Appropriate stringency conditions which promote DNA hybridization, for example, 6.0 x sodium chloride/sodium citrate (SSC) at about 45 °C, followed by a wash of 2.0 x SSC at 50 °C, are known to those skilled in the art or can be found in Current Protocols in Molecular Biology, John Wiley & Sons, N.Y. (1989), 6.3.1-12.3.6. For example, the salt concentration in the wash step can be selected from a low stringency of about 2.0 x SSC at 50 °C to a high stringency of about 0.2 x SSC at 50 °C. In addition, the temperature in the wash step can be increased from low stringency conditions at room temperature, about 22 °C, to high stringency conditions at about 65 °C. Both temperature and salt may be varied, or temperature or salt concentration may be held constant while the other variable is changed. In a preferred embodiment, a nucleic acid of the present invention will bind to one of SEQ ID Nos. 1-383, preferably SEQ ID Nos. 1-127, or a sequence complementary thereto, under moderately stringent conditions, for example at about 2.0 x SSC and about 40 °C. In a particularly preferred embodiment, a nucleic acid of the present invention will bind to one of SEQ ID Nos. 1-383, preferably SEQ ID Nos. 1-127, or a sequence complementary thereto, under high stringency conditions.

In one embodiment, the invention provides nucleic acids which hybridize under low stringency conditions of 6 x SSC at room temperature followed by a wash at 2 x SSC at room temperature.

In another embodiment, the invention provides nucleic acids which hybridize under high stringency conditions of 2 x SSC at 65 °C followed by a wash at 0.2 x SSC at 65 °C.

Nucleic acids having a sequence that differs from the nucleotide sequences shown in one of SEQ ID Nos. 1-383, preferably SEQ ID Nos. 1-127, or a sequence complementary thereto, due to degeneracy in the genetic code, are also within the scope of the invention. Such nucleic acids encode functionally equivalent peptides (i.e., a peptide having equivalent or similar biological activity) but differ in sequence from the sequence shown in the sequence listing due to degeneracy in the genetic code. For example, a number of amino acids are designated by more than one triplet. Codons that specify the same amino acid, or synonyms (for example, CAU and CAC each encode histidine) may result in "silent" mutations which do not affect the amino acid sequence of a polypeptide. However, it is expected that DNA sequence polymorphisms that do lead to changes in the amino acid sequences of the subject polypeptides will exist among mammals. One skilled in the art will appreciate that these variations in one or more nucleotides (e.g., up to about 3-5% of the nucleotides) of the nucleic acids encoding polypeptides having an activity of a polypeptide may exist among individuals of a given species due to natural allelic variation.

Also within the scope of the invention are nucleic acids encoding splicing variants of proteins encoded by a nucleic acid of SEQ ID Nos. 1-850, preferably SEQ ID Nos. 1-383, even more preferably SEQ ID Nos. 1-127, or a sequence complementary thereto, or natural homologs of such proteins. Such homologs can be cloned by hybridization or PCR, as further described herein.

The polynucleotide sequence may also encode for a leader sequence, e.g., the natural leader sequence or a heterologous leader sequence, for a subject polypeptide. For example, the desired DNA sequence may be fused in the same reading frame to a DNA sequence which aids in expression and secretion of the polypeptide from the host cell, for example, a leader sequence which functions as a secretory sequence for controlling transport of the polypeptide from the cell. The protein having a leader sequence is a preprotein and may have the leader sequence cleaved by the host cell to form the mature form of the protein.

The polynucleotide of the present invention may also be fused in frame to a marker sequence, also referred to herein as "Tag sequence" encoding a "Tag peptide", which allows for marking and/or purification of the polypeptide of the present invention. In a preferred embodiment, the marker sequence is a hexahistidine tag, e.g., supplied by a PQE-9 vector. Numerous other Tag peptides are available commercially. Other frequently used Tags include myc-epitopes (e.g., see Ellison et al. (1991) *J Biol Chem 266*:21150-21157) which includes a 10-residue sequence from c-myc, the pFLAG system (International Biotechnologies, Inc.), the pEZZ-protein A system (Pharmacia, NJ), and a 16 amino acid portion of the *Haemophilus influenza* hemagglutinin protein. Furthermore, any polypeptide can be used as a Tag so long as a reagent, e.g., an antibody interacting specifically with the Tag polypeptide is available or can be prepared or identified.

As indicated by the examples set out below, nucleic acids can be obtained from mRNA present in any of a number of eukaryotic cells, e.g., and are preferably obtained from metazoan cells, more preferably from vertebrate cells, and even more preferably from mammalian cells. It should also be possible to obtain nucleic acids of the present invention from genomic DNA from both adults and embryos. For example, a gene can be cloned from either a cDNA or a genomic library in accordance with protocols generally known to persons skilled in the art. cDNA can be obtained by isolating total mRNA from a cell, e.g., a vertebrate cell, a mammalian cell, or a human cell, including embryonic cells. Double stranded cDNAs can then be prepared from the total mRNA, and subsequently inserted into a suitable plasmid or bacteriophage vector using any one of a number of known techniques. The gene can also be cloned using established polymerase chain reaction techniques in accordance with the nucleotide sequence information provided by the invention.

In certain embodiments, a nucleic acid, probe, vector, or other construct of the present invention includes at least about five, at least about ten, or at least about twenty nucleic acids from a region designated as novel in Table 2. In certain other embodiments, a nucleic acid of the present invention includes at least about five, at least about ten, or at least about twenty nucleic acids which are not included in the clones whose accession numbers are listed in Table 2.

The invention includes within its scope a polynucleotide having the nucleotide sequence of nucleic acid obtained from this biological material, wherein the nucleic acid hybridizes under stringent conditions (at least about 4 x SSC at 65°C, or at least about 4 x SSC at 42°C; see, for example, U.S. Patent No. 5,707,829, incorporated herein by reference) with at least 15 contiguous nucleotides of at least one of SEQ ID Nos. 1-850. By this is intended that when at least 15 contiguous nucleotides of one of SEQ ID Nos. 1-850 is used as a probe, the probe will preferentially hybridize with a gene or mRNA (of the biological material) comprising the complementary sequence, allowing the identification and retrieval of the nucleic acids of the biological material that uniquely hybridize to the selected probe. Probes from more than one of SEQ ID Nos. 1-850 will hybridize with the same gene or mRNA if the cDNA from which they were derived corresponds to one mRNA. Probes of more than 15 nucleotides can be used, but 15 nucleotides represents enough sequence for unique identification.

Because the present nucleic acids represent partial mRNA transcripts, two or more nucleic acids of the invention may represent different regions of the same mRNA transcript and the same gene. Thus, if two or more of SEQ ID Nos. 1-850 are identified as belonging to the same clone, then either sequence can be used to obtain the full-length mRNA or gene.

Nucleic acid-related polynucleotides can also be isolated from cDNA libraries. These libraries are preferably prepared from mRNA of human colon cells, more preferably, human colon cancer cells, even more preferably, from a human colon adenocarcinoma cell line, SW480. Alignment of SEQ ID Nos. 1-850, as described above, can indicated that a cell line or tissue source of a related protein or polynucleotide can also be used as a source of the nucleic acid-related cDNA.

Techniques for producing and probing nucleic acid sequence libraries are described, for example, in Sambrook *et al*., "Molecular Cloning: A Laboratory Manual" (New York, Cold Spring Harbor Laboratory, 1989). The cDNA can be prepared by using primers based on a sequence from SEQ ID Nos. 1-850. In one embodiment, the cDNA library can be made from only poly-adenylated mRNA. Thus, poly-T primers can be used to prepare cDNA from the mRNA. Alignment of SEQ ID Nos. 1-850 can result in identification of a related polypeptide or polynucleotide. Some of the polynucleotides disclosed herein contains repetitive regions that were subject to masking during the search procedures. The information about the repetitive regions is discussed below.

Constructs of polynucleotides having sequences of SEQ ID Nos. 1-850 can be generated synthetically. Alternatively, single-step assembly of a gene and entire plasmid from large numbers of oligodeoxyribonucleotides is described by Stemmer et al., *Gene (Amsterdam)* (1995) *164(1)*:49-53. In this method, assembly PCR (the synthesis of long DNA sequences from large numbers of oligodeoxyribonucleotides (oligos)) is described. The method is derived from DNA shuffling (Stemmer, *Nature* (1994) *370*:389-391), and does not rely on DNA ligase, but instead relies on DNA polymerase to build increasingly longer DNA fragments during the assembly process. For example, a 1.1-kb fragment containing the TEM-1 beta-lactamase-encoding gene (bla) can be assembled in a single reaction from a total of 56 oligos, each 40 nucleotides (nt) in length. The synthetic gene can be PCR amplified and cloned in a vector containing the tetracycline-resistance gene (Tc-R) as the sole selectable marker. Without relying on ampicillin (Ap) selection, 76% of the Tc-R colonies were Ap-R, making this approach a general method for the rapid and cost-effective synthesis of any gene.

### IV. Identification of Functional and Structural Motifs of Novel Genes Using Art-Recognized Methods

Translations of the nucleotide sequence of the nucleic acids, cDNAs, or full genes can be aligned with individual known sequences. Similarity with individual sequences can be used to determine the activity of the polypeptides encoded by the polynucleotides of the invention. For example, sequences that show similarity with a chemokine sequence may exhibit chemokine activities. Also, sequences exhibiting similarity with more than one individual sequence may exhibit activities that are characteristic of either or both individual sequences.

The full length sequences and fragments of the polynucleotide sequences of the nearest neighbors can be used as probes and primers to identify and isolate the full length sequence of the nucleic acid. The nearest neighbors can indicate a tissue or cell type to be used to construct a library for the full-length sequences of the nucleic acid.

Typically, the nucleic acids are translated in all six frames to determine the best alignment with the individual sequences. The sequences disclosed herein in the Sequence Listing are in a 5' to 3' orientation and translation in three frames can be sufficient (with a few specific exceptions as described in the Examples). These amino acid sequences are referred to, generally, as query sequences, which will be aligned with the individual sequences.

Nucleic acid sequences can be compared with known genes by any of the methods disclosed above. Results of individual and query sequence alignments can be divided into three categories: high similarity, weak similarity, and no similarity. Individual alignment results ranging from high similarity to weak similarity provide a basis for determining polypeptide activity and/or structure.

Parameters for categorizing individual results include: percentage of the alignment region length where the strongest alignment is found, percent sequence identity, and p value.

The percentage of the alignment region length is calculated by counting the number of residues of the individual sequence found in the region of strongest alignment. This number is divided by the total residue length of the query sequence to find a percentage. An example is shown below:

Query sequence:          ASNPERTMIPVTRVGLIRYM
                             |   ||| |||| |||
Individual sequence:     YMMTEYLAIPV.RVGLPRYM
                         1   5    10    15

The region of alignment begins at amino acid 9 and ends at amino acid 19. The total length of the query sequence is 20 amino acids. The percent of the alignment region length is 11/20 or 55%.

Percent sequence identity is calculated by counting the number of amino acid matches between the query and individual sequence and dividing total number of matches by the number of residues of the individual sequence found in the region of strongest alignment. For the example above, the percent identity would be 10 matches divided by 11 amino acids, or approximately 90.9%.

P value is the probability that the alignment was produced by chance. For a single alignment, the p value can be calculated according to Karlin *et al*., Proc. Natl. Acad. Sci. 87: 2264 (1990) and Karlin *et al*., Proc. Natl. Acad. Sci. 90: (1993). The p value of multiple alignments using the same query sequence can be calculated using an heuristic approach described in Altschul *et al*., Nat. Genet. 6: 119 (1994). Alignment programs such as BLAST program can calculate the p value.

The boundaries of the region where the sequences align can be determined according to Doolittle, Methods in Enzymology, *supra*; BLAST or FASTA programs; or by determining the area where the sequence identity is highest.

Another factor to consider for determining identity or similarity is the location of the similarity or identity. Strong local alignment can indicate similarity even if the length of alignment is short. Sequence identity scattered throughout the length of the query sequence also can indicate a similarity between the query and profile sequences.

### High SimilarityError! Bookmark not defined.

For the alignment results to be considered high similarity, the percent of the alignment region length, typically, is at least about 55% of total length query sequence; more typically, at least about 58%; even more typically; at least about 60% of the total residue length of the query sequence. Usually, percent length of the alignment region can be as much as about 62%; more usually, as much as about 64%; even more usually, as much as about 66%.

Further, for high similarity, the region of alignment, typically, exhibits at least about 75% of sequence identity; more typically, at least about 78%; even more typically; at least about 80% sequence identity. Usually, percent sequence identity can be as much as about 82%; more usually, as much as about 84%; even more usually, as much as about 86%.

The p value is used in conjunction with these methods. If high similarity is found, the query sequence is considered to have high similarity with a profile sequence when the p value is less than or equal to about 10⁻²; more usually; less than or equal to about 10⁻³; even more usually; less than or equal to about 10⁻⁴. More typically, the p value is no more than about 10⁻⁵; more typically; no more than or equal to about 10⁻¹⁰; even more typically; no more than or equal to about 10⁻¹⁵ for the query sequence to be considered high similarity.

### Weak Similarity

For the alignment results to be considered weak similarity, there is no minimum percent length of the alignment region nor minimum length of alignment. A better showing of weak similarity is considered when the region of alignment is, typically, at least about 15 amino acid residues in length; more typically, at least about 20; even more typically; at least about 25 amino acid residues in length. Usually, length of the alignment region can be as much as about 30 amino acid residues; more usually, as much as about 40; even more usually, as much as about 60 amino acid residues.

Further, for weak similarity, the region of alignment, typically, exhibits at least about 35% of sequence identity; more typically, at least about 40%; even more typically; at least about 45% sequence identity. Usually, percent sequence identity can be as much as about 50%; more usually, as much as about 55%; even more usually, as much as about 60%.

If low similarity is found, the query sequence is considered to have weak similarity with a profile sequence when the p value is usually less than or equal to about 10⁻²; more usually; less than or equal to about 10⁻³; even more usually; less than or equal to about 10⁻⁴. More typically, the p value is no more than about 10⁻⁵; more usually; no more than or equal to about 10⁻¹⁰; even more usually; no more than or equal to about 10⁻¹⁵ for the query sequence to be considered weak similarity.

### Similarity Determined by Sequence Identity AloneError! Bookmark not defined.

Sequence identity alone can be used to determine similarity of a query sequence to an individual sequence and can indicate the activity of the sequence. Such an alignment, preferably, permits gaps to align sequences. Typically, the query sequence is related to the profile sequence if the sequence identity over the entire query sequence is at least about 15%; more typically, at least about 20%; even more typically, at least about 25%; even more typically, at least about 50%. Sequence identity alone as a measure of similarity is most useful when the query sequence is usually, at least 80 residues in length; more usually, 90 residues; even more usually, at least 95 amino acid residues in length. More typically, similarity can be concluded based on sequence identity alone when the query sequence is preferably 100 residues in length; more preferably, 120 residues in length; even more preferably, 150 amino acid residues in length.

### Determining Activity from Alignments with Profile and Multiple Aligned Sequences

Translations of the nucleic acids can be aligned with amino acid profiles that define either protein families or common motifs. Also, translations of the nucleic acids can be aligned to multiple sequence alignments (MSA) comprising the polypeptide sequences of members of protein families or motifs. Similarity or identity with profile sequences or MSAs can be used to determine the activity of the polypeptides encoded by nucleic acids or corresponding cDNA or genes. For example, sequences that show an identity or similarity with a chemokine profile or MSA can exhibit chemokine activities.

Profiles can designed manually by (1) creating a MSA, which is an alignment of the amino acid sequence of members that belong to the family and (2) constructing a statistical representation of the alignment. Such methods are described, for example, in Birney *et al*., Nucl. Acid Res. 24(14): 2730-2739 (1996).

MSAs of some protein families and motifs are publicly available. For example, these include MSAs of 547 different families and motifs. These MSAs are described also in Sonnhammer *et al*., Proteins 28: 405-420 (1997). Other sources are also available in the world wide web. A brief description of these MSAs is reported in Pascarella *et al*., Prot. Eng. 9(3): 249-251 (1996).

Techniques for building profiles from MSAs are described in Sonnhammer *et al., supra*; Birney *et al., supra*; and Methods in Enzymology, vol. 266: "Computer Methods for Macromolecular Sequence Analysis," 1996, ed. Doolittle, Academic Press, Inc., a division of Harcourt Brace & Co., San Diego, California, USA.

Similarity between a query sequence and a protein family or motif can be determined by (a) comparing the query sequence against the profile and/or (b) aligning the query sequence with the members of the family or motif.

Typically, a program such as Searchwise can be used to compare the query sequence to the statistical representation of the multiple alignment, also known as a profile. The program is described in Birney *et al*., *supra*. Other techniques to compare the sequence and profile are described in Sonnhammer *et al*., *supra* and Doolittle, *supra*.

Next, methods described by Feng *et al*., J. Mol. Evol. 25: 351-360 (1987) and Higgins *et al*., CABIOS 5: 151-153 (1989) can be used align the query sequence with the members of a family or motif, also known as a MSA. Computer programs, such as PILEUP, can be used. See Feng *et al*., *infra*.

The following factors are used to determine if a similarity between a query sequence and a profile or MSA exists: (1) number of conserved residues found in the query sequence, (2) percentage of conserved residues found in the query sequence, (3) number of frameshifts, and (4) spacing between conserved residues.

Some alignment programs that both translate and align sequences can make any number of frameshifts when translating the nucleotide sequence to produce the best alignment. The fewer frameshifts needed to produce an alignment, the stronger the similarity or identity between the query and profile or MSAs. For example, a weak similarity resulting from no frameshifts can be a better indication of activity or structure of a query sequence, than a strong similarity resulting from two frameshifts. Preferably, three or fewer frameshifts are found in an alignment; more preferably two or fewer frameshifts; even more preferably, one or fewer frameshifts; even more preferably, no frameshifts are found in an alignment of query and profile or MSAs.

Conserved residues are those amino acids that are found at a particular position in all or some of the family or motif members. For example, most known chemokines contain four conserved cysteines. Alternatively, a position is considered conserved if only a certain class of amino acids is found in a particular position in all or some of the family members. For example, the N-terminal position may contain a positively charged amino acid, such as lysine, arginine, or histidine.

Typically, a residue of a polypeptide is conserved when a class of amino acids or a single amino acid is found at a particular position in at least about 40% of all class members; more typically, at least about 50%; even more typically, at least about 60% of the members. Usually, a residue is conserved when a class or single amino acid is found in at least about 70% of the members of a family or motif; more usually, at least about 80%; even more usually, at least about 90%; even more usually, at least about 95%.

A residue is considered conserved when three unrelated amino acids are found at a particular position in the some or all of the members; more usually, two unrelated amino acids. These residues are conserved when the unrelated amino acids are found at particular positions in at least about 40% of all class member; more typically, at least about 50%; even more typically, at least about 60% of the members. Usually, a residue is conserved when a class or single amino acid is found in at least about 70% of the members of a family or motif; more usually, at least about 80%; even more usually, at least about 90%; even more usually, at least about 95%.

A query sequence has similarity to a profile or MSA when the query sequence comprises at least about 25% of the conserved residues of the profile or MSA; more usually, at least about 30%; even more usually; at least about 40%. Typically, the query sequence has a stronger similarity to a profile sequence or MSA when the query sequence comprises at least about 45% of the conserved residues of the profile or MSA; more typically, at least about 50%; even more typically; at least about 55%.

### V. Probes and Primers

The nucleotide sequences determined from the cloning of genes from tumor cells, especially colon cancer cell lines and tissues will further allow for the generation of probes and primers designed for identifying and/or cloning homologs in other cell types, e.g., from other tissues, as well as homologs from other mammalian organisms. Nucleotide sequences useful as probes/primers may include all or a portion of the sequences listed in SEQ ID Nos. 1-850 or sequences complementary thereto or sequences which hybridize under stringent conditions to all or a portion of SEQ ID Nos. 1-850. For instance, the present invention also provides a probe/primer comprising a substantially purified oligonucleotide, which oligonucleotide comprising a nucleotide sequence that hybridizes under stringent conditions to at least approximately 12, preferably 25, more preferably 40, 50, or 75 consecutive nucleotides up to the full length of the sense or anti-sense sequence selected from the group consisting of SEQ ID Nos. 1-850, preferably SEQ ID Nos. 1-383, even more preferably SEQ ID Nos. 1-127, or a sequence complementary thereto, or naturally occurring mutants thereof. For instance, primers based on a nucleic acid represented in SEQ ID Nos. 1-850, preferably SEQ ID Nos. 1-383, even more preferably SEQ ID Nos. 1-127, or a sequence complementary thereto, can be used in PCR reactions to clone homologs of that sequence.

In yet another embodiment, the invention provides probes/primers comprising a nucleotide sequence that hybridizes under moderately stringent conditions to at least approximately 12, 16, 25, 40, 50 or 75 consecutive nucleotides up to the full length of the sense or antisense sequence selected from the group consisting of SEQ ID Nos. 1-850, preferably SEQ ID Nos. 1-383, even more preferably SEQ ID Nos. 1-127, or naturally occurring mutants thereof.

In particular, these probes are useful because they provide a method for detecting mutations in wild-type genes of the present invention. Nucleic acid probes which are complementary to a wild-type gene of the present invention and can form mismatches with mutant genes are provided, allowing for detection by enzymatic or chemical cleavage or by shifts in electrophoretic mobility.

Likewise, probes based on the subject sequences can be used to detect transcripts or genomic sequences encoding the same or homologous proteins, for use, for example, in prognostic or diagnostic assays. In preferred embodiments, the probe further comprises a label group attached thereto and able to be detected, e.g., the label group is selected from radioisotopes, fluorescent compounds, chemiluminescent compounds, enzymes, and enzyme co-factors.

Full-length cDNA molecules comprising the disclosed nucleic acids are obtained as follows. A subject nucleic acid or a portion thereof comprising at least about 12, 15, 18, or 20 nucleotides up to the full length of a sequence represented in SEQ ID Nos. 1-850, preferably SEQ ID Nos. 1-383, even more preferably SEQ ID Nos. 1-127, or a sequence complementary thereto, may be used as a hybridization probe to detect hybridizing members of a cDNA library using probe design methods, cloning methods, and clone selection techniques as described in U.S. Patent No. 5,654,173, "Secreted Proteins and Polynucleotides Encoding Them," incorporated herein by reference. Libraries of cDNA may be made from selected tissues, such as normal or tumor tissue, or from tissues of a mammal treated with, for example, a pharmaceutical agent. Preferably, the tissue is the same as that used to generate the nucleic acids, as both the nucleic acid and the cDNA represent expressed genes. Most preferably, the cDNA library is made from the biological material described herein in the Examples. Alternatively, many cDNA libraries are available commercially. (Sambrook *et al*., *Molecular Cloning: A Laboratory Manual, 2nd Ed*. (Cold Spring Harbor Press, Cold Spring Harbor, NY 1989). The choice of cell type for library construction may be made after the identity of the protein encoded by the nucleic acid-related gene is known. This will indicate which tissue and cell types are likely to express the related gene, thereby containing the mRNA for generating the cDNA.

Members of the library that are larger than the nucleic acid, and preferably that contain the whole sequence of the native message, may be obtained. To confirm that the entire cDNA has been obtained, RNA protection experiments may be performed as follows. Hybridization of a full-length cDNA to an mRNA may protect the RNA from RNase degradation. If the cDNA is not full length, then the portions of the mRNA that are not hybridized may be subject to RNase degradation. This may be assayed, as is known in the art, by changes in electrophoretic mobility on polyacrylamide gels, or by detection of released monoribonucleotides. Sambrook *et al**., Molecular Cloning: A Laboratory Manual, 2nd Ed.* (Cold Spring Harbor Press, Cold Spring Harbor, NY 1989). In order to obtain additional sequences 5' to the end of a partial cDNA, 5' RACE (PCR Protocols: A Guide to Methods and Applications (Academic Press, Inc. 1990)) may be performed.

Genomic DNA may be isolated using nucleic acids in a manner similar to the isolation of full-length cDNAs. Briefly, the nucleic acids, or portions thereof, may be used as probes to libraries of genomic DNA. Preferably, the library is obtained from the cell type that was used to generate the nucleic acids. Most preferably, the genomic DNA is obtained from the biological material described herein in the Example. Such libraries may be in vectors suitable for carrying large segments of a genome, such as P1 or YAC, as described in detail in Sambrook *et al*., 9.4-9.30. In addition, genomic sequences can be isolated from human BAC libraries, which are commercially available from Research Genetics, Inc., Huntville, Alabama, USA, for example. In order to obtain additional 5' or 3' sequences, chromosome walking may be performed, as described in Sambrook *et al*., such that adjacent and overlapping fragments of genomic DNA are isolated. These may be mapped and pieced together, as is known in the art, using restriction digestion enzymes and DNA ligase.

Using the nucleic acids of the invention, corresponding full length genes can be isolated using both classical and PCR methods to construct and probe cDNA libraries. Using either method, Northern blots, preferably, may be performed on a number of cell types to determine which cell lines express the gene of interest at the highest rate.

Classical methods of constructing cDNA libraries are taught in Sambrook et al., supra. With these methods, cDNA can be produced from mRNA and inserted into viral or expression vectors. Typically, libraries of mRNA comprising poly(A) tails can be produced with poly(T) primers. Similarly, cDNA libraries can be produced using the instant sequences as primers.

PCR methods may be used to amplify the members of a cDNA library that comprise the desired insert. In this case, the desired insert may contain sequence from the full length cDNA that corresponds to the instant nucleic acids. Such PCR methods include gene trapping and RACE methods.

Gene trapping may entail inserting a member of a cDNA library into a vector. The vector then may be denatured to produce single stranded molecules. Next, a substrate-bound probe, such a biotinylated oligo, may be used to trap cDNA inserts of interest. Biotinylated probes can be linked to an avidin-bound solid substrate. PCR methods can be used to amplify the trapped cDNA. To trap sequences corresponding to the full length genes, the labeled probe sequence may be based on the nucleic acids of the invention, e.g., SEQ ID Nos. 1-383, preferably SEQ ID Nos. 1-127, or a sequence complementary thereto. Random primers or primers specific to the library vector can be used to amplify the trapped cDNA. Such gene trapping techniques are described in Gruber *et al*., PCT WO 95/04745 and *Gruber et al*., U.S. Pat. No. 5,500,356. Kits are commercially available to perform gene trapping experiments from, for example, Life Technologies, Gaithersburg, Maryland, USA.

"Rapid amplification of cDNA ends," or RACE, is a PCR method of amplifying cDNAs from a number of different RNAs. The cDNAs may be ligated to an oligonucleotide linker and amplified by PCR using two primers. One primer may be based on sequence from the instant nucleic acids, for which full length sequence is desired, and a second primer may comprise a sequence that hybridizes to the oligonucleotide linker to amplify the cDNA. A description of this method is reported in PCT Pub. No. WO 97/19110.

In preferred embodiments of RACE, a common primer may be designed to anneal to an arbitrary adaptor sequence ligated to cDNA ends (Apte and Siebert, Biotechniques 15:890-893, 1993; Edwards et al., Nuc. Acids Res. 19:5227-5232, 1991). When a single gene-specific RACE primer is paired with the common primer, preferential amplification of sequences between the single gene specific primer and the common primer occurs. Commercial cDNA pools modified for use in RACE are available.

Another PCR-based method generates full-length cDNA library with anchored ends without specific knowledge of the cDNA sequence. The method uses lock-docking primers (I-VI), where one primer, poly TV (I-III) locks over the polyA tail of eukaryotic mRNA producing first strand synthesis and a second primer, polyGH (IV-VI) locks onto the polyC tail added by terminal deoxynucleotidyl transferase (TdT). This method is described in PCT Pub. No. WO 96/40998.

The promoter region of a gene generally is located 5' to the initiation site for RNA polymerase II. Hundreds of promoter regions contain the "TATA" box, a sequence such as TATTA or TATAA, which is sensitive to mutations. The promoter region can be obtained by performing 5' RACE using a primer from the coding region of the gene. Alternatively, the cDNA can be used as a probe for the genomic sequence, and the region 5' to the coding region is identified by "walking up."

If the gene is highly expressed or differentially expressed, the promoter from the gene may be of use in a regulatory construct for a heterologous gene.

Once the full-length cDNA or gene is obtained, DNA encoding variants can be prepared by site-directed mutagenesis, described in detail in Sambrook *et al*., 15.3-15.63. The choice of codon or nucleotide to be replaced can be based on the disclosure herein on optional changes in amino acids to achieve altered protein structure and/or function.

As an alternative method to obtaining DNA or RNA from a biological material, nucleic acid comprising nucleotides having the sequence of one or more nucleic acids of the invention can be synthesized. Thus, the invention encompasses nucleic acid molecules ranging in length from 12 nucleotides (corresponding to at least 12 contiguous nucleotides which hybridize under stringent conditions to or are at least 80% identical to a nucleic acid represented by one of SEQ ID Nos. 1-850, preferably SEQ ID Nos. 1-383, even more preferably SEQ ID Nos. 1-127, or a sequence complementary thereto) up to a maximum length suitable for one or more biological manipulations, including replication and expression, of the nucleic acid molecule. The invention includes but is not limited to (a) nucleic acid having the size of a full gene, and comprising at least one of SEQ ID Nos. 1-850, preferably SEQ ID Nos. 1-383, even more preferably SEQ ID Nos. 1-127, or a sequence complementary thereto; (b) the nucleic acid of (a) also comprising at least one additional gene, operably linked to permit expression of a fusion protein; (c) an expression vector comprising (a) or (b); (d) a plasmid comprising (a) or (b); and (e) a recombinant viral particle comprising (a) or (b). Construction of (a) can be accomplished as described below in part IV.

The sequence of a nucleic acid of the present invention is not limited and can be any sequence of A, T, G, and/or C (for DNA) and A, U, G, and/or C (for RNA) or modified bases thereof, including inosine and pseudouridine. The choice of sequence will depend on the desired function and can be dictated by coding regions desired, the intron-like regions desired, and the regulatory regions desired.

### VI. Vectors Carrying Nucleic Acids of the Present Invention

The invention further provides plasmids and vectors, which can be used to express a gene in a host cell. The host cell may be any prokaryotic or eukaryotic cell. Thus, a nucleotide sequence derived from any one of SEQ ID Nos. 1-850, preferably SEQ ID Nos. 1-383, even more preferably SEQ ID Nos. 1-127, or a sequence complementary thereto, encoding all or a selected portion of a protein, can be used to produce a recombinant form of an polypeptide via microbial or eukaryotic cellular processes. Ligating the polynucleotide sequence into a gene construct, such as an expression vector, and transforming or transfecting into hosts, either eukaryotic (yeast, avian, insect or mammalian) or prokaryotic (bacterial cells), are standard procedures well known in the art.

Vectors that allow expression of a nucleic acid in a cell are referred to as expression vectors. Typically, expression vectors contain a nucleic acid operably linked to at least one transcriptional regulatory sequence. Regulatory sequences are art-recognized and are selected to direct expression of the subject nucleic acids. Transcriptional regulatory sequences are described in Goeddel; Gene Expression Technology: Methods in Enzymology 185, Academic Press, San Diego, CA (1990). In one embodiment, the expression vector includes a recombinant gene encoding a peptide having an agonistic activity of a subject polypeptide, or alternatively, encoding a peptide which is an antagonistic form of a subject polypeptide.

The choice of plasmid will depend on the type of cell in which propagation is desired and the purpose of propagation. Certain vectors are useful for amplifying and making large amounts of the desired DNA sequence. Other vectors are suitable for expression in cells in culture. Still other vectors are suitable for transfer and expression in cells in a whole animal or person. The choice of appropriate vector is well within the skill of the art. Many such vectors are available commercially. The nucleic acid or full-length gene is inserted into a vector typically by means of DNA ligase attachment to a cleaved restriction enzyme site in the vector. Alternatively, the desired nucleotide sequence may be inserted by homologous recombination in vivo. Typically this is accomplished by attaching regions of homology to the vector on the flanks of the desired nucleotide sequence. Regions of homology are added by ligation of oligonucleotides, or by polymerase chain reaction using primers comprising both the region of homology and a portion of the desired nucleotide sequence, for example.

Nucleic acids or full-length genes are linked to regulatory sequences as appropriate to obtain the desired expression properties. These may include promoters (attached either at the 5' end of the sense strand or at the 3' end of the antisense strand), enhancers, terminators, operators, repressors, and inducers. The promoters may be regulated or constitutive. In some situations it may be desirable to use conditionally active promoters, such as tissue-specific or developmental stage-specific promoters. These are linked to the desired nucleotide sequence using the techniques described above for linkage to vectors. Any techniques known in the art may be used.

When any of the above host cells, or other appropriate host cells or organisms, are used to replicate and/or express the polynucleotides or nucleic acids of the invention, the resulting replicated nucleic acid, RNA, expressed protein or polypeptide, is within the scope of the invention as a product of the host cell or organism. The product is recovered by any appropriate means known in the art.

Once the gene corresponding to the nucleic acid is identified, its expression can be regulated in the cell to which the gene is native. For example, an endogenous gene of a cell can be regulated by an exogenous regulatory sequence as disclosed in U.S. Patent No. 5,641,670, "Protein Production and Protein Delivery."

A number of vectors exist for the expression of recombinant proteins in yeast (see, for example, Broach *et al*. (1983) in Experimental Manipulation of Gene Expression, ed. M. Inouye, Academic Press, p. 83, incorporated by reference herein). In addition, drug resistance markers such as ampicillin can be used. In an illustrative embodiment, a polypeptide is produced recombinantly utilizing an expression vector generated by sub-cloning one of the nucleic acids represented in one of SEQ ID Nos. 1-850, preferably SEQ ID Nos. 1-383, even more preferably SEQ ID Nos. 1-127, or a sequence complementary thereto.

The preferred mammalian expression vectors contain both prokaryotic sequences, to facilitate the propagation of the vector in bacteria, and one or more eukaryotic transcription units that are expressed in eukaryotic cells. The various methods employed in the preparation of plasmids and transformation of host organisms are well known in the art. For other suitable expression systems for both prokaryotic and eukaryotic cells, as well as general recombinant procedures, see Molecular Cloning: A Laboratory Manual, 2^{nd} Ed., ed. by Sambrook, Fritsch and Maniatis (Cold Spring Harbor Laboratory Press: 1989) Chapters 16 and 17.
When it is desirable to express only a portion of a gene, e.g., a truncation mutant, it may be necessary to add a start codon (ATG) to the oligonucleotide fragment containing the desired sequence to be expressed. It is well known in the art that a methionine at the N-terminal position can be enzymatically cleaved by the use of the enzyme methionine aminopeptidase (MAP). MAP has been cloned from E. coli (Ben-Bassat *et al*. (1987) J. Bacteriol. 169:751-757) and Salmonella typhimurium and its *in vitro* activity has been demonstrated on recombinant proteins (Miller *et al*. (1987) PNAS 84:2718-1722). Therefore, removal of an N-terminal methionine, if desired, can be achieved either *in vivo* by expressing polypeptides in a host which produces MAP (e.g., E. coli or CM89 or S. cerevisiae), or *in vitro* by use of purified MAP (e.g., procedure of Miller *et al*., *supra*).

Moreover, the nucleic acid constructs of the present invention can also be used as part of a gene therapy protocol to deliver nucleic acids such as antisense nucleic acids. Thus, another aspect of the invention features expression vectors for *in vivo* or *in vitro* transfection with an antisense oligonucleotide.

In addition to viral transfer methods, non-viral methods can also be employed to introduce a subject nucleic acid, e.g., a sequence represented by one of SEQ ID Nos. 1-850, preferably SEQ ID Nos. 1-383, even more preferably SEQ ID Nos. 1-127, or a sequence complementary thereto, into the tissue of an animal. Most nonviral methods of gene transfer rely on normal mechanisms used by mammalian cells for the uptake and intracellular transport of macromolecules. In preferred embodiments, non-viral targeting means of the present invention rely on endocytic pathways for the uptake of the subject nucleic acid by the targeted cell. Exemplary targeting means of this type include liposomal derived systems, polylysine conjugates, and artificial viral envelopes.

A nucleic acid of any of SEQ ID Nos. 1-850, preferably SEQ ID Nos. 1-383, even more preferably SEQ ID Nos. 1-127, or a sequence complementary thereto, the corresponding cDNA, or the full-length gene may be used to express the partial or complete gene product. Appropriate nucleic acid constructs are purified using standard recombinant DNA techniques as described in, for example, Sambrook *et al*., (1989) *Molecular Cloning: A Laboratory Manual*, 2nd ed. (Cold Spring Harbor Press, Cold Spring Harbor, New York), and under current regulations described in United States Dept. of HHS, National Institute of Health (NIH) Guidelines for Recombinant DNA Research. The polypeptides encoded by the nucleic acid may be expressed in any expression system, including, for example, bacterial, yeast, insect, amphibian and mammalian systems. Suitable vectors and host cells are described in U.S. Patent No. 5,654,173.

Bacteria. Expression systems in bacteria include those described in *Chang et al*., *Nature* (1978) *275*:615, Goeddel *et al*., *Nature* (1979) *281*:544, Goeddel *et al*., *Nucleic Acids Res.* (1980) *8*:4057; EP 0 036,776, U.S. Patent No. 4,551,433, DeBoer *et al*., *Proc. Natl. Acad. Sci. (USA)* (1983) *80*:2125, and Siebenlist *et al*., *Cell* (1980) *20*:269.

Yeast. Expression systems in yeast include those described in Hinnen *et al*., *Proc. Natl. Acad. Sci. (USA)* (1978) *75*:1929; Ito *et al*., *J. Bacteriol*. (1983) *153*:163; Kurtz *et al*., *Mol. Cell. Biol*. (1986) *6*:142; *Kunze et al*., *J. Basic Microbiol*. (1985) *25*:141; Gleeson *et al*., *J. Gen. Microbiol.* (1986) *132*:3459, Roggenkamp *et al*., *Mol. Gen. Genet*. (1986) *202*:302) *Das et al*., *J. Bacteriol*. (1984) *158*:1165; De Louvencourt *et al*., *J. Bacteriol*. (1983) *154*:737, Van den *Berg et al*., *Bio*/*Technology* (1990) *8*:135; Kunze *et al*., *J. Basic Microbiol.* (1985) *25*:141; Cregg *et al*., *Mol. Cell. Biol*. (1985) *5*:3376, U.S. Patent Nos. 4,837,148 and 4,929,555; Beach and Nurse, *Nature* (1981) *300*:706; Davidow *et al*., *Curr. Genet.* (1985) *10*:380, Gaillardin *et al*., *Curr. Genet.* (1985) *10*:49, Ballance *et al*., *Biochem. Biophys. Res. Commun.* (1983) *112*:284289; Tilburn *et al*., *Gene* (1983) *26*:205221, *Yelton et al*., *Proc. Natl. Acad. Sci. (USA)* (1984) *81*:14701474, Kelly and Hynes, *EMBO J.* (1985) *4*:475479; EP 0 244,234, and WO 91/00357.

Insect Cells. Expression of heterologous genes in insects is accomplished as described in U.S. Patent No. 4,745,051, Friesen *et al*. (1986) "The Regulation of Baculovirus Gene Expression" in: The Molecular Biology Of Baculoviruses (W. Doerfler, ed.), EP 0 127,839, EP 0 155,476, and *Vlak et al*., *J. Gen. Virol*. (1988) *69*:765776, Miller *et al*., *Ann. Rev. Microbiol*. (1988) *42*:177, Carbonell *et al*., *Gene* (1988) *73*:409, Maeda *et al*., *Nature* (1985) *315*:592594, LebacqVerheyden *et al*., *Mol. Cell. Biol*. (1988) *8*:3129; *Smith et al*., *Proc. Natl. Acad. Sci. (USA)* (1985) 82:8404, Miyajima *et al*., *Gene* (1987) *58*:273; and Martin *et al*., *DNA* (1988) 7:99. Numerous baculoviral strains and variants and corresponding permissive insect host cells from hosts are described in Luckow *et al*., *Bio*/*Technology* (1988) *6*:4755, Miller *et al*., Generic Engineering (Setlow, J.K. *et al*. eds.), Vol. 8 (Plenum Publishing, 1986), pp. 277279, and Maeda *et al*., *Nature,* (1985) *315*:592-594.

Mammalian Cells. Mammalian expression is accomplished as described in Dijkema *et al*., *EMBO J.* (1985) *4*:761, Gorman *et al*., *Proc. Natl. Acad. Sci. (USA)* (1982) *79*:6777, Boshart *et al*., *Cell* (1985) *41*:521 and U.S. Patent No. 4,399,216. Other features of mammalian expression are facilitated as described in Ham and Wallace, *Meth. Enz*. (1979) *58*:44, Bames and Sato, *Anal. Biochem.* (1980) *102*:255, U.S. Patent Nos. 4,767,704, 4,657,866, 4,927,762, 4,560,655, WO 90/103430, WO 87/00195, and U.S. RE 30,985.

### VII. Therapeutic Nucleic Acid Constructs

One aspect of the invention relates to the use of the isolated nucleic acid, e.g., SEQ ID Nos. 1-850, preferably SEQ ID Nos. 1-383, even more preferably SEQ ID Nos. 1-127, or a sequence complementary thereto, in antisense therapy. As used herein, antisense therapy refers to administration or *in situ* generation of oligonucleotide molecules or their derivatives which specifically hybridize (e.g., bind) under cellular conditions with the cellular mRNA and/or genomic DNA, thereby inhibiting transcription and/or translation of that gene. The binding may be by conventional base pair complementarity, or, for example, in the case of binding to DNA duplexes, through specific interactions in the major groove of the double helix. In general, antisense therapy refers to the range of techniques generally employed in the art, and includes any therapy which relies on specific binding to oligonucleotide sequences.

An antisense construct of the present invention can be delivered, for example, as an expression plasmid which, when transcribed in the cell, produces RNA which is complementary to at least a unique portion of the cellular mRNA. Alternatively, the antisense construct is an oligonucleotide probe which is generated *ex vivo* and which, when introduced into the cell, causes inhibition of expression by hybridizing with the mRNA and/or genomic sequences of a subject nucleic acid. Such oligonucleotide probes are preferably modified oligonucleotides which are resistant to endogenous nucleases, e.g., exonucleases and/or endonucleases, and are therefore stable *in vivo.* Exemplary nucleic acid molecules for use as antisense oligonucleotides are phosphoramidate, phosphorothioate and methylphosphonate analogs of DNA (see also U.S. Patents 5,176,996; 5,264,564; and 5,256,775). Additionally, general approaches to constructing oligomers useful in antisense therapy have been reviewed, for example, by Van der Krol et al. (1988) BioTechniques 6:958-976; and Stein et al. (1988) Cancer Res 48:2659-2668. With respect to antisense DNA, oligodeoxyribonucleotides derived from the translation initiation site, e.g., between the -10 and +10 regions of the nucleotide sequence of interest, are preferred.

Antisense approaches involve the design of oligonucleotides (either DNA or RNA) that are complementary to mRNA. The antisense oligonucleotides will bind to the mRNA transcripts and prevent translation. Absolute complementarity, although preferred, is not required. In the case of double-stranded antisense nucleic acids, a single strand of the duplex DNA may thus be tested, or triplex formation may be assayed. The ability to hybridize will depend on both the degree of complementarity and the length of the antisense nucleic acid. Generally, the longer the hybridizing nucleic acid, the more base mismatches with an RNA it may contain and still form a stable duplex (or triplex, as the case may be). One skilled in the art can ascertain a tolerable degree of mismatch by use of standard procedures to determine the melting point of the hybridized complex.

Oligonucleotides that are complementary to the 5' end of the mRNA, e.g., the 5' untranslated sequence up to and including the AUG initiation codon, should work most efficiently at inhibiting translation. However, sequences complementary to the 3' untranslated sequences of mRNAs have recently been shown to be effective at inhibiting translation of mRNAs as well. (Wagner, R. 1994. Nature 372:333). Therefore, oligonucleotides complementary to either the 5' or 3' untranslated, noncoding regions of a gene could be used in an antisense approach to inhibit translation of endogenous mRNA. Oligonucleotides complementary to the 5' untranslated region of the mRNA should include the complement of the AUG start codon. Antisense oligonucleotides complementary to mRNA coding regions are typically less efficient inhibitors of translation but could also be used in accordance with the invention. Whether designed to hybridize to the 5', 3', or coding region of subject mRNA, antisense nucleic acids should be at least six nucleotides in length, and are preferably less that about 100 and more preferably less than about 50, 25, 17 or 10 nucleotides in length.

Regardless of the choice of target sequence, it is preferred that *in vitro* studies are first performed to quantitate the ability of the antisense oligonucleotide to quantitate the ability of the antisense oligonucleotide to inhibit gene expression. It is preferred that these studies utilize controls that distinguish between antisense gene inhibition and nonspecific biological effects of oligonucleotides. It is also preferred that these studies compare levels of the target RNA or protein with that of an internal control RNA or protein. Additionally, it is envisioned that results obtained using the antisense oligonucleotide are compared with those obtained using a control oligonucleotide. It is preferred that the control oligonucleotide is of approximately the same length as the test oligonucleotide and that the nucleotide sequence of the oligonucleotide differs from the antisense sequence no more than is necessary to prevent specific hybridization to the target sequence.

The oligonucleotides can be DNA or RNA or chimeric mixtures or derivatives or modified versions thereof, single-stranded or double-stranded. The oligonucleotide can be modified at the base moiety, sugar moiety, or phosphate backbone, for example, to improve stability of the molecule, hybridization, etc. The oligonucleotide may include other appended groups such as peptides (e.g., for targeting host cell receptors), or agents facilitating transport across the cell membrane (see, e.g., Letsinger et al., 1989, Proc. Natl. Acad. Sci. U.S.A. 86:6553-6556; Lemaitre et al., 1987, Proc. Natl. Acad. Sci. 84:648-652; PCT Publication No. WO 88/09810, published December 15, 1988) or the blood-brain barrier (see, e.g., PCT Publication No. WO 89/10134, published April 25, 1988), hybridization-triggered cleavage agents (See, e.g., Krol et al., 1988, BioTechniques 6:958-976), or intercalating agents (See, e.g., Zon, 1988, Pharm. Res. 5:539-549). To this end, the oligonucleotide may be conjugated to another molecule, e.g., a peptide, hybridization triggered cross-linking agent, transport agent, hybridization-triggered cleavage agent, etc.

The antisense oligonucleotide may comprise at least one modified base moiety which is selected from the group including but not limited to 5-fluorouracil, 5-bromouracil, 5-chlorouracil, 5-iodouracil, hypoxanthine, xantine, 4-acetylcytosine, 5-(carboxyhydroxytriethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5- oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine.

The antisense oligonucleotide may also comprise at least one modified sugar moiety selected from the group including but not limited to arabinose, 2-fluoroarabinose, xylulose, and hexose.

The antisense oligonucleotide can also contain a neutral peptide-like backbone. Such molecules are termed peptide nucleic acid (PNA)-oligomers and are described, e.g., in Perry-O'Keefe et al. (1996) Proc. Natl. Acad. Sci. U.S.A. 93:14670 and in Eglom *et al*. (1993) Nature 365:566. One advantage of PNA oligomers is their capability to bind to complementary DNA essentially independently from the ionic strength of the medium due to the neutral backbone of the DNA. In yet another embodiment, the antisense oligonucleotide comprises at least one modified phosphate backbone selected from the group consisting of a phosphorothioate, a phosphorodithioate, a phosphoramidothioate, a phosphoramidate, a phosphordiamidate, a methylphosphonate, an alkyl phosphotriester, and a formacetal or analog thereof.

In yet a further embodiment, the antisense oligonucleotide is an α-anomeric oligonucleotide. An α-anomeric oligonucleotide forms specific double-stranded hybrids with complementary RNA in which, contrary to the usual β-units, the strands run parallel to each other (Gautier et al., 1987, Nucl. Acids Res. 15:6625-6641). The oligonucleotide is a 2'-O-methylribonucleotide (Inoue et al., 1987, Nucl. Acids Res. 15:6131-12148), or a chimeric RNA-DNA analogue (Inoue et al., 1987, FEBS Lett. 215:327-330).

Oligonucleotides of the invention may be synthesized by standard methods known in the art, e.g., by use of an automated DNA synthesizer (such as are commercially available from Biosearch, Applied Biosystems, etc.). As examples, phosphorothioate oligonucleotides may be synthesized by the method of Stein et al. (1988, Nucl. Acids Res. 16:3209), methylphosphonate olgonucleotides can be prepared by use of controlled pore glass polymer supports (Sarin et al., 1988, Proc. Natl. Acad. Sci. U.S.A. 85:7448-7451), etc.

While antisense nucleotides complementary to a coding region sequence can be used, those complementary to the transcribed untranslated region and to the region comprising the initiating methionine are most preferred.

The antisense molecules can be delivered to cells which express the target nucleic acid *in vivo.* A number of methods have been developed for delivering antisense DNA or RNA to cells; e.g., antisense molecules can be injected directly into the tissue site, or modified antisense molecules, designed to target the desired cells (e.g., antisense linked to peptides or antibodies that specifically bind receptors or antigens expressed on the target cell surface) can be administered systemically.

However, it is often difficult to achieve intracellular concentrations of the antisense sufficient to suppress translation on endogenous mRNAs. Therefore, a preferred approach utilizes a recombinant DNA construct in which the antisense oligonucleotide is placed under the control of a strong pol III or pol II promoter. The use of such a construct to transfect target cells in the patient will result in the transcription of sufficient amounts of single stranded RNAs that will form complementary base pairs with the endogenous transcripts and thereby prevent translation of the target mRNA. For example, a vector can be introduced *in vivo* such that it is taken up by a cell and directs the transcription of an antisense RNA. Such a vector can remain episomal or become chromosomally integrated, as long as it can be transcribed to produce the desired antisense RNA. Such vectors can be constructed by recombinant DNA technology methods standard in the art. Vectors can be plasmid, viral, or others known in the art for replication and expression in mammalian cells. Expression of the sequence encoding the antisense RNA can be by any promoter known in the art to act in mammalian, preferably human cells. Such promoters can be inducible or constitutive. Such promoters include but are not limited to: the SV40 early promoter region (Bernoist and Chambon, 1981, Nature 290:304-310), the promoter contained in the 3' long terminal repeat of Rous sarcoma virus (Yamamoto *et al*., 1980, Cell 22:787-797), the herpes thymidine kinase promoter (Wagner et al., 1981, Proc. Natl. Acad. Sci. U.S.A. 78:1441-1445), the regulatory sequences of the metallothionein gene (Brinster et al, 1982, Nature 296:39-42), etc. Any type of plasmid, cosmid, YAC or viral vector can be used to prepare the recombinant DNA construct which can be introduced directly into the tissue site; e.g., the choroid plexus or hypothalamus. Alternatively, viral vectors can be used which selectively infect the desired tissue (e.g., for brain, herpesvirus vectors may be used), in which case administration may be accomplished by another route (e.g., systemically).

In another aspect of the invention, ribozyme molecules designed to catalytically cleave target mRNA transcripts can be used to prevent translation of target mRNA and expression of a target protein (See, e.g., PCT International Publication WO90/11364, published October 4, 1990; Sarver *et al*., 1990, Science 247:1222-1225 and U.S. Patent No. 5,093,246). While ribozymes that cleave mRNA at site specific recognition sequences can be used to destroy target mRNAs, the use of hammerhead ribozymes is preferred. Hammerhead ribozymes cleave mRNAs at locations dictated by flanking regions that form complementary base pairs with the target mRNA. The sole requirement is that the target mRNA have the following sequence of two bases: 5'-UG-3'. The construction and production of hammerhead ribozymes is well known in the art and is described more fully in Haseloff and Gerlach, 1988, Nature, 334:585-591. Preferably the ribozyme is engineered so that the cleavage recognition site is located near the 5' end of the target mRNA; i.e., to increase efficiency and minimize the intracellular accumulation of non-functional mRNA transcripts.

The ribozymes of the present invention also include RNA endoribonucleases (hereinafter "Cech-type ribozymes") such as the one which occurs naturally in *Tetrahymena thermophila* (known as the IVS, or L-19 IVS RNA) and which has been extensively described by Thomas Cech and collaborators (Zaug, et al., 1984, Science, 224:574-578; Zaug and Cech, 1986, Science, 231:470-475; Zaug, et al., 1986, Nature, 324:429-433; published International patent application No. WO88/04300 by University Patents Inc.; Been and Cech, 1986, Cell, 47:207-216). The Cech-type ribozymes have an eight base pair active site which hybridizes to a target RNA sequence whereafter cleavage of the target RNA takes place. The invention encompasses those Cech-type ribozymes which target eight base-pair active site sequences that are present in a target gene.

As in the antisense approach, the ribozymes can be composed of modified oligonucleotides (e.g., for improved stability, targeting, etc.) and should be delivered to cells which express the target gene *in vivo.* A preferred method of delivery involves using a DNA construct "encoding" the ribozyme under the control of a strong constitutive pol III or pol II promoter, so that transfected cells will produce sufficient quantities of the ribozyme to destroy endogenous messages and inhibit translation. Because ribozymes, unlike antisense molecules, are catalytic, a lower intracellular concentration is required for efficiency.

Antisense RNA, DNA, and ribozyme molecules of the invention may be prepared by any method known in the art for the synthesis of DNA and RNA molecules. These include techniques for chemically synthesizing oligodeoxyribonucleotides and oligoribonucleotides well known in the art such as for example solid phase phosphoramidite chemical synthesis. Alternatively, RNA molecules may be generated by *in vitro* and *in vivo* transcription of DNA sequences encoding the antisense RNA molecule. Such DNA sequences may be incorporated into a wide variety of vectors which incorporate suitable RNA polymerase promoters such as the T7 or SP6 polymerase promoters. Alternatively, antisense cDNA constructs that synthesize antisense RNA constitutively or inducibly, depending on the promoter used, can be introduced stably into cell lines.

Moreover, various well-known modifications to nucleic acid molecules may be introduced as a means of increasing intracellular stability and half-life. Possible modifications include but are not limited to the addition of flanking sequences of ribonucleotides or deoxyribonucleotides to the 5' and/or 3' ends of the molecule or the use of phosphorothioate or 2' O-methyl rather than phosphodiesterase linkages within the oligodeoxyribonucleotide backbone.

### VIII. Polypeptides of the Present Invention

The present invention makes available isolated polypeptides which are isolated from, or otherwise substantially free of other cellular proteins, especially other signal transduction factors and/or transcription factors which may normally be associated with the polypeptide. Subject polypeptides of the present invention include polypeptides encoded by the nucleic acids of SEQ ID Nos. 1-850, preferably SEQ ID Nos. 1-383, even more preferably SEQ ID Nos. 1-127, or a sequence complementary thereto, or polypeptides encoded by genes of which a sequence in SEQ ID Nos. 1-850, preferably SEQ ID Nos. 1-383, even more preferably SEQ ID Nos. 1-127, or a sequence complementary thereto, is a fragment. Polypeptides of the present invention include those proteins which are differentially regulated in tumor cells, especially colon cancer-derived cell lines (relative to normal cells, e.g., normal colon tissue and non-colon tissue). In preferred embodiments, the polypeptides are upregulated in tumor cells, especially colon cancer cancer-derived cell lines. In other embodiments, the polypeptides are downregulated in tumor cells, especially colon cancer-derived cell lines. Proteins which are upregulated, such as oncogenes, or downregulated, such as tumor suppressors, in aberrantly proliferating cells may be targets for diagnostic or therapeutic techniques. For example, upregulation of the *cdc2* gene induces mitosis. Overexpression of the *myt1* gene, a mitotic deactivator, negatively regulates the activity of *cdc2.* Aberrant proliferation may thus be induced either by upregulating *cdc2* or by downregulating *myt1*

The term "substantially free of other cellular proteins" (also referred to herein as "contaminating proteins") or "substantially pure or purified preparations" are defined as encompassing preparations of polypeptides having less than about 20% (by dry weight) contaminating protein, and preferably having less than about 5% contaminating protein. Functional forms of the subject polypeptides can be prepared, for the first time, as purified preparations by using a cloned nucleic acid as described herein. Full length proteins or fragments corresponding to one or more particular motifs and/or domains or to arbitrary sizes, for example, at least about 5, 10, 25, 50, 75, or 100 amino acids in length are within the scope of the present invention.

For example, isolated polypeptides can be encoded by all or a portion of a nucleic acid sequence shown in any of SEQ ID Nos. 1-850, preferably SEQ ID Nos. 1-383, even more preferably SEQ ID Nos. 1-127, or a sequence complementary thereto. Isolated peptidyl portions of proteins can be obtained by screening peptides recombinantly produced from the corresponding fragment of the nucleic acid encoding such peptides. In addition, fragments can be chemically synthesized using techniques known in the art such as conventional Merrifield solid phase f-Moc or t-Boc chemistry. For example, a polypeptide of the present invention may be arbitrarily divided into fragments of desired length with no overlap of the fragments, or preferably divided into overlapping fragments of a desired length. The fragments can be produced (recombinantly or by chemical synthesis) and tested to identify those peptidyl fragments which can function as either agonists or antagonists of a wild-type (e.g., "authentic") protein.

Another aspect of the present invention concerns recombinant forms of the subject proteins. Recombinant polypeptides preferred by the present invention, in addition to native proteins as described above are encoded by a nucleic acid, which is at least 60%, more preferably at least 80%, and more preferably 85%, and more preferably 90%, and more preferably 95% identical to an amino acid sequence encoded by SEQ ID NOs. 1-850. Polypeptides which are encoded by a nucleic acid that is at least about 98-99% identical with the sequence of SEQ ID Nos. 1-850 are also within the scope of the invention. Also included in the present invention are peptide fragments comprising at least a portion of such a protein.

In a preferred embodiment, a polypeptide of the present invention is a mammalian polypeptide and even more preferably a human polypeptide. In particularly preferred embodiment, the polypeptide retains wild-type bioactivity. It will be understood that certain post-translational modifications, e.g., phosphorylation and the like, can increase the apparent molecular weight of the polypeptide relative to the unmodified polypeptide chain.

The present invention further pertains to recombinant forms of one of the subject polypeptides. Such recombinant polypeptides preferably are capable of functioning in one of either role of an agonist or antagonist of at least one biological activity of a wild-type ("authentic") polypeptide of the appended sequence listing. The term "evolutionarily related to", with respect to amino acid sequences of proteins, refers to both polypeptides having amino acid sequences which have arisen naturally, and also to mutational variants of human polypeptides which are derived, for example, by combinatorial mutagenesis.

In general, polypeptides referred to herein as having an activity (e.g., are "bioactive") of a protein are defined as polypeptides which include an amino acid sequence encoded by all or a portion of the nucleic acid sequences shown in one of SEQ ID Nos. 1-850, preferably SEQ ID Nos. 1-383, even more preferably SEQ ID Nos. 1-127, or a sequence complementary thereto, and which mimic or antagonize all or a portion of the biological/biochemical activities of a naturally occurring protein. According to the present invention, a polypeptide has biological activity if it is a specific agonist or antagonist of a naturally occurring form of a protein.

Assays for determining whether a compound, e.g, a protein or variant thereof, has one or more of the above biological activities are well known in the art. In certain embodiments, the polypeptides of the present invention have activities such as those outlined above.

In another embodiment, the coding sequences for the polypeptide can be incorporated as a part of a fusion gene including a nucleotide sequence encoding a different polypeptide. This type of expression system can be useful under conditions where it is desirable to produce an immunogenic fragment of a polypeptide (see, for example, EP Publication No: 0259149; and Evans *et al*. (1989) Nature 339:385; Huang *et al*. (1988) J. Virol. 62:3855; and Schlienger *et al*. (1992) J. Virol. 66:2). In addition to utilizing fusion proteins to enhance immunogenicity, it is widely appreciated that fusion proteins can also facilitate the expression of proteins, and, accordingly, can be used in the expression of the polypeptides of the present invention (see, for example, Current Protocols in Molecular Biology, eds. Ausubel et al. (N.Y.: John Wiley & Sons, 1991)). In another embodiment, a fusion gene coding for a purification leader sequence, such as a poly-(His)/enterokinase cleavage site sequence at the N-terminus of the desired portion of the recombinant protein, can allow purification of the expressed fusion protein by affinity chromatography using a Ni²⁺ metal resin. The purification leader sequence can then be subsequently removed by treatment with enterokinase to provide the purified protein (e.g., see Hochuli *et al*. (1987) J. Chromatography 411:177; and Janknecht *et al*. PNAS 88:8972).

Techniques for making fusion genes are known to those skilled in the art. Essentially, the joining of various DNA fragments coding for different polypeptide sequences is performed in accordance with conventional techniques, employing blunt-ended or stagger-ended termini for ligation, restriction enzyme digestion to provide for appropriate termini, filling-in of cohesive ends as appropriate, alkaline phosphatase treatment to avoid undesirable joining, and enzymatic ligation. In another embodiment, the fusion gene can be synthesized by conventional techniques including automated DNA synthesizers. Alternatively, PCR amplification of nucleic acid fragments can be carried out using anchor primers which give rise to complementary overhangs between two consecutive nucleic acid fragments which can subsequently be annealed to generate a chimeric nucleic acid sequence (see, for example, Current Protocols in Molecular Biology, eds. Ausubel et al. John Wiley & Sons: 1992).

The present invention further pertains to methods of producing the subject polypeptides. For example, a host cell transfected with a nucleic acid vector directing expression of a nucleotide sequence encoding the subject polypeptides can be cultured under appropriate conditions to allow expression of the peptide to occur. Suitable media for cell culture are well known in the art. The recombinant polypeptide can be isolated from cell culture medium, host cells, or both using techniques known in the art for purifying proteins including ion-exchange chromatography, gel filtration chromatography, ultrafiltration, electrophoresis, and immunoaffinity purification with antibodies specific for such peptide. In a preferred embodiment, the recombinant polypeptide is a fusion protein containing a domain which facilitates its purification, such as GST fusion protein.

Moreover, it will be generally appreciated that, under certain circumstances, it may be advantageous to provide homologs of one of the subject polypeptides which function in a limited capacity as one of either an agonist (mimetic) or an antagonist, in order to promote or inhibit only a subset of the biological activities of the naturally occurring form of the protein. Thus, specific biological effects can be elicited by treatment with a homolog of limited function, and with fewer side effects relative to treatment with agonists or antagonists which are directed to all of the biological activities of naturally occurring forms of subject proteins.

Homologs of each of the subject polypeptide can be generated by mutagenesis, such as by discrete point mutation(s), or by truncation. For instance, mutation can give rise to homologs which retain substantially the same, or merely a subset, of the biological activity of the polypeptide from which it was derived. Alternatively, antagonistic forms of the polypeptide can be generated which are able to inhibit the function of the naturally occurring form of the protein, such as by competitively binding to a receptor.

The recombinant polypeptides of the present invention also include homologs of the wild-type proteins, such as versions of those proteins which are resistant to proteolytic cleavage, for example, due to mutations which alter ubiquitination or other enzymatic targeting associated with the protein.

Polypeptides may also be chemically modified to create derivatives by forming covalent or aggregate conjugates with other chemical moieties, such as glycosyl groups, lipids, phosphate, acetyl groups and the like. Covalent derivatives of proteins can be prepared by linking the chemical moieties to functional groups on amino acid sidechains of the protein or at the N-terminus or at the C-terminus of the polypeptide.

Modification of the structure of the subject polypeptides can be for such purposes as enhancing therapeutic or prophylactic efficacy, stability (e.g., *ex vivo* shelf life and resistance to proteolytic degradation), or post-translational modifications (e.g., to alter phosphorylation pattern of protein). Such modified peptides, when designed to retain at least one activity of the naturally occurring form of the protein, or to produce specific antagonists thereof, are considered functional equivalents of the polypeptides described in more detail herein. Such modified peptides can be produced, for instance, by amino acid substitution, deletion, or addition. The substitutional variant may be a substituted conserved amino acid or a substituted non-conserved amino acid.

For example, it is reasonable to expect that an isolated replacement of a leucine with an isoleucine or valine, an aspartate with a glutamate, a threonine with a serine, or a similar replacement of an amino acid with a structurally related amino acid (i.e., isosteric and/or isoelectric mutations) will not have a major effect on the biological activity of the resulting molecule. Conservative replacements are those that take place within a family of amino acids that are related in their side chains. Genetically encoded amino acids can be divided into four families: (1) acidic = aspartate, glutamate; (2) basic = lysine, arginine, histidine; (3) nonpolar = alanine, valine, leucine, isoleucine, proline, phenylalanine, methionine, tryptophan; and (4) uncharged polar = glycine, asparagine, glutamine, cysteine, serine, threonine, tyrosine. In similar fashion, the amino acid repertoire can be grouped as (1) acidic = aspartate, glutamate; (2) basic = lysine, arginine histidine, (3) aliphatic = glycine, alanine, valine, leucine, isoleucine, serine, threonine, with serine and threonine optionally be grouped separately as aliphatic-hydroxyl; (4) aromatic = phenylalanine, tyrosine, tryptophan; (5) amide = asparagine, glutamine; and (6) sulfur -containing = cysteine and methionine. (see, for example, Biochemistry, 2^{nd} ed., Ed. by L. Stryer, WH Freeman and Co.: 1981). Whether a change in the amino acid sequence of a peptide results in a functional homolog (e.g., functional in the sense that the resulting polypeptide mimics or antagonizes the wild-type form) can be readily determined by assessing the ability of the variant peptide to produce a response in cells in a fashion similar to the wild-type protein, or competitively inhibit such a response. Polypeptides in which more than one replacement has taken place can readily be tested in the same manner.The variant may be designed so as to retain biological activity of a particular region of the protein. In a non-limiting example, Osawa et al., 1994, Biochemistry and Molecular International 34:1003-1009, discusses the actin binding region of a protein from several different species. The actin binding regions of the these species are considered homologous based on the fact that they have amino acids that fall within "homologous residue groups." Homologous residues are judged according to the following groups (using single letter amino acid designations): STAG; ILVMF; HRK; DEQN; and FYW. For example, an S, a T, an A or a G can be in a position and the function (in this case actin binding) is retained.

Additional guidance on amino acid substitution is available from studies of protein evolution. Go et al., 1980, Int. J. Peptide Protein Res. 15:211-224, classified amino acid residue sites as interior or exterior depending on their accessibility. More frequent substitution on exterior sites was confirmed to be general in eight sets of homologous protein families regardless of their biological functions and the presence or absence of a prosthetic group. Virtually all types of amino acid residues had higher mutabilities on the exterior than in the interior. No correlation between mutability and polarity was observed of amino acid residues in the interior and exterior, respectively. Amino acid residues were classified into one of three groups depending on their polarity: polar (Arg, Lys, His, Gln, Asn, Asp, and Glu); weak polar (Ala, Pro, Gly, Thr, and Ser), and nonpolar (Cys, Val, Met, Ile, Leu, Phe, Tyr, and Trp). Amino acid replacements during protein evolution were very conservative: 88% and 76% of them in the interior or exterior, respectively, were within the same group of the three. Inter-group replacements are such that weak polar residues are replaced more often by nonpolar residues in the interior and more often by polar residues on the exterior.

Querol et al., 1996, Prot. Eng. 9:265-271, provides general rules for amino acid substitutions to enhance protein thermostability. New glycosylation sites can be introduced as discussed in Olsen and Thomsen, 1991, J. Gen. Microbiol. 137:579-585. An additional disulfide bridge can be introduced, as discussed by Perry and Wetzel, 1984, Science 226:555-557; Pantoliano et al., 1987, Biochemistry 26:2077-2082; Matsumura et al., 1989, Nature 342:291-293; Nishikawa et al., 1990, Protein Eng. 3:443-448; Takagi et al., 1990, J. Biol. Chem. 265:6874-6878; Clarke et al., 1993, Biochemistry 32:4322-4329; and Wakarchuk et al., 1994, Protein Eng. 7:1379-1386.

An additional metal binding site can be introduced, according to Toma et al., 1991, Biochemistry 30:97-106, and Haezerbrouck et al., 1993, Protein Eng. 6:643-649. Substitutions with prolines in loops can be made according to Masul et al., 1994, Appl. Env. Microbiol. 60:3579-3584; and Hardy et al., FEBS Lett. 317:89-92.

Cysteine-depleted muteins are considered variants within the scope of the invention. These variants can be constructed according to methods disclosed in U.S. Patent No. 4,959,314, which discloses how to substitute other amino acids for cysteines, and how to determine biological activity and effect of the substitution. Such methods are suitable for proteins according to this invention that have cysteine residues suitable for such substitutions, for example to eliminate disulfide bond formation.

To learn the identity and function of the gene that correlates with an nucleic acid, the nucleic acids or corresponding amino acid sequences can be screened against profiles of protein families. Such profiles focus on common structural motifs among proteins of each family. Publicly available profiles are described above. Additional or alternative profiles are described below.

In comparing a new nucleic acid with known sequences, several alignment tools are available. Examples include PileUp, which creates a multiple sequence alignment, and is described in Feng *et al*., *J. Mol. Evol.* (1987) *25*:351-360. Another method, GAP, uses the alignment method of Needleman *et al*., *J. Mol. Biol*. (1970) *48*:443-453. GAP is best suited for global alignment of sequences. A third method, BestFit, functions by inserting gaps to maximize the number of matches using the local homology algorithm of Smith and Waterman, *Adv. Appl. Math.* (1981) *2*:482-489.

Examples of such profiles are described below.

### Chemokines

Chemokines are a family of proteins that have been implicated in lymphocyte trafficking, inflammatory diseases, angiogenesis, hematopoiesis, and viral infection. See, for example, Rollins, *Blood* (1997) *90(3)*:909-928, and Wells *et al., J. Leuk Biol.* (1997) *61*:545-550. U.S. Patent No. 5,605,817 discloses DNA encoding a chemokine expressed in fetal spleen. U.S. Patent No. 5,656,724 discloses chemokine-like proteins and methods of use. U.S. Patent No. 5,602,008 discloses DNA encoding a chemokine expressed by liver.

Mutants of the encoded chemokines are polypeptides having an amino acid sequence that possesses at least one amino acid substitution, addition, or deletion as compared to native chemokines. Fragments possess the same amino acid sequence of the native chemokines; mutants may lack the amino and/or carboxyl terminal sequences. Fusions are mutants, fragments, or the native chemokines that also include amino and/or carboxyl terminal amino acid extensions.

The number or type of the amino acid changes is not critical, nor is the length or number of the amino acid deletions, or amino acid extensions that are incorporated in the chemokines as compared to the native chemokine amino acid sequences. A polynucleotide encoding one of these variant polypeptides will retain at least about 80% amino acid identity with at least one known chemokine. Preferably, these polypeptides will retain at least about 85% amino acid sequence identity, more preferably, at least about 90%; even more preferably, at least about 95%. In addition, the variants will exhibit at least 80%; preferably about 90%; more preferably about 95% of at least one activity exhibited by a native chemokine. Chemokine activity includes immunological, biological, receptor binding, and signal transduction functions of the native chemokine.

Chemotaxis. Assays for chemotaxis relating to neutrophils are described in Walz *et al., Biochem. Biophys. Res. Commun.* (1987) *149*:755, Yoshimura *et al*., *Proc. Natl. Acad Sci. (USA)* (1987) *84*:9233, and Schroder *et al., J. Immunol.* (1987) *139*:3474; to lymphocytes, Larsen *et al., Science* (1989) *243*:1464, Carr *et al., Proc. Natl. Acad. Sci. (USA)* (1994) *91*:3652; to tumor-infiltrating lymphocytes, Liao *et al., J. Exp. Med* (1995). *182*:1301; to hemopoietic progenitors, Aiuti *et al., J. Exp. Med.* (1997) *185*:111; to monocytes, Valente *et al., Biochem.* (1988) *27*:4162; and to natural killer cells, Loetscher *et al., J. Immunol.* (1996) *156*:322, and Allavena *et al., Eur. J. Immunol*. (1994) *24*:3233.

Assays for determining the biological activity of attracting eosinophils are described in Dahinden *et al., J. Exp. Med.* (1994) *179*:751, Weber *et al., J. Immunol.* (1995) *154*:4166, and Noso *et al., Biochem. Biophys. Res. Commun.* (1994) *200*:1470; for attracting dendritic cells, Sozzani *et al., J. Immunol.* (1995) *155*:3292; for attracting basophils, in Dahinden *et al., J. Exp. Med.* (1994) *179*:751, Alam *et al., J. Immunol.* (1994) *152*:1298, Alam *et al., J. Exp. Med.* (1992) *176*:781; and for activating neutrophils, Maghazaci *et al., Eur. J. Immunol*. (1996) *26*:315, and Taub *et al., J. Immunol*. (1995) *155*:3877. Native chemokines can act as mitogens for fibroblasts, assayed as described in Mullenbach *et al., J. Biol. Chem.* (1986) *261*:719.

Receptor Binding. Native chemokines exhibit binding activity with a number of receptors. Description of such receptors and assays to detect binding are described in, for example, Murphy *et al., Science* (1991) *253*:1280; Combadiere *et al., J. Biol. Chem.* (1995) *270*:29671; Daugherty *et al., J. Exp. Med*. (1996) *183*:2349; Samson *et al., Biochem*. (1996) *35*:3362; *Raport et al., J. Biol. Chem.* (1996) *271*:17161; Combadiere *et al., J. Leukoc. Biol.* (1996) *60*:147; Baba *et al., J. Biol. Chem.* (1997) *23*:14893; Yosida *et al., J. Biol. Chem.* (1997) *272*:13803; Arvannitakis *et al., Nature* (1997) *385*:347, and many other assays are known in the art.

Kinase Activiation. Assays for kinase activation are described by Yen *et al*., *J. Leukoc. Biol*. (1997) *61*:529; Dubois *et al*., *J. Immunol*. (1996) *156*:1356; Turner *et al*., *J. Immunol*. (1995) *155*:2437. Assays for inhibition of angiogenesis or cell proliferation are described in *Maione et al*., *Science* (1990) *247*:77. Glycosaminoglycan production can be induced by native chemokines, assayed as described in Castor *et al*., *Proc. Natl. Acad. Sci. (USA)* (1983) *80*:765. Chemokine-mediated histamine release from basophils is assayed as described in Dahinden *et al*., *J. Exp. Med*. (1989) *170*:1787; and White *et al*., *Immunol. Lett.* (1989) *22*:151. Heparin binding is described in Luster *et al*., *J. Exp. Med*. (1995) *182*:219.

Dimerization Activity. Chemokines can possess dimerization activity, which can be assayed according to Burrows *et al*., *Biochem*. (1994) *33*:12741; and Zhang *et al*., *Mol. Cell. Biol*. (1995) *15*:4851. Native chemokines can play a role in the inflammatory response of viruses. This activity can be assayed as described in Bleul *et al*., *Nature* (1996) *382*:829; and Oberlin *et al*., *Nature* (1996) *382*:833. Exocytosis of monocytes can be promoted by native chemokines. The assay for such activity is described in Uguccioni *et al*., *Eur. J. Immunol*. (1995) *25*:64. Native chemokines also can inhibit hemapoietic stem cell proliferation. The method for testing for such activity is reported in Graham *et al*., *Nature* (1990) *344*:442.

### Death Domain Proteins

Several protein families contain death domain motifs (Feinstein and Kimchi, *TIBS Letters* (1995) *20*:242-244). Some death domain-containing proteins are implicated in cytotoxic intracellular signaling (Cleveland and Ihle, *Cell* (1995) *81*:479-482, *Pan et al*, *Science* (1997) *276*:111-113, Duan and Dixit, *Nature* (1997) *385*:86-89, and Chinnaiyan *et al*, *Science* (1996) *274*:990-992). U.S. Patent No. 5,563,039 describes a protein homologous to TRADD (Tumor Necrosis Factor Receptor-1 Associated Death Domain containing protein), and modifications of the active domain of TRADD that retain the functional characteristics of the protein, as well as apoptosis assays for testing the function of such death domain containing proteins. U.S. Patent No. 5,658,883 discloses biologically active TGF-B1 peptides. U.S. Patent No. 5,674,734 discloses protein RIP which contains a C-terminal death domain and an N-terminal kinase domain.

### Leukemia Inhibitory Factor (LIF)

An LIF profile is constructed from sequences of leukemia inhibitor factor, CT-1 (cardiotrophin-1), CNTF (ciliary neurotrophic factor), OSM (oncostatin M), and IL-6 (interleukin-6). This profile encompasses a family of secreted cytokines that have pleiotropic effects on many cell types including hepatocytes, osteoclasts, neuronal cells and cardiac myocytes, and can be used to detect additional genes encoding such proteins. These molecules are all structurally related and share a common co-receptor gp130 which mediates intracellular signal transduction by cytoplasmic tyrosine kinases such as src.

Novel proteins related to this family are also likely to be secreted, to activate gp130 and to function in the development of a variety of cell types. Thus new members of this family would be candidates to be developed as growth or survival factors for the cell types that they stimulate. For more details on this family of cytokines, see Pennica *et al*, *Cytokine and Growth Factor Reviews* (1996) *7*:81-91. U.S. Patent No. 5,420,247 discloses LIF receptor and fusion proteins. U.S. Patent No. 5,443,825 discloses human LIF.

### Angiopoietin

Angiopoietin-1 is a secreted ligand of the TIE-2 tyrosine kinase; it functions as an angiogenic factor critical for normal vascular development. Angiopoietin-2 is a natural antagonist of angiopoietin-1 and thus functions as an anti-angiogenic factor. These two proteins are structurally similar and activate the same receptor. (Folkman and D'Amore, *Cell* (1996) *87*:1153-1155, and Davis *et al*., *Cell* (1996) *87*:1161-1169.)

The angiopoietin molecules are composed of two domains, a coiled-coil region and a region related to fibrinogen. The fibrinogen domain is found in many molecules including ficolin and tesascin, and is well defined structurally with many members.

### Receptor Protein-Tyrosine Kinases

Receptor Protein-Tyrosine Kinases or RPTKs are described in Lindberg, *Annu. Rev. Cell Biol.* (1994) *10*:251-337.

### Growth Factors: Epidermal Growth Factor (EGF) and Fibroblast Growth Factor (FGF)

For a discussion of growth factor superfamilies, see Growth Factors: A Practical Approach, Appendix A1 (Ed. McKay and Leigh, Oxford University Press, NY, 1993) pp. 237-243.

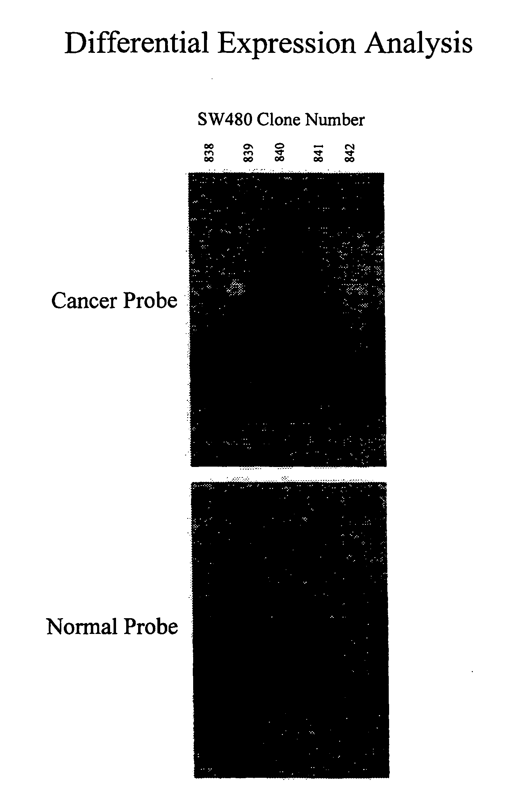

The alignments (pretty box) for EGF and FGF are shown in Figures 1 and 2, respectively. U.S. Patent No. 4,444,760 discloses acidic brain fibroblast growth factor, which is active in the promotion of cell division and wound healing. U.S. Patent No. 5,439,818 discloses DNA encoding human recombinant basic fibroblast growth factor, which is active in wound healing. U.S. Patent No. 5,604,293 discloses recombinant human basic fibroblast growth factor, which is useful for wound healing. U.S. Patent No. 5,410,832 discloses brain-derived and recombinant acidic fibroblast growth factor, which act as mitogens for mesoderm and neuroectoderm-derived cells in culture, and promote wound healing in soft tissue, cartilaginous tissue and musculo-skeletal tissue. U.S. Patent No. 5,387,673 discloses biologically active fragments of FGF that retain activity.

### Proteins of the TNF Family

A profile derived from the TNF family is created by aligning sequences of the following TNF family members: nerve growth factor (NGF), lymphotoxin, Fas ligand, tumor necrosis factor (TNF), CD40 ligand, TRAIL, ox40 ligand, 4-1BB ligand, CD27 ligand, and CD30 ligand. The profile is designed to identify sequences of proteins that constitute new members or homologues of this family of proteins.

U.S. Patent No. 5,606,023 discloses mutant TNF proteins; U.S. Patent No. 5,597,899 and U.S. Patent No. 5,486,463 disclose TNF muteins; and U.S. Patent No. 5,652,353 discloses DNA encoding TNFα muteins.

Members of the TNF family of proteins have been show in vitro to multimerize, as described in Burrows *et al., Biochem.* (1994) *33*:12741 and Zhang *et al., Mol. Cell. Biol.* (1995) 154851 and bind receptors as described in Browning *et al., J. Immunol.* (1994) *147*:1230, Androlewicz *et al., J. Biol. Chem.*(1992) *267*:2542, and Crowe *et al., Science* (1994) *264*:707.

In vivo, TNFs proteolytically cleave a target protein as described in Kriegel *et al*., *Cell* (1988) *53*:45 and *Mohler et al*., *Nature* (1994) *370*:218 and demonstrate cell proliferation and differentiation activity. T-cell or thymocyte proliferation is assayed as described in Armitage *et al*., *Eur. J. Immunol*. (1992) *22*:447; Current Protocols in Immunology, ed. J.E. Coligan *et al*., 3.1-3.19; Takai *et al*., *J. Immunol.* (1986) *137*:3494-3500, Bertagnoli *et al*., *J. Immunol*. (1990) *145*:1706-1712, Bertagnoli *et al*., *J*. *Immunol*. (1991) *133*:327-340, Bertagnoli *et al*., *J. Immunol*. (1992) *149*:3778-3783, and *Bowman et al*., *J. Immunol*. (1994) *152*:1756-1761. B cell proliferation and Ig secretion are assayed as described in Maliszewski, *J. Immunol*. (1990) *144*:3028-3033, and Assays for B Cell Function: In vitro antibody production, Mond and Brunswick, Current Protocols in Immunol., Coligan Ed vol 1 pp 3.8.1-3.8.16, John Wiley and Sons, Toronto 1994, Kehrl *et al*., *Science* (1987) *238*:1144 and Boussiotis *et al*., *PNAS USA* (1994) *91*:7007.

Other in vivo activities include upregulation of cell surface antigens, upregulation of costimulatory molecules, and cellular aggregation/adhesion as described in Barrett *et al*., *J. Immunol*. (1991) *146*:1722; Bjorck *et al*., *Eur. J Immunol*. (1993) *23*:1771; *Clark et al*., *Annu Rev. Immunol*. (1991) *9*:97; *Ranheim et al*., *J Exp. Med.* (1994) *177*:925; Yellin, *J Immunol*. (1994) *153*:666; and *Gruss et al*., *Blood* (1994) *84*:2305.

Proliferation and differentiation of hematopoietic and lymphopoietic cells has also been shown in vivo for TNFs, using assays for embryonic differentiation and hematopoiesis as described in Johansson *et al., Cellular Biology* (1995) *15*:141-151, Keller *et al., Mol. Cell. Biol.* (1993) *13*:473-486, McClanahan *et al., Blood* (1993) *81*:2903-2915 and using assays to detect stem cell survival and differentiation as described in Culture of Hematopoietic Cells, Freshney *et al*. eds, pp 1-21, 23-29, 139-162, 163-179, and 265-268, Wiley-Liss, Inc., New York, NY, 1994, and Hirajama *et al., PNAS USA* (1992) *89*:5907-5911.

In vivo activities of TNFs also include lymphocyte survival and apoptosis, assayed as described in Darzynkewicz *et al., Cytometry* (1992) *13*:795-808; Gorczca *et al*., *Leukemia* (1993) *7*:659-670; *Itoh et al., Cell* (1991) *66*:233-243; Zacharduk, *J Immunol*. (1990) *145*:4037-4045; Zamai *et al., Cytometry* (1993) *14*:891-897; and Gorczyca *et al., Int'l J Oncol.* (1992) *1*:639-648.

Some members of the TNF family are cleaved from the cell surface; others remain membrane bound. The three-dimensional structure of TNF is discussed in Sprang and Eck, Tumor Necrosis Factors; *supra*.

TNF proteins include a transmembrane domain. The protein is cleaved into a shorter soluble version, as described in Kriegler *et al., Cell* (1988) *53*:45-53, Perez *et al., Cell* (1990) *63*:251-258, and Shaw *et al., Cell* (1986) *46*:659-667. The transmembrane domain is between amino acid 46 and 77 and the cytoplasmic domain is between position 1 and 45 on the human form of TNFα. The 3-dimensional motifs of TNF include a sandwich of two pleated β sheets. Each sheet is composed of antiparallel α strands. α Strands facing each other on opposite sites of the sandwich are connected by short polypeptide loops, as described in Van Ostade *et al., Protein Engineering* (1994) *7(1):*5-22, and Sprang *et al*., Tumor Necrosis Factors; *supra*.

Residues of the TNF family proteins that are involved in the β sheet secondary structure have been identified as described in Van Ostade *et al., Protein Engineering* (1994) *7(1)*:5-22, and Sprang *et al*., Tumor Necrosis Factors; *supra*.

TNF receptors are disclosed in U.S. Patent No. 5,395,760. A profile derived from the TNF receptor family is created by aligning sequences of the TNF receptor family, including Apol/Fas, TNFR I and II, death receptor3 (DR3), CD40, ox40, CD27, and CD30. Thus, the profile is designed to identify, from the nucleic acids of the invention, sequences of proteins that constitute new members or homologs of this family of proteins.

Tumor necrosis factor receptors exist in two forms in humans: p55 TNFR and p75 TNFR, both of which provide intracellular signals upon binding with a ligand. The extracellular domains of these receptor proteins are cysteine rich. The receptors can remain membrane bound, although some forms of the receptors are cleaved forming soluble receptors. The regulation, diagnostic, prognostic, and therapeutic value of soluble TNF receptors is discussed in Aderka, *Cytokine and Growth Factor Reviews,* (1996) 7(3):231-240.

### PDGF Family

U.S. Patent No. 5,326,695 discloses platelet derived growth factor agonists; bioactive portions of PDGF-B are used as agonists. U.S. Patent No. 4,845,075 discloses biologically active B-chain homodimers, and also includes variants and derivatives of the PDGF-B chain. U.S. Patent No. 5,128,321 discloses PDGF analogs and methods of use. Proteins having the same bioactivity as PDGF are disclosed, including A and B chain proteins.

### Kinase (Including MKK) Family

U.S. Patent No. 5,650,501 discloses serine/threonine kinase, associated with mitotic and meiotic cell division; the protein has a kinase domain in its N-terminal and 3 PEST regions in the C-terminus. U.S. Patent No. 5,605,825 discloses human PAK65, a serine protein kinase.

The foregoing discussion provides a few examples of the protein profiles that can be compared with the nucleic acids of the invention. One skilled in the art can use these and other protein profiles to identify the genes that correlate with the nucleic acids.

### IX. Determining the Function of the Encoded Expression Products

Ribozymes, antisense constructs, dominant negative mutants, and triplex formation can be used to determine function of the expression product of an nucleic acid-related gene.

### A. Ribozymes

Trans-cleaving catalytic RNAs (ribozymes) are RNA molecules possessing endoribonuclease activity. Ribozymes are specifically designed for a particular target, and the target message must contain a specific nucleotide sequence. They are engineered to cleave any RNA species site-specifically in the background of cellular RNA. The cleavage event renders the mRNA unstable and prevents protein expression. Importantly, ribozymes can be used to inhibit expression of a gene of unknown function for the purpose of determining its function in an in vitro or in vivo context, by detecting the phenotypic effect.

One commonly used ribozyme motif is the hammerhead, for which the substrate sequence requirements are minimal. Design of the hammerhead ribozyme is disclosed in Usman *et al*., *Current Opin. Struct. Biol*. (1996) *6*:527-533. Usman also discusses the therapeutic uses of ribozymes. Ribozymes can also be prepared and used as described in Long *et al., FASEB J* (1993) *7*:25; Symons, *Ann. Rev. Biochem.* (1992) *61*:641; Perrotta *et al., Biochem.* (1992) *31*:16-17; Ojwang *et al., Proc. Natl. Acad. Sci. (USA)* (1992) *89*:10802-10806; and U.S. Patent No. 5,254,678. Ribozyme cleavage of HIV-I RNA is described in U.S. Patent No. 5,144,019; methods of cleaving RNA using ribozymes is described in U.S. Patent No. 5,116,742; and methods for increasing the specificity of ribozymes are described in U.S. Patent No. 5,225,337 and Koizumi *et al*., *Nucleic Acid Res.* (1989) *17*:7059-7071. Preparation and use of ribozyme fragments in a hammerhead structure are also described by Koizumi *et al*., *Nucleic Acids Res.* (1989) *17*:7059-7071. Preparation and use of ribozyme fragments in a hairpin structure are described by Chowrira and Burke, *Nucleic Acids Res.* (1992) *20*:2835. Ribozymes can also be made by rolling transcription as described in Daubendiek and Kool, *Nat. Biotechnol.* (1997) *15(3):*273-277.

The hybridizing region of the ribozyme may be modified or may be prepared as a branched structure as described in Horn and Urdea, *Nucleic Acids Res.* (1989) *17*:6959-67. The basic structure of the ribozymes may also be chemically altered in ways familiar to those skilled in the art, and chemically synthesized ribozymes can be administered as synthetic oligonucleotide derivatives modified by monomeric units. In a therapeutic context, liposome mediated delivery of ribozymes improves cellular uptake, as described in Birikh *et al., Eur. J. Biochem.* (1997) *245*:1-16.

Using the nucleic acid sequences of the invention and methods known in the art, ribozymes are designed to specifically bind and cut the corresponding mRNA species. Ribozymes thus provide a means to inhibit the expression of any of the proteins encoded by the disclosed nucleic acids or their full-length genes. The full-length gene need not be known in order to design and use specific inhibitory ribozymes. In the case of an nucleic acid or cDNA of unknown function, ribozymes corresponding to that nucleotide sequence can be tested in vitro for efficacy in cleaving the target transcript. Those ribozymes that effect cleavage in vitro are further tested in vivo. The ribozyme can also be used to generate an animal model for a disease, as described in Birikh *et al., Eur. J Biochem.* (1997) *245*:1-16. An effective ribozyme is used to determine the function of the gene of interest by blocking its transcription and detecting a change in the cell. Where the gene is found to be a mediator in a disease, an effective ribozyme is designed and delivered in a gene therapy for blocking transcription and expression of the gene.

Therapeutic and functional genomic applications of ribozymes proceed beginning with knowledge of a portion of the coding sequence of the gene to be inhibited. Thus, for many genes, a partial nucleic acid sequence provides adequate sequence for constructing an effective ribozyme. A target cleavage site is selected in the target sequence, and a ribozyme is constructed based on the 5' and 3' nucleotide sequences that flank the cleavage site. Retroviral vectors are engineered to express monomeric and multimeric hammerhead ribozymes targeting the mRNA of the target coding sequence. These monomeric and multimeric ribozymes are tested in vitro for an ability to cleave the target mRNA. A cell line is stably transduced with the retroviral vectors expressing the ribozymes, and the transduction is confirmed by Northern blot analysis and reverse-transcription polymerase chain reaction (RT-PCR). The cells are screened for inactivation of the target mRNA by such indicators as reduction of expression of disease markers or reduction of the gene product of the target mRNA.

### B. Antisense

Antisense nucleic acids are designed to specifically bind to RNA, resulting in the formation of RNA-DNA or RNA-RNA hybrids, with an arrest of DNA replication, reverse transcription or messenger RNA translation. Antisense polynucleotides based on a selected nucleic acid sequence can interfere with expression of the corresponding gene. Antisense polynucleotides are typically generated within the cell by expression from antisense constructs that contain the antisense nucleic acid strand as the transcribed strand. Antisense nucleic acids will bind and/or interfere with the translation of nucleic acid-related mRNA. The expression products of control cells and cells treated with the antisense construct are compared to detect the protein product of the gene corresponding to the nucleic acid. The protein is isolated and identified using routine biochemical methods.

One rationale for using antisense methods to determine the function of the gene corresponding to an nucleic acid is the biological activity of antisense therapeutics. Antisense therapy for a variety of cancers is in clinical phase and has been discussed extensively in the literature. Reed reviewed antisense therapy directed at the Bcl-2 gene in tumors; gene transfer-mediated overexpression of Bcl-2 in tumor cell lines conferred resistance to many types of cancer drugs. (Reed, J.C., *N.C.I.* (1997) *89*:988-990). The potential for clinical development of antisense inhibitors of *ras* is discussed by Cowsert, L.M., *Anti-Cancer Drug Design* (1997) *12*:359-371. Additional important antisense targets include leukemia (Geurtz, A.M., *Anti-Cancer Drug Design* (1997) *12*:341-358); human C-ref kinase (Monia, *B.P., Anti-Cancer Drug Design* (1997) *12*:327-339); and protein kinase C (McGraw *et al., Anti-Cancer Drug Design* (1997) *12*:315-326.

Given the extensive background literature and clinical experience in antisense therapy, one skilled in the art can use selected nucleic acids of the invention as additional potential therapeutics. The choice of nucleic acid can be narrowed by first testing them for binding to "hot spot" regions of the genome of cancerous cells. If an nucleic acid is identified as binding to a "hot spot", testing the nucleic acid as an antisense compound in the corresponding cancer cells clearly is warranted.

Ogunbiyi *et al., Gastroenterology* (1997) *113(3)*:761-766 describe prognostic use of allelic loss in colon cancer; Barks *et al., Genes, Chromosomes, and Cancer* (1997) *19(4)*:278-285 describe increased chromosome copy number detected by FISH in malignant melanoma; Nishizake *et al., Genes, Chromosomes, and Cancer* (1997) *19(4)*:267-272 describe genetic alterations in primary breast cancer and their metastases and direct comparison using modified comparative genome hybridization; and Elo *et al., Cancer Research* (1997) *57(16)*:3356-3359 disclose that loss of heterozygosity at 16z24.1-q24.2 is significantly associated with metastatic and aggressive behavior of prostate cancer.

### C. Dominant Negative Mutations

As an alternative method for identifying function of the nucleic acid-related gene, dominant negative mutations are readily generated for corresponding proteins that are active as homomultimers. A mutant polypeptide will interact with wild-type polypeptides (made from the other allele) and form a non-functional multimer. Thus, a mutation is in a substrate-binding domain, a catalytic domain, or a cellular localization domain. Preferably, the mutant polypeptide will be overproduced. Point mutations are made that have such an effect. In addition, fusion of different polypeptides of various lengths to the terminus of a protein can yield dominant negative mutants. General strategies are available for making dominant negative mutants. See Herskowitz, *Nature* (1987) 329:219-222. Such a technique can be used for creating a loss-of-function mutation, which is useful for determining the function of a protein.

### D. Triplex Formation

Endogenous gene expression can also be reduced by inactivating or "knocking out" the gene or its promoter using targeted homologous recombination. (E.g., see Smithies *et al*., 1985, Nature 317:230-234; Thomas & Capecchi, 1987, Cell 51:503-512; Thompson et al., 1989 Cell 5:313-321; each of which is incorporated by reference herein in its entirety). For example, a mutant, non-functional gene (or a completely unrelated DNA sequence) flanked by DNA homologous to the endogenous gene (either the coding regions or regulatory regions of the gene) can be used, with or without a selectable marker and/or a negative selectable marker, to transfect cells that express that gene *in vivo.* Insertion of the DNA construct, via targeted homologous recombination, results in inactivation of the gene.

Alternatively, endogenous gene expression can be reduced by targeting deoxyribonucleotide sequences complementary to the regulatory region of the target gene (i.e., the gene promoter and/or enhancers) to form triple helical structures that prevent transcription of the gene in target cells in the body. (See generally, Helene, C. 1991, Anticancer Drug Des., 6(6):569-84; Helene, C., et al., 1992, Ann, N.Y. Accad. Sci., 660:27-36; and Maher, L.J., 1992, Bioassays 14(12):807-15).

Nucleic acid molecules to be used in triple helix formation for the inhibition of transcription are preferably single stranded and composed of deoxyribonucleotides. The base composition of these oligonucleotides should promote triple helix formation via Hoogsteen base-pairing rules, which generally require sizable stretches of either purines or pyrimidines to be present on one strand of a duplex. Nucleotide sequences may be pyrimidine-based, which will result in TAT and CGC triplets across the three associated strands of the resulting triple helix. The pyrimidine-rich molecules provide base complementarity to a purine-rich region of a single strand of the duplex in a parallel orientation to that strand. In addition, nucleic acid molecules may be chosen that are purine-rich, for example, containing a stretch of G residues. These molecules will form a triple helix with a DNA duplex that is rich in GC pairs, in which the majority of the purine residues are located on a single strand of the targeted duplex, resulting in CGC triplets across the three strands in the triplex.

Alternatively, the potential sequences that can be targeted for triple helix formation may be increased by creating a so called "switchback" nucleic acid molecule. Switchback molecules are synthesized in an alternating 5'-3', 3'-5' manner, such that they base pair with first one strand of a duplex and then the other, eliminating the necessity for a sizable stretch of either purines or pyrimidines to be present on one strand of a duplex.

Antisense RNA and DNA, ribozyme, and triple helix molecules of the invention may be prepared by any method known in the art for the synthesis of DNA and RNA molecules. These include techniques for chemically synthesizing oligodeoxyribonucleotides and oligoribonucleotides well known in the art such as for example solid phase phosphoramidite chemical synthesis. Alternatively, RNA molecules may be generated by *in vitro* and *in vivo* transcription of DNA sequences encoding the antisense RNA molecule. Such DNA sequences may be incorporated into a wide variety of vectors which incorporate suitable RNA polymerase promoters such as the T7 or SP6 polymerase promoters. Alternatively, antisense cDNA constructs that synthesize antisense RNA constitutively or inducibly, depending on the promoter used, can be introduced stably into cell lines.

Moreover, various well known modifications to nucleic acid molecules may be introduced as a means of increasing intracellular stability and half-life. Possible modifications include but are not limited to the addition of flanking sequences of ribonucleotides or deoxyribonucleotides to the 5' and/or 3' ends of the molecule or the use of phosphorothioate or 2' O-methyl rather than phosphodiesterase linkages within the oligodeoxyribonucleotide backbone.

### X. Diagnostic & Prognostic Assays and Drug Screening Methods

The present invention provides method for determining whether a subject is at risk for developing a disease or condition characterized by unwanted cell proliferation by detecting the disclosed biomarkers, i.e., the disclosed nucleic acid markers (SEQ ID Nos: 1-850) and/or polypeptide markers for colon cancer encoded thereby.

In clinical applications, human tissue samples can be screened for the presence and/or absence of the biomarkers identified herein. Such samples could consist of needle biopsy cores, surgical resection samples, lymph node tissue, or serum. For example, these methods include obtaining a biopsy, which is optionally fractionated by cryostat sectioning to enrich tumor cells to about 80% of the total cell population. In certain embodiments, nucleic acids extracted from these samples may be amplified using techniques well known in the art. The levels of selected markers detected would be compared with statistically valid groups of metastatic, non-metastatic malignant, benign, or normal colon tissue samples.

In one embodiment, the diagnostic method comprises determining whether a subject has an abnormal mRNA and/or protein level of the disclosed markers, such as by Northern blot analysis, reverse transcription-polymerase chain reaction (RT-PCR), *in situ* hybridization, immunoprecipitation, Western blot hybridization, or immunohistochemistry. According to the method, cells are obtained from a subject and the levels of the disclosed biomarkers, protein or mRNA level, is determined and compared to the level of these markers in a healthy subject. An abnormal level of the biomarker polypeptide or mRNA levels is likely to be indicative of cancer such as colon cancer.

Accordingly, in one aspect, the invention provides probes and primers that are specific to the unique nucleic acid markers disclosed herein. Accordingly, the nucleic acid probes comprise a nucleotide sequence at least 12 nucleotides in length, preferably at least 15 nucleotides, more preferably, 25 nucleotides, and most preferably at least 40 nucleotides, and up to all or nearly all of the coding sequence which is complementary to a portion of the coding sequence of a marker nucleic acid sequence, which nucleic acid sequence is represented by SEQ ID Nos: 1-850 or a sequence complementary thereto.

In one embodiment, the method comprises using a nucleic acid probe to determine the presence of cancerous cells in a tissue from a patient. Specifically, the method comprises:
1. providing a nucleic acid probe comprising a nucleotide sequence at least 12 nucleotides in length, preferably at least 15 nucleotides, more preferably, 25 nucleotides, and most preferably at least 40 nucleotides, and up to all or nearly all of the coding sequence which is complementary to a portion of the coding sequence of a nucleic acid sequence represented by SEQ ID Nos: 1-850 or a sequence complementary thereto and is differentially expressed in tumors cells, such as colon cancer cells;
2. obtaining a tissue sample from a patient potentially comprising cancerous cells;
3. providing a second tissue sample containing cells substantially all of which are non-cancerous;
4. contacting the nucleic acid probe under stringent conditions with RNA of each of said first and second tissue samples (e.g., in a Northern blot or in situ hybridization assay); and
5. comparing (a) the amount of hybridization of the probe with RNA of the first tissue sample, with (b) the amount of hybridization of the probe with RNA of the second tissue sample;
wherein a statistically significant difference in the amount of hybridization with the RNA of the first tissue sample as compared to the amount of hybridization with the RNA of the second tissue sample is indicative of the presence of cancerous cells in the first tissue sample.

In one aspect, the method comprises in situ hybridization with a probe derived from a given marker nucleic acid sequence, which nucleic acid sequence is represented by SEQ ID Nos: 1-850 or a sequence complementary thereto. The method comprises contacting the labeled hybridization probe with a sample of a given type of tissue potentially containing cancerous or precancerous cells as well as normal cells, and determining whether the probe labels some cells of the given tissue type to a degree significantly different (e.g., by at least a factor of two, or at least a factor of five, or at least a factor of twenty, or at least a factor of fifty) than the degree to which it labels other cells of the same tissue type.

Also within the invention is a method of determining the phenotype of a test cell from a given human tissue, e.g., whether the cell is (a) normal, or (b) cancerous or precancerous, by contacting the mRNA of a test cell with a nucleic acid probe at least 12 nucleotides in length, preferably at least 15 nucleotides, more preferably at least 25 nucleotides, and most preferably at least 40 nucleotides, and up to all or nearly all of a sequence which is complementary to a portion of the coding sequence of a nucleic acid sequence represented by SEQ ID Nos: 1-850 or a sequence complementary thereto, and which is differentially expressed in tumor cells as compared to normal cells of the given tissue type; and determining the approximate amount of hybridization of the probe to the mRNA, an amount of hybridization either more or less than that seen with the mRNA of a normal cell of that tissue type being indicative that the test cell is cancerous or precancerous.

Alternatively, the above diagnostic assays may be carried out using antibodies to detect the protein product encoded by the marker nucleic acid sequence, which nucleic acid sequence is represented by SEQ ID Nos: 1-850 or a sequence complementary thereto. Accordingly, in one embodiment, the assay would include contacting the proteins of the test cell with an antibody specific for the gene product of a nucleic acid represented by SEQ ID Nos: 1-850 or a sequence complementary thereto, the marker nucleic acid being one which is expressed at a given control level in normal cells of the same tissue type as the test cell, and determining the approximate amount of immunocomplex formation by the antibody and the proteins of the test cell, wherein a statistically significant difference in the amount of the immunocomplex formed with the proteins of a test cell as compared to a normal cell of the same tissue type is an indication that the test cell is cancerous or precancerous.

Another such method includes the steps of: providing an antibody specific for the gene product of a marker nucleic acid sequence represented by SEQ ID Nos 1-850, the gene product being present in cancerous tissue of a given tissue type (e.g., colon tissue) at a level more or less than the level of the gene product in noncancerous tissue of the same tissue type; obtaining from a patient a first sample of tissue of the given tissue type, which sample potentially includes cancerous cells; providing a second sample of tissue of the same tissue type (which may be from the same patient or from a normal control, e.g. another individual or cultured cells), this second sample containing normal cells and essentially no cancerous cells; contacting the antibody with protein (which may be partially purified, in lysed but unfractionated cells, or in situ) of the first and second samples under conditions permitting immunocomplex formation between the antibody and the marker nucleic acid sequence product present in the samples; and comparing (a) the amount of immunocomplex formation in the first sample, with (b) the amount of immunocomplex formation in the second sample, wherein a statistically significant difference in the amount of immunocomplex formation in the first sample less as compared to the amount of immunocomplex formation in the second sample is indicative of the presence of cancerous cells in the first sample of tissue.

The subject invention further provides a method of determining whether a cell sample obtained from a subject possesses an abnormal amount of marker polypeptide which comprises (a) obtaining a cell sample from the subject, (b) quantitatively determining the amount of the marker polypeptide in the sample so obtained, and (c) comparing the amount of the marker polypeptide so determined with a known standard, so as to thereby determine whether the cell sample obtained from the subject possesses an abnormal amount of the marker polypeptide. Such marker polypeptides may be detected by immunohistochemical assays, dot-blot assays, ELISA and the like.

Immunoassays are commonly used to quantitate the levels of proteins in cell samples, and many other immunoassay techniques are known in the art. The invention is not limited to a particular assay procedure, and therefore is intended to include both homogeneous and heterogeneous procedures. Exemplary immunoassays which can be conducted according to the invention include fluorescence polarization immunoassay (FPIA), fluorescence immunoassay (FIA), enzyme immunoassay (EIA), nephelometric inhibition immunoassay (NIA), enzyme linked immunosorbent assay (ELISA), and radioimmunoassay (RIA). An indicator moiety, or label group, can be attached to the subject antibodies and is selected so as to meet the needs of various uses of the method which are often dictated by the availability of assay equipment and compatible immunoassay procedures. General techniques to be used in performing the various immunoassays noted above are known to those of ordinary skill in the art.

In another embodiment, the level of the encoded product, i.e., the product encoded by SEQ ID Nos 1-850 or a sequence complementary thereto, in a biological fluid (e.g., blood or urine) of a patient may be determined as a way of monitoring the level of expression of the marker nucleic acid sequence in cells of that patient. Such a method would include the steps of obtaining a sample of a biological fluid from the patient, contacting the sample (or proteins from the sample) with an antibody specific for a encoded marker polypeptide, and determining the amount of immune complex formation by the antibody, with the amount of immune complex formation being indicative of the level of the marker encoded product in the sample. This determination is particularly instructive when compared to the amount of immune complex formation by the same antibody in a control sample taken from a normal individual or in one or more samples previously or subsequently obtained from the same person.

In another embodiment, the method can be used to determine the amount of marker polypeptide present in a cell, which in turn can be correlated with progression of a hyperproliferative disorder, e.g., colon cancer. The level of the marker polypeptide can be used predictively to evaluate whether a sample of cells contains cells which are, or are predisposed towards becoming, transformed cells. Moreover, the subject method can be used to assess the phenotype of cells which are known to be transformed, the phenotyping results being useful in planning a particular therapeutic regimen. For instance, very high levels of the marker polypeptide in sample cells is a powerful diagnostic and prognostic marker for a cancer, such as colon cancer. The observation of marker polypeptide level can be utilized in decisions regarding, e.g., the use of more aggressive therapies.

As set out above, one aspect of the present invention relates to diagnostic assays for determining, in the context of cells isolated from a patient, if the level of a marker polypeptide is significantly reduced in the sample cells. The term "significantly reduced " refers to a cell phenotype wherein the cell possesses a reduced cellular amount of the marker polypeptide relative to a normal cell of similar tissue origin. For example, a cell may have less than about 50%, 25%, 10%, or 5% of the marker polypeptide that a normal control cell. In particular, the assay evaluates the level of marker polypeptide in the test cells, and, preferably, compares the measured level with marker polypeptide detected in at least one control cell, e.g., a normal cell and/or a transformed cell of known phenotype.

Of particular importance to the subject invention is the ability to quantitate the level of marker polypeptide as determined by the number of cells associated with a normal or abnormal marker polypeptide level. The number of cells with a particular marker polypeptide phenotype may then be correlated with patient prognosis. In one embodiment of the invention, the marker polypeptide phenotype of the lesion is determined as a percentage of cells in a biopsy which are found to have abnormally high/low levels of the marker polypeptide. Such expression may be detected by immunohistochemical assays, dot-blot assays, ELISA and the like.

Where tissue samples are employed, immunohistochemical staining may be used to determine the number of cells having the marker polypeptide phenotype. For such staining, a multiblock of tissue is taken from the biopsy or other tissue sample and subjected to proteolytic hydrolysis, employing such agents as protease K or pepsin. In certain embodiments, it may be desirable to isolate a nuclear fraction from the sample cells and detect the level of the marker polypeptide in the nuclear fraction.

The tissue samples are fixed by treatment with a reagent such as formalin, glutaraldehyde, methanol, or the like. The samples are then incubated with an antibody, preferably a monoclonal antibody, with binding specificity for the marker polypeptides. This antibody may be conjugated to a label for subsequent detection of binding. Samples are incubated for a time sufficient for formation of the immuno-complexes. Binding of the antibody is then detected by virtue of a label conjugated to this antibody. Where the antibody is unlabeled, a second labeled antibody may be employed, e.g., which is specific for the isotype of the anti-marker polypeptide antibody. Examples of labels which may be employed include radionuclides, fluorescers, chemiluminescers, enzymes and the like.

Where enzymes are employed, the substrate for the enzyme may be added to the samples to provide a colored or fluorescent product. Examples of suitable enzymes for use in conjugates include horseradish peroxidase, alkaline phosphatase, malate dehydrogenase and the like. Where not commercially available, such antibody-enzyme conjugates are readily produced by techniques known to those skilled in the art.

In one embodiment, the assay is performed as a dot blot assay. The dot blot assay finds particular application where tissue samples are employed as it allows determination of the average amount of the marker polypeptide associated with a single cell by correlating the amount of marker polypeptide in a cell-free extract produced from a predetermined number of cells.

It is well established in the cancer literature that tumor cells of the same type (e.g., breast and/or colon tumor cells) may not show uniformly increased expression of individual oncogenes or uniformly decreased expression of individual tumor suppressor genes. There may also be varying levels of expression of a given marker gene even between cells of a given type of cancer, further emphasizing the need for reliance on a battery of tests rather than a single test. Accordingly, in one aspect, the invention provides for a battery of tests utilizing a number of probes of the invention, in order to improve the reliability and/or accuracy of the diagnostic test.

In one embodiment, the present invention also provides a method wherein nucleic acid probes are immobilized on a DNA chip in an organized array. Oligonucleotides can be bound to a solid support by a variety of processes, including lithography. For example a chip can hold up to 250,000 oligonucleotides (GeneChip, Affymetrix). These nucleic acid probes comprise a nucleotide sequence at least about 12 nucleotides in length, preferably at least about 15 nucleotides, more preferably at least about 25 nucleotides, and most preferably at least about 40 nucleotides, and up to all or nearly all of a sequence which is complementary to a portion of the coding sequence of a marker nucleic acid sequence represented by SEQ ID Nos: 1-850 and is differentially expressed in tumor cells, such as colon cancer cells. The present invention provides significant advantages over the available tests for various cancers, such as colon cancer, because it increases the reliability of the test by providing an array of nucleic acid markers on a single chip.

The method includes obtaining a biopsy, which is optionally fractionated by cryostat sectioning to enrich tumor cells to about 80% of the total cell population. The DNA or RNA is then extracted, amplified, and analyzed with a DNA chip to determine the presence of absence of the marker nucleic acid sequences.

In one embodiment, the nucleic acid probes are spotted onto a substrate in a two-dimensional matrix or array. Samples of nucleic acids can be labeled and then hybridized to the probes. Double-stranded nucleic acids, comprising the labeled sample nucleic acids bound to probe nucleic acids, can be detected once the unbound portion of the sample is washed away.

The probe nucleic acids can be spotted on substrates including glass, nitrocellulose, etc. The probes can be bound to the substrate by either covalent bonds or by non-specific interactions, such as hydrophobic interactions. The sample nucleic acids can be labeled using radioactive labels, fluorophores, chromophores, etc.

Techniques for constructing arrays and methods of using these arrays are described in EP No. 0 799 897; PCT No. WO 97/29212; PCT No. WO 97/27317; EP No. 0 785 280; PCT No. WO 97/02357; U.S. Pat. No. 5,593,839; U.S. Pat. No. 5,578,832; EP No. 0 728 520; U.S. Pat. No. 5,599,695; EP No. 0 721 016; U.S. Pat. No. 5,556,752; PCT No. WO 95/22058; and U.S. Pat. No. 5,631,734.

Further, arrays can be used to examine differential expression of genes and can be used to determine gene function. For example, arrays of the instant nucleic acid sequences can be used to determine if any of the nucleic acid sequences are differentially expressed between normal cells and cancer cells, for example. High expression of a particular message in a cancer cell, which is not observed in a corresponding normal cell, can indicate a cancer specific protein.

In yet another embodiment, the invention contemplates using a panel of antibodies which are generated against the marker polypeptides of this invention, which polypeptides are encoded by SEQ ID Nos 1-850. Such a panel of antibodies may be used as a reliable diagnostic probe for colon cancer. The assay of the present invention comprises contacting a biopsy sample containing cells, e.g., colon cells, with a panel of antibodies to one or more of the encoded products to determine the presence or absence of the marker polypeptides.

The diagnostic methods of the subject invention may also be employed as follow-up to treatment, e.g., quantitation of the level of marker polypeptides may be indicative of the effectiveness of current or previously employed cancer therapies as well as the effect of these therapies upon patient prognosis.

Accordingly, the present invention makes available diagnostic assays and reagents for detecting gain and/or loss of marker polypeptides from a cell in order to aid in the diagnosis and phenotyping of proliferative disorders arising from, for example, tumorigenic transformation of cells.

The diagnostic assays described above can be adapted to be used as prognostic assays, as well. Such an application takes advantage of the sensitivity of the assays of the invention to events which take place at characteristic stages in the progression of a tumor. For example, a given marker gene may be up- or downregulated at a very early stage, perhaps before the cell is irreversibly committed to developing into a malignancy, while another marker gene may be characteristically up or down regulated only at a much later stage. Such a method could involve the steps of contacting the mRNA of a test cell with a nucleic acid probe derived from a given marker nucleic acid which is expressed at different characteristic levels in cancerous or precancerous cells at different stages of tumor progression, and determining the approximate amount of hybridization of the probe to the mRNA of the cell, such amount being an indication of the level of expression of the gene in the cell, and thus an indication of the stage of tumor progression of the cell; alternatively, the assay can be carried out with an antibody specific for the gene product of the given marker nucleic acid, contacted with the proteins of the test cell. A battery of such tests will disclose not only the existence and location of a tumor, but also will allow the clinician to select the mode of treatment most appropriate for the tumor, and to predict the likelihood of success of that treatment.

The methods of the invention can also be used to follow the clinical course of a tumor. For example, the assay of the invention can be applied to a tissue sample from a patient; following treatment of the patient for the cancer, another tissue sample is taken and the test repeated. Successful treatment will result in either removal of all cells which demonstrate differential expression characteristic of the cancerous or precancerous cells, or a substantial increase in expression of the gene in those cells, perhaps approaching or even surpassing normal levels.

In yet another embodiment, the invention provides methods for determining whether a subject is at risk for developing a disease, such as a predisposition to develop cancer, for example colon cancer, associated with an aberrant activity of any one of the polypeptides encoded by nucleic acids of SEQ ID Nos: 1-850, wherein the aberrant activity of the polypeptide is characterized by detecting the presence or absence of a genetic lesion characterized by at least one of (i) an alteration affecting the integrity of a gene encoding a marker polypeptides, or (ii) the mis-expression of the encoding nucleic acid. To illustrate, such genetic lesions can be detected by ascertaining the existence of at least one of (i) a deletion of one or more nucleotides from the nucleic acid sequence, (ii) an addition of one or more nucleotides to the nucleic acid sequence, (iii) a substitution of one or more nucleotides of the nucleic acid sequence, (iv) a gross chromosomal rearrangement of the nucleic acid sequence, (v) a gross alteration in the level of a messenger RNA transcript of the nucleic acid sequence, (vii) aberrant modification of the nucleic acid sequence, such as of the methylation pattern of the genomic DNA, (vii) the presence of a non-wild type splicing pattern of a messenger RNA transcript of the gene, (viii) a non-wild type level of the marker polypeptide, (ix) allelic loss of the gene, and/or (x) inappropriate post-translational modification of the marker polypeptide.

The present invention provides assay techniques for detecting lesions in the encoding nucleic acid sequence. These methods include, but are not limited to, methods involving sequence analysis, Southern blot hybridization, restriction enzyme site mapping, and methods involving detection of absence of nucleotide pairing between the nucleic acid to be analyzed and a probe.

Specific diseases or disorders, e.g., genetic diseases or disorders, are associated with specific allelic variants of polymorphic regions of certain genes, which do not necessarily encode a mutated protein. Thus, the presence of a specific allelic variant of a polymorphic region of a gene in a subject can render the subject susceptible to developing a specific disease or disorder. Polymorphic regions in genes, can be identified, by determining the nucleotide sequence of genes in populations of individuals. If a polymorphic region is identified, then the link with a specific disease can be determined by studying specific populations of individuals, e.g, individuals which developed a specific disease, such as colon cancer. A polymorphic region can be located in any region of a gene, e.g., exons, in coding or non coding regions of exons, introns, and promoter region.

In an exemplary embodiment, there is provided a nucleic acid composition comprising a nucleic acid probe including a region of nucleotide sequence which is capable of hybridizing to a sense or antisense sequence of a gene or naturally occurring mutants thereof, or 5' or 3' flanking sequences or intronic sequences naturally associated with the subject genes or naturally occurring mutants thereof. The nucleic acid of a cell is rendered accessible for hybridization, the probe is contacted with the nucleic acid of the sample, and the hybridization of the probe to the sample nucleic acid is detected. Such techniques can be used to detect lesions or allelic variants at either the genomic or mRNA level, including deletions, substitutions, etc., as well as to determine mRNA transcript levels.

A preferred detection method is allele specific hybridization using probes overlapping the mutation or polymorphic site and having about 5, 10, 20, 25, or 30 nucleotides around the mutation or polymorphic region. In a preferred embodiment of the invention, several probes capable of hybridizing specifically to allelic variants are attached to a solid phase support, e.g., a "chip". Mutation detection analysis using these chips comprising oligonucleotides, also termed "DNA probe arrays" is described e.g., in Cronin et al. (1996) Human Mutation 7:244. In one embodiment, a chip comprises all the allelic variants of at least one polymorphic region of a gene. The solid phase support is then contacted with a test nucleic acid and hybridization to the specific probes is detected. Accordingly, the identity of numerous allelic variants of one or more genes can be identified in a simple hybridization experiment.

In certain embodiments, detection of the lesion comprises utilizing the probe/primer in a polymerase chain reaction (PCR) (see, e.g. U.S. Patent Nos. 4,683,195 and 4,683,202), such as anchor PCR or RACE PCR, or, alternatively, in a ligase chain reaction (LCR) (see, e.g., Landegran *et al*. (1988) *Science* 241:1077-1080; and Nakazawa *et al*. (1994) *PNAS* 91:360-364), the latter of which can be particularly useful for detecting point mutations in the gene (see Abravaya et al. (1995) *Nuc Acid Res* 23:675-682). In a merely illustrative embodiment, the method includes the steps of (i) collecting a sample of cells from a patient, (ii) isolating nucleic acid (e.g., genomic, mRNA or both) from the cells of the sample, (iii) contacting the nucleic acid sample with one or more primers which specifically hybridize to a nucleic acid sequence under conditions such that hybridization and amplification of the nucleic acid (if present) occurs, and (iv) detecting the presence or absence of an amplification product, or detecting the size of the amplification product and comparing the length to a control sample. It is anticipated that PCR and/or LCR may be desirable to use as a preliminary amplification step in conjunction with any of the techniques used for detecting mutations described herein.

Alternative amplification methods include: self sustained sequence replication (Guatelli, J.C. *et al*., 1990, Proc. Natl. Acad. Sci. USA 87:1874-1878), transcriptional amplification system (Kwoh, D.Y. et al., 1989, Proc. Natl. Acad. Sci. USA 86:1173-1177), Q-Beta Replicase (Lizardi, P.M. *et al*., 1988, Bio/Technology 6:1197), or any other nucleic acid amplification method, followed by the detection of the amplified molecules using techniques well known to those of skill in the art. These detection schemes are especially useful for the detection of nucleic acid molecules if such molecules are present in very low numbers.

In a preferred embodiment of the subject assay, mutations in, or allelic variants, of a gene from a sample cell are identified by alterations in restriction enzyme cleavage patterns. For example, sample and control DNA is isolated, amplified (optionally), digested with one or more restriction endonucleases, and fragment length sizes are determined by gel electrophoresis. Moreover, the use of sequence specific ribozymes (see, for example, U.S. Patent No. 5,498,531) can be used to score for the presence of specific mutations by development or loss of a ribozyme cleavage site.

Another aspect of the invention is directed to the identification of agents capable of modulating the differentiation and proliferation of cells characterized by aberrant proliferation. In this regard, the invention provides assays for determining compounds that modulate the expression of the marker nucleic acids (SEQ ID Nos: 1-850) and/or alter for example, inhibit the bioactivity of the encoded polypeptide.

Several in vivo methods can be used to identify compounds that modulate expression of the marker nucleic acids (SEQ ID Nos: 1-850) and/or alter for example, inhibit the bioactivity of the encoded polypeptide.

Drug screening is performed by adding a test compound to a sample of cells, and monitoring the effect. A parallel sample which does not receive the test compound is also monitored as a control. The treated and untreated cells are then compared by any suitable phenotypic criteria, including but not limited to microscopic analysis, viability testing, ability to replicate, histological examination, the level of a particular RNA or polypeptide associated with the cells, the level of enzymatic activity expressed by the cells or cell lysates, and the ability of the cells to interact with other cells or compounds. Differences between treated and untreated cells indicates effects attributable to the test compound.

Desirable effects of a test compound include an effect on any phenotype that was conferred by the cancer-associated marker nucleic acid sequence. Examples include a test compound that limits the overabundance of mRNA, limits production of the encoded protein, or limits the functional effect of the protein. The effect of the test compound would be apparent when comparing results between treated and untreated cells.

The invention thus also encompasses methods of screening for agents which inhibit expression of the nucleic acid markers (SEQ ID Nos: 1-850) in vitro, comprising exposing a cell or tissue in which the marker nucleic acid mRNA is detectable in cultured cells to an agent in order to determine whether the agent is capable of inhibiting production of the mRNA; and determining the level of mRNA in the exposed cells or tissue, wherein a decrease in the level of the mRNA after exposure of the cell line to the agent is indicative of inhibition of the marker nucleic acid mRNA production.

Alternatively, the screening method may include in vitro screening of a cell or tissue in which marker protein is detectable in cultured cells to an agent suspected of inhibiting production of the marker protein; and determining the level of the marker protein in the cells or tissue, wherein a decrease in the level of marker protein after exposure of the cells or tissue to the agent is indicative of inhibition of marker protein production.

The invention also encompasses in vivo methods of screening for agents which inhibit expression of the marker nucleic acids, comprising exposing a mammal having tumor cells in which marker mRNA or protein is detectable to an agent suspected of inhibiting production of marker mRNA or protein; and determining the level of marker mRNA or protein in tumor cells of the exposed mammal. A decrease in the level of marker mRNA or protein after exposure of the mammal to the agent is indicative of inhibition of marker nucleic acid expression.

Accordingly, the invention provides a method comprising incubating a cell expressing the marker nucleic acids (SEQ ID Nos: 1-850) with a test compound and measuring the mRNA or protein level. The invention further provides a method for quantitatively determining the level of expression of the marker nucleic acids in a cell population, and a method for determining whether an agent is capable of increasing or decreasing the level of expression of the marker nucleic acids in a cell population. The method for determining whether an agent is capable of increasing or decreasing the level of expression of the marker nucleic acids in a cell population comprises the steps of (a) preparing cell extracts from control and agent-treated cell populations, (b) isolating the marker polypeptides from the cell extracts, (c) quantifying (e.g., in parallel) the amount of an immunocomplex formed between the marker polypeptide and an antibody specific to said polypeptide. The marker polypeptides of this invention may also be quantified by assaying for its bioactivity. Agents that induce increased the marker nucleic acid expression may be identified by their ability to increase the amount of immunocomplex formed in the treated cell as compared with the amount of the immunocomplex formed in the control cell. In a similar manner, agents that decrease expression of the marker nucleic acid may be identified by their ability to decrease the amount of the immunocomplex formed in the treated cell extract as compared to the control cell.

mRNA levels can be determined by Northern blot hybridization. mRNA levels can also be determined by methods involving PCR. Other sensitive methods for measuring mRNA, which can be used in high throughput assays, e.g., a method using a DELFIA endpoint detection and quantification method, are described, e.g., in Webb and Hurskainen (1996) *Journal of Biomolecular Screening* 1:119. Marker protein levels can be determined by immunoprecipitations or immunohistochemistry using an antibody that specifically recognizes the protein product encoded by SEQ ID Nos: 1-850.

Agents that are identified as active in the drug screening assay are candidates to be tested for their capacity to block cell proliferation activity. These agents would be useful for treating a disorder involving aberrant growth of cells, especially colon cells.

A variety of assay formats will suffice and, in light of the present disclosure, those not expressly described herein will nevertheless be comprehended by one of ordinary skill in the art. For instance, the assay can be generated in many different formats, and include assays based on cell-free systems, e.g., purified proteins or cell lysates, as well as cell-based assays which utilize intact cells.

In many drug screening programs which test libraries of compounds and natural extracts, high throughput assays are desirable in order to maximize the number of compounds surveyed in a given period of time. Assays of the present invention which are performed in cell-free systems, such as may be derived with purified or semi-purified proteins or with lysates, are often preferred as "primary" screens in that they can be generated to permit rapid development and relatively easy detection of an alteration in a molecular target which is mediated by a test compound. Moreover, the effects of cellular toxicity and/or bioavailability of the test compound can be generally ignored in the *in vitro* system, the assay instead being focused primarily on the effect of the drug on the molecular target as may be manifest in an alteration of binding affinity with other proteins or changes in enzymatic properties of the molecular target.

### A. Use of Nucleic Acids as Probes in Mapping and in Tissue Profiling

### Probes

Polynucleotide probes as described above, e.g., comprising at least 12 contiguous nucleotides selected from the nucleotide sequence of an nucleic acid as shown in SEQ ID Nos. 1-850, preferably SEQ ID Nos. 1-383, even more preferably SEQ ID Nos. 1-127, or a sequence complementary thereto, are used for a variety of purposes, including identification of human chromosomes and determining transcription levels. Additional disclosure about preferred regions of the nucleic acid sequences is found in the accompanying tables.

The nucleotide probes are labeled, for example, with a radioactive, fluorescent, biotinylated, or chemiluminescent label, and detected by well known methods appropriate for the particular label selected. Protocols for hybridizing nucleotide probes to preparations of metaphase chromosomes are also well known in the art. A nucleotide probe will hybridize specifically to nucleotide sequences in the chromosome preparations which are complementary to the nucleotide sequence of the probe. A probe that hybridizes specifically to an nucleic acid should provide a detection signal at least 5-, 10-, or 20-fold higher than the background hybridization provided with other unrelated sequences.

In a non-limiting example, commercial programs are available for identifying regions of chromosomes commonly associated with disease, such as cancer. Nucleic acids of the invention can be used to probe these regions. For example, if, through profile searching, a nucleic acid is identified as corresponding to a gene encoding a kinase, its ability to bind to a cancer-related chromosomal region will suggest its role as a kinase in one or more stages of tumor cell development/growth. Although some experimentation would be required to elucidate the role, the nucleic acid constitutes a new material for isolating a specific protein that has potential for developing a cancer diagnostic or therapeutic.

Nucleotide probes are used to detect expression of a gene corresponding to the nucleic acid. For example, in Northern blots, mRNA is separated electrophoretically and contacted with a probe. A probe is detected as hybridizing to an mRNA species of a particular size. The amount of hybridization is quantitated to determine relative amounts of expression, for example under a particular condition. Probes are also used to detect products of amplification by polymerase chain reaction. The products of the reaction are hybridized to the probe and hybrids are detected. Probes are used for in situ hybridization to cells to detect expression. Probes can also be used in vivo for diagnostic detection of hybridizing sequences. Probes are typically labeled with a radioactive isotope. Other types of detectable labels may be used such as chromophores, fluorophores, and enzymes.

Expression of specific mRNA can vary in different cell types and can be tissue specific. This variation of mRNA levels in different cell types can be exploited with nucleic acid probe assays to determine tissue types. For example, PCR, branched DNA probe assays, or blotting techniques utilizing nucleic acid probes substantially identical or complementary to nucleic acids of SEQ ID Nos. 1-850, preferably SEQ ID Nos. 1-383, even more preferably SEQ ID Nos. 1-127, or a sequence complementary thereto, can determine the presence or absence of target cDNA or mRNA.

Examples of a nucleotide hybridization assay are described in Urdea *et al*., PCT WO92/02526 and Urdea *et al*., U.S. Patent No. 5,124,246, both incorporated herein by reference. The references describe an example of a sandwich nucleotide hybridization assay.

Alternatively, the Polymerase Chain Reaction (PCR) is another means for detecting small amounts of target nucleic acids, as described in Mullis *et al., Meth. Enzymol*. (1987) *155*:335-350; U.S. Patent No. 4,683,195; and U.S. Patent No. 4,683,202, all incorporated herein by reference. Two primer polynucleotides nucleotides hybridize with the target nucleic acids and are used to prime the reaction. The primers may be composed of sequence within or 3' and 5' to the polynucleotides of the Sequence Listing. Alternatively, if the primers are 3' and 5' to these polynucleotides, they need not hybridize to them or the complements. A thermostable polymerase creates copies of target nucleic acids from the primers using the original target nucleic acids as a template. After a large amount of target nucleic acids is generated by the polymerase, it is detected by methods such as Southern blots. When using the Southern blot method, the labeled probe will hybridize to a polynucleotide of the Sequence Listing or complement.

Furthermore, mRNA or cDNA can be detected by traditional blotting techniques described in Sambrook *et al*., "Molecular Cloning: A Laboratory Manual" (New York, Cold Spring Harbor Laboratory, 1989). mRNA or cDNA generated from mRNA using a polymerase enzyme can be purified and separated using gel electrophoresis. The nucleic acids on the gel are then blotted onto a solid support, such as nitrocellulose. The solid support is exposed to a labeled probe and then washed to remove any unhybridized probe. Next, the duplexes containing the labeled probe are detected. Typically, the probe is labeled with radioactivity.

### Mapping

Nucleic acids of the present invention are used to identify a chromosome on which the corresponding gene resides. Using fluorescence in situ hybridization (FISH) on normal metaphase spreads, comparative genomic hybridization allows total genome assessment of changes in relative copy number of DNA sequences. See Schwartz and Samad, *Current Opinions in Biotechnology* (1994) *8*:70-74; Kallioniemi *et al*., *Seminars in Cancer Biology* (1993) *4*:41-46; Valdes and Tagle, *Methods in Molecular Biology* (1997) *68*:1, Boultwood, ed., Human Press, Totowa, NJ.

Preparations of human metaphase chromosomes are prepared using standard cytogenetic techniques from human primary tissues or cell lines. Nucleotide probes comprising at least 12 contiguous nucleotides selected from the nucleotide sequence of SEQ ID Nos. 1-850, preferably SEQ ID Nos. 1-383, even more preferably SEQ ID Nos. 1-127, or a sequence complementary thereto, are used to identify the corresponding chromosome. The nucleotide probes are labeled, for example, with a radioactive, fluorescent, biotinylated, or chemiluminescent label, and detected by well known methods appropriate for the particular label selected. Protocols for hybridizing nucleotide probes to preparations of metaphase chromosomes are also well known in the art. A nucleotide probe will hybridize specifically to nucleotide sequences in the chromosome preparations that are complementary to the nucleotide sequence of the probe. A probe that hybridizes specifically to a target gene provides a detection signal at least 5-, 10-, or 20-fold higher than the background hybridization provided with unrelated coding sequences.

Nucleic acids are mapped to particular chromosomes using, for example, radiation hybrids or chromosome-specific hybrid panels. See Leach *et al., Advances in Genetics,* (1995) *33*:63-99; Walter *et al., Nature Genetics* (1994) *7*:22-28; Walter and Goodfellow, *Trends in Genetics* (1992) *9*:352. Panels for radiation hybrid mapping are available from Research Genentics, Inc., Huntsville, Alabama, USA. Databases for markers using various panels are available via the world wide web at http:/F/shgc-www.stanford.edu; and other locations. The statistical program RHMAP can be used to construct a map based on the data from radiation hybridization with a measure of the relative likelihood of one order versus another. RHMAP is available via the world wide web at http://www.sph.umich.edu/group/statgen/soflware.

Such mapping can be useful in identifying the function of the target gene by its proximity to other genes with known function. Function can also be assigned to the target gene when particular syndromes or diseases map to the same chromosome.

### Tissue Profiling

The nucleic acids of the present invention can be used to determine the tissue type from which a given sample is derived. For example, a metastatic lesion is identified by its developmental organ or tissue source by identifying the expression of a particular marker of that organ or tissue. If a nucleic acid is expressed only in a specific tissue type, and a metastatic lesion is found to express that nucleic acid, then the developmental source of the lesion has been identified. Expression of a particular nucleic acid is assayed by detection of either the corresponding mRNA or the protein product. Immunological methods, such as antibody staining, are used to detect a particular protein product. Hybridization methods may be used to detect particular mRNA species, including but not limited to in situ hybridization and Northern blotting.

### Use of Polymorphisms

A nucleic acid will be useful in forensics, genetic analysis, mapping, and diagnostic applications if the corresponding region of a gene is polymorphic in the human population. A particular polymorphic form of the nucleic acid may be used to either identify a sample as deriving from a suspect or rule out the possibility that the sample derives from the suspect. Any means for detecting a polymorphism in a gene are used, including but not limited to electrophoresis of protein polymorphic variants, differential sensitivity to restriction enzyme cleavage, and hybridization to an allele-specific probe.

### B. Use of Nucleic Acids and Encoded Polypeptides to Raise Antibodies

Expression products of a nucleic acid, the corresponding mRNA or cDNA, or the corresponding complete gene are prepared and used for raising antibodies for experimental, diagnostic, and therapeutic purposes. For nucleic acids to which a corresponding gene has not been assigned, this provides an additional method of identifying the corresponding gene. The nucleic acid or related cDNA is expressed as described above, and antibodies are prepared. These antibodies are specific to an epitope on the encoded polypeptide, and can precipitate or bind to the corresponding native protein in a cell or tissue preparation or in a cell-free extract of an in vitro expression system.

Immunogens for raising antibodies are prepared by mixing the polypeptides encoded by the nucleic acids of the present invention with adjuvants. Alternatively, polypeptides are made as fusion proteins to larger immunogenic proteins. Polypeptides are also covalently linked to other larger immunogenic proteins, such as keyhole limpet hemocyanin. Immunogens are typically administered intradermally, subcutaneously, or intramuscularly. Immunogens are administered to experimental animals such as rabbits, sheep, and mice, to generate antibodies. Optionally, the animal spleen cells are isolated and fused with myeloma cells to form hybridomas which secrete monoclonal antibodies. Such methods are well known in the art. According to another method known in the art, the nucleic acid is administered directly, such as by intramuscular injection, and expressed in vivo. The expressed protein generates a variety of protein-specific immune responses, including production of antibodies, comparable to administration of the protein.

Preparations of polyclonal and monoclonal antibodies specific for nucleic acid-encoded proteins and polypeptides are made using standard methods known in the art. The antibodies specifically bind to epitopes present in the polypeptides encoded by a nucleic acid of SEQ ID Nos. 1-850, preferably SEQ ID Nos. 1-383, even more preferably SEQ ID Nos. 1-127, or a sequence complementary thereto. In another embodiment, the antibodies specifically bind to epitopes present in a polypeptide encoded by SEQ ID Nos. 1-850. Typically, at least about 6, 8, 10, or 12 contiguous amino acids are required to form an epitope. However, epitopes which involve non-contiguous amino acids may require more, for example, at least about 15, 25, or 50 amino acids. A short sequence of a nucleic acid may then be unsuitable for use as an epitope to raise antibodies for identifying the corresponding novel protein, because of the potential for cross-reactivity with a known protein. However, the antibodies may be useful for other purposes, particularly if they identify common structural features of a known protein and a novel polypeptide encoded by a nucleic acid of the invention.

Antibodies that specifically bind to human nucleic acid-encoded polypeptides should provide a detection signal at least about 5-, 10-, or 20-fold higher than a detection signal provided with other proteins when used in Western blots or other immunochemical assays. Preferably, antibodies that specifically bind nucleic acid T-encoded polypeptides do not detect other proteins in immunochemical assays and can immunoprecipitate nucleic acid-encoded proteins from solution.

To test for the presence of serum antibodies to the nucleic acid-encoded polypeptide in a human population, human antibodies are purified by methods well known in the art. Preferably, the antibodies are affinity purified by passing antiserum over a column to which an nucleic acid-encoded protein, polypeptide, or fusion protein is bound. The bound antibodies can then be eluted from the column, for example using a buffer with a high salt concentration.

In addition to the antibodies discussed above, genetically engineered antibody derivatives are made, such as single chain antibodies.

Antibodies may be made by using standard protocols known in the art (See, for example, Antibodies: A Laboratory Manual ed. by Harlow and Lane (Cold Spring Harbor Press: 1988)). A mammal, such as a mouse, hamster, or rabbit can be immunized with an immunogenic form of the peptide (e.g., a mammalian polypeptide or an antigenic fragment which is capable of eliciting an antibody response, or a fusion protein as described above).

In one aspect, this invention includes monoclonal antibodies that show a subject polypeptide is highly expressed in colorectal tissue or tumor tissue, especially colon cancer tissue or colon cancer-derived cell lines. Therefore, in one embodiment, this invention provides a diagnostic tool for the analysis of expression of a subject polypeptide in general, and in particular, as a diagnostic for colon cancer.

Techniques for conferring immunogenicity on a protein or peptide include conjugation to carriers or other techniques well known in the art. An immunogenic portion of a protein can be administered in the presence of adjuvant. The progress of immunization can be monitored by detection of antibody titers in plasma or serum. Standard ELISA or other immunoassays can be used with the immunogen as antigen to assess the levels of antibodies. In a preferred embodiment, the subject antibodies are immunospecific for antigenic determinants of a protein of a mammal, e.g., antigenic determinants of a protein encoded by one of SEQ ID Nos. 1-850 or closely related homologs (e.g., at least 90% identical, and more preferably at least 95% identical).

Following immunization of an animal with an antigenic preparation of a polypeptide, antisera can be obtained and, if desired, polyclonal antibodies isolated from the serum. To produce monoclonal antibodies, antibody-producing cells (lymphocytes) can be harvested from an immunized animal and fused by standard somatic cell fusion procedures with immortalizing cells such as myeloma cells to yield hybridoma cells. Such techniques are well known in the art, and include, for example, the hybridoma technique (originally developed by Kohler and Milstein, (1975) Nature, 256: 495-497), the human B cell hybridoma technique (Kozbar *et al*., (1983) Immunology Today, 4: 72), and the EBV-hybridoma technique to produce human monoclonal antibodies (Cole et al., (1985) Monoclonal Antibodies and Cancer Therapy, Alan R. Liss, Inc. pp. 77-96). Hybridoma cells can be screened immunochemically for production of antibodies specifically reactive with a polypeptide of the present invention and monoclonal antibodies isolated from a culture comprising such hybridoma cells.

The term antibody as used herein is intended to include fragments thereof which are also specifically reactive with one of the subject polypeptides. Antibodies can be fragmented using conventional techniques and the fragments screened for utility in the same manner as described above for whole antibodies. For example, F(ab)₂ fragments can be generated by treating antibody with pepsin. The resulting F(ab)₂ fragment can be treated to reduce disulfide bridges to produce Fab fragments. The antibody of the present invention is further intended to include bispecific, single-chain, and chimeric and humanized molecules having affinity for a polypeptide conferred by at least one CDR region of the antibody. In preferred embodiments, the antibodies, the antibody further comprises a label attached thereto and able to be detected, (e.g., the label can be a radioisotope, fluorescent compound, chemiluminescent compound, enzyme, or enzyme co-factor).

Antibodies can be used, e.g., to monitor protein levels in an individual for determining, e.g., whether a subject has a disease or condition, such as colon cancer, associated with an aberrant protein level, or allowing determination of the efficacy of a given treatment regimen for an individual afflicted with such a disorder. The level of polypeptides may be measured from cells in bodily fluid, such as in blood samples.

Another application of antibodies of the present invention is in the immunological screening of cDNA libraries constructed in expression vectors such as gt11, gt18-23, ZAP, and ORF8. Messenger libraries of this type, having coding sequences inserted in the correct reading frame and orientation, can produce fusion proteins. For instance, gt11 will produce fusion proteins whose amino termini consist of β-galactosidase amino acid sequences and whose carboxyl termini consist of a foreign polypeptide. Antigenic epitopes of a protein, e.g., other orthologs of a particular protein or other paralogs from the same species, can then be detected with antibodies, as, for example, reacting nitrocellulose filters lifted from infected plates with antibodies. Positive phage detected by this assay can then be isolated from the infected plate. Thus, the presence of homologs can be detected and cloned from other animals, as can alternate isoforms (including splicing variants) from humans.

In another embodiment, a panel of monoclonal antibodies may be used, wherein each of the epitope's involved functions are represented by a monoclonal antibody. Loss or perturbation of binding of a monoclonal antibody in the panel would be indicative of a mutational attention of the protein and thus of the corresponding gene.

### C. Differential Expression

The present invention also provides a method to identify abnormal or diseased tissue in a human. For nucleic acids corresponding to profiles of protein families as described above, the choice of tissue may be dictated by the putative biological function. The expression of a gene corresponding to a specific nucleic acid is compared between a first tissue that is suspected of being diseased and a second, normal tissue of the human. The normal tissue is any tissue of the human, especially those that express the target gene including, but not limited to, brain, thymus, testis, heart, prostate, placenta, spleen, small intestine, skeletal muscle, pancreas, and the mucosal lining of the colon.

The tissue suspected of being abnormal or diseased can be derived from a different tissue type of the human, but preferably it is derived from the same tissue type; for example an intestinal polyp or other abnormal growth should be compared with normal intestinal tissue. A difference between the target gene, mRNA, or protein in the two tissues which are compared, for example in molecular weight, amino acid or nucleotide sequence, or relative abundance, indicates a change in the gene, or a gene which regulates it, in the tissue of the human that was suspected of being diseased.

The target genes in the two tissues are compared by any means known in the art. For example, the two genes are sequenced, and the sequence of the gene in the tissue suspected of being diseased is compared with the gene sequence in the normal tissue. The target genes, or portions thereof, in the two tissues are amplified, for example using nucleotide primers based on the nucleotide sequence shown in the Sequence Listing, using the polymerase chain reaction. The amplified genes or portions of genes are hybridized to nucleotide probes selected from a corresponding nucleotide sequence shown SEQ ID No. 1-850. A difference in the nucleotide sequence of the target gene in the tissue suspected of being diseased compared with the normal nucleotide sequence suggests a role of the nucleic acid-encoded proteins in the disease, and provides a lead for preparing a therapeutic agent. The nucleotide probes are labeled by a variety of methods, such as radiolabeling, biotinylation, or labeling with fluorescent or chemiluminescent tags, and detected by standard methods known in the art.

Alternatively, target mRNA in the two tissues is compared. PolyA⁺RNA is isolated from the two tissues as is known in the art. For example, one of skill in the art can readily determine differences in the size or amount of target mRNA transcripts between the two tissues using Northern blots and nucleotide probes selected from the nucleotide sequence shown in the Sequence Listing. Increased or decreased expression of a target mRNA in a tissue sample suspected of being diseased, compared with the expression of the same target mRNA in a normal tissue, suggests that the expressed protein has a role in the disease, and also provides a lead for preparing a therapeutic agent.

Any method for analyzing proteins is used to compare two nucleic acid-encoded proteins from matched samples. The sizes of the proteins in the two tissues are compared, for example, using antibodies of the present invention to detect nucleic acid-encoded proteins in Western blots of protein extracts from the two tissues. Other changes, such as expression levels and subcellular localization, can also be detected immunologically, using antibodies to the corresponding protein. A higher or lower level of nucleic acid-encoded protein expression in a tissue suspected of being diseased, compared with the same nucleic acid-encoded protein expression level in a normal tissue, is indicative that the expressed protein has a role in the disease, and provides another lead for preparing a therapeutic agent.

Similarly, comparison of gene sequences or of gene expression products, e.g., mRNA and protein, between a human tissue that is suspected of being diseased and a normal tissue of a human, are used to follow disease progression or remission in the human. Such comparisons of genes, mRNA, or protein are made as described above.

For example, increased or decreased expression of the target gene in the tissue suspected of being neoplastic can indicate the presence of neoplastic cells in the tissue. The degree of increased expression of the target gene in the neoplastic tissue relative to expression of the gene in normal tissue, or differences in the amount of increased expression of the target gene in the neoplastic tissue over time, is used to assess the progression of the neoplasia in that tissue or to monitor the response of the neoplastic tissue to a therapeutic protocol over time.

The expression pattern of any two cell types can be compared, such as low and high metastatic tumor cell lines, or cells from tissue which have and have not been exposed to a therapeutic agent. A genetic predisposition to disease in a human is detected by comparing an target gene, mRNA, or protein in a fetal tissue with a normal target gene, mRNA, or protein. Fetal tissues that are used for this purpose include, but are not limited to, amniotic fluid, chorionic villi, blood, and the blastomere of an in vitro-fertilized embryo. The comparable normal target gene is obtained from any tissue. The mRNA or protein is obtained from a normal tissue of a human in which the target gene is expressed. Differences such as alterations in the nucleotide sequence or size of the fetal target gene or mRNA, or alterations in the molecular weight, amino acid sequence, or relative abundance of fetal target protein, can indicate a germline mutation in the target gene of the fetus, which indicates a genetic predisposition to disease.

### D. Use of Nucleic Acids, and Encoded Polypeptides to Screen for Peptide Analogs and Antagonists

Polypeptides encoded by the instant nucleic acids, e.g., SEQ ID Nos. 1-850, preferably SEQ ID Nos. 1-383, even more preferably SEQ ID Nos. 1-127, or a sequence complementary thereto, and corresponding full length genes can be used to screen peptide libraries to identify binding partners, such as receptors, from among the encoded polypeptides.

A library of peptides may be synthesized following the methods disclosed in U.S. Pat. No. 5,010,175, and in PCT WO 91/17823. As described below in brief, one prepares a mixture of peptides, which is then screened to identify the peptides exhibiting the desired signal transduction and receptor binding activity. In the '175 method, a suitable peptide synthesis support (e.g., a resin) is coupled to a mixture of appropriately protected, activated amino acids. The concentration of each amino acid in the reaction mixture is balanced or adjusted in inverse proportion to its coupling reaction rate so that the product is an equimolar mixture of amino acids coupled to the starting resin. The bound amino acids are then deprotected, and reacted with another balanced amino acid mixture to form an equimolar mixture of all possible dipeptides. This process is repeated until a mixture of peptides of the desired length (*e*.*g*., hexamers) is formed. Note that one need not include all amino acids in each step: one may include only one or two amino acids in some steps (e.g., where it is known that a particular amino acid is essential in a given position), thus reducing the complexity of the mixture. After the synthesis of the peptide library is completed, the mixture of peptides is screened for binding to the selected polypeptide. The peptides are then tested for their ability to inhibit or enhance activity. Peptides exhibiting the desired activity are then isolated and sequenced.

The method described in WO 91/17823 is similar. However, instead of reacting the synthesis resin with a mixture of activated amino acids, the resin is divided into twenty equal portions (or into a number of portions corresponding to the number of different amino acids to be added in that step), and each amino acid is coupled individually to its portion of resin. The resin portions are then combined, mixed, and again divided into a number of equal portions for reaction with the second amino acid. In this manner, each reaction may be easily driven to completion. Additionally, one may maintain separate "subpools" by treating portions in parallel, rather than combining all resins at each step. This simplifies the process of determining which peptides are responsible for any observed receptor binding or signal transduction activity.

In such cases, the subpools containing, e.g., 1-2,000 candidates each are exposed to one or more polypeptides of the invention. Each subpool that produces a positive result is then resynthesized as a group of smaller subpools (sub-subpools) containing, e.g., 20-100 candidates, and reassayed. Positive sub-subpools may be resynthesized as individual compounds, and assayed finally to determine the peptides that exhibit a high binding constant. These peptides can be tested for their ability to inhibit or enhance the native activity. The methods described in WO 91/7823 and U.S. Patent No. 5,194,392 (herein incorporated by reference) enable the preparation of such pools and subpools by automated techniques in parallel, such that all synthesis and resynthesis may be performed in a matter of days.

Peptide agonists or antagonists are screened using any available method, such as signal transduction, antibody binding, receptor binding, mitogenic assays, chemotaxis assays, etc. The methods described herein are presently preferred. The assay conditions ideally should resemble the conditions under which the native activity is exhibited *in vivo*, that is, under physiologic pH, temperature, and ionic strength. Suitable agonists or antagonists will exhibit strong inhibition or enhancement of the native activity at concentrations that do not cause toxic side effects in the subject. Agonists or antagonists that compete for binding to the native polypeptide may require concentrations equal to or greater than the native concentration, while inhibitors capable of binding irreversibly to the polypeptide may be added in concentrations on the order of the native concentration.

The end results of such screening and experimentation will be at least one novel polypeptide binding partner, such as a receptor, encoded by a nucleic acid of the invention, and at least one peptide agonist or antagonist of the novel binding partner. Such agonists and antagonists can be used to modulate, enhance, or inhibit receptor function in cells to which the receptor is native, or in cells that possess the receptor as a result of genetic engineering. Further, if the novel receptor shares biologically important characteristics with a known receptor, information about agonist/antagonist binding may help in developing improved agonists/antagonists of the known receptor.

### E. Pharmaceutical Compositions and Therapeutic Uses

Pharmaceutical compositions can comprise polypeptides, antibodies, or polynucleotides of the claimed invention. The pharmaceutical compositions will comprise a therapeutically effective amount of either polypeptides, antibodies, or polynucleotides of the claimed invention.

The term "therapeutically effective amount" as used herein refers to an amount of a therapeutic agent to treat, ameliorate, or prevent a desired disease or condition, or to exhibit a detectable therapeutic or preventative effect. The effect can be detected by, for example, chemical markers or antigen levels. Therapeutic effects also include reduction in physical symptoms, such as decreased body temperature. The precise effective amount for a subject will depend upon the subject's size and health, the nature and extent of the condition, and the therapeutics or combination of therapeutics selected for administration. Thus, it is not useful to specify an exact effective amount in advance. However, the effective amount for a given situation can be determined by routine experimentation and is within the judgment of the clinician.

For purposes of the present invention, an effective dose will be from about 0.01 mg/ kg to 50 mg/kg or 0.05 mg/kg to about 10 mg/kg of the DNA constructs in the individual to which it is administered.

A pharmaceutical composition can also contain a pharmaceutically acceptable carrier. The term "pharmaceutically acceptable carrier" refers to a carrier for administration of a therapeutic agent, such as antibodies or a polypeptide, genes, and other therapeutic agents. The term refers to any pharmaceutical carrier that does not itself induce the production of antibodies harmful to the individual receiving the composition, and which may be administered without undue toxicity. Suitable carriers may be large, slowly metabolized macromolecules such as proteins, polysaccharides, polylactic acids, polyglycolic acids, polymeric amino acids, amino acid copolymers, and inactive virus particles. Such carriers are well known to those of ordinary skill in the art.

Pharmaceutically acceptable salts can be used therein, for example, mineral acid salts such as hydrochlorides, hydrobromides, phosphates, sulfates, and the like; and the salts of organic acids such as acetates, propionates, malonates, benzoates, and the like. A thorough discussion of pharmaceutically acceptable excipients is available in *Remington's Pharmaceutical Sciences* (Mack Pub. Co., N.J. 1991).

Pharmaceutically acceptable carriers in therapeutic compositions may contain liquids such as water, saline, glycerol and ethanol. Additionally, auxiliary substances, such as wetting or emulsifying agents, pH buffering substances, and the like, may be present in such vehicles. Typically, the therapeutic compositions are prepared as injectables, either as liquid solutions or suspensions; solid forms suitable for solution in, or suspension in, liquid vehicles prior to injection may also be prepared. Liposomes are included within the definition of a pharmaceutically acceptable carrier.

### Delivery Methods

Once formulated, the nucleic acid compositions of the invention can be (1) administered directly to the subject; (2) delivered ex vivo, to cells derived from the subject; or (3) delivered in vitro for expression of recombinant proteins.

Direct delivery of the compositions will generally be accomplished by injection, either subcutaneously, intraperitoneally, intravenously or intramuscularly, or delivered to the interstitial space of a tissue. The compositions can also be administered into a tumor or lesion. Other modes of administration include oral and pulmonary administration, suppositories, and transdermal applications, needles, and gene guns or hyposprays. Dosage treatment may be a single dose schedule or a multiple dose schedule.

Methods for the ex vivo delivery and reimplantation of transformed cells into a subject are known in the art and described in e.g., International Publication No. WO 93/14778. Examples of cells useful in ex vivo applications include, for example, stem cells, particularly hematopoetic, lymph cells, macrophages, dendritic cells, or tumor cells.

Generally, delivery of nucleic acids for both ex vivo and in vitro applications can be accomplished by, for example, dextran-mediated transfection, calcium phosphate precipitation, polybrene mediated transfection, protoplast fusion, electroporation, encapsulation of the polynucleotide(s) in liposomes, and direct microinjection of the DNA into nuclei, all well known in the art.

Once a subject gene has been found to correlate with a proliferative disorder, such as neoplasia, dysplasia, and hyperplasia, the disorder may be amenable to treatment by administration of a therapeutic agent based on the nucleic acid or corresponding polypeptide.

Preparation of antisense polypeptides is discussed above. Neoplasias that are treated with the antisense composition include, but are not limited to, cervical cancers, melanomas, colorectal adenocarcinomas, Wilms' tumor, retinoblastoma, sarcomas, myosarcomas, lung carcinomas, leukemias, such as chronic myelogenous leukemia, promyelocytic leukemia, monocytic leukemia, and myeloid leukemia, and lymphomas, such as histiocytic lymphoma. Proliferative disorders that are treated with the therapeutic composition include disorders such as anhydric hereditary ectodermal dysplasia, congenital alveolar dysplasia, epithelial dysplasia of the cervix, fibrous dysplasia of bone, and mammary dysplasia. Hyperplasias, for example, endometrial, adrenal, breast, prostate, or thyroid hyperplasias or pseudoepitheliomatous hyperplasia of the skin, are treated with antisense therapeutic compositions. Even in disorders in which mutations in the corresponding gene are not implicated, downregulation or inhibition of nucleic acid-related gene expression can have therapeutic application. For example, decreasing nucleic acid-related gene expression can help to suppress tumors in which enhanced expression of the gene is implicated.

Both the dose of the antisense composition and the means of administration are determined based on the specific qualities of the therapeutic composition, the condition, age, and weight of the patient, the progression of the disease, and other relevant factors. Administration of the therapeutic antisense agents of the invention includes local or systemic administration, including injection, oral administration, particle gun or catheterized administration, and topical administration. Preferably, the therapeutic antisense composition contains an expression construct comprising a promoter and a polynucleotide segment of at least about 12, 22, 25, 30, or 35 contiguous nucleotides of the antisense strand of a nucleic acid. Within the expression construct, the polynucleotide segment is located downstream from the promoter, and transcription of the polynucleotide segment initiates at the promoter.

Various methods are used to administer the therapeutic composition directly to a specific site in the body. For example, a small metastatic lesion is located and the therapeutic composition injected several times in several different locations within the body of tumor. Alternatively, arteries which serve a tumor are identified, and the therapeutic composition injected into such an artery, in order to deliver the composition directly into the tumor. A tumor that has a necrotic center is aspirated and the composition injected directly into the now empty center of the tumor. The antisense composition is directly administered to the surface of the tumor, for example, by topical application of the composition. X-ray imaging is used to assist in certain of the above delivery methods.

Receptor-mediated targeted delivery of therapeutic compositions containing an antisense polynucleotide, subgenomic polynucleotides, or antibodies to specific tissues is also used. Receptor-mediated DNA delivery techniques are described in, for example, Findeis *et al*., *Trends in Biotechnol*. (1993) *11*:202-205; Chiou *et al*., (1994) Gene Therapeutics: Methods And Applications Of Direct Gene Transfer (J.A. Wolff, ed.); Wu & Wu, *J. Biol. Chem.* (1988) *263*:621-24; Wu *et al*., *J. Biol. Chem.* (1994) *269*:542-46; Zenke *et al*., *Proc. Natl. Acad. Sci. (USA)* (1990) *87*:3655-59; Wu *et al*., *J. Biol. Chem.* (1991) *266*:338-42. Preferably, receptor-mediated targeted delivery of therapeutic compositions containing antibodies of the invention is used to deliver the antibodies to specific tissue.

Therapeutic compositions containing antisense subgenomic polynucleotides are administered in a range of about 100 ng to about 200 mg of DNA for local administration in a gene therapy protocol. Concentration ranges of about 500 ng to about 50 mg, about 1 mg to about 2 mg, about 5 mg to about 500 mg, and about 20 mg to about 100 mg of DNA can also be used during a gene therapy protocol. Factors such as method of action and efficacy of transformation and expression are considerations which will affect the dosage required for ultimate efficacy of the antisense subgenomic nucleic acids. Where greater expression is desired over a larger area of tissue, larger amounts of antisense subgenomic nucleic acids or the same amounts readministered in a successive protocol of administrations, or several administrations to different adjacent or close tissue portions of, for example, a tumor site, may be required to effect a positive therapeutic outcome. In all cases, routine experimentation in clinical trials will determine specific ranges for optimal therapeutic effect. A more complete description of gene therapy vectors, especially retroviral vectors, is contained in U.S. Serial No. 08/869,309, which is expressly incorporated herein, and in section F below.

For genes encoding polypeptides or proteins with anti-inflammatory activity, suitable use, doses, and administration are described in U.S. Patent No. 5,654,173, incorporated herein by reference. Therapeutic agents also include antibodies to proteins and polypeptides encoded by the subject nucleic acids, as described in U.S. Patent No. 5,654,173.

### F. Gene Therapy

The therapeutic nucleic acids of the present invention may be utilized in gene delivery vehicles. The gene delivery vehicle may be of viral or non-viral origin (see generally, Jolly, *Cancer Gene Therapy* (1994) *1*:51-64; Kimura, *Human Gene Therpay* (1994) *5*:845-852; Connelly, *Human Gene Therapy* (1995) *1*:185-193; and Kaplitt, *Nature Genetics* (1994) *6*:148-153). Gene therapy vehicles for delivery of constructs including a coding sequence of a therapeutic of the invention can be administered either locally or systemically. These constructs can utilize viral or non-viral vector approaches. Expression of such coding sequences can be induced using endogenous mammalian or heterologous promoters. Expression of the coding sequence can be either constitutive or regulated.

The present invention can employ recombinant retroviruses which are constructed to carry or express a selected nucleic acid molecule of interest. Retrovirus vectors that can be employed include those described in EP 0 415 731; WO 90/07936; WO 94/03622; WO 93/25698; WO 93/25234; U.S. Patent No. 5, 219,740; WO 93/11230; WO 93/10218; Vile and Hart, *Cancer Res.* (1993) *53*:3860-3864; Vile and Hart, *Cancer Res.* (1993) *53*:962-967; Ram et al., *Cancer Res.* (1993) *53*:83-88; Takamiya et al., J. *Neurosci. Res.* (1992) *33*:493-503; Baba et al., *J. Neurosurg.* (1993) *79*:729-735; U.S. Patent no. 4,777,127; GB Patent No. 2,200,651; and EP 0 345 242. Preferred recombinant retroviruses include those described in WO 91/02805.

Packaging cell lines suitable for use with the above-described retroviral vector constructs may be readily prepared (see PCT publications WO 95/30763 and WO 92/05266), and used to create producer cell lines (also termed vector cell lines) for the production of recombinant vector particles. Within particularly preferred embodiments of the invention, packaging cell lines are made from human (such as HT1080 cells) or mink parent cell lines, thereby allowing production of recombinant retroviruses that can survive inactivation in human serum.

The present invention also employs alphavirus-based vectors that can function as gene delivery vehicles. Such vectors can be constructed from a wide variety of alphaviruses, including, for example, Sindbis virus vectors, Semliki forest virus (ATCC VR-67; ATCC VR-1247), Ross River virus (ATCC VR-373; ATCC VR-1246) and Venezuelan equine encephalitis virus (ATCC VR-923; ATCC VR-1250; ATCC VR 1249; ATCC VR-532). Representative examples of such vector systems include those described in U.S. Patent Nos. 5,091,309; 5,217,879; and 5,185,440; and PCT Publication Nos. WO 92/10578; WO 94/21792; WO 95/27069; WO 95/27044; and WO 95/07994.

Gene delivery vehicles of the present invention can also employ parvovirus such as adeno-associated virus (AAV) vectors. Representative examples include the AAV vectors disclosed by Srivastava in WO 93/09239, Samulski et al., *J. Vir*. (1989) *63*:3822-3828; Mendelson et al., *Virol.* (1988) *166*:154-165; and Flotte et al., *PNAS* (1993) *90*:10613-10617.

Representative examples of adenoviral vectors include those described by Berkner, *Biotechniques* (1988) *6*:616-627; Rosenfeld et al., *Science* (1991) *252*:431-434; WO 93/19191; Kolls et al., *PNAS* (1994) *91*:215-219; Kass-Eisler et al., *PNAS* (1993) *90*:11498-11502; Guzman et al., *Circulation* (1993) *88*:2838-2848; Guzman et al., *Cir. Res.* (1993) *73*:1202-1207; Zabner et al., *Cell* (1993) *75*:207-216; Li et al., *Hum. Gene Ther.* (1993) *4*:403-409; Cailaud et al., *Eur. J. Neurosci.* (1993) *5*:1287-1291; Vincent et al., *Nat. Genet.* (1993) *5*:130-134; Jaffe et al., *Nat. Genet.* (1992) *1*:372-378; and Levrero et al., *Gene* (1991) *101*:195-202. Exemplary adenoviral gene therapy vectors employable in this invention also include those described in WO 94/12649, WO 93/03769; WO 93/19191; WO 94/28938; WO 95/11984 and WO 95/00655. Administration of DNA linked to killed adenovirus as described in Curiel, *Hum. Gene Ther*. (1992) *3*:147-154 may be employed.

Other gene delivery vehicles and methods may be employed, including polycationic condensed DNA linked or unlinked to killed adenovirus alone, for example Curiel, *Hum. Gene Ther*. (1992) *3*:147-154; ligand linked DNA, for example see Wu, *J. Biol. Chem.* (1989) *264*:16985-16987; eukaryotic cell delivery vehicles cells, for example see U.S. Serial No. 08/240,030, filed May 9, 1994, and U.S. Serial No. 08/404,796; deposition of photopolymerized hydrogel materials; hand-held gene transfer particle gun, as described in U.S. Patent No. 5,149,655; ionizing radiation as described in U.S. Patent No. 5,206,152 and in WO92/11033; nucleic charge neutralization or fusion with cell membranes. Additional approaches are described in Philip, *Mol. Cell Biol*. (1994) *14*:2411-2418, and in Woffendin, *Proc. Natl. Acad. Sci.* (1994) *91*:1581-1585.

Naked DNA may also be employed. Exemplary naked DNA introduction methods are described in WO 90/11092 and U.S. Patent No. 5,580,859. Uptake efficiency may be improved using biodegradable latex beads. DNA coated latex beads are efficiently transported into cells after endocytosis initiation by the beads. The method may be improved further by treatment of the beads to increase hydrophobicity and thereby facilitate disruption of the endosome and release of the DNA into the cytoplasm. Liposomes that can act as gene delivery vehicles are described in U.S. Patent No. 5,422,120, PCT Nos. WO 95/13796, WO 94/23697, and WO 91/14445, and EP No. 0 524 968.

Further non-viral delivery suitable for use includes mechanical delivery systems such as the approach described in Woffendin *et al*., *Proc. Natl. Acad. Sci. USA* (1994) *91*(24):11581-11585. Moreover, the coding sequence and the product of expression of such can be delivered through deposition of photopolymerized hydrogel materials. Other conventional methods for gene delivery that can be used for delivery of the coding sequence include, for example, use of hand-held gene transfer particle gun, as described in U.S. Patent No. 5,149,655; use of ionizing radiation for activating transferred gene, as described in U.S. Patent No. 5,206,152 and PCT No. WO 92/11033.

### G. Transgenic Animals

One aspect of the present invention relates to transgenic non-human animals having germline and/or somatic cells in which the biological activity of one or more genes are altered by a chromosomally incorporated transgene.

In a preferred embodiments, the transgene encodes a mutant protein, such as dominant negative protein which antagonizes at least a portion of the biological function of a wild-type protein.

Yet another preferred transgenic animal includes a transgene encoding an antisense transcript which, when transcribed from the transgene, hybridizes with a gene or a mRNA transcript thereof, and inhibits expression of the gene.

In one embodiment, the present invention provides a desired non-human animal or an animal (including human) cell which contains a predefined, specific and desired alteration rendering the non-human animal or animal cell predisposed to cancer. Specifically, the invention pertains to a genetically altered non-human animal (most preferably, a mouse), or a cell (either non-human animal or human) in culture, that is defective in at least one of two alleles of a tumor-suppressor gene. The inactivation of at least one of these tumor suppressor alleles results in an animal with a higher susceptibility to tumor induction or other proliferative or differentiative disorders, or disorders marked by aberrant signal transduction, e.g., from a cytokine or growth factor. A genetically altered mouse of this type is able to serve as a useful model for hereditary cancers and as a test animal for carcinogen studies. The invention additionally pertains to the use of such non-human animals or animal cells, and their progeny in research and medicine.

Furthermore, it is contemplated that cells of the transgenic animals of the present invention can include other transgenes, e.g., which alter the biological activity of a second tumor suppressor gene or an oncogene. For instance, the second transgene can functionally disrupt the biological activity of a second tumor suppressor gene, such as p53, p73, DCC, p21^{cip1}, p27^{kip1}, Rb, Mad or E2F. Alternatively, the second transgene can cause overexpression or loss of regulation of an oncogene, such as ras, myc, a cdc25 phosphatase, Bcl-2, Bcl-6, a transforming growth factor, neu, int-3, polyoma virus middle T antigen, SV40 large T antigen, a papillomaviral E6 protein, a papillomaviral E7 protein, CDK4, or cyclin D1.

A preferred transgenic non-human animal of the present invention has germline and/or somatic cells in which one or more alleles of a gene are disrupted by a chromosomally incorporated transgene, wherein the transgene includes a marker sequence providing a detectable signal for identifying the presence of the transgene in cells of the transgenic animal, and replaces at least a portion of the gene or is inserted into the gene or disrupts expression of a wild-type protein.

Still another aspect of the present invention relates to methods for generating non-human animals and stem cells having a functionally disrupted endogenous gene. In a preferred embodiment, the method comprises the steps of:
(i) constructing a transgene construct including (a) a recombination region having at least a portion of the gene, which recombination region directs recombination of the transgene with the gene, and (b) a marker sequence which provides a detectable signal for identifying the presence of the transgene in a cell;
(ii) transfering the transgene into stem cells of a non-human animal;
(iii) selecting stem cells having a correctly targeted homologous recombination between the transgene and the gene;
(iv) transfering cells identified in step (iii) into a non-human blastocyst and implanting the resulting chimeric blastocyst into a non-human female; and
(v) collecting offspring harboring an endogenous gene allele having the correctly targeted recombination.

Yet another aspect of the invention provides a method for evaluating the carcinogenic potential of an agent by (i) contacting a transgenic animal of the present invention with a test agent, and (ii) comparing the number of transformed cells in a sample from the treated animal with the number of transformed cells in a sample from an untreated transgenic animal or transgenic animal treated with a control agent. The difference in the number of transformed cells in the treated animal, relative to the number of transformed cells in the absence of treatment with a control agent, indicates the carcinogenic potential of the test compound.

Another aspect of the invention provides a method of evaluating an anti-proliferative activity of a test compound. In preferred embodiments, the method includes contacting a transgenic animal of the present invention, or a sample of cells from such animal, with a test agent, and determining the number of transformed cells in a specimen from the transgenic animal or in the sample of cells. A statistically significant decrease in the number of transformed cells, relative to the number of transformed cells in the absence of the test agent, indicates the test compound is a potential anti-proliferative agent.

The practice of the present invention will employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, *Molecular Cloning A Laboratory Manual*, 2nd Ed., ed. by Sambrook, Fritsch and Maniatis (Cold Spring Harbor Laboratory Press: 1989); *DNA Cloning,* Volumes I and II (D. N. Glover ed., 1985); *Oligonucleotide Synthesis* (M. J. Gait ed., 1984); Mullis *et al*. U.S. Patent No. 4,683,195; *Nucleic Acid Hybridization* (B. D. Hames & S. J. Higgins eds. 1984); *Transcription And Translation* (B. D. Hames & S. J. Higgins eds. 1984); *Culture Of Animal Cells* (R. I. Freshney, Alan R. Liss, Inc., 1987); *Immobilized Cells And Enzymes* (IRL Press, 1986); B. Perbal, *A Practical Guide To Molecular Cloning* (1984); the treatise, *Methods In Enzymology* (Academic Press, Inc., N.Y.); *Gene Transfer Vectors For Mammalian Cells* (J. H. Miller and M. P. Calos eds., 1987, Cold Spring Harbor Laboratory); *Methods In Enzymology,* Vols. 154 and 155 (Wu et al. eds.), *Immunochemical Methods In Cell And Molecular Biology* (Mayer and Walker, eds., Academic Press, London, 1987); *Handbook Of Experimental Immunology,* Volumes I-IV (D. M. Weir and C. C. Blackwell, eds., 1986); *Manipulating the Mouse Embryo,* (Cold Spring Harbor Laboratory Press, Cold Spring Harbor, N.Y., 1986).

As mentioned above, the sequences described herein are believed to have particular utility in regards to colon cancer. However, they may also be useful with other types of cancers and other disease states.

The present invention will now be illustrated by reference to the following examples which set forth particularly advantageous embodiments. However, it should be noted that these embodiments are illustrative and are not to be construed as restricting the invention in any way.

### XI. Examples

### A. Identification of differentially expressed sequences in the SW480 library

### Description of the SW480 library

SEQ ID NO 1-850 were derived from the SW480 library. The SW480 library is a normalized, subtracted cDNA library that was generated from the RNA derived from colon cancer cell line SW480 and normal human colon tissue. Human colorectal adenocarcinoma (cancer) cell line SW480; ATCC #CCL228 (Leibovitz et al., Cancer Research 36:4562-4569, 1976) was used to generate double-stranded cDNA that was subsequently used as the tester sample for the subtraction experiment. Poly A⁺ RNA from normal human colon tissue (purchased from OriGene Technologies, Inc. Rockville, MD) was used was used to generate double-stranded cDNA that was used as the driver sample for the subtraction experiment.

The growth conditions of the driver and tester sources in this library were different as SW480 is a rapidly growing cell line and may have higher cellular metabolism. Therefore some of the differential expression in this library might be due to non-relevant growth effects of the two sources of tissue.

### Construction of the SW480 library

Double-stranded cDNA was generated using the Clontech SMART PCR cDNA Synthesis Kit (purchased from Clontech Laboratories Inc, Palo Alto, CA) following the manufacturer's instructions. Subtraction hybridization steps were performed in accordance with the manufacturer's instructions for the Clontech PCR-Select kit (purchased from Clontech Laboratories Inc, Palo Alto, CA). The subtracted cDNAs were then directly inserted into a T/A cloning vector (TOPO TA Cloning Kit, Invitrogen Corporation, Carlsbad, CA) according to manufacturer's instructions, transformed into *E. coli,* and plated onto LB-amp plates, containing X-gal and IPTG. 1248 bacterial colonies were picked, transferred to LB-amp broth and propagated. Plasmids were isolated using column chromatography (QIAprep 96 Turbo Miniprep Kits, Qiagen Corporation, Valencia, CA) on the QIAGEN Biorobot 9600.

### Initial validation of differential expression

The inserts from subtracted clones were amplified by PCR and 10ul of the PCR reaction product was run on a 2.0% agarose gel for 2 hr at 100 volts. The gel was blotted onto a nylon membrane according to standard methods and hybridized as follows: 50 ng aliquots of the RSA1 cut SW480 and normal colon cDNA libraries were labeled with [α-³²P] dCTP by Prime-It RmT Random Primer labeling kit (Stratagene, La Jolla, CA). Nylon membranes containing the PCR amplified DNA from the SW480 library clones were hybridized to the labeled probes at 4 x 10⁶ cpm/ml in Express hybridization buffer (Clonetech) at 68°C for approximately16 hours. The membranes were subjected to stringent washes (0.1 X SSC; 0.1% SDS) done at 68°C and were then exposed to phosphorimager screens. The screens were analyzed using Molecular Dynamics ImageQuant software. Clones that exhibited a stronger hybridization signal with the SW480 probe relative to the normal colon probe were deemed to be differentially expressed.

### Validation of differential expression in colon cancer

To validate that the differentially expressed sequences found in this library were specific to colon cancer, the clones were screened with cDNAs prepared from a colon cancer specific library, Delaware (DE), and a normal tissue specific library Maryland (MD).

The DE library is specific for sequences expressed in colon cancer [proximal and distal Dukes' B, microsatellite instibility negative (MSI-)] but not expressed in normal tissues, including colon. This colon cancer tissue specific cDNA library, was made using pooled colon cancer cDNA as tester (tumor tissue cDNA pooled from eight patients with either proximal stage B MSI⁻ or distal stage B MSI⁻ cancers). The driver cDNA consisted a combination of cDNAs made from 50% normal colon tissue and a pool of peripheral blood leukocytes (PBL), and normal liver, spleen, lung, kidney, heart, small intestine, skeletal muscle, and prostate tissue cDNAs as the remaining 50% of the driver.

The MD library is specific for sequences expressed in normal tissue, but not expressed in proximal and distal Dukes' B, MSI- colon cancers. The tester cDNA in this case was made up of 50% normal colon tissue cDNA while the other 50% was made up of PBL, liver, spleen, lung, kidney, heart, small intestine, skeletal muscle, and prostate tissue cDNAs. The driver for this library was generated from pools of proximal stage B, MSI⁻ and distal stage B, MSI⁻ tumor tissue cDNAs obtained from eight cancer patients.

SW 480 clones that hybridized with the DE probe, but hybridized to a lesser degree (or not at all) to the MD probe were determined to be differentially expressed. This confirmation of differential expression is additional evidence that the up regulation of the individual clones is related to colon cancer.

### Sequencing and analysis of differentially expressed clones

The nucleotide sequence of the inserts from clones shown to be differentially expressed was determined by single-pass sequencing from either the T7 or M13 promoter sites using fluorescently labeled dideoxynucleotides via the Sanger sequencing method. Sequences were analyzed according to methods described in the text (XI., Examples; B. Results of Public Database Search).

Each nucleic acid represents sequence from at least a partial mRNA transcript. The nucleic acids of the invention were assigned a sequence identification number (see attachments). The DNA sequences are provided in the attachments containing the sequences.

Of the 1248 colonies examined, 826 individual clones were found to be differentially expressed using the SW480 and normal colon probes. Of these, 681 were found to be differentially expressed using the DE and MD tissue probes. 145 clones that previously showed differential expression with the SW480 and normal colon probes did not show differential expression with the DE and MD probes. 363 of these clones contained known sequences, 213 contained ESTs, and 105 contained novel sequences. An examination of the known sequences revealed that many of the genes are involved in cellular metabolism.

An example of an experiment to identify differentially expressed clones is shown in the Figure, "Differential Expression Analysis". The inserts from subtracted clones were amplified, electrophoresed, and blotted on to membranes as described above. The gel was hybridized with RSA1 cut DE and MD cDNA probes as described above.

In the Figure, individual clones are designated by a number at the top of each lane; the blots are aligned so that the same clone is represented in the same vertical lane in both the upper ("Cancer Probe") and lower ("Normal Probe") blot. Lanes labeled "O" indicate clones that are overexpressed, i.e., show a darker, more prominent band in the upper blot ("Cancer Probe") relative to that observed, in the same lane, in the lower blot ("Normal Probe"). The Lane labeled "U" indicates a clone that is underexpressed, i.e., shows a darker, more prominent band in the lower blot ("Normal Probe") relative to that observed, in the same lane, in the upper blot ("Cancer Probe"). The lane labeled "M", indicates a clone that is marginally overexpressed in cancer and normal cells.

### B. Results of Public Database searches

The nucleotide sequence of SEQ ID Nos. 1-850 were aligned with individual sequences that were publicly available. Genbank and divisions of GenBank, such as dbEST, CGAP, and Unigene were the primary databases used to perform the sequence similarity searches. The patent database, GENESEQ, was also utilized.

A total of 850 sequences were analyzed; most sequences were between 200 and 700 nucleotides in length. The sequences were first masked to identify vector-derived sequences, which were subsequently removed. The remaining sequence information was used to create the sequences listed in the Sequence Listing (SEQ ID Nos. 1-850). Each of these sequences was used as the query sequence to perform a Blast 2 search against the databases listed above. The Blast 2 search differs from the traditional Blast search in that it allows for the introduction of gaps in order to produce an optimal alignment of two sequences.

A proprietary algorithm was developed to utilize the output from the Blast 2 searches and categorize the sequences based upon high similarity (e value < le-40) or identity to entries contained in the GenBank and dbEST databases. Three categories were created as follows: 1) matches to known human genes, 2) matches to human EST sequences, and 3) no significant match to either 1 or 2, and therefore a potentially novel human sequence.

Those skilled in the art will recognize, or be able to ascertain, using not more than routine experimentation, many equivalents to the specific embodiments of the invention described herein. Such specific embodiments and equivalents are intended to be encompassed by the following claims.

All patents, published patent applications, and publications cited herein are incorporated by reference as if set forth fully herein.

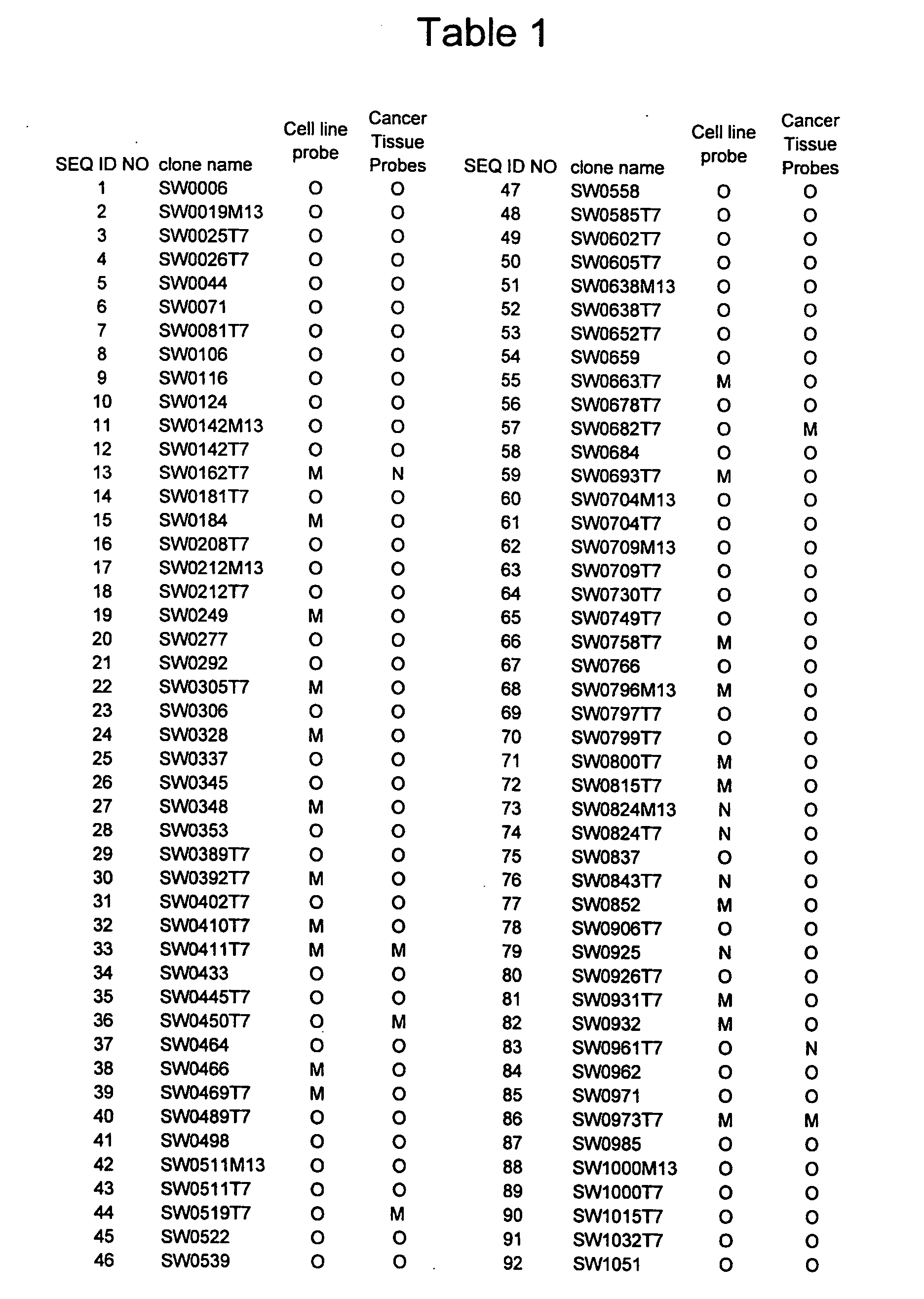

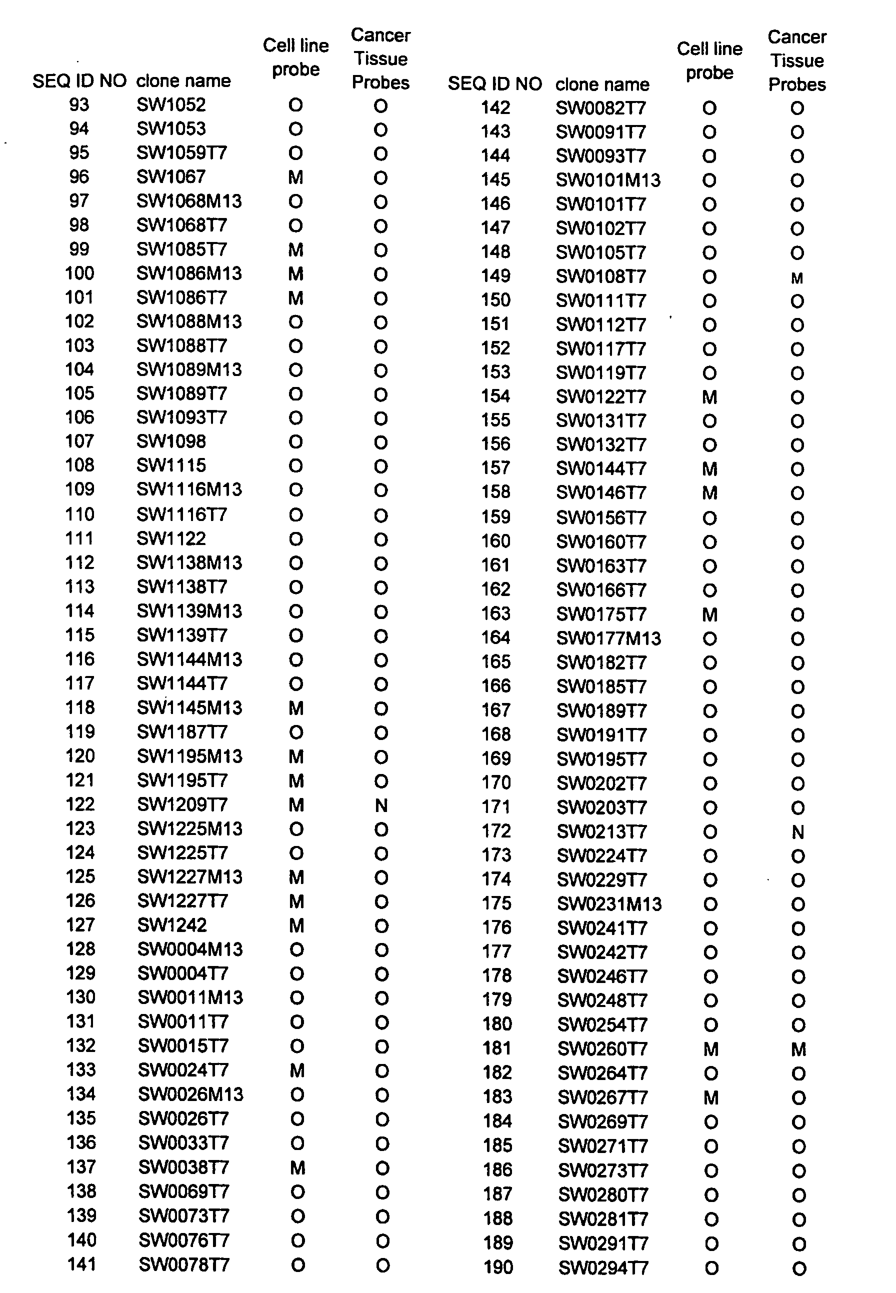

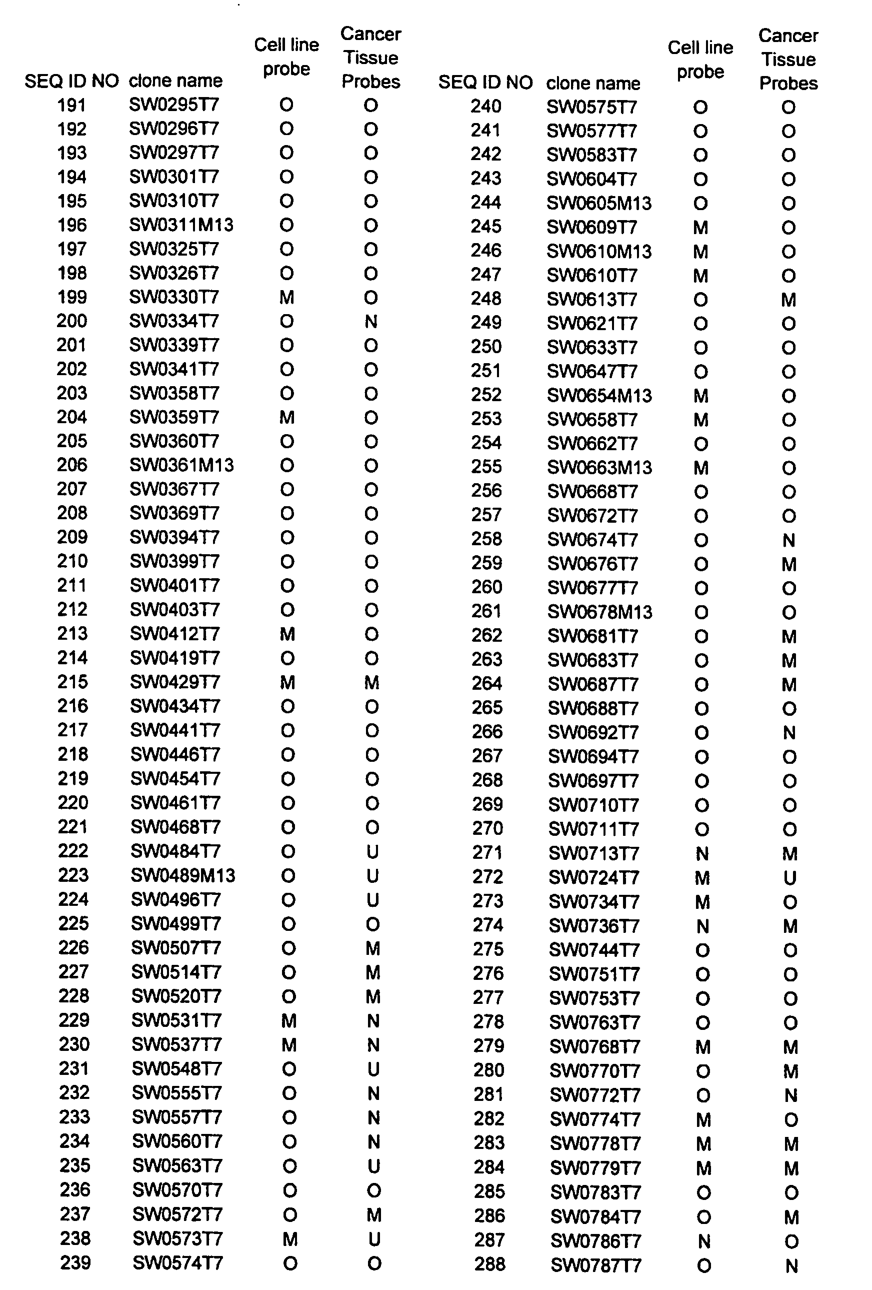

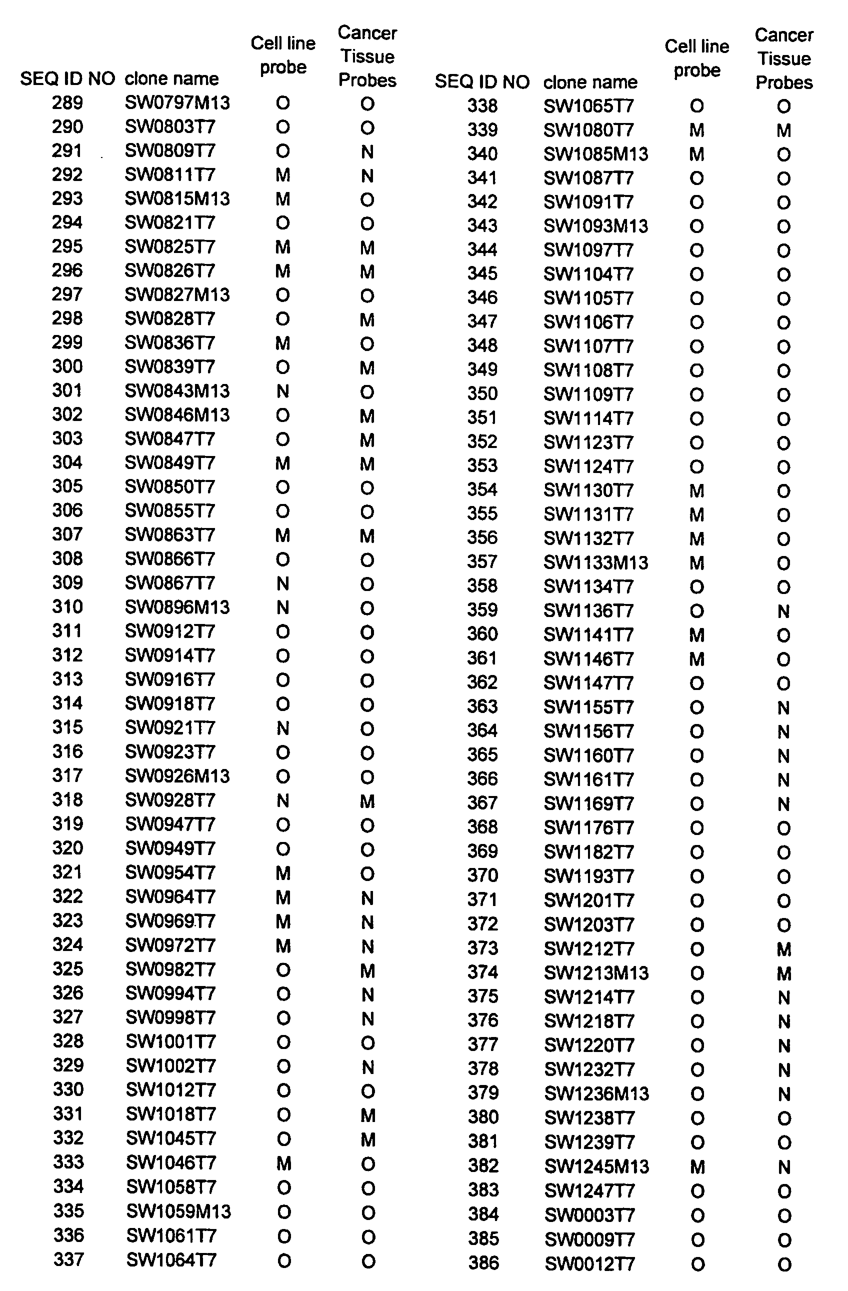

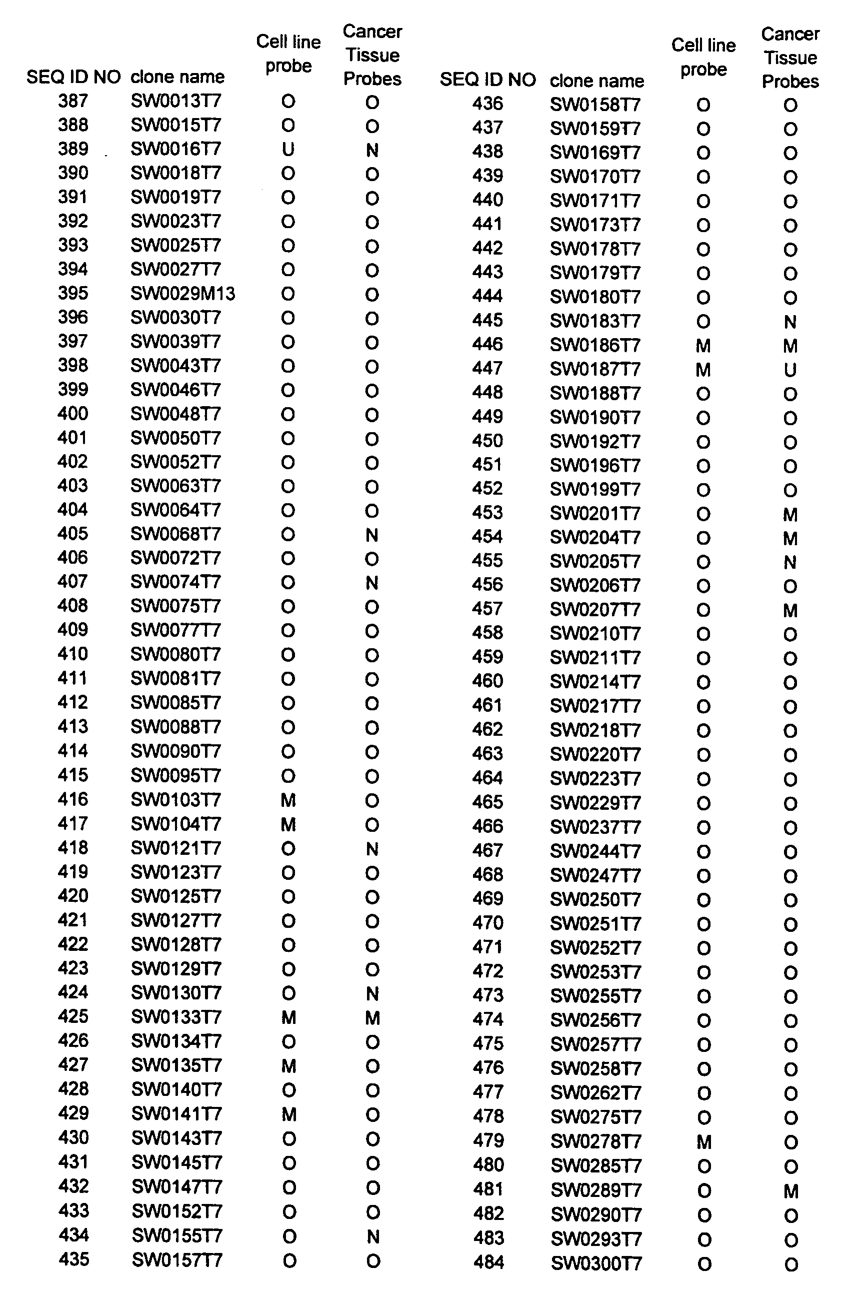

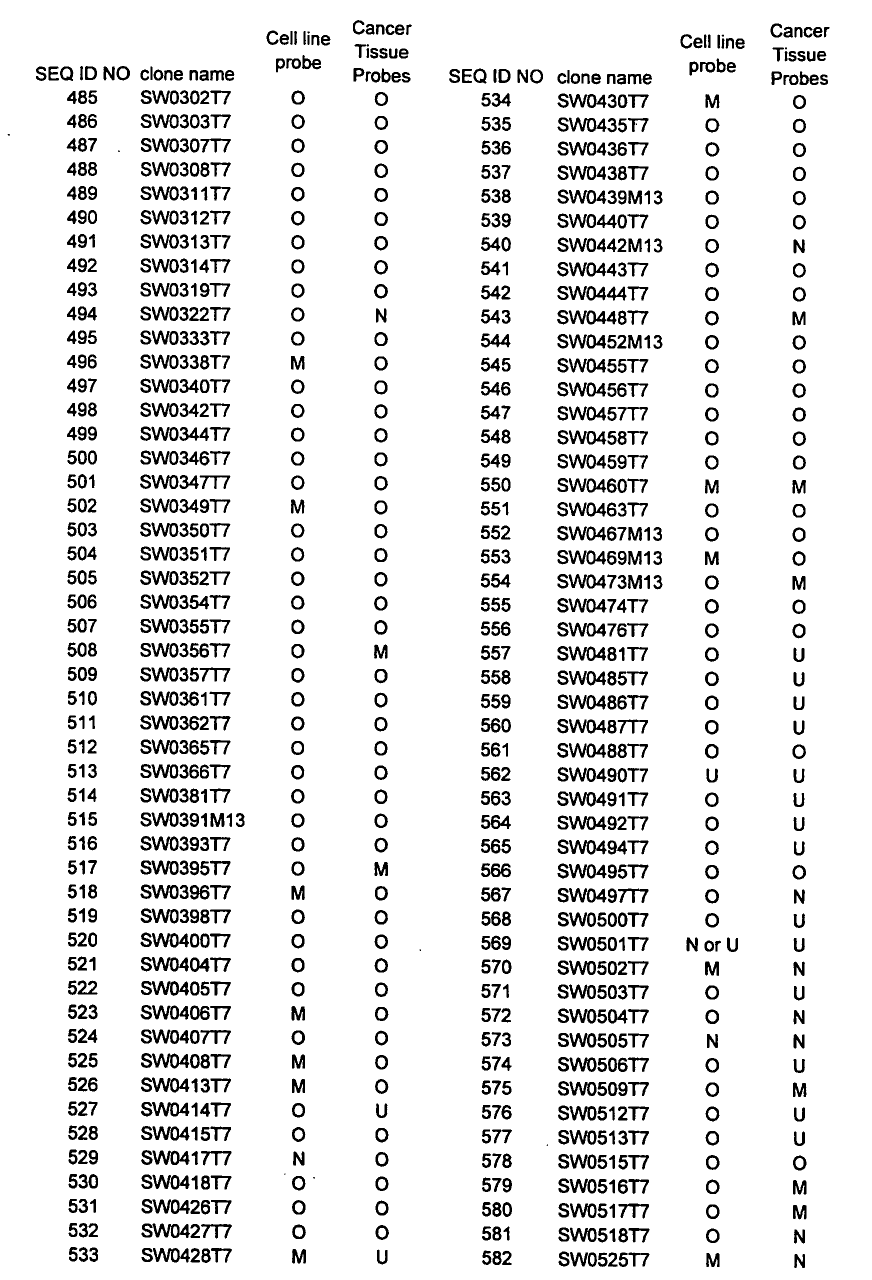

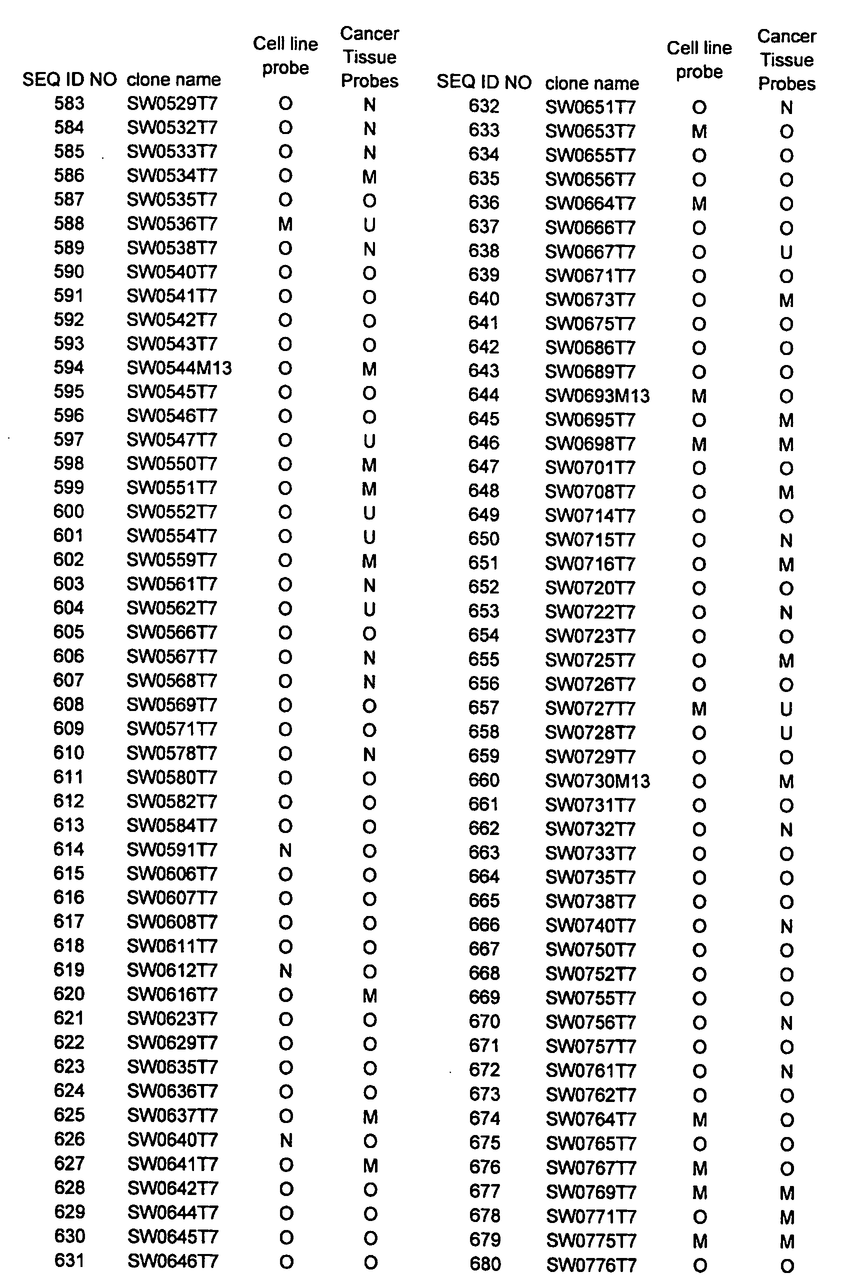

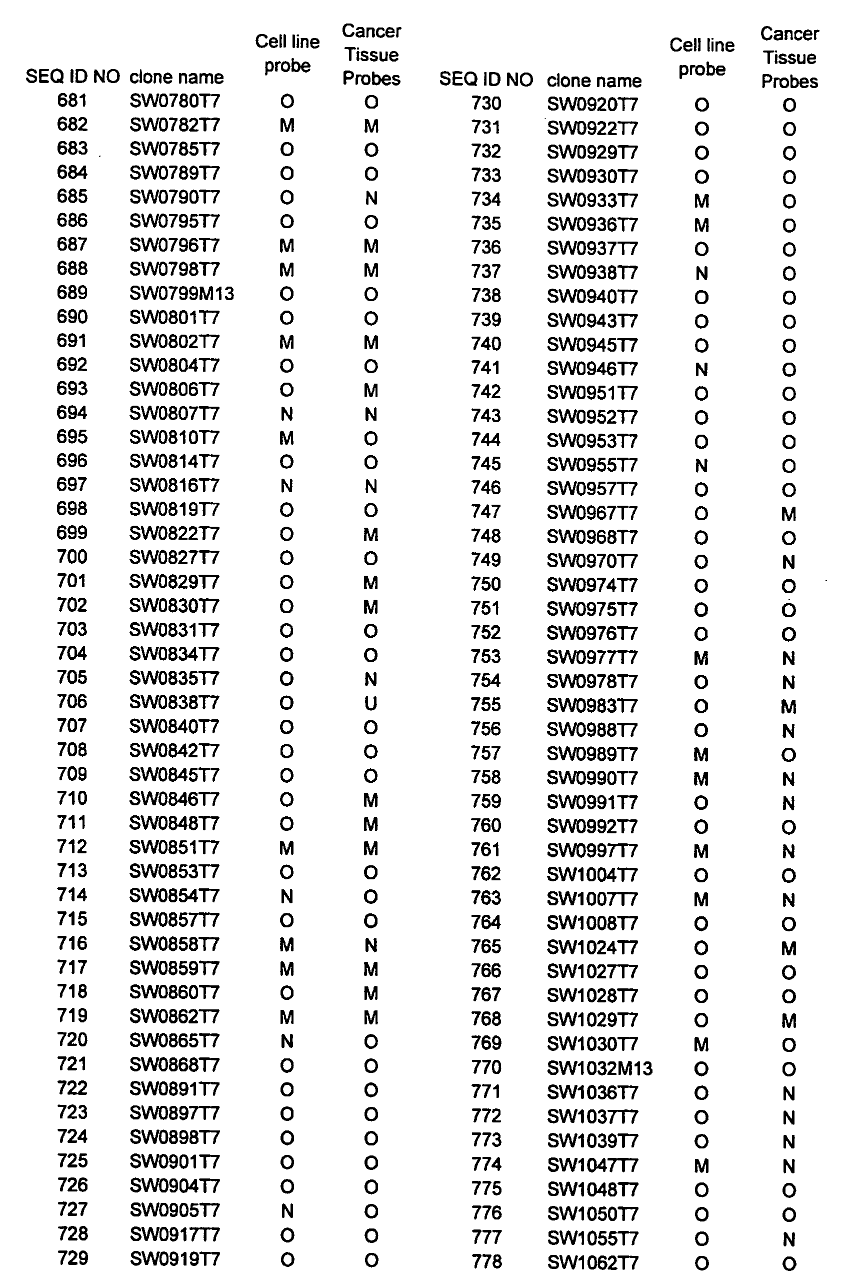

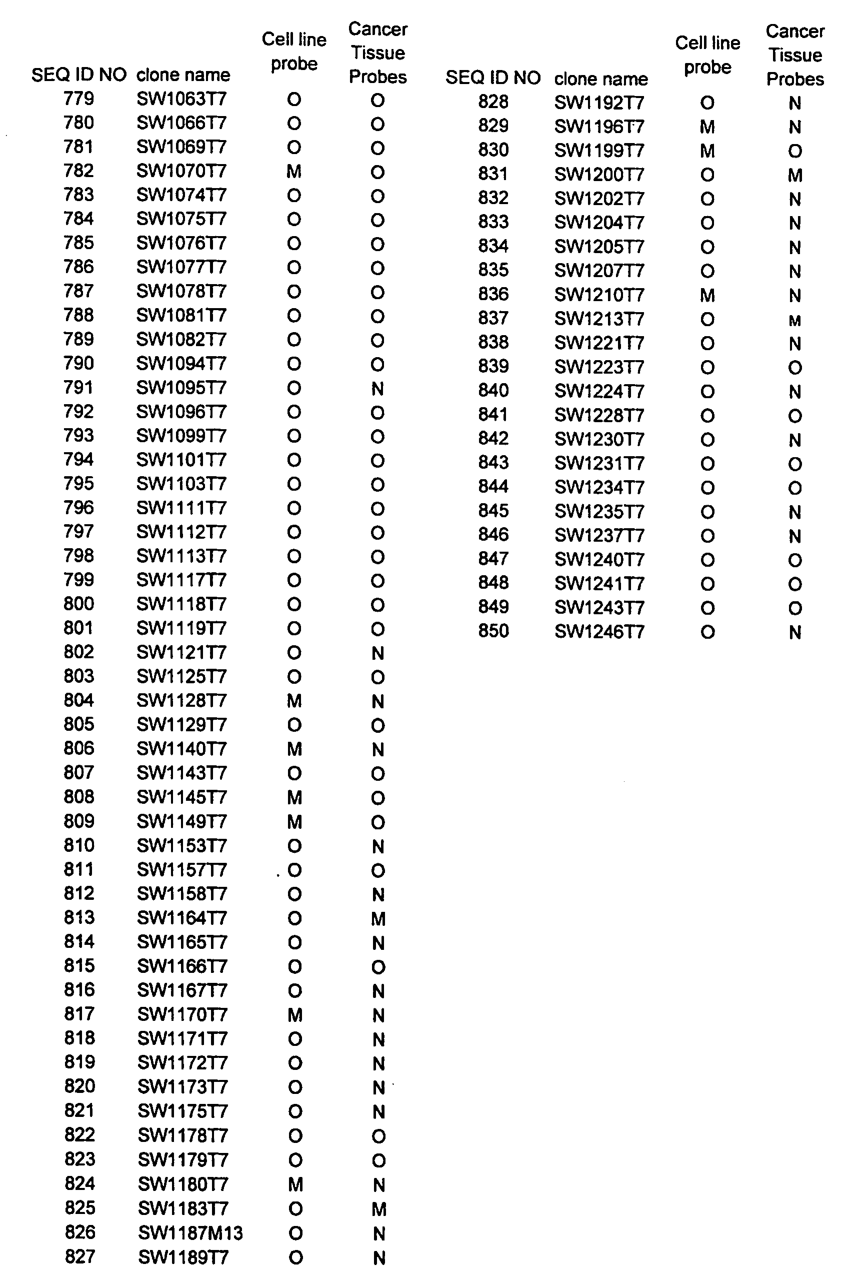

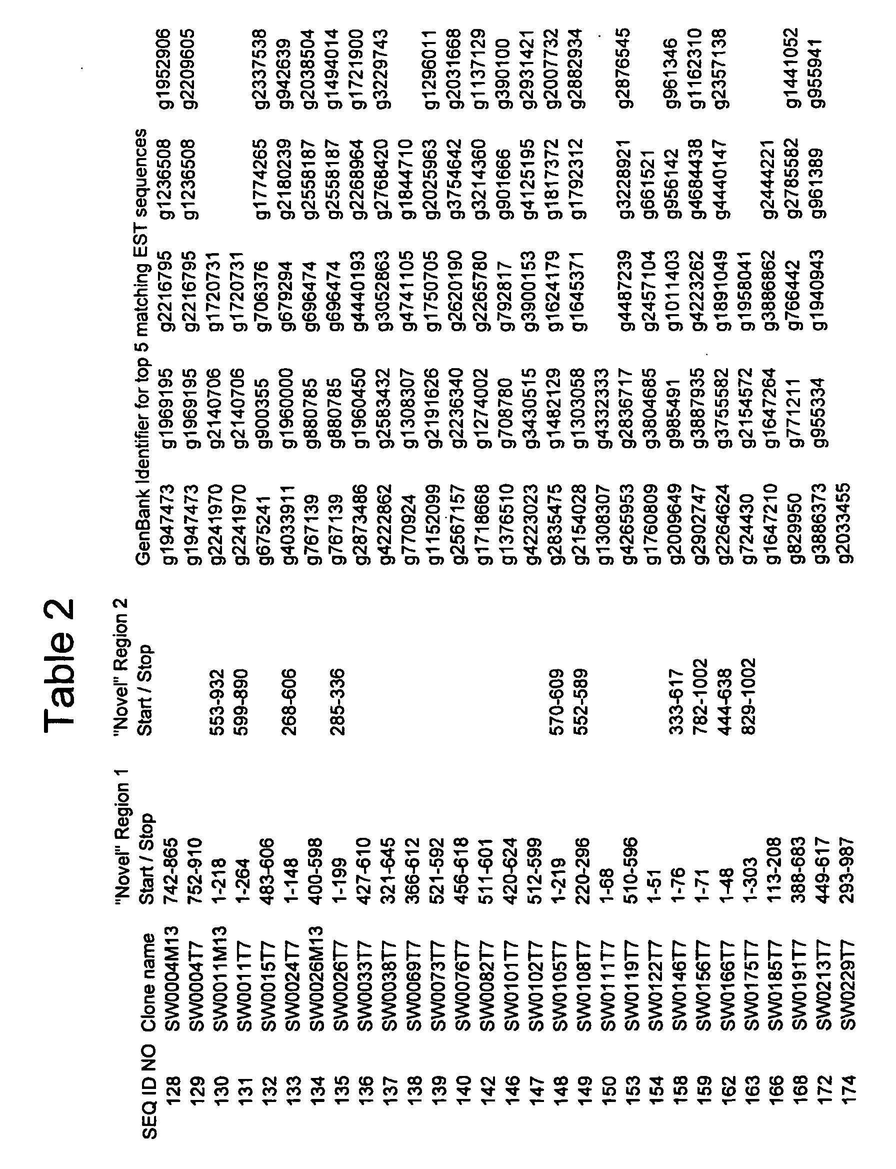

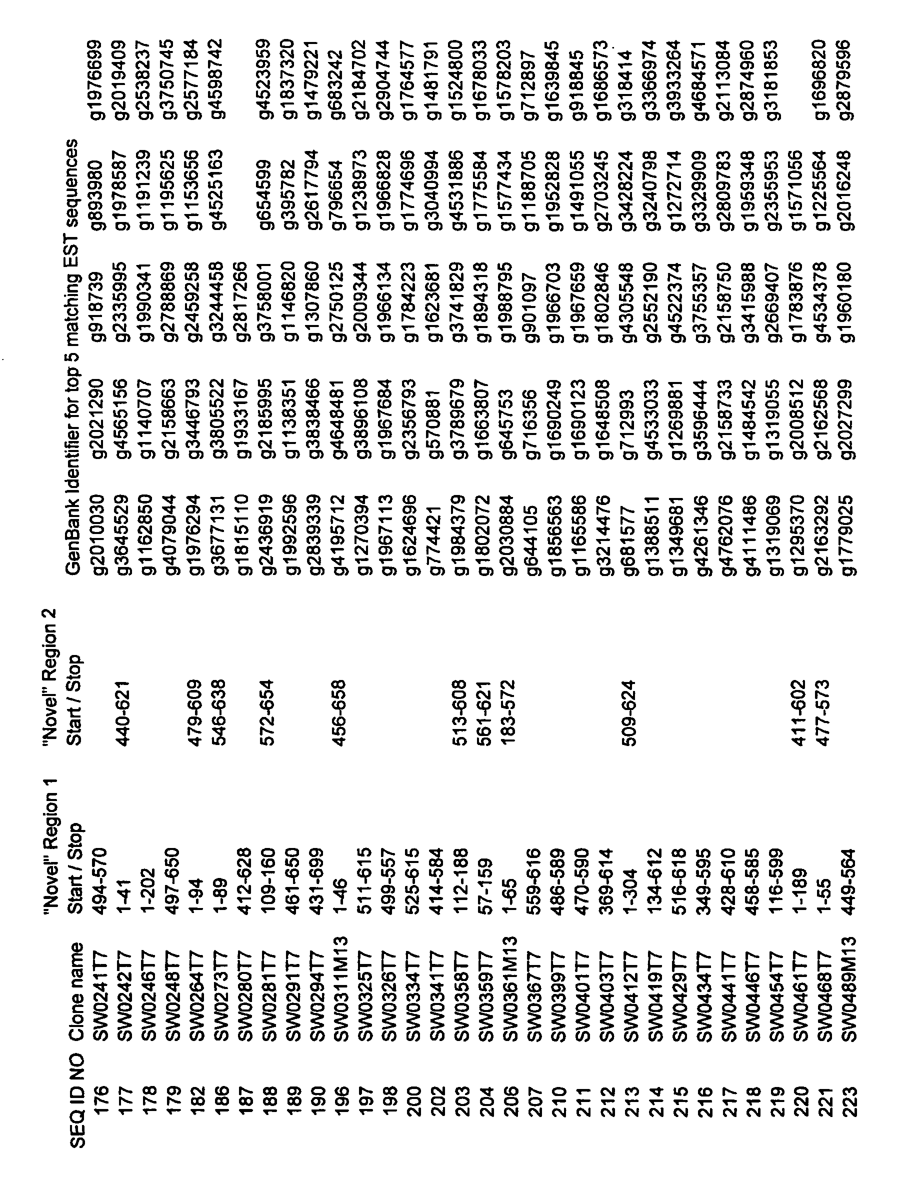

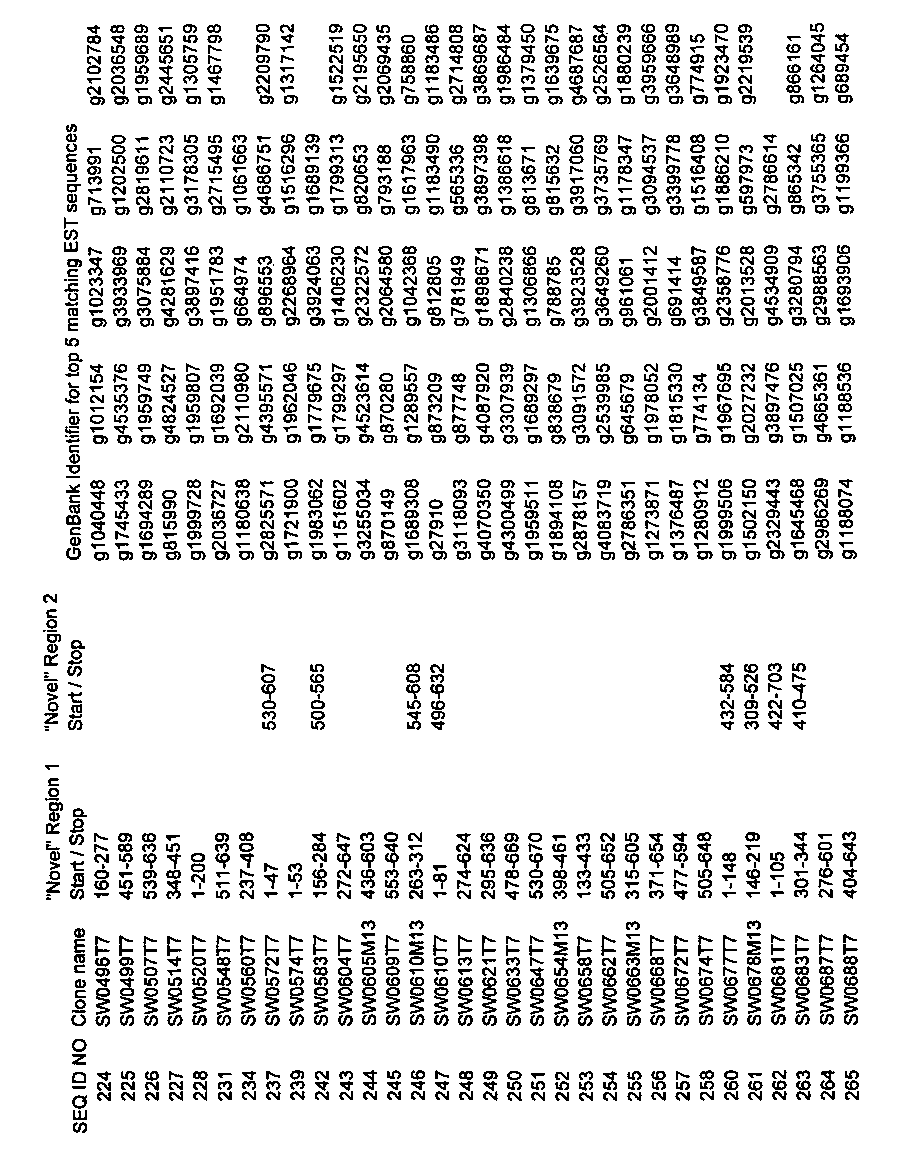

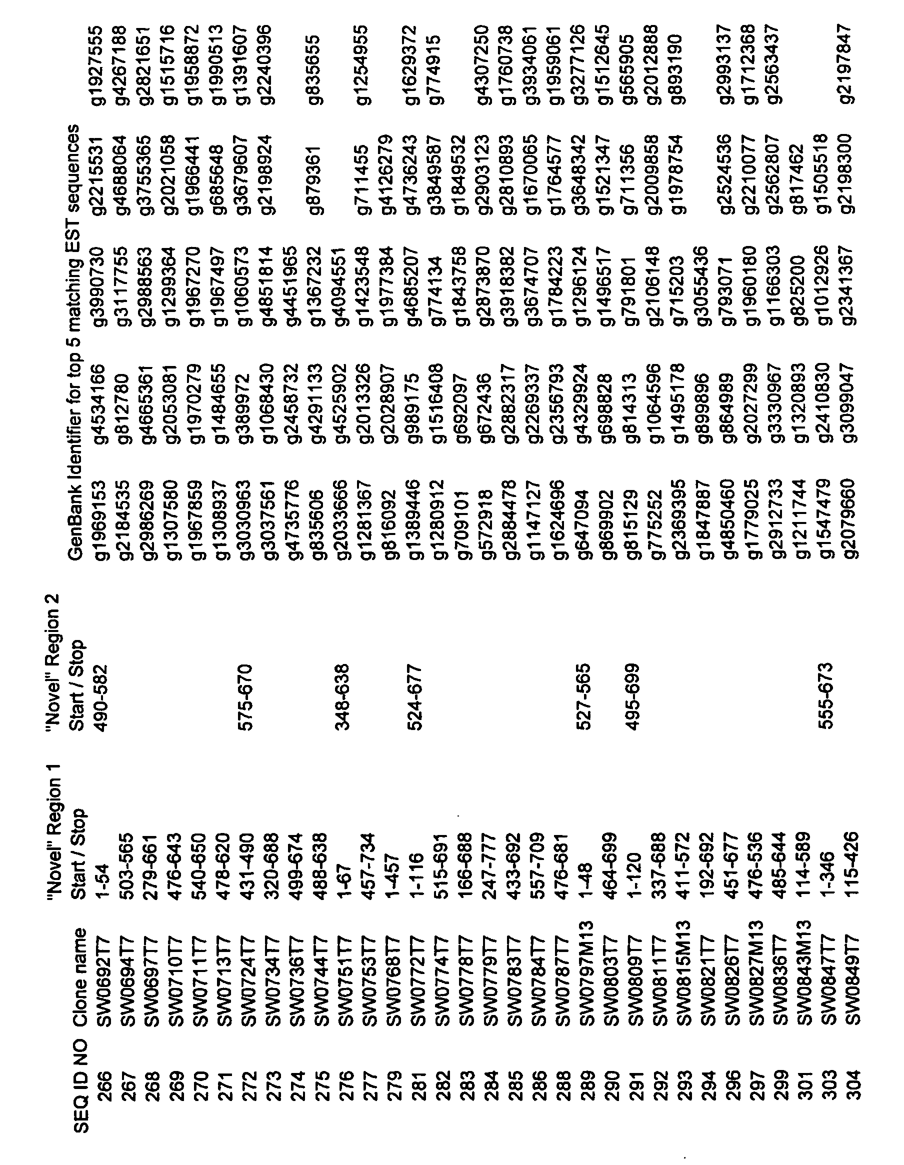

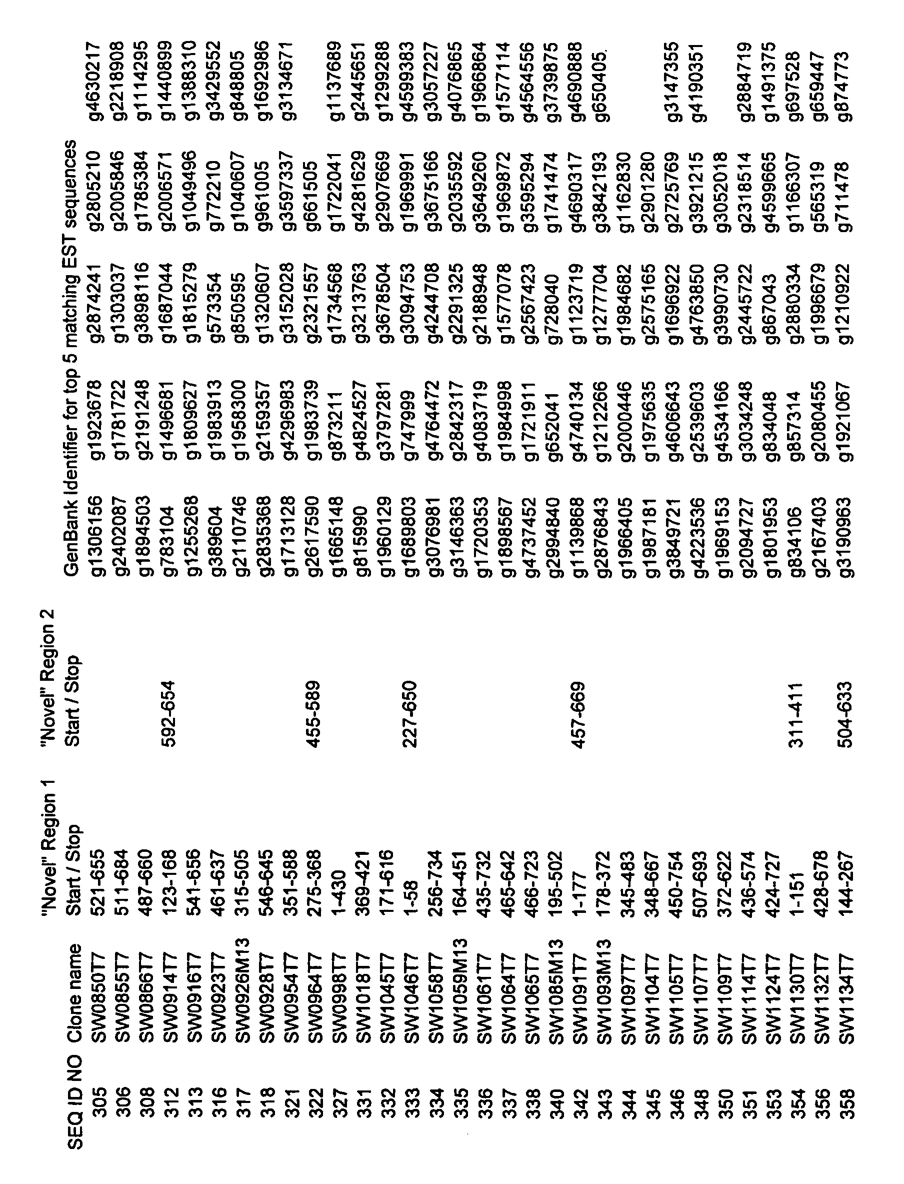

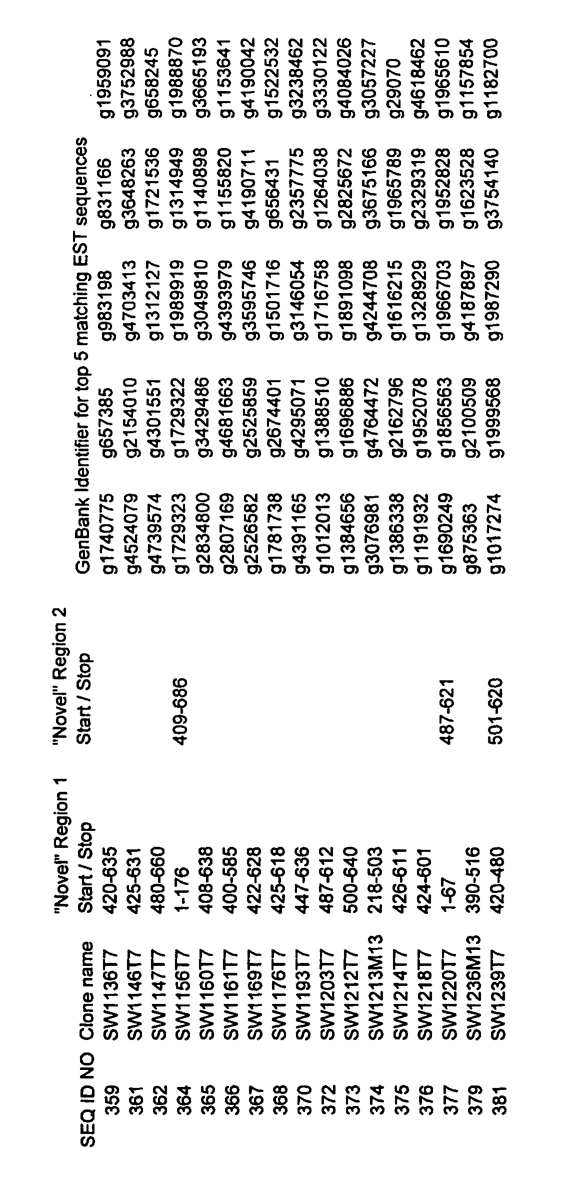

## Claims

1. An isolated nucleic acid molecule consisting of a nucleotide sequence selected from the group consisting of SEQ ID Nos. 34, 40, 55, 57, 60, 72, 128, 408, 790, and 815.

2. An isolated nucleic acid molecule consisting of a nucleotide sequence at least 85% identical to a sequence selected from the group consisting of SEQ ID Nos. 34, 40, 55, 57, 60, 72, 128, 408, 790, and 815.

3. An expression vector, capable of replicating in at least one of a prokaryotic cell and eukaryotic cell, consisting of vector sequences and the nucleic acid of claim 1.

4. The expression vector of claim 3, wherein the vector sequences include a transcriptional regulatory sequence operably linked to said nucleotide sequence so as to render said expression vector capable of replicating in said at least one of a prokaryotic cell and eukaryotic cell.

5. A host cell transfected with the expression vector of claim 3.

6. A pharmaceutical composition comprising a nucleic acid molecule consisting of a sequence selected from the group consisting of SEQ ID Nos. 34, 40, 55, 57, 60, 72, 128, 408, 790, and 815.

7. The isolated nucleic acid molecule of claim 1, wherein said nucleic acid molecule is resistant to cleavage by a nuclease.

8. An isolated nucleic acid molecule consisting of a nucleotide sequence complementary to a nucleotide sequence selected from the group consisting of SEQ ID Nos. 34, 40, 55, 57, 60, 72, 128, 408, 790, and 815.

9. An isolated nucleic acid molecule consisting of a nucleotide sequence complementary to a nucleotide sequence at least 85% identical to a sequence selected from the group consisting of SEQ ID Nos. 34, 40, 55, 57, 60, 72, 128, 408, 790, and 815.

10. A pharmaceutical composition comprising a nucleic acid molecule consisting of a nucleotide sequence complementary to a nucleic acid molecule selected from the group consisting of SEQ ID Nos. 34, 40, 55, 57, 60, 72, 128, 408, 790, and 815.
